(19) Europäisches Patentamt
European Patent Office
Office européen des brevets

(11) EP 4 198 057 A1

(12) EUROPEAN PATENT APPLICATION

(43) Date of publication:
**21.06.2023 Bulletin 2023/25**

(21) Application number: **22152336.8**

(22) Date of filing: **04.12.2019**

(51) International Patent Classification (IPC):
***C07K 16/30*** (2006.01) ***C12N 5/0783*** (2010.01)
***A61P 35/00*** (2006.01) ***A61K 35/17*** (2015.01)
***A61K 39/395*** (2006.01) ***A61K 39/00*** (2006.01)
***C07K 16/22*** (2006.01) ***C07K 16/28*** (2006.01)
***G01N 33/50*** (2006.01)

(52) Cooperative Patent Classification (CPC):
(C-Sets available)
**C07K 16/30; A61K 35/17; A61K 39/395; A61P 35/00; C12N 5/0636; G01N 33/505;**
A61K 39/3955; A61K 2039/505; A61K 2039/507; C07K 16/22; C07K 16/2827; C07K 2317/24; C07K 2317/76; C07K 2319/00; C07K 2319/33;

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.12.2018 US 201862775720 P**
**21.11.2019 US 201962938427 P**

(62) Document number(s) of the earlier application(s) in accordance with Art. 76 EPC:
**19828097.6 / 3 891 185**

(27) Previously filed application:
**04.12.2019 PCT/US2019/064498**

(71) Applicant: **F. Hoffmann-La Roche AG**
**4070 Basel (CH)**

(72) Inventors:
- **WU, Thomas D.**
**South San Francisco, 94080 (US)**
- **GROGAN, Jane Louise**
**South San Francisco, 94080 (US)**

(74) Representative: **Kaschau, Nikolai Elias**
**F. Hoffmann-La Roche AG**
**Patent Department CLP**
**Grenzacherstrasse 124**
**4070 Basel (CH)**

Remarks:
•The complete document including Reference Table(s) and the Sequence Listing(s) can be downloaded from the EPO website
•This application was filed on 20-01-2022 as a divisional application to the application mentioned under INID code 62.
•Claims filed after the date of filing of the application (Rule 68(4) EPC).

# (54) DIAGNOSTIC METHODS AND COMPOSITIONS FOR CANCER IMMUNOTHERAPY

(57) The present invention provides diagnostic methods, therapeutic methods, and compositions for the treatment of cancer. The compositions and methods described herein can be used, for example, to determine the propensity of a patient to benefit from treatment with a PD-L1 axis binding antagonist and to treat such patients accordingly. Using the compositions and methods of the disclosure, a patient, such as a human cancer patient, may be determined to be likely to benefit from treatment with a PD-L1 axis binding antagonist if the patient exhibits an elevated pre-treatment expression level of one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. Exemplary PD-L1 axis binding antagonists that may be used in conjunction with the compositions and methods of the disclosure are PD-L1 binding antagonists, such as anti-PD-L1 antibodies and antigen-binding fragments thereof, including atezolizumab, as well as PD-1 binding antagonists, such as anti-PD-1 antibodies and antigen-binding fragments thereof.

EP 4 198 057 A1

(52) Cooperative Patent Classification (CPC): (Cont.)
C12N 2510/00

C-Sets
**A61K 35/17, A61K 2300/00;**
A61K 39/3955, A61K 2300/00

## Description

## SEQUENCE LISTING

The instant application contains a Sequence Listing which has been submitted electronically in ASCII format and is hereby incorporated by reference in its entirety. Said ASCII copy, created on November 27, 2019 is named 50474-188WO3_Sequence_Listing_11.27.19_ST25 and is 195,457 bytes in size.

## FIELD OF THE INVENTION

The present invention is directed to diagnostic and therapeutic methods for the treatment of cancer using PD-L1 axis binding antagonists. Also provided are related assays and kits.

## BACKGROUND OF THE INVENTION

Cancer remains one of the most deadly threats to human health. In the U.S., cancer affects nearly 1.3 million new patients each year and is the second leading cause of death after heart disease, accounting for approximately 1 in 4 deaths. It is also predicted that cancer may surpass cardiovascular diseases as the number one cause of death within 5 years. Solid tumors are responsible for most of those deaths.

Studies in humans with immune checkpoint inhibitors have demonstrated the promise of harnessing the immune system to control and eradicate tumor growth. The programmed death 1 (PD-1) receptor and its ligand programmed death-ligand 1 (PD-L1) are immune checkpoint proteins that have been implicated in the suppression of immune system responses during chronic infections, pregnancy, tissue allografts, autoimmune diseases, and cancer. PD-L1 regulates the immune response by binding to the inhibitory receptor PD-1, which is expressed on the surface of T-cells, B-cells, and monocytes. PD-L1 negatively regulates T-cell function also through interaction with another receptor, B7-1. Formation of the PD-L1/PD-1 and PD-L1/B7-1 complexes negatively regulates T-cell receptor signaling, resulting in the subsequent downregulation of T-cell activation and suppression of anti-tumor immune activity.

Although there have been significant advances in the medical treatment of certain cancers, the overall 5-year survival rate for all cancers has improved only by about 10% in the past 20 years. Malignant solid tumors, in particular, metastasize and grow rapidly in an uncontrolled manner, making their timely detection and treatment extremely difficult.

Despite the significant advancement in the treatment of cancer, improved diagnostic methods and cancer therapies and are still being sought.

## SUMMARY OF THE INVENTION

The present disclosure provides therapeutic and diagnostic methods and compositions for treating an individual having a cancer (e.g., lung cancer (e.g., non-small cell lung cancer (NSCLC)), endometrial cancer, colon adenocarcinoma, renal cell carcinoma, bladder cancer (e.g., urothelial carcinoma (UC)), kidney cancer (e.g., renal cell carcinoma (RCC)), and breast cancer (e.g., triple-negative breast cancer (TNBC))). The disclosure is based, at least in part, on the discovery that patients having cancer (e.g., lung cancer (e.g., NSCLC), endometrial cancer, colon adenocarcinoma, renal cell carcinoma, bladder cancer (e.g., UC), kidney cancer (e.g., RCC), and breast cancer (e.g., TNBC)) that exhibit clonally expanded T cells in peripheral circulation are particularly likely to benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody). Clonally expanded T cells can be identified in various ways, including by sequencing a patient's T cell receptors to identify the extent of clonal expansion and/or by detecting an elevated immune-score expression level of one or more genes associate with clonal expansion in the patient's peripheral blood. Examples of such genes include CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, 1FITM1, TMSB10, CD247, COX2, COX1, CLIC3, S100A4, and CYBA.

Using the compositions and methods of the disclosure, a patient may be identified as likely to respond to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) on the basis of a finding that the patient exhibits a clonally expanded T cell population in peripheral blood. The determination that the patient exhibits clonally expanded T cells in peripheral blood may be made by sequencing T cell receptors and/or by evaluating the patient's gene expression profile in a blood sample obtained from the patient. In this context, a finding that the patient exhibits an elevated immune score expression level of, for example, one or more genes selected from CST7, NKG7, GZMH, MT-ND4, HLA-H, CCL5, CD8A, CMC1, CD8B, HCST, MT-CYB, NT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA can indicate that the patient has clonally expanded T cells in peripheral circulation, and that the patient is likely to respond to treatment that includes a PD-L1 axis binding antagonist.

In a first aspect, the disclosure features a method of producing a chimeric antigen receptor (CAR) T cell, the method comprising:

(a) obtaining a sample of peripheral blood from an individual having cancer;
(b) determining (i) an amino acid sequence of one or more complementarity-determining regions (CDRs) of a T cell receptor (TCR) expressed by one or more T cells in the sample and/or (ii) a nucleic acid sequence encoding the amino acid sequence; and
(c) modifying a precursor cell to express a CAR comprising the one or more CDRs, thereby producing a CAR T cell.

In another aspect, the disclosure features a method of producing a CAR T cell, the method comprising modifying a precursor cell to express a CAR comprising one or more CDRs of a TCR expressed by one or more T cells in a sample of peripheral blood obtained from an individual having cancer.

In some embodiments, the T cells in the sample are clonally expanded T cells.

In some embodiments, the clonally expanded T cells are CD8+ T cells.

In some embodiments, the CD8+ T cells are CTL.

In some embodiments, the TCR expressed by the one or more T cells specifically binds an antigen expressed by a cancer cell in the individual.

In some embodiments, the clonally expanded T cells comprise at least 0.01 % of all T cells in the sample.

In some embodiments, the clonally expanded T cells comprise at least 0.1 % of all T cells in the sample.

In some embodiments, the clonally expanded T cells comprise at least 1 % of all T cells in the sample.

In some embodiments, the clonally expanded T cells comprise at least 2% of all T cells in the sample.

In some embodiments, the clonally expanded T cells comprise at least 3% of all T cells in the sample.

In some embodiments, the clonally expanded T cells comprise at least 4% of all T cells in the sample.

In some embodiments, the clonally expanded T cells comprise at least 5% of all T cells in the sample.

In some embodiments, the clonally expanded T cells comprise at least 10% of all T cells in the sample.

In some embodiments, the clonally expanded T cells comprise at least 15% of all T cells in the sample.

In some embodiments, the clonally expanded T cells comprise at least 20% of all T cells in the sample.

In some embodiments, the clonally expanded T cells comprise at least 25% of all T cells in the sample.

In some embodiments, the precursor cell is a T cell.

In some embodiments, the method produces at least about $1 \times 10^3$ CAR T cells.

In some embodiments, the method produces from about $1 \times 10^3$ CAR T cells to about $1 \times 10^{12}$ CAR T cells (e.g., from about $2 \times 10^3$ CAR T cells to about $9 \times 10^{11}$ CAR T cells, from about $3 \times 10^3$ CAR T cells to about 8 x $10^{11}$ CAR T cells, from about $4 \times 10^3$ CAR T cells to about $7 \times 10^{11}$ CAR T cells, from about $5 \times 10^3$ CAR T cells to about $6 \times 10^{11}$ CAR T cells, from about $6 \times 10^3$ CAR T cells to about $4 \times 10^{11}$ CAR T cells, from about $7 \times 10^3$ CAR T cells to about $3 \times 10^{11}$ CAR T cells, from about $8 \times 10^3$ CAR T cells to about $2 \times 10^{11}$ CAR T cells, from about $9 \times 10^3$ CAR T cells to about $1 \times 10^{11}$ CAR T cells, from about $1 \times 10^4$ CAR T cells to about $9 \times 10^{10}$ CAR T cells, from about $2 \times 10^4$ CAR T cells to about $8 \times 10^{10}$ CAR T cells, from about $3 \times 10^4$ CAR T cells to about $7 \times 10^{10}$ CAR T cells, from about $4 \times 10^4$ CAR T cells to about $6 \times 10^{10}$ CAR T cells, from about $5 \times 10^4$ CAR T cells to about $5 \times 10^{10}$ CAR T cells, from about $6 \times 10^4$ CAR T cells to about $4 \times 10^{10}$ CAR T cells, from about $7 \times 10^4$ CAR T cells to about $3 \times 10^{10}$ CAR T cells, from about $8 \times 10^4$ CAR T cells to about $2 \times 10^{10}$ CAR T cells, from about $9 \times 10^4$ CAR T cells to about $1 \times 10^{10}$ CAR T cells, or from about $1 \times 10^5$ CAR T cells to about $9 \times 10^9$ CAR T cells, such as about $1 \times 10^3$ CAR T cells, $2 \times 10^3$ CAR T cells, $3 \times 10^3$ CAR T cells, $4 \times 10^3$ CAR T cells, $5 \times 10^3$ CAR T cells, $6 \times 10^3$ CART cells, $7 \times 10^3$ CART cells, $8 \times 10^3$ CAR T cells, $9 \times 10^3$ CART cells, $1 \times 10^4$ CAR T cells, $2 \times 10^4$ CAR T cells, $3 \times 10^4$ CAR T cells, $4 \times 10^4$ CAR T cells, $5 \times 10^4$ CAR T cells, $6 \times 10^4$ CAR T cells, $7 \times 10^4$ CAR T cells, $8 \times 10^4$ CAR T cells, $9 \times 10^4$ CAR T cells, $1 \times 10^5$ CAR T cells, $2 \times 10^5$ CAR T cells, $3 \times 10^5$ CART cells, $4 \times 10^5$ CAR T cells, $5 \times 10^5$ CAR T cells, $6 \times 10^5$ CAR T cells, $7 \times 10^5$ CART cells, $8 \times 10^5$ CART cells, $9 \times 10^5$ CAR T cells, $1 \times 10^6$ CAR T cells, $2 \times 10^6$ CAR T cells, $3 \times 10^6$ CAR T cells, $4 \times 10^6$ CART cells, $5 \times 10^6$ CAR T cells, $6 \times 10^6$ CART cells, $7 \times 10^6$ CAR T cells, $8 \times 10^6$ CART cells, $9 \times 10^6$ CAR T cells, $1 \times 10^7$ CAR T cells, $2 \times 10^7$ CART cells, $3 \times 10^7$ CAR T cells, $4 \times 10^7$ CAR T cells, $5 \times 10^7$ CAR T cells, $6 \times 10^7$ CAR T cells, $7 \times 10^7$ CAR T cells, $8 \times 10^7$ CAR T cells, $9 \times 10^7$ CAR T cells, $1 \times 10^8$ CAR T cells, $2 \times 10^8$ CAR T cells, $3 \times 10^8$ CAR T cells, $4 \times 10^8$ CAR T cells, $5 \times 10^8$ CAR T cells, $6 \times 10^8$ CART cells, $7 \times 10^8$ CAR T cells, $8 \times 10^8$ CAR T cells, $9 \times 10^8$ CAR T cells, $1 \times 10^9$ CAR T cells, $2 \times 10^9$ CAR T cells, $3 \times 10^9$ CAR T cells, $4 \times 10^9$ CART cells, $5 \times 10^9$ CAR T cells, $6 \times 10^9$ CAR T cells, $7 \times 10^9$ CAR T cells, $8 \times 10^9$ CAR T cells, $9 \times 10^9$ CAR T cells, $1 \times 10^{10}$ CAR T cells, $2 \times 10^{10}$ CAR T cells, $3 \times 10^{10}$ CAR T cells, $4 \times 10^{10}$ CAR T cells, $5 \times 10^{10}$ CAR T cells, $6 \times 10^{10}$ CAR T cells, $7 \times 10^{10}$ CAR T cells, $8 \times 10^{10}$ CAR T cells, $9 \times 10^{10}$ CAR

T cells, 1 $\times$ $10^{11}$ CAR T cells, 2 $\times$ $10^{11}$ CAR T cells, 3 $\times$ $10^{11}$ CART cells, 4 $\times$ $10^{11}$ CAR T cells, 5 $\times$ $10^{11}$ CAR T cells, 6 $\times$ $10^{11}$ CAR T cells, 7 $\times$ $10^{11}$ CAR T cells, 8x $10^{11}$ CAR T cells, 9 $\times$ $10^{11}$ CAR T cells, or $\times$ $10^{12}$ CAR T cells, or more).

In some embodiments, the method produces from about 1 $\times$ $10^4$ CAR T cells to about 1 $\times$ $10^{11}$ CAR T cells (e.g., from about 1 $\times$ $10^4$ CAR T cells to about 9 $\times$ $10^{10}$ CAR T cells, from about 2 $\times$ $10^4$ CAR T cells to about 8 $\times$ $10^{10}$ CAR T cells, from about 3 $\times$ $10^4$ CAR T cells to about 7 $\times$ $10^{10}$ CAR T cells, from about 4 $\times$ $10^4$ CAR T cells to about 6 $\times$ $10^{10}$ CAR T cells, from about 5 $\times$ $10^4$ CAR T cells to about 5 $\times$ $10^{10}$ CAR T cells, from about 6 $\times$ $10^4$ CAR T cells to about 4 $\times$ $10^{10}$ CAR T cells, from about 7 $\times$ $10^4$ CAR T cells to about 3 $\times$ $10^{10}$ CAR T cells, from about 8 $\times$ $10^4$ CAR T cells to about 2 $\times$ $10^{10}$ CAR T cells, from about 9 $\times$ $10^4$ CAR T cells to about 1 $\times$ $10^{10}$ CAR T cells, or from about 1 $\times$ $10^5$ CAR T cells to about 9 $\times$ $10^9$ CAR T cells, such as about 1 $\times$ $10^4$ CAR T cells, 2 $\times$ $10^4$ CAR T cells, 3 $\times$ $10^4$ CAR T cells, 4 $\times$ $10^4$ CAR T cells, 5 $\times$ $10^4$ CAR T cells, 6 $\times$ $10^4$ CAR T cells, 7 $\times$ $10^4$ CAR T cells, 8 $\times$ $10^4$ CAR T cells, 9 $\times$ $10^4$ CAR T cells, 1 $\times$ $10^5$ CAR T cells, 2 $\times$ $10^5$ CAR T cells, 3 $\times$ $10^5$ CAR T cells, 4 $\times$ $10^5$ CAR T cells, 5 $\times$ $10^5$ CAR T cells, 6 $\times$ $10^5$ CAR T cells, 7 $\times$ $10^5$ CAR T cells, 8 $\times$ $10^5$ CAR T cells, 9 $\times$ $10^5$ CAR T cells, 1 $\times$ $10^6$ CAR T cells, 2 $\times$ $10^6$ CAR T cells, 3 $\times$ $10^6$ CAR T cells, 4 $\times$ $10^6$ CART cells, 5 $\times$ $10^6$ CART cells, 6 $\times$ $10^6$ CAR T cells, 7 $\times$ $10^6$ CAR T cells, 8 $\times$ $10^6$ CAR T cells, 9 $\times$ $10^6$ CAR T cells, 1 $\times$ $10^7$ CAR T cells, 2 $\times$ $10^7$ CAR T cells, 3 $\times$ $10^7$ CAR T cells, 4 $\times$ $10^7$ CAR T cells, 5 $\times$ $10^7$ CAR T cells, 6 $\times$ $10^7$ CAR T cells, 7 $\times$ $10^7$ CAR T cells, 8 $\times$ $10^7$ CART cells, 9 $\times$ $10^7$ CAR T cells, 1 $\times$ $10^8$ CART cells, 2 $\times$ $10^8$ CART cells, 3 $\times$ $10^8$ CAR T cells, 4 $\times$ $10^8$ CAR T cells, 5 $\times$ $10^8$ CAR T cells, 6 $\times$ $10^8$ CART cells, 7 $\times$ $10^8$ CART cells, 8 $\times$ $10^8$ CART cells, 9 $\times$ $10^8$ CAR T cells, 1 $\times$ $10^9$ CAR T cells, 2 $\times$ $10^9$ CAR T cells, 3 $\times$ $10^9$ CART cells, 4 $\times$ $10^9$ CART cells, 5 $\times$ $10^9$ CAR T cells, 6 $\times$ $10^9$ CAR T cells, 7 $\times$ $10^9$ CAR T cells, 8 $\times$ $10^9$ CAR T cells, 9 $\times$ $10^9$ CAR T cells, 1 $\times$ $10^{10}$ CAR T cells, 2 $\times$ $10^{10}$ CAR T cells, 3 $\times$ $10^{10}$ CAR T cells, 4 $\times$ $10^{10}$ CAR T cells, 5 $\times$ $10^{10}$ CAR T cells, 6 $\times$ $10^{10}$ CART cells, 7 $\times$ $10^{10}$ CAR T cells, 8 $\times$ $10^{10}$ CAR T cells, 9 $\times$ $10^{10}$ CAR T cells, or 1 $\times$ $10^{11}$ CAR T cells, or more).

In some embodiments, the method produces from about 1 $\times$ $10^5$ CAR T cells to about 1 $\times$ $10^{10}$ CAR T cells (e.g., from about 1 $\times$ $10^5$ CAR T cells to about 9 $\times$ $10^9$ CAR T cells, such as about 1 $\times$ $10^5$ CAR T cells, 2 $\times$ $10^5$ CAR T cells, 3 $\times$ $10^5$ CAR T cells, 4 $\times$ $10^5$ CAR T cells, 5x $10^5$ CAR T cells, 6 $\times$ $10^5$ CAR T cells, 7 $\times$ $10^5$ CAR T cells, 8 $\times$ $10^5$ CAR T cells, 9 $\times$ $10^5$ CAR T cells, 1 $\times$ $10^6$ CAR T cells, 2 $\times$ $10^6$ CAR T cells, 3 $\times$ $10^6$ CART cells, 4 $\times$ $10^6$ CAR T cells, 5 $\times$ $10^6$ CART cells, 6 $\times$ $10^6$ CAR T cells, 7 $\times$ $10^6$ CAR T cells, 8 $\times$ $10^6$ CART cells, 9 $\times$ $10^6$ CART cells, 1 $\times$ $10^7$ CART cells, 2 $\times$ $10^7$ CAR T cells, 3 $\times$ $10^7$ CAR T cells, 4 $\times$ $10^7$ CART cells, 5 $\times$ $10^7$ CAR T cells, 6 $\times$ $10^7$ CART cells, 7 $\times$ $10^7$ CAR T cells, 8 $\times$ $10^7$ CAR T cells, 9 $\times$ $10^7$ CART cells, 1 $\times$ $10^8$ CART cells, 2 $\times$ $10^8$ CAR T cells, 3 $\times$ $10^8$ CAR T cells, 4 $\times$ $10^8$ CAR T cells, 5 $\times$ $10^8$ CART cells, 6 $\times$ $10^8$ CART cells, 7 $\times$ $10^8$ CAR T cells, 8 $\times$ $10^8$ CAR T cells, 9 $\times$ $10^8$ CAR T cells, 1 $\times$ $10^9$ CART cells, 2 $\times$ $10^9$ CART cells, 3 $\times$ $10^9$ CAR T cells, 4 $\times$ $10^9$ CAR T cells, 5 $\times$ $10^9$ CAR T cells, 6 $\times$ $10^9$ CAR T cells, 7 $\times$ $10^9$ CAR T cells, 8 $\times$ $10^9$ CAR T cells, 9 $\times$ $10^9$ CAR T cells, or 1 $\times$ $10^{10}$ CAR T cells, or more).

In some embodiments, the method produces from about 1 $\times$ $10^6$ CAR T cells to about 1 $\times$ $10^9$ CAR T cells (e.g., from about 1 $\times$ $10^6$ CAR T cells, 2 $\times$ $10^6$ CAR T cells, 3 $\times$ $10^6$ CAR T cells, 4 $\times$ $10^6$ CAR T cells, 5 $\times$ $10^6$ CAR T cells, 6 $\times$ $10^6$ CAR T cells, 7 $\times$ $10^6$ CAR T cells, 8 $\times$ $10^6$ CAR T cells, 9 $\times$ $10^6$ CAR T cells, 1 $\times$ $10^7$ CAR T cells, 2 $\times$ $10^7$ CAR T cells, 3 $\times$ $10^7$ CAR T cells, 4 $\times$ $10^7$ CAR T cells, 5 $\times$ $10^7$ CAR T cells, 6 $\times$ $10^7$ CAR T cells, 7 $\times$ $10^7$ CAR T cells, 8 $\times$ $10^7$ CAR T cells, 9 $\times$ $10^7$ CAR T cells, 1 $\times$ $10^8$ CAR T cells, 2 $\times$ $10^8$ CAR T cells, 3 $\times$ $10^8$ CAR T cells, 4 $\times$ $10^8$ CAR T cells, 5 $\times$ $10^8$ CAR T cells, 6 $\times$ $10^8$ CAR T cells, 7 $\times$ $10^8$ CART cells, 8 $\times$ $10^8$ CAR T cells, 9 $\times$ $10^8$ CART cells, 1 $\times$ $10^9$ CART cells, or more).

In another aspect, the disclosure features a CAR T cell produced by the method of the preceding aspect of the disclosure, or of any embodiments thereof.

In another aspect, the disclosure features a pharmaceutical composition comprising (i) one or more CAR T cells produced by the method of any of the above aspects or embodiments of the disclosure, or progeny thereof, and (ii) one or more carriers, diluents, and/or excipients.

In another aspect, the disclosure features a method of treating cancer in an individual in need thereof, the method comprising administering to the individual the CAR T cell of the preceding aspect, or progeny thereof, or the pharmaceutical composition recited above.

In some embodiments, the cancer is a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy.

In some embodiments, the cancer is a lung cancer, a kidney cancer, a bladder cancer, or a breast cancer.

In some embodiments, the lung cancer is a non-small cell lung cancer (NSCLC).

In some embodiments, the kidney cancer is a renal cell carcinoma (RCC).

In some embodiments, the bladder cancer is an urothelial carcinoma (UC).

In some embodiments, the breast cancer is a triple negative breast cancer (TNBC).

In some embodiments, the patient is further administered a PD-L1 axis binding antagonist.

In some embodiments, the PD-L1 axis binding antagonist is selected from the group consisting of a PD-L1 binding antagonist, a PD-1 binding antagonist, and a PD-L2 binding antagonist.
In some embodiments, the PD-L1 axis binding antagonist is a PD-L1 binding antagonist.
In some embodiments, the PD-L1 binding antagonist inhibits the binding of PD-L1 to one or more of its ligand binding partners.
In some embodiments, the PD-L1 binding antagonist inhibits the binding of PD-L1 to PD-1.
In some embodiments, the PD-L1 binding antagonist inhibits the binding of PD-L1 to B7-1.
In some embodiments, the PD-L1 binding antagonist inhibits the binding of PD-L1 to both PD-1 and B7-1.
In some embodiments, the PD-L1 binding antagonist is an antibody or antigen-binding fragment thereof.
In some embodiments, the antibody is selected from the group consisting of atezolizumab (MPDL3280A), YW243.55.S70, MDX-1105, MEDI4736 (durvalumab), and MSB0010718C (avelumab).
In some embodiments, the antibody comprises a heavy chain comprising HVR-H1 sequence of SEQ ID NO: 41, HVR-H2 sequence of SEQ ID NO: 42, and HVR-H3 sequence of SEQ ID NO: 43; and a light chain comprising HVR-L1 sequence of SEQ ID NO: 44, HVR-L2 sequence of SEQ ID NO: 45, and HVR-L3 sequence of SEQ ID NO: 46.
In some embodiments, the antibody comprises a heavy chain variable region comprising the amino acid sequence of SEQ ID NO: 47 and a light chain variable region comprising the amino acid sequence of SEQ ID NO: 48.
In some embodiments, the PD-L1 axis binding antagonist is a PD-1 binding antagonist.
In some embodiments, the PD-1 binding antagonist inhibits the binding of PD-1 to one or more of its ligand binding partners.
In some embodiments, the PD-1 binding antagonist inhibits the binding of PD-1 to PD-L1.
In some embodiments, the PD-1 binding antagonist inhibits the binding of PD-1 to PD-L2.
In some embodiments, the PD-1 binding antagonist inhibits the binding of PD-1 to both PD-L1 and PD-L2.
In some embodiments, the PD-1 binding antagonist is an antibody or antigen-binding fragment thereof.
In some embodiments, the antibody is selected from the group consisting of: MDX-1106 (nivolumab), MK-3475 (pembrolizumab), MEDI-0680 (AMP-514), PDR001, REGN2810, and BGB-108.
In some embodiments, the PD-1 binding antagonist is an Fc-fusion protein.
In some embodiments, the Fc-fusion protein is AMP-224.
In another aspect, the disclosure features a method of identifying an individual having a cancer who may benefit from a treatment comprising a PD-L1 axis binding antagonist, the method comprising determining whether the individual has clonally expanded T cells in a sample of peripheral blood from the individual, wherein clonally expanded T cells in the sample identify the individual as one who may benefit from a treatment comprising a PD-L1 axis binding antagonist.
In another aspect, the disclosure features a method of selecting a therapy for an individual having a cancer, the method comprising determining whether the individual has clonally expanded T cells in a sample of peripheral blood from the individual, wherein clonally expanded T cells in the sample identify the individual as one who may benefit from a treatment comprising a PD-L1 axis binding antagonist.
In some embodiments, the sample comprises clonally expanded T cells and the method further comprises administering to the individual an effective amount of a PD-L1 axis binding antagonist.
In another aspect, the disclosure features a method of treating an individual having a cancer, the method comprising:

(a) determining that the individual has clonally expanded T cells in a sample of peripheral blood from the individual; and
(b) administering an effective amount of a PD-L1 axis binding antagonist to the individual.

In another aspect, the disclosure features a method of treating cancer in an individual that has been determined to have clonally expanded T cells in a sample of peripheral blood from the individual, the method comprising administering to the individual an effective amount of a PD-L1 axis binding antagonist.
In another aspect, the disclosure features a method of inducing expansion of a population of T cells in an individual having a cancer, the method comprising:

(a) determining that the individual has clonally expanded T cells in a sample of peripheral blood from the individual; and
(b) administering an effective amount of a PD-L1 axis binding antagonist to the individual.

In another aspect, the disclosure features a method of inducing expansion of a population of T cells in an individual having a cancer and that has been determined to have clonally expanded T cells in a sample of peripheral blood from the individual, the method comprising administering to the individual an effective amount of a PD-L1 axis binding antagonist.
In some embodiments, the clonally expanded T cells are CD8+ T cells.
In some embodiments, the CD8+ T cells are CTL.

In some embodiments, the clonally expanded T cells express TCRs comprising alpha and beta chains each comprising CDRs CDR-1, CDR-2, and CDR-3, and wherein the nucleic acid sequences encoding corresponding alpha chain CDR-1, CDR-2, or CDR-3, and/or beta chain CDR-1, CDR-2, or CDR-3 are at least 85% identical to one another.

In some embodiments, the nucleic acid sequences encoding corresponding alpha chain CDR1 are at least 85% identical to one another.

In some embodiments, the nucleic acid sequences encoding corresponding alpha chain CDR1 are at least 90% identical to one another.

In some embodiments, the nucleic acid sequences encoding corresponding alpha chain CDR1 are at least 95% identical to one another.

In some embodiments, the nucleic acid sequences encoding corresponding alpha chain CDR1 are 100% identical to one another.

In some embodiments, the nucleic acid sequences encoding corresponding alpha chain CDR2 are at least 85% identical to one another.

In some embodiments, the nucleic acid sequences encoding corresponding alpha chain CDR2 are at least 90% identical to one another.

In some embodiments, the nucleic acid sequences encoding corresponding alpha chain CDR2 are at least 95% identical to one another.

In some embodiments, the nucleic acid sequences encoding corresponding alpha chain CDR2 are 100% identical to one another.

In some embodiments, the nucleic acid sequences encoding corresponding alpha chain CDR3 are at least 85% identical to one another.

In some embodiments, the nucleic acid sequences encoding corresponding alpha chain CDR3 are at least 90% identical to one another.

In some embodiments, the nucleic acid sequences encoding corresponding alpha chain CDR3 are at least 95% identical to one another.

In some embodiments, the nucleic acid sequences encoding corresponding alpha chain CDR3 are 100% identical to one another.

In some embodiments, the nucleic acid sequences encoding corresponding beta chain CDR1 are at least 85% identical to one another.

In some embodiments, the nucleic acid sequences encoding corresponding beta chain CDR1 are at least 90% identical to one another.

In some embodiments, the nucleic acid sequences encoding corresponding beta chain CDR1 are at least 95% identical to one another.

In some embodiments, the nucleic acid sequences encoding corresponding beta chain CDR1 are 100% identical to one another.

In some embodiments, the nucleic acid sequences encoding corresponding beta chain CDR2 are at least 85% identical to one another.

In some embodiments, the nucleic acid sequences encoding corresponding beta chain CDR2 are at least 90% identical to one another.

In some embodiments, the nucleic acid sequences encoding corresponding beta chain CRD2 are at least 95% identical to one another.

In some embodiments, the nucleic acid sequences encoding corresponding beta chain CDR2 are 100% identical to one another.

In some embodiments, the nucleic acid sequences encoding corresponding beta chain CDR3 are at least 85% identical to one another.

In some embodiments, the nucleic acid sequences encoding corresponding beta chain CDR3 are at least 90% identical to one another.

In some embodiments, the nucleic acid sequences encoding corresponding beta chain CDR3 are at least 95% identical to one another.

In some embodiments, the nucleic acid sequences encoding corresponding beta chain CDR3 are 100% identical to one another.

In some embodiments, the clonally expanded T cells express TCRs comprising alpha and/or beta chains having nucleic acid sequences that are al least 85% identical to one another.

In some embodiments, the clonally expanded T cells express TCRs comprising alpha chains having nucleic acid sequences that are at least 85% identical to one another.

In some embodiments, the clonally expanded T cells express TCRs comprising alpha chains having nucleic acid sequences that are at least 90% identical to one another.

In some embodiments, the clonally expanded T cells express TCRs comprising alpha chains having nucleic acid sequences that are at least 95% identical to one another.

In some embodiments, the clonally expanded T cells express TCRs comprising alpha chains having nucleic acid sequences that are 100% identical to one another.

In some embodiments, the clonally expanded T cells express TCRs comprising beta chains having nucleic acid sequences that are at least 85% identical to one another.

In some embodiments, the clonally expanded T cells express TCRs comprising beta chains having nucleic acid sequences that are at least 90% identical to one another.

In some embodiments, the clonally expanded T cells express TCRs comprising beta chains having nucleic acid sequences that are at least 95% identical to one another.

In some embodiments, the clonally expanded T cells express TCRs comprising beta chains having nucleic acid sequences that are 100% identical to one another.

In some embodiments, the clonally expanded T cells comprise at least 0.01 % of all T cells in the sample.

In some embodiments, the clonally expanded T cells comprise at least 0.1% of all T cells in the sample.

In some embodiments, the clonally expanded T cells comprise at least 1% of all T cells in the sample.

In some embodiments, the clonally expanded T cells comprise at least 2% of all T cells in the sample.

In some embodiments, the clonally expanded T cells comprise at least 3% of all T cells in the sample.

In some embodiments, the clonally expanded T cells comprise at least 4% of all T cells in the sample.

In some embodiments, the clonally expanded T cells comprise at least 5% of all T cells in the sample.

In some embodiments, the clonally expanded T cells comprise at least 10% of all T cells in the sample.

In some embodiments, the clonally expanded T cells comprise at least 15% of all T cells in the sample.

In some embodiments, the clonally expanded T cells comprise at least 20% of all T cells in the sample.

In some embodiments, the clonally expanded T cells comprise at least 25% of all T cells in the sample.

In another aspect, the disclosure features a method of identifying an individual having a cancer who may benefit from a treatment comprising a PD-L1 axis binding antagonist, the method comprising sequencing of T cell receptors or determining the expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual, wherein an immune-score expression level of the one or more genes that is above a reference immune-score expression level of the one or more genes identifies the individual as one who may benefit from a treatment comprising a PD-L1 axis binding antagonist.

In another aspect, the disclosure features a method of selecting a therapy for an individual having a cancer, the method comprising sequencing of T cell receptors or determining the expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual, wherein an immune-score expression level of the one or more genes in the sample that is above a reference immune-score expression level of the one or more genes identifies the individual as one who may benefit from a treatment comprising a PD-L1 axis binding antagonist.

In some embodiments, the immune-score expression level of the one or more genes in the sample is above the reference immune-score expression level and the method further comprises administering to the individual an effective amount of a PD-L1 axis binding antagonist.

In some embodiments, the reference immune-score expression level is an immune-score expression level of the one or more genes in a reference population.

In another aspect, the disclosure features a method of treating an individual having a cancer, the method comprising:

(a) determining the expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3. S100A4, CYBA, and COX1 in a sample of peripheral blood from the individual, wherein an immune-score expression level of the one or more genes in the sample is determined to be above a reference immune-score expression level of the one or more genes; and

(b) administering an effective amount of a PD-L1 axis binding antagonist to the individual.

In another aspect, the disclosure features a method of treating cancer in an individual that has been determined to have an immune-score expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual that is above a reference immune-score expression level of the one or more genes, the method comprising administering to the individual an effective amount of a PD-L1 axis binding antagonist.

In another aspect, the disclosure features a method of inducing expansion of a population of T cells in an individual having a cancer, the method comprising:

(a) determining the expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual, wherein an immune-score expression level of the one or more genes in the sample is determined to be above a reference immune-score expression level of the one or more genes; and
(b) administering an effective amount of a PD-L1 axis binding antagonist to the individual.

In another aspect, the disclosure features a method of inducing expansion of a population of T cells in an individual having a cancer and that has been determined to have an immune-score expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual that is above a reference immune-score expression level of the one or more genes, the method comprising administering to the individual an effective amount of a PD-L1 axis binding antagonist.

In some embodiments, the T cells are CD8+ T cells.

In some embodiments, the CD8+ cells are cytotoxic T cells (CTL) or effector memory T cells (Tern).

In some embodiments, the reference immune-score expression level is an immune-score expression level of the one or more genes in a reference population.

In some embodiments, the reference population is a population of individuals having the cancer.

In some embodiments, the population of individuals comprises a first subset of individuals who have been treated with a PD-L1 axis binding antagonist and a second subset of individuals who have been treated with therapy that does not comprise a PD-L1 axis binding antagonist.

In some embodiments, the reference immune-score expression level significantly separates each of the first and second subsets of individuals based on a significant difference between an individual's responsiveness to treatment with the PD-L1 axis binding antagonist and an individual's responsiveness to treatment with the therapy that does not comprise a PD-L1 axis binding antagonist above the reference immune-score expression level, wherein the individual's responsiveness to treatment with the PD-L1 axis binding antagonist is significantly improved relative to the individual's responsiveness to treatment with the therapy that does not comprise a PD-L1 axis binding antagonist.

In some embodiments, the reference immune-score expression level significantly separates each of the first and second subsets of individuals based on a significant difference between an individual's responsiveness to treatment with the PD-L1 axis binding antagonist and an individual's responsiveness to treatment with the therapy that does not comprise a PD-L1 axis binding antagonist below the reference immune-score expression level, wherein the individual's responsiveness to treatment with the therapy that does not comprise a PD-L1 axis binding antagonist is significantly improved relative to the individual's responsiveness to treatment with the PD-L1 axis binding antagonist.

In some embodiments, the therapy that does not comprise a PD-L1 axis binding antagonist comprises an anti-neoplastic agent, a chemotherapeutic agent, a growth inhibitory agent, an anti-angiogenic agent, a radiation therapy, a cytotoxic agent, or a combination thereof.

In some embodiments, the therapy that does not comprise a PD-L1 axis binding antagonist comprises a chemotherapeutic agent.

In some embodiments, the chemotherapeutic agent is docetaxel.

In some embodiments, responsiveness to treatment comprises an increase in progression-free survival (PFS).

In some embodiments, responsiveness to treatment comprises an increase in overall survival (OS).

In some embodiments, the reference immune-score expression level is a median of the expression level of each of the one or more genes in the reference population.

In some embodiments, the immune-score expression level of the one or more genes in the reference population is a median of a normalized expression level of each of the one or more genes in the reference population.

In some embodiments, the reference immune-score expression level is an average of the expression level of each of the one or more genes in the reference population.

In some embodiments, the average of the expression level of each of the one or more genes in the reference population is an average of a normalized expression level of each of the one or more genes in the reference population.

In some embodiments, the normalized expression level of each of the one or more genes is the expression level of each of the one or more genes normalized to a reference gene.

In some embodiments, the reference gene is CD8A.

In some embodiments, the reference immune-score expression level is a pre-assigned expression level of the one or more genes.

In some embodiments, benefit from the treatment comprising a PD-L1 axis binding antagonist comprises an increase in OS.

In some embodiments, benefit from the treatment comprising a PD-L1 axis binding antagonist comprises an increase in PFS.

In some embodiments, benefit from the treatment comprising a PD-L1 axis binding antagonist comprises an increase in OS and PFS.

In some embodiments, the genes comprise two or more of NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1.

In some embodiments, the genes comprise three or more of NKG7, GNLY, PRF1, GZMH, GPR56, IFITM2, IFITM1, GZMB, TMSB10, PLAC8, LITAF, CD247, CST7, COX2, KLRD1, CLIC3, S100A-4, CYBA, and COX1.

In some embodiments, the genes comprise four or more of NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1.

In some embodiments, the expression level is a nucleic acid expression level.

In some embodiments, the nucleic acid expression level is an mRNA expression level.

In some embodiments, the mRNA expression level is determined by RNA-seq, RT-qPCR, qPCR, multiplex qPCR or RT-qPCR, microarray analysis, SAGE, MassARRAY technique, FISH, or a combination thereof.

In some embodiments, the mRNA expression level is detected using RNA-seq.

In some embodiments, the mRNA expression level is detected using RT-qPCR.

In some embodiments, the cancer is a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy.

In some embodiments, the cancer is a lung cancer, a kidney cancer, a bladder cancer, or a breast cancer.

In some embodiments, the lung cancer is a non-small cell lung cancer (NSCLC).

In some embodiments, the kidney cancer is a renal cell carcinoma (RCC).

In some embodiments, the bladder cancer is an urothelial carcinoma (UC).

In some embodiments, the breast cancer is a triple negative breast cancer (TNBC).

In some embodiments, the PD-L1 axis binding antagonist is selected from the group consisting of a PD-L1 binding antagonist, a PD-1 binding antagonist, and a PD-L2 binding antagonist.

In some embodiments, the PD-L1 axis binding antagonist is a PD-L1 binding antagonist.

In some embodiments, the PD-L1 binding antagonist inhibits the binding of PD-L1 to one or more of its ligand binding partners.

In some embodiments, the PD-L1 binding antagonist inhibits the binding of PD-L1 to PD-1.

In some embodiments, the PD-L1 binding antagonist inhibits the binding of PD-L1 to B7-1.

In some embodiments, the PD-L1 binding antagonist inhibits the binding of PD-L1 to both PD-1 and B7-1.

In some embodiments, the PD-L1 binding antagonist is an antibody or antigen-binding fragment thereof.

In some embodiments, the antibody is selected from the group consisting of atezolizumab (MPDL3280A), YW243.55.S70, MDX-1105, MEDI4736 (durvalumab), and MSB0010718C (avelumab).

In some embodiments, the antibody comprises a heavy chain comprising HVR-H1 sequence of SEQ ID NO: 41, HVR-H2 sequence of SEQ ID NO: 42, and HVR-H3 sequence of SEQ ID NO: 43; and a light chain comprising HVR-L1 sequence of SEQ ID NO: 44, HVR-L2 sequence of SEQ ID NO: 45, and HVR-L3 sequence of SEQ ID NO: 46.

In some embodiments, the antibody comprises a heavy chain variable region comprising the amino acid sequence of SEQ ID NO: 47 and a light chain variable region comprising the amino acid sequence of SEQ ID NO: 48.

In some embodiments, the PD-L1 axis binding antagonist is a PD-1 binding antagonist.

In some embodiments, the PD-1 binding antagonist inhibits the binding of PD-1 to one or more of its ligand binding partners.

In some embodiments, the PD-1 binding antagonist inhibits the binding of PD-1 to PD-L1.

In some embodiments, the PD-1 binding antagonist inhibits the binding of PD-1 to PD-L2.

In some embodiments, the PD-1 binding antagonist inhibits the binding of PD-1 to both PD-L1 and PD-L2.

In some embodiments, the PD-1 binding antagonist is an antibody or antigen-binding fragment thereof.

In some embodiments, the antibody is selected from the group consisting of: MDX-1106 (nivolumab), MK-3475 (pembrolizumab), MEDI-0680 (AMP-514), PDR001, REGN2810, and BGB-108.

In some embodiments, the PD-1 binding antagonist is an Fc-fusion protein.

In some embodiments, the Fc-fusion protein is AMP-224.

In some embodiments, the individual has not been previously treated for the cancer.

In some embodiments, the individual has not been previously administered a PD-L1 axis binding antagonist.

In some embodiments, the individual has previously been treated for the cancer by administration of a platinum-containing chemotherapeutic agent to the individual, and wherein the individual has failed to respond to the chemotherapeutic agent.

In some embodiments, the treatment comprising a PD-L1 axis binding antagonist is a monotherapy.

In some embodiments, the method further comprises administering to the individual an effective amount of one or more additional therapeutic agents.

In some embodiments, the one or more additional therapeutic agents comprise an anti-neoplastic agent, a chemotherapeutic agent, a growth inhibitory agent, an anti-angiogenic agent, a radiation therapy, a cytotoxic agent, an immunomodulatory agent, or a combination thereof.

In some embodiments, the one or more additional therapeutic agents comprise an immunomodulatory agent.

In some embodiments, the immunomodulatory agent is an antibody or antigen-binding fragment thereof.

In some embodiments, the individual is a human.

In another aspect, the disclosure features a method of identifying an individual (e.g., a human) having a cancer who may benefit from a treatment including a PD-L1 axis binding antagonist. The method may include sequencing T cell receptors to measure the extent of clonal expansion or determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 (e.g., determining the expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2) in a sample from the individual, wherein an immune-score expression level of the one or more genes that is above a reference immune-score expression level of the one or more genes identifies the individual as one who may benefit from a treatment including a PD-L1 axis binding antagonist.

In another aspect, the disclosure features a method of selecting a therapy for an individual (e.g., a human) having a cancer. The method may include determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 (e.g., determining the expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2) in a sample from the individual, wherein an immune-score expression level of the one or more genes in the sample that is above a reference immune-score expression level of the one or more genes identifies the individual as one who may benefit from a treatment including a PD-L1 axis binding antagonist.

In some embodiments of either of the above aspects, the immune-score expression level of the one or more genes in the sample is above the reference immune-score expression level. In some embodiments, the method further includes administering to the individual an effective amount of a PD-L1 axis binding antagonist.

In some embodiments, the reference immune-score expression level is an immune-score expression level of the one or more genes in a reference population.

In an additional aspect, the disclosure features a method of treating an individual (e.g., a human) having a cancer. The method may include: (a) determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 (e.g., determining the expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2) in a sample from the individual, wherein an immune-score expression level of the one or more genes in the sample is determined to be above a reference immune-score expression level; and (b) administering an effective amount of a PD-L1 axis binding antagonist to the individual.

In another aspect, the disclosure features a method of treating cancer in an individual (e.g., a human) that has been determined to have an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 (e.g., an individual that has been determined to have an immune-score expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2) in a sample from the individual that is above a reference immune-score expression level of the one or more genes. The method may include administering to the individual an effective amount of a PD-L1 axis binding antagonist.

In an additional aspect, the disclosure features a method of inducing expansion of a population of T cells in an individual (e.g., a human) having a cancer. The method may include: (a) determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 (e.g., determining the expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2) in a sample from the individual, wherein an immune-score expression level of the one or more genes in the sample is determined to be above a reference immune-score expression level; and (b) administering an effective amount of a PD-L1 axis binding antagonist to the individual.

In a further aspect, the disclosure features a method of inducing expansion of a population of T cells in an individual (e.g., a human) having a cancer. The method may include administering to the individual an effective amount of a PD-L1 axis binding antagonist, wherein prior to treatment, the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 in a sample from the individual has been determined and an immune-score expression level of the one or more genes in the sample has been determined to be above a reference immune-score expression level of the one or more genes. For example, prior to treatment, the expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 in a sample from the individual may have been determined, and an immune-score expression level of the one or more genes in the sample may have been determined to be above a reference immune-score expression level of the one or more genes. In some embodiments, the T cells are CD8+ T cells, such as cytotoxic T cells (CTL, also referred to herein as "Tcyt") or effector memory T cells (Tern).

In some embodiments of any of the above aspects, the reference immune-score expression level is an immune-score expression level of the one or more genes in a reference population. In some embodiments, the reference population is a population of individuals having the cancer. In some embodiments, the population of individuals includes a first subset of individuals who have been treated with a PD-L1 axis binding antagonist and a second subset of individuals who have been treated with therapy that does not comprise a PD-L1 axis binding antagonist. The reference immune-score expression level may, for example, significantly separate each of the first and second subsets of individuals based on a significant difference between an individual's responsiveness to treatment with the PD-L1 axis binding antagonist and an individual's responsiveness to treatment with the therapy that does not contain a PD-L1 axis binding antagonist above the reference immune-score expression level. In these instances, the individual's responsiveness to treatment with the PD-L1 axis binding antagonist may be significantly improved relative to the individual's responsiveness to treatment with the therapy that does not comprise a PD-L1 axis binding antagonist.

In some embodiments, the reference immune-score expression level significantly separates each of the first and second subsets of individuals based on a significant difference between an individual's responsiveness to treatment with the PD-L1 axis binding antagonist and an individual's responsiveness to treatment with the therapy that does not comprise a PD-L1 axis binding antagonist below the reference immune-score expression level. In these instances, the individual's responsiveness to treatment with the therapy that does not contain a PD-L1 axis binding antagonist is significantly improved relative to the individual's responsiveness to treatment with the PD-L1 axis binding antagonist.

In some embodiments, the therapy that does not contain a PD-L1 axis binding antagonist contains an anti-neoplastic agent, a chemotherapeutic agent, a growth inhibitory agent, an anti-angiogenic agent, a radiation therapy, a cytotoxic agent, or a combination thereof. For example, the therapy that does not contain a PD-L1 axis binding antagonist contains a chemotherapeutic agent, such as docetaxel.

In some embodiments, responsiveness to treatment includes an increase in progression-free survival (PFS). In some embodiments, responsiveness to treatment includes an increase in overall survival (OS).

In some embodiments, the reference immune-score expression level is a median of the expression level of each of the one or more genes in the reference population. For example, the immune-score expression level of the one or more genes in the reference population may be a median of a normalized expression level of each of the one or more genes in the reference population.

In some embodiments, the reference immune-score expression level is an average of the expression level of each of the one or more genes in the reference population. For example, the immune-score expression level of the one or more genes in the reference population may be an average of a normalized expression level of each of the one or more genes in the reference population. In some embodiments, the normalized expression level of each of the one or more genes is the expression level of each of the one or more genes normalized to a reference gene, such as CD8A.

In some embodiments, the reference immune-score expression level is a pre-assigned expression level of the one or more genes.

In some embodiments, benefit from the treatment including a PD-L1 axis binding antagonist includes an increase in OS. In some embodiments, benefit from the treatment including a PD-L1 axis binding antagonist includes an increase in PFS. In some embodiments, benefit from treatment including a PD-L1 axis binding antagonist includes an increase is OS and PFS.

In some embodiments, the one or more genes include more than one of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. For example, in some embodiments, the one or more genes include two or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include three or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include four or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include five or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include six or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include seven or more CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include eight or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include nine or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. in some embodiments, the one or more genes include ten or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 11 or more of CST7, NKG7, GZMH, MT-ND4, HLA-H, CCL5, CD8A, CMC1, CD8B, HCST, MT-CYB, MT ND4L KLRG1 , MT-CO2, MT ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 12 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 13 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 14 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 15 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 16 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 17 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 18 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 19 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 20 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 21 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 22 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 23 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 24 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 25 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 26 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 27 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 28 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 29 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 30 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 31 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 32 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include all 33 of CST7, NKG7, GZMH, MT-ND4, HLA-H, CCL5, CD8A, CMC1, CD8B, HCST, MT-CYB, MT-ND4L,

KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

In some embodiments, the one or more genes include one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. For example, the one or more genes may include 1, 2, 3, 4, 5, 6, 7 8, 9, 10, 11, 12, 13, 14, 15, 16, 17, 18, 19, or all 20 of CST7 NKG7, GZMH, MT-ND4, HLA-H CCL5, CD8A, CMC1, CD8B, HCST, MT-CYB, MT-ND4L, KLRG1, MT-CO2, MT-ATP6, PLEK, CTSW, HLA-C, LYAR, and LITAF.

in some embodiments, the one or more genes include 2 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 3 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 4 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 5 or more of CST7, NKG7, GZMH, MT-ND4, HLA-H,

CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 6 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 7 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 8 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 9 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 10 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 11 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 12 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 13 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 14 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 15 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 16 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 17 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 18 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 19 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include all 20 of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes consist of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

In some embodiments, the one or more genes include a two-gene panel set forth in Table 1, below.

**Table 1: Exemplary 2-gene panels for use in conjunction with the compositions and methods described herein**

| | |
|---|---|
| CST7 and NKG7 | NKG7 and MT-ND4 |
| CST7 and GZMH | NKG7 and HLA-H |
| CST7 and MT-ND4 | GZMH and MT-ND4 |
| CST7 and HLA-H | GZMH and HLA-H |
| NKG7 and GZMH | MT-ND4 and HLA-H |

In some embodiments, the one or more genes include a three-gene panel set forth in Table 2, below.

**Table 2: Exemplary 3-gene panels for use in conjunction with the compositions and methods described herein**

| | |
|---|---|
| CST7, NKG7, and GZMH | CST7, MT-ND4, and HLA-H |
| CST7, NKG7 and MT-ND4 | NKG7, GZMH, and MT-ND4 |
| CST7, NKG7, and HLA-H | NKG7, GZMH, and HLA-H |
| CST7, GZMH, and MT-ND4 | NKG7, MT-ND4, and HLA-H |
| CST7, GZMH, and HLA-H | GZMH, MT-ND4, and HLA-H |

In some embodiments, the one or more genes include a four-gene panel set forth in Table 3, below.

**Table 3: Exemplary 4-gene panels for use in conjunction with the compositions and methods described herein**

| | |
|---|---|
| CST7, NKG7, GZMH, and MT-ND4 | CST7, NKG7, MT-ND4, and HLA-H |
| CST7, NKG7, GZMH, and HLA-H | CST7, GZMH, MT-ND4, and HLA-H |
| NKG7, GZMH, MT-ND4, and HLA-H | |

In some embodiments, the one or more genes include a pane! of CST7, NKG7, GZMH, MT-ND4, and HLA-H.

In some embodiments, the one or more genes include one or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. For example, the one or more genes may include 1, 2, 3, 4, 5, 6, 7, 8, 9, 10, 11, 12, 13, 14, 15, 16, 17, 18, 19, or all 20 of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

In some embodiments, the one or more genes include 2 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 3 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 4 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 5 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 6 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 7 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 8 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 9 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 10 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 11 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 12 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 13 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 14 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 15 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 16 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 17 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 18 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 19 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include all 20 of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes consist of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

In some embodiments, the one or more genes include one or more cytotoxic genes, such as one or more of CST7, PLEK, HCST, CTSW, KLRG1, CD8A, CCL5, CD8B, NKG7, GZMH, and HLA-C. In some embodiments, the one or more genes include two or more of CST7, PLEK, HCST, CTSW, KLRG1, CD8A, CCL5, CD8B, NKG7, GZMH, and HLA-C. In some embodiments, the one or more genes include three or more of CST7, PLEK, HCST, CTSW, KLRG1, CD8A, CCL5, CD8B, NKG7, GZMH, and HLA-C. In some embodiments, the one or more genes include four or more of CST7, PLEK, HCST, CTSW, KLRG1, CD8A, CCL5, CD8B, NKG7, GZMH, and HLA-C. In some embodiments, the one or more genes include five or more of CST7, PLEK, HCST, CTSW, KLRG1, CD8A, CCL5, CD8B, NKG7, GZMH, and HLA-C. In some embodiments, the one or more genes include six or more of CST7, PLEK, HCST, CTSW, KLRG1, CD8A, CCL5, CD8B, NKG7, GZMH, and HLA-C. In some embodiments, the one or more genes include seven or more of CST7, PLEK, HCST, CTSW, KLRG1, CD8A, CCL5, CD8B, NKG7, GZMH, and HLA-C. In some embodiments, the one or more genes include eight or more of CST7, PLEK, HCST, CTSW, KLRG1, CD8A, CCL5, CD8B, NKG7, GZMH, and HLA-C. In some embodiments, the one or more genes include nine or more of CST7, PLEK, HCST, CTSW, KLRG1, CD8A, CCL5, CD8B, NKG7, GZMH, and HLA-C. In some embodiments, the one or more genes include ten or more of CST7, PLEK, HCST, CTSW, KLRG1, CD8A, CCL5, CD8B, NKG7, GZMH, and HLA-C. In some embodiments, the one or more genes include all 11 of CST7, PLEK, HCST, CTSW, KLRG1, CD8A, CCL5, CD8B, NKG7, GZMH, and HLA-C. In some embodiments, the one or more genes consist of CST7, PLEK, HCST, CTSW, KLRG1, CD8A, CCL5, CD8B, NKG7, GZMH, and HLA-C.

In some embodiments, the one or more genes include one or more mitochondrial genes, such as one or more of MT-ND4, MT-CYB, MT-CO2, MT-ND4L, and MT-ATP6. In some embodiments, the one or more genes include two or more of MT-ND4, MT-CYB, MT-CO2, MT-ND4L, and MT-ATP6. In some embodiments, the one or more genes include three or more of MT-ND4, MT-CYB, MT-CO2, MT-ND4L, and MT-ATP6. In some embodiments, the one or more genes include four or more of MT-ND4, MT-CYB, MT-CO2, MT-ND4L, and MT-ATP6. In some embodiments, the one or more genes include all five of MT-ND4 MT-CYB MT-CO2, MT-ND4L, and MT-ATP6. In some embodiments, theone or more genes consist of MT-ND4, MT-CYB, MT-CO2, MT-ND4L, and MT-ATP6.

In some embodiments, the one or more genes include one or more cytotoxic genes (e.g., one or more of CST7, PLEK, HCST, CTSW, KLRG1, CD8A, CCL5, CD8B, NKG7, GZMH, and HLA-C) and one or more mitochondrial genes (e.g., one or more of MT-ND4, MT-CYB, MT-CO2, MT-ND4L, and MT-ATP6). For example, in some embodiments, the one or more genes include a cytotoxic gene and one or more mitochondrial genes. In some embodiments, the one or more genes include CST7 and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include PLEK and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include HCST and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include CTSW and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include KLRG1 and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include CD8A and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include CCL5 and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include CD8B and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include NKG7 and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include GZMH and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include HLA-C and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6).

In some embodiments, the one or more genes include a mitochondrial gene and one or more cytotoxic genes. For example, in some embodiments, the one or more genes include MT-ND4 and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C). In some embodiments, the one or more genes include MT-CYB and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C). In some embodiments, the one or more genes include MT-CO2 and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C). In some embodiments, the one or more genes include MT-ND4L and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C). In some embodiments, the one or more genes include MT-ATP6 and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C).

In some embodiments, the one or more genes include a combination of cytotoxic and mitochondrial genes set forth in Table 4, below.

**Table 4: Exemplary panels of cytotoxic and mitochondrial genes for use in conjunction with the compositions and methods described herein**

| Cytotoxic Gene | Mitochondrial Gene |
|---|---|
| CST7 | MT-ND4 |
| CST7 | MT-CYB |
| CST7 | MT-CO2 |
| CST7 | MT-ND4L |
| CST7 | MT-ATP6 |
| PLEK | MT-ND4 |
| PLEK | MT-CYB |
| PLEK | MT-CO2 |
| PLEK | MT-ND4L |
| PLEK | MT-ATP6 |
| HCST | MT-ND4 |
| HCST | MT-CYB |
| HCST | MT-CO2 |
| HCST | MT-ND4L |
| HCST | MT-ATP6 |
| CTSW | MT-ND4 |
| CTSW | MT-CYB |
| CTSW | MT-CO2 |
| CTSW | MT-ND4L |
| CTSW | MT-ATP6 |
| KLRG1 | MT-ND4 |
| KLRG1 | MT-CYB |
| KLRG1 | MT-CO2 |
| KLRG1 | MT-ND4L |
| KLRG1 | MT-ATP6 |
| CD8A | MT-ND4 |
| CD8A | MT-CYB |
| CD8A | MT-CO2 |

(continued)

| Cytotoxic Gene | Mitochondrial Gene |
|---|---|
| CD8A | MT-ND4L |
| CD8A | MT-ATP6 |
| CCL5 | MT-ND4 |
| CCL5 | MT-CYB |
| CCL5 | MT-CO2 |
| CCL5 | MT-ND4L |
| CCL5 | MT-ATP6 |
| CD8B | MT-ND4 |
| CD8B | MT-CYB |
| CD8B | MT-CO2 |
| CD8B | MT-ND4L |
| CD8B | MT-ATP6 |
| NKG7 | MT-ND4 |
| NKG7 | MT-CYB |
| NKG7 | MT-CO2 |
| NKG7 | MT-ND4L |
| NKG7 | MT-ATP6 |
| GZMH | MT-ND4 |
| GZMH | MT-CYB |
| GZMH | MT-CO2 |
| GZMH | MT-ND4L |
| GZMH | MT-ATP6 |
| HLA-C | MT-ND4 |
| HLA-C | MT-CYB |
| HLA-C | MT-CO2 |
| HLA-C | MT-ND4L |
| HLA-C | MT-ATP6 |

In some embodiments, the one or more genes include one or more of NKG7, CST7, GZMH, LITAF, CTSW, CCL5, and CD8A. For example, the one or more genes may include 1, 2, 3, 4, 5, 6, or all 7 of NKG7, CST7, GZMH, LITAF, CTSW, CCL5, and CD8A.

In some embodiments, the one or more genes include 2 or more of NKG7, CST7, GZMH, LITAF, CTSW, CCL5, and CD8A. In some embodiments, the one or more genes include 3 or more of NKG7, CST7, GZMH, LITAF, CTSW, CCL5, and CD8A. In some embodiments, the one or more genes include 4 or more of NKG7, CST7, GZMH, LITAF, CTSW, CCL5, and CD8A. In some embodiments, the one or more genes include 5 or more of NKG7, CST7, GZMH, LITAF, CTSW, CCL5, and CD8A. In some embodiments, the one or more genes include 6 or more of NKG7, CST7, GZMH, LITAF, CTSW, CCL5, and CD8A. In some embodiments, the one or more genes include all 7 of NKG7, CST7, GZMH, LITAF, CTSW, CCL5, and CD8A. In some embodiments, the one or more genes consist of NKG7, CST7, GZMH, LITAF, CTSW, CCL5, and CD8A.

In some embodiments, the expression level is a nucleic acid expression level, such as an mRNA expression level. The mRNA expression level may be determine, for example, by RNA-seq, RT-qPCR, qPCR, multiplex qPCR or RT-qPCR, microarray analysis, SAGE, MassARRAY technique, FISH, or a combination thereof.

In some embodiments, the expression level is detected in tumor cells, tumor-infiltrating immune cells, stromal cells, normal adjacent tissue (NAT) cells, or a combination thereof. In some embodiments, the sample is a tissue sample, a cell sample, a whole blood sample, a plasma sample, a serum sample, or a combination thereof. The tissue sample may be, for example, a tumor tissue sample, such as a tumor tissue sample containing tumor cells, tumor-infiltrating immune cells, stromal cells, NAT cells, or a combination thereof. In some embodiments, the tumor tissue sample is a formalin-fixed and paraffinembedded (FFPE) sample, an archival sample, a fresh sample, or a frozen sample. In some embodiments, the tumor tissue sample is FFPE sample.

In some embodiments, the cancer is a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy. For example, the cancer may be a lung cancer, a kidney cancer, a bladder cancer, or a breast cancer. In some embodiments, the lung cancer is a non-small cell lung cancer (NSCLC). In some embodiments, the kidney cancer is a renal cell carcinoma (RCC). In some embodiments, the bladder cancer is an urothelial carcinoma (UC). In some embodiments, the breast cancer is a triple negative breast cancer (TNBC).

In some embodiments, the PD-L1 axis binding antagonist is a PD-L1 binding antagonist, a PD-1 binding antagonist, or a PD-L2 binding antagonist. In some embodiments, the PD-L1 axis binding antagonist is a PD-L1 binding antagonist. The PD-L1 binding antagonist may inhibit the binding of PD-L1 to one or more of its ligand binding partners. In some embodiments, the PD-L1 binding antagonist inhibits the binding of PD-L1 to PD-1. In some embodiments, the PD-L1 binding antagonist inhibits the binding of PD-L1 to B7-1. In some embodiments, the PD-L1 binding antagonist inhibits the binding of PD-L1 to both PD-1 and B7-1.

In some embodiments, the PD-L1 binding antagonist is an antibody or antigen-binding fragment thereof. The antibody may be, for example, atezolizumab (MPDL3280A), YW243.55.S70, MDX-1105, MEDI4736 (durvalumab), and MSB0010718C (avelumab). In some embodiments, the PD-L1 binding antagonist is atezolizumab (MPDL3280A).

In some embodiments, the antibody contains a heavy chain including HVR-H1 sequence of SEQ ID NO: 41, HVR-H2 sequence of SEQ ID NO: 42, and HVR-H3 sequence of SEQ ID NO: 43; and a light chain including HVR-L1 sequence of SEQ ID NO: 44, HVR-L2 sequence of SEQ ID NO: 45, and HVR-L3 sequence of SEQ ID NO: 46.

In some embodiments, the antibody contains a heavy chain variable region including the amino acid sequence of SEQ ID NO: 47 and a light chain variable region including the amino acid sequence of SEQ ID NO: 48.

In some embodiments, the PD-L1 axis binding antagonist is a PD-1 binding antagonist. The PD1 binding antagonist may, for example, inhibit the binding of PD-1 to one or more of its ligand binding partners. In some embodiments, the PD-1 binding antagonist inhibits the binding of PD-1 to PD-L1. In some embodiments, the PD-1 binding antagonist inhibits the binding of PD-1 to PD-L2. In some embodiments, the PD-1 binding antagonist inhibits the binding of PD-1 to both PD-L1 and PD-L2.

In some embodiments, the PD-1 binding antagonist is an antibody or antigen-binding fragment thereof. The antibody may be, for example, MDX-1106 (nivolumab), MK-3475 (pembrolizumab), MEDI-0680 (AMP-514), PDR001, REGN2810, and BGB-108.

In some embodiments, the PD-1 binding antagonist is an Fc-fusion protein, such as AMP-224.

In some embodiments, the individual has not been previously treated for the cancer. In some embodiments, the individual has not been previously administered a PD-L1 axis binding antagonist.

In some embodiments, the individual has previously been treated for the cancer by administration of a platinum-containing chemotherapeutic agent to the individual. In some embodiments, the individual has failed to respond to the chemotherapeutic agent.

In some embodiments, the treatment including a PD-L1 axis binding antagonist is a monotherapy.

In some embodiments, the method further includes administering to the individual an effective amount of one or more additional therapeutic agents. The one or more additional therapeutic agents may include, for example, an anti-neoplastic agent, a chemotherapeutic agent, a growth inhibitory agent, an anti-angiogenic agent, a radiation therapy, a cytotoxic agent, an immunomodulatory agent, or a combination thereof. In some embodiments, the one or more additional therapeutic agents includes an immunomodulatory agent. The immunomodulatory agent may be, for example, an antibody or antigen-binding fragment thereof. Exemplary immunomodulatory agents that may be used in conjunction with the compositions and methods of the disclosure include anti-TIGIT antibodies and antigen-binding fragments thereof, anti-CTLA-4 antibodies or antigen-binding fragments thereof, anti-CD27 antibodies or antigen-binding fragments thereof, anti-CD30 antibodies or antigen-binding fragments thereof, anti-CD40 antibodies or antigen-binding fragments thereof, anti-4-1BB antibodies or antigen-binding fragments thereof, anti-GITR antibodies or antigen-binding fragments thereof, anti-OX40 antibodies or antigen-binding fragments thereof, anti-TRAILR1 antibodies or antigen-binding fragments thereof, anti-TRAILR2 antibodies or antigen-binding fragments thereof, anti-TWEAK antibodies or antigen-binding fragments thereof, anti-TWEAKR antibodies or antigen-binding fragments thereof, anti-BRAF antibodies or antigen-binding fragments thereof, anti-MEK antibodies or antigen-binding fragments thereof, anti-CD33 antibodies or antigen-binding fragments thereof, anti-CD20 antibodies or antigen-binding fragments thereof, anti-CD52 antibodies or antigen-binding fragments thereof, anti-A33 antibodies or antigen-binding fragments thereof, anti-GD3 antibodies or antigen-binding fragments thereof, anti-PSMA antibodies or antigen-binding fragments thereof, anti-Ceacan 1 antibodies or antigen-binding fragments thereof, anti-Galedin 9 antibodies or antigen-binding fragments thereof, anti-HVEM antibodies or antigen-binding fragments thereof, anti-VISTA antibodies or antigen-binding fragments thereof, anti-B7 H4 antibodies or antigen-binding fragments thereof, anti-HHLA2 antibodies or antigen-binding fragments thereof, anti-CD155 antibodies or antigen-binding fragments thereof, anti-CD80 antibodies or antigen-binding fragments thereof, anti-BTLA antibodies or antigen-binding fragments thereof, anti-CD160 antibodies or antigen-binding fragments thereof, anti-CD28 antibodies or antigen-binding fragments thereof, anti-CD226 antibodies or antigen-binding fragments thereof, anti-CEACAM1 antibodies or antigen-binding fragments thereof, anti-TIM3 antibodies or antigen-binding fragments thereof, anti-CD96 antibodies or antigen-binding fragments thereof, anti-CD70 antibodies or antigen-binding fragments thereof, anti-CD27 antibodies or antigen-binding fragments thereof, anti-LIGHT antibodies or antigen-binding fragments thereof, anti-CD137 antibodies or antigen-binding fragments thereof, anti-DR4 antibodies or antigen-binding fragments thereof, anti-CR5 antibodies or antigen-binding fragments thereof, anti-FAS antibodies or antigen-binding fragments thereof, anti-CD95 antibodies or antigen-binding fragments thereof, anti-TRAIL antibodies or antigen-binding fragments thereof, anti-DR6 antibodies or antigen-binding fragments thereof, anti-EDAR antibodies or antigen-binding fragments thereof, anti-NGFR antibodies or antigen-binding fragments thereof, anti-OPG antibodies or antigen-binding fragments thereof, anti-RANKL antibodies or antigen-binding fragments thereof, anti-LTβR antibodies or antigen-binding fragments thereof, anti-BCMA antibodies or antigen-binding fragments thereof, anti-TACI antibodies or antigen-binding fragments thereof, anti-BAFFR antibodies or antigen-binding fragments thereof, anti-EDAR2 antibodies or antigen-binding fragments thereof, anti-TROY antibodies or antigen-binding fragments thereof, and anti-RELT antibodies or antigen-binding fragments thereof.

In an additional aspect, the disclosure features a kit for identifying an individual (e.g., a human) having a cancer who may benefit from a treatment including a PD-L1 axis binding antagonist. The kit may contain one or more reagents for determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 in a sample from the individual (e.g., one or more reagents for determining the expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 in a sample from the individual). In some embodiments, the kit further contains instructions for using the one or more reagents to identify an individual (e.g., a human) having a cancer who may benefit from a treatment including a PD-L1 axis binding antagonist according to the method of any of the above aspects or embodiments of the disclosure.

In a further aspect, the disclosure features an assay for identifying an individual (e.g., a human) having a cancer who is a candidate for a treatment that contains a PD-L1 axis binding antagonist. The assay may include determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 in a sample from the individual (e.g., determining the expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2 in a sample from the individual). An immune-score expression level of the one or more genes in the sample that is above a reference immune-score expression level of the one or more genes identifies the individual as one who may benefit from the treatment including a PD-L1 axis binding antagonist.

In some embodiments of the foregoing aspect, the reference immune-score expression level is an immune-score expression level of the one or more genes in a reference population. In some embodiments, the reference population is a population of individuals having the cancer. In some embodiments, the population of individuals includes a first subset of individuals who have been treated with a PD-L1 axis binding antagonist and a second subset of individuals who have been treated with therapy that does not comprise a PD-L1 axis binding antagonist. The reference immune-score expression level may, for example, significantly separate each of the first and second subsets of individuals based on a significant difference between an individual's responsiveness to treatment with the PD-L1 axis binding antagonist and an individual's responsiveness to treatment with the therapy that does not contain a PD-L1 axis binding antagonist above the reference immune-score expression level. In these instances, the individual's responsiveness to treatment with the PD-L1 axis binding antagonist may be significantly improved relative to the individual's responsiveness to treatment with the therapy that does not comprise a PD-L1 axis binding antagonist.

In some embodiments, the reference immune-score expression level significantly separates each of the first and second subsets of individuals based on a significant difference between an individual's responsiveness to treatment with the PD-L1 axis binding antagonist and an individual's responsiveness to treatment with the therapy that does not comprise a PD-L1 axis binding antagonist below the reference immune-score expression level. In these instances, the individual's responsiveness to treatment with the therapy that does not contain a PD-L1 axis binding antagonist is significantly improved relative to the individual's responsiveness to treatment with the PD-L1 axis binding antagonist.

In some embodiments, the therapy that does not contain a PD-L1 axis binding antagonist contains an anti-neoplastic agent, a chemotherapeutic agent, a growth inhibitory agent, an anti-angiogenic agent, a radiation therapy, a cytotoxic agent, or a combination thereof. For example, the therapy that does not contain a PD-L1 axis binding antagonist contains a chemotherapeutic agent, such as docetaxel.

In some embodiments, responsiveness to treatment includes an increase in progression-free survival (PFS). In some embodiments, responsiveness to treatment includes an increase in overall survival (OS).

In some embodiments, the reference immune-score expression level is a median of the expression level of each of the one or more genes in the reference population. For example, the immune-score expression level of the one or more genes in the reference population may be a median of a normalized expression level of each of the one or more genes in the reference population.

In some embodiments, the reference immune-score expression level is an average of the expression level of each of the one or more genes in the reference population. For example, the immune-score expression level of the one or more genes in the reference population may be an average of a normalized expression level of each of the one or more genes in the reference population. In some embodiments, the normalized expression level of each of the one or more genes is the expression level of each of the one or more genes normalized to a reference gene, such as CD8A.

In some embodiments, the reference immune-score expression level is a pre-assigned expression level of the one or more genes.

In some embodiments, benefit from the treatment including a PD-L1 axis binding antagonist includes an increase in OS. In some embodiments, benefit from the treatment including a PD-L1 axis binding antagonist includes an increase in PFS. In some embodiments, benefit from treatment including a PD-L1 axis binding antagonist includes an increase is OS and PFS.

In some embodiments of either of the foregoing two aspects, the one or more genes include more than one of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. For example, in some embodiments, the one or more genes include two or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include three or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include four or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include five or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include six or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include seven or more CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include eight or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include nine or more of CST7, NKG7, GZMH MT-ND4,HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include ten or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRG1, KLRK1, B2M, GZMA, ID2, CX3CR1, PRSS23, GNLY, PRF1, and PATL2. In some embodiments, the one or more genes include 11 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 12 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 13 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 14 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 15 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 16 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 17 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 18 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 19 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 20 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 21 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 22 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 23 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 24 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 25 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 26 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 27 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 28 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 29 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 30 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 31 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include 32 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2. In some embodiments, the one or more genes include all 33 of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

In some embodiments of either of the foregoing two aspect, the one or more genes include one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. For example, the one or more genes may include 1, 2, 3, 4, 5, 6, 7, 8, 9, 10, 11, 12, 13, 14, 15, 16, 17, 18, 19, or all 20 of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

For example, in some embodiments of either of the foregoing two aspects, the one or more genes include 2 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 3 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 4 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 5 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 6 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 7 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 8 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 9 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 10 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 11 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 12 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 13 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 14 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 15 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and L!TAF. In some embodiments, the one or more genes include 16 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 17 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 18 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include 19 or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes include all 20 of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF. In some embodiments, the one or more genes consist of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

In some embodiments of either of the foregoing two aspects, the one or more genes include a panel of genes set forth in any one of Tables 1-3, above. In some embodiments, the one or more genes include a panel of CST7, NKG7, GZMH, MT-ND4, and HLA-H.

In some embodiments of either of the foregoing two aspects, the one or more genes include one or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. For example, the one or more genes may include 1, 2, 3, 4, 5, 6, 7, 8, 9, 10, 11, 12, 13, 14, 15, 16, 17, 18, 19, or all 20 of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

For example, in some embodiments of either of the foregoing two aspects, the one or more genes include 2 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 3 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 4 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 5 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 6 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. in some embodiments, the one or more genes include 7 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. in some embodiments, the one or more genes include 8 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 9 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 10 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 11 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 12 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 13 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 14 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 15 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 16 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 17 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 18 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include 19 or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes include all 20 of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A. In some embodiments, the one or more genes consist of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

In some embodiments of either of the foregoing two aspects, the one or more genes include one or more cytotoxic genes, such as one or more of CST7, PLEK, HCST, CTSW, KLRG1, CD8A, CCL5, CD8B, NKG7, GZMH, and HLA-C. In some embodiments, the one or more genes include two or more of CST7, PLEK, HCST, CTSW, KLRG1, CD8A, CCL5, CD8B, NKG7, GZMH, and HLA-C. In some embodiments, the one or more genes include three or more of CST7, PLEK, HCST, CTSW, KLRG1, CD8A, CCL5, CD8B, NKG7, GZMH, and HLA-C. In some embodiments, the one or more genes include four or more of CST7, PLEK, HCST, CTSW, KLRG1, CD8A, CCL5, CD8B, NKG7, GZMH, and HLA-C. In some embodiments, the one or more genes include five or more of CST7, PLEK, HCST, CTSW, KLRG1, CD8A, CCL5, CD8B, NKG7, GZMH, and HLA-C. In some embodiments, the one or more genes include six or more of CST7, PLEK, HCST, CTSW, KLRG1, CD8A, CCL5, CD8B, NKG7, GZMH, and HLA-C. In some embodiments, the one or more genes include seven or more of CST7, PLEK, HCST, CTSW, KLRG1, CD8A, CCL5, CD8B, NKG7, GZMH, and HLA-C. In some embodiments, the one or more genes include eight or more of CST7, PLEK, HCST, CTSW, KLRG1, CD8A, CCL5, CD8B, NKG7, GZMH, and HLA-C. In some embodiments, the one or more genes include nine or more of CST7, PLEK, HCST, CTSW, KLRG1, CD8A, CCL5, CD8B, NKG7, GZMH, and HLA-C. In some embodiments, the one or more genes include ten or more of CST7, PLEK, HCST, CTSW, KLRG1, CD8A, CCL5, CD8B, NKG7, GZMH, and HLA-C. In some embodiments, the one or more genes include all 11 of CST7, PLEK, HCST, CTSW, KLRG1, CD8A, CCL5, CD8B, NKG7, GZMH, and HLA-C. In some embodiments, the one or more genes consist of CST7, PLEK, HCST, CTSW, KLRG1, CD8A, CCL5, CD8B, NKG7, GZMH, and HLA-C.

In some embodiments of either of the foregoing two aspects, the one or more genes include one or more mitochondrial genes, such as one or more of MT-ND4, MT-CYB, MT-CO2, MT-ND4L, and MT-ATP6. In some embodiments, the one or more genes include two or more of MT-ND4, MT-CYB, MT-CO2, MT-ND4L, and MT-ATP6. In some embodiments, the one or more genes include three or more of MT-ND4, MT-CYB, MT-CO2, MT-ND4L, and MT-ATP6. In some embodiments, the one or more genes include four or more of MT-ND4, MT-CYB, MT-CO2, MT-ND4L, and MT-ATP6. In some embodiments, the one or more genes include all five of MT-ND4, MT-CYB, MT-CO2, MT-ND4L, and MT-ATP6. In some embodiments, the one or more genes consist of MT-ND4, MT-CYB, MT-CO2, MT-ND4L, and MT-ATP6.

In some embodiments of either of the foregoing two aspects, the one or more genes include one or more cytotoxic genes (e.g., one or more of CST7, PLEK, HCST, CTSW, KLRG1, CD8A, CCL5, CD8B, NKG7, GZMH, and HLA-C) and one or more mitochondrial genes (e.g., one or more of MT-ND4, MT-CYB, MT-CO2, MT-ND4L, and MT-ATP6). For example, in some embodiments, the one or more genes include a cytotoxic gene and one or more mitochondrial genes. In some embodiments, the one or more genes include CST7 and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include PLEK and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include HCST and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include CTSW and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include KLRG1 and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include CD8A and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include CCL5 and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include CD8B and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include NKG7 and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include GZMH and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB,

MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6). In some embodiments, the one or more genes include HLA-C and one or more of MT-ND4, MT-CYB, MT-CO2, MT-ND4L, and MT-ATP6 (e.g., any one of MT-ND4, MT-CYB, MT-CO2, MT-ND4L, and MT-ATP6, two or more of MT-ND4, MT-CYB, MT-CO2, MT-ND4L, and MT-ATP6, three or more of MT-ND4, MT-CYB, MT-CO2, MT-ND4L, and MT-ATP6, four or more of MT-ND4, MT-CYB, MT-CO2, MT-ND4L, and MT-ATP6, or all five of MT-ND4, MT-CYB, MT-CO2, MT-ND4L, and MT-ATP6).

In some embodiments of either of the foregoing two aspects, the one or more genes include a mitochondrial gene and one or more cytotoxic genes. For example, in some embodiments, the one or more genes include MT-ND4 and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C). In some embodiments, the one or more genes include MT-CYB and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C). In some embodiments, the one or more genes include MT-CO2 and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C). In some embodiments, the one or more genes include MT-ND4L and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C). In some embodiments, the one or more genes include MT-ATP6 and one or more of CST7, PLEK, HCST, CTSW, KLRG1, CD8A, CCL5, CD8B, NKG7, GZMH, and HLA-C (e.g., any one of CST7, PLEK, HCST, CTSW, KLRG1, CD8A, CCL5, CD8B, NKG7, GZMH, and HLA-C, two or more of CST7, PLEK, HCST, CTSW, KLRG1, CD8A, CCL5, CD8B, NKG7, GZMH, and HLA-C, three or more of CST7, PLEK, HCST, CTSW, KLRG1, CD8A, CCL5, CD8B, NKG7, GZMH, and HLA-C, four or more of CST7, PLEK, HCST, CTSW, KLRG1, CD8A, CCL5, CD8B, NKG7, GZMH, and HLA-C, five or more of CST7, PLEK, HCST, CTSW, KLRG1, CD8A, CCL5, CD8B, NKG7, GZMH, and HLA-C, six or more of CST7, PLEK, HCST, CTSW, KLRG1, CD8A, CCL5, CD8B, NKG7, GZMH, and HLA-C, seven or more of CST7, PLEK, HCST, CTSW, KLRG1, CD8A, CCL5, CD8B, NKG7, GZMH, and HLA-C, eight or more of CST7, PLEK, HCST, CTSW, KLRG1, CD8A, CCL5, CD8B, NKG7, GZMH, and HLA-C, nine or more of CST7, PLEK, HCST, CTSW, KLRG1, CD8A, CCL5, CD8B, NKG7, GZMH, and HLA-C, ten or more of CST7, PLEK, HCST, CTSW, KLRG1, CD8A, CCL5, CD8B, NKG7, GZMH, and HLA-C, or all 11 of CST7, PLEK, HCST, CTSW, KLRG1, CD8A, CCL5, CD8B, NKG7, GZMH, and HLA-C).

In some embodiments of either of the foregoing two aspects, the one or more genes include a combination of cytotoxic and mitochondrial genes set forth in Table 4, above.

In some embodiments of either of the foregoing two aspects, the one or more genes include one or more of NKG7, CST7, GZMH, LITAF, CTSW, CCL5, and CD8A. For example, the one or more genes may include 1, 2, 3, 4, 5, 6, or all 7 of NKG7, CST7, GZMH, LITAF, CTSW, CCL5, and CD8A.

For example, in some embodiments of either of the foregoing two aspects, the one or more genes include 2 or more of NKG7, CST7, GZMH, LITAF, CTSW, CCL5, and CD8A. In some embodiments, the one or more genes include 3 or more of NKG7, CST7, GZMH, LITAF, CTSW, CCL5, and CD8A. In some embodiments, the one or more genes include 4 or more of NKG7, CST7, GZMH, LITAF, CTSW, CCL5, and CD8A. In some embodiments, the one or more genes include 5 or more of NKG7, CST7, GZMH, LITAF, CTSW, CCL5, and CD8A. In some embodiments, the one or more genes include 6 or more of NKG7, CST7, GZMH, LITAF, CTSW, CCL5, and CD8A. In some embodiments, the one or more genes include all 7 of NKG7, CST7, GZMH, LITAF, CTSW, CCL5, and CD8A. In some embodiments, the one or more genes consist of NKG7, CST7, GZMH, LITAF, CTSW, CCL5, and CD8A.

In some embodiments of either of the foregoing two aspects, the expression level is a nucleic acid expression level, such as an mRNA expression level. The mRNA expression level may be determine, for example, by RNA-seq, RT-qPCR, qPCR, multiplex qPCR or RT-qPCR, microarray analysis, SAGE, MassARRAY technique, FISH, or a combination thereof.

In some embodiments of either of the foregoing two aspects, the expression level is detected in tumor cells, tumor-infiltrating immune cells, stromal cells, normal adjacent tissue (NAT) cells, or a combination thereof. In some embodiments, the sample is a tissue sample, a cell sample, a whole blood sample, a plasma sample, a serum sample, or a combination thereof. The tissue sample may be, for example, a tumor tissue sample, such as a tumor tissue sample containing tumor cells, tumor-infiltrating immune cells, stromal cells, NAT cells, or a combination thereof. In some embodiments, the tumor tissue sample is a formalin-fixed and paraffin-embedded (FFPE) sample, an archival sample, a fresh sample, or a frozen sample. In some embodiments, the tumor tissue sample is FFPE sample.

In some embodiments of either of the foregoing two aspects, the cancer is a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy. For example, the cancer may be a lung cancer, a kidney cancer, a bladder cancer, or a breast cancer. In some embodiments, the lung cancer is a non-small cell lung cancer (NSCLC). In some embodiments, the kidney cancer is a renal cell carcinoma (RCC). In some embodiments, the bladder cancer is an urothelial carcinoma (UC). In some embodiments, the breast cancer is a triple negative breast cancer (TNBC).

In some embodiments of either of the foregoing two aspects, the PD-L1 axis binding antagonist is a PD-L1 binding antagonist, a PD-1 binding antagonist, or a PD-L2 binding antagonist. In some embodiments, the PD-L1 axis binding antagonist is a PD-L1 binding antagonist. The PD-L1 binding antagonist may inhibit the binding of PD-L1 to one or more of its ligand binding partners. In some embodiments, the PD-L1 binding antagonist inhibits the binding of PD-L1 to PD-1. In some embodiments, the PD-L1 binding antagonist inhibits the binding of PD-L1 to B7-1. In some embodiments, the PD-L1 binding antagonist inhibits the binding of PD-L1 to both PD-1 and B7-1.

In some embodiments, the PD-L1 binding antagonist is an antibody or antigen-binding fragment thereof. The antibody may be, for example, atezolizumab (MPDL3280A), YW243.55.S70, MDX-1105, MEDI4736 (durvalumab), and MSB0010718C (avelumab). In some embodiments, the PD-L1 binding antagonist is atezolizumab (MPDL3280A).

In some embodiments, the antibody contains a heavy chain including HVR-H1 sequence of SEQ ID NO: 41, HVR-H2 sequence of SEQ ID NO: 42, and HVR-H3 sequence of SEQ ID NO: 43; and a light chain including HVR-L1 sequence of SEQ ID NO: 44, HVR-L2 sequence of SEQ ID NO: 45, and HVR-L3 sequence of SEQ ID NO: 46.

In some embodiments, the antibody contains a heavy chain variable region including the amino acid sequence of SEQ ID NO: 47 and a light chain variable region including the amino acid sequence of SEQ ID NO: 48

In some embodiments of either of the foregoing two aspects, the PD-L1 axis binding antagonist is a PD-1 binding antagonist. The PD-1 binding antagonist may, for example, inhibit the binding of PD-1 to one or more of its ligand binding partners. In some embodiments, the PD-1 binding antagonist inhibits the binding of PD-1 to PD-L1. In some embodiments, the PD-1 binding antagonist inhibits the binding of PD-1 to PD-L2. In some embodiments, the PD-1 binding antagonist inhibits the binding of PD-1 to both PD-L1 and PD-L2.

In some embodiments, the PD-1 binding antagonist is an antibody or antigen-binding fragment thereof. The antibody may be, for example, MDX-1106 (nivolumab), MK-3475 (pembrolizumab), MEDI-0680 (AMP-514), PDR001, REGN2810, and BGB-108.

In some embodiments, the PD-1 binding antagonist is an Fc-fusion protein, such as AMP-224.

## BRIEF DESCRIPTION OF THE DRAWINGS

**FIG. 1A** is a chart showing statistics for the dataset of six samples described in Example 1 after bioinformatics filtering. The samples come from the tumor (T) and adjacent normal (N) compartments of the three patients described in Example 1, indicated by the labels T1 through N3. Total counts are given for the numbers of sequenced transcripts from RNA sequencing (column labeled "Transcripts"), cells with gene expression data from RNA sequencing ("Cells"), and cells with clonotype assignments from TCR sequencing ("Typed"). In the rightmost column, Venn diagrams show the numbers of distinct clonotypes that were unique to, and shared between, the tumor and NAT compartments of each patient.

**FIG. 1B** is a graph showing the fraction of TCR clonotypes in samples obtained from the patients described in Example 1 by clonal residency pattern. Fractions are normalized separately for each patient (abbreviated "Pat") 1 through 3. Each fraction is represented by the length of a bar region, which is stacked from left to right and shaded according to the legend at the top, indicating the fraction of sequenced TCR clonotypes with a given observed cell count (singleton or multiplet) and location of its cells, with "NAT" and "Tumor" indicating exclusive residency of all cells in those respective compartments, and "Dual-resident" indicating cells found in both compartments.

**FIG. 1C** is a scatterplot of T cell clones by clonal residency pattern. Each symbol represents a distinct clonotype, with a different symbol used for each patient. The x- and y- coordinates of each clonotype are plotted on a logarithmic scale corresponding to its observed clone counts (number of cells with the given clonotype) in NAT and tumor, respectively, and random jitter added to show the number of dots at each discrete coordinate. Different positions on the graph are shaded using a 2-dimensional palette based on NAT and tumor clone counts shown in the upper right. The rounded rectangles and the polygon indicate groupings of clones based on the count and location of their cells as NAT multiplets, tumor multiplets, and dual-resident clones. Labels and arrows point to clones that are NAT or tumor singletons.

**FIG. 1D** is a barplot showing the fraction of cells by clonal residency pattern. The barplot follows the same format as for FIG. 1A, except that fractions are based on cells, rather than distinct clonotypes. Therefore, expanded clones with multiple cells comprise proportionately larger fractions. Cells from dual-resident clones can be further distinguished by their location in NAT or tumor, giving rise to two separate categories.

**FIG. 2A** is a chart showing the association of clonal residency patterns with gene expression. Cells from each clonal residency pattern are plotted separately on a common tSNE map, computed from the gene expression data for all T cells in the dataset. The tSNE map represents each T cell as a dot, and places cells with similar gene expression close to one another, and cells with different gene expression far from one another. Each clonal residency pattern is represented by a separate plot, organized into a 3 x 2 grid based on the location and clone count of the cells. For each of the six plots, all cells from the dataset are plotted on the same background, and cells from the respective clonal residency pattern are shaded using the 2-dimensional palette from FIG. 1C. These plots indicate that clonal residency patterns occupy separate regions of the tSNE map, indicating a possible association with gene expression.

**FIG. 2B** is a series of plots showing the expression of selected marker genes for T cell subsets. Sixteen plots are shown, each one representing the expression of a gene on the same tSNE map described in FIG. 2A. Each cell is shaded according to its expression of the given gene on a continuous scale based on its quantile, after normalization and log transformation by Seurat, where light gray indicates the lowest expression and darker shades indicate higher expression in the dataset. The shaded ovals and black lines on each tSNE map provide uniform landmarks for the T cell subsets and the division of clusters by CD8 and CD4 expression. These landmarks and the T cell subsets indicated on the labels are depicted in more detail in FIG. 2C.

**FIG. 2C** is a plot showing the clustering of T cells by gene expression into subsets. The clustering of cells is indicated on the tSNE map by an arbitrarily assigned shade for each cluster and by shaded ovals that approximate the region of each cluster. Each cluster was named, as indicated by the labels, according to its overall gene expression or characteristic marker genes, also indicated in some labels. A label is shown for each cluster to indicate its name, provide a name for each cluster, based on its gene expression. Additional labels and lines indicate groupings of subclusters for the natural killer T (NKT) cells and T resident memory (Trm) cells. Black lines indicate the division of clusters based on the expression of CD8 or CD4. Some clusters have both CD8+ and CD4+ cells, and are designated CD3+.

**FIG. 2D** is a graph showing the clonal residency patterns for each T cell subset. Each T cell subset from FIG. 2C is characterized by the fraction of its cells belonging to each clonal residency pattern. The fraction of cells is represented by the length of each bar region, shaded according to the legend at the top. T cell subsets are shown to differ in their distribution of clonal residency patterns.

**FIG. 2E** is a plot showing various clonal residency patterns. The same tSNE plot from FIG. 2D is shown, except that each cell is shaded according to its tumor and NAT clone size, using a 2-dimensional palette from FIG. 1C, and also shown here in the upper right. Shaded ovals, black lines, and cluster labels from FIG. 2D are also plotted to provide landmarks.

**FIG. 3A** is a series of scatterplots of clonal count and residency for each T cell cluster described in Example 1. Each plot is a subset of the data in FIG. 1C for each T cell cluster in FIG. 2C. The space of tumor and NAT clone counts is divided into four regions, as indicated by the gray lines: tumor-exclusive, containing clones for which the NAT clone count is zero; NAT-exclusive, where the tumor clone count is zero; tumor-predominant, where the tumor and NAT counts are nonzero and the tumor count is greater than or equal to the NAT count; and NAT-predominant, where the tumor and NAT counts are nonzero and the NAT count is greater than the tumor count. The numbers in each region indicate the p-value from the one-sided Poisson test where the alternative hypothesis is that the observed clone counts for a given T cell subset is greater than expected from the observed mean over all T cell subsets. P-values less than 0.05 are colored in red, and are used to classify the T cell subsets into different clonal expansion behaviors.

**FIG. 3B** is a schematic of clonal expansion behaviors. This plot summarizes the clonal expansion behaviors illustrated in FiG. 3A. The clones are represented by dots using the same convention as for FIG. 1C. Each clonal expansion behavior is represented by a label indicating the T cell subsets involved, and with a shaded region indicating the clones for each behavior. Lines indicate the divisions between different types of clonal expansion: tumor-exclusive, NAT-exclusive, tumor-predominant, and NAT-predominant, as defined in FIG. 3A.

**FIG. 4A** is a plot showing matches of cell TCRs to known viral-reactive CDR3 sequences from VDJdb, as described in Example 1. Each T cell cluster from FIG. 2C is characterized by its fraction of cells having a CDR3 amino acid sequence matching a CDR3 sequence in VDJdb reacting to a known epitope from a commonly occurring virus (cytomegalovirus, Epstein-Barr virus, and influenza A). For each T cell subset, the count of cells is divided by the total number of cells assigned to that T cell subset and having a clonotype. Cells are further divided according to their clonal count and residency, as indicated by a bar region shaded according to the legend on top.

**FIG. 4B** is a plot showing matches of cell TCRs to multi-cancer CDR3 sequences from the Cancer Genome Atlas (TCGA). Matching was performed between the CDR3 amino acid sequences from the dataset obtained in Example 1 and a set of 716,720 TCR sequences assembled from TCGA as described in Li, B. et al. Nat. Genet. 48, 725-732, 2016, the disclosure of which is incorporated herein by reference in its entirety. Matches were counted if the CDR3 amino acid sequence occurred in three or more different tumor types in TCGA, suggesting reactivity to a viral antigen instead of a tumor antigen. The barplot follows the same conventions as for FIG. 4A.

**FIG. 4C** is a heat map showing T cell composition of clonotypes. Each column in this heat map represents a distinct clone among those with at least 10 observed counts overall. Shades within the heat map indicate the proportion of cells for that clone across T cell subtypes. Some T cell subtypes are further divided into their location in NAT (N) or tumor (T) to reveal additional information. Clones are grouped by similarity to one another to illustrate distinct mixtures of T cell clusters, highlighted by rectangles with thick lines and labeled on the bottom with seven observed mixtures of T cell subtypes. Shading on the top of the heat map indicates the clone count and residency of each clonotype, using the scheme of FIG. 1C. Shading of the mixtures on the bottom of the heat map with a corresponding legend shows the clonal expansion behaviors in each mixture, supported by its range of clone counts and residency patterns on the top of the heat map.

**FIG. 4D** is a graph showing association between clonal residency of 8.2-Tem cells and differentiation into 8.3-Trm cells. Each circle represents a highly expanded clonotype containing at least one 8.2-Tem cell, with the x- and y-coordinates corresponding to the counts of the 8.2-Tem cells in NAT and tumor, respectively, and shaded according to the 2-dimensional palette from FIG. 1C. Random jitter has been added to the integer values of the x- and y-coordinates to show multiple occurrences of points with the same counts. The size of each circle indicates the total count of 8.3-Trm cells (8.3a-Trm plus 8.3b-Trm plus 8.3c-Trm) in the clonotype, with the scale given below the plot.

**FIG. 4E** is a graph showing association between clonal residency of 4.3-Activated cells and expansion of 4.1-Trm cells. The same plot as FIG. 4D is shown, except for clones containing at least one 4.3-Activated cell and with the size of the circle indicating the total count of 4.1-Trm cells for each clone.

**FIG. 4F** is a graph showing association between clonal residency of 4.2-Activated cells and counts of 4.4 and 4.5 cells. The same plot as FIG. 4D is shown, except for clones containing at least one 4.3-Activated cell and with the size of the circle indicating the total count of 4.4-HSP, 4.5t-Ribo, and 4.5n-Ribo cells.

**FIG. 4G** is a graph showing mitotic activity in highly expanded clonotypes. Each mixture of T cell subtypes shown at the bottom of FIG. 4C is characterized by the fraction of highly expanded clones with at least one cell assigned to the 3.2-Mitotic cluster in tumor (T), NAT (N), or both.

**FIG. 5A** is a plot showing the fraction of cells across T cell subtypes described in Examples 1 and 2. Each patient (abbreviated "Pat") is characterized by its fraction of cells assigned to the different T cell subtypes in FIG. 2C. The fraction is represented by the length of each bar region, shaded according to the legend at the top. T cell subtypes with the largest fractions are also labeled, space permitting.

**FIG. 5B** is a plot showing gene set enrichment analysis (GSEA) applied to clinical trial data. A signed GSEA analysis was performed on the 1101 most highly variable genes from the Seurat-based analysis described in Example 2. These genes are ranked for each T cell cluster, as indicated by gradients of white to black, with genes with upregulation (fold change > 1) on the left and those with downregulation (fold change < 1) on the right, and ordered so that genes with the smallest (most significant) p-values are at both ends of the ranking. Highly variable genes were also evaluated for positive and negative association with overall survival based upon bulk RNAseq expression from the POPLAR phase II clinical trial of patients with non-small cell lung cancer (NSCLC). For each T cell cluster, genes with positive association to clinical response genes are indicated by red ticks (for atezolizumab response) and blue ticks (for docetaxel response) at the corresponding rank among upregulated genes. Genes with negative association to clinical response are indicated by black ticks at the corresponding rank among downregulated genes. Statistical tests for differences in distributions were performed separately for atezolizumab and for docetaxel using the enrichment score method from the Connectivity Map (Lamb, J. et al. Science 313, 1929-1935, 2006) compared with the distribution from 100,000 random permutations, with results indicated by p-values and as q-values, or the local false discovery rate corrected for multiple hypothesis testing. Sample sizes are given as "n1" for each statistic, indicating the intersecting number of genes associated with clinical response. In all cases, n2=1101 genes associated with cluster membership. T cell clusters with statistically significant results for atezolizumab ($q < 0.05$) are highlighted with gray boxes.

**FIG. 5C** is a plot depicting the ranking of clinically associated genes in each T cell subset. This heat map shows the genes and their ranks in each T cell subset as computed in the gene set enrichment analysis shown in FIG. 5B. These values correspond to the tick marks of FIG. 5B, representing genes that showed statistically significant associations with response to atezolizumab therapy. Each column represents a distinct T cell subtype, and the shade indicates the rank of the gene, where the rank was based on the p-value for differentia! expression of the gene for cells in the T cell subtype relative to all other cells. Cells with gray indicate that the gene did not show a positive log fold change for that T cell subset. The mean rank was computed for each gene among its T cell subsets with a positive log fold change, and genes were sorted from top to bottom by ascending mean rank. The mean ranks are also indicated by the plot to the right of the heat map.

**FIGS. 5D - 5I** are plots showing the association of expansion modes with clinical response in NSCLC. Kaplan-Meier curves are shown for both overall survival (OS) and progression-free survival (PFS) in the POPLAR clinical trial, split by arm (atezolizumab, uppermost hazard ratio and p values, n=93 patients; docetaxel, lowermost hazard ratio and p values, n=100 patients) and signature expression (continuous: high; dashed: low; split at the median) for gene signatures for tumor-resident multiplets (FIGS. 5F and 5G), NAT-resident multiplets (FIGS. 5H and 5I), and dual-resident clones (FIGS. 5D and 5E).

**FIGS. 5J** - **5O** are forest plots depicting the OS (upper panels) and PFS (lower panels) hazard ratios at various cutoffs for dual (FIGS. 5J and 5K), tumor, (FIGS. 5L and 5M) and NAT (FIGS. 5N and 5O) expansion signatures in the single-arm IMvigor210 clinical trial of atezolizumab (n=354 patients) in urothelial carcinoma. Whiskers represent the 95% confidence interval.

**FIG.** 6 is a heat map of genes and their expression in highly expanded clones, grouped according to clonal expansion mode. Each column in this heat map represents the average gene expression of a single clonotype, using highly expanded clones with counts of 10 or more in NAT (NAT-resident multiplets), tumor (tumor-resident multiplets), or both (dual-resident clones). Dual-residency clones are further subdivided into cells located in NAT and those in tumor. Columns for the NAT and tumor singleton modes were constructed by pooling 20 singleton cells at random and computing their mean gene expression. Regions of high gene expression are outlined with rectangles and labeled as categories A through H with brief descriptions.

**FIG. 7** is a heat map showing the cross-correlation of expression levels of genes set forth in Table 7 in Example 2. The heat map illustrates the existence of two subsets of genes within the panel recited in Table 7 that exhibit a particularly high internal correlation. The first subset contains 5 mitochondrial genes: MT-ND4L, MT-ND4, MT-ATP6, MT-CO2, and MT-CYB. The second subset contains 11 cytotoxic genes: PLEK, HCST, CTSW, KLRG1, CD8A, CCL5, CD8B, CST7, NKG7, GZMH, and HLA-C. Correlation coefficients are shown by way of shading on a continuum from 0 to 1.00.

**FIGS. 8A** - **8N** are plots showing the association of expression levels of particular cytotoxic and mitochondrial genes with clinical response in NSCLC. Kaplan-Meier curves are shown for both OS and PFS in the POPLAR clinical trial, split by arm (atezolizumab, uppermost hazard ratio and p values, n=93 patients; docetaxel, lowermost hazard ratio and p values, n=100 patients) and expression level (continuous: high; dashed: low; split at the median) for the following genes: CST7 (FIGS. 8A and 8B), MT-ND4 (FIGS. 8C and 8D), combined expression of CST7 and MT-ND4 (FIGS. 8E and 8F), MT-CYB (FIGS. 8G and 8H), combined expression of CST7 and MT-CYB (FIGS. 8I and 8J), MT-CO2 (FIGS. 8K and 8L), and combined expression of CST7 and MT-CO2 (FIGS. 8M and 8N).

**FIGS. 9A** - **9F** are plots showing the association of expression levels of cytotoxic and mitochondrial gene panels with clinical response in NSCLC. Kaplan-Meier curves are shown for both OS and PFS in the POPLAR clinical trial, split by arm (atezolizumab, uppermost hazard ratio and p values, n=93 patients; docetaxel, lowermost hazard ratio and p values, n=100 patients) and expression level (continuous: high; dashed: low; split at the median) for the following gene panels: cytotoxic genes (PLEK, HCST, CTSW, KLRG1, CD8A, CCL5, CD8B, CST7, NKG7, GZMH, and HLA-C, FIGS. 9A and 9B), mitochondrial genes (MT-ND4L, MT-ND4, MT-ATP6, MT-CO2, and MT-CYB, FIGS. 9C and 9D), and combined expression of cytotoxic genes and mitochondrial genes (PLEK, HCST, CTSW, KLRG1, CD8A, CCL5, CD8B, CST7, NKG7, GZMH, HLA-C, MT-ND4L, MT-ND4, MT-ATP6, MT-CO2, and MT-CYB, FIGS. 9E and 9F).

**FIGS. 10A - 10J** are plots showing clonal count and residency types for T cell clones described in a recent report by Guo, X. et al. Nat. Med. 24, 978 (2018), the disclosure of which is incorporated herein by reference. The clustering analysis described for the three patients analyzed in Example 1, below, was applied to the dataset of Guo et al., which contains various samples from tumor, NAT, and peripheral blood across 14 patients. Each plot represents a T cell subset from the present framework, with the corresponding cluster from the other dataset, as determined by representative genes. The plots are similar to those from FIGS. 1C and 3A, where NAT and tumor clone counts are represented by coordinates on the x- and y-axes, respectively, and shaded according to the 2-dimensional palette shown at the bottom. Peripheral blood clone counts are represented here by the size of each dot, according to each legend.4

**FIGS. 11A - 11D** are plots showing the association of expansion modes with clinical response in NSCLC using the T cell gene signatures obtained from the dataset of Guo, X. et al. Nat. Med. 24, 978 (2018). Kaplan-Meier curves are shown for both overall survival (OS) and progression-free survival (PFS) in the POPLAR clinical trial, split by arm (atezolizumab, uppermost hazard ratio and p values, n=93 patients; docetaxel, lowermost hazard ratio and p values, n=100 patients) and signature expression (continuous: high; dashed: low; split at the median) for gene signatures for dual-resident clones (FIGS. 11A and 11B) and tumor-resident clones (FIGS. 11C and 11D).

**FIGS. 11E** - **11H** are forest plots depicting the OS (upper panels) and PFS (lower panels) hazard ratios at various cutoffs using the T cell gene signatures obtained from the dataset of Guo, X. et al. Nat. Med. 24, 978 (2018). The plots illustrate the threshold-dependent hazard ratios for the dual-resident T cell gene signature (FIGS. 11E and 11F) and the tumor-resident T cell gene signature (FIGS. 11G and 11H) in the single-arm IMvigor210 clinical trial of atezolizumab (n=354 patients) in urothelial carcinoma. Whiskers represent the 95% confidence interval.

FIGS. 12A- 12I are plots showing that dual clonal expansion associates with peripheral clonal expansion. (FIG. 12A) Clonal expansion scatterplots for NAT and tumor compartments. Each patient is represented by a separate scatterplot, with each dot representing a separate clonotype, and the x- and y-coordinates on scales corresponding to log((1+clonotype cell count)/(total cells)) in NAT and tumor compartments, respectively, to allow for the representation of zero counts. These coordinates indicate the relative fraction of cells for that clonotype in the respective compartment. Dots are colored using a 2-dimensional palette in the lower right, where blue shades darken with increasing NAT count, pink with increasing tumor count, and purple with increasing counts in both compartments. Clonotypes with only a single observed clone count, or singletons, are colored with either yellow (NAT clone count of 1) or orange (tumor clone count of 1). Vertical and horizontal gray lines indicate divisions between absence (count of 0 cells for that clonotype) and presence (count of 1 or more cells) within each compartment, which are used in the definitions of clonal expansion patterns. Diagonal gray lines indicate equal cell fractions in the two compartments. The numbers in each title indicate the extent of paraiiei expansion, measured by the Pearson correlation coefficient of the dual-expanded clones on a log-log scale, and patients are ordered by decreasing coefficient. (FIG. 12B) Clonal expansion scatterplots for tumor, NAT, and blood compartments. These plots are similar to those for (FIG. 12A), but provide data on four patients with blood scTCR-seq samples. Green and brown colors are added to indicate blood singletons and multiplets, respectively, although the predominance of blood singletons means few blood multiplets are seen. The number of blood cells observed for each clone is represented by the size of each dot, according to the legend at the bottom. As with (FIG. 12A), numbers in each title indicate the extent of parallel expansion, and patients are ordered by decreasing coefficient. (FIG. 12C) Venn diagrams of cells by compartment. Each patient with a blood sample is represented by a Venn diagram that matches a corresponding scatterplot in (FIG. 12B). Each Venn diagram contains ovals across the middle for the five tissue-based clonal expansion patterns, colored by the legend to the right, further subdivided by large ovals on the top and bottom to indicate clonotypes present in blood, with representation by a single cell in blood (blood non-expanded clones) indicated by the top, dark green oval, and those by multiple cells in blood (blood expanded clones) by the bottom, brown oval. Cells with no representative cells are therefore represented by counts in the middle section without an overlapping large oval. Cells from clonotypes in blood only and not in tumor or NAT are also represented by regions in the large ovals for blood singletons (light green regions) and blood multiplets (light brown regions). Numbers to the right of each Venn diagram indicate the totals for the top and bottom ovals, representing the total number of cells from blood non-expanded and expanded clonotypes. (FIG. 12D) Blood expansion. Each barplot corresponds to the Venn diagram above, showing the fraction of cells in blood non-expanded clonotypes (top ovals) and expanded clonotypes (bottom ovals), as green and brown bars, respectively. Numbers above each bar show the counts from the right of each Venn diagram used to compute the fractions. (FIG. 12E) Infiltration by blood expanded clones into tumor/NAT. Each barplot shows the distribution of blood expanded clonotypes (bottom ovals) into the five tissue-based clonal expansion patterns, plus blood multiplets, colored according to the legend to the right of (FIG. 12C). Numbers above each bar show the counts from the Venn diagrams used to compute the fractions, provided where space permits. (FIG. 12F) Presence of tumor/NAT TCRs in blood expanded clones. Each barplot shows the percentage of cells from each tissue-based clonal expansion pattern that share a TCR with a blood expanded clonotype, or bottom oval in (FIG. 12C). Bars are color-coded per the legend to the right of (FIG. 12C), and also labeled by these abbreviations: n=NAT singletons, N=NAT multiplets, D=dual-expanded, T=tumor multiplets, and t=tumor singletons. Numbers above the dual-expanded pattern of each barplot show the counts from the Venn diagrams used to compute the percentages. (FIG. 12G) Correlation of TCR repertoires between blood, tumor, and NAT. Each dot represents a clone from the four patients with blood samples, plotted on a log-log scale by absolute clone size in blood versus clone size in tumor (left) or NAT (right), and color-coded by clonal expansion pattern using the two-dimensional palette on the right. A value of 1 is added to each clone size to permit representation of zero counts. Gray horizontal and vertical lines separate a count of 0 from a count of 1. (FIG. 12H) Corroboration of clone size correlations by an external dataset. The same plots in (FIG. 12G) are presented using the dataset from Guo et al., 2018. (FIG. 12I) Association between blood expansion and parallel tissue clonal expansion. Each dot represents a patient from the dataset in Guo et al., 2018, summarizing the analysis in FIG. 21. The x-coordinate represents the extent of blood expansion, computed as the fraction of cells in blood expanded versus non-expanded clonotypes. The y-coordinate is the extent of parallel tumor/NAT clonal expansion, measured as the Pearson correlation coefficient of dual-expanded clonotypes, weighted by (1+clone size in blood). Only 10 of the 14 patients in the dataset had sufficient sampling to compute y coordinates, with 2 patients not having sampling in NAT and 2 patients having only a single dual-expanded clone. Statistical testing using Spearman correlation yielded a p-value of 0.04, shown in the lower right corner.

**FIGS. 13A** - **13J** are plots showing that T cell subtypes associate with clonal expansion patterns. (FIG. 13A) Two-dimensional mapping of gene expression. The plot shows each T cell from the dataset plotted according to the first two components, as x- and y- coordinates, respectively, from dimensionality reduction of single-cell gene expression data. A separate colour was chosen for each cluster, and cells are colored according to their cluster assignment. Ovals and labels added manually to approximate cluster regions and to provide biological meaning. A curved line through the middle of the figure indicates a division between CD8+ (left) and CD4+ (right) T cells. (FIG. 13B) Comparison of T cell subtypes with external gene signatures. A normalized cross-tabulation of cells is shown between cluster assignments from the present study in columns and those provided in external gene signatures from Guo et al., 2018 (upper panel) and Yost et al, 2019 bottom panel) in rows. Intensities of the displayed heatmaps indicate the frequency that cells were classified into both sets of clusters. Columns, representing clusters from the present study, are colored according to the scheme in (FIG. 13A). (FIG. 13C) Selected biomarkers. Boxplots display the distributions of expression of selected genes across clusters, taken from the scaled data from the integrated assay computed using Seurat. Boxes indicate the interquartile range and the median value is shown with a line across the middle. Whiskers show an additional 1.5 times the interquartile range from the median, limited by the range of the data. Ranges for each gene were selected to contain the interquartile ranges of all boxplots. Each boxplot is colored according to the scheme in (FIG. 13A). (FIG. 13D) Stem-like versus terminal exhaustion. The upper figure shows the gene expression of PDCD1 (PD-1) overlaid onto the map of (FIG. 13A), using a continuous colour scheme on the quantile of the count from the SCT assay computed using Seurat, as well as the source of the cell: tumor (red), NAT (blue), or blood (green). The bottom figure shows the values of a published gene signature score for stem-like exhaustion versus terminal exhaustion (Im 2015). Cluster ovals from (FIG. 13A) are provided for reference. (FIG. 13E) Tumor and NAT origins. Cells from the tumor and NAT compartments are plotted onto the map from (FIG. 13A) and colored as red and blue, respectively. Cluster ovals from (FIG. 13A) are provided for reference. The overall fraction of tumor cells is given in the legend, and the fraction of tumor cells for each cluster across all 14 patients is provided. (FIG. 13F) Comparison of resident memory T cells. Summary statistics are provided for the 8.3a, b, and c clusters representing resident memory T cells, showing numerical values from (FIG. 13D) and (FIG. 13E) for each cluster. Bars are colored using the scheme in (FIG. 13A). (FIG.13G) Blood expansion. Cells from the blood compartment are plotted onto the from (FIG. 13A) and colored green for blood count of 1, and a gradient of brown for increasing blood counts greater than 1. Cells from the tumor and NAT compartments are not plotted. Fractions are not provided because of the high degree of variability across the four patients, as shown in FIG. 7D. (FIG. 13H) Clonal expansion patterns by cell. Each cell from tumor or NAT is plotted onto the map from (FiG.13A) and colored according to the tissue-based clonal expansion pattern of its parent clone, using the two-dimensional palette on the bottom left. Ovals from (FIG. 13A) are provided for reference. (FIG. 13I) Clonal expansion patterns by subtype. Each dot represents a clone combined from the four patients with blood samples, plotted according to its absolute cell count in NAT and tumor using logarithmic scales. The clone size in blood is indicated by the size of the dot according to the legend at the bottom. Clones were classified according to their primary cell cluster and placed in separate plots, colored using the scheme in (FIG. 13A). Clones from the 8.3a, 8.3b, and 8.3c clusters were combined into a single plot, but colored separately according to their cluster. (FIG. 13J) Clonal expansion patterns by patient. Clones from the four patients with blood samples are shown using the same representation as in FIG. 12B. As in that figure, x- and y-coordinates indicate the cell fractions in NAT and tumor, respectively, and the size of the dot represents its absolute cell count in blood using the legend at the bottom. However, clones are now colored by their primary cell cluster instead of their clonal expansion pattern, using the scheme in (FIG. 13A).

**FIGS. 14A - 14D** are plots showing that non-exhausted CD8+ T cells in tumor accumulate from peripheral blood. (FIG. 14A) TCR representation is correlated between novel post-treatment CD8+ clonotypes and pre-treatment blood. Scatterplots are shown for the 6 patients from Yost et al., 2019 with matching clones in pre- and post-treatment tumor and either a preor post-treatment bulk TCR-seq sample from blood. Novel post-treatment clonotypes with CD8+ subtype as their primary post-treatment cell cluster were identified by their occurrence in post-treatment but not pre-treatment scTCR-seq assays, and plotted according to their clone sizes in pre-treatment blood from bulk TCR-seq (resorting to post-treatment blood for patient bcc.su002) and in post-treatment tumor from scTCR-seq. Clones are colored according to their primary cell cluster in post-treatment tumor as determined in the external analysis. A vertical gray bar separates clones with a clone size of 0 in blood from those with a clone size of 1 or more, thereby separating novel clones into blood-independent clones on the left and blood-associated clones on the right. Patients are ordered according to their total number of cells from novel CD8+ clonotypes sharing TCRs with blood, indicated by the numbers at the bottom. (FIG. 14B) TCR repertoires in blood before and after treatment. Scatterplots are shown for the 6 patients in (FIG. 14A) for their pre- and post-treatment clone sizes in blood. Coordinates represent the number of templates observed for each TCR beta chain. For the patient bcc.su002 without a pre-treatment blood sample, a one-dimensional stripchart is shown instead, with horizontal jitter added to display all points. Numbers below each plot indicate the pre- and post-treatment clonal diversity for each distribution of clone sizes, measured as the Shannon entropy for all clone sizes of 10 or above, with the threshold chosen to reduce the effect of noise from low-level sampling. The increasing amount of clonal diversity can also be observed visually as increasing presence of clones along the main diagonal from the lower left to upper right. (FIG. 14C) Tracking of CD8+ T cell clones in tumor. For each patient in (FIG. 14A), clones were selected for a CD8+ subtype as their primary cell cluster in either pre- or post-treatment tumor. These clones were separated according to whether they shared a TCR in pre-treatment blood (or post-treatment blood for patient bcc.su002, which lacked a pre-treatment blood sample). Blood-associated novel clones, or those sharing TCRs with blood, are plotted in the upper panel with the plots titled "Blood+". Blood-independent novel clones, or those not sharing TCRs with blood) are plotted in the lower panel with "Blood-". Within each plot, clones were further classified according to their pre-treatment ("Pre-rx") or post-treatment ("Post-rx") measurements, and further as Disappearing (present in pre-treatment but not post-treatment scRNA-seq), Remaining (present in both), or Novel (present in post-treatment but not pre-treatment scRNA-seq), abbreviated as "Disapp", "Remain", and "Novel", respectively. The y-coordinates indicate the size of each clone in pre- or post-treatment tumor, and colored according to their pre- or post-treatment cluster assignment, as appropriate, as determined by the original paper. Matching pre- and post-treatment clonotypes in the Remaining category are connected by gray lines. The mean ratio of clone sizes in the post-treatment condition relative to the pre-treatment conditions in the Remaining category is computed for blood-matching and blood-non matching categories and provided as numbers below each pair of plots. (FIG. 14D) Distribution of cell clusters for novel CD8+ clonotypes according to tumor expansion and TCR sharing with blood. Blood-associated and blood-independent novel clones in tumor with a CD8+ clonotype were identified and further separated into non-expanded (tumor clone size = 1) or expanded (tumor clone size > 1) clones, abbreviated in the figure as "Non-exp" and "Exp", respectively. For each category, cells belonging to the clone were tabulated according to their cluster assignment from the original analysis, and the distributions shown in barplots, colored according to the legend at the bottom. A grouping of clusters into exhausted and non-exhausted categories is also provided in the legend.

**FIGS. 15A - 15D** are plots showing clinical associations for CD8+ clusters. (FIG. 15A) Gene expression profiles for CD8+ clusters across patients. The heatmap shows the gene expression profile of each gene (row) in each patient cluster (column) for the CD8+ clusters from the unsupervised analysis of FIG. 13A. Genes for each signature are grouped into rows, selected as the 20 with the highest signed deviance by logistic regression. Genes that were duplicated in more than one signature were assigned to the one with the lower rank. Cells for each patient were grouped by their cluster and the mean log fold change was computed for each group against all other CD8+ clusters for that patient. Each cell in the heatmap indicates the resulting log fold change, colored by the palette at the bottom. (FIG. 15B) Gene expression profiles for CD8+ clusters across clonal expansion patterns. The same representation as for (FIG. 15A), except that cells in each patient were grouped according to their clonal expansion pattern of their parent clone. (FIG. 15C) Forest plots of gene signatures for CD8+ clusters. Each dot represents a gene from the 20-gene signatures for a CD8+ cluster, with the x-coordinate indicating the hazard ratio for progression-free survival (PFS) in each arm of a clinical trial, shown as a separate row. Vertical jitter was added to allow all dots to be visualized. Hazard ratios less than 1 indicate a favorable association with survival. Numbers on the right of each signature indicate the mean hazard ratio (HR) for the genes and the one-sided p-value against the null hypothesis that the hazard ratio is 1 or more, adjusted for multiple hypothesis testing using the Benjamini-Hochberg procedure. Statistical results yielding adjusted p-values < 0.05 are colored in red. (FIG. 15D) Kaplan-Meier survival curves. Kaplan-Meier curves are plotted for the 8.1-Eff (left) and 8.2-Tem (right) signatures, with patients dichotomized by signature scores above (red) and below (black) their respective medians in each trial. Each curve shows progression-free survival (PFS) on the x-axis and the fraction of patients surviving on the y-axis. Plus signs on each survival curve indicate censoring events. The legend for each plot indicates the hazard ratio (HR) and p-value of a Cox proportional hazards model comparing the two patient populations, with p-values < 0.05 labeled with an asterisk.

**FIGS. 16A - 16F** are plots showing sample statistics and clonal expansion patterns. (FIG. 16A) Representation of CD3- and CD45-selected samples across clusters. Because samples of immune cells in the study were selected for either CD3 (expected to contain only T cells) or CD45 (containing both T and non-T cells), it was necessary to separate T and non-T cells computationally. The results of the separation process for T cells should yield clusters containing cells from both CD3- and CD45-selected samples. The barplots show the distribution of samples across the T cell clusters obtained from an unsupervised cluster analysis, and show the expected representation by CD3- and CD45-selected samples, colored by red and blue, respectively. (FIG. 16B) Mixing of T cells across patients. A two-dimensional map of all cells is shown, corresponding to the dimensionality reduction computation for FIG. 13A. Each cell is coloured by the patient of origin. Patients are observed to be well mixed across the map, indicating adequate integration of the individual datasets. (FIG. 16C) Sample statistics. Statistics are provided for the cells in the dataset after separation into T and non-T categories. The presence of CD45-selected samples allowed us to identify contaminating non-T cells in the CD3-selected samples, accounting for the counts of non-T cells in those samples. Patients were obtained from four cancer types: non-small-cell lung carcinoma (Lung), endometrial adenocarcinoma (Endo), colorectal adenocarcinoma (Colon), and renal clear cell carcinoma (Renal). Numbers indicate the total number of transcripts and cells from scRNA-seq, as well as the number of cells with clonotypes from scTCR-seq. Cells were grouped into distinct clonotypes, and the number of distinct clonotypes is shown for each sample. (FIG. 16D) TCR sharing across compartments. Clonotypes were matched across the compartments from each patient. Venn diagrams for each sample are presented adjacent to the corresponding row in (FIG. 16C). Each Venn diagram shows the numbers of distinct clonotypes unique to each compartment or shared across compartments (N=NAT, T=tumor, B=blood). (FIG. 16E) Distribution of clones by clonal expansion patterns. Each clone was classified into a clonal expansion pattern based on its clone size in the tumor and NAT compartments. A NAT singleton was a clone with a NAT cell count (or clone size) of 1 and a tumor clone size of 0, and a NAT multiplet had a NAT clone size of 2 or more and a tumor clone size of 0. Conversely, a tumor singleton was a clone with a tumor clone size of 1 and a NAT clone size of 0, and a tumor multiplet had a tumor clone size of 2 or more and a NAT clone size of 0. A dual-expanded clone had a clone size of 1 or more in both tumor and NAT. Barplots show the distribution of clones by this classification in each patient, and colored according to the legend at the bottom.. Most clonotypes were singletons, but 9-18% of clonotypes in each patient were multiplets or dual-expanded clones. (FIG. 16F) Distribution of cells by clonal expansion patterns. Each T cell in NAT (left) and in tumor (right) was classified according to the clonal expansion pattern of its parent clone. Barplots show the distribution of cells by this classification in each patient, and colored according to the legend at the bottom. Cells from dual-expanded clones represent a large fraction of T cells in tumor and NAT, constituting the majority of T cells in some cases.

**FIG. 17** provides plots showing association of parallel tumor/NAT expansion and peripheral clonal expansion in external dataset. The 14 non-small cell lung carcinoma patients from Guo et al., 2018 are each depicted by a scatterplot showing a dot for each clonotype, plotted by its clone size in NAT and tumor, with the size of the dot representing the clone size in blood according to the legend at the lower right. Colors of each dot are based on the two-dimensional palette at the bottom right. Each patient is also characterized by the extent of blood expansion, infiltration of blood-expanded clones into tumor/NAT, and presence of tumor/NAT TCRs in blood-expanded clones, using the same representation and color scheme as in FIGS. 12D - 12F. Patients are ordered by the amount of blood expansion. A tendency can be seen for dual-expanded clones to be more highly expanded in blood, and for these clones to lie along the main diagonal, reflecting a parallel mode of dual expansion. Patients with higher levels of blood expansion have a tendency for an overall parallel clonal expansion in tumor and NAT, summarized in FIG. 12I.

**FIGS. 18A - 18E** are plots showing T cell subsets and clonal expansion behaviour. (FIG. 18A) Comparison of T cell subtypes with external gene signatures from Zhang et al., 2018. A normalized cross-tabulation of cells is shown between cluster assignments from the study in columns and those provided in external gene signatures from Zhang et al., 2018. Intensities of the displayed heatmaps indicate the frequency that cells were classified into both sets of clusters. Columns, representing clusters from the study, are colored according to the scheme in (FIG. 18A). The computation and representation is the same as for FIG. 13B, except with a different set of reference gene signatures. A relationship can be seen between the 8.1-Eff cluster in the study and the CD8_C3-CXCR1 cluster in the external dataset, and also between 8.2-Tem and CD8-C4-GZMK. (FIG. 18B) Selected gene expression for CD4+ subsets. The expression for selected genes depicts differences across CD4+ subsets. The representation is the same as for FIG. 13C, except that genes are selected for high expression in CD4+ subsets. (FIG. 18C) Exhaustion biomarkers. Gene expression values are plotted onto the map of FIG. 13A for several genes associated with T cell exhaustion. Representation is the same as that for FIG. 13D (upper panel), with colors based on the quantile of the count-based gene expression, as well as the source of the cell: tumor (red), NAT (blue), or blood (green). A gradient can be observed for each gene of increasing expression from the 8.3c to the 8.3b and 8.3a clusters. High expression of ITGAE in cluster 4.1-S100A6 suggests that it represents CD4+ resident memory T cells. (FIG. 18D) Clonal expansion patterns by T cell cluster. Clonal expansion patterns were tabulated for clones and cells of each cluster from the unsupervised analysis of FIG. 13A. The barplot on the left shows the distribution for clones and their primary cluster, while the four barplots in the middle shows the distribution for cells and their parent clone. Barplots for cells are further divided according to the origin in NAT (left of each pair) and tumor (right of each pair). Separate barplots are provided for cells from dual-expanded clones. Bars are colored according to the legend at the bottom. Asterisks next to each bar indicate clonal expansion patterns with statistically significant over-representation relative to the overall distribution, as determined by a Bonferroni-adjusted p-value < 0.01. Asterisks are labeled by column using the following abbreviations: n=NAT singleton, N=NAT multiplet, T=tumor multiplet, t=tumor singleton, D=dual-expanded clone. The four barplots on the right show the distributions of blood non-expanded and expanded cells separately for the four patients of the study with blood samples, with bars colored according to the legend at the bottom. (FIG. 18E) T cell clusters exhibit distinct clonal expansion patterns. Clones are combined from all 14 patients and grouped by their primary cell cluster. Each scatterplot shows the clones plotted by their sizes in NAT and tumor on a linear scale, truncated at 50 on each axis, to display the expansion patterns of the majority of clones, which have relatively low levels of expansion. Dots are colored according to the two-dimensional palette in the upper right.

**FIGS. 19A and 19B** provide plots showing clonal expansion patterns from external datasets. (FIG. 19A) Analysis of data from Guo et al., 2018, on 14 non-small cell carcinoma patients. (FIG. 19B) Analysis of data from Zhang et al., 2018, on 12 colorectal adenocarcinoma patients. Both datasets were analyzed using the same methodology as FIG. 12B. Each scatterplot shows a separate clonotype, plotted according to its size in NAT and tumor, with the clone size in blood represented by the size of the dot according to the legend at the bottom left. Clones are colored according to their clonal expansion pattern using the two-dimensional palette on the bottom left. Clones were classified according to their primary cell cluster, as determined in the external analyses, and plotted in their respective scatterplots. The results show that dual tumor/NAT expansion and corresponding peripheral clonal expansion are characteristic of the CD8-C3-CX3CR1 and CD4-C3-GNLY clusters, which associate with the 8.1-Eff cluster in the dataset (FIG. 13B). Dual tumor/NAT expansion with lower levels of blood expansion was also observed in the CD8-C4-GZMK cluster, which associates with the 8.2-Tem cluster in the dataset.

**FIGS. 20A - 20C** are plots showing pre- and post-treatment CD4+ clonotypes in tumor with and without TCR sharing in blood. Analysis was performed on the CD4+ clonotypes from Yost et. al., 2019 in the same manner as for CD8+ clonotypes in FIG. 14. Patients are the same as those in FIG. 14 and presented in the same order. (FIG 20A) TCR representation is not correlated between novel post-treatment CD8+ clonotypes and pre-treatment blood. The analysis of FIG. 14A was performed on novel clones with a CD4+ clonotype as their primary post-treatment cell cluster. Each dot represents a clonotype plotted according to its clone size in pre-treatment blood (or post-treatment blood for patient bcc.su002, which lacked a pre-treatment blood sample) and in post-treatment tumor. In contrast with CD8+ clonotypes, CD4+ clone sizes do not show the same degree of association with their matched clonotypes in blood. The total number of cells from novel CD4+ clonotypes sharing TCRs in blood is provided below each scatterplot, and they do not correlate with peripheral clonal diversity across patients. (FIG. 20B) Tracking of CD4+ T cell clones in tumor. The analysis of FIG. 14C was performed on clones with a CD4+ subtype as their primary cell cluster in either pre- or post-treatment tumor. Under this inclusive criterion, some clones were included that showed a change in primary cell cluster from a CD4+ Naive subtype pre-treatment to a CD8_act post-treatment. In contrast with CD8+ clonotypes, where Remaining clones showed higher clonal expansion in the blood-associated condition (FIG. 14C), CD4+ clonotypes generally showed higher clonal expansion in the blood-independent condition. Another difference is that patients had more Disappearing CD4+ clones and with larger clone sizes than Disappearing CD8+ clones, suggesting that CD4+ clones may be reacting more dynamically to antigenic changes than CD8+ clones. (FIG. 20C) Distribution of cell clusters for novel CD4+ clonotypes according to tumor expansion and TCR sharing with blood. The analysis of FIG. 14D was performed on novel clones with a CD4+ subtype as their post-treatment primary cell cluster. A difference can be observed in the association of T follicular helper (Tfh) cells with blood-independent clones, suggesting that these novel clones are originating or expanding locally rather than from peripheral blood.

**FIGS. 21A - 21C** are plots showing clonotypes matching databases of known and putative virally-reactive TCRs. (FIG. 21A) Diversity of clonotypes reactive to viral antigens. Clonotypes from each patient were matched against TCRs listed as reacting against common viral antigens from the VDJdb database (Shugay 2018). Viruses included cytomegalovirus (p65 antigen), Epstein-Barr virus (BMLF1, EBNA3), and influenza A (M1). Other antigens from these viruses are categorized as Other. Each matching clone is represented by a rectangular region above its corresponding antigen, with a color corresponding to its primary cell cluster, using the color scheme from FIG. 13A and shown also in the legend in the bottom right, and a height indicating its total clone size in tumor plus NAT. Clones are stacked on top of one another in random order. In cases where the adjacent clones have the same color, black lines were used to separate them when both clones had a clone count greater than 5, indicating a need to resolve them. These plots show that the majority of matching clonotypes were singletons, but that patients often had a few virally-reactive clones that expanded greatly. (FIG. 21B) Association of dual-expanded and blood-multiplet clones with viral reactivity. Clones matching VDJdb and multi-cancer TCRs from the Cancer Genome Atlas (TCGA), suggesting reactivity to a viral antigen, were grouped according to their clonal expansion pattern (n=NAT singletons, N=NAT multiplets, D=dual expanded, T=tumor multiplets, t=tumor singletons, b=blood singletons, B=blood multiplets). Frequencies of matches are shown for each database separately, as well as their union. Dual-expanded clones were most likely to match a virally-reactive TCR. Statistical testing with a chi-square test indicates a significant dependence in each case of viral reactivity to clonal expansion patterns, with p-values shown in the legend. (FIG. 21C) T cell clusters associate with viral reactivity. Clones were grouped according to their primary cell cluster, using the unsupervised analysis in FIG. 13A. The fraction of clones matching VDJdb (left) and multi-cancer TCRs from TCGA (right) are plotted for each cluster, with the mean value over all clones shown as a vertical line. Colors for each bar correspond to the scheme from FIG. 13A and shown in the legend for (FIG. 21A). Adjusted p-values from a chi-square test after multiple hypothesis testing using the Benjamini-Hochberg procedure are shown for clusters with values < 0.05.

**FIGS. 22A and 22B** are plots showing clinical associations with CD8+ cluster gene signatures. (FIG. 22A) Correlation between gene signatures and CD8A expression. Each dot represents a pre-treatment patient sample from one of three clinical trials of the anti-PD-L1 therapeutic atezolizumab: IMvigor210 (top row), POPLAR (middle row), and IMmotion150 (bottom row). Dots are colored according to their randomized treatment arm, if any, using the legend at the bottom right of each row. Expression of CD8A is compared with scores for each of the five CD8+ gene signatures, each in a separate column. Numbers in the titles indicate the Pearson correlation coefficient for the correlation between scores. The 8.1-Eff and 8.2-Tem signature scores show the highest correlations with CD8A expression, an indicator of the CD8+ T cell presence in tumors, suggesting their relative importance in explaining the variance in CD8+ T cell prevalence across patients. (FIG. 22B) Kaplan-Meier survival curves. Kaplan-Meier curves are plotted for the CD8A, 8.3a-Trm, 8.3b-Trm, and 8.3c-Trm signature scores to supplement those provided in FIG. 15D. The representation is the same as in FIG. 15D, with patients dichotomized by signature scores above (red) and below (black) the median. Each curve shows progression-free survival (PFS) on the x-axis and the fraction of patients surviving on the y-axis. Plus signs on each survival curve indicate censoring events. The legend for each plot indicates the hazard ratio (HR) and p-value of a Cox proportional hazards model comparing the two patient populations, with p-values < 0.05 labeled with an asterisk. CD8A curves are included because of the known association of CD8+ T cell prevalence in tumors with survival in cancer immunotherapy.

## DETAILED DESCRIPTION

The present disclosure provides diagnostic methods, therapeutic methods, and compositions for the treatment of cancer (e.g., lung cancer (e.g., non-small cell lung cancer (NSCLC)), bladder cancer (e.g., urothelial carcinoma (UC)), kidney cancer (e.g., renal cell carcinoma (RCC)), and breast cancer (e.g., triple-negative breast cancer (TNBC))). The disclosure is based, at least in part, on the discovery that the presence of clonally expanded T cells in peripheral blood of an individual having cancer, as evidenced, for example, by an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample obtained from the individual, can be used as a biomarker (e.g., predictive biomarker) in methods of identifying whether the individual is likely to respond to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)); selecting a therapy for treating the individual; optimizing therapeutic efficacy of a treatment that includes a PD-L1 axis binding antagonist; and/or monitoring the response of the individual to a treatment that includes a PD-L1 axis binding antagonist.

## I. DEFINITIONS

The term "about" as used herein refers to the usual error range for the respective value readily known to the skilled person in this technical field. Reference to "about" a value or parameter herein includes (and describes) embodiments that are directed to that value or parameter per se.

As used herein, "administering" is meant a method of giving a dosage of a compound (e.g., a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) or a composition (e.g., a pharmaceutical composition, e.g., a pharmaceutical composition including a PD-L1 axis binding antagonist) to a subject. The compounds and/or compositions utilized in the methods described herein can be administered, for example, intravenously (e.g., by intravenous infusion), subcutaneously, intramuscularly, intradermally, percutaneously, intraarterially, intraperitoneally, intralesionally, intracranially, intraarticularly, intraprostatically, intrapleurally, intratracheally, intranasally, intravitreally, intravaginally, intrarectally, topically, intratumorally, peritoneally, subconjunctivally, intravesicularlly, mucosally, intrapericardially, intraumbilically, intraocularly, orally, topically, locally, by inhalation, by injection, by infusion, by continuous infusion, by localized perfusion bathing target cells directly, by catheter, by lavage, in creams, or in lipid compositions. The method of administration can vary depending on various factors (e.g., the compound or composition being administered and the severity of the condition, disease, or disorder being treated).

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant ($K_D$). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "antibody" herein is used in the broadest sense and encompasses various antibody structures, including, but not limited to, monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include, but are not limited to, Fv, Fab, Fab', Fab'-SH, $F(ab')_2$; diabodies; linear antibodies; single-chain antibody molecules (e.g., scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The terms "anti-PD-L1 antibody" and "an antibody that binds to PD-L1" refer to an antibody that is capable of binding PD-L1 with sufficient affinity such that the antibody is useful as a diagnostic and/or therapeutic agent in targeting PD-L1. In one embodiment, the extent of binding of an anti-PD-L1 antibody to an unrelated, non-PD-L1 protein is less than about 10% of the binding of the antibody to PD-L1 as measured, e.g., by a radioimmunoassay (RIA). In certain embodiments, an anti-PD-L1 antibody binds to an epitope of PD-L1 that is conserved among PD-L1 from different species. In certain embodiments, the anti-PD-L1 antibody is atezolizumab (MPDL3280A). PD-L1 (programmed death ligand 1) is also referred to in the art as "programmed cell death 1 ligand 1," "PDCD1LG1," "CD274," "B7-H," and "PDL1." An exemplary human PD-L1 is shown in UniProtKB/Swiss-Prot Accession No.Q9NZQ7.1.

The term "anti-cancer therapy" refers to a therapy useful for treating a cancer (e.g., a lung cancer (e.g., non-small cell lung cancer (NSCLC)), a bladder cancer (e.g., a urothelial carcinoma (UC)), a kidney cancer (e.g., a renal cell carcinoma (RCC)), or a breast cancer (e.g., a triple-negative breast cancer (TNBC))). Examples of anti-cancer therapeutic agents include, but are limited to, e.g., chemotherapeutic agents, growth inhibitory agents, cytotoxic agents, agents used in radiation therapy, anti-angiogenesis agents, apoptotic agents, anti-tubulin agents, and other agents to treat cancer, for example, anti-CD20 antibodies, platelet derived growth factor inhibitors (e.g., GLEEVEC™ (imatinib mesylate)), a COX-2 inhibitor (e.g., celecoxib), interferons, cytokines, antagonists (e.g., neutralizing antibodies) that bind to one or more of the following targets: PDGFR-P, BlyS, APRIL, BCMA receptor(s), TRAIL/Apo2, other bioactive and organic chemical agents, and the like. Combinations thereof are also included in the invention.

An "article of manufacture" is any manufacture (e.g., a package or container) or kit comprising at least one reagent, e.g., a medicament for treatment of a disease or disorder (e.g., a cancer, e.g., a lung cancer (e.g., NSCLC), a bladder cancer (e.g., UC), a kidney cancer (e.g., RCC), or a breast cancer (e.g., TNBC)), or a probe for specifically detecting a biomarker described herein. In certain embodiments, the manufacture or kit is promoted, distributed, or sold as a unit for performing the methods described herein.

The phrase "based on" when used herein means that the information about one or more biomarkers is used to inform a treatment decision, information provided on a package insert, or marketing/promotional guidance, etc.

A "blocking" antibody or an "antagonist" antibody is one which inhibits or reduces biological activity of the antigen it binds. Preferred blocking antibodies or antagonist antibodies substantially or completely inhibit the biological activity of the antigen.

By "binding domain" is meant a part of a compound or a molecule that specifically binds to a target epitope, antigen, ligand, or receptor. Binding domains include, but are not limited to, antibodies (e.g., monoclonal, polyclonal, recombinant, humanized, and chimeric antibodies), antibody fragments or portions thereof (e.g., Fab fragments, Fab'$_2$, scFv antibodies, SMIP, domain antibodies, diabodies, minibodies, scFv-Fc, affibodies, nanobodies, and VH and/or VL domains of antibodies), receptors, ligands, aptamers, and other molecules having an identified binding partner.

The term "biomarker" as used herein refers to an indicator, e.g., predictive, diagnostic, and/or prognostic, which can be detected in a sample (e.g., one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA). The biomarker may serve as an indicator of a particular subtype of a disease or disorder (e.g., a cancer, e.g., a lung cancer (e.g., NSCLC), a bladder cancer (e.g., UC), a kidney cancer (e.g., RCC), or a breast cancer (e.g., TNBC)) characterized by certain molecular, pathological, histological, and/or clinical features. In some embodiments, a biomarker is a gene. Biomarkers include, but are not limited to, polynucleotides (e.g., DNA, and/or RNA), polynucleotide copy number alterations (e.g., DNA copy numbers), polypeptides, polypeptide and polynucleotide modifications (e.g., posttranslational modifications), carbohydrates, and/or glycolipid-based molecular markers.

The terms "biomarker signature," "signature," "biomarker expression signature," or "expression signature" are used interchangeably herein and refer to one or a combination of biomarkers whose expression is an indicator, e.g., predictive, diagnostic, and/or prognostic (e.g., the immune-score expression level of one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA). The biomarker signature may serve as an indicator of a particular subtype of a disease or disorder (e.g., a cancer, e.g., a lung cancer (e.g., NSCLC), a bladder cancer (e.g., UC), a kidney cancer (e.g., RCC), or a breast cancer (e.g., TNBC)) characterized by certain molecular, pathological, histological, and/or clinical features. In some embodiments, the biomarker signature is a "gene signature." The term "gene signature" is used interchangeably with "gene expression signature" and refers to one or a combination of polynucleotides whose expression is an indicator, e.g., predictive, diagnostic, and/or prognostic. In some embodiments, the biomarker signature is a "protein signature." The term "protein signature" is used interchangeably with "protein expression signature" and refers to one or a combination of polypeptides whose expression is an indicator, e.g., predictive, diagnostic, and/or prognostic.

The term "clonally expanded T cells" refers to T cells having a common antigen specificity, as assessed, for example, by T cell receptor (TCR) sequence homology. For instance, clonally expanded T cells may share at least 85% sequence identity (e.g., 85%, 86%, 87%, 88%, 89%, 90%, 91%, 92%, 93%, 94%, 95%, 96%, 97%, 98%, 99%, 99.9%, or 100% sequence identity) across their TCR alpha and/or beta chains (e.g., 85%, 86%, 87%, 88%, 89%, 90%, 91%, 92%, 93%, 94%, 95%, 96%, 97%, 98%, 99%, 99.9%, or 100% sequence identity across the CDR1, CDR2, and/or CDR3 regions of their TCR alpha and/or beta chains).

As used herein in the context of two or more TCRs, the term "corresponding" complementarity-determining regions (CDRs) refers to equivalent CDRs present within each TCR, i.e., CDR-1 of a particular TCR alpha chain is said to "correspond" to CDR-1 of another TCR alpha chain. Similarly, CDR-2 of a particular TCR alpha chain is said to "correspond" to CDR-2 of another TCR alpha chain; CDR-3 of a particular TCR alpha chain is said to "correspond" to CDR-3 of another TCR alpha chain; CDR-1 of a particular TCR beta chain is said to "correspond" to CDR-1 of another TCR beta chain; CDR-2 of a particular TCR beta chain is said to "correspond" to CDR-2 of another TCR beta chain; and CDR-3 of a particular TCR beta chain is said to "correspond" to CDR-3 of another TCR beta chain.

As used herein, the term "chimeric antigen receptor" ("CAR") refers to a recombinant polypeptide containing one or more antigen-binding regions (e.g., one or more CDRs) that recognize, and specifically bind to, a given antigen (e.g., an antigen expressed by a tumor cell). A CAR may contain one or more antigen-binding regions of an endogenous TCR (e.g., a TCR expressed by a T cell in peripheral blood of a patient that has cancer, such as a cancer described herein). However, the CAR need not have the molecular composition of a naturally-occurring TCR.

CARs, as described herein, generally contain at least an extracellular antigen binding domain, a transmembrane domain and a cytoplasmic signaling domain (also referred to herein as "an intracellular signaling domain") comprising a functional signaling domain derived from a stimulatory molecule as defined herein. The stimulatory molecule may be the zeta chain associated with the T cell receptor complex. In some embodiments, the intracellular signaling domain further contains one or more functional signaling domains derived from at least one costimulatory molecule, as described below. The costimulatory molecule may contain, for example, 4-1BB (i.e., CD137), CD27, and/or CD28. In some embodiments, the CAR contains a chimeric fusion protein having an extracellular antigen recognition domain, a transmembrane domain, and a cytoplasmic signaling domain comprising a functional signaling domain derived from a stimulatory molecule. The CAR may contain, for example, a chimeric fusion protein having an extracellular antigen recognition domain, a transmembrane domain and a cytoplasmic signaling domain comprising a functional signaling domain derived from a co-stimulatory molecule and a functional signaling domain derived from a stimulatory molecule. In some embodiments, a CAR contains a chimeric fusion protein having an extracellular antigen recognition domain, a transmembrane domain, and an intracellular signaling domain comprising two functional signaling domains derived from one or more co-stimulatory molecule(s) and a functional signaling domain derived from a stimulatory molecule. In some embodiments, the CAR contains a chimeric fusion protein having an extracellular antigen recognition domain, a transmembrane domain, and an intracellular signaling domain comprising at least two functional signaling domains derived from one or more co-stimulatory molecule(s) and a functional signaling domain derived from a stimulatory molecule. A CAR may contain a leader sequence at the amino-terminus of the CAR fusion protein. In some embodiments, a CAR further contains a leader sequence at the N-terminus of the extracellular antigen recognition domain, which may be cleaved from the antigen recognition domain, e.g., (a scFv) during cellular processing and localization of the CAR to the cellular membrane. For the avoidance of doubt, as used herein, the terms "intracellular domain" and "cytoplasmic domain" are used interchangeably.

The term "signaling domain" refers to the functional portion of a protein which acts by transmitting information within the cell to regulate cellular activity via defined signaling pathways by generating second messengers or functioning as effectors by responding to such messengers.

A CAR described herein may contain an antibody or antibody fragment thereof, which may exist in a variety of forms. For example, the antigen-binding domain may be expressed as part of a contiguous polypeptide chain including, for example, a single domain antibody fragment (sdAb), a single chain antibody (scFv) and a humanized antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, N.Y.; Houston et al., 1988, Proc. Natl. Acad. Sci. USA 85:5879-5883; Bird et al., 1988, Science 242:423-426).

A "stimulatory molecule," as the term is used herein, means a molecule expressed by a T cell that provides the primary cytoplasmic signaling sequence(s) that regulate primary activation of the TCR complex in a stimulatory way for at least some aspect of the T cell signaling pathway. In one aspect, the primary signal is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing primary cytoplasmic signaling sequence that is of particular use in the invention includes, but is not limited to, those derived from TCR zeta, FcR gamma, FcR beta, CD3 gamma, CD3 delta, CD3 epsilon, CD5, CD22, CD79a, CD79b, CD278 (also known as "ICOS") and CD66d. In a specific CAR molecule of the invention, the intracellular signaling domain in any one or more CAR molecules of the invention comprises an intracellular signaling sequence, e.g., a primary signaling sequence of CD3-zeta. In a specific CAR of the invention, the primary signaling sequence of CD3-zeta is the human sequence (SEQ ID NO: 99), or the equivalent residues from a non-human species, e.g., mouse, rodent, monkey, ape and the like.

An "intracellular signaling domain," as the term is used herein, refers to an intracellular portion of a molecule. The intracellular signaling domain generates a signal that promotes an immune effector function of the CAR containing cell, e.g., a CAR T cell. Examples of immune effector function, e.g., in a CAR T cell, include cytolytic activity and helper activity, including the secretion of cytokines.

In some embodiments, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a CAR T, a primary intracellular signaling domain can comprise a cytoplasmic sequence of a T cell receptor, and a costimulatory intracellular signaling domain can comprise cytoplasmic sequence from co-receptor or costimulatory molecule.

A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, FcR gamma, FcR beta, CD3 gamma, CD3 delta, CD3 epsilon, CD5, CD22, CD79a, CD79b, and CD66d DAP10 and DAP12.

As used herein "zeta" or alternatively "zeta chain", "CD3-zeta" or "TCR-zeta" is defined as the protein provided as GenBank Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain that are sufficient to functionally transmit an initial signal necessary for T cell activation. In one aspect the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO: 100 or SEQ ID NO: 99.

A "costimulatory molecule" refers to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are required for an efficient immune response. Costimulatory molecules include, but are not limited to, an MHC class I molecule, BTLA and a Toll ligand receptor, as well as OX40, CD27, CD28, CDS, ICAM-1, LFA-1 (GD11alCU18) and 4-1BB (CD137).

A costimulatory intracellular signaling domain can be derived from the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors. Examples of such molecules include CD27, CD28, 4-1BB (CD137), OX40, GITR, CD30, CD40, ICOS, BAFFR, HVEM, lymphocyte function-associated antigen-1 (LFA-1), CD2, CD7, LIGHT, NKG2C, SLAMF7, NKp80, CD160, B7-H3, and a ligand that specifically binds with CD83, and the like. The intracellular signaling domain can comprise the entire intracellular portion, or the entire native intracellular signaling domain, of the molecule from which it is derived, or a functional fragment thereof.

As used herein "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB costimulatory domain" is defined as amino acid residues 214-255 of GenBank Acc. No. AAA62478.2, or the equivalent residues from a non-human species, e.g., mouse, rodent, monkey, ape and the like. In one aspect, the "4-1BB costimulatory domain" is the human sequence or the equivalent residues from a non-human species, e.g., mouse, rodent, monkey, ape and the like. In one aspect, the "4-1BB costimulatory domain" is the sequence provided as SEQ ID NO: 102 or the equivalent residues from a non-human species, e.g., mouse, rodent, monkey, ape and the like.

The term "CST7" as used herein refers to the Cystatin F gene, including any native CST7 from any vertebrate source, including mammals such as primates (e.g., humans) and rodents (e.g., mice and rats), unless otherwise indicated. The term encompasses "full-length," unprocessed CST7 as well as any form of CST7 that results from processing in the cell. The term also encompasses naturally occurring variants of CST7 e.g., splice variants or allelic variants. The nucleic acid sequence of an exemplary human CST7 is listed in SEQ ID NO: 1 (GENBANK™ Accession No. AJ510170). The amino acid sequence of an exemplary protein encoded by human CST7 is shown in SEQ ID NO: 2 (GENBANK™ Accession No. CAD52872.1).

The term "NKG7" as used herein refers to the Natural Killer Cell Granule Protein 7 gene, including any native NKG7 from any vertebrate source, including mammals such as primates (e.g., humans) and rodents (e.g., mice and rats), unless otherwise indicated. The term encompasses "full-length," unprocessed NKG7 as well as any form of NKG7 that results from processing in the cell. The term also encompasses naturally occurring variants of NKG7, e.g., splice variants or allelic variants. The nucleic acid sequence of an exemplary human NKG7 is listed in SEQ ID NO: 3 (GENBANK™ Accession No. BC015759.1). The amino acid sequence of an exemplary protein encoded by human NKG7 is shown in SEQ ID NO: 4 (UNIPROT™ Accession No. Q16617-1).

The term "GZMH" as used herein refers to the Granzyme H gene, including any native GZMH from any vertebrate source, including mammals such as primates (e.g., humans) and rodents (e.g., mice and rats), unless otherwise indicated. The term encompasses "full-length," unprocessed GZMH as well as any form of GMZH that results from processing in the cell. The term also encompasses naturally occurring variants of GZMH, e.g., splice variants or allelic variants. The nucleic acid sequence of an exemplary human GZMH is listed in SEQ ID NO: 5 (GENBANK™ Accession No. AAA74885.1). The amino acid sequence of an exemplary protein encoded by human GZMH is shown in SEQ ID NO: 6 (UNIPROT™ Accession No. P20718-1).

The term "MT-ND4" as used herein refers to the Mitochondrially Encoded NADH:Ubiquinone Oxidoreductase Core Subunit 4 gene, including any native MT-ND4 from any vertebrate source, including mammals such as primates (e.g., humans) and rodents (e.g., mice and rats), unless otherwise indicated. The term encompasses "full-length," unprocessed MT-ND4 as well as any form of MT-ND4 that results from processing in the cell. The term also encompasses naturally occurring variants of MT-ND4, e.g., splice variants or allelic variants. The nucleic acid sequence of an exemplary human MT-ND4 is listed in SEQ ID NO: 7 (GENBANK™ Accession No. AAR92518.1). The amino acid sequence of an exemplary protein encoded by human MT-ND4 is shown in SEQ ID NO: 8 (UNIPROT™ Accession No. P03905-1).

The term "HLA-H" as used herein refers to the Major Histocompatibility Complex, Class I, H gene, including any native HLA-H from any vertebrate source, including mammals such as primates (e.g., humans) and rodents (e.g., mice and rats), unless otherwise indicated. The term encompasses "full-length," unprocessed HLA-H as well as any form of HLA-H that results from processing in the cell. The term also encompasses naturally occurring variants of HLA-H, e.g., splice variants or allelic variants. The nucleic acid sequence of an exemplary human HLA-H is listed in SEQ ID NO: 9 (European Nucleotide Archive Accession No. AAA36218.1). The amino acid sequence of an exemplary protein encoded by human HLA-H is shown in SEQ ID NO: 10 (UNIPROT™ Accession No. P01893-1).

The term "CCL5" as used herein refers to the C-C Motif Chemokine Ligand 5 gene, including any native CCL5 from any vertebrate source, including mammals such as primates (e.g., humans) and rodents (e.g., mice and rats), unless otherwise indicated. The term encompasses "full-length," unprocessed CCL5 as well as any form of CCL5 that results from processing in the cell. The term also encompasses naturally occurring variants of CCL5, e.g., splice variants or allelic variants. The nucleic acid sequence of an exemplary human CCL5 is listed in SEQ ID NO: 11 (European Nucleotide Archive Accession No. AF043341.1). The amino acid sequence of an exemplary protein encoded by human CCL5 is shown in SEQ ID NO: 12 (UNIPROT™ Accession No. P13501-1).

The term "CD8A" as used herein refers to the CD8A Molecule gene, including any native CD8A from any vertebrate source, including mammals such as primates (e.g., humans) and rodents (e.g., mice and rats), unless otherwise indicated. The term encompasses "full-length," unprocessed CD8A as well as any form of CD8A that results from processing in the cell. The term also encompasses naturally occurring variants of CD8A, e.g., splice variants or allelic variants. The nucleic acid sequence of an exemplary human CD8A is listed in SEQ ID NO: 13 (GENBANK™ Accession No. M12828.1). The amino acid sequence of an exemplary protein encoded by human CD8A is shown in SEQ ID NO: 14 (UNIPROT™ Accession No. P01732-1).

The term "CMC1" as used herein refers to the C-X9-C Motif Containing 1 gene, any native CMC1 from any vertebrate source, including mammals such as primates (e.g., humans) and rodents (e.g., mice and rats), unless otherwise indicated. The term encompasses "full-length," unprocessed CMC1 as well as any form of CMC1 that results from processing in the cell. The term also encompasses naturally occurring variants of CMC1, e.g., splice variants or allelic variants. The nucleic acid sequence of an exemplary human CMC1 is listed in SEQ ID NO: 15 (RefSeq Accession No. NM_182523.1). The amino acid sequence of an exemplary protein encoded by human CMC1 is shown in SEQ ID NO: 16 (UNIPROT™ Accession No. Q7Z7K0-1).

The term "CD8B" as used herein refers to the CD8B Molecule gene, including any native CD8B from any vertebrate source, including mammals such as primates (e.g., humans) and rodents (e.g., mice and rats), unless otherwise indicated. The term encompasses "full-length," unprocessed CD8B as well as any form of CD8B that results from processing in the cell. The term also encompasses naturally occurring variants of CD8B, e.g., splice variants or allelic variants. The nucleic acid sequence of an exemplary human CD8B is listed in SEQ ID NO: 17 (European Nucleotide Archive Accession No. BC100911.1). The amino acid sequence of an exemplary protein encoded by human CD8B is shown in SEQ ID NO: 18 (UNIPROT™ Accession No. P10966-1).

The term "HCST" as used herein refers to the Hematopoietic Cell Signal Transducer gene, including any native HCST from any vertebrate source, including mammals such as primates (e.g., humans) and rodents (e.g., mice and rats), unless otherwise indicated. The term encompasses "full-length," unprocessed HCST as well as any form of HCST that results from processing in the cell. The term also encompasses naturally occurring variants of HCST, e.g., splice variants or allelic variants. The nucleic acid sequence of an exemplary human HCST is listed in SEQ ID NO: 19 (European Nucleotide Archive Accession No. AF285447.1). The amino acid sequence of an exemplary protein encoded by human HCST is shown in SEQ ID NO: 20 (UNIPROT™ Accession No. Q9UBK5-1).

The term "MT-CYB" as used herein refers to the Mitochondrially Encoded Cytochrome B gene, including any native MT-CYB from any vertebrate source, including mammals such as primates (e.g., humans) and rodents (e.g., mice and rats), unless otherwise indicated. The term encompasses "full-length," unprocessed MT-CYB as well as any form of MT-CYB that results from processing in the cell. The term also encompasses naturally occurring variants of MT-CYB, e.g., splice variants or allelic variants. The nucleic acid sequence of an exemplary human MT-CYB is listed in SEQ ID NO: 21 (European Nucleotide Archive Accession No. M28016.1). The amino acid sequence of an exemplary protein encoded by human MT-CYB is shown in SEQ ID NO: 22 (UNIPROT™ Accession No. P00156-1).

The term "MT-ND4L" as used herein refers to the Mitochondrially Encoded NADH:Ubiquinone Oxidoreductase Core Subunit 4L gene, including any native MT-ND4L from any vertebrate source, including mammals such as primates (e.g., humans) and rodents (e.g., mice and rats), unless otherwise indicated. The term encompasses "full-length," unprocessed MT-ND4L as well as any form of MT-ND4L that results from processing in the cell. The term also encompasses naturally occurring variants of MT-ND4L, e.g., splice variants or allelic variants. The nucleic acid sequence of an exemplary human MT-ND4L is listed in SEQ ID NO: 23 (RefSeq Accession No. NC_012920.1). The amino acid sequence of an exemplary protein encoded by human MT-ND4L is shown in SEQ ID NO: 24 (UNIPROT™ Accession No. P03901-1).

The term "KLRG1" as used herein refers to the Killer Cell Lectin Like Receptor G1 gene, including any native KLRG1 from any vertebrate source, including mammals such as primates (e.g., humans) and rodents (e.g., mice and rats), unless otherwise indicated. The term encompasses "full-length," unprocessed KLRG1 as well as any form of KLRG1 that results from processing in the cell. The term also encompasses naturally occurring variants of KLRG1, e.g., splice variants or allelic variants. The nucleic acid sequence of an exemplary human KLRG1 is listed in SEQ ID NO: 25 (European Nucleotide Archive Accession No. BC012621.1). The amino acid sequence of an exemplary protein encoded by human KLRG1 is shown in SEQ ID NO: 26 (UNIPROT™ Accession No. Q96E93-1).

The term "MT-CO2" as used herein refers to the Mitochondrially Encoded Cytochrome C Oxidase II gene, including any native MT-CO2 from any vertebrate source, including mammals such as primates (e.g., humans) and rodents (e.g., mice and rats), unless otherwise indicated. The term encompasses "full-length," unprocessed MT-CO2 as well as any form of MT-CO2 that results from processing in the cell. The term also encompasses naturally occurring variants of MT-CO2, e.g., splice variants or allelic variants. The nucleic acid sequence of an exemplary human MT-CO2 is listed in SEQ ID NO: 27 (European Nucleotide Archive Accession No. X55654.1). The amino acid sequence of an exemplary protein encoded by human MT-CO2 is shown in SEQ ID NO: 28 (UNIPROT™ Accession No. P00403-1).

The term "MT-ATP6" as used herein refers to the Mitochondrially Encoded ATP Synthase Membrane Subunit 6 gene, including any native MT-ATP6 from any vertebrate source, including mammals such as primates (e.g., humans) and rodents (e.g., mice and rats), unless otherwise indicated. The term encompasses "full-length," unprocessed MT-ATP6 as well as any form of MT-ATP6 that results from processing in the cell. The term also encompasses naturally occurring variants of MT-ATP6, e.g., splice variants or allelic variants. The nucleic acid sequence of an exemplary human MT-ATP6 is listed in SEQ ID NO: 29 (RefSeq Accession No. NC_012920.1). The amino acid sequence of an exemplary protein encoded by human MT-ATP6 is shown in SEQ ID NO: 30 (UNIPROT™ Accession No. P00846-1).

The term "PLEK" as used herein refers to the Pleckstrin gene, including any native PLEK from any vertebrate source, including mammals such as primates (e.g., humans) and rodents (e.g., mice and rats), unless otherwise indicated. The term encompasses "full-length," unprocessed PLEK as well as any form of PLEK that results from processing in the cell. The term also encompasses naturally occurring variants of PLEK, e.g., splice variants or allelic variants. The nucleic acid sequence of an exemplary human PLEK is listed in SEQ ID NO: 31 (European Nucleotide Archive Accession No. AK313756.1). The amino acid sequence of an exemplary protein encoded by human PLEK is shown in SEQ ID NO: 32 (UNIPROT™ Accession No. P08567-1).

The term "CTSW" as used herein refers to the Cathepsin W gene, including any native CTSW from any vertebrate source, including mammals such as primates (e.g., humans) and rodents (e.g., mice and rats), unless otherwise indicated. The term encompasses "full-length," unprocessed CTSW as well as any form of CTSW that results from processing in the cell. The term also encompasses naturally occurring variants of CTSW, e.g., splice variants or allelic variants. The nucleic acid sequence of an exemplary human CTSW is listed in SEQ ID NO: 33 (European Nucleotide Archive Accession No. BC048255.1). The amino acid sequence of an exemplary protein encoded by human CTSW is shown in SEQ ID NO: 34 (UNIPROT™ Accession No. P56202-1).

The term "HLA-C" as used herein refers to the Major Histocompatibility Complex, Class I, C gene, including any native HLA-C from any vertebrate source, including mammals such as primates (e.g., humans) and rodents (e.g., mice and rats), unless otherwise indicated. The term encompasses "full-length," unprocessed HLA-C as well as any form of HLA-C that results from processing in the cell. The term also encompasses naturally occurring variants of HLA-C, e.g., splice variants or allelic variants. The nucleic acid sequence of an exemplary human HLA-C is listed in SEQ ID NO: 35 (European Nucleotide Archive Accession No. D64152.1). The amino acid sequence of an exemplary protein encoded by human HLA-C is shown in SEQ ID NO: 36 (UNIPROT™ Accession No. P30508-1).

The term "LYAR" as used herein refers to the Ly1 Antibody Reactive gene, including any native LYAR from any vertebrate source, including mammals such as primates (e.g., humans) and rodents (e.g., mice and rats), unless otherwise indicated. The term encompasses "full-length," unprocessed LYAR as well as any form of LYAR that results from processing in the cell. The term also encompasses naturally occurring variants of LYAR, e.g., splice variants or allelic variants. The nucleic acid sequence of an exemplary human LYAR is listed in SEQ ID NO: 37 (European Nucleotide Archive Accession No. BC015796.2). The amino acid sequence of an exemplary protein encoded by human LYAR is shown in SEQ ID NO: 38 (UNIPROT™ Accession No. Q9NX58-1).

The term "LITAF" as used herein refers to the Lipopolysaccharide Induced TNF Factor gene, including any native LITAF from any vertebrate source, including mammals such as primates (e.g., humans) and rodents (e.g., mice and rats), unless otherwise indicated. The term encompasses "full-length," unprocessed LITAF as well as any form of LITAF that results from processing in the cell. The term also encompasses naturally occurring variants of LITAF, e.g., splice variants or allelic variants. The nucleic acid sequence of an exemplary human LITAF is listed in SEQ ID NO: 39 (GENBANK™ Accession No. AK095955). The amino acid sequence of an exemplary protein encoded by human LITAF is shown in SEQ ID NO: 40 (UNIPROT™ Accession No. Q99732-1).

The term "GZMB" as used herein refers to the Granzyme B gene, including any native GZMB from any vertebrate source, including mammals such as primates (e.g., humans) and rodents (e.g., mice and rats), unless otherwise indicated. The term encompasses "full-length," unprocessed GZMB as well as any form of GZMB that results from processing in the cell. The term also encompasses naturally occurring variants of GZMB, e.g., splice variants or allelic variants. The nucleic acid sequence of an exemplary human GZMB is listed in SEQ ID NO: 53 (GENBANK™ Accession No. J03072). The amino acid sequence of an exemplary protein encoded by human GZMB is shown in SEQ ID NO: 54 (UNIPROT™ Accession No. P10144-1).

The term "KLRD1" as used herein refers to the Killer Cell Lectin Like Receptor D1 gene, including any native KLRD1 from any vertebrate source, including mammals such as primates (e.g., humans) and rodents (e.g., mice and rats), unless otherwise indicated. The term encompasses "full-length," unprocessed KLRD1 as well as any form of KLRD1 that results from processing in the cell. The term also encompasses naturally occurring variants of KLRD1, e.g., splice variants or allelic variants. The nucleic acid sequence of an exemplary human KLRD1 is listed in SEQ ID NO: 55 (GENBANK™ Accession No. AJ000001). The amino acid sequence of an exemplary protein encoded by human KLRD1 is shown in SEQ ID NO: 56 (UNIPROT™ Accession No. Q13241-1).

The term "FGFBP2" as used herein refers to the Fibroblast Growth Factor Binding Protein 2 gene, including any native FGFBP2 from any vertebrate source, including mammals such as primates (e.g., humans) and rodents (e.g., mice and rats), unless otherwise indicated. The term encompasses "full-length," unprocessed FGFBP2 as well as any form of FGFBP2 that results from processing in the cell. The term also encompasses naturally occurring variants of FGFBP2, e.g., splice variants or allelic variants. The nucleic acid sequence of an exemplary human FGFBP2 is listed in SEQ ID NO: 57 (GENBANK™ Accession No. BC025720). The amino acid sequence of an exemplary protein encoded by human FGFBP2 is shown in SEQ ID NO: 58 (UNIPROT™ Accession No. Q9BYJ0-1).

The term "KLRC4-KLRK1" as used herein refers to the KLRC4-KLRK1 readthrough gene, including any native KLRC4-KLRK1 from any vertebrate source, including mammals such as primates (e.g., humans) and rodents (e.g., mice and rats), unless otherwise indicated. The term encompasses "full-length," unprocessed KLRC4-KLRK1 as well as any form of KLRC4-KLRK1 that results from processing in the cell. The term also encompasses naturally occurring variants of KLRC4-KLRK1, e.g., splice variants or allelic variants. The nucleic acid sequence of an exemplary human KLRC4-KLRK1 is listed in SEQ ID NO: 59 (RefSeq Accession No. NM_001199805.1). The amino acid sequence of an exemplary protein encoded by human KLRC4-KLRK1 is shown in SEQ ID NO: 60 (UNIPROT™ Accession No. H3BQV0-1).

The term "KLRK1" as used herein refers to the Killer Cell Lectin Like Receptor K1 gene, including any native KLRK1 from any vertebrate source, including mammals such as primates (e.g., humans) and rodents (e.g., mice and rats), unless otherwise indicated. The term encompasses "full-length," unprocessed KLRK1 as well as any form of KLRK1 that results from processing in the cell. The term also encompasses naturally occurring variants of KLRK1, e.g., splice variants or allelic variants. The nucleic acid sequence of an exemplary human KLRK1 is listed in SEQ ID NO: 61 (GENBANK™ Accession No. AF260135). The amino acid sequence of an exemplary protein encoded by human KLRK1 is shown in SEQ ID NO: 62 (UNIPROT™ Accession No. P26718-1).

The term "B2M" as used herein refers to the Beta-2-Microglobulin gene, including any native B2M from any vertebrate source, including mammals such as primates (e.g., humans) and rodents (e.g., mice and rats), unless otherwise indicated. The term encompasses "full-length," unprocessed B2M as well as any form of B2M that results from processing in the cell. The term also encompasses naturally occurring variants of B2M, e.g., splice variants or allelic variants. The nucleic acid sequence of an exemplary human B2M is listed in SEQ ID NO: 63 (European Nucleotide Archive Accession No. AF072097.1). The amino acid sequence of an exemplary protein encoded by human B2M is shown in SEQ ID NO: 64 (UNIPROT™ Accession No. P61769-1).

The term "GZMA" as used herein refers to the Granzyme A gene, including any native GZMA from any vertebrate source, including mammals such as primates (e.g., humans) and rodents (e.g., mice and rats), unless otherwise indicated. The term encompasses "full-length," unprocessed GZMA as well as any form of GZMA that results from processing in the cell. The term also encompasses naturally occurring variants of GZMA, e.g., splice variants or allelic variants. The nucleic acid sequence of an exemplary human GZMA is listed in SEQ ID NO: 65 (GENBANK™ Accession No. BC015739). The amino acid sequence of an exemplary protein encoded by human GZMA is shown in SEQ ID NO: 66 (UNIPROT™ Accession No. P12544-1).

The term "ID2" as used herein refers to the Inhibitor of DNA Binding 2 gene, including any native ID2 from any vertebrate source, including mammals such as primates (e.g., humans) and rodents (e.g., mice and rats), unless otherwise indicated. The term encompasses "full-length," unprocessed ID2 as well as any form of ID2 that results from processing in the cell. The term also encompasses naturally occurring variants of ID2, e.g., splice variants or allelic variants. The nucleic acid sequence of an exemplary human ID2 is listed in SEQ ID NO: 67 (European Nucleotide Archive Accession No. BC030639). The amino acid sequence of an exemplary protein encoded by human ID2 is shown in SEQ ID NO: 68 (UNIPROT™ Accession No. Q02363-1).

The term "CX3CR1" as used herein refers to the C-X3-C Motif Chemokine Receptor 1 gene, including any native CX3CR1 from any vertebrate source, including mammals such as primates (e.g., humans) and rodents (e.g., mice and rats), unless otherwise indicated. The term encompasses "full-length," unprocessed CX3CR1 as well as any form of CX3CR1 that results from processing in the cell. The term also encompasses naturally occurring variants of CX3CR1, e.g., splice variants or allelic variants. The nucleic acid sequence of an exemplary human CX3CR1 is listed in SEQ ID NO: 69 (GENBANK™ Accession No. AK312373). The amino acid sequence of an exemplary protein encoded by human CX3CR1 is shown in SEQ ID NO: 70 (UNIPROT™ Accession No. P49238-1).

The term "PRSS23" as used herein refers to the Serine Protease 23 gene, including any native PRSS23 from any vertebrate source, including mammals such as primates (e.g., humans) and rodents (e.g., mice and rats), unless otherwise indicated. The term encompasses "full-length," unprocessed PRSS23 as well as any form of PRSS23 that results from processing in the cell. The term also encompasses naturally occurring variants of PRSS23, e.g., splice variants or allelic variants. The nucleic acid sequence of an exemplary human PRSS23 is listed in SEQ ID NO: 71 (GENBANK™ Accession No. AY359033). The amino acid sequence of an exemplary protein encoded by human PRSS23 is shown in SEQ ID NO: 72 (UNIPROT™ Accession No. 095084-1).

The term "GNLY" as used herein refers to the Granulysin gene, including any native GNLY from any vertebrate source, including mammals such as primates (e.g., humans) and rodents (e.g., mice and rats), unless otherwise indicated. The term encompasses "full-length," unprocessed GNLY as well as any form of GNLY that results from processing in the cell. The term also encompasses naturally occurring variants of GNLY, e.g., splice variants or allelic variants. The nucleic acid sequence of an exemplary human GNLY is listed in SEQ ID NO: 73 (GENBANK™ Accession No. CR541859). The amino acid sequence of an exemplary protein encoded by human GNLY is shown in SEQ ID NO: 74 (UNIPROT™ Accession No. P22749-1).

The term "PRF1" as used herein refers to the Perforin 1 gene, including any native PRF1 from any vertebrate source, including mammals such as primates (e.g., humans) and rodents (e.g., mice and rats), unless otherwise indicated. The term encompasses "full-length," unprocessed PRF1 as well as any form of PRF1 that results from processing in the cell. The term also encompasses naturally occurring variants of PRF1, e.g., splice variants or allelic variants. The nucleic acid sequence of an exemplary human PRF1 is listed in SEQ ID NO: 75 (GENBANK™ Accession No. AK312754). The amino acid sequence of an exemplary protein encoded by human PRF1 is shown in SEQ ID NO: 76 (UNIPROT™ Accession No. P14222-1).

The term "PATL2" as used herein refers to the PAT1 Homolog 2 gene, including any native PATL2 from any vertebrate source, including mammals such as primates (e.g., humans) and rodents (e.g., mice and rats), unless otherwise indicated. The term encompasses "full-length," unprocessed PATL2 as well as any form of PATL2 that results from processing in the cell. The term also encompasses naturally occurring variants of PATL2, e.g., splice variants or allelic variants. The nucleic acid sequence of an exemplary human PATL2 is listed in SEQ ID NO: 77 (RefSeq Accession No. NM_001145112.1). The amino acid sequence of an exemplary protein encoded by human PATL2 is shown in SEQ ID NO: 78 (UNIPROT™ Accession No. C9JE40-1).

The term "GPR56" as used herein refers to the adhesion G-protein coupled receptor G1 gene, including any native GPR56 from any vertebrate source, including mammals such as primates (e.g., humans) and rodents (e.g., mice and rats), unless otherwise indicated. The term encompasses "full-length," unprocessed GPR56 as well as any form of GPR56 that results from processing in the cell. The term also encompasses naturally occurring variants of GPR56, e.g., splice variants or allelic variants. The nucleic acid sequence of an exemplary human GPR56 is listed in SEQ ID NO: 79 (RefSeq Accession No. NM_001145770.2). The amino acid sequence of an exemplary protein encoded by human GPR56 is shown in SEQ ID NO: 80 (UNIPROT™ Accession No. Q9Y653-1).

The term "IFITM2" as used herein refers to the interferon-induced transmembrane protein 2 gene, including any native IFITM2 from any vertebrate source, including mammals such as primates (e.g., humans) and rodents (e.g., mice and rats), unless otherwise indicated. The term encompasses "full-length," unprocessed IFITM2 as well as any form of IFITM2 that results from processing in the cell. The term also encompasses naturally occurring variants of IFITM2, e.g., splice variants or allelic variants. The nucleic acid sequence of an exemplary human IFITM2 is listed in SEQ ID NO: 81 (RefSeq Accession No. NM_003641.3). The amino acid sequence of an exemplary protein encoded by human IFITM2 is shown in SEQ ID NO: 82 (UNIPROT™ Accession No. Q01629-1).

The term "IFITM1" as used herein refers to the interferon-induced transmembrane protein 1 gene, including any native IFITM1 from any vertebrate source, including mammals such as primates (e.g., humans) and rodents (e.g., mice and rats), unless otherwise indicated. The term encompasses "full-length," unprocessed IFITM1 as well as any form of IFITM1 that results from processing in the cell. The term also encompasses naturally occurring variants of IFITM1, e.g., splice variants or allelic variants. The nucleic acid sequence of an exemplary human IFITM1 is listed in SEQ ID NO: 83 (RefSeq Accession No. NM_001145112.1). The amino acid sequence of an exemplary protein encoded by human PATL2 is shown in SEQ ID NO: 84 (UNIPROT™ Accession No. P13164-1).

The term "TMSB10" as used herein refers to the thymosin beta-10 gene, including any native TMSB10 from any vertebrate source, including mammals such as primates (e.g., humans) and rodents (e.g., mice and rats), unless otherwise indicated. The term encompasses "full-length," unprocessed TMSB10 as well as any form of TMSB10 that results from processing in the cell. The term also encompasses naturally occurring variants of TMSB10, e.g., splice variants or allelic variants. The nucleic acid sequence of an exemplary human TMSB10 is listed in SEQ ID NO: 85 (RefSeq Accession No. NM_021103.3). The amino acid sequence of an exemplary protein encoded by human TMSB10 is shown in SEQ ID NO: 86 (UNIPROT™ Accession No. P63313-1).

The term "CD247" as used herein refers to the T-cell surface glycoprotein CD3 zeta chain gene, including any native CD247 from any vertebrate source, including mammals such as primates (e.g., humans) and rodents (e.g., mice and rats), unless otherwise indicated. The term encompasses "full-length," unprocessed CD247 as well as any form of CD247 that results from processing in the cell. The term also encompasses naturally occurring variants of CD247, e.g.,

splice variants or allelic variants. The nucleic acid sequence of an exemplary human CD247 is listed in SEQ ID NO: 87 (RefSeq Accession No. NM_000734.3). The amino acid sequence of an exemplary protein encoded by human CD247 is shown in SEQ ID NO: 88 (UNIPROT™ Accession No. P20963-1).

The term "COX2" as used herein refers to the prostaglandin G/H synthase 2 gene, including any native COX2 from any vertebrate source, including mammals such as primates (e.g., humans) and rodents (e.g., mice and rats), unless otherwise indicated. The term encompasses "full-length," unprocessed COX2 as well as any form of COX2 that results from processing in the cell. The term also encompasses naturally occurring variants of COX2, e.g., splice variants or allelic variants. The nucleic acid sequence of an exemplary human COX2 is listed in SEQ ID NO: 89 (RefSeq Accession No. NM 000963.3). The amino acid sequence of an exemplary protein encoded by human COX2 is shown in SEQ ID NO: 90 (UNIPROT™ Accession No. P35354-1).

The term "COX1" as used herein refers to the prostaglandin G/H synthase 1 gene, including any native COX1 from any vertebrate source, including mammals such as primates (e.g., humans) and rodents (e.g., mice and rats), unless otherwise indicated. The term encompasses "full-length," unprocessed COX1 as well as any form of COX1 that results from processing in the cell. The term also encompasses naturally occurring variants of COX1, e.g., splice variants or allelic variants. The nucleic acid sequence of an exemplary human COX1 is listed in SEQ ID NO: 91 (RefSeq Accession No. NM_000962.3). The amino acid sequence of an exemplary protein encoded by human COX1 is shown in SEQ ID NO: 92 (UNIPROT™ Accession No. P23219-1).

The term "CLIC3" as used herein refers to the chloride intracellular channel protein 3gene, including any native CLIC3 from any vertebrate source, including mammals such as primates (e.g., humans) and rodents (e.g., mice and rats), unless otherwise indicated. The term encompasses "full-length," unprocessed CLIC3 as well as any form of CLIC3 that results from processing in the cell. The term also encompasses naturally occurring variants of CLIC3, e.g., splice variants or allelic variants. The nucleic acid sequence of an exemplary human CLIC3 is listed in SEQ ID NO: 93 (RefSeq Accession No. NM_004669.2). The amino acid sequence of an exemplary protein encoded by human CLIC3 is shown in SEQ ID NO: 94 (UNIPROT™ Accession No. 095833-1).

The term "S100A4" as used herein refers to the Protein S100-A4 gene, including any native S100A4 from any vertebrate source, including mammals such as primates (e.g., humans) and rodents (e.g., mice and rats), unless otherwise indicated. The term encompasses "full-length," unprocessed S100A4 as well as any form of S100A4 that results from processing in the cell. The term also encompasses naturally occurring variants of S100A4, e.g., splice variants or allelic variants. The nucleic acid sequence of an exemplary human S100A4 is listed in SEQ ID NO: 95 (RefSeq Accession No. NM_002961.2). The amino acid sequence of an exemplary protein encoded by human S100A4 is shown in SEQ ID NO: 96 (UNIPROT™ Accession No. P26447-1).

The term "CYBA" as used herein refers to the cytochrome b-245 light chain gene, including any native CYBA from any vertebrate source, including mammals such as primates (e.g., humans) and rodents (e.g., mice and rats), unless otherwise indicated. The term encompasses "full-length," unprocessed CYBA as well as any form of CYBA that results from processing in the cell. The term also encompasses naturally occurring variants of CYBA, e.g., splice variants or allelic variants. The nucleic acid sequence of an exemplary human CYBA is listed in SEQ ID NO: 97 (RefSeq Accession No. NM_000101.3). The amino acid sequence of an exemplary protein encoded by human CYBA is shown in SEQ ID NO: 98 (UNIPROT™ Accession No. P13498-1).

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia or lymphoid malignancies. More particular examples of such cancers include, but are not limited to, lung cancer, including small-cell lung cancer, non-small cell lung cancer, adenocarcinoma of the lung, and squamous carcinoma of the lung; bladder cancer (e.g., urothelial carcinoma cancer (UC), muscle invasive bladder cancer (MIBC), and BCG-refractory non-muscle invasive bladder cancer (NMIBC)); kidney or renal cancer (e.g., renal cell carcinoma (RCC)); cancer of the urinary tract; breast cancer (e.g., HER2+ breast cancer and triple-negative breast cancer (TNBC), which are estrogen receptors (ER-), progesterone receptors (PR-), and HER2 (HER2-) negative); prostate cancer, such as castration-resistant prostate cancer (CRPC); cancer of the peritoneum; hepatocellular cancer; gastric or stomach cancer, including gastrointestinal cancer and gastrointestinal stromal cancer; pancreatic cancer; glioblastoma; cervical cancer; ovarian cancer; liver cancer; hepatoma; colon cancer; rectal cancer; colorectal cancer; endometrial or uterine carcinoma; salivary gland carcinoma; prostate cancer; vulval cancer; thyroid cancer; hepatic carcinoma; anal carcinoma; penile carcinoma; melanoma, including superficial spreading melanoma, lentigo maligna melanoma, acral lentiginous melanomas, and nodular melanomas; multiple myeloma and B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); acute myelogenous leukemia (AML); hairy cell leukemia; chronic myeloblastic leukemia (CML); post-transplant lymphoproliferative disorder (PTLD); and myelodysplastic syndromes (MDS), as well as abnormal vascular proliferation associated with phakomatoses, edema (such as that associated with brain tumors), Meigs' syndrome, brain cancer, head and neck cancer, and associated metastases.

The terms "cell proliferative disorder" and "proliferative disorder" refer to disorders that are associated with some degree of abnormal cell proliferation. In one embodiment, the cell proliferative disorder is a cancer (e.g., a lung cancer (e.g., NSCLC), a bladder cancer (e.g., UC), a kidney cancer (e.g., RCC), or a breast cancer (e.g., TNBC)). In another embodiment, the cell proliferative disorder is a tumor.

A "chemotherapeutic agent" is a chemical compound useful in the treatment of a cancer (e.g., cancer, e.g., a lung cancer (e.g., NSCLC), a bladder cancer (e.g., UC), a kidney cancer (e.g., RCC), or a breast cancer (e.g., TNBC)). Examples of chemotherapeutic agents include alkylating agents such as thiotepa and cyciosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin $\gamma$1' and calicheamicin $\omega$1' (see, e.g., Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); combretastatin; folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, e.g., paclitaxel (TAXOL®, Bristol-Myers Squibb Oncology, Princeton, N.J.), albumin-engineered nanoparticle formulation of paclitaxel (ABRAXANE™), and docetaxel (TAXOTERE®, Rhome-Poulene Rorer, Antony, France); chloranbucil; 6-thioguanine; mercaptopurine; methotrexate; platinum agents such as cisplatin, oxaliplatin (e.g., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETINO); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELIDO), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R) (e.g., erlotinib (TARCEVA™)); and VEGF-A that reduce cell proliferation; vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); BAY439006 (sorafenib; Bayer);

SU-11248 (sunitinib, SUTENT®, Pfizer); perifosine, COX-2 inhibitor (e.g., celecoxib or etoricoxib), proteosome inhibitor (e.g., PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors; tyrosine kinase inhibitors; serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin, and pharmaceutically acceptable salts, acids or derivatives of any of the above; as well as combinations of two or more of the above.

Chemotherapeutic agents as defined herein also include "anti-hormonal agents" or "endocrine therapeutics" which act to regulate, reduce, block, or inhibit the effects of hormones that can promote the growth of cancer (e.g., a lung cancer (e.g., NSCLC), a bladder cancer (e.g., UC), a kidney cancer (e.g., RCC), or a breast cancer (e.g., TNBC)). They may be hormones themselves, including, but not limited to: anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON.cndot.toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISORO vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in aberrant cell proliferation, such as, for example, PKC-alpha, Raf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; Vinorelbine and Esperamicins (see U.S. Pat. No. 4,675,187), and pharmaceutically acceptable salts, acids or derivatives of any of the above; as well as combinations of two or more of the above.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "cytotoxic gene" as used herein refers to a gene whose expression is indicative of the presence and/or activity of a cytotoxic T lymphocyte (CTL). Cytotoxic genes include those that encode proteins present on the CTL surface, such as CD8A, as well as those that encode secretory proteins associated with CTL function, such as CCL5. Exemplary cytotoxic genes for use in conjunction with the compositions and methods described herein include PLEK, HCST, CTSW, KLRG1, CD8A, CCL5, CD8B, CST7, NKG7, GZMH, and HLA-C.

The term "mitochondrial gene" as used herein refers to a gene encoding an RNA and/or protein product involved in mitochondrial function, such as the oxidative generation of adenosine triphosphate. Exemplary mitochondrial genes for use in conjunction with the compositions and methods described herein include MT-ND4, MT-ND4L, MT-ATP6, MT-CO2, and MT-CYB.

The term "cytotoxic agent" as used herein refers to a substance that inhibits or prevents a cellular function and/or causes cell death or destruction. Cytotoxic agents include, but are not limited to, radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

As used herein, "delaying progression" of a disorder or disease means to defer, hinder, slow, retard, stabilize, and/or postpone development of the disease or disorder (e.g., a cancer, e.g., a lung cancer (e.g., NSCLC), a bladder cancer (e.g., UC), a kidney cancer (e.g., RCC), or a breast cancer (e.g., TNBC)). This delay can be of varying lengths of time, depending on the history of the disease and/or subject being treated. As is evident to one skilled in the art, a sufficient or significant delay can, in effect, encompass prevention, in that the subject does not develop the disease.

The terms "determination," "determining," "detection," "detecting," and grammatical variations thereof include any means of determining or detecting, including direct and indirect determination or detection.

A "disorder" or "disease" is any condition that would benefit from treatment including, but not limited to, chronic and acute disorders or diseases including those pathological conditions which predispose the mammal to the disorder in question (e.g., cancer, e.g., a lung cancer (e.g., NSCLC), a bladder cancer (e.g., UC), a kidney cancer (e.g., RCC), or a breast cancer (e.g., TNBC)).

The term "diagnosis" is used herein to refer to the identification or classification of a molecular or pathological state, disease or condition (e.g., cancer, e.g., a lung cancer (e.g., NSCLC), a bladder cancer (e.g., UC), a kidney cancer (e.g., RCC), or a breast cancer (e.g., TNBC)). For example, "diagnosis" may refer to identification of a particular type of cancer. "Diagnosis" may also refer to the classification of a particular subtype of cancer, e.g., by histopathological criteria, or by molecular features (e.g., a subtype characterized by expression of one or a combination of biomarkers (e.g., particular genes or proteins encoded by the genes)).

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g., PD-L1); and B cell activation.

An "effective amount" of a compound, for example, an PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody))PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), or a composition (e.g., pharmaceutical composition) thereof, is at least the minimum amount required to achieve the desired therapeutic or prophylactic result, such as a measurable increase in overall survival (OS) or progression-free survival (PFS) of a particular disease or disorder (e.g., a cancer, e.g., a lung cancer (e.g., NSCLC), a bladder cancer (e.g., UC), a kidney cancer (e.g., RCC), or a breast cancer (e.g., TNBC)). An effective amount herein may vary according to factors such as the disease state, age, sex, and weight of the individual, and the ability of the antibody to elicit a desired response in the subject. An effective amount is also one in which any toxic or detrimental effects of the treatment are outweighed by the therapeutically beneficial effects. For prophylactic use, beneficial or desired results include results such as eliminating or reducing the risk, lessening the severity, or delaying the onset of the disease, including biochemical, histological and/or behavioral symptoms of the disease, its complications, and intermediate pathological phenotypes presenting during development of the disease. An effective amount can be administered in one or more administrations. For purposes of this invention, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may optionally be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved. For example, an effective amount of a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) as a cancer treatment may reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), the response rates (RR), duration of response, and/or quality of life.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full-length antibody," "intact antibody," and "whole antibody" are used herein interchangeably to refer to an antibody having a structure substantially similar to a native antibody structure or having heavy chains that contain an Fc region as defined herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, e.g., U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26 35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The word "label" when used herein refers to a detectable compound or composition. The label is typically conjugated or fused directly or indirectly to a reagent, such as a polynucleotide probe or an antibody, and facilitates detection of the reagent to which it is conjugated or fused. The label may itself be detectable (e.g., radioisotope labels or fluorescent labels) or, in the case of an enzymatic label, may catalyze chemical alteration of a substrate compound or composition which results in a detectable product.

The terms "level of expression" or "expression level" in general are used interchangeably and generally refer to the amount of a biomarker in a biological sample. "Expression" generally refers to the process by which information (e.g., gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications (e.g., posttranslational modification of a polypeptide). Fragments of the transcribed polynucleotide, the translated polypeptide, or polynucleotide and/or polypeptide modifications (e.g., post-translational modification of a polypeptide) shall also be regarded as expressed whether they originate from a transcript generated by alternative splicing or a degraded transcript, or from a post-translational processing of the polypeptide, e.g., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs). Expression level can be measured by methods known to one skilled in the art and also disclosed herein, including, for example, RT-qPCR and RNA-seq. The expression level assessed can be used to determine the response to the treatment.

The term "immune-score expression level" refers to a numerical value that reflects the expression level (e.g., a normalized expression level) of a single gene of interest, or an aggregated expression level for more than one gene of interest (e.g., at least two, at least three, at least four, at least five, or more genes of interest), related to immune response. An immune-score expression level for more than one gene of interest may be determined by aggregation methods known to one skilled in the art and also disclosed herein, including, for example, by calculating the median or mean of the expression levels of all of the genes of interest. Before aggregation, the expression level of each gene of interest may be normalized by using statistical methods known to one skilled in the art and also disclosed herein, including, for example, normalized to the expression level of one or more housekeeping genes, or normalized to a total library size, or normalized to the median or mean expression level value across all genes measured. In some instances, before aggregation across multiple genes of interest, the normalized expression level of each gene of interest may be standardized by calculating the Z-score of the normalized expression level of each gene of interest. In some instances, each gene of interest may have an assigned weight score, and the immune-score expression level of multiple genes of interest may be calculated by incorporating the weight score to determine the mean of the weighted expression levels of all of the genes of interest. For example, an immune-score expression level may refer to a numerical value that reflects the normalized expression level of a single gene selected from CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA. Alternatively, an immune-score expression level may, for example, refer to a numerical value that reflects the aggregated normalized expression level (e.g., median of the normalized expression levels, or mean of the normalized expression levels) for at least two, at least three, at least four, at least five, at least six, at least seven, at least eight, at least nine, at least 10, at least 11, at least 12, at least 13, at least 14, at least 15, at least 16, at least 17, at least 18, at least 19, at least 20, or more, of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, or combinations thereof. In some instances, an immune-score expression level may, for example, refer to a numerical value that reflects the aggregated Z-score expression level (e.g., mean of the Z-score normalized expression level, or median of the Z-score normalized expression level) for at least two, at least three, at least four, at least five, at least six, at least seven, at least eight, at least nine, at least 10, at least 11, at least 12, at least 13, at least 14, at least 15, at least 16, at least 17, at least 18, at least 19, at least 20, or more, of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, or combinations thereof.

As used herein, the term "reference immune-score expression level" refers to an immune-score expression level against which another immune-score expression level (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) is compared, e.g., to make a diagnostic, predictive, prognostic, and/or therapeutic determination. For example, the reference immune-score expression level may be derived from expression levels (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) in a reference sample, a reference population, and/or a pre-assigned value (e.g., a cut-off value which was previously determined to significantly (e.g., statistically significantly) separate a first subset of individuals who have been treated with a PD-L1 axis binding antagonist (e.g., a PD-L1 binding antagonist (e.g., anti-PD-L1 antibody, e.g., atezolizumab (MPDL3280A)) or a PD-1 binding antagonist (e.g., anti-PD-1 antibody)) therapy in a reference population and a second subset of individuals who have been treated with a non-PD-L1 axis binding antagonist therapy that does not comprise a PD-L1 axis binding antagonist in the same reference population based on a significant difference between an individual's responsiveness to treatment with the PD-L1 axis binding antagonist therapy and an individual's responsiveness to treatment with the non-PD-L1 axis binding antagonist therapy above the cut-off value and/or below the cut-off value, wherein the individual's responsiveness to treatment with the PD-L1 axis binding antagonist therapy is significantly (e.g., statistically significantly) improved relative to the individual's responsiveness to treatment with the non-PD-L1 axis binding antagonist therapy above the cut-off value and/or the individual's responsiveness to treatment with the non-PD-L1 axis binding antagonist therapy is significantly (e.g., statistically significantly) improved relative to the individual's responsiveness to treatment with the PD-L1 axis binding antagonist therapy below the cut-off value). It will be appreciated by one skilled in the art that the numerical value for the reference immune-score expression level may vary depending on the indication (e.g., a cancer (e.g., a breast cancer, a lung cancer, a kidney cancer, or a bladder cancer), the methodology used to detect expression levels (e.g., RNA-seq or RT-qPCR), the statistical methods used to generate an immune-score, and/or the specific combinations of genes examined.

"Elevated expression," "elevated expression levels," or "elevated levels" refers to an increased expression or increased levels of a gene or combination of genes (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) in a subject relative to a control, such as a subject or subjects who are not suffering from the disease or disorder (e.g., a cancer, e.g., a lung cancer (e.g., NSCLC), a bladder cancer (e.g., UC), a kidney cancer (e.g., RCC), or a breast cancer (e.g., TNBC)) or an internal control (e.g., housekeeping gene), or a reference level, such as a reference immune-score expression level.

"Reduced expression," "reduced expression levels," or "reduced levels" refers to a decrease expression or decreased levels of a gene or combination of genes (e.g., for one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) in a subject relative to a control, such as a subject or subjects who are not suffering from the disease or disorder (e.g., a cancer, e.g., a lung cancer (e.g., NSCLC), a bladder cancer (e.g., UC), a kidney cancer (e.g., RCC), or a breast cancer (e.g., TNBC)) or an internal control (e.g., housekeeping gene), or a reference level, such as a reference immune-score expression level. In some embodiments, reduced expression is little or no expression.

A "reference gene" as used herein, refers to a gene or group of genes (e.g., one, two, three, four, five, or six or more genes) that is used for comparison purposes, such as a housekeeping gene. A "housekeeping gene" refers herein to a gene or group of genes (e.g., one, two, three, four, five, or six or more genes) which encode proteins whose activities are essential for the maintenance of cell function and which are typically similarly present in all cell types.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N-to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "oligonucleotide" refers to a relatively short polynucleotide (e.g., less than about 250 nucleotides in length), including, without limitation, single-stranded deoxyribonucleotides, single- or double-stranded ribonucleotides, RNA:DNA hybrids and double-stranded DNAs. Oligonucleotides, such as single- stranded DNA probe oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells and organisms.

The term "package insert" is used to refer to instructions customarily included in commercial packages of therapeutic products, that contain information about the indications, usage, dosage, administration, combination therapy, contraindications and/or warnings concerning the use of such therapeutic products.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "protein," as used herein, refers to any native protein from any vertebrate source, including mammals such as primates (e.g., humans) and rodents (e.g., mice and rats), unless otherwise indicated. The term encompasses "full-length," unprocessed protein as well as any form of the protein that results from processing in the cell. The term also encompasses naturally occurring variants of the protein, e.g., splice variants or allelic variants.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The terms "Programmed Death Ligand 1" and "PD-L1" refer herein to a native sequence PD-L1 polypeptide, polypeptide variants, and fragments of a native sequence polypeptide and polypeptide variants (which are further defined herein). The PD-L1 polypeptide described herein may be that which is isolated from a variety of sources, such as from human tissue types or from another source, or prepared by recombinant or synthetic methods.

"PD-L1 polypeptide variant", or variations thereof, means a PD-L1 polypeptide, generally an active PD-L1 polypeptide, as defined herein having at least about 80% amino acid sequence identity with any of the native sequence PD-L1 polypeptide sequences as disclosed herein. Such PD-L1 polypeptide variants include, for instance, PD-L1 polypeptides wherein one or more amino acid residues are added, or deleted, at the N- or C-terminus of a native amino acid sequence. Ordinarily, a PD-L1 polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a native sequence PD-L1 polypeptide sequence as disclosed herein. Ordinarily, PD-L1 variant polypeptides are at least about 10 amino acids in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 281, 282, 283, 284, 285, 286, 287, 288, 289amino acids in length, or more. Optionally, PD-L1 variant polypeptides will have no more than one conservative amino acid substitution as compared to a native PD-L1 polypeptide sequence, alternatively no more than 2, 3, 4, 5, 6, 7, 8, 9, or 10 conservative amino acid substitution as compared to the native PD-L1 polypeptide sequence.

A "native sequence PD-L1 polypeptide" comprises a polypeptide having the same amino acid sequence as the corresponding PD-L1 polypeptide derived from nature.

The term "PD-L1 axis binding antagonist" refers to a molecule that inhibits the interaction of a PD-L1 axis binding partner with one or more of its binding partners, so as to remove T cell dysfunction resulting from signaling on the PD-1 signaling axis, with a result being restored or enhanced T cell function. As used herein, a PD-L1 axis binding antagonist includes a PD-L1 binding antagonist and a PD-1 binding antagonist, as well as molecules that interfere with the interaction between PD-L1 and PD-1 (e.g., a PD-L2-Fc fusion).

The term "PD-L1 binding antagonist" refers to a molecule that decreases, blocks, inhibits, abrogates, or interferes with signal transduction resulting from the interaction of PD-L1 with either one or more of its binding partners, such as PD-1 or B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen-binding fragments thereof, immunoadhesins, fusion proteins, oligopeptides, and other molecules that decrease, block, inhibit, abrogate, or interfere with signal transduction resulting from the interaction of PD-L1 with one or more of its binding partners, such as PD-1 or B7-1. In one embodiment, a PD-L1 binding antagonist reduces the negative costimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antibody. In a specific embodiment, the anti-PD-L1 antibody is atezolizumab (CAS Registry Number: 1422185-06-5), also known as MPDL3280A, and described herein, in another specific embodiment, the anti-PD-L1 antibody is YW243.55.S70, described herein. In another specific embodiment, the anti-PD-L1 antibody is MDX-1 105, described herein. In still another specific aspect, the anti-PD-L1 antibody is MEDI4736 (durvalumab), described herein. In still another specific aspect, the anti-PD-L1 antibody is MSB0010718C (avelumab), described herein.

As used herein, a "PD-1 binding antagonist" is a molecule that decreases, blocks, inhibits, abrogates or interferes with signal transduction resulting from the interaction of PD-1 with one or more of its binding partners, such as PD-L1 and/or PD-L2. In some embodiments, the PD-1 binding antagonist is a molecule that inhibits the binding of PD-1 to its binding partners. In a specific aspect, the PD-1 binding antagonist inhibits the binding of PD-1 to PD-L1 and/or PD-L2. For example, PD-1 binding antagonists include anti PD-1 antibodies and antigen-binding fragments thereof, immunoadhesins, fusion proteins, oligopeptides, small molecule antagonists, polynucleotide antagonists, and other molecules that decrease, block, inhibit, abrogate or interfere with signal transduction resulting from the interaction of PD-1 with PD-L1 and/or PD-L2. In one embodiment, a PD-1 binding antagonist reduces the negative signal mediated by or through cell surface proteins expressed on T lymphocytes, and other cells, mediated signaling through PD-1 or PD-L1 so as render a dysfunctional T cell less dysfunctional. In some embodiments, the PD-1 binding antagonist is an anti-PD-1 antibody. In a specific aspect, a PD-1 binding antagonist is MDX-1106 (nivolumab). In another specific aspect, a PD-1 binding antagonist is MK-3475 (pembrolizumab). In another specific aspect, a PD-1 binding antagonist is CT-011 (pidilizumab). In another specific aspect, a PD-1 binding antagonist is MEDI-0680 (AMP-514). In another specific aspect, a PD-1 binding antagonist is PDR001. In another specific aspect, a PD-1 binding antagonist is REGN2810. In another specific aspect, a PD-1 binding antagonist is BGB-108. In another specific aspect, a PD-1 binding antagonist is AMP-224.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR2 ("amidate"), P(O)R, P(O)OR', CO or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

The technique of "polymerase chain reaction" or "PCR" as used herein generally refers to a procedure wherein minute amounts of a specific piece of nucleic acid, RNA and/or DNA, are amplified as described in U.S. Pat. No. 4,683,195 issued 28 July 1987. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage or plasmid sequences, etc. See generally Mullis et al., Cold Spring Harbor Symp. Quant. Biol., 51: 263 (1987); Erlich, ed., PCR Technology, (Stockton Press, NY, 1989). As used herein, PCR is considered to be one, but not the only, example of a nucleic acid polymerase reaction method for amplifying a nucleic acid test sample, comprising the use of a known nucleic acid (DNA or RNA) as a primer and utilizes a nucleic acid polymerase to amplify or generate a specific piece of nucleic acid or to amplify or generate a specific piece of nucleic acid which is complementary to a particular nucleic acid.

As used herein, the term "reverse transcriptase polymerase chain reaction" or "RT-PCR" refers to the replication and amplification of RNA sequences. In this method, reverse transcription is coupled to PCR, e.g., as described in U.S. Patent No. 5,322,770, herein incorporated by reference in its entirety. In RT-PCR, the RNA template is converted to cDNA due to the reverse transcriptase activity of an enzyme, and then amplified using the polymerizing activity of the same or a different enzyme. Both thermostable and thermolabile reverse transcriptase and polymerase can be used. The "reverse transcriptase" (RT) may include reverse transcriptases from retroviruses, other viruses, as well as a DNA polymerase exhibiting reverse transcriptase activity.

As used herein, the term "reverse transcriptase quantitative polymerase chain reaction" or "RT-qPCR" is a form of PCR wherein the nucleic acid to be amplified is RNA that is first reverse transcribed into cDNA and the amount of PCR product is measured at each step in a PCR reaction.

"Quantitative real time polymerase chain reaction" or "qRT-PCR" refers to a form of PCR wherein the amount of PCR product is measured at each step in a PCR reaction. This technique has been described in various publications including Cronin et al., Am. J. Pathol. 164(1):35-42 (2004); and Ma et al., Cancer Cell 5:607-616 (2004).

The term "multiplex-PCR" refers to a single PCR reaction carried out on nucleic acid obtained from a single source (e.g., an individual) using more than one primer set for the purpose of amplifying two or more DNA sequences in a single reaction.

The term "RNA-seq," also called "Whole Transcriptome Shotgun Sequencing (WTSS)," refers to the use of high-throughput sequencing technologies to sequence and/or quantify cDNA to obtain information about a sample's RNA content. Publications describing RNA-seq include: Wang et al. "RNA-Seq: a revolutionary tool for transcriptomics" Nature Reviews Genetics 10 (1): 57-63 (January 2009); Ryan et al. BioTechniques 45 (1): 81-94 (2008); and Maher et al. "Transcriptome sequencing to detect gene fusions in cancer". Nature 458 (7234): 97-101 (January 2009).

The term "polynucleotide," when used in singular or plural, generally refers to any polyribonucleotide or polydeoxyribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple- stranded regions comprising RNA or DNA or both RNA and DNA The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

"Response to a treatment," "responsiveness to treatment," or "benefit from a treatment" can be assessed using any endpoint indicating a benefit to the individual, including, without limitation, (1) inhibition, to some extent, of disease progression (e.g., cancer progression), including slowing down and complete arrest; (2) a reduction in tumor size; (3) inhibition (i.e., reduction, slowing down or complete stopping) of cancer cell infiltration into adjacent peripheral organs and/or tissues; (4) inhibition (i.e. reduction, slowing down or complete stopping) of metastasis; (5) relief, to some extent, of one or more symptoms associated with the disease or disorder (e.g., cancer); (6) increase or extend in the length of survival, including overall survival (OS HR < 1) and progression free survival (PFS HR < 1); and/or (9) decreased mortality at a given point of time following treatment (e.g., a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). As used herein, a patient that "fails to respond" to a particular form of treatment is one that fails to exhibit any or all of the above-described benefits following administration of the therapy of interest.

As used herein, "progression-free survival" or "PFS" refers to the length of time during and after treatment during which the disease being treated (e.g., cancer, e.g., a lung cancer (e.g., NSCLC), a bladder cancer (e.g., UC), a kidney cancer (e.g., RCC), or a breast cancer (e.g., TNBC)) does not progress or get worse. Progression-free survival may include the amount of time individuals have experienced a complete response or a partial response, as well as the amount of time individuals have experienced stable disease.

As used herein, "overall survival" or "OS" refers to the percentage of subjects in a group who are likely to be alive after a particular duration of time (e.g., 6 months, 1 year, 2 years, 3 years, 4 years, 5 years, 10 years, 15 years, 20 years, or more than 20 years from the time of diagnosis or treatment).

As used herein, "complete response" or "CR" refers to disappearance of all signs of cancer in response to treatment. This does not necessarily mean the cancer has been cured.

As used herein, "partial response" or "PR" refers to a decrease in the size of one or more tumors or lesions, or in the extent of cancer in the body, in response to treatment.

As used herein, "hazard ratio" or "HR" is a statistical definition for rates of events. For the purpose of the invention, hazard ratio is defined as representing the probability of an event (e.g., PFS or OS) in the experimental (e.g., treatment) group/arm divided by the probability of an event in the control group/arm at any specific point in time. An HR with a value of 1 indicates that the relative risk of an endpoint (e.g., death) is equal in both the "treatment" and "control" groups; a value greater than 1 indicates that the risk is greater in the treatment group relative to the control group; and a value less than 1 indicates that the risk is greater in the control group relative to the treatment group. "Hazard ratio" in progression-free survival analysis (i.e., PFS HR) is a summary of the difference between two progression-free survival curves, representing the reduction in the risk of death on treatment compared to control, over a period of follow-up. "Hazard ratio" in overall survival analysis (i.e., OS HR) is a summary of the difference between two overall survival curves, representing the reduction in the risk of death on treatment compared to control, over a period of follow-up.

By "extending survival" is meant increasing overall survival or progression free survival in a treated individual relative to an untreated individual (i.e. relative to an individual not treated with the medicament), or relative to an individual who does not express a biomarker at the designated level, and/or relative to an individual treated with an approved anti-tumor agent. An objective response refers to a measurable response, including complete response (CR) or partial response (PR).

By "reduce or inhibit" is meant the ability to cause an overall decrease of 20%, 30%, 40%, 50%, 60%, 70%, 75%, 80%, 85%, 90%, 95%, or greater. Reduce or inhibit can refer to the symptoms of the disorder being treated (e.g., a cancer, e.g., a lung cancer (e.g., NSCLC), a bladder cancer (e.g., UC), a kidney cancer (e.g., RCC), or a breast cancer (e.g., TNBc)), the presence or size of metastases, or the size of the primary tumor.

A "reference sample," "reference cell," "reference tissue," "control sample," "control cell," or "control tissue," as used herein, refers to a sample, cell, tissue, standard, or level that is used for comparison purposes. In one embodiment, a reference sample, reference cell, reference tissue, control sample, control cell, or control tissue is obtained from the same subject or individual. In another embodiment, a reference sample is obtained from one or more individuals who are not the subject or individual. In either of the preceding embodiments, the one or more individuals from which the reference sample, reference cell, reference tissue, control sample, control cell, or control tissue is obtained has a cancer. In certain embodiments, the one or more individuals from which the reference sample, reference cell, reference tissue, control sample, control cell, or control tissue is obtained has a cancer and has been previously treated with an anti-cancer therapy (e.g., one or more doses of a PD-L1 axis binding antagonist). In other embodiments, the one or more individuals from which the reference sample, reference cell, reference tissue, control sample, control cell, or control tissue is obtained has a cancer and is treatment naïve. In any of the preceding embodiments, the subject/individual and the one or more individuals who are not the subject or individual have the same cancer. In yet another embodiment, a reference sample, reference cell, reference tissue, control sample, control cell, or control tissue is obtained from a healthy and/or non-diseased part of the body (e.g., tissue or cells) of the same subject or individual. For example, healthy and/or non-diseased cells or tissue adjacent to the diseased cells or tissue (e.g., cells or tissue adjacent to a tumor). In another embodiment, a reference sample is obtained from an untreated tissue and/or cell of the body of the same subject or individual. In yet another embodiment, a reference sample, reference cell, reference tissue, control sample, control cell, or control tissue is obtained from a healthy and/or non-diseased part of the body (e.g., tissues or cells) of an individual who is not the subject or individual. In even another embodiment, a reference sample, reference cell, reference tissue, control sample, control cell, or control tissue is obtained from an untreated tissue and/or cell of the body of an individual who is not the subject or individual.

The term "sample," as used herein, refers to a composition that is obtained or derived from a subject and/or individual of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof.

As used herein, the terms "individual," "patient," and "subject" are used interchangeably and refer to any single animal, more preferably a mammal (including such non-human animals as, for example, dogs, cats, horses, rabbits, zoo animals, cows, pigs, sheep, and non-human primates) for which treatment is desired. In certain embodiments, the individual, patient, or subject is a human.

As used herein, "treatment" (and grammatical variations thereof, such as "treat" or "treating") refers to clinical intervention in an attempt to alter the natural course of the subject being treated, and can be performed either for prophylaxis or during the course of clinical pathology. Desirable effects of treatment include, but are not limited to, preventing occurrence or recurrence of a disease (e.g., a cancer, e.g., a lung cancer (e.g., NSCLC), a bladder cancer (e.g., UC), a kidney cancer (e.g., RCC), or a breast cancer (e.g., TNBC)), alleviation of symptoms, diminishment of any direct or indirect pathological consequences of the disease, preventing metastasis, decreasing the rate of disease progression, amelioration or palliation of the disease state, and remission or improved prognosis. In some embodiments, the treatments described herein are used to delay development of a disease or to slow the progression of a disease (e.g., a cancer, e.g., a lung cancer (e.g., NSCLC), a bladder cancer (e.g., UC), a kidney cancer (e.g., RCC), or a breast cancer (e.g., TNBC)). In some instances, the treatment may increase overall survival (OS) (e.g., by about 20% or greater, about 25% or greater, about 30% or greater, about 35% or greater, about 40% or greater, about 45% or greater, about 50% or greater, about 55% or greater, about 60% or greater, about 65% or greater, about 70% or greater, about 75% or greater, about 80% or greater, about 85% or greater, about 90% or greater, about 95% or greater, about 96% or greater, about 97% or greater, about 98% or greater, or about 99% or greater). In some instances, the treatment may increase OS, e.g., by about 5% to about 500%, e.g., from about 10% to about 450%, e.g., from about 20% to about 400%, e.g., from about 25% to about 350%, e.g., from about 30% to about 400%, e.g., from about 35% to about 350%, e.g., from about 40% to about 300%, e.g., from about 45% to about 250%, e.g., from about 50% to about 200%, e.g., from about 55% to about 150%, e.g., from about 60% to about 100%, e.g., from about 65% to about 100%, e.g., from about 70% to about 100%, e.g., from about 75% to about 100%, e.g., from about 80% to about 100%, e.g., from about 85% to about 100%, e.g., from about 90% to about 100%, e.g., from about 95% to about 100%, e.g., from about 98% to about 100%. In some instances, the treatment may increase the progression-free survival (PFS) (e.g., by about 20% or greater, about 25% or greater, about 30% or greater, about 35% or greater, about 40% or greater, about 45% or greater, about 50% or greater, about 55% or greater, about 60% or greater, about 65% or greater, about 70% or greater, about 75% or greater, about 80% or greater, about 85% or greater, about 90% or greater, about 95% or greater, about 96% or greater, about 97% or greater, about 98% or greater, or about 99% or greater). In some instances, the treatment may increase PFS, e.g., by about 5% to about 500%, e.g., from about 10% to about 450%, e.g., from about 20% to about 400%, e.g., from about 25% to about 350%, e.g., from about 30% to about 400%, e.g., from about 35% to about 350%, e.g., from about 40% to about 300%, e.g., from about 45% to about 250%, e.g., from about 50% to about 200%, e.g., from about 55% to about 150%, e.g., from about 60% to about 100%, e.g., from about 65% to about 100%, e.g., from about 70% to about 100%, e.g., from about 75% to about 100%, e.g., from about 80% to about 100%, e.g., from about 85% to about 100%, e.g., from about 90% to about 100%, e.g., from about 95% to about 100%, e.g., from about 98% to about 100%.

By "tissue sample" or "cell sample" is meant a collection of similar cells obtained from a tissue of a subject or individual. The source of the tissue or cell sample may be solid tissue as from a fresh, frozen, and/or preserved organ, tissue sample, biopsy, and/or aspirate; blood or any blood constituents such as plasma; bodily fluids such as cerebralspinal fluid, amniotic fluid, peritonealfluid, or interstitial fluid; cells from any time in gestation or development of the subject. The tissue sample may also be primary or cultured cells or cell lines. Optionally, the tissue or cell sample is obtained from a disease (e.g., prostate cancer, e.g., CRPC, e.g., mCRPC or locally confined, inoperable CRPC) tissue/organ. The tissue sample may contain compounds which are not naturally intermixed with the tissue in nature such as preservatives, anticoagulants, buffers, fixatives, nutrients, antibiotics, or the like.

For the purposes herein a "section" of a tissue sample is meant a single part or piece of a tissue sample, e.g. a thin slice of tissue or cells cut from a tissue sample. It is understood that multiple sections of tissue samples may be taken and subjected to analysis, provided that it is understood that the same section of tissue sample may be analyzed at both morphological and molecular levels, or analyzed with respect to both polypeptides and polynucleotides.

"Tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder," and "tumor" are not mutually exclusive as referred to herein.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

## II. DIAGNOSTIC METHODS AND ASSAYS

Provided herein are methods and assays for identifying an individual having a cancer (e.g., a lung cancer (e.g., non-small cell lung cancer (NSCLC)), a bladder cancer (e.g., a urothelial carcinoma (UC)), a kidney cancer (e.g., a renal cell carcinoma (RCC)), or a breast cancer (e.g., triple-negative breast cancer (TNBC))) who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). The methods and assays described herein are based, in part, on the finding that the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., an immune-score expression level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) in a sample from the individual may be used to determine the likelihood that the individual will benefit from treatment with a PD-L1 axis binding antagonist therapy, e.g., a PD-L1 axis binding antagonist monotherapy or combination therapy including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

Further provided herein are methods and assays for selecting a therapy for an individual having a cancer (e.g., lung cancer (e.g., NSCLC), bladder cancer (e.g., UC), kidney cancer (e.g., RCC), or breast cancer (e.g., TNBC)); methods for determining whether an individual having a cancer is likely to respond to treatment including a PD-L1 axis binding antagonist; methods for predicting the responsiveness of an individual having a cancer to treatment comprising a PD-L1 axis binding antagonist; and methods for monitoring the response of an individual having a cancer to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). Any of the methods provided herein may further include administering to the individual a PD-L1 axis binding antagonist (e.g., as described below in Section III) to the individual.

### *A. One-gene immune-scores and multi-gene immune-score combinations*

In some embodiments, the methods and assays provided herein may be used to determine an immune-score expression level of a single gene selected from CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA. For example, the determination step may include determining the expression level of any one gene selected from CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA. In some embodiments, the determination step includes determining the expression levels of a panel of two or more genes selected from CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, such as a panel of 2, 3, 4, 5, 6, 7, 8, 9, 10, 11, 12, 13, 14, 15, 16, 17, 18, 19, 20, or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., a panel that contains one or more cytotoxic genes and one or more mitochondrial genes). In some embodiments, the determination step includes determining the expression levels of a gene panel set forth in any one of Tables 1-3, above.

In some embodiments, the determination step includes determining the expression levels of a panel of genes that includes one or more cytotoxic genes (e.g., one or more of CST7, PLEK, HCST, CTSW, KLRG1, CD8A, CCL5, CD8B, NKG7, GZMH, and HLA-C). In some embodiments, the determination step includes determining the expression levels of a panel of genes that includes one or more mitochondrial genes (e.g., one or more of MT-ND4, MT-CYB, MT-CO2, MT-ND4L, and MT-ATP6). In some embodiments, the determination step includes determining the expression levels of a panel of genes that includes one or more cytotoxic genes (e.g., one or more of CST7, PLEK, HCST, CTSW, KLRG1, CD8A, CCL5, CD8B, NKG7, GZMH, and HLA-C) and one or more mitochondrial genes (e.g., one or more of MT-ND4, MT-CYB, MT-CO2, MT-ND4L, and MT-ATP6). In some embodiments, the determination step includes determining the expression levels of a gene panel set forth in Table 4, above.

For example, in one aspect, provided herein are methods for identifying an individual having a cancer (e.g., lung cancer (e.g., NSCLC), bladder cancer (e.g., UC), kidney cancer (e.g., RCC), or breast cancer (e.g., TNBC)) who may benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), the methods including determining the expression level of one or more genes selected from CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, such as a panel of 2, 3, 4, 5, 6, 7, 8, 9, 10, 11, 12, 13, 14, 15, 16, 17, 18, 19, 20, or more of the above genes that contains one or more cytotoxic genes and one or more mitochondrial genes, in a sample from the individual (e.g., a tumor tissue sample). In this context, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample that is above a reference immune-score expression level (e.g., an immune-score expression level of the same one or more genes in a reference population) identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). Alternatively, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample that is below a reference immune-score expression level (e.g., an immune-score expression level of the same one or more genes in a reference population) identifies the individual as one who is less likely to benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In another aspect, also provided herein are methods for selecting a therapy for an individual having a cancer (e.g., lung cancer (e.g., NSCLC), bladder cancer (e.g., UC), kidney cancer (e.g., RCC), or breast cancer (e.g., TNBC)), the methods including determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, such as a panel of 2, 3, 4, 5, 6, 7, 8, 9, 10, 11, 12, 13, 14, 15, 16, 17, 18, 19, 20, or more of the above genes that contains one or more cytotoxic genes and one or more mitochondrial genes, in a sample from the individual, wherein an immune-score expression level of one or more genes selected from CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample that is above a reference immune-score expression level (e.g., an immune-score expression level of the same one or more genes in a reference population) identities an individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). Alternatively, an immune-score expression level of one or more genes selected from CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample that is below a reference immune-score expression level (e.g., an immune-score expression level of the same one or more genes in a reference population) identifies the individual as one who is less likely to benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

Further provided herein are methods for determining whether an individual with a cancer (e.g., lung cancer (e.g., NSCLC), bladder cancer (e.g., UC), kidney cancer (e.g., RCC), or breast cancer (e.g., TNBC)) is likely to respond to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), the methods including determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, such as a panel of 2, 3, 4, 5, 6, 7, 8, 9, 10, 11, 12, 13, 14, 15, 16, 17, 18, 19, 20, or more of the above genes that contains one or more cytotoxic genes and one or more mitochondrial genes, in a sample from the individual (e.g., a tumor tissue sample), wherein an immune-score expression level of the one or more genes in the sample that is above a reference immune-score expression level (e.g., an immune-score expression level of the one or more genes in a reference population) indicates that the individual is likely to respond to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). Alternatively, an immune-score expression level of the one or more genes in the sample that is below a reference immune-score expression level (e.g., an immune-score expression level of the one or more genes in a reference population) indicates that the individual is less likely to respond to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

Further provided herein are methods for predicting the responsiveness of an individual with a cancer (e.g., lung cancer (e.g., NSCLC), bladder cancer (e.g., UC), kidney cancer (e.g., RCC), or breast cancer (e.g., TNBC)) to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), the methods including determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, such as a panel of 2, 3, 4, 5, 6, 7, 8, 9, 10, 11, 12, 13, 14, 15, 16, 17, 18, 19, 20, or more of the above genes that contains one or more cytotoxic genes and one or more mitochondrial genes, in a sample from the individual (e.g., tumor tissue), wherein an immune-score expression level of the one or more genes in the sample that is above a reference immune-score expression level (e.g., an immune-score expression level of the one or more genes in a reference population) indicates that the individual is more likely to be responsive to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). Alternatively, an immune-score expression level of the one or more genes in the sample that is below a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) indicates that the individual is less likely to be responsive to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

Further provided herein are methods for determining the likelihood that an individual with a cancer (e.g., lung cancer (e.g., NSCLC), bladder cancer (e.g., UC), kidney cancer (e.g., RCC), or breast cancer (e.g., TNBC)) will exhibit benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), the methods including determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample from the individual (e.g., tumor tissue), wherein an immune-score expression level of the one or more genes in the sample that is above a reference immune-score expression level (e.g., an immune-score expression level of the one or more genes in a reference population) indicates that the individual will have an increased likelihood of benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). Alternatively, an immune-score expression level of the one or more genes in the sample that is below a reference immune-score expression level (e.g., an immune-score expression level of the one or more genes in a reference population) indicates that the individual will have a decreased likelihood of benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In any of the preceding methods, the individual having a cancer (e.g., lung cancer (e.g., NSCLC), bladder cancer (e.g., UC), kidney cancer (e.g., RCC), or breast cancer (e.g., TNBC)) may be provided a recommendation prior to administration of the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), based on the immune-score expression level of one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA determined in accordance with any of the above methods. In some instances, the methods further include providing a recommendation that the individual will be likely to respond to, or benefit from, treatment with a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). In some instances, the methods include providing a recommendation that the therapy selected for the individual includes treatment with a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In any of the preceding methods, the methods may further include administering to the individual an effective amount of a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) to the individual. In some instances, the methods further include administering to the individual an effective amount of a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), wherein the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample from the individual is above a reference immune-score expression level and (e.g., an immune-score expression level of the one or more genes in a reference population). The PD-L1 axis binding antagonist may be any PD-L1 axis binding antagonist known in the art or described herein, for example, in Section III.F, below. For example, in some instances, the PD-L1 axis binding antagonist is a PD-L1 binding antagonist. In some instances, the PD-L1 binding antagonist is an antibody. In some instances, the antibody is selected from the group consisting of: YW243.55.S70, MPDL3280A (atezolizumab), MDX-1105, MEDI4736 (durvalumab), and MSB0010718C (avelumab). In some instances, the antibody comprises a heavy chain comprising HVR-H1 sequence of SEQ ID NO: 41, HVR-H2 sequence of SEQ ID NO: 42, and HVR-H3 sequence of SEQ ID NO: 43; and a light chain comprising HVR-L1 sequence of SEQ ID NO: 44, HVR-L2 sequence of SEQ ID NO: 45, and HVR-L3 sequence of SEQ ID NO: 46. In some instances, the antibody comprises a heavy chain variable region comprising the amino acid sequence of SEQ ID NO: 47 and a light chain variable region comprising the amino acid sequence of SEQ ID NO: 48.

In some instances, the methods further include administering to the individual an effective amount of an additional therapeutic agent. In some instances, the additional therapeutic agent is selected from the group consisting of a cytotoxic agent, a growth-inhibitory agent, a radiation therapy, an anti-angiogenic agent, as described herein, or a combination thereof.

Alternatively, in cases for which an individual is determined to have a decreased immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA relative to a reference immune-score expression level, the methods may further include administering to the individual an effective amount of an anti-cancer therapy other than, or in addition to, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). For example, the anti-cancer therapy other than, or in addition to, a PD-L1 axis binding antagonist may include a cytotoxic agent, a growth-inhibitory agent, a radiation therapy, an anti-angiogenic agent, as described herein, or a combination thereof, alone, or in addition to a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) and/or any additional therapeutic agent described herein.

*(i) Increased immune-score expression level of one or more of genes CST7, NKG7, GZMH, MT-ND4, HLA-H, CCL5,*

*CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA*

An immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) in a sample from the individual having cancer that is above or higher than a reference immune-score expression level of the one or more genes may indicate that the individual is more likely to benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), wherein the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population.

For example, in some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample that is in about the top $50^{th}$ percentile (equal to, or higher than, about the 50% prevalence level), about the top $45^{th}$ percentile (equal to, or higher than, about the 55% prevalence level), about the top $40^{th}$ percentile (equal to, or higher than, about the 60% prevalence level), about the top $35^{th}$ percentile (equal to, or higher than, about the 65% prevalence level), about the top $30^{th}$ percentile (equal to, or higher than, about the 70% prevalence level), about the top $25^{th}$ percentile (equal to, or higher than, about the 75% prevalence level), about the top $20^{th}$ percentile (equal to, or higher than, about the 80% prevalence level), about the top $15^{th}$ percentile (equal to, or higher than, about the 85% prevalence level), about the top $10^{th}$ percentile (equal to, or higher than, about the 90% prevalence level), about the top $5^{th}$ percentile (equal to, or higher than, about the 95% prevalence level), or about the top $1^{st}$ percentile (equal to, or higher than, about the 99% prevalence level) of the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the reference population identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample that is in about the top $50^{th}$ to about the top $99^{th}$ percentile, about the top $50^{th}$ to about the top $95^{th}$ percentile, about the top $50^{th}$ to about the top $90^{th}$ percentile, about the top $50^{th}$ to about the top $85^{th}$ percentile, about the top $50^{th}$ to about the top $80^{th}$ percentile, about the top $50^{th}$ to about the top $75^{th}$ percentile, about the top $50^{th}$ to about the top $60^{th}$ percentile, about the top $60^{th}$ to about the top $99^{th}$ percentile, about the top $60^{th}$ to about the top $95^{th}$ percentile, about the top $60^{th}$ to about the top $90^{th}$ percentile, about the top $60^{th}$ to about the top $85^{th}$ percentile, about the top $60^{th}$ to about the top $80^{th}$ percentile, about the top $60^{th}$ to about the top $75^{th}$ percentile, about the top $75^{th}$ to about the top $99^{th}$ percentile, about the top $75^{th}$ to about the top $95^{th}$ percentile, about the top $75^{th}$ to about the top $90^{th}$ percentile, about the top $75^{th}$ to about the top $85^{th}$ percentile, or about the top $75^{th}$ to about the top $80^{th}$ percentile, among others, of the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the reference population identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, an immune-score expression level that is higher than a reference immune-score expression level refers to an overall increase of about 10%, 20%, 30%, 40%, 50%, 60%, 70%, 80%, 90%, 95%, 96%, 97%, 98%, or 99% or greater in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA as detected by standard art-known methods, such as those described herein, as compared to the immune-score expression level of he one or more genes in a reference sample, reference cell, reference tissue, control sample, control cell, or control tissue. In some instances, an immune-score expression level that is higher than a reference immune-score expression level refers to an increase in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample, wherein the increase is at least about 1.5x, 1.75x, 2x, 3x, 4x, 5x, 6x, 7x, 8x, 9x, 10x, 25x, 50x, 75x, or 100x the immune-score expression level of the one or more genes in a reference sample, reference cell, reference tissue, control sample, control cell, or control tissue. In some instances, an immune-score expression level that is higher than a reference immune-score expression level refers to an overall increase in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is greater than about 1.5-fold, about 1.75-fold, about 2-fold, about 2.25-fold, about 2.5-fold, about 2.75-fold, about 3.0-fold, or about 3.25-fold as compared to the immune-score expression level of the one or more genes in a reference sample, reference cell, reference tissue, control sample, control cell, or control tissue.

In some instances, an immune-score expression level for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is higher than a reference immune-score expression level refers to an overall increase of about 10%, 20%, 30%, 40%, 50%, 60%, 70%, 80%, 90%, 95%, 96%, 97%, 98%, or 99% or greater in the immune-score expression level of the one or more genes, detected by standard art-known methods such as those described herein, as compared to a pre-assigned immune-score expression level of the one or more genes. In certain instances, an immune-score expression level for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is higher than a reference immune-score expression level refers to an increase in the immune-score expression level of the one or more genes in the sample, wherein the increase is at least about 1.5x, 1.75x, 2x, 3x, 4x, 5x, 6x, 7x, 8x, 9x, 10x, 25x, 50x, 75x, or 100x a pre-assigned immune-score expression level of the one or more genes. In some instances, an immune-score expression level for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is higher than a reference immune-score expression level refers to an overall increase in the immune-score expression level of the one or more genes that is greater than about 1.5-fold, about 1.75-fold, about 2-fold, about 2.25-fold, about 2.5-fold, about 2.75-fold, about 3.0-fold, or about 3.25-fold as compared to a pre-assigned immune-score expression level of the one or more genes.

*(ii) Decreased immune-score expression level one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA*

An immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) in a sample from the individual having cancer that is below or lower than a reference immune-score expression level of the one or more genes may indicate that the individual is less likely to benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), wherein the reference immune-score expression level is an immune-score expression level of the one or more genes in a reference population.

In some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFTIM1, IMSB10, CD247, COX2, COX1, CLIC3, S100A4, and CYBA in the sample that is in about the bottom 99$^{th}$ percentile (equal to, or lower than, about the 99% prevalence level), about the bottom 95$^{th}$ percentile (equal to, or lower than, about the 95% prevalence level), about the bottom 90$^{th}$ percentile (equal to, or lower than, about the 90% prevalence level), about the bottom 85$^{th}$ percentile (equal to, or lower than, about the 85% prevalence level), about the bottom 80$^{th}$ percentile (equal to, or lower than, about the 80% prevalence level), about the bottom 75$^{th}$ percentile (equal to, or lower than, about the 75% prevalence level), about the bottom 70$^{th}$ percentile (equal to, or lower than, about the 70% prevalence level), about the bottom 65$^{th}$ percentile (equal to, or lower than, about the 65% prevalence level), about the bottom 60$^{th}$ percentile (equal to, or lower than, about the 60% prevalence level), about the bottom 55$^{th}$ percentile (equal to, or lower than, about the 55% prevalence level), or about the bottom 50$^{th}$ percentile (equal to, or lower than, about the 50% prevalence level) of the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the reference population identifies the individual as one who is less likely to benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample that is in about the bottom 50$^{th}$ to about the bottom 99$^{th}$ percentile, about the bottom 50$^{th}$ to about the bottom 95$^{th}$ percentile, about the bottom 50$^{th}$ to about the bottom 90$^{th}$ percentile, about the bottom 50$^{th}$ to about the bottom 85$^{th}$ percentile, about the bottom 50$^{th}$ to about the bottom 80$^{th}$ percentile, about the bottom 50$^{th}$ to about the bottom 75$^{th}$ percentile, about the bottom 50$^{th}$ to about the bottom 60$^{th}$ percentile, about the bottom 60$^{th}$ to about the bottom 99$^{th}$ percentile, about the bottom 60$^{th}$ to about the bottom 95$^{th}$ percentile, about the bottom 60$^{th}$ to about the bottom 90$^{th}$ percentile, about the bottom 60$^{th}$ to about the bottom 85$^{th}$ percentile, about the bottom 60$^{th}$ to about the bottom 80$^{th}$ percentile, about the bottom 60$^{th}$ to about the bottom 75$^{th}$ percentile, about the bottom 75$^{th}$ to about the bottom 99$^{th}$ percentile, about the bottom 75$^{th}$ to about the bottom 95$^{th}$ percentile, about the bottom 75$^{th}$ to about the bottom 90$^{th}$ percentile, about the bottom 75$^{th}$ to about the bottom 85$^{th}$ percentile, or about the bottom 75$^{th}$ to about the bottom 80$^{th}$ percentile, among others, of the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the reference population identifies the individual as one who is less likely to benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, an immune-score expression level that is lower than a reference immune-score expression level refers to a decrease of about 10%, 20%, 30%, 40%, 50%, 60%, 70%, 80%, 90%, 95%, 96%, 97%, 98%, or 99% or greater in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA as detected by standard art-known methods, such as those described herein, as compared to the immune-score expression level of the one or more genes in a reference sample, reference cell, reference tissue, control sample, control cell, or control tissue. In certain instances, an immune-score expression level that is lower than a reference immune-score expression level refers to a decrease in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample, wherein the decrease is at least about 1.5x, 1.75x, 2x, 3x, 4x, 5x, 6x, 7x, 8x, 9x, 10x, 25x, 50x, 75x, or 100x the immune-score expression level of the one or more genes in a reference sample, reference cell, reference tissue, control sample, control cell, or control tissue. In some instances, an immune-score expression level that is lower than a reference immune-score expression level refers to a decrease in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is greater than about 1.5-fold, about 1.75-fold, about 2-fold, about 2.25-fold, about 2.5-fold, about 2.75-fold, about 3.0-fold, or about 3.25-fold as compared to the immune-score expression level of the one or more genes in a reference sample, reference cell,

reference tissue, control sample, control cell, or control tissue.

In some instances, an immune-score expression level that is lower than a reference immune-score expression level refers to an overall decrease of about 10%, 20%, 30%, 40%, 50%, 60%, 70%, 80%, 90%, 95%, 96%, 97%, 98%, or 99% or greater in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA as detected by standard art-known methods, such as those described herein, as compared to a pre-assigned immune-score expression level of the one or more genes. In certain instances, an immune-score expression level that is lower than a reference immune-score expression level refers to a decrease in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample, wherein the decrease is at least about 1.5x, 1.75x, 2x, 3x, 4x, 5x, 6x, 7x, 8x, 9x, 10x, 25x, 50x, 75x, or 100x relative to a pre-assigned immune-score expression level of the one or more genes. In some instances, an immune-score expression level that is lower than a reference immune-score expression level refers to an overall decrease in the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is greater than about 1 .5-fold, about 1.75-fold, about 2-fold, about 2.25-fold, about 2.5-fold, about 2.75-fold, about 3.0-fold, or about 3.25-fold as compared to a pre-assigned immune-score expression level of the one or more genes.

*(iii) Reference immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA*

The reference immune-score expression level described herein may be based on the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) in a reference population. In some instances, the reference immune-score expression level described herein is an immune-score expression level of the one or more genes in a reference population that includes two or more (e.g., two or more, three or more, four or more, or five or more) subsets of individuals.

In some instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population, wherein the reference population includes at least one subset of individuals having a cancer (e.g., lung cancer (e.g., NSCLC), bladder cancer (e.g., UC), kidney cancer (e.g., RCC), or breast cancer (e.g., TNBC)).

In some instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population, wherein the reference population includes at least one subset of individuals having a cancer (e.g., lung cancer (e.g., NSCLC), bladder cancer (e.g., UC), kidney cancer (e.g., RCC), or breast cancer (e.g., TNBC)) who have been administered one or more doses (e.g., at least one, two, three, four, five, six, seven, eight, nine, or ten or more doses) of a PD-L1 axis binding antagonist (e.g., as part of a PD-L1 axis binding antagonist monotherapy or combination therapy including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody))).

In some instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population, wherein the reference population includes at least one subset of individuals having a cancer (e.g., lung cancer (e.g., NSCLC), bladder cancer (e.g., UC), kidney cancer (e.g., RCC), or breast cancer (e.g., TNBC)) who have received treatment with a PD-L1 axis binding antagonist therapy, wherein the PD-L1 axis binding antagonist therapy is a monotherapy (e.g., a PD-L1 axis binding antagonist monotherapy including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody))).

In some instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population, wherein the reference population includes at least one subset of individuals having a cancer (e.g., lung cancer (e.g., NSCLC), bladder cancer (e.g., UC), kidney cancer (e.g., RCC), or breast cancer (e.g., TNBC)) who have received treatment with a PD-L1 axis binding antagonist therapy, wherein the PD-L1 axis binding antagonist therapy is a combination therapy (e.g., a combination therapy including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) and an additional therapeutic agent (e.g., anti-cancer therapy (e.g., a cytotoxic agent, a growth-inhibitory agent, a radiation therapy, an anti-angiogenic agent, or a combination thereof))).

In some instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLiC3, S100A4, and CYBA in a reference population, wherein the reference population includes at least one subset of individuals having a cancer (e.g., lung cancer (e.g., NSCLC), bladder cancer (e.g., UC), kidney cancer (e.g., RCC), or breast cancer (e.g., TNBC)) who have received treatment with a non-PD-L1 axis binding antagonist therapy, wherein the non-PD-L1 axis binding antagonist therapy does not include a PD-L1 axis binding antagonist and includes an anti-cancer therapy (e.g., a cytotoxic agent, a growth-inhibitory agent, a radiation therapy, an anti-angiogenic agent, or a combination thereof))).

For example, in some instances, the reference population includes a first subset of individuals who have been treated with a PD-L1 axis binding antagonist therapy (e.g., PD-L1 binding antagonist (e.g., anti-PD-L1 antibody, e.g., atezolizumab (MPDL3280A)) or PD-1 binding antagonist (e.g., anti-PD-1 antibody)) and a second subset of individuals who have been treated with a non-PD-L1 axis binding antagonist therapy, wherein the non-PD-L1 axis binding antagonist therapy does not include a PD-L1 axis binding antagonist.

In some instances, the reference immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA significantly separates each of the first and second subsets of individuals based on a significant difference between an individual's responsiveness (e.g., ORR, PFS, or OS) to treatment with the PD-L1 axis binding antagonist therapy and an individual's responsiveness to treatment with the non-PD-L1 axis binding antagonist therapy above the reference immune-score expression level, wherein the individual's responsiveness to treatment with the PD-L1 axis binding antagonist therapy is significantly improved relative to the individual's responsiveness to treatment with the non-PD-L1 axis binding antagonist therapy. For example, in some instances, the reference immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA optimally separates each of the first and second subsets of individuals based on a maximum difference between an individual's responsiveness (e.g., ORR, PFS, or OS) to treatment with the PD-L1 axis binding antagonist therapy and an individual's responsiveness to treatment with the non-PD-L1 axis binding antagonist therapy above the reference immune-score expression level, wherein the individual's responsiveness to treatment with the PD-L1 axis binding antagonist therapy is significantly improved relative to the individual's responsiveness to treatment with the non-PD-L1 axis binding antagonist therapy.

In some instances, the reference immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA significantly separates each of the first and second subsets of individuals based on a significant difference between an individual's responsiveness (e.g., ORR, PFS, or OS) to treatment with the PD-L1 axis binding antagonist therapy and an individual's responsiveness to treatment with the non-PD-L1 axis binding antagonist therapy below the reference immune-score expression level , wherein the individual's responsiveness to treatment with the non-PD-L1 axis binding antagonist therapy is significantly improved relative to the individual's responsiveness to treatment with the PD-L1 axis binding antagonist therapy. For example, in some instances, the reference immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA optimally separates each of the first and second subsets of individuals based on a maximum difference between an individual's responsiveness (e.g., ORR, PFS, or OS) to treatment with the PD-L1 axis binding antagonist therapy and an individual's responsiveness to treatment with the non-PD-L1 axis binding antagonist therapy below the reference immune-score expression level , wherein the individual's responsiveness to treatment with the non-PD-L1 axis binding antagonist therapy is significantly improved relative to the individual's responsiveness to treatment with the PD-L1 axis binding antagonist therapy.

In some instances, an optimal separation or significant separation may be based on a hazard ratio (HR) determined from an analysis of the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the first and second subsets of individuals, wherein the HR is less than 1, e.g., an HR of about 0.95, about 0.9, about 0.8, about 0.7, about 0.6, about 0.5, about 0.4, about 0.3, about 0.2, about 0.1 or lower. For example, in particular instances, an optimal separation or significant separation may be based on a hazard ratio (HR) determined from an analysis of the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the first and second subsets of individuals, wherein the upper bound of the 95% confidence interval of the HR is less than 1, e.g., an upper bound of the 95% confidence interval of the HR of about 0.95, about 0.9, about 0.8, about 0.7, about 0.6, about 0.5, about 0.4, about 0.3, about 0.2, about 0.1 or lower.

Additionally or alternatively, the reference immune-score expression level may be an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population, wherein the reference population includes at least one subset of individuals who do not have a cancer (e.g., individuals not having NSCLC, UC, RCC, or TNBC) or have cancer but are treatment naïve.

*(iv) Indications*

The compositions and methods described herein may be used, for example, to determine the therapeutic response of an individual having a cancer to treatment with a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, the cancer may be a lung cancer, a kidney cancer, a bladder cancer, a breast cancer, a colorectal cancer, an ovarian cancer, a pancreatic cancer, a gastric carcinoma, an esophageal cancer, mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy.

In certain instances, the cancer may be a lung cancer. For example, the lung cancer may be a non-small cell lung cancer (NSCLC), including but not limited to a locally advanced or metastatic (e.g., stage IIIB, stage IV, or recurrent) NSCLC. In some instances, the lung cancer (e.g., NSCLC) is unresectable/inoperable lung cancer (e.g., NSCLC). For example, the methods described herein may be used for identifying an individual having a lung cancer (e.g., NSCLC) who may benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), the methods including determining an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) in a sample from the individual (e.g., a tumor tissue sample), wherein the immune-score expression level of the one or more genes in the sample that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In certain instances, the cancer may be a bladder cancer. For example, the bladder cancer may be a urothelial carcinoma, including but not limited to a non-muscle invasive urothelial carcinoma, a muscle-invasive urothelial carcinoma, or a metastatic urothelial carcinoma. In some instances, the urothelial carcinoma is a metastatic urothelial carcinoma. For example, the methods described herein may be used for identifying an individual having a bladder cancer (e.g., UC) who may benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), the methods including determining an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample from the individual (e.g., a tumor tissue sample), wherein the immune-score expression level of the one or more genes in the sample that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In certain instances, the cancer may be a kidney cancer. In some instances, the kidney cancer may be a renal cell carcinoma (RCC), including stage I RCC, stage II RCC, stage III RCC, stage IV RCC, or recurrent RCC. For example, the methods described herein may be used for identifying an individual having a kidney cancer (e.g., RCC) who may benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), the methods including determining an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample from the individual (e.g., a tumor tissue sample), wherein the immune-score expression level of the one or more genes in the sample that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In certain instances, the cancer may be a breast cancer. For example, the breast cancer may be TNBC, estrogen receptor-positive breast cancer, estrogen receptor-positive/HER2-negative breast cancer, HER2-negative breast cancer, HER2-positive breast cancer, estrogen receptor-negative breast cancer, progesterone receptor-positive breast cancer, or progesterone receptor-negative breast cancer. In some instances, the breast cancer may be a TNBC. For example, the methods described herein may be used for identifying an individual having a breast cancer (e.g., TNBC) who may benefit from treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), the methods including determining an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample from the individual (e.g., a tumor tissue sample), wherein the immune-score expression level of the one or more genes in the sample that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, the individual having a cancer, such as a cancer described herein, has not been previously treated for the cancer (treatment naive). For example, in some instances, the individual having a cancer has not previously received a PD-L1 axis binding antagonist therapy (e.g., PD-L1 binding antagonist (e.g., anti-PD-L1 antibody, e.g., atezolizumab (MPDL3280A)) or PD-1 binding antagonist (e.g., anti-PD-1 antibody)). For example, in some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual having cancer (e.g., lung cancer (e.g., NSCLC), bladder cancer (e.g., UC), kidney cancer (e.g., RCC), or breast cancer (e.g., TNBC)) as one who may benefit from a first-line treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, the individual having a cancer has previously received treatment for the cancer. In some instances, the individual having a cancer has previously received treatment including a non-PD-L1 axis binding antagonist therapy (e.g., an anti-cancer therapy (e.g., a cytotoxic agent, a growth-inhibitory agent, a radiation therapy, an anti-angiogenic agent, or a combination thereof)). For example, in some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual having cancer (e.g., lung cancer (e.g., NSCLC), bladder cancer (e.g., UC), kidney cancer (e.g., RCC), or breast cancer (e.g., TNBC)) as one who may benefit from a second-line treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

*(v) Treatment Benefits*

An individual who benefits from receiving treatment with a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may experience, for example, a delay or prevention in the occurrence or recurrence of a cancer (e.g., a lung cancer (e.g., NSCLC), a bladder cancer (e.g., UC), a kidney cancer (e.g., RCC), or a breast cancer (e.g., TNBC)), alleviation of symptoms, diminishment of any direct or indirect pathological consequences of the cancer, prevention of metastasis, decrease in the rate of disease progression, amelioration or palliation of the disease state, or remission or improved prognosis. In some instances, the treatments described herein are used to delay development of a cancer or to slow the progression of a cancer (e.g., a lung cancer (e.g., NSCLC), a bladder cancer (e.g., UC), a kidney cancer (e.g., RCC), or a breast cancer (e.g., TNBC)). In some instances, the benefit may be an increase in overall survival (OS), progression-free survival (PFS), complete response (CR), partial response (PR), or a combination thereof.

In some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), wherein the benefit is an increase in OS, PFS, CR, PR, or a combination thereof, relative to a treatment that does not include a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), wherein the benefit is an increase in OS (e.g., by 20% or greater, 25% or greater, 30% or greater, 35% or greater, 40% or greater, 45% or greater, 50% or greater, 55% or greater, 60% or greater, 65% or greater, 70% or greater, 75% or greater, 80% or greater, 85% or greater, 90% or greater, 95% or greater, 96% or greater, 97% or greater, 98% or greater, or 99% or greater) relative to a treatment that does not include a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) identifies the individual as one who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), wherein the benefit is an increase in PFS (e.g., by 20% or greater, 25% or greater, 30% or greater, 35% or greater, 40% or greater, 45% or greater, 50% or greater, 55% or greater, 60% or greater, 65% or greater, 70% or greater, 75% or greater, 80% or greater, 85% or greater, 90% or greater, 95% or greater, 96% or greater, 97% or greater, 98% or greater, or 99% or greater) relative to a treatment that does not include a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

*B. Determination of expression levels*

*(i) Detection methods*

The immune-score expression level of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, such as 1, 2, 3, 4, 5, 6, 7, 8, 9, 10, 11, 12, 13, 14, 15, 16, 17, 18, 19, or all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., a panel of genes set forth in any one of Tables 1-4, above, and/or a panel of genes that includes one or more cytotoxic genes and one or more mitochondrial genes)) may be based on a nucleic acid expression level, and preferably, an mRNA expression level. Presence and/or expression levels/amount of the genes described herein can be determined qualitatively and/or quantitatively based on any suitable criterion known in the art, including but not limited to DNA, mRNA, cDNA, proteins, protein fragments, and/or gene copy number.

In some instances, nucleic acid expression levels of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) may be measured by polymerase chain reaction (PCR)-based assays, e.g., quantitative PCR, real-time PCR, quantitative real-time PCR (qRT-PCR), reverse transcriptase PCR (RT-PCR), and reverse transcriptase quantitative PCR (RTqPCR). Platforms for performing quantitative PCR assays include Fluidigm (e.g., BIOMARK™ HD System).

Other amplification-based methods include, for example, transcript-mediated amplification (TMA), strand displacement amplification (SDA), nucleic acid sequence based amplification (NASBA), and signal amplification methods such as bDNA.

In some instances, nucleic acid expression levels of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) also may be measured by sequencing-based techniques, such as, for example, RNA-seq, serial analysis of gene expression (SAGE), high-throughput sequencing technologies (e.g., massively parallel sequencing), and Sequenom MassARRAY® technology. Nucleic acid expression levels (e.g., expression levels of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) also may be measured by, for example, NanoString nCounter, and high-coverage expression profiling (HiCEP). Additional protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis).

Other methods for detecting nucleic acid levels of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. Hybridization of a labeled probe with a particular array member indicates that the sample from which the probe was derived expresses that gene.

Primers and probes may be labeled with a detectable marker, such as, for example, a radioisotope, fluorescent compound, bioluminescent compound, a chemiluminescent compound, metal chelator, or enzyme. Such probes and primers can be used to detect the presence of expressed genes, such as one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample. As will be understood by the skilled artisan, many different primers and probes may be prepared based on the sequences provided herein (or, in the case of genomic DNA, their adjacent sequences) and used effectively to amplify, clone, and/or determine the presence and/or expression levels of the genes described herein.

Other methods to detect nucleic acid expression levels of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) include electrophoresis, Northern and Southern blot analyses, in situ hybridization (e.g., single or multiplex nucleic acid in situ hybridization), RNAse protection assays, and microarrays (e.g., Illumina BEADARRAY™ technology; Beads Array for Detection of Gene Expression (BADGE)).

In some instances, the immune-score expression level of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) can be analyzed by a number of methodologies, including, but not limited to, RNA-seq, PCR, RT-qPCR, qPCR, multiplex qPCR, multiplex RT-qPCR, NANOSTRING® nCOUNTER® Gene Expression Assay, microarray analysis, serial analysis of gene expression (SAGE), Northern blot analysis, MassARRAY, ISH, and whole genome sequencing, or combinations thereof.

In further instances, the immune-score expression level of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) may be detected in the sample using a method selected from the group consisting of RNA-seq, RT-qPCR, qPCR, multiplex qPCR, multiplex RT-qPCR, microarray analysis, SAGE, MassARRAY technique, FACS, Western blot, ELISA, immunoprecipitation, immunohistochemistry, immunofluorescence, radioimmunoassay, dot blotting, immunodetection methods, HPLC, surface plasmon resonance, optical spectroscopy, mass spectrometery, HPLC, and ISH, or combinations thereof.

In some instances, the immune-score expression level of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) is detected using RT-qPCR. For example, in some instances, the immune-score expression level of one of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of two of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of three of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of four of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of five of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of six of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of seven of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of eight of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of nine of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of ten of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 11 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 12 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 13 of genes CST7, NKG7, GZMH, MT-ND4,

HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 14 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 15 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 16 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 17 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 18 of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 19 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 20 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 21 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 22 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 23 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 24 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 25 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 26 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 27 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 28 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 29 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 30 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 31 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of 32 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR. In some instances, the immune-score expression level of all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RT-qPCR.

In some instances, the immune-score expression level for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected using RNA-seq. For example, in some instances, the immune-score expression level of one of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of two of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression ievei of three of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of four of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of five of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of six of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of seven of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of eight of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of nine of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of ten of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 11 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 12 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 13 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 14 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 15 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 16 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 17 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 18 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 19 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 20 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 21 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 22 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 23 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 24 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 25 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 26 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 27 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 28 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 29 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 30 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 31 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of 32 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq. In some instances, the immune-score expression level of all 33 of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA is detected based on mRNA expression level(s) using RNA-seq.

*(ii) RT-qPCR*

In some instances, nucleic acid expression levels of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (such as a panel of genes set forth in any one of Tables 1-4, above, and/or a panel of genes that includes one or more cytotoxic genes and one or more mitochondrial genes)) can be detected using reverse transcription quantitative polymerase chain reaction (RT-qPCR). The technique of RT-qPCR is a form of PCR wherein the nucleic acid to be amplified is RNA that is first reverse transcribed into cDNA and the amount of PCR product is measured at each step in a PCR reaction. As RNA cannot serve as a template for PCR, the first step in gene expression profiling by PCR is the reverse transcription of the RNA template into cDNA, followed by its amplification in a PCR reaction. For example, reverse transcriptases may include avilo myeloblastosis virus reverse transcriptase (AMY-RT) or Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GENEAMP™ RNA PCR kit (Perkin Elmer, Calif, USA), following the manufacturer's instructions. The derived cDNA can then be used as a template in the subsequent PCR reaction.

A variation of the PCR technique is quantitative real time PCR (qRT-PCR), which measures PCR product accumulation through a dual-labeled fluorigenic probe (i.e., TAQMAN® probe). The technique of quantitative real time polymerase chain reaction refers to a form of PCR wherein the amount of PCR product is measured at each step in a PCR reaction. This technique has been described in various publications including Cronin et al., Am. J. Pathol. 164(l):35-42 (2004); and Ma et al., Cancer Cell 5:607-616 (2004). Real time PCR is compatible both with quantitative competitive PCR, where an internal competitor for each target sequence is used for normalization, and/or with quantitative comparative PCR using a normalization gene contained within the sample, or a housekeeping gene for PCR. For further details see, e.g. Held et al., Genome Research 6:986-994 (1996).

The steps of a representative protocol for profiling gene expression using fixed, paraffin-embedded tissues as the RNA source, including mRNA isolation, purification, primer extension and amplification are given in various published journal articles (for example: Godfrey et al., Malec. Diagnostics 2: 84-91 (2000); Specht et al., Am. J. Pathol. 158: 419-29 (2001)). Briefly, a representative process starts with cutting a section (e.g., a 10 microgram section) of a paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by PCR.

The nucleic acid expression level determined by an amplification-based method (e.g., RT-qPCR) may be expressed as a cycle threshold value (Ct). From this value, a normalized expression level for each gene can be determined, e.g., using the delta Ct (dCt) method as follows: Ct(Control/Reference Gene) - Ct(Gene of Interest/Target Gene) = dCt (Gene of Interest/Target Gene). One of skill in the art will appreciate that the dCt value obtained may be a negative dCt value or a positive dCt value. As defined herein, a higher dCt value indicates a higher expression level of the gene of interest relative to the control gene. Conversely, a lower dCt value indicates a lower expression level of the gene of interest relative to the control gene. In cases where the expression levels of a plurality of genes has been determined, the expression level for each gene, e.g., expressed as a dCt value, may then be used to determine a single value that represents an aggregate or composite expression level for the plurality of genes (e.g., an immune-score expression level). The immune-score expression level may be the mean or median of dCt values determined for each target gene/gene of interest. Thus, in some instances, the immune-score expression level described herein may be the mean or median of dCt values determined for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA. As defined herein, a higher averaged dCt or median dCt value indicates a higher aggregative expression level of the plurality of target genes relative to the control gene (or plurality of control genes). A lower averaged dCt or median dCt value indicates a lower aggregative expression level of the plurality of target genes relative to the control gene (or plurality of control genes). As described herein, an immune-score expression level may in turn be compared to a reference immune-score expression level as further defined herein.

In some embodiments, the nucleic acid expression levels described herein may be determined using a method including:

(a) obtaining or providing a sample from the individual, wherein the sample includes a tumor tissue sample (e.g., a paraffin-embedded, formalin-fixed NSCLC, UC, RCC, or TNBC tumor tissue sample);
(b) isolating mRNA from the sample;

(c) performing reverse transcription of the mRNA into cDNA (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA);

(d) amplifying the cDNA (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) using PCR; and

(e) quantifying the nucleic acid expression levels (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA).

One or more genes (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) may be detected in a single assay depending on the primers or probes used. Further, the assay may be performed across one or more tubes (e.g., one, two, three, four, five, or six or more tubes).

In some instances, the method further comprises (f) normalizing the nucleic acid expression level of the gene(s) (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) in the sample to the expression level of one or more reference genes (e.g., one, two, three, four, five, six, seven, eight, nine, or more reference genes, e.g., a housekeeping gene (e.g., TMEM55B)). For example, RT-qPCR may be used to analyze the immune-score expression level of the genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) to generate an immune-score expression level that reflects a normalized, averaged dCT value for the analyzed genes.

*(iii) RNA-seq*

In some instances, nucleic acid expression levels of the genes described herein (e.g., one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (such as a panel of genes set forth in any one of Tables 1-4, above, and/or a panel of genes that includes one or more cytotoxic genes and one or more mitochondrial genes)) can be detected using RNA-seq. RNA-seq, also called Whole Transcriptome Shotgun Sequencing (WTSS), refers to the use of high-throughput sequencing technologies to sequence and/or quantify cDNA in order to obtain information about a sample's RNA content. Publications describing RNA-Seq include: Wang et al. "RNA-Seq: a revolutionary tool for transcriptomics" Nature Reviews Genetics 10 (1): 57-63 (January 2009); Ryan et al. BioTechniques 45 (1): 81-94 (2008); and Maher et al. "Transcriptome sequencing to detect gene fusions in cancer". Nature 458 (7234): 97-101 (January 2009).

*(iv) Samples*

The samples described herein may be taken, for example, from an individual who is suspected of having, or is diagnosed as having a cancer, and hence is likely in need of treatment, or from a healthy individual who is not suspected of having a cancer or who does not have cancer but has a family history of a cancer. For assessment of gene expression, samples, such as those containing cells, or proteins or nucleic acids produced by these cells, may be used in the methods of the present invention. The expression level of a gene can be determined by assessing the amount (e.g., the absolute amount or concentration) of the markers in a sample (e.g., a tissue sample, e.g., a tumor tissue sample, such as a biopsy). In addition, the level of a gene can be assessed in bodily fluids or excretions containing detectable levels of genes. Bodily fluids or secretions useful as samples in the present invention include, e.g., blood, urine, saliva, stool, pleural fluid, lymphatic fluid, sputum, ascites, prostatic fluid, cerebrospinal fluid (CSF), or any other bodily secretion or derivative thereof. The word "blood" is meant to include whole blood, plasma, serum, or any derivative of blood. Assessment of a gene in such bodily fluids or excretions can sometimes be preferred in circumstances where an invasive sampling method is inappropriate or inconvenient. In other embodiments, a tumor tissue sample is preferred.

The sample may be frozen, fresh, fixed (e.g., formalin fixed), centrifuged, and/or embedded (e.g., paraffin embedded), etc. The cell sample can be subjected to a variety of well-known post-collection preparative and storage techniques (e.g., nucleic acid and/or protein extraction, fixation, storage, freezing, ultrafiltration, concentration, evaporation, centrifugation, etc.) prior to assessing the amount of the marker in the sample. Likewise, biopsies may also be subjected to post-collection preparative and storage techniques, e.g., fixation, such as formalin fixation.

In some embodiments, the sample is a clinical sample. In another instance, the sample is used in a diagnostic assay, such as a diagnostic assay or diagnostic method of the invention. In some instances, the sample is obtained from a primary or metastatic tumor. Tissue biopsy is often used to obtain a representative piece of tumor tissue. Alternatively, tumor cells can be obtained indirectly in the form of tissues or fluids that are known or thought to contain the tumor cells of interest. For example, samples of lung cancer lesions may be obtained by resection, bronchoscopy, fine needle aspiration, bronchial brushings, or from sputum, pleural fluid or blood. Genes or gene products can be detected from cancer or tumor tissue or from other body samples such as urine, sputum, serum or plasma. The same techniques discussed above for detection of target genes or gene products in cancerous samples can be applied to other body samples. Cancer cells may be sloughed off from cancer lesions and appear in such body samples. By screening such body samples, a simple early diagnosis can be achieved for these cancers. In addition, the progress of therapy can be monitored more easily by testing such body samples for target genes or gene products.

In some instances, the sample from the individual is a tissue sample, a whole blood sample, a plasma sample, a serum sample, or a combination thereof. In some instances, the sample is a tissue sample. In some instances, the sample is a tumor tissue sample. In some instances, the sample is obtained prior to treatment with a PD-L1 axis binding antagonist (e.g., PD-L1 binding antagonist, e.g., anti-PD-L1 antibody, e.g., atezolizumab (MPDL3280A)). In some instances, the tissue sample is formalin-fixed and paraffin-embedded (FFPE) sample, an archival sample, a fresh sample, or a frozen sample.

In some instances, the sample from the individual is a tissue sample. In some instances, the tissue sample is a tumor tissue sample (e.g., biopsy tissue). In some instances, the tumor tissue sample includes tumor cells, tumor-infiltrating immune cells, stromal cells, or a combination thereof. In some instances, the tissue sample is lung tissue. In some instances, the tissue sample is bladder tissue. In some instances, the tissue sample is renal tissue. In some instances, the tissue sample is breast tissue. In some instances, the tissue sample is skin tissue. In some instances, the tissue sample is pancreatic tissue. In some instances, the tissue sample is gastric tissue. In some instances, the tissue sample is esophageal tissue. In some instances, the tissue sample is mesothelial tissue. In some instances, the tissue sample is thyroid tissue. In some instances, the tissue sample is colorectal tissue. In some instances, the tissue sample is head or neck tissue. In some instances, the tissue sample is osteosarcoma tissue. In some instances, the tissue sample is prostate tissue. In some instances, the tissue sample is ovarian tissue, HCC (liver), blood cells, lymph nodes, or bone/bone marrow.

In some instances, the tumor tissue sample is extracted from a malignant cancerous tumor (i.e., cancer). In some instances, the cancer is a solid tumor, or a non-solid or soft tissue tumor. Examples of soft tissue tumors include leukemia (e.g., chronic myelogenous leukemia, acute myelogenous leukemia, adult acute lymphoblastic leukemia, acute myelogenous leukemia, mature B-cell acute lymphoblastic leukemia, chronic lymphocytic leukemia, polymphocytic leukemia, or hairy cell leukemia) or lymphoma (e.g., non-Hodgkin's lymphoma, cutaneous T-cell lymphoma, or Hodgkin's disease). A solid tumor includes any cancer of body tissues other than blood, bone marrow, or the lymphatic system. Solid tumors can be further divided into those of epithelial cell origin and those of non-epithelial cell origin. Examples of epithelial cell solid tumors include tumors of the gastrointestinal tract, colon, colorectal (e.g., basaloid colorectal carcinoma), breast, prostate, lung, kidney, liver, pancreas, ovary (e.g., endometrioid ovarian carcinoma), head and neck, oral cavity, stomach, duodenum, small intestine, large intestine, anus, gall bladder, labium, nasopharynx, skin, uterus, male genital organ, urinary organs (e.g., urothelium carcinoma, dysplastic urothelium carcinoma, transitional cell carcinoma), bladder, and skin. Solid tumors of non-epithelial origin include sarcomas, brain tumors, and bone tumors. In some instances, the cancer is non-small cell lung cancer (NSCLC). In some instances, the cancer is second-line or third-line locally advanced or metastatic non-small cell lung cancer. In some instances, the cancer is adenocarcinoma. In some instances, the cancer is squamous cell carcinoma.

*(v) RNA extraction*

Prior to detecting the level of a nucleic acid, mRNA may be isolated from a target sample. In some instances, the mRNA is total RNA isolated from tumors or tumor cell lines or, alternatively, normal tissues or cell lines. RNA can be isolated from a variety of tumor tissues, including breast, lung, colon, prostate, brain, liver, kidney, pancreas, stomach, gall bladder, spleen, thymus, testis, ovary, uterus, etc., the corresponding normal tissues, or tumor cell lines. If the source of mRNA is a primary tumor, mRNA can be extracted, for example, from frozen or archived paraffin-embedded and fixed (e.g. formalin-fixed) tissue samples. General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andres et al., Bio Techniques 18:42044 (1995). In particular, RNA isolation can be performed using a purification kit, buffer set, and protease from commercial manufacturers, such as Qiagen, according to the manufacturer's instructions. For example, total RNA from cells in culture can be isolated using Qiagen RNeasy mini- columns. Other commercially available RNA isolation kits include MASTERPURE® Complete DNA and RNA Purification Kit (EPICENTRE®, Madison, Wis.), and Paraffin Block RNA Isolation Kit (Ambion, Inc.). Total RNA from tissue samples can be isolated, for example, by using RNA Stat-60 (TelTest). RNA prepared from tumor tissue samples can also be isolated, for example, by cesium chloride density gradient centrifugation.

*(vi) Immune-score expression level*

The immune-score expression level may reflect the expression levels of one or more genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (such as a panel of genes set forth in any one of Tables 1-4, above, and/or a panel of genes that includes one or more cytotoxic genes and one or more mitochondrial genes)). In certain instances, to determine an immune-score expression level, the detected expression level of each gene is normalized using any one of the standard normalization methods known in the art. One of skill in the art will appreciate that the normalization method used may depend on the gene expression methodology used (e.g., one or more housekeeping genes may be used for normalization in the context of an RT-qPCR methodology, but a whole genome or substantially whole genome may be used as a normalization baseline in the context of an RNA-seq methodology). For example, the detected expression level of each gene assayed can be normalized for both differences in the amount of the gene(s) assayed, variability in the quality of the samples used, and/or variability between assay runs.

In some instances, normalization may be accomplished by detecting expression of certain one or more normalizing gene(s), including reference gene(s) (e.g., a housekeeping). For example, in some instances, the nucleic acid expression levels detected using the methods described herein (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) may be normalized to the expression level of one or more reference genes (e.g., one, two, three, four, five, six, seven, eight, nine, or more reference genes, e.g., a housekeeping gene (e.g., TMEM55B)). Alternatively, normalization can be based on the average signal or median signal of all of the assayed genes. On a gene-by-gene basis, a measured normalized amount of a subject tumor mRNA can be compared to the amount found in a reference immune-score expression level. The presence and/or expression level/amount measured in a particular subject sample to be analyzed will fall at some percentile within this range, which can be determined by methods well known in the art.

In some instances, to determine an immune-score expression level, the detected expression level of each assayed gene is not normalized.

The immune-score expression level may reflect the aggregate or composite expression level of a single gene or a plurality of genes described herein (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA). Any statistical approaches known in the art may be used to determine the immune-score expression level.

For example, the immune-score expression level may reflect the median expression level, mean expression level, or a numerical value that reflects the aggregated Z-score expression level for the combination of genes assayed (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA).

In some instances, the immune-score expression level reflects the median normalized expression level, mean normalized expression level, or a numerical value that reflects the aggregated Z-score normalized expression level for the combinations of genes assayed (e.g., for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA).

For example, the immune-score expression level may reflect an average (mean) of the expression levels of each gene in a combination of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., an average of the expression levels of 2, 3, 4, 5, 6, 7, 8, 9, 10, 11, 12, 13, 14, 15, 16, 17, 18, 19, 20, or more, of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA). In some instances, the immune-score expression level reflects an average (mean) of the normalized expression levels of each gene in a combination of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., normalized to a reference gene, e.g., a housekeeping gene, e.g., TMEM55B). In some instances, the immune-score expression level reflects a median of the expression levels of each gene in a combination of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., a median of the expression levels of each gene in a combination of 2, 3, 4, 5, 6, 7, 8, 9, 10, 11, 12, 13, 14, 15, 16, 17, 18, 19, 20, or more, of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA). In some instances, the immune-score expression level reflects a median of the normalized expression levels of each gene in a combination of two of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., normalized to a reference gene, e.g., a housekeeping gene, e.g., TMEM55B). In some instances, the immune-score expression level reflects the Z-score for each gene in a combination of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., the Z-score for each gene in a combination of 2, 3, 4, 5, 6, 7, 8, 9, 10, 11, 12, 13, 14, 15, 16, 17, 18, 19, 20, or more, of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA).

*(vii) Reference immune-score expression level*

The reference immune-score expression level may be a value derived from analysis of any of the reference populations described herein. In some instances, the reference immune-score expression level may be a "cut-off" value selected based on a reference immune-score expression level that divides a reference population into subsets, e.g., subsets that exhibit significant differences (e.g., statistically significant differences) in treatment response to a PD-L1 axis binding antagonist therapy and a non-PD-L1 axis binding antagonist therapy. In such instances, relative treatment response may be evaluated based on progression-free survival (PFS) or overall survival (OS), expressed for example as a hazard ratio (HR) (e.g., progression-free survival HR (PFS HR) or overall survival HR (OS HR)).

In certain instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) in a reference population that significantly (e.g., statistically significantly) separates a first subset of individuals who have been treated with a PD-L1 axis binding antagonist (e.g., a PD-L1 binding antagonist (e.g., anti-PD-L1 antibody, e.g., atezolizumab (MPDL3280A)) or a PD-1 binding antagonist (e.g., anti-PD-1 antibody)) therapy in a reference population and a second subset of individuals who have been treated with a non-PD-L1 axis binding antagonist therapy that does not comprise a PD-L1 axis binding antagonist in the same reference population based on a significant difference between an individual's responsiveness to treatment with the PD-L1 axis binding antagonist therapy and an individual's responsiveness to treatment with the non-PD-L1 axis binding antagonist therapy above the reference immune-score expression level (i.e., above the cut-off), wherein the individual's responsiveness to treatment with the PD-L1 axis binding antagonist therapy is significantly improved relative to the individual's responsiveness to treatment with the non-PD-L1 axis binding antagonist therapy.

In some instances, the reference immune-score expression level is an immune-score expression level of 1, 2, 3, 4, 5, 6, 7, 8, 9, 10, 11, 12, 13, 14, 15, 16, 17, 18 19, 20, 21, 22, 23, 24, 25, 26, 27, 28, 29, 30, 31, 32, or all 33, of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population that substantially optimally separates a first subset of individuals who have been treated with a PD-L1 axis binding antagonist (e.g., a PD-L1 binding antagonist (e.g., anti-PD-L1 antibody, e.g., atezolizumab (MPDL3280A)) or a PD-1 binding antagonist (e.g., anti-PD-1 antibody)) therapy in a reference population and a second subset of individuals who have been treated with a non-PD-L1 axis binding antagonist therapy that does not comprise a PD-L1 axis binding antagonist in the same reference population based on a substantially maximal difference between an individual's responsiveness to treatment with the PD-L1 axis binding antagonist therapy and an individual's responsiveness to treatment with the non-PD-L1 axis binding antagonist therapy above the reference immune-score expression level (i.e., above the cut-off), wherein the individual's responsiveness to treatment with the PD-L1 axis binding antagonist therapy is significantly (e.g., statistically significantly) improved relative to the individual's responsiveness to treatment with the non-PD-L1 axis binding antagonist therapy.

In certain particular instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population that optimally separates a first subset of individuals who have been treated with a PD-L1 axis binding antagonist (e.g., a PD-L1 binding antagonist (e.g., anti-PD-L1 antibody, e.g., atezolizumab (MPDL3280A)) or a PD-1 binding antagonist (e.g., anti-PD-1 antibody)) therapy in a reference population and a second subset of individuals who have been treated with a non-PD-L1 axis binding antagonist therapy that does not comprise a PD-L1 axis binding antagonist in the same reference population based on a maximal difference between an individual's responsiveness to treatment with the PD-L1 axis binding antagonist therapy and an individual's responsiveness to treatment with the non-PD-L1 axis binding antagonist therapy above the reference immune-score expression level (i.e., above the cut-off), wherein the individual's responsiveness to treatment with the PD-L1 axis binding antagonist therapy is significantly (e.g., statistically significantly) improved relative to the individual's responsiveness to treatment with the non-PD-L1 axis binding antagonist therapy.

In certain instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population that significantly (e.g., statistically significantly) separates a first subset of individuals who have been treated with a PD-L1 axis binding antagonist (e.g., a PD-L1 binding antagonist (e.g., anti-PD-L1 antibody, e.g., atezolizumab (MPDL3280A)) or a PD-1 binding antagonist (e.g., anti-PD-1 antibody)) therapy in a reference population and a second subset of individuals who have been treated with a non-PD-L1 axis binding antagonist therapy that does not comprise a PD-L1 axis binding antagonist in the same reference population based on a significant difference between an individual's responsiveness to treatment with the PD-L1 axis binding antagonist therapy and an individual's responsiveness to treatment with the non-PD-L1 axis binding antagonist therapy below the reference immune-score expression level (i.e., below the cut-off), wherein the individual's responsiveness to treatment with the non-PD-L1 axis binding antagonist therapy is significantly (e.g., statistically significantly) improved relative to the individual's responsiveness to treatment with the PD-L1 axis binding antagonist therapy.

In some instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population that substantially optimally separates a first subset of individuals who have been treated with a PD-L1 axis binding antagonist (e.g., a PD-L1 binding antagonist (e.g., anti-PD-L1 antibody, e.g., atezolizumab (MPDL3280A)) or a PD-1 binding antagonist (e.g., anti-PD-1 antibody)) therapy in a reference population and a second subset of individuals who have been treated with a non-PD-L1 axis binding antagonist therapy that does not comprise a PD-L1 axis binding antagonist in the same reference population based on a substantially maximal difference between an individual's responsiveness to treatment with the PD-L1 axis binding antagonist therapy and an individual's responsiveness to treatment with the non-PD-L1 axis binding antagonist therapy below the reference immune-score expression level (i.e., below the cut-off), wherein the individual's responsiveness to treatment with the non-PD-L1 axis binding antagonist therapy is significantly (e.g., statistically significantly) improved relative to the individual's responsiveness to treatment with the PD-L1 axis binding antagonist therapy.

In certain particular instances, the reference immune-score expression level is an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population that optimally separates a first subset of individuals who have been treated with a PD-L1 axis binding antagonist (e.g., a PD-L1 binding antagonist (e.g., anti-PD-L1 antibody, e.g., atezolizumab (MPDL3280A)) or a PD-1 binding antagonist (e.g., anti-PD-1 antibody)) therapy in a reference population and a second subset of individuals who have been treated with a non-PD-L1 axis binding antagonist therapy that does not comprise a PD-L1 axis binding antagonist in the same reference population based on a maximal difference between an individual's responsiveness to treatment with the PD-L1 axis binding antagonist therapy and an individual's responsiveness to treatment with the non-PD-L1 axis binding antagonist therapy below the reference immune-score expression level (i.e., below the cut-off), wherein the individual's responsiveness to treatment with the non-PD-L1 axis binding antagonist therapy is significantly (e.g., statistically significantly) improved relative to the individual's responsiveness to treatment with the PD-L1 axis binding antagonist therapy.

The reference immune-score expression level may be determined from any number of individuals in a reference population and/or any number of reference samples (e.g., reference cell, reference tissue, control sample, control cell, or control tissue). The reference sample may be a single sample or a combination of multiple samples. A reference immune-score expression level based on a reference sample may be based on any number of reference samples (e.g., 2 or more, 5 or more, 10 or more, 50 or more, 100 or more, 500 or more, or 1000 or more reference samples). In certain instances, a reference sample includes pooled mRNA samples derived from samples obtained from multiple individuals. Further, a reference immune-score expression level based on a reference population, or samples therefrom, may be based on any number of individuals in the reference population (e.g., 2 or more, 5 or more, 10 or more, 50 or more, 100 or more, 500 or more, or 1000 or more individuals in a reference population). Any statistical methods known in the art may be used to determine a reference immune-score expression level from measurements based on multiple samples or multiple individuals in a reference population. See e.g., Sokal R. R. and Rholf, F. J. (1995) "Biometry: the principles and practice of statistics in biological research," W.H. Freeman and Co. New York, N.Y.

*(viii) Reference population*

The reference immune-score expression level may reflect the expression level(s) of one or more genes described herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (such as a panel of genes set forth in any one of Tables 1-4, above, and/or a panel of genes that includes one or more cytotoxic genes and one or more mitochondrial genes)) in one or more reference populations (or reference samples), or as a pre-assigned reference value.

In some instances, the reference immune-score expression level is an immune-score expression level for one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population.

In some instances, the reference population is a population of individuals having a cancer. In some instances, the reference population is a population of individuals having lung cancer (e.g., NSCLC). In some instances, the reference population is a population of individuals having kidney cancer (e.g., RCC). In some instances, the reference population is a population of individuals having bladder cancer (e.g., UC). In some instances, the reference population is a population of individuals having breast cancer (e.g., TNBC). In some instances, the reference population is a population of individuals who do not have a cancer.

Further, the reference population may include one or more subsets of individuals (e.g., one or more, two or more, three or more, four or more, five or more, six or more, seven or more, eight or more, nine or more, or ten or more subsets).

In some instances, the reference population is a population of individuals having the cancer, wherein the population of individuals includes a subset of individuals who have been treated with at least one dose (e.g., at least one, at least two, at least three, at least four, at least five, at least six, at least seven, at least eight, at least nine, at least ten, or more than ten doses) of a therapy including a PD-L1 axis binding antagonist (e.g., PD-L1 binding antagonist

(e.g., anti-PD-L1 antibody, e.g., atezolizumab (MPDL3280A)) or PD-1 binding antagonist (e.g., anti-PD-1 antibody)). In some instances, the therapy including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) is a monotherapy. In other instances, the therapy including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) is a combination treatment that includes, in addition to the PD-L1 axis binding antagonist, at least one additional therapeutic agent (e.g., an anti-cancer therapy (e.g., an anti-neoplastic agent, a chemotherapeutic agent, a growth inhibitory agent, a cytotoxic agent, a radiotherapy, or combinations thereof)).

In some instances, the reference population is a population of individuals having the cancer, wherein the population of individuals includes a subset of individuals who have been treated with a non-PD-L1 axis binding antagonist therapy (e.g., an anti-cancer therapy, (e.g., an anti-neoplastic agent, a chemotherapeutic agent, a growth inhibitory agent, a cytotoxic agent, a radiotherapy, or combinations thereof)) that does not include a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, the reference population includes a combination of individuals from different subsets. For example, in some instances, the reference population may be a population of individuals having the cancer, the population of individuals consisting of (i) a first subset of individuals who have been treated with a PD-L1 axis binding antagonist therapy (e.g., a PD-L1 binding antagonist therapy) that includes a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) and (ii) a second subset of individuals who have been treated with a non-PD-L1 axis binding antagonist therapy (e.g., a non-PD-L1 binding antagonist therapy) that does not include a PD-L1 axis binding antagonist (e.g., an anti-cancer therapy (e.g., an anti-neoplastic agent, a chemotherapeutic agent, a growth inhibitory agent, a cytotoxic agent, a radiotherapy, or combinations thereof). The PD-L1 axis binding antagonist therapy (e.g., a PD-L1 binding antagonist therapy) in the first subset may have been administered as either a monotherapy or a combination therapy.

## III. METHODS OF TREATMENT

Using the compositions and methods of the disclosure, a chimeric antigen receptor (CAR) T cell may be designed based on the T cell receptor (TCR) profile that a patient exhibits in peripheral circulation. As is described in further detail in the Examples below, it has presently been discovered that the presence of clonally expanded T cells in the peripheral blood of a patient having cancer (e.g., lung cancer (e.g., NSCLC), endometrial cancer, colon adenocarcinoma, renal cell cancer, bladder cancer (e.g., UC), kidney cancer (e.g., RCC), or breast cancer (e.g., TNBC)) specifically bind to, and mount an immune response against, tumor tissue. Based on this discovery, one can look to a cancer patient's circulating TCR profile to inform the design of CARs that may be included in a CAR T cancer therapy.

For example, using the compositions and methods of the disclosure, one may withdraw a sample of blood from a patient having cancer (e.g., lung cancer (e.g., NSCLC), bladder cancer (e.g., UC), kidney cancer (e.g., RCC), or breast cancer (e.g., TNBC)) and may subsequently isolate the T cells from the sample. One may then determine the amino acid sequence of one or more TCR alpha and beta chains expressed by the T cells and/or the nucleic acid sequence encoding one or more of the TCR alpha and beta chains. Using this information, one may design a CAR T cell containing a CAR containing one or more CDRs, alpha chains, and/or beta chains having the amino acid sequence of a corresponding CDR, alpha chain, and/or beta chain within the TCR identified from the blood sample obtained from the patient.

CAR T cells can be produced by engineering a precursor cell, such as a T lymphocyte, to express a CAR that is specific for a target antigen, such as a tumor-associated antigen. The identification of TCRs expressed within the blood sample obtained from the patient can inform the design and construction of CARs Structurally, CARs contain a cytoplasmic and transmembrane domain from a naturally-occurring TCR. The cytoplasmic and transmembrane domains may be operably joined to an extracellular antibody fragment, such as an scFv fragment, that specifically binds to a particular antigenic peptide. T cells can be genetically modified in order to express an antigen receptor that specifically binds to a particular tumor antigen by any of a variety of genome editing techniques described herein or known in the art. Exemplary techniques for modifying a T cell genome so as to incorporate a gene encoding a chimeric antigen receptor include the CRISPR/Cas, zinc finger nuclease, TALEN, and ARCUS™ platforms. Methods for the genetic engineering of CAR T lymphocytes have been described, e.g., in WO 2014/127261, WO 2014/039523, WO 2014/099671, and WO 2012/079000; the disclosures of each of which are incorporated by reference herein.

CAR T cells that may be used in conjunction with the compositions and methods described herein include those that have been genetically modified such that the cell does not express its endogenous T cell receptor. For instance, a CAR T cell may be modified by genome-editing techniques, such as those described herein, so as to suppress expression of the endogenous T cell receptor in order to prevent graft-versus-host reactions in a patient receiving a CAR T infusion. Additionally, or alternatively, CAR T cells can be genetically modified so as to reduce the expression of one or more endogenous major histocompatibility complex (MHC) proteins. This is a particularly useful technique for the infusion of allogeneic T lymphocytes, as recognition of foreign MHC proteins represents one mechanism that promotes allograft rejection. One of skill in the art can also modify a T lymphocyte so as to suppress the expression of immune suppressor proteins that, when activated, attenuate T cell activation. Infusion of CAR T cells that have been genetically modified so as to diminish the expression of one or more immunosuppressor proteins represents one strategy that can be used to prolong the T lymphocyte-mediated cytotoxicity *in vivo.*

Also provided herein are methods, medicaments, and uses thereof, for treating an individual having a cancer (e.g., lung cancer (e.g., NSCLC), bladder cancer (e.g., UC), kidney cancer (e.g., RCC), or breast cancer (e.g., TNBC)), the methods including administering to the individual an effective amount of a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) based on expression levels of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) that have been determined in a sample from the individual.

For example, in one aspect, provided herein are methods for treating an individual having a cancer (e.g., lung cancer (e.g., NSCLC), bladder cancer (e.g., UC), kidney cancer (e.g., RCC), or breast cancer (e.g., TNBC)), the methods including (a) determining the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample from the individual, wherein an immune-score expression level of the one or more genes in the sample has been determined to be above a reference immune-score expression (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population), and (b) administering an effective amount of a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) to the individual based on the immune-score expression level of the one or more genes determined in step (a).

In another aspect, provided herein are methods for treating an individual having a cancer, the methods including administering to the individual an effective amount of a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), wherein prior to treatment, the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample from the individual has been determined and an immune-score expression level of the one or more genes in the sample that is above a reference immune-score expression level (e.g., an immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1 , KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a reference population) has been determined.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered as a first-line therapy. Alternatively, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered as a second-line therapy.

*A. Single-gene and multi-gene immune-scores*

In particular instances, the methods and medicaments provided herein may be used to treat an individual having a cancer (e.g., lung cancer (e.g., NSCLC), bladder cancer (e.g., UC), kidney cancer (e.g., RCC), or breast cancer (e.g., TNBC)) based on a determination of the immune-score expression levels of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA. In some embodiments, the determination is based on an immune-score expression level of a combination of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA, such as a combination of 2, 3, 4, 5, 6, 7, 8, 9, 10, 11, 12, 13, 14, 15, 16, 17, 18, 19, 20, or more, of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA. In some embodiments, the determination is based on an immune-score expression level of a panel of genes set forth in any one of Tables 1-4, above, and/or a panel of genes that includes one or more cytotoxic genes and one or more mitochondrial genes.

*B. PD-L1 Axis Binding Antagonist*

PD-L1 axis binding antagonists include PD-1 binding antagonists, PD-L1 binding antagonists, and PD-L2 binding antagonists. PD-1 (programmed death 1) is also referred to in the art as "programmed cell death 1," "PDCD1," "CD279," and "SLEB2." An exemplary human PD-1 is shown in UniProtKB/Swiss-Prot Accession No. Q15116. PD-L1 (programmed death ligand 1) is also referred to in the art as "programmed cell death 1 ligand 1," "PDCD1LG1," "CD274," "B7-H," and "PDL1." An exemplary human PD-L1 is shown in UniProtKB/Swiss-Prot Accession No.Q9NZQ7.1. PD-L2 (programmed death ligand 2) is also referred to in the art as "programmed cell death 1 ligand 2," "PDCD1LG2," "CD273," "B7-DC," "Btdc," and "PDL2." An exemplary human PD-L2 is shown in UniProtKB/Swiss-Prot Accession No. Q9BQ51. In some embodiments, PD-1, PD-L1, and PD-L2 are human PD-1, PD-L1, and PD-L2. The PD-1 axis binding antagonist may, in some instances, be a PD-1 binding antagonist, a PD-L1 binding antagonist, or a PD-L2 binding antagonist.

*(i) PD-L1 binding antagonist*

In some instances, the PD-L1 binding antagonist inhibits the binding of PD-L1 to one or more of its ligand binding partners. In other instances, the PD-L1 binding antagonist inhibits the binding of PD-L1 to PD-1. In yet other instances, the PD-L1 binding antagonist inhibits the binding of PD-L1 to B7-1. In some instances, the PD-L1 binding antagonist inhibits the binding of PD-L1 to both PD-1 and B7-1. In some instances, the PD-L1 binding antagonist is an antibody. In some instances, the antibody is selected from the group consisting of: YW243.55.S70, MPDL3280A (atezolizumab), MDX-1105, MEDI4736 (durvalumab), and MSB0010718C (avelumab).

In some instances, the anti-PD-L1 antibody is a monoclonal antibody. In some instances, the anti-PD-L1 antibody is an antibody fragment selected from the group consisting of Fab, Fab'-SH, Fv, scFv, and $(Fab')_2$ fragments. In some instances, the anti-PD-L1 antibody is a humanized antibody. In some instances, the anti-PD-L1 antibody is a human antibody. In some instances, the anti-PD-L1 antibody described herein binds to human PD-L1. In some particular instances, the anti-PD-L1 antibody is atezolizumab (CAS Registry Number: 1422185-06-5). Atezolizumab (Genentech) is also known as MPDL3280A.

In some instances, the anti-PD-L1 antibody comprises a heavy chain variable region (HVR-H) comprising an HVR-H1, HVR-H2, and HVR-H3 sequence, wherein:

(a) the HVR-H1 sequence is GFTFSDSWIH (SEQ ID NO: 41);
(b) the HVR-H2 sequence is AWISPYGGSTYYADSVKG (SEQ ID NO: 42); and
(c) the HVR-H3 sequence is RHWPGGFDY (SEQ ID NO: 43).

In some instances, the anti-PD-L1 antibody further comprises a light chain variable region (HVR-L) comprising an HVR-L1, HVR-L2, and HVR-L3 sequence, wherein:

(a) the HVR-L1 sequence is RASQDVSTAVA (SEQ ID NO: 44);
(b) the HVR-L2 sequence is SASFLYS (SEQ ID NO: 45); and
(c) the HVR-L3 sequence is QQYLYHPAT (SEQ ID NO: 46).

In some instances, the anti-PD-L1 antibody comprises a heavy chain and a light chain sequence, wherein:

(a) the heavy chain variable (VH) region sequence comprises the amino acid sequence:

EVQLVESGGGLVQPGGSLRLSCAASGFTFSDSWIHWVRQAPGKGLEWVAWISPYGGSTYYADSVKGRF
TISADTSKNTAYLQMNSLRAEDTAVYYCARRHWPGGFDYWGQGTLVTVSS (SEQ ID NO: 47);

and

(b) the light chain variable (VL) region sequence comprises the amino acid sequence:

DIQMTQSPSSLSASVGDRVTITCRASQDVSTAVAWYQQKPGKAPKLLIYSASFLYSGVPSRFSGSGSGTD FTLTISSLQPEDFATYYCQQYLYHPATFGQGTKVEIKR (SEQ ID NO: 48).

In some instances, the anti-PD-L1 antibody comprises a heavy chain and a light chain sequence, wherein:

(a) the heavy chain comprises the amino acid sequence:

EVQLVESGGGLVQPGGSLRLSCAASGFTFSDSWIHWVRQAPGKGLEWVAWISPYGGSTYYADSVKGRF TISADTSKNTAYLQMNSLRAEDTAVYYCARRHWPGGFDYWGQGTLVTVSSASTKGPSVFPLAPSSKSTS GGTAALGCLVKDYFPEPVTVSWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLMISRTPEVTCVVVDVSHEDPEVKFN WYVDGVEVHNAKTKPREEQYASTYRVVSVLTVLHQDWLNGKEYKCKVSNKALPAPIEKTISKAKGQPRE PQVYTLPPSREEMTKNQVSLTCLVKGFYPSDIAVEWESNGQPENNYKTTPPVLDSDGSFFLYSKLTVDKS RWQQGNVFSCSVMHEALHNHYTQKSLSLSPG (SEQ ID NO: 49);

and

(b) the light chain comprises the amino acid sequence:

DIQMTQSPSSLSASVGDRVTITCRASQDVSTAVAWYQQKPGKAPKLLIYSASFLYSGVPSRFSGSGSGTD FTLTISSLQPEDFATYYCQQYLYHPATFGQGTKVEIKRTVAAPSVFIFPPSDEQLKSGTASVVCLLNNFYP REAKVQWKVDNALQSGNSQESVTEQDSKDSTYSLSSTLTLSKADYEKHKVYACEVTHQGLSSPVTKSFN RGEC (SEQ ID NO: 50).

In some instances, the anti-PD-L1 antibody comprises (a) a VH domain comprising an amino acid sequence comprising having at least 95% sequence identity (e.g., at least 95%, 96%, 97%, 98%, or 99% sequence identity) to, or the sequence of (SEQ ID NO: 47); (b) a VL domain comprising an amino acid sequence comprising having at least 95% sequence identity (e.g., at least 95%, 96%, 97%, 98%, or 99% sequence identity) to, or the sequence of (SEQ ID NO: 48); or (c) a VH domain as in (a) and a VL domain as in (b). In other instances, the anti-PD-L1 antibody is selected from the group consisting of YW243.55.S70, MDX-1105, MEDI4736 (durvalumab), and MSB0010718C (avelumab). Antibody YW243.55.S70 is an anti-PD-L1 described in PCT Pub. No. WO 2010/077634. MDX-1105, also known as BMS-936559, is an anti-PD-L1 antibody described in PCT Pub. No. WO 2007/005874. MEDI4736 (durvalumab) is an anti-PD-L1 monoclonal antibody described in PCT Pub. No. WO 2011/066389 and U.S. Pub. No. 2013/034559. Examples of anti-PD-L1 antibodies useful for the methods of this invention, and methods for making thereof are described in PCT Pub. Nos. WO 2010/077634, WO 2007/005874, and WO 2011/066389, and also in U.S. Pat. No. 8,217,149, and U.S. Pub. No. 2013/034559, which are incorporated herein by reference.

*(ii) PD-1 binding antagonist*

In some instances, the PD-L1 axis binding antagonist is a PD-1 binding antagonist. For example, in some instances, the PD-1 binding antagonist inhibits the binding of PD-1 to one or more of its ligand binding partners. In some instances, the PD-1 binding antagonist inhibits the binding of PD-1 to PD-L1. In other instances, the PD-1 binding antagonist inhibits the binding of PD-1 to PD-L2. In yet other instances, the PD-1 binding antagonist inhibits the binding of PD-1 to both PD-L1 and PD-L2. In some instances, the PD-1 binding antagonist is an antibody. In some instances, the antibody is selected from the group consisting of: MDX 1106 (nivolumab), MK-3475 (pembrolizumab), MEDI-0680 (AMP-514), PDR001, REGN2810, and BGB-108. In some instances, the PD-1 binding antagonist is an Fc-fusion protein. For example, in some instances, the Fc-fusion protein is AMP-224.

In a further aspect, the invention provides for the use of a PD-L1 axis binding antagonist in the manufacture or preparation of a medicament. In one embodiment, the medicament is for treatment of a cancer. In a further embodiment, the medicament is for use in a method of treating a cancer comprising administering to a patient suffering from kidney cancer (e.g., a renal cell carcinoma (RCC), e.g., advanced RCC or metastatic RCC (mRCC), e.g., previously untreated advanced RCC or mRCC) an effective amount of the medicament. In one such embodiment, the method further comprises administering to the individual an effective amount of at least one additional therapeutic agent, e.g., as described below.

In some embodiments, the PD-1 binding antagonist is a molecule that inhibits the binding of PD-1 to its ligand binding partners. In a specific aspect the PD-1 ligand binding partners are PD-L1 and/or PD-L2. In another embodiment, a PD-L1 binding antagonist is a molecule that inhibits the binding of PD-L1 to its binding ligands. In a specific aspect, PD-L1 binding partners are PD-1 and/or B7-1. In another embodiment, the PD-L2 binding antagonist is a molecule that inhibits the binding of PD-L2 to its ligand binding partners. In a specific aspect, the PD-L2 binding ligand partner is PD-1. The antagonist may be an antibody, an antigen binding fragment thereof, an immunoadhesin, a fusion protein, or oligopeptide.

In some embodiments, the PD-1 binding antagonist is an anti-PD-1 antibody (e.g., a human antibody, a humanized antibody, or a chimeric antibody), for example, as described below. In some embodiments, the anti-PD-1 antibody is selected from the group consisting of MDX-1106 (nivolumab), MK-3475 (pembrolizumab), MEDI-0680 (AMP-514), PDR001, REGN2810, and BGB-108. MDX-1106, also known as MDX-1106-04, ONO-4538, BMS-936558, or nivolumab, is an anti-PD-1 antibody described in WO2006/121168. MK-3475, also known as pembrolizumab or lambrolizumab, is an anti-PD-1 antibody described in WO 2009/114335. In some embodiments, the PD-1 binding antagonist is an immunoadhesin (e.g., an immunoadhesin comprising an extracellular or PD-1 binding portion of PD-L1 or PD-L2 fused to a constant region (e.g., an Fc region of an immunoglobulin sequence). In some embodiments, the PD-1 binding antagonist is AMP-224. AMP-224, also known as B7-DCIg, is a PD-L2-Fc fusion soluble receptor described in WO 2010/027827 and WO 2011/066342.

In some embodiments, the anti-PD-1 antibody is MDX-1106. Alternative names for "MDX-1106" include MDX-1106-04, ONO-4538, BMS-936558, and nivolumab. In some embodiments, the anti-PD-1 antibody is nivolumab (CAS Registry Number: 946414-94-4). In a still further embodiment, provided is an isolated anti-PD-1 antibody comprising a heavy chain variable region comprising the heavy chain variable region amino acid sequence from SEQ ID NO: 51 and/or a light chain variable region comprising the light chain variable region amino acid sequence from SEQ ID NO: 52.

In a still further embodiment, provided is an isolated anti-PD-1 antibody comprising a heavy chain and/or a light chain sequence, wherein:

(a) the heavy chain sequence has at least 85%, at least 90%, at least 91%, at least 92%, at least 93%, at least 94%, at least 95%, at least 96%, at least 97%, at least 98%, at least 99% or 100% sequence identity to the heavy chain sequence:

QVQLVESGGGVVQPGRSLRLDCKASGITFSNSGMHWVRQAPGKGLEWVAVIWYDGSKRYYADSVKGR
FTISRDNSKNTLFLQMNSLRAEDTAVYYCATNDDYWGQGTLVTVSSASTKGPSVFPLAPCSRSTSESTAA
LGCLVKDYFPEPVTVSWNSGALTSGVHTFPAVLQSSGLYSLSSVVTVPSSSLGTKTYTCNVDHKPSNTK
VDKRVESKYGPPCPPCPAPEFLGGPSVFLFPPKPKDTLMISRTPEVTCVVVDVSQEDPEVQFNWYVDGV
EVHNAKTKPREEQFNSTYRVVSVLTVLHQDWLNGKEYKCKVSNKGLPSSIEKTISKAKGQPREPQVYTLP
PSQEEMTKNQVSLTCLVKGFYPSDIAVEWESNGQPENNYKTTPPVLDSDGSFFLYSRLTVDKSRWQEG
NVFSCSVMHEALHNHYTQKSLSLSLGK (SEQ ID NO: 51),

and

(b)the light chain sequences has at least 85%, at least 90%, at least 91%, at least 92%, at least 93%, at least 94%, at least 95%, at least 96%, at least 97%, at least 98%, at least 99% or 100% sequence identity to the light chain sequence:

EIVLTQSPATLSLSPGERATLSCRASQSVSSYLAWYQQKPGQAPRLLIYDASNRATGIPARFSGSGSGTD
FTLTISSLEPEDFAVYYCQQSSNWPRTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 52).

*(iii) Substitution, Insertion, and Deletion Variants*

In certain instances, the anti-PD-L1 antibody (e.g., atezolizumab (MPDL3280A) variants having one or more amino acid substitutions are provided for use in the methods, compositions, and/or kits of the invention. Sites of interest for substitutional mutagenesis include the HVRs and FRs. Conservative substitutions are shown in Table 5, below, under the heading of "preferred substitutions." More substantial changes are provided in Table 5 under the heading of "exemplary substitutions," and as further described below in reference to amino acid side chain classes. Amino acid substitutions may be introduced into an antibody of interest and the products screened for a desired activity, for example, retained/improved antigen binding, decreased immunogenicity, or improved ADCC or CDC.

**Table 5: Exemplary and Preferred Amino Acid Substitutions**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gin |
| Asp (D) | Glu; Asn | Glu |
| Cys (C) | Ser; Ala | Ser |
| Gln (Q) | Asn; Glu | Asn |
| Glu (E) | Asp; Gin | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g., a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g., binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001). In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g., for ADEPT) or a polypeptide which increases the serum half-life of the antibody.

*(iv) Glycosylation variants*

In some instances, the anti-PD-L1 antibody (e.g., atezolizumab (MPDL3280A)) variant has been modified to increase or decrease the extent to which the bispecific antibody is glycosylated. Addition or deletion of glycosylation sites to an anti-PD-L1 antibody *e.g., atezolizumab (MPDL3280A)) may be conveniently accomplished by altering the amino acid sequence such that one or more glycosylation sites is created or removed.

Where the bispecific antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In some instances, the anti-PD-L1 antibody (e.g., atezolizumab (MPDL3280A)) variant has a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e.g., complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about $\pm$ 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al.,* especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO 2003/085107).

In view of the above, in some instances, the methods of the invention involve administering to the subject in the context of a fractionated, dose-escalation dosing regimen an anti-PD-L1 antibody (e.g., atezolizumab (MPDL3280A)) variant that comprises an aglycosylation site mutation. In some instances, the aglycosylation site mutation reduces effector function of the bispecific antibody. In some instances, the aglycosylation site mutation is a substitution mutation. In some instances, the bispecific antibody comprises a substitution mutation in the Fc region that reduces effector function. In some instances, the substitution mutation is at amino acid residue N297, L234, L235, and/or D265 (EU numbering). In some instances, the substitution mutation is selected from the group consisting of N297G, N297A, L234A, L235A, D265A, and P329G. In some instances, the substitution mutation is at amino acid residue N297. In a preferred embodiment, the substitution mutation is N297A.

In other instances, bispecific antibody variants with bisected oligosaccharides are used in accordance with the methods of the invention, for example, in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

*(v) Fc region variants*

In some instances, an anti-PD-L1 antibody, e.g., atezolizumab (MPDL3280A) variant that has one or more amino acid modifications introduced into the Fc region (i.e., an Fc region variant (see e.g., US 2012/0251531)) of the bispecific antibody may be administered to a subject having cancer (e.g., prostate cancer, e.g., CRPC, e.g., mCRPC or locally confined, inoperable CRPC) in accordance with the methods of the invention. The Fc region variant may comprise a human Fc region sequence (e.g., a human IgG1, IgG2, IgG3 or IgG4 Fc region) comprising an amino acid modification (e.g., a substitution) at one or more amino acid positions.

In some instances, the bispecific Fc region antibody variant possesses some but not all effector functions, which makes it a desirable candidate for applications in which the half-life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g., Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CYTOTOX96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g*., in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al. J. Immunol. Methods 202:163 (1996); Cragg, M.S. et al. Blood. 101:1045-1052 (2003); and Cragg, M.S. and M.J. Glennie Blood. 103:2738-2743 (2004)). FcRn binding and *in vivo* clearance/half-life determinations can also be performed using methods known in the art (see, e.g., Petkova, S.B. et al. Int'l. Immunol. 18(12):1759-1769 (2006)).

Antibodies with reduced effector function include those with substitution of one or more of Fc region residues 238, 265, 269, 270, 297, 327 and 329 (U.S. Patent Nos. 6,737,056 and 8,219,149). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581 and 8,219,149).

In certain instances, the proline at position 329 of a wild-type human Fc region in the antibody is substituted with glycine or arginine or an amino acid residue large enough to destroy the proline sandwich within the Fc/Fcγ receptor interface that is formed between the proline 329 of the Fc and tryptophan residues Trp 87 and Trp 110 of FcgRIII (Sondermann et al. Nature. 406, 267-273 (2000)). In certain embodiments, the bispecific antibody comprises at least one further amino acid substitution. In one embodiment, the further amino acid substitution is S228P, E233P, L234A, L235A, L235E, N297A, N297D, or P331S, and still in another embodiment the at least one further amino acid substitution is L234A and L235A of the human IgG1 Fc region or S228P and L235E of the human IgG4 Fc region (see e.g., US 2012/0251531), and still in another embodiment the at least one further amino acid substitution is L234A and L235A and P329G of the human IgG1 Fc region.

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain instances, the anti-PD-L1 antibody (e.g., atezolizumab (MPDL3280A)) comprises an Fc region with one or more amino acid substitutions which improve ADCC, e.g., substitutions at positions 298, 333, and/or 334 of the Fc region (EU numbering of residues).

In some instances, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half-lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

*(vi) Cysteine engineered antibody variants*

In certain embodiments, it may be desirable to create cysteine engineered anti-PD-L1 antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541

*(vii) Other antibody derivatives*

In some instances, the anti-PD-L1 antibody (e.g., atezolizumab (MPDL3280A)) may be modified to contain additional non-proteinaceous moieties that are known in the art and readily available and administered to the subject in accordance with the methods described herein. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

*C. Administration*

The PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), or compositions thereof, utilized in the methods, uses, assays, and kits described herein can be formulated for administration or administered by any suitable method, including, for example, intravenously, intramuscularly, subcutaneously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subconjunctivally, intravesicularly, mucosally, intrapericardially, intraumbilically, intraocularly, intraorbitally, orally, topically, transdermally, intravitreally (e.g., by intravitreal injection), by eye drop, by inhalation, by injection, by implantation, by infusion, by continuous infusion, by localized perfusion bathing target cells directly, by catheter, by lavage, in creams, or in lipid compositions. The compositions utilized in the methods described herein can also be administered systemically or locally. The method of administration can vary depending on various factors (e.g., the compound or composition being administered and the severity of the condition, disease, or disorder being treated). In some instances, the PD-L1 axis binding antagonist (e.g., PD-L1 binding antagonist, e.g., an anti-PD-L1 antibody, e.g., atezolizumab (MPDL3280A)) is administered intravenously, intramuscularly, subcutaneously, topically, orally, transdermally, intraperitoneally, intraorbitally, by implantation, by inhalation, intrathecally, intraventricularly, or intranasally. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

The PD-L1 axis binding antagonist (e.g., PD-L1 binding antagonist, e.g., an anti-PD-L1 antibody, e.g., atezolizumab (MPDL3280A)) and any additional therapeutic agent may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The PD-L1 axis binding antagonist (e.g., PD-L1 binding antagonist, e.g., an anti-PD-L1 antibody, e.g., atezolizumab (MPDL3280A)) need not be, but is optionally formulated with and/or administered concurrently with, one or more agents currently used to prevent or treat the disorder in question. The effective amount of such other agents depends on the amount of the PD-L1 axis binding antagonist (e.g., PD-L1 binding antagonist, e.g., an anti-PD-L1 antibody, e.g., atezolizumab (MPDL3280A)) present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of a cancer (e.g., a lung cancer (NSCLC), a bladder cancer, (UC), a kidney cancer (RCC), or a breast cancer (TNBC)), the appropriate dosage of a PD-L1 axis binding antagonist (e.g., PD-L1 binding antagonist, e.g., an anti-PD-L1 antibody, e.g., atezolizumab (MPDL3280A)) described herein (when used alone or in combination with one or more other additional therapeutic agents) will depend on the type of disease to be treated, the severity and course of the disease, whether the PD-L1 axis binding antagonist (e.g., PD-L1 binding antagonist, e.g., an anti-PD-L1 antibody, e.g., atezolizumab (MPDL3280A)) is administered for preventive or therapeutic purposes, previous therapy, the patient's clinical history and response to the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)), and the discretion of the attending physician. The PD-L1 axis binding antagonist (e.g., PD-L1 binding antagonist, e.g., an anti-PD-L1 antibody, e.g., atezolizumab (MPDL3280A)) is suitably administered to the patient at one time or over a series of treatments. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. Such doses may be administered intermittently, e.g., every week or every three weeks (e.g., such that the patient receives, for example, from about two to about twenty, or e.g., about six doses of the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody))). An initial higher loading dose, followed by one or more lower doses may be administered. However, other dosage regimens may be useful. The progress of this therapy is easily monitored by conventional techniques and assays.

In some instances, an effective amount of the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be between about 60 mg to about 5000 mg (e.g., between about 60 mg to about 4500 mg, between about 60 mg to about 4000 mg, between about 60 mg to about 3500 mg, between about 60 mg to about 3000 mg, between about 60 mg to about 2500 mg, between about 650 mg to about 2000 mg, between about 60 mg to about 1500 mg, between about 100 mg to about 1500 mg, between about 300 mg to about 1500 mg, between about 500 mg to about 1500 mg, between about 700 mg to about 1500 mg, between about 1000 mg to about 1500 mg, between about 1000 mg to about 1400 mg, between about 1100 mg to about 1300 mg, between about 1150 mg to about 1250 mg, between about 1175 mg to about 1225 mg, or between about 1190 mg to about 1210 mg, e.g., about 1200 mg ± 5 mg, about 1200 ± 2.5 mg, about 1200 ± 1.0 mg, about 1200 ± 0.5 mg, about 1200 ± 0.2 mg, or about 1200 ± 0.1 mg). In some instances, the methods include administering to the individual the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) at about 1200 mg (e.g., a fixed dose of about 1200 mg or about 15 mg/kg).

In some instances, the amount of the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) administered to individual (e.g., human) may be in the range of about 0.01 to about 50 mg/kg of the individual's body weight (e.g., between about 0.01 to about 45 mg/kg, between about 0.01 mg/kg to about 40 mg/kg, between about 0.01 mg/kg to about 35 mg/kg, between about 0.01 mg/kg to about 30 mg/kg, between about 0.1 mg/kg to about 30 mg/kg, between about 1 mg/kg to about 30 mg/kg, between about 2 mg/kg to about 30 mg/kg, between about 5 mg/kg to about 30 mg/kg, between about 5 mg/kg to about 25 mg/kg, between about 5 mg/kg to about 20 mg/kg, between about 10 mg/kg to about 20 mg/kg, or between about 12 mg/kg to about 18 mg/kg, e.g., about 15 ± 2 mg/kg, about 15 ± 1 mg/kg, about 15 ± 0.5 mg/kg, about 15 ± 0.2 mg/kg, or about 15 ± 0.1 mg/kg). In some instances, the methods include administering to the individual the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) at about 15 mg/kg.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) is administered to the individual (e.g., a human) at 1200 mg intravenously every three weeks (q3w). The dose may be administered as a single dose or as multiple doses (e.g., 2, 3, 4, 5, 6, 7, or more than 7 doses), such as infusions. In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) administered to the individual (e.g., a human) may be administered alone or in combination with an additional therapeutic agent described herein (e.g., a VEGF antagonist (e.g., bevacizumab) and/or a chemotherapeutic (e.g., carboplatin and paclitaxel)), in four to six doses (e.g., every three weeks). The dose of the antibody administered in a combination treatment may be reduced as compared to a single treatment. The progress of this therapy is easily monitored by conventional techniques. In one instance, the PD-L1 axis binding antagonist (e.g., PD-L1 binding antagonist, e.g., anti-PD-L1 antibody, e.g., atezolizumab (e.g., MPDL3280A)) is administered as a monotherapy to the individual to treat a cancer. In other instances, the PD-L1 axis binding antagonist (e.g., PD-L1 binding antagonist, e.g., anti-PD-L1 antibody, e.g., atezolizumab (e.g., MPDL3280A)) is administered as a combination therapy, as described herein, to the individual to treat a cancer.

*D. Indications*

The methods and medicaments described herein are useful for treating a patient having a cancer (e.g., lung cancer (e.g., NSCLC), bladder cancer (e.g., UC), kidney cancer (e.g., RCC), or breast cancer (e.g., TNBC)) by administering to the individual an effective amount of a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). For example, the cancer may be a lung cancer, a kidney cancer, a bladder cancer, a breast cancer, a colorectal cancer, an ovarian cancer, a pancreatic cancer, a gastric carcinoma, an esophageal cancer, mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy.

In some instances, the cancer is a lung cancer. For example, the lung cancer may be a non-small cell lung cancer (NSCLC), including but not limited to a locally advanced or metastatic (e.g., stage IIIB, stage IV, or recurrent) NSCLC. In some instances, the lung cancer (e.g., NSCLC) is unresectable/inoperable lung cancer (e.g., NSCLC). In some instances, the lung cancer is a chemotherapy-naïve lung cancer (e.g., a chemotherapy-naive metastatic NSCLC (mNSCLC)). In some instances, the lung cancer is a non-squamous lung cancer (e.g., a non-squamous mNSCLC). In some instances, the lung cancer is a stage IV lung cancer (e.g., a stage IV mNSCLC). In some instances, the lung cancer is a recurrent lung cancer (e.g., a recurrent mNSCLC). In some instances, the patient having the lung cancer (e.g., NSCLC) has an *EGFR* or *ALK* genomic alteration. In some instances, the patient having lung cancer with a *EGFR* or ALK genomic alteration has disease progression/treatment intolerance with one or more approved tyrosine kinase inhibitors (TKI).

In some instances, the cancer may be a bladder cancer. For example, the bladder cancer may be a urothelial carcinoma (UC), including but not limited to a non-muscle invasive urothelial carcinoma, a muscle-invasive urothelial carcinoma, or a metastatic urothelial carcinoma. In some instances, the urothelial carcinoma is a metastatic urothelial carcinoma.

In some instances, the cancer may be a kidney cancer. For example, the kidney cancer may be a renal cell carcinoma (RCC), including stage I RCC, stage II RCC, stage III RCC, stage IV RCC, or recurrent RCC.

In some instances, the cancer may be a breast cancer. In some instances, the breast cancer may be a triple-negative breast cancer. For example, the breast cancer may be triple-negative breast cancer, estrogen receptor-positive breast cancer, estrogen receptor-positive/HER2-negative breast cancer, HER2-negative breast cancer, HER2-positive breast cancer, estrogen receptor-negative breast cancer, progesterone receptor-positive breast cancer, or progesterone receptor-negative breast cancer.

In some instances, the individual having a cancer, e.g., cancers described herein, has not been previously treated for the cancer. For example, the individual having a cancer has not previously received a PD-L1 axis binding antagonist therapy (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, the individual having a cancer has previously received treatment for the cancer. In some instances, the individual having a cancer has previously received treatment including a non-PD-L1 axis binding antagonist therapy (e.g., an anti-cancer therapy (e.g., a cytotoxic agent, a growth-inhibitory agent, a radiation therapy, an anti-angiogenic agent, or a combination thereof)).

*E. Combination therapies*

In any of the methods herein, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in combination with an effective amount of one or more additional therapeutic agents. Suitable additional therapeutic agents include, for example, an anti-neoplastic agent, a chemotherapeutic agent, a growth inhibitory agent, a cytotoxic agent, a radiotherapy, or combinations thereof.

In some instances, the methods further involve administering to the patient an effective amount of one or more additional therapeutic agents. In some instances, the additional therapeutic agent is selected from the group consisting of a cytotoxic agent, a chemotherapeutic agent, a growth-inhibitory agent, a radiation therapy agent, an anti-angiogenic agent, and combinations thereof. In some instances, a PD-L1 axis binding antagonist (e.g., PD-L1 binding antagonist, e.g., anti-PD-L1 antibody, e.g., atezolizumab (MPDL3280A)) may be administered in conjunction with a chemotherapy or chemotherapeutic agent. In some instances, a PD-L1 axis binding antagonist (e.g., PD-L1 binding antagonist, e.g., an anti-PD-L1 antibody, e.g., atezolizumab (MPDL3280A)) may be administered in conjunction with a radiation therapy agent. In some instances, a PD-L1 axis binding antagonist (e.g., PD-L1 binding antagonist, e.g., an anti-PD-L1 antibody, e.g., atezolizumab (MPDL3280A)) may be administered in conjunction with a targeted therapy or targeted therapeutic agent. In some instances, a PD-L1 axis binding antagonist (e.g., PD-L1 binding antagonist, e.g., an anti-PD-L1 antibody, e.g., atezolizumab (MPDL3280A)) may be administered in conjunction with an immunotherapy or immunotherapeutic agent, for example a monoclonal antibody. In some instances, the additional therapeutic agent is an agonist directed against an activating co-stimulatory molecule. In some instances, the additional therapeutic agent is an antagonist directed against an inhibitory co-stimulatory molecule.

Such combination therapies noted above encompass combined administration (where two or more therapeutic agents are included in the same or separate formulations), and separate administration, in which case, administration of a PD-L1 axis binding antagonist (e.g., PD-L1 binding antagonist, e.g., an anti-PD-L1 antibody, e.g., atezolizumab (MPDL3280A)) can occur prior to, simultaneously, and/or following, administration of the additional therapeutic agent or agents. in one instance, administration of PD-L1 axis binding antagonist (e.g., PD-L1 binding antagonist, e.g., an anti-PD-L1 antibody, e.g., atezolizumab (MPDL3280A)) and administration of an additional therapeutic agent occur within about one month, or within about one, two or three weeks, or within about one, two, three, four, five, or six days, of each other.

Without wishing to be bound to theory, it is thought that enhancing T-cell stimulation, by promoting an activating co-stimulatory molecule or by inhibiting a negative co-stimulatory molecule, may promote tumor cell death thereby treating or delaying progression of cancer. In some instances, a PD-L1 axis binding antagonist (e.g., PD-L1 binding antagonist, e.g., an anti-PD-L1 antibody, e.g., atezolizumab (MPDL3280A)) may be administered in conjunction with an agonist directed against an activating co-stimulatory molecule. In some instances, an activating co-stimulatory molecule may include CD40, CD226, CD28, OX40, GITR, CD137, CD27, HVEM, or CD127. In some instances, the agonist directed against an activating co-stimulatory molecule is an agonist antibody that binds to CD40, CD226, CD28, OX40, GITR, CD137, CD27, HVEM, or CD127. In some instances, a PD-L1 axis binding antagonist (e.g., PD-L1 binding antagonist, e.g., an anti-PD-L1 antibody, e.g., atezolizumab (MPDL3280A)) may be administered in conjunction with an antagonist directed against an inhibitory co-stimulatory molecule. In some instances, an inhibitory co-stimulatory molecule may include CTLA-4 (also known as CD152), TIM-3, BTLA, VISTA, LAG-3, B7-H3, B7-H4, IDO, TIGIT, MICA/B, or arginase. In some instances, the antagonist directed against an inhibitory co-stimulatory molecule is an antagonist antibody that binds to CTLA-4, TIM-3, BTLA, VISTA, LAG-3, B7-H3, B7-H4, IDO, TIGIT, MICA/B, or arginase.

In some instances, a PD-L1 axis binding antagonist (e.g., PD-L1 binding antagonist, e.g., an anti-PD-L1 antibody, e.g., atezolizumab (MPDL3280A)) may be administered in conjunction with an antagonist directed against CTLA-4 (also known as CD152), e.g., a blocking antibody. In some instances, a PD-L1 axis binding antagonist (e.g., PD-L1 binding antagonist, e.g., an anti-PD-L1 antibody, e.g., atezolizumab (MPDL3280A)) may be administered in conjunction with ipilimumab (also known as MDX-010, MDX-101, or YERVOY®). In some instances, a PD-L1 axis binding antagonist (e.g., PD-L1 binding antagonist, e.g., an anti-PD-L1 antibody, e.g., atezolizumab (MPDL3280A)) may be administered in conjunction with tremelimumab (also known as ticilimumab or CP-675,206). In some instances, a PD-L1 axis binding antagonist (e.g., PD-L1 binding antagonist, e.g., an anti-PD-L1 antibody, e.g., atezolizumab (MPDL3280A)) may be administered in conjunction with an antagonist directed against B7-H3 (also known as CD276), e.g., a blocking antibody. In some instances, a PD-L1 axis binding antagonist (e.g., PD-L1 binding antagonist, e.g., an anti-PD-L1 antibody, e.g., atezolizumab (MPDL3280A)) may be administered in conjunction with MGA271. In some instances, a PD-L1 axis binding antagonist (e.g., PD-L1 binding antagonist, e.g., an anti-PD-L1 antibody, e.g., atezolizumab (MPDL3280A)) may be administered in conjunction with an antagonist directed against a TGF-beta, e.g., metelimumab (also known as CAT-192), fresolimumab (also known as GC1008), or LY2157299.

In some instances, a PD-L1 axis binding antagonist (e.g., PD-L1 binding antagonist, e.g., an anti-PD-L1 antibody, e.g., atezolizumab (MPDL3280A)) may be administered in conjunction with a treatment comprising adoptive transfer of a T-cell (e.g., a cytotoxic T-cell or CTL) expressing a chimeric antigen receptor (CAR). In some instances, a PD-L1 axis binding antagonist (e.g., PD-L1 binding antagonist, e.g., an anti-PD-L1 antibody, e.g., atezolizumab (MPDL3280A)) may be administered in conjunction with a treatment comprising adoptive transfer of a T-cell comprising a dominant-negative TGF beta receptor, e.g., a dominant-negative TGF beta type II receptor. In some instances, a PD-L1 axis binding antagonist (e.g., PD-L1 binding antagonist, e.g., an anti-PD-L1 antibody, e.g., atezolizumab (MPDL3280A)) may be administered in conjunction with a treatment comprising a HERCREEM protocol (see, e.g., ClinicalTrials.gov Identifier NCT00889954).

In some instances, a PD-L1 axis binding antagonist (e.g., PD-L1 binding antagonist, e.g., an anti-PD-L1 antibody, e.g., atezolizumab (MPDL3280A)) may be administered in conjunction with an agonist directed against CD137 (also known as TNFRSF9, 4-1BB, or ILA), e.g., an activating antibody. In some instances, a PD-L1 axis binding antagonist (e.g., PD-L1 binding antagonist, e.g., an anti-PD-L1 antibody, e.g., atezolizumab (MPDL3280A)) may be administered in conjunction with urelumab (also known as BMS-663513). In some instances, a PD-L1 axis binding antagonist (e.g., PD-L1 binding antagonist, e.g., an anti-PD-L1 antibody, e.g., atezolizumab (MPDL3280A)) may be administered in conjunction with an agonist directed against CD40, e.g., an activating antibody. In some instances, a PD-L1 axis binding antagonist (e.g., PD-L1 binding antagonist, e.g., an anti-PD-L1 antibody, e.g., atezolizumab (MPDL3280A)) may be administered in conjunction with CP-870893. In some instances, a PD-L1 axis binding antagonist (e.g., PD-L1 binding antagonist, e.g., an anti-PD-L1 antibody, e.g., atezolizumab (MPDL3280A)) may be administered in conjunction with an agonist directed against OX40 (also known as CD134), e.g., an activating antibody. In some instances, a PD-L1 axis binding antagonist (e.g., PD-L1 binding antagonist, e.g., an anti-PD-L1 antibody, e.g., atezolizumab (MPDL3280A)) may be administered in conjunction with an anti-OX40 antibody (e.g., AgonOX). In some instances, a PD-L1 axis binding antagonist (e.g., PD-L1 binding antagonist, e.g., an anti-PD-L1 antibody, e.g., atezolizumab (MPDL3280A)) may be administered in conjunction with an agonist directed against CD27, e.g., an activating antibody. In some instances, a PD-L1 axis binding antagonist (e.g., PD-L1 binding antagonist, e.g., an anti-PD-L1 antibody, e.g., atezolizumab (MPDL3280A)) may be administered in conjunction with CDX-1127. In some instances, a PD-L1 axis binding antagonist (e.g., PD-L1 binding antagonist, e.g., an anti-PD-L1 antibody, e.g., atezolizumab (MPDL3280A)) may be administered in conjunction with an antagonist directed against indoleamine-2,3-dioxygenase (IDO). In some instances, with the IDO antagonist is 1-methyl-D-tryptophan (also known as 1-D-MT).

In some instances, a PD-L1 axis binding antagonist (e.g., PD-L1 binding antagonist, e.g., an anti-PD-L1 antibody, e.g., atezolizumab (MPDL3280A)) may be administered in conjunction with an antibody-drug conjugate. In some instances, the antibody-drug conjugate comprises mertansine or monomethyl auristatin E (MMAE). In some instances, a PD-L1 axis binding antagonist (e.g., PD-L1 binding antagonist, e.g., an anti-PD-L1 antibody, e.g., atezolizumab (MPDL3280A)) may be administered in conjunction with an anti-NaPi2b antibody-MMAE conjugate (also known as DNIB0600A or RG7599). In some instances, a PD-L1 axis binding antagonist (e.g., PD-L1 binding antagonist, e.g., an anti-PD-L1 antibody, e.g., atezolizumab (MPDL3280A)) may be administered in conjunction with trastuzumab emtansine (also known as T-DM1, ado-trastuzurnab emtansine, or KADCYLA®, Genentech). In some instances, a PD-L1 axis binding antagonist (e.g., PD-L1 binding antagonist, e.g., an anti-PD-L1 antibody, e.g., atezolizumab (MPDL3280A)) may be administered in conjunction with DMUC5754A. In some instances, a PD-L1 axis binding antagonist (e.g., PD-L1 binding antagonist, e.g., an anti-PD-L1 antibody, e.g., atezolizumab (MPDL3280A)) may be administered in conjunction with an antibody-drug conjugate targeting the endothelin B receptor (EDNBR), e.g., an antibody directed against EDNBR conjugated with MMAE.

In some instances, a PD-L1 axis binding antagonist (e.g., PD-L1 binding antagonist, e.g., an anti-PD-L1 antibody, e.g., atezolizumab (MPDL3280A)) may be administered in conjunction with an anti-angiogenesis agent. In some instances, a PD-L1 axis binding antagonist (e.g., PD-L1 binding antagonist, e.g., an anti-PD-L1 antibody, e.g., atezolizumab (MPDL3280A)) may be administered in conjunction with an antibody directed against a VEGF, e.g., VEGF-A. In some instances, a PD-L1 axis binding antagonist (e.g., PD-L1 binding antagonist, e.g., an anti-PD-L1 antibody, e.g., atezolizumab (MPDL3280A)) may be administered in conjunction with bevacizumab (also known as AVASTIN®, Genentech). For example, atezolizumab (MPDL3280A) may be administered in combination with bevacizumab. In further instances, atezolizumab (MPDL3280A)) may be administered in combination with bevacizumab and one or more chemotherapeutic agents (e.g., carboplatin and/or paclitaxel). In certain instances, atezolizumab (MPDL3280A)) may be administered in combination with bevacizumab, carboplatin, and paclitaxel. In some instances, a PD-L1 axis binding antagonist (e.g., PD-L1 binding antagonist, e.g., an anti-PD-L1 antibody, e.g., atezolizumab (MPDL3280A)) may be administered in conjunction with an antibody directed against angiopoietin 2 (also known as Ang2). In some instances, a PD-L1 axis binding antagonist (e.g., PD-L1 binding antagonist, e.g., an anti-PD-L1 antibody, e.g., atezolizumab (MPDL3280A)) may be administered in conjunction with MEDI3617.

The VEGF antagonist (e.g., bevacizumab) administered to the individual (e.g., human) in conjunction with a PD-L1 axis binding antagonist (e.g., PD-L1 binding antagonist, e.g., an anti-PD-L1 antibody, e.g., atezolizumab (MPDL3280A)) may be in the range of about 0.01 to about 50 mg/kg of the individual's body weight (e.g., between about 0.01 to about 45 mg/kg, between about 0.01 mg/kg to about 40 mg/kg, between about 0.01 mg/kg to about 35 mg/kg, between about 0.01 mg/kg to about 30 mg/kg, between about 0.1 mg/kg to about 30 mg/kg, between about 1 mg/kg to about 30 mg/kg, between about 2 mg/kg to about 30 mg/kg, between about 5 mg/kg to about 30 mg/kg, between about 5 mg/kg to about 25 mg/kg, between about 5 mg/kg to about 20 mg/kg, between about 10 mg/kg to about 20 mg/kg, or between about 12 mg/kg to about 18 mg/kg, e.g., about 15 $\pm$ 2 mg/kg, about 15 $\pm$ 1 mg/kg, about 15 $\pm$ 0.5 mg/kg, about 15 $\pm$ 0.2 mg/kg, or about 15 $\pm$ 0.1 mg/kg). For example, in some instances, the methods include administering to the individual a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) at about 1200 mg in conjunction with a VEGF antagonist (e.g., bevacizumab) at about 15 mg/kg of the individual's body weight. The method may further include administration of one or more chemotherapeutic agents, such as carboplatin and/or paclitaxel.

In some instances, a PD-L1 axis binding antagonist (e.g., PD-L1 binding antagonist, e.g., an anti-PD-L1 antibody, e.g., atezolizumab (MPDL3280A)) may be administered in conjunction with an antineoplastic agent. In some instances, a PD-L1 axis binding antagonist (e.g., PD-L1 binding antagonist, e.g., an anti-PD-L1 antibody, e.g., atezolizumab (MPDL3280A)) may be administered in conjunction with an agent targeting CSF-1R (also known as M-CSFR or CD115). In some instances, a PD-L1 axis binding antagonist (e.g., PD-L1 binding antagonist, e.g., an anti-PD-L1 antibody, e.g., atezolizumab (MPDL3280A)) may be administered in conjunction with anti-CSF-1R (also known as !MC-CS4). In some instances, a PD-L1 axis binding antagonist (e.g., PD-L1 binding antagonist, e.g., an anti-PD-L1 antibody, e.g., atezolizumab (MPDL3280A)) may be administered in conjunction with an interferon, for example interferon alpha or interferon gamma. In some instances, a PD-L1 axis binding antagonist (e.g., PD-L1 binding antagonist, e.g., an anti-PD-L1 antibody, e.g., atezolizumab (MPDL3280A)) may be administered in conjunction with Roferon-A (also known as recombinant Interferon alpha-2a). In some instances, a PD-L1 axis binding antagonist (e.g., PD-L1 binding antagonist, e.g., an anti-PD-L1 antibody, e.g., atezolizumab (MPDL3280A)) may be administered in conjunction with GM-CSF (also known as recombinant human granulocyte macrophage colony stimulating factor, rhu GM-CSF, sargramostim, or LEUKINE®). In some instances, a PD-L1 axis binding antagonist (e.g., PD-L1 binding antagonist, e.g., an anti-PD-L1 antibody, e.g., atezolizumab (MPDL3280A)) may be administered in conjunction with IL-2 (also known as aldesleukin or PROLEUKIN®). In some instances, a PD-L1 axis binding antagonist (e.g., PD-L1 binding antagonist, e.g., an anti-PD-L1 antibody, e.g., atezolizumab (MPDL3280A)) may be administered in conjunction with IL-12. In some instances, a PD-L1 axis binding antagonist (e.g., PD-L1 binding antagonist, e.g., an anti-PD-L1 antibody, e.g., atezolizumab (MPDL3280A)) may be administered in conjunction with an antibody targeting CD20. In some instances, the antibody targeting CD20 is obinutuzumab (also known as GA101 or GAZYVA®) or rituximab. In some instances, a PD-L1 axis binding antagonist (e.g., PD-L1 binding antagonist, e.g., an anti-PD-L1 antibody, e.g., atezolizumab (MPDL3280A)) may be administered in conjunction with an antibody targeting GITR. in some instances, the antibody targeting GITR is TRX518.

In some instances, a PD-L1 axis binding antagonist (e.g., PD-L1 binding antagonist, e.g., an anti-PD-L1 antibody, e.g., atezolizumab (MPDL3280A)) may be administered in conjunction with a cancer vaccine. In some instances, the cancer vaccine is a peptide cancer vaccine, which in some instances is a personalized peptide vaccine. In some instances the peptide cancer vaccine is a multivalent long peptide, a multi-peptide, a peptide cocktail, a hybrid peptide, or a peptide-pulsed dendritic cell vaccine (see, e.g., Yamada et al., Cancer Sci. 104:14-21, 2013). In some instances, a PD-L1 axis binding antagonist (e.g., PD-L1 binding antagonist, e.g., an anti-PD-L1 antibody, e.g., atezolizumab (MPDL3280A)) may be administered in conjunction with an adjuvant. In some instances, a PD-L1 axis binding antagonist (e.g., PD-L1 binding antagonist, e.g., an anti-PD-L1 antibody, e.g., atezolizumab (MPDL3280A)) may be administered in conjunction with a treatment comprising a TLR agonist, e.g., Poly-ICLC (also known as HILTONOL®), LPS, MPL, or CpG ODN. In some instances, a PD-L1 axis binding antagonist (e.g., PD-L1 binding antagonist, e.g., an anti-PD-L1 antibody, e.g., atezolizumab (MPDL3280A)) may be administered in conjunction with tumor necrosis factor (TNF) alpha (TNF-$\alpha$). In some instances, a PD-L1 axis binding antagonist (e.g., PD-L1 binding antagonist, e.g., an anti-PD-L1 antibody, e.g., atezolizumab (MPDL3280A)) may be administered in conjunction with IL-1. In some instances, a PD-L1 axis binding antagonist (e.g., PD-L1 binding antagonist, e.g., an anti-PD-L1 antibody, e.g., atezolizumab (MPDL3280A)) may be administered in conjunction with HMGB1. In some instances, a PD-L1 axis binding antagonist (e.g., PD-L1 binding antagonist, e.g., an anti-PD-L1 antibody, e.g., atezolizumab (MPDL3280A)) may be administered in conjunction with an IL-10 antagonist. In some instances, a PD-L1 axis binding antagonist (e.g., PD-L1 binding antagonist, e.g., an anti-PD-L1 antibody, e.g., atezolizumab (MPDL3280A)) may be administered in conjunction with an IL-4 antagonist. In some instances, a PD-L1 axis binding antagonist (e.g., PD-L1 binding antagonist, e.g., an anti-PD-L1 antibody, e.g., atezolizumab (MPDL3280A)) may be administered in conjunction with an IL-13 antagonist. In some instances, a PD-L1 axis binding antagonist (e.g., PD-L1 binding antagonist, e.g., an anti-PD-L1 antibody, e.g., atezolizumab (MPDL3280A)) may be administered in conjunction with an HVEM antagonist. In some instances, a PD-L1 axis binding antagonist (e.g., PD-L1 binding antagonist, e.g., an anti-PD-L1 antibody, e.g., atezolizumab (MPDL3280A)) may be administered in conjunction with an ICOS agonist, e.g., by administration of ICOS-L, or an agonistic antibody directed against ICOS. In some instances, a PD-L1 axis binding antagonist (e.g., PD-L1 binding antagonist, e.g., an anti-PD-L1 antibody, e.g., atezolizumab (MPDL3280A)) may be administered in conjunction with a treatment targeting CX3CL1. In some instances, a PD-L1 axis binding antagonist (e.g., PD-L1 binding antagonist, e.g., an anti-PD-L1 antibody, e.g., atezolizumab (MPDL3280A)) may be administered in conjunction with a treatment targeting CXCL9. In some instances, a PD-L1 axis binding antagonist (e.g., PD-L1 binding antagonist, e.g., an anti-PD-L1 antibody, e.g., atezolizumab (MPDL3280A)) may be administered in conjunction with a treatment targeting CXCL10. In some instances, a PD-L1 axis binding antagonist (e.g., PD-L1 binding antagonist, e.g., an anti-PD-L1 antibody, e.g., atezolizumab (MPDL3280A)) may be administered in conjunction with a treatment targeting CCL5. In some instances, a PD-L1 axis binding antagonist (e.g., PD-L1 binding antagonist, e.g., an anti-PD-L1 antibody, e.g., atezolizumab (MPDL3280A)) may be administered in conjunction with an LFA-1 or ICAM1 agonist. In some instances, a PD-L1 axis binding antagonist (e.g., PD-L1 binding antagonist, e.g., an anti-PD-L1 antibody, e.g., atezolizumab (MPDL3280A)) may be administered in conjunction with a Selectin agonist.

In some instances, a PD-L1 axis binding antagonist (e.g., PD-L1 binding antagonist, e.g., an anti-PD-L1 antibody, e.g., atezolizumab (MPDL3280A)) may be administered in conjunction with a targeted therapy. In some instances, a PD-L1 axis binding antagonist (e.g., PD-L1 binding antagonist, e.g., an anti-PD-L1 antibody, e.g., atezolizumab (MPDL3280A)) may be administered in conjunction with an inhibitor of B-Raf. In some instances, a PD-L1 axis binding antagonist (e.g., PD-L1 binding antagonist, e.g., an anti-PD-L1 antibody, e.g., atezolizumab (MPDL3280A)) may be administered in conjunction with vemurafenib (also known as ZELBORAF®). In some instances, a PD-L1 axis binding antagonist (e.g., PD-L1 binding antagonist, e.g., an anti-PD-L1 antibody, e.g., atezolizumab (MPDL3280A)) may be administered in conjunction with dabrafenib (also known as TAFINLAR®). In some instances, a PD-L1 axis binding antagonist (e.g., PD-L1 binding antagonist, e.g., an anti-PD-L1 antibody, e.g., atezolizumab (MPDL3280A)) may be administered in conjunction with erlotinib (also known as TARCEVA®). In some instances, a PD-L1 axis binding antagonist (e.g., PD-L1 binding antagonist, e.g., an anti-PD-L1 antibody, e.g., atezolizumab (MPDL3280A)) may be administered in conjunction with an inhibitor of a MEK, such as MEK1 (also known as MAP2K1) or MEK2 (also known as MAP2K2). In some instances, a PD-L1 axis binding antagonist (e.g., PD-L1 binding antagonist, e.g., an anti-PD-L1 antibody, e.g., atezolizumab (MPDL3280A)) may be administered in conjunction with cobimetinib (also known as GDC-0973 or XL-518). In some instances, a PD-L1 axis binding antagonist (e.g., PD-L1 binding antagonist, e.g., an anti-PD-L1 antibody, e.g., atezolizumab (MPDL3280A)) may be administered in conjunction with trametinib (also known as MEKINIST®). In some instances, a PD-L1 axis binding antagonist (e.g., PD-L1 binding antagonist, e.g., an anti-PD-L1 antibody, e.g., atezolizumab (MPDL3280A)) may be administered in conjunction with an inhibitor of K-Ras. In some instances, a PD-L1 axis binding antagonist (e.g., PD-L1 binding antagonist, e.g., an anti PD-L1 antibody, e.g., atezolizumab (MPDL3280A)) may be administered in conjunction with an inhibitor of c-Met. In some instances, a PD-L1 axis binding antagonist (e.g., PD-L1 binding antagonist, e.g., an anti-PD-L1 antibody, e.g., atezolizumab (MPDL3280A)) may be administered in conjunction with onartuzumab (also known as MetMAb). In some instances, a PD-L1 axis binding antagonist (e.g., PD-L1 binding antagonist, e.g., an anti-PD-L1 antibody, e.g., atezolizumab (MPDL3280A)) may be administered in conjunction with an inhibitor of Alk. in some instances, a PD-L1 axis binding antagonist (e.g., PD-L1 binding antagonist, e.g., an anti-PD-L1 antibody, e.g., atezolizumab (MPDL3280A)) may be administered in conjunction with AF802 (also known as CH5424802 or alectinib). In some instances, a PD-L1 axis binding antagonist (e.g., PD-L1 binding antagonist, e.g., an anti-PD-L1 antibody, e.g., atezolizumab (MPDL3280A)) may be administered in conjunction with an inhibitor of a phosphatidylinositol 3-kinase (PI3K). In some instances, a PD-L1 axis binding antagonist (e.g., PD-L1 binding antagonist, e.g., an anti-PD-L1 antibody, e.g., atezolizumab (MPDL3280A)) may be administered in conjunction with BKM120. In some instances, a PD-L1 axis binding antagonist (e.g., PD-L1 binding antagonist, e.g., an anti-PD-L1 antibody, e.g., atezolizumab (MPDL3280A)) may be administered in conjunction with idelalisib (also known as GS-1101 or CAL-101). In some embodiments, a PD-L1 axis binding antagonist (e.g., PD-L1 binding antagonist, e.g., an anti-PD L1 antibody, e.g., atezolizumab (MPDL3280A)) may be administered in conjunction with perifosine (also known as KRX-0401). In some embodiments, a PD-L1 axis binding antagonist (e.g., PD-L1 binding antagonist, e.g., an anti-PD-L1 antibody, e.g., atezolizumab (MPDL3280A)) may be administered in conjunction with an inhibitor of an Akt. In some embodiments, a PD-L1 axis binding antagonist (e.g., PD-L1 binding antagonist, e.g., an anti-PD-L1 antibody,

e.g., atezolizumab (MPDL3280A)) may be administered in conjunction with MK2206. In some instances, a PD-L1 axis binding antagonist (e.g., PD-L1 binding antagonist, e.g., an anti-PD-L1 antibody, e.g., atezolizumab (MPDL3280A)) may be administered in conjunction with GSK690693. In some instances, a PD-L1 axis binding antagonist (e.g., PD-L1 binding antagonist, e.g., an anti-PD-L1 antibody, e.g., atezolizumab (MPDL3280A)) may be administered in conjunction with GDC-0941. In some instances, a PD-L1 axis binding antagonist (e.g., PD-L1 binding antagonist, e.g., an anti-PD-L1 antibody, e.g., atezolizumab (MPDL3280A)) may be administered in conjunction with an inhibitor of mTOR. In some instances, a PD-L1 axis binding antagonist (e.g., PD-L1 binding antagonist, e.g., an anti-PD-L1 antibody, e.g., atezolizumab (MPDL3280A)) may be administered in conjunction with sirolimus (also known as rapamycin). In some instances, a PD-L1 axis binding antagonist (e.g., PD-L1 binding antagonist, e.g., an anti-PD-L1 antibody, e.g., atezolizumab (MPDL3280A)) may be administered in conjunction with temsirolimus (also known as CCI-779 or TORISEL®). In some instances, a PD-L1 axis binding antagonist (e.g., PD-L1 binding antagonist, e.g., an anti-PD-L1 antibody, e.g., atezolizumab (MPDL3280A)) may be administered in conjunction with everolimus (also known as RAD001). In some instances, a PD-L1 axis binding antagonist (e.g., PD-L1 binding antagonist, e.g., an anti-PD-L1 antibody, e.g., atezolizumab (MPDL3280A)) may be administered in conjunction with ridaforolimus (also known as AP-23573, MK-8669, or deforolimus). In some instances, a PD-L1 axis binding antagonist (e.g., PD-L1 binding antagonist, e.g., an anti-PD-L1 antibody, e.g., atezolizumab (MPDL3280A)) may be administered in conjunction with OSI-027. In some instances, a PD-L1 axis binding antagonist (e.g., PD-L1 binding antagonist, e.g., an anti-PD-L1 antibody, e.g., atezolizumab (MPDL3280A)) may be administered in conjunction with AZD8055. In some instances, a PD-L1 axis binding antagonist (e.g., PD-L1 binding antagonist, e.g., an anti-PD-L1 antibody, e.g., atezolizumab (MPDL3280A)) may be administered in conjunction with INK128. In some instances, a PD-L1 axis binding antagonist (e.g., PD-L1 binding antagonist, e.g., an anti-PD-L1 antibody, e.g., atezolizumab (MPDL3280A)) may be administered in conjunction with a dual PI3K/mTOR inhibitor. In some instances, a PD-L1 axis binding antagonist (e.g., PD-L1 binding antagonist, e.g., an anti-PD-L1 antibody, e.g., atezolizumab (MPDL3280A)) may be administered in conjunction with XL765. In some instances, a PD-L1 axis binding antagonist (e.g., PD-L1 binding antagonist, e.g., an anti-PD-L1 antibody, e.g., atezolizumab (MPDL3280A)) may be administered in conjunction with GDC-0980. In some instances, a PD-L1 axis binding antagonist (e.g., PD-L1 binding antagonist, e.g., an anti-PD-L1 antibody, e.g., atezolizumab (MPDL3280A)) may be administered in conjunction with BEZ235 (also known as NVP-BEZ235). In some instances, a PD-L1 axis binding antagonist (e.g., PD-L1 binding antagonist, e.g., an anti-PD-L1 antibody, e.g., atezolizumab (MPDL3280A)) may be administered in conjunction with BGT226. In some instances, a PD-L1 axis binding antagonist (e.g., PD-L1 binding antagonist, e.g., an anti-PD-L1 antibody, e.g., atezolizumab (MPDL3280A)) may be administered in conjunction with GSK2126458. In some instances, a PD-L1 axis binding antagonist (e.g., PD-L1 binding antagonist, e.g., an anti-PD-L1 antibody, e.g., atezolizumab (MPDL3280A)) may be administered in conjunction with PF-04691502. In some instances, a PD-L1 axis binding antagonist (e.g., PD-L1 binding antagonist, e.g., an anti-PD-L1 antibody, e.g., atezolizumab (MPDL3280A)) may be administered in conjunction with PF-05212384 (also known as PKI-587).

*(i) Combination therapies in clinical trials*

PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) can be administered to an individual in conjunction with one or more additional therapeutic agents, wherein, prior or subsequent to treatment, the individual has undergone diagnostic testing according to any one of the diagnostic methods described herein and has been identified as one who is likely to benefit from treatment with a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). As described further below, the additional therapeutic agents may be one that has been tested or is undergoing testing in a clinical trial for cancer therapies that include atezolizumab.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with obinutuzumab and polatuzumab vedotin (e.g., in the treatment of lymphoma (e.g., relapsed or refractory follicular lymphoma or diffuse large B-cell lymphoma)), as in the clinical trial NCT02729896.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with paclitaxel (e.g., albumin-bound paclitaxel (nab-paclitaxel (ABRAXANE®), e.g., in the treatment of breast cancer (e.g., TNBC)), as in the clinical trial NCT02530489.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®) (e.g., in the treatment of locally advanced or metastatic tumors (e.g., in breast cancer, cervical cancer, kidney cancer, gastric cancer, ovarian cancer, or bladder cancer), as in the clinical trial NCT01633970.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®) and leucovorin / oxaliplatin / 5-fluorouracil (FOLFOX) (e.g., in the treatment of locally advanced or metastatic tumors, e.g., in breast cancer, cervical cancer, kidney cancer, gastric cancer, ovarian cancer, or bladder cancer), as in the clinical trial NCT01633970.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with paclitaxel (e.g., albumin-bound paclitaxel (nab-paclitaxel (ABRAXANE®)) and carboplatin (e.g., PARAPLATIN®) (e.g., in the treatment of locally advanced or metastatic tumors, e.g., in the treatment of lung cancer (NSCLC), breast cancer, cervical cancer, kidney cancer, gastric cancer, ovarian cancer, or bladder cancer), as in the clinical trial NCT01633970.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with paclitaxel (e.g., albumin bound paclitaxel (nab-paclitaxel (ABRAXANE®)), e.g., in the treatment of locally advanced or metastatic tumors (e.g., in the treatment of lung cancer (NSCLC), breast cancer, cervical cancer, kidney cancer, gastric cancer, ovarian cancer, or bladder cancer), as in the clinical trial NCT01633970.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with pemetrexed (e.g., ALIMTA®) and carboplatin (e.g., PARAPLATIN®) (e.g., in the treatment of locally advanced or metastatic tumors, e.g., in the treatment of breast cancer, cervical cancer, kidney cancer, gastric cancer, ovarian cancer, or bladder cancer), as in the clinical trial NCT01633970.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with etoposide (e.g., ETOPOPHOS®, TOPOSAR®) and carboplatin (e.g., PARAPLATIN®) (e.g., in the treatment of lung cancer (e.g., small cell lung cancer (SCLC))), as in the clinical trial NCT02748889.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with paclitaxel (e.g., albumin-bound paclitaxel (nab-paclitaxel (ABRAXANE®)) and carboplatin (e.g., PARAPLATIN®) (e.g., in the treatment of locally advanced or metastatic tumors, e.g., in the treatment of lung cancer (NSCLC)), as in the clinical trial NCT02716038.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with epacadostat (e.g., INCB024360) (e.g., in the treatment of lung cancer (e.g., NSCLC) or bladder cancer (e.g., urothelial carcinoma)), as in the clinical trial NCT02298153.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with radiation therapy and a chemotherapy (e.g., carboplatin and/or paclitaxel), e.g., in the treatment of lung cancer (e.g., NSCLC), as in the clinical trial NCT02525757.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with veliparib (e.g., in the treatment of breast cancer, e.g., TNBC, BRCA1 gene mutation, BRCA2 gene mutation, estrogen receptor negative breast cancer, Her2/Neu negative breast cancer, stage IIIA breast cancer, stage IIIB breast cancer, stage IIIC breast cancer, or stage IV breast cancer), as in the clinical trial NCT02849496.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with alectinib (also known as ALECENSA®) (e.g., in the treatment of lung cancer (e.g., NSCLC), as in the clinical trial NCT02013219.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with erlotinib (also known as TARCEVA®) (e.g., in the treatment of lung cancer (e.g., NSCLC), as in the clinical trial NCT02013219.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with MTIG7192A (e.g., in the treatment of advanced metastatic tumors), as in the clinical trial NCT02794571.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with vemurafenib (also known as ZELBORAF®) (e.g., in the treatment of skin cancer (e.g., a malignant melanoma), as in the clinical trial NCT01656642.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with vemurafenib (also known as ZELBORAF®) and cobimetinib (also known as (COTELLIC®) (e.g., in the treatment of skin cancer (e.g., a malignant melanoma)), as in the clinical trial NCT01656642.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®, Genentech) (e.g., in the treatment of ovarian, fallopian tube, or peritoneal cancer), as in the clinical trial NCT02839707.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with obinutuzumab (e.g., in the treatment of lymphoma (e.g., lymphocytic lymphoma or relapsed refractory or chronic lymphocytic leukemia (CLL))), as in the clinical trial NCT02846623.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with carboplatin and pemetrexed (e.g., in the treatment of lung cancer (e.g., NSCLC)), as in clinical trial NCT02657434.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with cisplatin and pemetrexed (e.g., in the treatment of lung cancer (e.g., NSCLC)), as in the clinical trial NCT02657434.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with tazemetostat (e.g., in the treatment of lymphoma (e.g., follicular lymphoma or diffuse large b-cell lymphoma)), as in the clinical trial NCT02220842.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with obinutuzumab (e.g., in the treatment of lymphoma (e.g., follicular lymphoma or diffuse large b-cell lymphoma)), as in the clinical trial NCT02220842.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with lenalidomide (e.g., in the treatment of multiple myeloma), as in the clinical trial NCT02431208.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with daratumumab (e.g., in the treatment of multiple myeloma), as in the clinical trial NCT02431208.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with daratumumab and lenalidomide (e.g., in the treatment of multiple myeloma), as in the clinical trial NCT02431208.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with daratumumab and pomalidomide (e.g., in the treatment of multiple myeloma), as in the clinical trial NCT02431208.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®, Genentech) (e.g., in the treatment of kidney cancer (e.g., renal cell carcinoma)), as in the clinical trial NCT02420821.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with stereotactic body radiation (e.g., in the treatment of lung cancer (e.g., NSCLC)), as in the clinical trial NCT02400814.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with rociletinib (e.g., in the treatment of lung cancer (e.g., NSCLC)), as in the clinical trial NCT02630186.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with GDC-0919 (e.g., in the treatment of a solid tumor (e.g., renal cell cancer (RCC), urothelial carcinoma (UC), triple-negative breast cancer (TNBC), non-small cell lung cancer (NSCLC), melanoma, head and neck squamous cell carcinoma (HNSCC), gastric cancer, ovarian cancer, cervical cancer, endometrial cancer, or Merkel cell cancer)), as in the clinical trial NCT02471846.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with radium-223 dichloride (e.g., in the treatment of lung prostate cancer (e.g., castrate-resistant prostate cancer)), as in the clinical trial NCT02814669.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with MOXR0916 (e.g., in the treatment of a solid tumor (e.g., locally advanced or metastatic solid tumors)), as in the clinical trial NCT02410512.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®, Genentech) and MOXR0916 (e.g., in the treatment of a solid tumor (e.g., locally advanced or metastatic solid tumors)), as in the clinical trial NCT02410512.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with azacitidine (e.g., in the treatment of a solid tumor (e.g., myelodysplastic syndromes)), as in the clinical trial NCT02508870.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with paclitaxel (e.g., albumin-bound paclitaxel (nab-paclitaxel (ABRAXANE®)) (e.g., in the treatment of breast cancer (e.g., TNBC))) as in the clinical trial NCT02425891.

In some instances, the PD-L1 axis binding antagonist (e.g., PD-L1 binding antagonist (e.g., anti-PD-L1 antibody, e.g, atezolizumab (MPDL3280A)) or PD-1 binding antagonist (e.g., anti-PD-1 antibody)) may be administered in conjunction with lenalidomide and obinutuzumab (e.g., in the treatment of lymphoma), as in the clinical trial NCT02631577.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with etoposide (e.g., ETOPOPHOS®, TOPOSAR®) and carboplatin (e.g., PARAPLATIN®) (e.g., in the treatment of lung cancer (e.g., small cell lung cancer (SCLC))), as in the clinical trial NCT02763579.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with ipilimumab (e.g., in the treatment of locally advanced or metastatic solid tumors), as in the clinical trial NCT02174172.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with interferon alfa-2b (e.g., in the treatment of locally advanced or metastatic solid tumors (e.g., NSCLC, melanoma, or RCC)), as in the clinical trial NCT02174172.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with hypofractionated image-guided radiotherapy (e.g., in the treatment of lung cancer (e.g., NSCLC)), as in the clinical trial NCT02463994.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with CDX-1401 (e.g., in the treatment of lung cancer (e.g., NSCLC)), as in the clinical trial NCT02495636.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with CDX-1401 (e.g., in the treatment of lung cancer (e.g., NSCLC)), as in the clinical trial NCT02495636.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with trastuzumab and pertuzumab (e.g., in the treatment of breast cancer (e.g., Her2-positive breast cancer)), as in the clinical trial NCT02605915.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with trastuzumab emtansine (e.g., in the treatment of breast cancer (e.g., Her2-positive breast cancer)), as in the clinical trial NCT02605915.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with doxorubicin and cyclophosphamide (e.g., in the treatment of breast cancer (e.g., Her2-positive breast cancer)), as in the clinical trial NCT02605915.

In some instances, the PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with trastuzumab, pertuzumab, and docetaxel (e.g., in the treatment of breast cancer (e.g., Her2-positive breast cancer)), as in the clinical trial NCT02605915.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®) (e.g., in the treatment of kidney cancer (e.g., advanced non-clear cell kidney cancer)), as in the clinical trial NCT02724878.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with CMB305 (e.g., in the treatment of sarcoma (e.g., myxoid/round cell liposarcoma, synovial sarcoma, metastatic sarcoma, recurrent adult soft tissue sarcoma, locally advanced sarcoma, or liposarcoma)), as in the clinical trial NCT02609984.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with RO7009789 (e.g., in the treatment of solid cancers (e.g., locally advanced and metastatic solid tumors)), as in the clinical trial NCT02304393.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with Bacille Calmette-Guerin (also known as ONCOTICE®) (e.g., in the treatment of bladder cancer (e.g., non-muscle invasive bladder cancer)), as in the clinical trial NCT02792192.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with stereotactic body radiation therapy (e.g., in the treatment of lung cancer (e.g., NSCLC)), as in the clinical trial NCT02599454.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with carboplatin, and nab-paclitaxel (also known as ABRAXANE®) (e.g., in the treatment of breast cancer (e.g., invasive ductal breast carcinoma)), as in the clinical trial NCT02620280.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with carboplatin, nab-paclitaxel (also known as ABRAXANE®), and an adjuvant chemotherapy including AC or EC (adriamycin or epirubicin and cyclophosphamide) or FEC (fluorouracil, epirubicin, and cyclophosphamide) (e.g., in the treatment of breast cancer (e.g., invasive ductal breast carcinoma)), as in the clinical trial NCT02620280.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with gemcitabine and carboplatin or cisplatin (e.g., in the treatment of urothelial carcinoma), as in the clinical trial NCT02807636.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with paclitaxel and carboplatin (e.g., in the treatment of lung cancer (e.g., NSCLC, e.g., non-squamous NSCLC)), as in the clinical trial NCT02366143.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab, paclitaxel, and carboplatin (e.g., in the treatment of lung cancer (e.g., NSCLC, e.g., non-squamous NSCLC)), as in the clinical trial NCT02366143.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with cergutuzumab (also known as RO6895882) (e.g., in the treatment of locally advanced and/or metastatic solid tumors), as in the clinical trial NCT02350673.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bendamustine and obinutuzumab (e.g., in the treatment of lymphoma (e.g., diffuse large B-cell lymphoma or follicular lymphoma)), as in the clinical trial NCT02596971.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bendamustine, cyclophosphamide, obinutuzumab, prednisone, and vincristine (e.g., in the treatment of lymphoma (e.g., diffuse large B-cell lymphoma or follicular lymphoma)), as in the clinical trial NCT02596971.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with cyclophosphamide, doxorubicin, obinutuzumab, prednisone, and vincristine (e.g., in the treatment of lymphoma (e.g., diffuse large B-cell lymphoma or follicular lymphoma)), as in the clinical trial NCT02596971.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with cyclophosphamide, doxorubicin, prednisone, vincristine, and rituximab (e.g., in the treatment of lymphoma (e.g., diffuse large B-cell lymphoma or follicular lymphoma)), as in the clinical trial NCT02596971.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with RO6958688 (e.g., in the treatment of locally advanced and/or metastatic solid tumors (e.g., carcinoembryonic antigen (CEA)-positive solid tumors)), as in the clinical trial NCT02650713.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with acetylsalicylic acid (e.g., in the treatment of ovarian cancer (e.g., ovarian neoplasms)), as in the clinical trial NCT02659384.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (e.g., in the treatment of ovarian cancer (e.g., ovarian neoplasms)), as in the clinical trial NCT02659384.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with vanucizumab (also known as RO5520985) (e.g., in the treatment of locally advanced and/or metastatic solid tumors), as in the clinical trial NCT01688206.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with carboplatin and nab-paclitaxel (e.g., in the treatment of lung cancer (e.g., non-squamous NSCLC)), as in the clinical trial NCT02367781.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®) (e.g., in the treatment of kidney cancer (e.g., renal cell carcinoma)), as in the clinical trial NCT01984242.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with cobimetinib (also known as GDC-0973) (e.g., in the treatment of locally advanced or metastatic solid tumors), as in the clinical trial NCT01988896.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with RO5509554 (e.g., in the treatment of locally advanced solid tumors (e.g., locally advanced and/or metastatic triple negative breast cancer, ovarian cancer, bladder cancer, gastric cancer, or soft tissue sarcoma)), as in the clinical trial NCT02323191.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with varlilumab (e.g., in the treatment of advanced cancer (e.g., melanoma, RCC, triple negative breast cancer, bladder cancer, head and neck cancer, or non-small cell lung cancer)), as in the clinical trial NCT02543645.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with cobimetinib (e.g., in the treatment of colorectal cancer), as in the clinical trial NCT02788279.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with cobimetinib (e.g., in the treatment of colorectal cancer), as in the clinical trial NCT02788279.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®) (e.g., in the treatment of solid tumors), as in the clinical trial NCT02715531.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with bevacizumab (also known as AVASTIN®), leucovorin, oxaliplatin, and optionally, capecitabine (e.g., in the treatment of solid tumors), as in the clinical trial NCT02715531.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with nab-paclitaxel and gemcitabine (e.g., in the treatment of solid tumors), as in the clinical trial NCT02715531.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with oxaliplatin, leucovorin, 5-fluorouracil (5-FU), oxaliplatin, and cisplatin (e.g., in the treatment of solid tumors), as in the clinical trial NCT02715531.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with nab-paclitaxel and carboplatin (e.g., in the treatment of lung cancer (e.g., squamous NSCLC)), as in the clinical trial NCT02367794.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with paclitaxel and carboplatin (e.g., in the treatment of lung cancer (e.g., squamous NSCLC)), as in the clinical trial NCT02367794.

In some instances, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)) may be administered in conjunction with CPI-444 (e.g., in the treatment of advanced cancers (e.g., non-small cell lung cancer, malignant melanoma, renal cell cancer, triple negative breast cancer, colorectal cancer with microsatellite instability (MSI), and bladder cancer)), as in the clinical trial NCT02655822.

## IV. PHARMACEUTICAL COMPOSITIONS AND FORMULATIONS

Pharmaceutical compositions and formulations as described herein can be prepared by mixing the active ingredient(s) (e.g., an anti-PD-L1 antibody (MPDL3280A) having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®; Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases. It is understood that any of the above pharmaceutical compositions or formulations may include an immunoconjugate described herein in place of, or in addition to, a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

Exemplary lyophilized antibody formulations are described in US Patent No. 6,267,958. Aqueous antibody formulations include those described in US Patent No. 6,171,586 and WO 2006/044908, the latter formulations including a histidine-acetate buffer.

The compositions and formulations herein may also contain more than one active ingredients as necessary for the particular indication being treated, preferably those with complementary activities that do not adversely affect each other. For example, it may be desirable to further provide an additional therapeutic agent (e.g., a chemotherapeutic agent, a cytotoxic agent, a growth inhibitory agent, and/or an anti-hormonal agent, such as those recited herein above). Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g., films, or microcapsules. The formulations to be used for *in vivo* administration are generally sterile. Sterility may be readily accomplished, for example, by filtration through sterile filtration membranes.

The disclosure additionally provides pharmaceutical compositions containing CAR T cells, such as CAR T cells that have been engineered to express a CAR having the amino acid sequence of one or more CDRs, an alpha chain, and/or a beta chain of a TCR identified in a sample of peripheral blood obtained from a patient having a cancer (e.g., lung cancer (e.g., NSCLC), bladder cancer (e.g., UC), endometrial cancer, colon adenocarcinoma, renal cell carcinoma, kidney cancer (e.g., RCC), or breast cancer (e.g., TNBC)). The composition may include, for example, from about $1 \times 10^3$ CAR T cells to about $1 \times 10^{12}$ CAR T cells (e.g., from about $2 \times 10^3$ CAR T cells to about $9 \times 10^{11}$ CAR T cells, from about $3 \times 10^3$ CAR T cells to about $8 \times 10^{11}$ CAR T cells, from about $4 \times 10^3$ CAR T cells to about $7 \times 10^{11}$ CAR T cells, from about $5 \times 10^3$ CAR T cells to about $6 \times 10^{11}$ CAR T cells, from about $6 \times 10^3$ CAR T cells to about $4 \times 10^{11}$ CAR T cells, from about $7 \times 10^3$ CAR T cells to about $3 \times 10^{11}$ CAR T cells, from about $8 \times 10^3$ CAR T cells to about $2 \times 10^{11}$ CAR T cells, from about $9 \times 10^3$ CAR T cells to about $1 \times 10^{11}$ CAR T cells, from about $1 \times 10^4$ CAR T cells to about $9 \times 10^{10}$ CAR T cells, from about $2 \times 10^4$ CAR T cells to about $8 \times 10^{10}$ CAR T cells, from about $3 \times 10^4$ CAR T cells to about $7 \times 10^{10}$ CAR T cells, from about $4 \times 10^4$ CAR T cells to about $6 \times 10^{10}$ CAR T cells, from about $5 \times 10^4$ CAR T cells to about $5 \times 10^{10}$ CAR T cells, from about $6 \times 10^4$ CAR T cells to about $4 \times 10^{10}$ CAR T cells, from about $7 \times 10^4$ CAR T cells to about $3 \times 10^{10}$ CAR T cells, from about $8 \times 10^4$ CAR T cells to about $2 \times 10^{10}$ CAR T cells, from about $9 \times 10^4$ CAR T cells to about $1 \times 10^{10}$ CAR T cells, or from about $1 \times 10^5$ CAR T cells to about $9 \times 10^9$ CAR T cells, such as about $1 \times 10^3$ CAR T cells, $2 \times 10^3$ CAR T cells, $3 \times 10^3$ CAR T cells, $4 \times 10^3$ CAR T cells, $5 \times 10^3$ CAR T cells, $6 \times 10^3$ CAR T cells, $7 \times 10^3$ CAR T cells, $8 \times 10^3$ CAR T cells, $9 \times 10^3$ CAR T cells, $1 \times 10^4$ CAR T cells, $2 \times 10^4$ CAR T cells, $3 \times 10^4$ CAR T cells, $4 \times 10^4$ CAR T cells, $5 \times 10^4$ CAR T cells, $6 \times 10^4$ CAR T cells, $7 \times 10^4$ CAR T cells, $8 \times 10^4$ CAR T cells, $9 \times 10^4$ CAR T cells, $1 \times 10^5$ CAR T cells, $2 \times 10^5$ CAR T cells, $3 \times 10^5$ CAR T cells, $4 \times 10^5$ CAR T cells, $5 \times 10^5$ CAR T cells, $6 \times 10^5$ CAR T cells, $7 \times 10^5$ CAR T cells, $8 \times 10^5$ CAR T cells, $9 \times 10^5$ CAR T cells, $1 \times 10^6$ CAR T cells, $2 \times 10^6$ CAR T cells, $3 \times 10^6$ CART cells, $4 \times 10^6$ CAR T cells, $5 \times 10^6$ CAR T cells, $6 \times 10^6$ CAR T cells, $7 \times 10^6$ CAR T cells, $8 \times 10^6$ CAR T cells, $9 \times 10^6$ CAR T cells, $1 \times 10^7$ CAR T cells, $2 \times 10^7$ CAR T cells, $3 \times 10^7$ CAR T cells, $4 \times 10^7$ CAR T cells, $5 \times 10^7$ CAR T cells, $6 \times 10^7$ CART cells, $7 \times 10^7$ CAR T cells, $8 \times 10^7$ CART cells, $9 \times 10^7$ CART cells, $1 \times 10^8$ CAR T cells, $2 \times 10^8$ CAR I cells, $3 \times 10^8$ CAR T cells, $4 \times 10^8$ CAR T cells, $5 \times 10^8$ CAR T cells, $6 \times 10^8$ CAR T cells, $7 \times 10^8$ CAR T cells, $8 \times 10^8$ CAR T cells, $9 \times 10^8$ CAR T cells, $1 \times 10^9$ CAR T cells, $2 \times 10^9$ CAR T cells, $3 \times 10^9$ CAR T cells, $4 \times 10^9$ CAR T cells, $5 \times 10^9$ CAR T cells, $6 \times 10^9$ CART cells, $7 \times 10^9$ CAR T cells, $8 \times 10^9$ CAR T cells, $9 \times 10^9$ CAR T cells, $1 \times 10^{10}$ CAR T cells, $2 \times 10^{10}$ CART cells, $3 \times 10^{10}$ CAR T cells, $4 \times 10^{10}$ CAR T cells, $5 \times 10^{10}$ CAR T cells, $6 \times 10^{10}$ CAR T cells, $7 \times 10^{10}$ CAR T cells, $8 \times 10^{10}$ CAR T cells, $9 \times 10^{10}$ CAR T cells, $1 \times 10^{11}$ CAR T cells, $2 \times 10^{11}$ CAR T cells, $3 \times 10^{11}$ CART cells, $4 \times 10^{11}$ CAR T cells, $5 \times 10^{11}$ CAR T cells, $6 \times 10^{11}$ CAR T cells, $7 \times 10^{11}$ CART cells, $8 \times 10^{11}$ CAR T cells, $9 \times 10^{11}$ CAR T cells, or $\times 10^{12}$ CAR T cells, or more).

In some embodiments, the composition includes from about $1 \times 10^4$ CAR T cells to about $1 \times 10^{11}$ CAR T cells (e.g., from about $1 \times 10^4$ CAR T cells to about $9 \times 10^{10}$ CAR T cells, from about $2 \times 10^4$ CAR T cells to about $8 \times 10^{10}$ CAR T cells, from about $3 \times 10^4$ CAR T cells to about $7 \times 10^{10}$ CAR T cells, from about $4 \times 10^4$ CAR T cells to about $6 \times 10^{10}$ CAR T cells, from about $5 \times 10^4$ CAR T cells to about $5 \times 10^{10}$ CAR T cells, from about $6 \times 10^4$ CAR T cells to about $4 \times 10^{10}$ CAR T cells, from about $7 \times 10^4$ CAR T cells to about $3 \times 10^{10}$ CAR T cells, from about $8 \times 10^4$ CAR T cells to about $2 \times 10^{10}$ CAR T cells, from about $9 \times 10^4$ CAR T cells to about $1 \times 10^{10}$ CAR T cells, or from about $1 \times 10^5$ CAR T cells to about $9 \times 10^9$ CAR T cells, such as about $1 \times 10^4$ CAR T cells, $2 \times 10^4$ CAR T cells, $3 \times 10^4$ CAR T cells, $4 \times 10^4$ CAR T cells, $5 \times 10^4$ CAR T cells, $6 \times 10^4$ CAR T cells, $7 \times 10^4$ CAR T cells, $8 \times 10^4$ CAR T cells, $9 \times 10^4$ CAR T cells, $1 \times 10^5$ CAR T cells, $2 \times 10^5$ CAR T cells, $3 \times 10^5$ CAR T cells, $4 \times 10^5$ CAR T cells, $5 \times 10^5$ CAR T cells, $6 \times 10^5$ CAR T cells, $7 \times 10^5$ CART cells, $8 \times 10^5$ CAR T cells, $9 \times 10^5$ CAR T cells, $1 \times 10^6$ CART cells, $2 \times 10^6$ CART cells, $3 \times 10^6$ CAR T cells, $4 \times 10^6$ CART cells, $5 \times 10^6$ CAR T cells, $6 \times 10^6$ CAR T cells, $7 \times 10^6$ CAR T cells, $8 \times 10^6$ CAR T cells, $9 \times 10^6$ CAR T cells, $1 \times 10^7$ CAR T cells, $2 \times 10^7$ CAR T cells, $3 \times 10^7$ CART cells, $4 \times 10^7$ CART cells, $5 \times 10^7$ CART cells, $6 \times 10^7$ CAR T cells, $7 \times 10^7$ CART cells, $8 \times 10^7$ CAR T cells, $9 \times 10^7$ CAR T cells, $1 \times 10^8$ CAR T cells, $2 \times 10^8$ CAR T cells, $3 \times 10^8$ CAR T cells, $4 \times 10^8$ CAR T cells, $5 \times 10^8$ CAR T cells, $6 \times 10^8$ CAR T cells, $7 \times 10^8$ CAR T cells, $8 \times 10^8$ CAR T cells, $9 \times 10^{11}$ CAR T cells, $1 \times 10^9$ CAR T cells, $2 \times 10^9$ CAR T cells, $3 \times 10^9$ CAR T cells, $4 \times 10^9$ CAR T cells, $5 \times 10^9$ CAR T cells, $6 \times 10^9$ CAR T cells, $7 \times 10^9$ CAR T cells, $8 \times 10^9$ CAR T cells, $9 \times 10^9$ CAR T cells, $1 \times 10^{10}$ CART cells, $2 \times 10^{10}$ CAR T cells, $3 \times 10^{10}$ CAR T cells, $4 \times 10^{10}$ CAR T cells, $5 \times 10^{10}$ CAR T cells, $6 \times 10^{10}$ CART cells, $7 \times 10^{10}$ CART cells, $8 \times 10^{10}$ CART cells, $9 \times 10^{10}$ CART cells, or $1 \times 10^{11}$ CAR T cells, or more).

In some embodiments, the composition includes from about $1 \times 10^5$ CAR T cells to about $1 \times 10^{10}$ CAR T cells (e.g., from about $1 \times 10^5$ CAR T cells to about $9 \times 10^9$ CAR T cells, such as about $1 \times 10^5$ CAR T cells, $2 \times 10^5$ CAR T cells, $3 \times 10^5$ CAR T cells, $4 \times 10^5$ CAR T cells, $5 \times 10^5$ CAR T cells, $6 \times 10^5$ CAR T cells, $7 \times 10^5$ CART cells,

$8 \times 10^5$ CART cells, $9 \times 10^5$ CAR T cells, $1 \times 10^6$ CAR T cells, $2 \times 10^6$ CAR T cells, $3 \times 10^6$ CAR T cells, $4 \times 10^6$ CART cells, $5 \times 10^6$ CART cells, $6 \times 10^6$ CAR T cells, $7 \times 10^6$ CAR T cells, $8 \times 10^6$ CART cells, $9 \times 10^6$ CART cells, $1 \times 10^7$ CART cells, $2 \times 10^7$ CAR T cells, $3 \times 10^7$ CAR T cells, $4 \times 10^7$ CART cells, $5 \times 10^7$ CART cells, $6 \times 10^7$ CART cells, $7 \times 10^7$ CAR T cells, $8 \times 10^7$ CAR T cells, $9 \times 10^7$ CAR T cells, $1 \times 10^8$ CAR T cells, $2 \times 10^8$ CAR T cells, $3 \times 10^8$ CAR T cells, $4 \times 10^8$ CAR T cells, $5 \times 10^8$ CAR T cells, $6 \times 10^8$ CART cells, $7 \times 10^8$ CAR T cells, $8 \times 10^8$ CART cells, $9 \times 10^8$ CAR T cells, $1 \times 10^9$ CAR T cells, $2 \times 10^9$ CAR T cells, $3 \times 10^9$ CAR T cells, $4 \times 10^9$ CAR T cells, $5 \times 10^9$ CAR T cells, $6 \times 10^9$ CART cells, $7 \times 10^9$ CART cells, $8 \times 10^9$ CAR T cells, $9 \times 10^9$ CART cells, or $1 \times 10^{10}$ CAR T cells, or more).

In some embodiments, the composition includes from about $1 \times 10^6$ CAR T cells to about $1 \times 10^9$ CAR T cells (e.g., from about $1 \times 10^6$ CAR T cells, $2 \times 10^6$ CAR T cells, $3 \times 10^6$ CAR T cells, $4 \times 10^6$ CAR T cells, $5 \times 10^6$ CAR T cells, $6 \times 10^6$ CAR T cells, $7 \times 10^6$ CAR T cells, $8 \times 10^6$ CAR T cells, $9 \times 10^6$ CAR T cells, $1 \times 10^7$ CAR T cells, $2 \times 10^7$ CAR T cells, $3 \times 10^7$ CAR T cells, $4 \times 10^7$ CAR T cells, $5 \times 10^7$ CAR T cells, $6 \times 10^7$ CAR T cells, $7 \times 10^7$ CAR T cells, $8 \times 10^7$ CART cells, $9 \times 10^7$ CART cells, $1 \times 10^8$ CAR T cells, $2 \times 10^8$ CART cells, $3 \times 10^8$ CAR T cells, $4 \times 10^8$ CAR T cells, $5 \times 10^8$ CART cells, $6 \times 10^8$ CAR T cells, $7 \times 10^8$ CART cells, $8 \times 10^8$ CAR T cells, $9 \times 10^8$ CAR T cells, $1 \times 10^9$ CAR T cells, or more).

## V. ARTICLES OF MANUFACTURE AND KITS

In another aspect of the disclosure, an article of manufacture or kit containing materials useful for the treatment, prevention, and/or diagnosis of individuals is provided.

In some instances, such articles of manufacture or kits can be used to identify an individual having a cancer (e.g., lung cancer (e.g., NSCLC), bladder cancer (e.g., UC), kidney cancer (e.g., RCC), or breast cancer (e.g., TNBC)) who may benefit from a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). Such articles of manufacture or kits may include (a) reagents for determining the immune-score expression level of one or more of genes in a sample from the individual and (b) instructions for using the reagents to identify an individual having a cancer (e.g., lung cancer (e.g., NSCLC), bladder cancer (e.g., UC), kidney cancer (e.g., RCC), or breast cancer (e.g., TNBC)) who may benefit from a treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

For example, in some instances, the article of manufacture or kit includes (a) reagents for determining the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) in a sample from the individual and (b) instructions for using the reagents to identify an individual having a cancer (e.g., lung cancer (e.g., NSCLC), bladder cancer (e.g., UC), kidney cancer (e.g., RCC), or breast cancer (e.g., TNBC)) who may benefit from a treatment comprising a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

In some instances, such articles of manufacture or kits include a PD-L1 axis binding antagonist (e.g., PD-L1 binding antagonist, e.g., an anti-PD-L1 antibody, e.g., atezolizumab (MPDL3280A)) for treating an individual with a cancer (e.g., lung cancer (e.g., NSCLC), bladder cancer (e.g., UC), kidney cancer (e.g., RCC), or breast cancer (e.g., TNBC)). In some instances, the article of manufacture or kit includes (a) a PD-L1 axis binding antagonist (e.g., PD-L1 binding antagonist, e.g., an anti-PD-L1 antibody, e.g., atezolizumab (MPDL3280A)) and (b) a package insert including instructions for administration of the PD-L1 axis binding antagonist (e.g., PD-L1 binding antagonist, e.g., an anti-PD-L1 antibody, e.g., atezolizumab (MPDL3280A)) to an individual having a cancer (e.g., lung cancer (e.g., NSCLC), bladder cancer (e.g., UC), kidney cancer (e.g., RCC), or breast cancer (e.g., TNBC)), wherein, prior to treatment, the immune-score expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in a sample from the individual has been determined and the expression level of one or more of the genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA in the sample is above a reference immune-score expression level.

Any of the articles of manufacture or kits described may include a carrier means being compartmentalized to receive in close confinement one or more container means such as vials, tubes, and the like, each of the container means comprising one of the separate elements to be used in the method. Where the article of manufacture or kit utilizes nucleic acid hybridization to detect the target nucleic acid, the kit may also have containers containing nucleotide(s) for amplification of the target nucleic acid sequence and/or a container comprising a reporter-means, such as an enzymatic, florescent, or radioisotope label.

In some instances, the article of manufacture or kit includes the container described above and one or more other containers including materials desirable from a commercial and user standpoint, including buffers, diluents, filters, needles, syringes, and package inserts with instructions for use. A label may be present on the container to indicate that the composition is used for a specific application, and may also indicate directions for either in vivo or in vitro use, such as those described above. For example, the article of manufacture or kit may further include a container including a pharmaceutically-acceptable buffer, such as bacteriostatic water for injection (BWFI), phosphate-buffered saline, Ringer's solution, and dextrose solution.

The articles of manufacture or kits described herein may have a number of embodiments. In one instance, the article of manufacture or kit includes a container, a label on the container, and a composition contained within the container, wherein the composition includes one or more polynucleotides that hybridize to a complement of a gene listed herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) under stringent conditions, and the label on the container indicates that the composition can be used to evaluate the presence of a gene listed herein (e.g., one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA) in a sample, and wherein the kit includes instructions for using the polynucleotide(s) for evaluating the presence of the gene RNA or DNA in a particular sample type.

For oligonucleotide-based articles of manufacture or kits, the article of manufacture or kit can include, for example: (1) an oligonucleotide, e.g., a detectably labeled oligonucleotide, which hybridizes to a nucleic acid sequence encoding a protein or (2) a pair of primers useful for amplifying a nucleic acid molecule. The article of manufacture or kit can also include, e.g., a buffering agent, a preservative, or a protein stabilizing agent. The article of manufacture or kit can further include components necessary for detecting the detectable label (e.g., an enzyme or a substrate). The article of manufacture or kit can also contain a control sample or a series of control samples that can be assayed and compared to the test sample. Each component of the article of manufacture or kit can be enclosed within an individual container and all of the various containers can be within a single package, along with instructions for interpreting the results of the assays performed using the kit.

## VI. EXAMPLES

The following are examples of the methods of the invention. It is understood that various other embodiments may be practiced, given the general description provided above.

### Example 1. T cell expansion modes in tumor microenvironments and normal adjacent tissue correlate with distinct gene expression patterns

Upon encountering their cognate antigens, T cells can undergo clonal expansion to produce multiple copies of a cell with a shared T cell receptor (TCR). Despite the fundamental role of clonal expansion in cancer immunity, little is known about its relationship with T cell subpopulations or antitumor responses in cancer patients. This example describes experiments in which TCRs and RNA were sequenced from single CD3+ T cells from primary non-small cell lung cancer and matched normal adjacent tissues (NAT). The data obtained from these studies demonstrate that, although most clonotypes were represented by a single cell, the remaining clonal lineages showed expansion in either NAT or tumor exclusively, or dual-residence with expansion in both compartments. Activated CD4+ T cells exhibited NAT expansion; resident memory T cells exhibited tumor expansion; cytotoxic T lymphocytes cells exhibited dual expansion; and natural killer T cells and effector memory T cells exhibited dual expansion, with clone counts greater in NAT than in tumor tissue. Dual-resident T cell clones showed evidence of infiltration from blood, based, for example, on mass cytometry observations. A fraction of cytotoxic T cells appeared to be specific for known viral antigens. As described in further detail in Example 2, the data obtained from these experiments show that CTLs and dual-resident clones are strongly associated with clinical response to anti-PD-L1 antibody immunotherapy in lung and bladder cancer, indicating their utility as specific indicators of the overall antitumor immune response.

***T cell clones exhibit distinct residency patterns based on clone count and presence in tumor or normal adjacent tissue***

In these experiments, 149 million transcripts were analyzed from 48,701 CD3+ T cells from fresh surgical samples obtained from three treatment-naive NSCLC patients (identified here as Patient 1 with adenocarcinoma; Patients 2 and 3 with squamous cell lung cancer), yielding 18,194 distinct T cell clonotypes, each representing a distinct genetic lineage (FIG. 1A). TCR sequencing data were used to group T cells with common clonotypes and to measure the degree to which each clonotype had expanded. Although individual clonotypes were rarely shared across patients, it was observed that many clonotypes had cells residing in both tumor and corresponding NAT from a given patient. These dual-resident clones were found in all three patients and represented a substantial portion of multiplet clones (those with more than one cell) (FIG. 1B). Otherwise, clones were observed to be either singletons (having only one cell) or multiplets with varying degrees of clonal expansion that were exclusive to the tumor or NAT compartments (FIG. 1C). The availability of single-cell TCR data facilitated the assignment of attributes of each clone to its component cells, revealing distinctions among cells that would otherwise have been latent. For example, a clonotype-informed analysis showed that cells belonging to dual-resident clones constituted a substantial fraction (24-44%) of the T cells in each patient (FIG. 1D).

***Clonal residency patterns associate with distinct T cell subtypes***

To investigate these clonal residency patterns further, T cells from all 6 samples were organized into a 2-dimensional map based on their gene expression profiles from single-cell RNA sequencing (scRNA-seq), using the t-SNE algorithm (van der Maaten, L. and Hinton, G. J Machine Learning Research 9, 2579-2605, 2008). Canonical correlation analysis was applied to remove batch effects, with the resulting normalized gene expression profiles distributed evenly across the three patients (Butler, A. et al. Nature Biotechnology 36, 411-420, 2018). With the exception of one cluster (described below as 4.1c-Trm), all clusters had similar contributions from patients. Clear distinctions were observed in the t-SNE map among cells based on their clonal residency patterns, with significant overlap between tumor and NAT singletons, but distinct regions of the map were occupied by tumor multiplets, NAT multiplets, and dual-resident clones (FIG. 2A). Since distinct regions on t-SNE maps signify differences in gene expression, it was inferred that each clonal residency pattern might associate with specific T cell subtypes. To probe this association, unsupervised clustering was applied to the gene expression data, and the resulting T cell clusters were identified by the expression of characteristic markers (FIG. 2B).

To investigate whether this clustering could be refined, a second iteration of unsupervised clustering was applied to each initial cluster. The availability of single-cell TCR data afforded an opportunity to identify subclusters based on developmentally distinct T cell subtypes rather than gene expression differences that may stem from other factors, such as the environment of the cell. Subclusters corresponding to distinct clonal lineages were evident when the pool of cells from each clone was assigned to either subcluster, rather than divided proportionately to both subclusters. The large dataset used in this investigation, with 8 times as many cells as previous single-cell RNA-seq studies of tumor-infiltrating T lymphocytes (Savas, P. et al. Nat. Med. 13, 228 (2018); and Guo, X. et al. Nat. Med. 24, 978 (2018)), integrated existing classifications and characterized T cell subsets with greater detail and granularity.

As shown in FIG. 2C, this analysis revealed 11 major T cell clusters that could be distinguished on the basis of *CD8A* expression (3 clusters), *CD4* expression (5 clusters), and mixtures of both CD8+ and CD4+ cells (3 clusters). In general, CD8+ T cells divided into cells expressing cytolytic genes (cluster 8.1, also referred to herein as "Tcyt"), effector memory (Tern) cells (cluster 8.2), and resident memory (Trm) cells (cluster 8.3) that showed high expression of hallmark genes *ITGAE, HAVCR2, GZMA, GZMB,* and *ALOX5AP.* CD4+ cells also contained Trms (cluster 4.1), as well as an activated population of T cells expressing *CD40LG* (*CD154, CD40L*) but not *CCR7* (*CD197*) (cluster 4.2), regulatory T cells (Tregs, cluster 4.3), and *CCR7*+ T cells that also exhibited an increase in either heat shock proteins (cluster 4.4) or ribosomal proteins (cluster 4.5). Of the clusters that contained cells from both CD8+ and CD4+ lineages, one cluster (cluster 3.1) corresponded to natural killer-like T (NKT) cells, one cluster (cluster 3.2) expressed genes indicative of active mitosis, and one cluster (cluster 3.3) expressed primarily mitochondrial genes, as well as high levels of *MALAT1,* which is upregulated by hypoxia (Kolling, M. et al. Scientific Reports 8, 3438 (2018)). Notably, a cluster of CD4+ cells (cluster 4.1b) was identified as exhibiting high expression of *CXCL13* (*BLC, BCA-1*) and *IL21,* similar to those described recently as tumor-infiltrating follicular helper T (Tfh) cells, which have also been described in mice as *Foxp3*-negative, PD-1-high inhibitory CD4+ T cells (Zappasodi, R. et al. Cancer Cell 33, 1017 1032, 2018). These cells can promote the development of tertiary lymphoid structures (TLS) (Gu-Trantien, C. et al. JCI Insight 2, e91487 (2017)), which can provide a structured and coordinated immune response locally within the tissue (Dieu-Nosjean, M-C. et al. Trends in Immunology 35, 571-580, 2014) and could serve as a locus for clonal expansion (Thompson, E. D. et al. J Exp Med 207, 1791-1804, 2010). In addition, two clusters of resident memory T cells (clusters 8.3c and 4.1c) were identified with overlapping expression and comparably high expression of chemokines *CXCL13* and *CCL20* (*MIP3A*), the TNFR-superfamily member *TNFRSF9* (*CD137, 4-1BB*), and the proinflammatory cytokine *IL17A,* which is also known to promote TLS formation (Grogan, J. L. and Ouyang W. Eur J Immunol 42, 2255-2262 (2012)). These cells also expressed high levels of *PDCD1 (PD-1),* and may therefore match the *CD8+ CXCL13+ PD-1+* T cells recently found to be localized in intra- and peritumoral TLSs in close vicinity to Tfh cells in NSCLC (Thommen, D. S. Nat. Med. 24, 994-1004, 2018). Finally, the subclustering analysis indicated the presence of two subtypes of regulatory T cells, consistent with a similar finding elsewhere (Guo, X. et al. Nat. Med. 24, 978 (2018)). One of these subtypes, cluster 4.3b, exhibits higher expression of *IL2RA, IL1R2, TNFRSF18,* and *TNFRSF4,* suggesting that these cells may be more activated.

By matching the clonal residency patterns from FIG. 2A with the T cell subsets in FIG. 2C, it was determined that T cell subsets contained markedly distinct populations of singletons and multiplets in tumor, NAT, and in both tissues (FIGS. 2D and 2E). Tumor-resident multiplets represented major fractions of CD8+ and CD4+ Trms, while cells from dual-resident clones were frequent in Tcyts, CD8+ Terns, and NKT cells. Singletons were the largest constituents of CD4+ cell subtypes, with the exception of the aforementioned CD4+ Trms and the 4.2-Activated cluster, which included substantial numbers of NAT-resident multiplets. The high prevalence of CD4+ singletons may represent: (i) T cells that have not yet been adequately stimulated in the presence of tumor antigens, (ii) initial stages of clonal expansion; or (iii) residence in areas of stress that are unfavorable for expansion, such as hypoxia or nutrient deprivation (de Silly, R. V., Dietrich, P-Y., and Walker, P. R. Oncoimmunology 5, e1232236, 2016; and Chang, C-H. and Pearce, E. L. Nat. Immunol. 17, 364-368, 2016).

***T cell subtypes exhibit distinct clonal expansion behaviors***

A quantitative view of clonal expansion was obtained by assigning each clonotype to its most frequent T cell subtype. This view delineated the range of clonal residency patterns for each T cell subtype from global single-cell measurements, revealing several distinct clonal expansion behaviors (FIGS. 3A and 3B). Most clones of CD8+ Trms and CD4+ Trms showed expansion in the tumor compartment, while some clones from cluster 8.3a-Trm showed expansion in NAT. Clones of activated CD4+ T cells showed expansion exclusively in the NAT compartment. A high fraction of Tcyt-containing clones showed dual expansion in both tumor and NAT, while CD8+ Tem and NKT clones showed expansion to a greater degree in NAT compared with tumor tissue.

There are several possible explanations for the presence of NAT-predominant clonal cells. One possibility is that cells from these clones are excluded from the tumor compartment (Joyce, J. A. and Fearon, D. T. Science 348, 74-80, 2015), either by conditions that inhibit their migration or proliferation within tumors or by factors in the tumor microenvironment that are unfavorable to their survival. Another possibility is that CD8+ Tem and NKT cells respond to antigens or chemoattractants that are more prevalent in NAT compared with tumor (Ohri, C. M. et al. BMC Cancer 10, 172, 2010).

***Dual-resident and tumor-resident clones exhibit distinct antigenic specificity***

The high prevalence of dual-resident clones raised the question of whether these T cells are responding to tumor or non-tumor antigens. Although tumor-reactive TCRs have been studied only for a few individuals so far (Parkhurst, M. et al. Clin Cancer Res 23, 2491-2505, 2017), the VDJdb database (Shugay, M. et al. Nucleic Acids Res 46, D419-D427 (2018)) contains numerous CDR3 sequences specific to common viral antigens. Despite limited coverage in the emerging field of TCR-epitope association, it was determined that a fraction of the CDR3 amino acid sequences from the TCRs of patients studied in this example matched TCRs known to react against common viral antigens (cytomegalovirus, Epstein-Barr virus, and influenza A), with Tcyts matching at 8.7% prevalence, significantly greater than in other T cell subsets (chi-square $P < 2.2e-16$) (FIG. 4A). It was observed that the few viral epitopes available in the database were recognized by a diverse repertoire of CDR3 amino acid sequences, consistent with previous reports on the diversity of the TCR repertoire against individual viral epitopes (Wang, G. C. et al. Sci Transl Med 4, 128ra42, 2012; and Klinger, M. PLoS ONE 10, e0141561, 2015). Most of the viral-reactive clones were singletons, which may represent the baseline response to prior or continual low-grade viral exposure (Deliyannis, G. et al. J Virol 76, 4212-4221, 2002).

To confirm these results, a similar analysis was performed using 716,720 TCR sequences assembled from 9,142 bulk RNA-seq samples across 29 cancer types from the Cancer Genome Atlas (TCGA) (Li, B. et al. Nat. Genet. 48, 725-732, 2016). Although the combination of TCGA whole-transcriptome sequencing and TCR assembly lacks sufficient depth to identify tumor-reactive antigens shared across individuals, it was reasoned that CDR3 amino acid sequences in common across three or more individuals with different cancer types were likely to be reactive against viral antigens rather than tumor antigens. The current analysis using TCGA-derived CDR3 sequences (FIG. 4B) was very similar to that using VDJdb, showing that Tcyts had the largest fraction of cells matching an inferred viral-reactive TCR (chi-square $P < 2.2e-16$), and with the vast majority of matching clones being singletons.

With the caveat that TCRs known to react against viral antigens could be cross-reacting against tumor antigens (Birnbaum, M.E. et al. Cell 157, 1073-1087, 2014), the data obtained from these experiments suggest that many dual-resident clones consisting primarily of Tcyts may be reacting to non-tumor antigens. Tcyts are among many T cells that lack expression of *ENTPD1* (*CD39*), with low expression recently shown to represent "bystander" T cells specific to non-tumor antigens (Simoni, Y. et al. Nature 557, 575-579 (2018)). Until comprehensive databases of tumor-specific TCRs become available, it may be postulated that tumor-resident clones in the present dataset are more likely to be tumor-reactive. Nevertheless, related evidence suggests that cells expressing PD-1, such as tumor-resident Trms, are enriched for tumor reactivity (Gros, A. et al. J. Clin. Invest. 124, 2246-2259, 2014), and that tumor reactivity correlates with high levels of clonal expansion (Pasetto, A. et al. Cancer Immunol Res 4, 734-743, 2016). While investigating the relationship between clonal expansion and antigenic specificity, it was determined that subcluster 8.3a in the present t-SNE map (FIG. 2E) exhibited a gradient of clonal expansion in the tumor compartment. Further analysis revealed that highly expanding CD8+ Trm clones had increased *ENTPD1* expression, whereas clones with lower expansion expressed higher levels of *IFNG* and the interferon-induced transmembrane proteins *IFITM1, IFITM2,* and *IFITM3,* which are involved in the control of viral infections (Diamond, M. S and Farzan, M. Nat Rev Immunol 13, 46-57 (2013)).

The expansion and migration patterns of TILs, including T cells responding to non-tumor antigens, poses an open question (Mueller, S. N. et al. Ann Rev Immunol 31, 137-161, 2013; and Masopust, D. and Schenkel, J. M. Nat Rev Immunol 13, 303-320, 2013) about whether they are generated peripherally (e.g., in draining lymph nodes) and infiltrate the tumor site, or expand locally within the tumor or in a TLS. To explore this issue, the present analytical framework was applied to a recent study (Guo, X. et al. Nat. Med. 24, 978 (2018)) that included TCR sequencing of peripheral blood cells in addition to tumor and NAT from the same patients. Upon analyzing this data set, clones comprised almost entirely of the NKT subtype were identified. These clones were triple-expanded in tumor, NAT, and peripheral blood, suggesting that the clones had infiltrated tumor from the blood, egressed from the tumor into the blood, or both. In contrast, tumor-resident clones were generally not found in peripheral blood, consistent with other studies (Schenkel, J. M. and Masopust, D. Immunity 41, P886-897, 2014), indicating that their expansion may occur locally as a result of tumor antigen-specific stimulation.

***Protein expression by single-cell mass cytometry (CyTOF) recapitulates T cell subtypes and residency***

To complement the subtyping of T cells based on scRNA-seq, the expression of 33 proteins was quantified using mass cytometry by time of flight (CyTOF) in six NSCLC patients: three having squamous cell lung carcinoma, and three having lung adenocarcinoma. By applying the same computational pipeline as that used for the scRNA-seq studies described in this example, a similar set of T cell clusters were obtained. Corresponding clusters were identified based on correlations between scaled gene and protein expression and characteristic marker proteins.

The availability of peripheral blood samples in the CyTOF dataset allowed the comparison of population frequencies in tumor, NAT, and blood. The tumor compartment exhibited higher proportions of both CD4+ and CD8+ Trms than the other compartments, while peripheral blood had the highest proportions of CD4+ naive T cells. Cells corresponding to 8.1-Tcyt were found in all three compartments, with tumor and NAT proportions higher than those in peripheral blood, while cells corresponding to Tem and NKT cells were found in higher proportions in NAT and peripheral blood, compared with the tumor compartment, consistent with the NAT-predominant clonal patterns observed in the scRNA-seq data. These data further support the inferences from scRNA-seq described herein, particularly that tumor-resident T cell clones expand locally and that dual-resident clones may be infiltrating from blood.

*Highly expanded clones contain homogeneous mixtures of T cell subtypes*

In order to further understand the relationship between clonal lineage and T cell subtypes, the cellular composition of each highly expanded TCR clonotype, defined as one containing 10 or more cells, was analyzed (FIG. 4C). Each column represents a distinct set of CDR3 nucleotide sequences, although, as was observed with viral-reactive TCRs, different CDR3 sequences may potentially react to the same epitope. The resulting heat map revealed seven different mixtures of T cell subtypes. Four of these mixtures consisted of homogeneous clones of NKTs, Tcyts, CD8+ Terns, and Tregs, each containing only a single T cell subtype, with rare exceptions arising possibly from ambiguities T cell cluster assignment. Homogeneity within these clones indicates that these T cell subtypes are developmentally distinct from one another.

In addition to these predominantly homogeneous clones, some clones contained more than one T cell subtype. For example, in many cases, the same TCR clonotype was found within both CD8+ Terns and CD8+ Trms, supporting a lineage relationship between these T cell subtypes (Slutter, B. et al. Science Immunology 2, eaag2013, 2017), distinct from Tcyts or NKT cells, which did not share their TCR clonotypes with other T cell subtypes. Likewise, in the CD4+ lineage, the same TCR clonotype could be found within CD4+ activated T cells and CD4+ Trms, suggesting that the former differentiates into the latter.

Furthermore, a striking relationship was observed between clonal residency patterns and types of heterogeneity. Clones containing CD8+ Terns could be classified as NAT-resident, dual-resident, and tumor-resident, but in general, only the tumor-resident clones differentiated and expanded into CD8+ Trms (FIG. 4D). Likewise, CD4+ Terns were NAT-resident or tumor-resident, with the tumor-resident clones differentiating and expanding highly into CD4+ Trms (FIG. 4E) and the NAT-resident clones containing CCR7+ cells in clusters 4.4 and 4.5 (FIG. 4F). These observations suggest that residency in the tumor environment by clones of CD8+ Terns and CD4+ activated T cells precedes their differentiation into Trms and clonal expansion within that compartment. The data generated in this example also indicate that it is not individual cells in the tumor environment that determine differentiation into Trms, but the residency of the entire clone that is the distinguishing factor.

Cells from the 3.2-Mitotic cluster provided indications as to whether clonal expansion was occurring locally or prior to migration into the tumor site. Although mitotic cells represented a small fraction (4%) of the overall population, they were distributed across highly expanded clonotypes, with 42% of highly expanded clonotypes containing at least one mitotic cell and with locations that were consistent with the clonal residency pattern (FIG. 4G). These mitotic cells suggest that at least part of the clonal expansion behavior observed in this study reflects local expansion in tumor and NAT, as opposed to infiltration into the tumor site.

***Conclusion***

In summary, the experiments described in this example applied single-cell TCR and RNA sequencing, as well as mass cytometry (CyTOF), to construct comprehensive maps of the gene expression and expansion behavior of T cells infiltrating tumors and adjacent tissues, yielding a detailed immune profile of the antitumor T cell response in NSCLC patients. In comparison with CyTOF, which could assay proportions of T cell subtypes in each compartment only independently, the combination of single-cell TCR and RNA sequencing was able to reveal how individual clonal lineages distribute across both compartments and across T cell subtypes.

The present analysis of paired tumor and NAT samples provides new insights into the interrelationship among clonal expansion, gene expression, and antigenic specificity. Several phenotypic differences were identified among dual-resident, tumor-resident, and NAT-resident clones. These differences are summarized in Table 11, shown in Example 2, below. Many of the differences shown in Table 11 derive from their association with distinct T cell subsets. Whereas Trm clonal expansion is restricted primarily to the tumor, CD8+ Tcyts, CD8+ Terns, and activated CD4+ T cells exhibit expansion in NAT as well. In addition, to the extent possible with available data, the data generated in this example suggest that a fraction of Tcyts recognize viral antigens, and circulate to or from peripheral blood, as opposed to Trms, which appear to be expanding locally in the tumor compartment and differentiating from tumor-resident clones containing CD8+ Tem and CD4+ activated T cells.

**Materials and Methods**

Reagents and antibodies

Antibodies used in FACS experiments are as follows, listed as antigen: supplier, clone name, lot number, catalogue number, and fluorochrome.

Flow cytometry antibodies:

CD45: Biolegend, 2D1, B237418, 368512, APC.
CD3: Biolegend, HIT3a, B256208, 300308, PE.
EpCAM: Biolegend, 9C4, B255542, 324222, PE/Cy7.
CD56: Biolegend, 5.1 H11, B263355, 362546, FITC.
CD14: Biolegend, 63D3, B257234, 367116, FITC.
CD11b: Biolegend, ICRF44, B218669, 301330, FITC.
CD16: Biolegend, B73.1, B238206, 360716, FITC.
CD19: Biolegend, HIB19, B2448329, 302206, FITC.

Antibodies used for CyTOF analysis are listed in Table 6, below.

**Table 6. Antibodies used for CyTOF analysis**

| Mass | Metal | Target Antigen |
|---|---|---|
| 89 | Y | CD45 |

(continued)

| Mass | Metal | Target Antigen |
|---|---|---|
| 113 | In | EpCAM |
| 115 | In | CD57 |
| 140 | Ce | EQ Beads |
| 141 | Pr | Perforin |
| 142 | Nd | CCR4 |
| 143 | Nd | CD127/ CD137/CCR8 |
| 144 | Nd | Granzyme B |
| 145 | Nd | CD4 |
| 146 | Nd | CD8 |
| 147 | Sm | CD11c |
| 148 | Nd | CD56 |
| 149 | Sm | Granzyme A |
| 150 | Nd | CD103 |
| 151 | Eu | ICOS |
| 152 | Sm | CD155 PVR/ CD39 |
| 153 | Eu | TIM3 |
| 154 | Sm | CD3 |
| 155 | Gd | CD27 |
| 156 | Gd | CXCR3 |
| 157 | Gd | CD14 |
| 158 | Gd | OX40 |
| 159 | Tb | CD226 |
| 160 | Gd | Tbet |
| 161 | Dy | CTLA4 |
| 162 | Dy | Foxp3 |
| 163 | Dy | Eomes |
| 164 | Du | CD161 |
| 165 | Ho | CD19 |
| 166 | Er | NKG2D |
| 167 | Er | CCR7 |
| 168 | Er | Ki67 |
| 169 | Tm | CD25 |
| 170 | Er | CD45RA |
| 171 | Yb | PDL1 |
| 172 | Yb | CD28 |
| 173 | Yb | TIGIT |
| 174 | Yb | HLA-DR |
| 175 | Lu | PD-1 |

(continued)

| Mass | Metal | Target Antigen |
|---|---|---|
| 176 | Yb | CD38 |
| 191 | Ir | Nucleic acid |
| 192 | Pt | Cisplatin |
| 193 | Ir | Nucleic acid |
| 195 | Pt | Cisplatin |
| 209 | Bi | CD16 |

Software versions

Computational analysis was performed using Cell Ranger software (10x Genomics, Pleasanton, CA) version 2.1.0, Perl version 5.18.2, R version 3.5.1, and the following packages and versions in R for analysis: CATALYST, 1.4.2 (Chevrier, S. et al. Cell Systems 6,612-620.e5, 2018); flowCore, 1.46.2 (Hahne, F. et al. BMC Bioinformatics 10, 106 (2009)); GenomicDataCommons, 1.4.3 (Morgan, M. and Davis, S. R. bioRxiv, https://doi.org/10.1101/117200, 2017); GEOquery, 2.48.0 (Davis, S. and Meltzer, P. S. Bioinformatics 23, 1846-1847, 2007); monocle, 2.8.0 (Qiu, X. et al. Nature Methods 14, 979-982, 2017); multiGSEA, 0.11.1; qvalue, 2.12 (Storey, K.D. J. Royal Stat Soc, Series B, 64, 479-498 (200)), Seurat, 2.3.4 (Butler, A. et al. Nature Biotechnology 36, 411-420, 2018); WGCNA, 1.66 (Langfelder, P. and Horvath, S. BMC Bioinformatics 9, 559, 2008); and survival, 2.42-6. Figures and tables were generated using the following packages and versions in R: colorspace, 1.3-2; dplyr, 0.7,8; ggplot2, 3.1.0; grid Extra, 2.3; RColorBrewer, 1.1-2; and superheat, 1.0.0 (Davis, S. and Meltzer, P. S. Bioinformatics 23, 1846-1847, 2007). The above R packages depended secondarily on the following support packages: Biobase, 2.40.0; BiocGenerics, 0.26.0; cowplot, 0.9.3; DDRTree, 0.1.5; edgeR, 2.13.0; irlba, 2.3.2; limma, 3.38.2; magrittr, 1.5; Matrix, 1.2-15; ranger, 0.10.1; and VGAM, 1.0-6.

Tissue dissociation

Surgical resections from treatment-naïve patients with NSCLC were procured (Folio Biosciences, Powell, OH). Fresh samples were separated into tumor and NAT compartments by the reviewing pathologist and shipped overnight to the site at which the experiments described in Example 1 were conducted. Upon arrival, samples were rinsed with PBS until no traces of blood wee visually detected. Subsequently, samples were digested with a combination of Collagenase D (0.5mg/ml) and DNAse (0.1 mg/ml) from 15min at 37°C with gentle shaking. Subsequently, samples were subjected to a gentleMACS dissociator (Miltenyi Biotec) and additional 10 min incubation at 37°C.

PBMC isolation

For CyTOF analysis, peripheral blood mononuclear cells from patients were isolated using 50mL LEUCOSEP™ tubes (Greiner Bio-One International, Germany) and FICOLL-PAQUE™ PLUS (GE Healthcare, Sweden). Whole blood drawn into sodium heparin blood collection tubes and diluted with phosphate-buffered saline (PBS) without calcium or magnesium (Lonza, Walkersville, Maryland) was centrifuged for 15 minutes at 800 × g at room temperature (RT). PBMCs were harvested and washed with PBS and subsequently centrifuged for 10 minutes at 250 × g at RT before further processing.

Pre-enrichment and FACS sorting

Following tissue enzymatic dissociation of tissues, single-cell suspensions were subjected to one round of live cell enrichment using magnetic separation, followed by antibody staining with a cocktail of anti-CD45 and -CD3 antibodies for identification of T cells, anti-EPCAM antibodies for exclusion of epithelial cells, and anti-CD56, -CD14, CD16, -CD11b, and -CD19 antibodies for exclusion of non-T cells from the sorting gate. Cells were purified by fluorescence-activated cell sorting (FACS) on a Becton Dickinson FACSAria cell sorter equipped with 4 lasers (405nm, 488nm, 561 nm, 638nm). A 70-micron nozzle running at 70 psi and 90kHz was used as the setup for each sort session. FACS gates were drawn to include only live single cells. Further gates were drawn to arrive at CD3+CD45+EpCAM- cells.

Single-cell RNA-seq and TCR V(D)J clonotype profiling

Sample processing for single-cell gene expression (RNA-seq) and T cell receptor (TCR) V(D)J clonotype was performed using Chromium Single Cell 5' Library and Gel Bead Kit (10x Genomics, Pleasanton, CA) following the manufacturer's user guide. Cell density and viability of FACS-sorted CD3+ T cells from tumors and NAT were determined by Vi-CELL XR cell counter (Beckman Coulter). All of the processed samples had cell viability at >90%. The cell density was used to impute the volume of single cell suspension needed in the reverse transcription (RT) master mix, aiming to achieve ~6,000 cells per sample. After Gel Bead-in-Emulsion reverse transcription (GEM-RT) reaction and clean-up, a total of 14 cycles of PCR amplification was performed to obtain sufficient cDNAs used for both RNA-seq library generation and TCR V(D)J targeted enrichment followed by V(D)J library generation. TCR V(D)J enrichment was performed per manufacturer's user guide using Chromium Single Cell V(D)J Enrichment Kit, Human T cell (10X Genomics). Libraries for RNA-seq and for TCR V(D)J were prepared following the manufacturer's user guide (10x Genomics), then profiled using Bioanalyzer High Sensitivity DNA kit (Agilent Technologies) and quantified with Kapa Library Quantification Kit (Kapa Biosystems). Single-cell RNA-seq libraries were sequenced in one lane of HiSeq4000 (Illumina); single-cell TCR V(D)J libraries were pooled and then sequenced in one lane of HiSeq2500 (Illumina); sequencing was done according to the manufacturer's specification (10x Genomics).

Processing of single-cell TCR sequencing data

T cell receptor (TCR) sequencing data for each sample was processed using Cell Ranger software with the command "cellranger vdj" and the pre-built human reference package to obtain a possible assignment of a clonotype for each cell, resulting in the output file
filtered_contig_annotations.csv, one per sample.

Because the Cell Ranger software is designed to assign clonotypes separately for each sample and the present analysis depends on identifying shared clonotypes across samples, a Perl script was written that re-assigns clonotypes across samples with a universal identifier. This script uses the set of CDR3 consensus nucleotide sequences from the filtered_contig_annotations.csv files, and considers two cells to have the same clonotype when they shared all alpha-chain CDR3 and all beta-chain CDR3 consensus nucleotide sequences in common. The script was tested on each sample individually to confirm that it recapitulated the classification of clonotypes produced by the Cell Ranger software on single samples.

Clones were classified into different clonal residency patterns based on counts of the cells in the tumor and NAT compartments. Tumor and NAT singletons were clones that contained a single cell in the tumor or NAT compartment, respectively. Clones with more than one cell were classified as multiplets. If all cells from a clone were exclusively from tumor or NAT compartments, they were classified as tumor and NAT multiplets, respectively. Otherwise, the remaining clones, which had cells from both tumor and NAT compartments, were dual-resident clones.

Processing of single-cell RNA sequencing data

Single-cell RNA-seq data were processed with Cell Ranger software. Illumina base call (BCL) files were converted to FASTQ files with the command "cellranger mkfastq". Expression data were processed with "cellranger count" using a custom reference package of 30,727 genes, based on human reference genome GRCh38 and RefSeq gene models

Counts of transcripts, measured as unique molecular identifiers (UMs), were extracted from the filtered barcodes.tsv, genes.tsv, and matrix.mtx files to construct matrices in R statistical software. The raw data were analyzed data to remove potential multiplets, by plotting the number of genes present in each cell against its total count of transcripts, or library size. By visual inspection of these plots for regions of linearity, cutoffs for the minimum and maximum numbers of genes were selected as follows: sample T1: 500-4500 (removing 0.2% of cells); N1, 400-4500 (0.1 %); T2. 500-4500 (0.1 %); N2, 400-4500 (0.2%); T3, 300-2300 (0.3%); N3, 300-3000 (0.1%).

Analysis of the gene expression dataset obtained in this example was performed using the Seurat package (Morgan, M. and Davis, S. R. bioRxiv, https://doi.org/10.1101/117200, 2017). The example protocol posted by the package authors was applied in order to combine data from different samples using canonical correlation analysis (CCA). Specifically, the NormalizeData, FindVariableGenes, and ScaleData functions were applied to regress out the number of distinct UMIs. The 1000 most variable genes were selected from each sample, as computed by the FindVariableGenes function, with the requirement that a gene be present in two or more samples, yielding a universe of 1101 genes. These genes were then used as input into the RunMultiCCA function to compute 20 canonical vectors. The AlignSubspace function was then run using the default reduction type of canonical correlation analysis, grouping by the 6 samples, and aligning dimensions 1 through 12.

Dimensionality reduction and clustering

For dimensionality reduction and clustering, the RunTSNE and FindClusters functions were applied, using the CCA aligned variables for the dimensional reduction on dimensions 1 through 12 and the default resolution of 0.8 for clustering. The FindClusters function in Seurat has been found to be one of the most accurate available (Duo, A. et al. F1000Research 7, 1141, 2018). In this automated clustering procedure, 10 clusters were obtained, ranging in size from 8267 to 2295 cells. Mapping of the cells from each patient onto this common map showed that the CCA procedure largely removed sample-specific batch effects, with each cluster represented by all three patients.

The large size of the present dataset enabled the consideration of a second level of clustering. Although a clustering can be forced, it is not certain whether such clusters correspond to developmentally distinct clonal lineages. Therefore, the present TCR information was applied in order to assist with this determination systematically. For each proposed division of a cluster into two subclusters, based on the FindClusters procedure from Seurat, clonotypes with 10 or more cells overall were identified. The division of cell counts was then plotted for each clonotype assigned to the two subclusters. It was reasoned that if cells from the same clonal lineage divided evenly between the two subclusters, then the subclusters did not correspond to developmentally distinct subtypes. However, if cells from the same clonal lineage partitioned largely into one subcluster or the other, then the subclusters did describe developmentally distinct subtypes. Based on this TCR-guided subclustering analysis, evidence was found for developmentally distinct subclusters in clusters 1, 2, 5, 8, and 9. Additional support for these subclusters came from the tSNE map, clonal residency patterns, and the identification of biomarkers for each subcluster. In particular, cluster 1 had one cluster with high clonal expansion in tumor and one with high clonal expansion in NAT. Cluster 2 showed evidence of three subclusters by gene expression. Cluster 5 was separated into two distinct regions in the tSNE map. Clusters 8 and 9 showed distinct biomarkers identifying each subcluster.

Division of cluster 5 yielded a subcluster containing 974 cells with both *CD8A* and CD4 expression, which were interpreted as two distinct clonal lineages having similar gene expression. To divide this cluster, cluster labels of other cells in the clones were used to classify 801 clones as predominantly CD8 or CD4. The remaining 173 clones were classified with the ClassifyCells function using the 801 classified clones as training data. The resulting classification yielded clusters 8.3c-Trm and 4.1c-Trm.

For analyses at the clonotype level requiring a single T cell subtype per clone, clones were assigned to the T cell subtype that was the most prevalent among its cells. In cases where more than one T cell subtype was among the most prevalent, assignment was made randomly among the ties.

Identification of differentially expressed and characteristic marker genes

For analyses requiring characterization of differentially expressed genes within or between subsets, the Monocle 2 package was used, which enabled the assignment of p-values to all requested genes. Raw data was converted from the Seurat objects into Monocle format using the importCDS function with import_all set to TRUE. The function estimateSizeFactors was then applied. Differentially expressed genes and associated P-values were obtained using the differentialGeneTest command.

To identify characteristic marker genes for each subset, a procedure for the task of classification was required, whose objective function is the smallest misclassification rate, rather than differential expression, whose objective function is a difference in means divided by the standard deviation. To perform this classification, logistic regression was performed using the glm function in R under the binomial family, where the response variable was a categorical variable indicating membership or non-membership in a given subset, and the predictor variables were the raw counts of a given gene across cells and the library size of the cells. This was treated as a full model, and the reduced model was considered to be the one with library size alone as the predictor, so the measurement of each gene as a potential biomarker was given by the deviance of the entire model minus the deviance of the reduced model. The sign of the coefficient for the gene counts indicated whether the gene was positively or negatively associated with membership.

Comparison of clusters across datasets

The same dimensionality reduction, clustering, and marker gene identification steps were applied to three publicly available single-cell RNA-seq datasets on tumor-infiltrating lymphocytes. The University of Melbourne study (Savas, P. et al. Nat. Med. 13, 228 (2018)) involved T cells from 2 tumor samples from patients with breast cancer, using the 10X Genomics platform for single-cell RNA-Seq. The data from GSE110686, provided in the form of barcodes.tsv, genes.tsv, and matrix.mtx files, were extracted. These data were processed in Seurat similar to the present analysis, except that since cells were already combined into a single dataset, the 1000 most variable genes were used without canonical correlation analysis. RunPCA was conducted instead. FindClusters was performed with a resolution of 0.8, which yielded 12 clusters, ranging in size from 1248 to 42 cells.

The Peking University study (Guo, X. et al. Nat. Med. 24, 978 (2018)) involved T cells from 14 patients with non-small cell lung cancer (11 adenocarcinomas and 3 squamous cell carcinomas), analyzed at the single-cell level using the Smart-Seq2 protocol for full-length transcriptome profiling. The data from GSE99254, provided in the form of a large matrix of counts, were extracted. The authors provided an identifier across 16 clusters for each cell, which was used in the present example instead of performing clustering de novo. The authors also provided, clonotype assignments, which were used in the present example for analyzing clonal residency patterns in tumor, NAT, and peripheral blood.

The study from Memorial Sloan-Kettering Cancer Center (Azizi, E. et al. Cell 174, 1-16, 2018) involved 11 breast cancer patients. Eight of the patients had single-cell RNA-Seq data on the full set CD45+ immune cells, while three patients had both single-cell RNA-Seq and TCR sequencing data from 10X Genomics from T cells in the tumor compartment. The latter three samples were analyzed in the present example given the interest in T cells. The data from GSE114727, provided in the form of barcodes.tsv, genes.tsv, and matrix.mtx files for 5 samples from three patients, were extracted. These data were processed in the same way as the data generated in the present example, with canonical correlation analysis to merge the five samples, and performing clustering using FindClusters with a resolution of 0.8. This process yielded 14 clusters, ranging in size from 6758 to 254 cells.

For each cluster in each study, the same procedure was performed for identifying characteristic biomarker genes as that used in the present example. The 20 genes with positive coefficients and highest deviance values were selected for investigation.

Analysis of clonal expansion behaviors

For the analysis shown in FIG. 3A, clonotypes were divided into four categories: tumor-exclusive, defined as having no counts in NAT; NAT-exclusive, having no counts in tumor; tumor-predominant; having counts in both tumor and NAT, with the tumor count greater than the NAT count; and NAT-predominant, having counts in both tumor and NAT, with the NAT count greater than the tumor count. These categories are depicted by the regions in each scatterplot in FIGS. 3A and 3B. Within each category of clonotypes and within each T cell subset, a Poisson test was performed in R to determine the presence of clonal expansion greater than the overall observed behavior. The Poisson test was performed in a one-sided manner to test for the alternative hypothesis that clonal expansion was greater than the overall observed behavior. The parameter lambda for the null hypothesis was taken from the total set of clonotypes before dividing them into T cell subsets, and found to be 1.64 for the tumor-exclusive clones; 1.53 for the NAT-exclusive clones; 11.38 for the tumor-predominant clones; and 15.10 for the NAT-predominant clones. The p-values are reported in each region of each scatterplot of FIG. 3A, with values less than 0.05 printed in red.

Comparison with TCR sequences from VDJdb and TCGA

The 2018-06-04 release of VDJdb is publically available by way of the internet (Shugay, M. et al. Nucleic Acids Res 46, D419-D427 (2018)). Using this version of the VDJdb, entries in which the TCR species was "HomoSapiens" and the epitope species was either "CMV", "EBV", or "InfluenzaA" were extracted and analyzed. Each clonotype from the data generated in this example were compared against the cdr3 field in the VDJdb database, requiring an exact match from any of the CDR3 amino acid sequences for the clonotype.

A set of 716,720 TCR sequences assembled from TCGA bulk RNA-Seq reads were obtained from the authors of the study (Wang, G.C. et al. Sci Transl Med 4, 128ra42, 2012). Each sequence was labeled with a putative CDR3 amino acid sequence in the header of the FASTA file. Each clonotype from the present example was compared, requiring an exact match from any of the CDR3 amino acid sequences for the clonotype against any substring of the TCGA-derived CDR3 sequence. Each FASTA file was annotated with an aliquot ID, which was linked to a cancer type using the R library GenomicDataCommons. A TCGA-derived CDR3 sequence was considered to be likely viral-reactive when it was observed in three or more individuals, all with different cancer types, and no two individuals had the same cancer type.

Staining of cells for CyTOF analysis

Washed patient PBMCs, tumor, and normal adjacent tissue infiltrating immune cells were resuspended at a cell concentration of $10^7$ cells/mL with PBS. Cells were then incubated with a viability reagent, CELL-ID™ cisplatin (Fluidigm, South San Francisco, California) at a final concentration of 5 $\mu$M for 5 minutes on ice. Viability staining was quenched with a 5x volume of MAXPAR™ Cell Staining Buffer (Fluidigm, South San Francisco, California) and centrifuged at 300 $\times$ g; cells were then re-suspended to a final concentration of 30 million cells/mL in staining buffer. For antibody labeling, at most 3 million cells were transferred to FALCON™ 5 mL 12 $\times$ 75 mm tubes (Corning, New York). To block Fc receptor binding, cells were incubated with 5 $\mu$L of Human TruStain FCX™ (BioLegend, San Diego, California) for 10 minutes on ice. A master mAb cocktail containing all metal-conjugated surface antibodies was added to samples for cell-surface staining and incubated for 30 minutes on ice. Cells were then washed once with 4 mL cell staining buffer to prepare for intracellular staining. Briefly, cells were resuspended in 1 mL of fixation/permeabilization solution by using the FoxP3 Staining Buffer Set (Affymetrix, eBioscience, San Diego, California) for 45 minutes on ice, washed with 3 mL of permeabilization buffer at 800 × g for 5 minutes, and re-suspended in 50 μL of permeabilization buffer. Cells were then stained for intracellular targets by addition of 50 μL antibody cocktail. After 30 minutes incubation on ice, the cells were washed with 4 mL cell staining buffer and fixed overnight at 4°C in a 1 mL solution containing CELL-ID™ Intercalator-Ir in 1.6% EMS Fix (Electron Microscopy Sciences, Hatfield, Pennsylvania).

Acquisition on CyTOF instrument

Cells were washed with 3 mL of MAXPAR™ cell staining buffer and centrifuged at 800 × g for 5 minutes A round of 4mL MAXPAR™ water (Fluidigm, South San Francisco, California) was used as a wash thereafter. In order to perform cell counting, cells were resuspended in 1mL of MAXPAR™ water. After obtaining cell counts, 3 mL of MAXPAR™ water was added and cells were pelleted one last time prior to instrument acquisition. Before introduction into the HELIOS™, a CyTOF system (Fluidigm, South San Francisco, California), pelleted cells were resuspended with MAXPAR™ water containing EQ™ Four Element Calibration Beads (Fluidigm, South San Francisco, California) and filtered using a 12 × 75 mm tube with a 35 μm nylon mesh cell-strainer cap (Corning, New York).

Data processing and analysis of CyTOF data

All FCS files were normalized using the MATLAB™ (MathWorks, Natick, Massachusetts) normalizer. CD3+, CD14-, CD19-, EPCAM- cells were selected from the population of singlet live CD45+ cells. Data were exported using FLOWJO™ software (Ashland, Oregon, version 10.2) into FCS version 3.0 files, and subsequently read using the flowCore package in R using default settings for read.FCS. Cross-talk between heavy metal isotopes was compensated for using the CATALYST package in R and the spillover matrix from the ss_exp dataset in the package, which covers pairwise cross-talk between mass channels 139, 141-156, and 158-176. This matrix contained spillover estimates for all pairs of isotopes in the present dataset, except for In 113 (used for EPCAM in the present study) to In115 (CD57), and from Gd157 (CD14) to Gc155 (CD27), Gd158 (OX40), Gd160 (Tbet), Sm152 (CD155_PVR), Sm154 (CD3), and Yb173 (TIGIT). However, both EPCAM and CD14 were selected against in the present gating procedure. Compensated FCS version 3.0 files were created using the non-negative least squares method to avoid generating negative values for expression.

To make analysis comparable between CyTOF and scRNA-Seq data, used the same analysis procedures from Seurat were used, based on 33 proteins in common across all 17 samples. Data for proteins CD127, CD137, CD155, and CCR8 were excluded because they were assayed in only some and not all of the samples. Data for proteins CD14, CD19, and EPCAM were excluded since they were selected against in the gating procedure. As with the scRNA-Seq data, samples were combined using canonical correlation analysis. It was found that the AlignSubspace command in Seurat required large amounts of memory, with the procedure failing upon reaching 100,000 total cells, despite computers used in this study having 128 GB of random access memory. Therefore, although the present dataset contained measurements on 1,074,626 CD3+ cells, the cells were sampled so as to yield a total of 24,000 tumor cells, 24,000 NAT cells, and 24,000 PBMC cells. To attain these numbers for the tumor and PBMC cells, a random selection of 4000 cells was obtained from each of the 6 patients. For the sampling of NAT cells, since a NAT sample was lacking for patient 2, and patient 7 had only 2156 cells total from the NAT compartment, 5461 samples were sampled from each of the remaining 4 patients to obtain the desired total of 24,000 NAT cells.

The FindClusters procedure using a resolution of 0.8 (the same used for scRNA-Seq) yielded 9 clusters, and the t-SNE procedure provided a 2-dimensional visualization of these clusters. Mapping of the cells from each patient onto this common map showed that the CCA procedure largely removed sample-specific batch effects, with each cluster represented by all 6 patients.

To find correspondences between the CyTOF and scRNA-Seq clusters, centroids of gene expression were computed for each cluster. Each of the 33 proteins from the CyTOF assay was matched to its equivalent mRNA transcript measured in the RNA-Seq assay. Some proteins had matches to multiple mRNA transcripts. For example, CD16 matches both *FCGR3A* and *FCGR3B*, so the average of both values from the RNA-Seq centroids was taken. Likewise, the average for *HLA-DRB1, HLA-DRB3, HLA-DRB4, HLA-DRB5,* and *HLA-DRB6* was taken to match against the HLA-DR protein. CD45RA represents an alternative splice form that is not captured in the CellRanger output, and was therefore excluded from this correspondence analysis. Centroids were computed by scaling each gene by dividing by its standard deviation and taking the mean value of each gene within each cluster. A pairwise correlation analysis was then performed between each CyTOF and each scRNA-Seq cluster, computing a robust correlation coefficient from the WGCNA R package across the 33 mean protein and gene expression values.

**Example 2. Relationship between gene expression patterns and responsiveness to PD-L1 axis binding antagonist therapy**

Across the TIL populations of the three patients analyzed in Example 1, variability was observed in the proportions of both clonal residency patterns (FIG. 1D) and T cell subsets. Especially apparent were differing fractions of Tcyt, Tern, and NKT cells across patients (FIG. 5A). It was thus explored whether gene expression patterns among TIL populations might be associated with clinical response to PD-L1 axis binding antagonist therapy, such as atezolizumab. To this end, gene set enrichment analysis (GSEA) (Subramanian, A. et al. Proc Natl Acad Sci USA 102, 15445-15550, 2005; and Lamb, J. et al. Science 313, 1929-1935, 2006) was applied to pre-treatment bulk tumor RNA-seq data from a randomized phase II clinical trial (POPLAR, (Fehrenbacher, L. et al. Lancet 387, 1837-1846 (2016))) that compared the anti-PD-L1 antibody atezolizumab with the chemotherapeutic agent docetaxel in 193 patients with NSCLC. A similar analysis was conducted using gene expression data from a single arm trial (IMvigor210 (Hoffman-Censits, J. H. et al. J Clin Oncology 34, 355-355 (2016))) of atezolizumab in 354 patients with locally advanced or metastatic urothelial carcinoma. The results of these analyses reveal gene signatures that are positively correlated with favorable response to PD-L1 axis binding antagonist therapy in these indications. These results are described in further detail in the sections that follow.

***Dual-resident T cell gene signatures correlate with patient responsiveness to PD-L1 axis binding antagonist therapy in non-small cell lung cancer and urothelial carcinoma***

From the POPLAR clinical trial data, a set of genes was identified as significantly associated with progression-free survival and overall survival following treatment. These genes were then matched against the genes for each T cell cluster, ranked according to the statistical significance of their differential expression in the scRNA-seq data (FIGS. 5B and 5C). Several statistically significant associations with response to atezolizumab were identified (local false discovery rate < 0.05), including Tcyts, two CD8+ Trm subsets, one NKT subset, and CD4+ activated T cells, and the *IL2RA*- and *IL1R2*-positive activated subset of Tregs (cluster 4.3b). By comparison, none of the associations with response to docetaxel were statistically significant. The association of 4.3b-Tregs with response to atezolizumab is particularly notable, as this subset was found by others (Guo, X. et al. Nat. Med. 24, 978 (2018)) to associate with poor prognosis in lung adenocarcinoma.

Gene signatures of cells in highly expanded tumor-resident multiplets, NAT-resident multiplets, and dual-residency clones were additionally identified (FIG. 6). Kaplan-Meier analysis of the POPLAR data revealed that a gene signature associated with dual-resident clones was strongly associated with both overall (HR = 0.54; P = 0.034) and progression-free (HR = 0.58; P = 0.02) survival in patients treated with atezolizumab but not in those treated with docetaxel (FIGS. 5D and 5E). In contrast, a tumor-resident clonal signature (FIGS. 5F and 5G) and NAT-resident clonal signature (FIGS. 5H and 5I) exhibited less significant associations with OS and PFS response to anti-PD-L1 therapy. The gene signature of dual-resident clones found to exhibit a strong and statistically significant association with OS and PFS response in the POPLAR trial is set forth in Table 7, below.

**Table 7: Genes expressed by dual-resident T cells and positively correlated with responsiveness to atezolizumab therapy**

| | | | |
|---|---|---|---|
| CST7 | CCL5 | MT-CYB | PLEK |
| NKG7 | CD8A | MT-ND4L | CTSW |
| GZMH | CMC1 | KLRG1 | HLA-C |
| MT-ND4 | CD8B | MT-CO2 | LYAR |
| HLA-H | HCST | MT-ATP6 | LITAF |

The gene signatures of tumor-resident and NAT-resident T cell clones, which were less strongly associated with responsiveness to PD-L1 axis binding antagonist therapy, are shown in Tables 8 and 9, respectively.

**Table 8: Genes expressed by tumor-resident T cells**

| | | | |
|---|---|---|---|
| ALOX5AP | CTSD | COTL1 | SNX9 |
| CD7 | GAPDH | SRGN | PDE7B |
| LOC101929531 | PDCD1 | NR3C1 | TIGIT |

(continued)

| | | | |
|---|---|---|---|
| RGS1 | ITGAE | HAVCR2 | CXCL13 |
| CD3D | CD2 | DUSP4 | TPI1 |

**Table 9: Genes expressed by NAT-resident T cells**

| | | | |
|---|---|---|---|
| S100A4 | ZFP36L2 | CD69 | CD97 |
| S100A6 | S100A10 | VIM | TXNIP |
| LGALS1 | MYL12A | ANKRD28 | ND3 |
| CD52 | TSC22D3 | CRIP1 | LGALS3 |
| SH3BGRL3 | AHNAK | LINC00892 | ND5 |

Following this discovery, a subsequent experiment was conducted in order to investigate whether an association exists between the genes expressed by dual-resident, tumor-resident, and NAT-resident T cell clones set forth in Tables 7-9, above, with responsiveness to PD-L1 axis binding antagonist therapy in urothelial carcinoma. To this end, the association analysis described above for the POPLAR trial was repeated using gene expression data from a single arm trial (IMvigor210 (Hoffman-Censits, J. H. et al. J Clin Oncology 34, 355-355 (2016))) of atezolizumab in 354 patients with locally advanced or metastatic urothelial carcinoma. The results of this investigation are shown in FIGS. 5J - 50. In this setting, genes expressed by dual-resident T cells, shown in Table 7, above, were strongly associated with favorable OS and PFS response to atezolizumab therapy (FIGS. 5J and 5K). Gene expression signatures from tumor-resident T cells (FIGS. 5L and 5M) and NAT-resident T cells (FIGS. 5N and 5O) exhibited less significant associations with OS and PFS response.

Taken together, these findings highlight the association between clonal expansion behavior and clinical outcome of cancer immunotherapy, such as in the treatment of lung and bladder tumors.

***Genes expressed by dual-resident T cell clones in a distinct patient population are also associated with responsiveness to PD-L1 axis binding antagonist therapy***

The association experiments described in this example have, thus far, focused on whether a correlation exists between the gene signatures shown in Tables 7-9, above, with responsiveness to PD-L1 axis binding antagonist therapy, such as atezolizumab. Having demonstrated that the dual-resident T cell genes shown in Table 7 are positively correlated with responsiveness to this form of treatment, the POPLAR and IMvigor210 association experiments described above were repeated to determine whether a correlation exists between patient responsiveness and a gene signature of dual-resident T cell clones obtained from a different NSCLC patient population, which was recently reported by Guo and colleagues (Guo, X. et al. Nat. Med. 24, 978 (2018), the disclosure of which is incorporated herein by reference in its entirety). Guo et al. performed deep single-cell RNA sequencing on T cells isolated from 14 treatment-naïve NSCLC patients, including 11 adenocarcinomas and three squamous cell carcinomas. Although a NAT-resident clonal signature was not identified from the dataset of Guo et al. due to the preponderance of NKT cells in NAT-expanded clones (FIGS. 10A - 10J), the dual-resident T cell gene signature from this dataset showed a strong association with responsiveness to atezolizumab in both the POPLAR trial of NSCLC (FIGS. 11A- 11D) and the IMvigor210 trial of urothelial carcinoma (FIGS. 11E - 11H).

The dual-resident T cell gene signature obtained from the dataset of Guo et al., which, in this example, has been shown to be positively associated with responsiveness to PD-L1 axis binding antagonist therapy, is provided in Table 10, below.

**Table 10. Genes expressed by dual-resident T cells in a distinct patient population and found to be positively correlated with responsiveness to atezolizumab therapy**

| | | | |
|---|---|---|---|
| GZMB | KLRC4-KLRK1 | CTSW | CCL5 |
| KLRD1 | KLRK1 | GZMA | GNLY |
| FGFBP2 | GZMH | ID2 | PRF1 |
| NKG7 | B2M | CX3CR1 | PATL2 |

(continued)

| | | | |
|---|---|---|---|
| CST7 | LITAF | PRSS23 | CD8A |

A comparison of Tables 7 and 10 reveals that the gene signature of dual-resident T cell clones obtained from patients studied in this example (Table 7) and the gene signature of dual-resident T cell clones obtained from patients studied by Guo et al. (Table 10) have the following genes in common: NKG7, CST7, GZMH, LITAF, CTSW, CCL5, and CD8A. These findings further support the positive correlation between a patient's propensity to respond favorably to PD-L1 axis binding antagonist therapy, such as atezolizumab, and the expression of genes associated with dual-resident T cell clones.

***Expression levels of genes associated with responsiveness to atezolizumab therapy are correlated with one another***

Following the identification of the genes, set forth in Table 7, that are expressed by dual-resident T cell clones from the patients studied in this example, it was discovered that the expression levels of individual genes within this panel are highly correlated with one another (FIG. 7). In view of the high cross-correlation of the genes recited in Table 7, it is appreciated that the expression of individual genes, and/or a sub-panel of these genes (e.g., a sub-panel of two, three, four, five, six, seven, eight, nine, ten, or more, of the genes set forth in Table 7) may confer the same predictive information as the entire panel. This observation provides an important clinical benefit, as one need not determine the expression levels of all 20 genes set forth in Table 7 in order to assess the likelihood that a patient will respond to a PD-L1 axis binding antagonist. Rather, one may make this determination on the basis of the expression level of individual genes recited in Table 7, or based on the expression level of a sub-panel of genes set forth in Table 7 (e.g., a sub-panel of two, three, four, five, six, seven, eight, nine, ten, or more, of the genes set forth in Table 7).

***Predictive utility can be improved by assessing the combined expression levels of cytotoxic** and **mitochondrial genes***

The gene correlation data shown in FIG. 7 demonstrate the existence of two subsets of genes within the panel set forth in Table 7. Particularly, Table 7 includes a set of cytotoxic genes (containing PLEK, HCST, CTSW, KLRG1, CD8A, CCL5, CD8B, CST7, NKG7, GZMH, and HLA-C) as well as a set of mitochondrial genes (containing MT-ND4L, MT-ND4, MT-ATP6, MT-CO2, and MT-CYB). As shown in FIG. 7, the genes within each of these subsets exhibit a particularly high internal correlation. The individual genes within each of these subsets thus confer the same predictive information as one another and as the entire subset. The Kaplan-Meier curves shown in FIGS. 8A - 8N illustrate the predictive utilities of several of these cytotoxic and mitochondrial genes, both alone and in combination with one another, as biomarkers for atezolizumab treatment outcomes among NSCLC patients in the POPLAR trial. Similarly, FIGS. 9A - 9F provide Kaplan-Meier curves showing the predictive utilities of the full panels of cytotoxic genes and mitochondrial genes recited above, both alone and in combination with one another. These figures demonstrate that, surprisingly, the predictive utility of a gene or panel of genes set forth in Table 7 can be improved by combining the expression level of one or more cytotoxic genes and the expression level of one or more mitochondrial genes in an assessment of a patient's likelihood of benefiting from PD-L1 axis binding antagonist therapy. An example of this improvement in predictive utility is shown in FIGS. 8A - 8N for the combination of the cytotoxic gene CST7 with the mitochondrial gene MT-ND4, MT-CYB, and MT-CO2.

This unexpected discovery, coupled with the high internal correlation of the cytotoxic and mitochondrial genes within Table 7, engenders a significant medical benefit. Particularly, based on these findings, the propensity of a patient (e.g., a cancer patient, such as a NCSLC or urothelial carcinoma patient) to respond to PD-L1 axis binding antagonist therapy can be determined with improved accuracy by evaluating one or more of the cytotoxic genes set forth in Table 7 in combination with one or more of the mitochondrial genes set forth in Table 7 in making the determination. This improved accuracy can increase the safety and efficacy of a PD-L1 axis binding antagonist for a given patient population, enabling patients identified as likely to benefit from this form of therapy to be treated accordingly while safeguarding patients determined to be less likely to respond from a potentially unnecessary medication. Given the high correlation of the genes within each of the cytotoxic and mitochondrial subsets, it is not necessary to use the entire panel of cytotoxic genes in combination with the entire panel of mitochondrial genes in order to achieve this improvement in accuracy. Rather, a patient's likelihood of benefiting from PD-L1 axis binding antagonist therapy can be determined with improved accuracy by combining the expression levels of one or more individual cytotoxic genes with one or more individual mitochondrial genes.

***Presence of unique T cell clonotypes***

Taken together, the data generated in Examples 1 and 2 demonstrate the presence of distinct T cell clonotypes in cancer patients, such as patients having lung and/or bladder tumors. The characteristics of these discrete T cell populations are summarized in Table 11, below.

**Table 11: Summary of characteristics across clonal residency patterns**

| | Dual-resident clones | Tumor multiplets | NAT multiplets |
|---|---|---|---|
| **T cell subtypes** | Cytotoxic T lymphocytes (Tcyt) Natural killer T (NKT) cells CD8+ T effector memory (Tern) cells | CD8+ T resident memory (Trm) cells CD4+ Trm cells | CD4+ activated T cells |
| **Clonal expansion behavior** | Unconstrained (Tcyt) NAT-predominant (NKT, Tern) | Tumor expansion | NAT expansion |
| **Matches to viral antigens** | More prevalent (Tcyt) | Less prevalent | Less prevalent |
| **Presence in peripheral blood** | Abundant. Evidence for NKT expansion. | Low levels | Uncertain |
| **Composition of clones** | Homogenous for Tcyts, NKT cells, and dual- or NAT-resident clones. Tumor-resident CD8+ Tem cells share clonotypes with CD8+ Trms. | CD8+ Trms share clonotypes with tumor-resident CD8+ Terns. CD4+ Trms share clonotypes with tumor-resident CD4+ activated T cells. | Share clonotypes with CD4+ Trm cells when tumor-resident. Share clonotypes with CCR7+ T cells when tumor-resident. |
| **Response to anti-PD-L1 immunotherapy** | Strongly associated | Associated | Not associated |

***Conclusion***

Although much of the prevailing focus of cancer immunotherapy has centered on the modulation of checkpoint inhibitors (e.g., PD-1, LAG-3, TIM-3, and TIGIT) abundantly expressed on resident memory T cells, the findings described in this example demonstrate that other T cell subtypes also contribute to the complexity of the antitumor response. Specifically, the present data show that responsiveness to PD-L1 axis binding antagonist therapy, such as atezolizumab, correlates with the expression of dual-resident T cell gene signatures, such as the dual-resident T cell gene signatures shown in Tables 7 and 10, above. Moreover, the magnitude of dual-resident clones may reflect the scope and quality of the patient's immune response to the tumor (Chen, D. S. and Mellman, I. Nature 541, 321-330 (2017)).

***Materials and Methods***

Phase II clinical trials of atezolizumab

Atezolizumab is a humanized monoclonal antibody against PD-L1. POPLAR (ClinicalTrials.gov registration number NCT01903993) is a randomized phase II trial of atezolizumab (n=93 patients with RNA-seq profiles) vs. docetaxel (n=100 patients with RNA-seq profiles) in 2L+ NSCLC. IMvigor210 is a single arm phase II trial of atezolizumab in both 1L (cohort 1, n=95, registration number NCT02951767) and 2L+ (cohort 2, n=259, registration number NCT021 08652) metastatic or locally advanced urothelial carcinoma. Overall response rate (ORR, RECIST1.1) and overall survival (OS) were obtained. Bulk transcriptomes from a single tumor biopsy or resection per patient obtained before atezolizumab or docetaxel treatment were characterized by RNA-seq. Data from each trial were processed as described previously (Fehrenbacher, L. et al. Lancet 387, 1837-1846 (2016); and Hoffman-Censits, J. H. et al. J Clin Oncology 34, 355-355 (2016)), normalized with TMM (trimmed mean of M-values), and log2-transformed.

Gene set enrichment analysis between T cell subsets and clinical trial data

The 1101 most highly variable genes were obtained from the Seurat-based analysis described in Example 1. 95 genes from the POPLAR dataset were matched among the 1101 genes analyzed in the present study with a p-value < 0.05 and a hazard ratio < 0.8 for overall survival (OS) as genes with positive association with atezolizumab response, and 37 with a p-value < 0.05 and a hazard ratio > 1.25 for OS as genes with a negative association with atezolizumab response. Likewise, 54 and 20 genes were identified with positive and negative association, respectively, with docetaxel response.

For each T cell subset, the Monocle package was used, applying the function estimateSizeFactors on the raw counts, and differentialGeneTest on the 1101 genes, using membership in the subset as the predictor, yielding a p-value for each gene. The mean expression of each gene for cells in the subset and outside of the subset was computed to determine if the gene was up- or down-regulated in the subset. Since the resulting profiles included both up- and down-regulated genes, the approach from the Connection Map project was followed (Lamb, J. et al. Science 313, 1929-1935, 2006), which performs a signed gene set enrichment analysis, looking for associations with the extreme ends of the profiles. Ranks (as determined by p-values) were then computed for the clinically responsive genes with positive association with survival against the up-regulated genes for each subset, as well as for the clinically responsive genes with negative association with survival against the down-regulated genes for each subset. These ranks yielded an enrichment score for the up-regulated genes, and an enrichment score for the down-regulated genes, with the maximum of these two scores used as the statistic for the given T cell subset. Statistical significance was determined by comparing the observed statistic against those derived from 100,000 random permutations of ranks. Adjustment was made for multiple comparisons across the 18 p-values tested for each drug to obtain local false discovery rates, or q-values, using the qvalue package in R.

Signature scoring in bulk tumor RNA-seq data from phase II trials

For each signature, an expression score was calculated for each patient within each trial, by calculating the z-score for each gene across the dataset (after log2-transformation), and computing the mean z-score from the genes forming the signature of interest. The signature score was further dichotomized as high (>= trial cohort median) or low (< trial cohort median) in POPLAR. For the univariate analysis of OS in POPLAR, a Cox-proportional hazards model was fitted to obtain log-ranked p-values for each gene, using the survival package in R. In the single-arm trial IMvigor210, several thresholds were tested for signature dichotomization (30%, 40%, 50%, 60% and 70%), and resulting hazard ratios with 95% confidence intervals were represented as a forest plot.

**Example 3. Determining the propensity of a patient to respond to PD-L1 axis binding antagonist therapy and treatment of the patient accordingly**

Using the compositions and methods described herein, the likelihood that a patient having a cancer will be responsive to PD-L1 axis binding antagonist therapy can be determined. For example, a patient having a cancer described herein, such as lung cancer (e.g., non-small cell lung cancer), bladder cancer (e.g., urothelial carcinoma), kidney cancer (e.g., renal cell carcinoma), or breast cancer (e.g., triple-negative breast cancer) may be subjected to one or more gene expression assays in order to determine whether the patient is likely to respond to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). To make this determination, a physician may determine the expression level of one or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, GPR56, IFITM2, IFITM1, TMSB10, CD247, COX2, COX1, CLIC3, S100A4, and CYBA (e.g., of a panel of genes set forth in any one of Tables 1-4, above, and/or a panel of genes that includes one or more cytotoxic genes and one or more mitochondrial genes) in a sample, such as a tumor tissue sample, obtained from the patient prior to any treatment of the patient with a PD-L1 axis binding antagonist. The gene expression analysis may be conducted using one or more methods described herein, such as polymerase chain reaction approach or an RNA-seq assay. Using the gene expression data obtained from these experiments, an immune-score expression level of the one or more genes may be determined. The immune-score expression level of the one or more genes may then be compared to a reference immune-score expression level, such as an immune-score expression level of the one or more genes in a reference population. A finding that the immune-score expression level of the one or more genes in the sample obtained from the patient exceeds the reference immune-score expression level indicates that the individual is likely to be responsive to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)). Alternatively, a finding that the immune-score expression level of the one or more genes in the sample obtained from the patient is below the reference immune-score expression level indicates that the individual is less likely to be responsive to treatment including a PD-L1 axis binding antagonist (e.g., PD-L1 binding antagonist (e.g., anti-PD-L1 antibody, e.g., atezolizumab (MPDL3280A)) or PD-1 binding antagonist (e.g., anti-PD-1 antibody)).

The PD-L1 axis binding antagonist may be, for example, a PD-L1 binding antagonist, such as an anti-PD-L1 antibody or antigen-binding fragment thereof. In some embodiments, the anti-PD-L1 antibody is atezolizumab. Thus, if the patient is determined to be likely to respond to PD-L1 axis binding antagonist therapy, atezolizumab may be selected for administration to the patient. Atezolizumab may be administered to the patient using any one or more of the dosing schedules and routes of administration described herein. For example, the atezolizumab may be administered to the patient at 1200 mg intravenously every three weeks. The dose may be administered as a single dose or as multiple doses (e.g., 2, 3, 4, 5, 6, 7, or more than 7 doses), such as infusions.

The atezolizumab may be administered to the patient alone or in combination with one or more additional anti-cancer therapies, such as an immunomodulatory agent described herein. For example, the atezolizumab may be administered to the patient in combination with one or more anti-TIGIT antibodies and antigen-binding fragments thereof, anti-CTLA-4 antibodies or antigen-binding fragments thereof, anti-CD27 antibodies or antigen-binding fragments thereof, anti-CD30 antibodies or antigen-binding fragments thereof, anti-CD40 antibodies or antigen-binding fragments thereof, anti-4-1 BB antibodies or antigen-binding fragments thereof, anti-GITR antibodies or antigen-binding fragments thereof, anti-OX40 antibodies or antigen-binding fragments thereof, anti-TRAILR1 antibodies or antigen-binding fragments thereof, anti-TRAILR2 antibodies or antigen-binding fragments thereof, anti-TWEAK antibodies or antigen-binding fragments thereof, anti-TWEAKR antibodies or antigen-binding fragments thereof, anti-BRAF antibodies or antigen-binding fragments thereof, anti-MEK antibodies or antigen-binding fragments thereof, anti-CD33 antibodies or antigen-binding fragments thereof, anti-CD20 antibodies or antigen-binding fragments thereof, anti-CD52 antibodies or antigen-binding fragments thereof, anti-A33 antibodies or antigen-binding fragments thereof, anti-GD3 antibodies or antigen-binding fragments thereof, anti-PSMA antibodies or antigen-binding fragments thereof, anti-Ceacan 1 antibodies or antigen-binding fragments thereof, anti-Galedin 9 antibodies or antigen-binding fragments thereof, anti-HVEM antibodies or antigen-binding fragments thereof, anti-VISTA antibodies or antigen-binding fragments thereof, anti-B7 H4 antibodies or antigen-binding fragments thereof, anti-HHLA2 antibodies or antigen-binding fragments thereof, anti-CD155 antibodies or antigen-binding fragments thereof, anti-CD80 antibodies or antigen-binding fragments thereof, anti-BTLA antibodies or antigen-binding fragments thereof, anti-CD160 antibodies or antigen-binding fragments thereof, anti-CD28 antibodies or antigen-binding fragments thereof, anti-CD226 antibodies or antigen-binding fragments thereof, anti-CEACAM1 antibodies or antigen-binding fragments thereof, anti-TIM3 antibodies or antigen-binding fragments thereof, anti-CD96 antibodies or antigen-binding fragments thereof, anti-CD70 antibodies or antigen-binding fragments thereof, anti-CD27 antibodies or antigen-binding fragments thereof, anti-LIGHT antibodies or antigen-binding fragments thereof, anti-CD137 antibodies or antigen-binding fragments thereof, anti-DR4 antibodies or antigen-binding fragments thereof, anti-CR5 antibodies or antigen-binding fragments thereof, anti-FAS antibodies or antigen-binding fragments thereof, anti-CD95 antibodies or antigen-binding fragments thereof, anti-TRAIL antibodies or antigen-binding fragments thereof, anti-DR6 antibodies or antigen-binding fragments thereof, anti-EDAR antibodies or antigen-binding fragments thereof, anti-NGFR antibodies or antigen-binding fragments thereof, anti-OPG antibodies or antigen-binding fragments thereof, anti-RANKL antibodies or antigen-binding fragments thereof, anti-LTβR antibodies or antigen-binding fragments thereof, anti-BCMA antibodies or antigen-binding fragments thereof, anti-TACI antibodies or antigen-binding fragments thereof, anti-BAFFR antibodies or antigen-binding fragments thereof, anti-EDAR2 antibodies or antigen-binding fragments thereof, anti-TROY antibodies or antigen-binding fragments thereof, and anti-RELT antibodies or antigen-binding fragments thereof.

Following administration of the PD-L1 axis binding antagonist to the patient, the physician may monitor the patient's responsiveness to the selected form of therapy. Responsiveness to the PD-L1 axis binding antagonist therapy may manifest as prolonged overall survival and/or progression-free survival, among other metrics described herein.

## Example 4. Peripheral clonal expansion of T lymphocytes associates with tumor infiltration and response to cancer immunotherapy.

### Summary

Despite the resounding clinical success in cancer treatment by antibodies that block the interaction of PD-1 with its ligand PD-L1, key unknowns remain concerning the mechanisms responsible. The most widely presumed mechanism assumes that PD-1 blockade acts to reverse a terminally differentiated or "exhausted" phenotype exhibited by chronically stimulated T cells, as originally described during chronic virus infection (Mellman, I., Coukos, G., and Dranoff, G. Nature 480, 480-489, 2011; Wherry, E. J. and Kurachi, M. Nature Rev Immunol 14, 486-499, 2015; Topalian, S. L., Drake, C. G., and Pardoll, D. M. Cancer Cell 27, 450-461, 2015). However, recent observations have questioned this model, showing that the terminally exhausted phenotype is epigenetically locked and difficult to alter (Pauken, K. E. et al. Science 354, 1060-1065, 2016; Philip, M. et al. Nature 545, 562-456, 2017; Khan, O. et al. Nature 571, 211-218, 2019; Scott, A. C., et al. Nature 571, 270-274, 2019). A major limitation to understanding the origin and fate of T cells in tumor immunity is the lack of quantitative information on the distribution of individual T cell clonotypes in cancer patients. In this Example, single-cell RNA sequencing (scRNA-seq) and deep analysis of TCR clonotypes (scTCR-seq) were performed in cancer patients across several indications, assessing the profiles of TCRs in the various populations of T cells in tumors, normal adjacent tissue (NAT) and peripheral blood. In a subset of patients, clear evidence was found of clonotypic expansion of T effector- and effector memory-like cells not only within the tumor but also in NAT. Patients with gene signatures of such clonotypic expansion appear to respond best to anti-PD-L1 therapy. Importantly, expanded clonotypes found in the tumor and NAT can also typically be detected in peripheral blood, suggesting a convenient approach to patient identification. Analysis of these data suggests that intratumoral T cells, especially in responsive patients, are continuously replenished with fresh, non-exhausted replacement cells from sites outside of the tumor, suggesting continued activity of the cancer immunity cycle in these patients, the acceleration of which may be associated with clinical response.

**Results**

325 million mRNA transcripts were sequenced across 142,752 TILs from fresh surgically resected samples taken from 14 treatment-naïve patients with four different cancer types (FIGS. 16A - 16C). For each patient, samples were obtained from tumor, non-tumor normal adjacent tissue (NAT), and, in 4 patients, from peripheral blood. TCR sequencing yielded one or more CDR3 regions of alpha or beta chains in 99,788 cells. T cells were grouped into 56,975 distinct clonotypes by matching all sequenced CDR3 regions, which allowed the measurement of clonal expansion and the tracking of clonal lineages across tissues. Although individual clonotypes were rarely shared across patients, many clonotypes resided in both the tumor and corresponding NAT from a given patient (FIG. 16D). Such dual-expanded clones contrasted with expanded clones residing solely in one compartment, which were termed tumor and NAT multiplets, and with singleton clones having only one observed cell in either compartment.

Although the majority of clonotypes were singletons (FIG. 16E), representing a diverse repertoire of T cells, 9-18% of clonotypes in each patient were clonally expanded multiplets and dual-expanded clones. Dual-expanded clonotypes represented substantial fractions of the observed cells, sometimes constituting the majority of T cells in a given compartment (FIG. 16F). More insight was gained by surveying the patterns of individual clones plotted by their cell counts in NAT and tumor (FIG. 12A). In some patients, e.g., Colon1, dual-expanded clones aligned along the main diagonal, reflecting approximately equal cell counts in the two compartments and suggesting similar or parallel processes in those compartments, effected by multiple independent clonotypes. Whereas in other patients, dual-expanded clones were scattered from the main diagonal, indicating that clones were predominantly expanded in tumor or NAT, suggesting a divergent pattern of dual clonal expansion. One measure of this difference across patients is the Pearson correlation coefficient of tumor and NAT cell counts among dual-expanded clones.

It was hypothesized that parallel expansion in tumor and NAT represents infiltration of T cells from the periphery, extravasating into both equally from inflamed blood vessels, while divergent expansion might represent local migration, expansion, or attrition of T cells within tissue. Evidence for this concept was found from matched blood samples, where clones with parallel expansion in tumor and NAT were also highly expanded in blood (FIG. 12B). Patients with a parallel mode of expansion also showed high levels of lymphocytic infiltration into tumor and NAT (FIG. 12C - 12E). Importantly, the fraction of expanded clones in blood turned out to be a proxy for the extent of dual expansion, and in patients with strong dual expansion, TCRs detected as expanded in the blood accurately reflected the TCR repertoire of dual-expanded clones, capturing up to 87% of those TCRs (FIG. 12F). This sensitivity suggests a convenient diagnostic test for measuring dual expansion and associated TCRs of tumor infiltrating lymphocytes (TILs) and expands upon other proposals for blood-based assays in cancer immunotherapy (Gros, A. et al. Nat Med 22, 433-438, 2016; Schumacher, T. N. and Scheper, W. Nat Med 22, 340-341, 2016; Manjarrez-Orduño, N. et al. Front Immunol 9, 1613, 2018; Hogan, S. A. et al. Cancer Immunol Res 7, 77-85, 2019. The associations between peripheral and intratumoral clonal expansion were replicated and expanded (FIG. 12G) in an external dataset of scTCR-seq from 14 NSCLC samples (Guo, X. et al. Nat Med 24, 978-985, 2018). (FIGS. 12H - 12I, FIG. 17). Overall, the results show that peripheral clonal expansion is associated with infiltration in both tumor and NAT.

The features of clonal expansion were explored further by simultaneously measuring gene expression using scRNA-seq. Unsupervised cluster analysis assigned T cells to clusters, as depicted in a two-dimensional map (FIG. 13A), corresponding to distinct CD8+ and CD4+ T cell subtypes or physiological states. Cells were classified using reference gene signatures from published sources (Guo et al 2018, Yost, K. et al. Nat Med 25, 1251-1259, 2019) (FIG. 13B, FIG. 18A) and suggest that cluster 8.1 corresponds to external CX3CR1 and CD8_eff clusters, consistent with T effector (Teff) cells, and cluster 8.2 to GZMK and CD8_mem clusters, consistent with T effector memory (Tern) cells (Bratke, K. et al. Eur J Immunol 35, 2608-2616, 2005; Abdelsamed, H. A. et al. J Exp Med 214, 1593-1606, 2017). These subtype assignments were further supported by cluster-specific gene expression biomarkers identified using logistic regression (FIG. 13C, FIG.18B). Cluster 8.4 had high expression of the DNA replication factor MCM7, indicating active mitosis. Clusters 3.1 and 3.2 expressed abundant mitochondrial genes, suggestive of apoptosis. These clusters were set aside as generic CD3+ cells to simplify analysis.

The three clusters 8.3a, 8.3b, and 8.3c were considered resident memory (Trm) cells (Schenkel, J. M. and Masopust, D. Immunity 41, P886-897, 2014) based on abundant expression of ITGAE (CD103). These clusters could also be differentiated on the basis of low, intermediate, and high expression of activation-exhaustion markers, including PDCD1 (PD-1) (FIG. 13D upper panel, FIG. 18C), and also by a published gene signature for stem-like exhausted versus terminally exhausted CD8+ T cells (Im, S. J. et ai. Nature 537, 417-421, 2016) (FIG. 13D lower panel), consistent with recently identified categories of progenitor exhausted and terminally exhausted Trms (Miller, B. C. et al. Nature Immunol 20, 326-336, 2019). Trm subtypes also differed in their expression of activation markers CD69 and XCL1 (Lymphotactin); ZNF683 (Hobit), a regulator of tissue residency (Mackay, L. K. et al. Science 352, 459-463, 2016); and killer cell lectin-like receptors, such as KLRC2 (FIG. 13C). In addition, clusters 8.3a through 8.3c showed increasing prevalence in tumor versus NAT (FIG. 13E), supporting a relationship between PD-1 expression, terminal exhaustion, and increased exposure to cognate antigens within the tumor microenvironment (FIG. 13F) (Thommen, D. S. et al. Nat Med 24, 994-1004, 2018). Expanded CD8+ T cells in blood belonged primarily to clusters 8.1-Eff and 8.2- Tem (FIG. 13G), although the composition in blood varied greatly across the four patients (FIG. 18D, right).

Mapping of scTCR-seq data onto the gene expression map (FIG. 13H) and tabulation of clonal expansion patterns by clusters (FIG. 18D, left) revealed that CD4+ cells comprise singletons, except for tumor and NAT multiplet expansion behaviour of 4.1-S100A6, which are likely to be CD4+ Trms based on their expression of ITGAE (FIG. 13C, FIG. 18C). In contrast, CD8+ cells are largely dual-expanded, with some 8.2-Tem and 8.3-Trm cells also belonging to tumor multiplet clones. T cell clusters exhibited distinctive clonal expansion behaviours (FIG. 13I, FIG. 18E), with Teff cells exhibiting parallel infiltration of expanded blood clones into tumor and NAT, while Tem cells showed reduced though appreciable correlation among the three compartments plus possible local migration or attrition. In contrast, Trm cells rarely exhibited expansion in blood, indicating local mechanisms of expansion. The analysis of external datasets (Guo, X. et al. Nat Med 24, 978-985, 2018; Zhang, L. et al. Nature 564, 268-272, 2018) yielded similar results, with the analogous CX3CR1 and GZMK clusters showing a similar pattern of clonal expansion as the 8.1-Eff and 8.2-Tem clusters (FIG. 19A, FIG. 19B). One possible mechanism for parallel clonal expansion by effector T cells in tumor and NAT is extravasation through inflamed blood vessels as opposed to migration along a chemokine gradient centered in the tumor (Shulman, Z. et al. Nature Immunol 13, 67-76, 2012). Regardless of mechanism, the findings from this Example show that the patient variability observed previously in FIG. 12B may reflect varying proportions of Teff, Tern, and Trm cells, each with a particular mode of clonal expansion (FIG. 13J).

The data generated in this Example represent a snapshot of clone sizes across tumor, NAT, and blood representing the accumulation over time of various dynamic processes. To understand these dynamics, an external dataset (Yost 2019) of pre- and post-treatment T cells from basal cell carcinomas was analyzed, with the understanding that the two sets of measurements differ both by the passage of time and by immunotherapy. The published analysis demonstrates that novel clones appear with clonotypes distinct from those harbored by preexisting exhausted TILs. By matching the scTCR-seq of novel clones in tumor with bulk TCR-seq in pre-treatment blood, correlations were found in TCR representation (FIG. 14A), suggesting that the source of the novel clones is external to the tumor. Moreover, patients differed substantially in number and size of novel clones, and these differences correlated with their degree of clonal diversity in peripheral blood (FIG. 14B). However, the relationship to clinical response could not be inferred from these data.

Nevertheless, by distinguishing clones based on whether they shared TCR sequences with pretreatment blood (FIG. 14C, FIG. 14D), a remarkable observation was made: without TCR representation in blood, cells from expanded novel clones were largely exhausted (CD8_ex or CD8_ex_act), but with blood representation, such cells were nonexhausted (CD8_mem) or even activated (CD_act). This observation suggests two possible modes of clonal expansion: (1) a local expansion that is independent of blood that leads to exhausted T cells; and (2) an infiltrative process that introduces novel, nonexhausted clones into the tumor. Aside from novel clones, the remaining clones that were present at both time points were also expanded upon treatment when they matched pretreatment blood (FIG. 14C), suggesting that both novel and existing clones are continuously supplied by replacement cells from the periphery. A similar analysis applied to novel CD4+ clonotypes did not show substantial correlations with blood clonotypes (FIG. 20A) or clonal diversity across patients (FIG. 20B), but a possible pattern of blood-independent expansion was observed for T follicular helper cells (FIG. 20C).

The prevalence of infiltrating T cells, which may be reactive not only to the tumor but also to non-tumor antigens, could account for recent observations of "bystander" ENTPD1- (CD39-) T cells (Simoni 2018) and might help to explain recent findings that less than 10% of intratumoral CD8+ T cells are reactive against tumor (Scheper, W. et al. Nat Med 25, 89-94, 2019). The TCR repertoires from this Example were compared with databases of TCRs known or likely to react to common viral antigens (Shugay, M. et al. Nucleic Acids Res 46, D419-D427 (2018); Li, B. et al. Nat Genet 48, 725-732, 2016), and found that virally reactive T cells derived from a large repertoire of singleton clones plus a few highly expanded clones in each patient (FIG. 21A), and that dual-expanded clonotypes were most likely to match (FIG. 21B). The cluster with the most frequent matches was 3.2- RNR1 (FIG. 21C), whose gene signature of apoptosis is consistent with an attrition of T cells that fail to encounter their cognate antigen (McNally, J. M. et al. J Virol 75, 5965-5976, 2001).

As the analysis of both the data generated from this Example and external data suggests, patients vary in their extent of peripheral clonal expansion and resulting infiltration of T cells, and this variability could potentially associate with clinical response to immune checkpoint blockade. Bulk RNA-seq tumor samples were obtained in three randomized phase II trials of the anti-PD-L1 antibody atezolizumab (Mariathasan, S. Nature 22, 544-548, 2018; Fehrenbacher, L. et al. Lancet 387, 1837-1846 (2016); McDermott, D. F. et al. Nat Med 24, 749-757, 2018), which were evaluated using gene signatures of the CD8+ T cell clusters identified in this study (FIG. 15A). Consistent with the different but overlapping dual expansion profiles for the corresponding cell types (FIG. 13H, FIG. 18D), these gene signatures associated with clonal expansion behaviour (FIG. 15B), with the 8.1-Eff signature highly expressed in dual-expanded clones and 8.3c-Trm in tumor multiplets. The 8.2-Tem signature was also somewhat enriched in tumor-containing compartments relative to NAT singletons or multiplets. Since these gene signatures represent subtypes of CD8+ T cells, most of them correlated highly with CD8A expression (FIG. 22A), known to be an indicator of CD8+ T cell prevalence and predictive of response to immunotherapy (FIG. 22B, top left). The strongest correlations were with the 8.1-Eff and 8.2-Tem gene signatures, suggesting their relevance in explaining the variance of CD8+ T cell populations across patients. Genes in the 8.1-Eff and 8.2-Tem signatures individually showed a bias toward improved progression-free survival (PFS) (FIG. 15C), and their combined scores also associated with improved PFS (FIG. 15D) in several atezolizumab-containing arms, with the exception of monotherapy in IMmotion150, which may reflect a proposed role of immunosuppression by myeloid cells in the absence of bevacizumab in renal cell carcinoma (McDermott 2018).

Overall, this Example demonstrates that the presence of dual-expanded clones in tumors and the associated replenishment by T cells from outside the tumor can be a key factor in explaining patient variability and clinical benefit from cancer immunotherapy. Patients vary in their extent of peripheral clonal expansion, and the data generated in this Example show that the degree of multi-site expansion can be more predictive of response than the mere presence of CD8+ TILs. Clinical benefit could arise from a direct effect of PD-1/PD-L1 blockade on these populations of T cells (Yan, Y. et al. JCI Insight 3, e97828, 2018; Yamaguchi, K. Cancer Sci 109, 3032-3042, 2018), or because blockade can act to increase the production of committed anti-tumoral T cells only in those patients with an ongoing T cell response and continued replenishment of TILs. These observations suggest that the supply of T cells in tumor or NAT may depend on availability of effector and effector memory T cells in the periphery. They also raise the possibility that, in some patients, sampling T cells in blood may provide information about the TCR repertoire of tissue-resident T cells, expanding the possibilities for "liquid biopsies".

## Materials and Methods

Reagents and antibodies

Antibodies used in FACS sorting are listed below:

CD45: Biolegend, 2D1, B237418, 368512, APC or 368516 APC/Cy7
CD3: Biolegend, HIT3a, B256208, 300308, PE.
EpCAM: Biolegend, 9C4, B255542, 324222, PE/Cy7.
CD56: Biolegend, 5.1H11, B263355, 362546, FITC.
CD14: Biolegend, 63D3, B257234, 367116, FITC.
CD11b: Biolegend, ICRF44, B218669, 301330, FITC.
CD16: Biolegend, B73.1, B238206, 360716, FITC.
CD19: Biolegend, HIB19, B2448329, 302206, FITC.

Software versions

Computational analysis was performed using Cell Ranger software (10x Genomics, Pleasanton, CA) version 2.2.0 (Zheng, G. X. Y. et al. Nature Communications 8, 14049, 2017), Perl version 5.18.2, R version 3.6.0, and the following packages and versions in R for analysis: Seurat, 3.0.2 (Stuart, T. et al. Cell 177, 1888-1902, 2019); limma, 3.41.15 (Ritchie, M.E. et al. Nucleic Acids Research 43, e47, 2015); annotate_1.63.0; homologene, 1.4.68.19.3.27; mouse4302.db, 3.2.3; RankProd, 3.11.0 (Del Carratore, F. et al. Bioinformatics 33, 2774-2775, 2017); qvalue, 2.17.0 (Storey, J. D. Journal of the Royal Statistical Society, Series B 64, 479-498, 2002); SingleR, 1.0.1 (Aran, D. et al. Nat Immunol 20, 163-172, 2019); and survival, 2.44-1.1 (Therneau, T. M. and Grambsch, P. M. Modeling Survival Data: Extending the Cox Model, Springer, New York, 2000). Figures were produced using the following packages and versions in R: colorspace, 1.4-1 (Zeileis, A. et al. colorspace: a toolbox for manipulating and assessing colors and palettes. Technical Report 1903.06490, arXiv.org, 20192019); RColorBrewer, 1.1-2; and pheatmap, 1.0.12. Data for external datasets was obtained using GenomicDataCommons, 1.9.0 (Morgan, M. T. and Davis, S. R. Genomic Data Commons:

a Bioconductor interface to the NCI Genomic Data Commons, Technical Report 117200, bioRxiv.org, 2017) and GEOquery, 2.53.0 (Davis, S. and Meltzer. Bioinformatics 14, 1846-1847, 2007).

Tissue dissociation

Surgical resections from treatment-naive patients with tumors classified as Endometrial, NSCLC, RCC and CRC were procured (Discovery Life Sciences, iSpecimen Inc, Avaden BioSciences and TriMetis Life Sciences). Fresh samples were separated into tumor and NAT compartments by the reviewing pathologist and shipped overnight. Upon arrival, samples were rinsed with PBS until no traces of blood were visually detected. Subsequently, samples were digested with a combination of Collagenase D (0.5mg/ml) and DNAse (0.1 mg/ml) from 15min at 37° C with gentle shaking. Subsequently, the samples were subjected to a gentleMACS dissociator (Miltenyi Biotec) followed by an additional 10 min incubation at 37 ° C.

PBMC isolation

Peripheral blood mononuclear cells from patients were isolated using 50mL Leucosep™ tubes (Greiner Bio-One International, Germany) and Ficoll-Paque ™ PLUS (GE Healthcare, Sweden). Whole blood drawn into sodium heparin blood collection tubes were 3x diluted with phosphate-buffered saline (PBS) without calcium or magnesium (Lonza, Walkersville, Maryland). Diluted cell suspension were carefully layered on Leucosep tubes and centrifuged for 15 minutes at 800 $\times$ g at room temperature (RT). Interphase containing PBMCs were harvested and washed with PBS and subsequently centrifuged for 10 minutes at 250 $\times$ g at RT before further processing.

Pre-enrichment and FACS sorting

Following tissue enzymatic dissociation of tissues, single-cell suspensions were subjected to one round of live cell or CD45+ enrichment using magnetic bead separation followed by antibody staining with a cocktail of anti-CD45 and -CD3 for identification of T cells, anti-EPCAM for exclusion of epithelial cells, and anti-CD56, -CD14, CD16, -CD11b, and -CD19 for exclusion of non-T cells from the sorting gate. Cells were purified by fluorescence-activated cell sorting (FACS) on a Becton Dickinson FACSAria cell sorter equipped with 4 lasers (405nm, 488nm, 561nm, 638nm). A 70-micron nozzle running at 70 psi and 90kHz was used as the setup for each sort session. FACS gates were drawn to include only live single cells based on Calcein Blue AM+ and Propedium Iodide- (Thermo Fisher Scientific). Further gates were drawn to arrive at CD3+CD45+EpCAMor CD45+EpCAM- cells.

Single-cell RNA-seq and TCR V(D)J clonotype profiling

Sample processing for single-cell gene expression (RNA-seq) and T cell receptor (TCR) V(D)J clonotype was done using the Chromium Single Cell 5' Library and Gel Bead Kit (10x Genomics, Pleasanton, CA) following the manufacturer's user guide. Cell density and viability of FACS-sorted CD3+ T cells or CD45+ ceiis from tumors, NAT, and blood were determined by Vi-CELL XR cell counter (Beckman Coulter). All of the processed samples had cell viability at >90%. The cell density was used to impute the volume of single cell suspension needed in the reverse transcription (RT) master mix, aiming to achieve ~6,000-10,000 cells per sample. After Gel Bead-in-Emulsion reverse transcription (GEM-RT) reaction and clean-up, a total of 14 cycles of PCR amplification was performed to obtain sufficient cDNAs used for both RNA-seq library generation and TCR V(D)J targeted enrichment followed by V(D)J library generation. TCR and BCR V(D)J enrichment was done per manufacturer's user guide using Chromium Single Cell V(D)J Enrichment Kit, Human T cell (10X Genomics). Libraries for RNA-seq and V(D)J were prepared following the manufacturer's user guide (10x Genomics), then profiled using Bioanalyzer High Sensitivity DNA kit (Agilent Technologies) and quantified with Qubit (Thermo Fisher Scientific) or Kapa Library Quantification Kit (Kapa Biosystems). Single-cell RNA-seq libraries were sequenced in one lane of HiSeq4000 (Illumina); single-cell TCR V(D)J libraries were pooled and then sequenced in one lane of HiSeq2500 (Illumina); sequencing was done according to the manufacturer's specification (10x Genomics).

Processing of single-cell TCR sequencing data

T cell receptor (TCR) sequencing data for each sample was processed using Cell Ranger software with the command "cellranger vdj" and the pre-built human reference package, to obtain a possible assignment of a clonotype for each cell, resulting in the output file
filtered_contig_annotations.csv, one per sample.
Because the Cell Ranger software assigns clonotypes separately for each sample, whereas the present analysis depends on identifying shared clonotypes across samples, clonotypes were regrouped across the tumor, NAT, and blood samples for each patient, requiring that they share all reported alpha- and beta-chain CDR3 consensus nucleotide sequences in common. This procedure was tested on each sample individually and confirmed that it grouped clonotypes consistently with the original clonotypes from the Cell Ranger software.

Processing of single-cell RNA sequencing data

Single-cell RNA-seq data was processed with Cell Ranger software. Illumina base call (BCL) files were converted to FASTQ files with the command "cellranger mkfastq". Expression data were processed with "cellranger count" using a custom reference package of 30,727 genes, based on human reference genome GRCh38 and RefSeq gene models and provided in the data and software package (see section below on Data and code availability), together with references to Entrez GeneIDs. In total, scRNA-seq data was obtained on 541,863,094 transcripts in 200,626 T and non-T cells.

Computational separation of T and non-T cells

Counts of transcripts were extracted from the filtered barcodes.tsv, genes.tsv, and matrix.mtx files using R statistical software. Count matrices for each sample were then processed using Seurat version 3 using the SCTransform procedure to perform a regularized negative binomial regression based on the 3000 most variable genes. Normalized datasets for the tumor, NAT, and blood (if available) samples for each patient were combined using the FindIntegrationAnchors and IntegrateData functions in Seurat with the default value of 30 dimensions. Datasets for all 14 patients were then combined using the FindIntegrationAnchors and IntegrateData functions in Seurat using the log-normalized counts method implemented in version 3.0.2 and the default value of 30 dimensions. Cluster analysis was performed using the FindClusters procedure at a resolution of 1.6, larger than the default value of 0.8, in order to obtain a finer resolution of cell subtypes, and yielded 33 clusters.

Desired T cell clusters were considered those to have a mixed representation of CD3- and CD45-selected samples, whereas samples containing contributions exclusively from CD45-selected samples were considered to contain primarily non-T cells. Based on this criterion, cells were then divided into T and non-T cell categories and the entire process of data transformation, integration, and cluster analysis was repeated on the two divisions. Because cluster analysis procedures can identify cells with outlier gene expression from the others, subsequent iterations were able to identify smaller sets of contaminating T cells in the non-T set, and conversely, contaminating non-T cells in the T set. Therefore, each iteration allowed for a refinement of the separation. One advantage of having CD45-selected samples is that their non-T cell population provided a way of removing non-T cell contaminants from the CD3-selected samples, and thereby help achieve a purer collection of T cells for analysis. The separation process was iterated for 5 cycles until all T cell clusters had a mixed representation of CD3- and CD45-selected samples, and no outlier clusters were identified.

Naming of T cell clusters

Unsupervised cluster analysis was performed on the T cell population using the FindClusters procedure in Seurat using default values. Dimensionality reduction was performed using the RunPCA and RunUMAP procedures. Two-dimensional gene expression maps were generated using coordinates from the Uniform Manifold Approximation and Projection (UMAP) algorithm (Becht, E. et al. Nature Biotechnology 37, 38-44, 2019). To characterize each cluster two procedures were applied: both the FindMarkers procedure in Seurat, which identified biomarkers using log fold changes of mean expression, as well as a logistic regression procedure, which identified biomarkers that separated cells of a given cluster from other cells. The logistic regression procedure was performed on each gene within each patient for each cluster using the SCTransform assay data, and yielded a signed deviance value, where the sign depended on the coefficient for the gene expression term. For each cluster, signed deviance values were combined across patients using the Rank Product procedure in the RankProd package in R. The results of the FindMarkers and logistic regression procedure were used with inspection of barplots, such as those in FIG. 13C, to identify a gene label for each cluster. However, these labels were used only for naming purposes and to select genes to display in FIG. 13C and FIG. 18B, and did not affect any subsequent computations. Barplots in FIG. 13C and FIG. 18B are computed using the scaled data from the integrated assay using Seurat. Plots of individual gene expression onto the two-dimensional map were computed using the quantile of counts from SCTransform array because many genes had low levels of expression in the scRNA-seq assay and were not scaled by the integration algorithm.

Analysis of data from Guo et al., 2018

Single-cell RNA-seq counts were obtained using the GEOquery library in R and running the command getGEOSuppFiles for GSE99254. Expression data was obtained over 23,459 genes in 12,348 cells. Metadata consisting of the patient, cluster assignment, and tissue source for each cell was downloaded from the data table at the Web site for Gene Expression Omnibus project GSE99254. TCR sequences and clonotype groupings were obtained from Supplementary Table 2 of the published paper by Guo et al., 2018. TCR sequences were obtained for 10,202 cells in 7398 distinct clonotypes.

Analysis of data from Zhang et al., 2018

Single-cell RNA-seq counts were obtained using the GEOquery library in R and running the command getGEOSuppFiles for GSE108989. Expression data was obtained over 23,459 genes in 11,140 cells. Metadata consisting of the patient, cluster assignment, and tissue source for each cell was downloaded from the data table at the Web site for Gene Expression Omnibus project GSE108989. TCR sequences and clonotype groupings were obtained from Supplementary Table 4 of the published paper by Zhang et al., 2018. TCR sequences were obtained for 9878 cells in 7274 distinct clonotypes.

Stem-like exhausted versus terminally exhausted signature

Microarray data, in the form of log2 RMA normalized intensities, was obtained from samples GSM2227309 through GSM2227314 from Gene Expression Omnibus project GSE84105 (Im 2015) using the getGEO procedure of the GEOquery package in R. Differentially expressed probes between the three Cxcr5+ samples and the three Tim-3+ samples were found using the lmFit, contrasts.fit, eBayes, and topTable procedures in the limma package in R, at a threshold log fold change of 3 or more. These probes were assigned to genes using the annotate and mouse4302.db packages, and then translated into human equivalents using the homologene package in R. The resulting 58 up-regulated genes were compared against the scRNA-seq dataset using the scaled data array from the integrated assay computed by Seurat, yielding 12 genes in common. An overall signature score for each T cell was calculated by taking the mean scaled data score for the genes in the signature.

Analysis of data from Yost et al., 2019

Single-cell RNA-seq counts and scTCR sequences were obtained using the GEOquery library in R and running the command getGEOSuppFiles for GSE123813. Metadata consisting of the patient, treatment status, and cluster assignment for each cell was obtained from the corresponding authors for Yost et al., 2019. Expression data that had matching metadata were analyzed, resulting in 33,106 cells measured on 23,309 genes. Single-cell TCR sequences were available on 28,371 cells with matching metadata. Bulk TCR-seq sequences and template counts were obtained from the ImmuneACCESS Web portal by Adaptive Biotechnologies.

The scTCR-seq data was provided as TCR-alpha and TCR-beta nucleotide and amino acid sequences without assigned clonotypes. Clonotype groupings were therefore created from pre- and post-treatment tumor samples by matching all available TCR chains between cells of a given patient, using a procedure analogous to the re-grouping of tumor, NAT, and blood clonotypes for the present dataset. Among the 11 patients in the dataset, this procedure identified 17,369 distinct clonotypes. Patient bcc.su003 had no clonotype matches between pre-treatment and post-treatment tumor samples, and could not be analyzed further. The remaining patients had pre-treatment and post-treatment bulk TCR-seq data from blood for 5 and 6 patients, respectively, with patient bcc.su002 having no pre-treatment blood sample. Bulk TCR-seq clonotypes were linked to tumor clonotypes by matching the single TCR beta chain to any of the TCR beta chains from a clonotype in the scTCR-seq data. Because the bulk TCR-seq data reports an 87-bp rearrangement sequence from each TCR beta chain that includes the CDR3 sequence, for the purpose of matching to scTCR-seq data, the CDR3 nucleotide sequence was extracted by finding a translated match to the given productive CDR3 amino acid sequence. Because scTCR-seq clonotypes can contain TCR-alpha chains as well as multiple TCR-beta chains, the potential exists for a many-to-one correspondence between a scTCR-seq clonotype from tumor and a bulk TCR-seq clonotype from blood. For analyses involving the presence or absence of a bulk TCR-seq match, in FIG. 14C, FIG. 14D and FIG. 20B, FIG. 20C, all matching clonotypes were considered to have an association with blood. For analyses requiring clone sizes, in FIG. 14A and FIG. 20A, multiple tumor clonotype matches for a given blood clonotype were combined by summing the scTCR-seq clone sizes and resolving multiple primary cell clusters by a majority vote or leaving the primary cell cluster unassigned in case of a tie. For the analysis comparing pre- and post-treatment bulk TCR sequences, in FIG. 14B, clonotypes were matched when the entire 87-bp rearrangement sequences were identical.

Comparison of cluster assignments against external clusters

Reference gene signatures were computed from the scRNA-seq data of the datasets for Guo et al., 2018, Zhang et al., 2018, and Yost et al., 2019. The first two datasets had count matrices that were already normalized by library sizes in the form of TPM (transcripts per million) matrices, and this same normalization was computed for the third dataset. For each cluster, based on the metadata from the original analyses, robust centroids were obtained by performing a 10% trimmed mean of each gene across cells in the cluster. These centroids were used as reference gene signatures for the R package SingleR, which assigned single-cell assignments to each cell in the present dataset. Gene expression measurements from the dataset were the SCTransform cell counts computed for each sample before integration. Counts of cells were cross-tabulated based on both the internal cluster assignments and the external assignments computed by SingleR, and normalized the resulting matrix first by the external assignments and then by the internal assignments to generate heatmaps for FIG. 13B and FIG. 18B.

Comparison of TCR sequences with VDJdb

The 2018-06-04 release of VDJdb (Shugay 2018) from its Web site was obtained. The file vdjdb.slim.txt was analyzed to extract entries where the TCR species was "HomoSapiens" and the epitope species was either "CMV", "EBV", or "InfluenzaA". Each clonotype from the dataset generated in this example was compared against the cdr3 field in the VDJdb database, requiring an exact match from any of the CDR3 amino acid sequences for the clonotype.

Comparison of TCR sequences with the Cancer Genome Atlas (TCGA)

A set of 716,720 TCR sequences was obtained, assembled from 9142 bulk RNA-Seq samples in the Cancer Genome Atlas (TCGA) using the TRUST algorithm (Li 2016). Those sequences were obtained from the authors after gaining authorized access to sequence-level data in TCGA. Each sequence was labeled with a putative CDR3 amino acid sequence in the header of the FASTA file. Each clonotype from the dataset generated in this Example was compared, requiring an exact match from any of the CDR3 amino acid sequences for the clonotype against any substring of the TCGA-derived CDR3 sequence. Each FASTA file was annotated with an aliquot ID, which was linked to a cancer type using the R library GenomicDataCommons. A TCGA-derived CDR3 sequence was considered to be likely virally-reactive when it was observed in three or more individuals, all with different cancer types, and no two individuals had the same cancer type.

Gene signatures for CD8+ T cell clusters

A logistic regression procedure similar to that used for finding characteristic biomarkers to name T cell clusters was applied in this Example, except that the data were restricted to the cells assigned to the 8.1-Eff, 8.2-Tem, and 8.3-Trm clusters in order to focus on differences between CD8+ clusters rather than biomarkers that distinguish CD8+ from CD4+ T cells. Data was also restricted to the tumor and NAT cells since only four samples had blood cells. The logistic regression procedure was performed on each gene within each patient for each cluster using the SCTransform assay data. The tumor or NAT source was included as a covariate to help remove batch effects. Each logistic regression fit yielded a signed deviance value for a given gene in a given cluster, where the sign depended on the coefficient for the gene expression term. For each cluster, signed deviance values were combined across patients using the Rank Product procedure in the RankProd package in R. A total of 20 genes was selected for each gene expression signature.

Analysis of clinical trial data

Bulk tumor RNA-seq data was obtained from three clinical trials: (1) IMvigor210 (Mariathasan 2018) (top row), a single-arm trial in 354 patients with locally advanced or metastatic urothelial carcinoma, (2) POPLAR (Fehrenbacher 2016) (middle row), a dual-arm trial comparing 93 non small-cell lung carcinoma patients treated with atezolizumab and 100 patients treated with the chemotherapeutic agent docetaxel, and (3) IMmotion150 (McDermott 2018) (bottom row), a triple-arm trial of patients with renal cell carcinoma, treated with atezolizumab (86 patients), atezolizumab plus the VEGF inhibitor bevacizumab (88 patients), or the tyrosine kinase inhibitor sunitinib (89 patients). RNA-seq counts were normalized by a trimmed mean of M-values, log2-transformed, and converted to Z-scores for each gene by subtracting the means and dividing by the standard deviation across all patients in the trial. Subsequent clinical analysis was performed using the survival package in R.

Progression-free survival (PFS) hazard ratios were computed for each gene by dichotomizing the expression of that gene based on the value being higher or lower than the median, and then fitting a Cox proportional hazards model to the censored PFS survival times using the dichotomized variable. To evaluate the overall set of hazard ratios for each gene signature, the gene set test proposed by Irizarry et al., 2009, was applied. Specifically, it was first confirmed from quantile plots that log-transformed hazard ratios were normally distributed. Then, an intersection of genes was found from each signature in a given clinical trial by matching the ENTREZ gene IDs. The overall hazard ratio was computed as their geometric mean, equivalent to an arithmetic mean of the log-transformed hazard ratios. A z-statistic was computed as the mean of the log-transformed hazard ratios multiplied by the square root of the number of intersecting genes, and the p-value was obtained by referring this value to the normal distribution.

To evaluate combined signature scores, an intersection of genes was found from each signature in a given clinical trial as before. The resulting n common genes in each signature were used to calculate an expression score for each patient in each trial as a weighted mean z-score, using a linear series of weights from the highest ranked gene in the common signature genes (based on signed deviance values) receiving a weight of n to the lowest ranked gene receiving a weight of 1. The signature score was dichotomized in each clinical trial based on its value being higher or lower than the median. A Cox proportional hazards model of this dichotomized variable was fitted to the censored PFS survival data in each arm of each clinical trial, and reported as the hazard ratio and p-value of the Wald statistic for each Kaplan-Meier survival plot.

Data and code availability

A complete data and software package capable of reproducing the analysis and figures in this paper is available publicly at http://research-pub.gene.com/tumor-immune-cells. In addition, the data and metadata for the scRNA-seq and scTCR-seq assays will be made available at the NCBI Gene Expression Omnibus (accession number pending acceptance of this manuscript for publication).

## VII. OTHER EMBODIMENTS

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

The disclosure is also described by the following numbered embodiments:

Embodiment 1. A method of producing a chimeric antigen receptor (CAR) T cell, the method comprising:

(a) obtaining a sample of peripheral blood from an individual having cancer;
(b) determining (i) an amino acid sequence of one or more complementarity-determining regions (CDRs) of a T cell receptor (TCR) expressed by one or more T cells in the sample and/or (ii) a nucleic acid sequence encoding the amino acid sequence; and
(c) modifying a precursor cell to express a CAR comprising the one or more CDRs, thereby producing a CAR T cell.

Embodiment 2. A method of producing a CAR T cell, the method comprising modifying a precursor cell to express a CAR comprising one or more CDRs of a TCR expressed by one or more T cells in a sample of peripheral blood obtained from an individual having cancer.

Embodiment 3. The method of embodiment 1 or 2, wherein the T cells in the sample are clonally expanded T cells.

Embodiment 4. The method of embodiment 3, wherein the clonally expanded T cells are CD8+ T cells.

Embodiment 5. The method of embodiment 4, wherein the CD8+ T cells are CTL.

Embodiment 6. The method of any one of embodiments 1-5, wherein the TCR expressed by the one or more T cells specifically binds an antigen expressed by a cancer cell in the individual.

Embodiment 7. The method of any one of embodiments 3-6, wherein the clonally expanded T cells comprise at least 0.01% of all T cells in the sample.

Embodiment 8. The method of embodiment 7, wherein the clonally expanded T cells comprise at least 0.1% of all T cells in the sample.

Embodiment 9. The method of embodiment 8, wherein the clonally expanded T cells comprise at least 1% of all T cells in the sample.

Embodiment 10. The method of embodiment 9, wherein the clonally expanded T cells comprise at least 2% of all T cells in the sample.

Embodiment 11. The method of embodiment 10, wherein the clonally expanded T cells comprise at least 3% of all T cells in the sample.

Embodiment 12. The method of embodiment 11, wherein the clonally expanded T cells comprise at least 4% of all T cells in the sample.

Embodiment 13. The method of embodiment 12, wherein the clonally expanded T cells comprise at least 5% of all T cells in the sample.

Embodiment 14. The method of embodiment 13, wherein the clonally expanded T cells comprise at least 10% of all T cells in the sample.

Embodiment 15. The method of embodiment 14, wherein the clonally expanded T cells comprise at least 15% of all T cells in the sample.

Embodiment 16. The method of embodiment 15, wherein the clonally expanded T cells comprise at least 20% of all T cells in the sample.

Embodiment 17. The method of embodiment 16, wherein the clonally expanded T cells comprise at least 25% of all T cells in the sample.

Embodiment 18. The method of any one of embodiments 1-17, wherein the precursor cell is a T cell.

Embodiment 19. The method of any one of embodiments 1-18, wherein the method produces at least about $1 \times 10^3$ CAR T cells.

Embodiment 20. The method of embodiment 19, wherein the method produces from about $1 \times 10^3$ CAR T cells to about $1 \times 10^{12}$ CAR T cells.

Embodiment 21. The method of embodiment 20, wherein the method produces from about $1 \times 10^4$ CAR T cells to about $1 \times 10^{11}$ CAR T cells.

Embodiment 22. The method of embodiment 21, wherein the method produces from about $1 \times 10^5$ CAR T cells to about $1 \times 10^{10}$ CAR T cells.

Embodiment 23. The method of embodiment 22, wherein the method produces from about $1 \times 10^6$ CAR T cells to about $1 \times 10^9$ CAR T cells.

Embodiment 24. A CAR T cell produced by the method of any one of embodiments 1-23.

Embodiment 25. A pharmaceutical composition comprising (i) one or more CAR T cells produced by the method of any one of embodiments 1-23, or progeny thereof, and (ii) one or more carriers, diluents, and/or excipients.

Embodiment 26. A method of treating cancer in an individual in need thereof, the method comprising administering to the individual the CAR T cell of embodiment 24, or progeny thereof, or the pharmaceutical composition of embodiment 25.

Embodiment 27. The method of embodiment 26, wherein the cancer is a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy.

Embodiment 28. The method of embodiment 27, wherein the cancer is a lung cancer, a kidney cancer, a bladder cancer, or a breast cancer.

Embodiment 29. The method of embodiment 28, wherein the lung cancer is a non-small cell lung cancer (NSCLC).

Embodiment 30. The method of embodiment 28, wherein the kidney cancer is a renal cell carcinoma (RCC).

Embodiment 31. The method of embodiment 28, wherein the bladder cancer is an urothelial carcinoma (UC).

Embodiment 32. The method of embodiment 28, wherein the breast cancer is a triple negative breast cancer (TNBC).

Embodiment 33. The method of any one of embodiments 26-32, wherein the patient is further administered a PD-L1 axis binding antagonist.

Embodiment 34. The method of embodiment 33, wherein the PD-L1 axis binding antagonist is selected from the group consisting of a PD-L1 binding antagonist, a PD-1 binding antagonist, and a PD-L2 binding antagonist.

Embodiment 35. The method of embodiment 34, wherein the PD-L1 axis binding antagonist is a PD-L1 binding antagonist.

Embodiment 36. The method of embodiment 35, wherein the PD-L1 binding antagonist inhibits the binding of PD-L1 to one or more of its ligand binding partners.

Embodiment 37. The method of embodiment 36, wherein the PD-L1 binding antagonist inhibits the binding of PD-L1 to PD-1.

Embodiment 38. The method of embodiment 36, wherein the PD-L1 binding antagonist inhibits the binding of PD-L1 to B7-1.

Embodiment 39. The method of any one of embodiments 36-38, wherein the PD-L1 binding antagonist inhibits the binding of PD-L1 to both PD-1 and B7-1.

Embodiment 40. The method of any one of embodiments 35-39, wherein the PD-L1 binding antagonist is an antibody or antigen-binding fragment thereof.

Embodiment 41. The method of embodiment 40, wherein the antibody is selected from the group consisting of atezolizumab (MPDL3280A), YW243.55.S70, MDX-1105, MEDI4736 (durvalumab), and MSB0010718C (avelumab).

Embodiment 42. The method of embodiment 41, wherein the antibody comprises a heavy chain comprising HVR-H1 sequence of SEQ ID NO: 41, HVR-H2 sequence of SEQ ID NO: 42, and HVR-H3 sequence of SEQ ID NO: 43; and a light chain comprising HVR-L1 sequence of SEQ ID NO: 44, HVR-L2 sequence of SEQ ID NO: 45, and HVR-L3 sequence of SEQ ID NO: 46.

Embodiment 43. The method of embodiment 42, wherein the antibody comprises a heavy chain variable region comprising the amino acid sequence of SEQ ID NO: 47 and a light chain variable region comprising the amino acid sequence of SEQ ID NO: 48.

Embodiment 44. The method of embodiment 34, wherein the PD-L1 axis binding antagonist is a PD-1 binding antagonist.

Embodiment 45. The method of embodiment 44, wherein the PD-1 binding antagonist inhibits the binding of PD-1 to one or more of its ligand binding partners.

Embodiment 46. The method of embodiment 45, wherein the PD-1 binding antagonist inhibits the binding of PD-1 to PD-L1.

Embodiment 47. The method of embodiment 46, wherein the PD-1 binding antagonist inhibits the binding of PD-1 to PD-L2.

Embodiment 48. The method of any one of embodiments 45-47, wherein the PD-1 binding antagonist inhibits the binding of PD-1 to both PD-L1 and PD-L2.

Embodiment 49. The method of any one of embodiments 44-48, wherein the PD-1 binding antagonist is an antibody or antigen-binding fragment thereof.

Embodiment 50. The method of embodiment 49, wherein the antibody is selected from the group consisting of: MDX-1106 (nivolumab), MK-3475 (pembrolizumab), MEDI-0680 (AMP-514), PDR001, REGN2810, and BGB-108.

Embodiment 51. The method of any one of embodiments 44-48, wherein the PD-1 binding antagonist is an Fc-fusion protein.

Embodiment 52. The method of embodiment 51, wherein the Fc-fusion protein is AMP-224.

Embodiment 53. A method of identifying an individual having a cancer who may benefit from a treatment comprising a PD-L1 axis binding antagonist, the method comprising determining whether the individual has clonally expanded T cells in a sample of peripheral blood from the individual, wherein clonally expanded T cells in the sample identify the individual as one who may benefit from a treatment comprising a PD-L1 axis binding antagonist.

Embodiment 54. A method of selecting a therapy for an individual having a cancer, the method comprising determining whether the individual has clonally expanded T cells in a sample of peripheral blood from the individual, wherein clonally expanded T cells in the sample identify the individual as one who may benefit from a treatment comprising a PD-L1 axis binding antagonist.

Embodiment 55. The method of embodiment 53 or 54, wherein the sample comprises clonally expanded T cells and the method further comprises administering to the individual an effective amount of a PD-L1 axis binding antagonist.

Embodiment 56. A method of treating an individual having a cancer, the method comprising:

(a) determining that the individual has clonally expanded T cells in a sample of peripheral blood from the individual; and
(b) administering an effective amount of a PD-L1 axis binding antagonist to the individual.

Embodiment 57. A method of treating cancer in an individual that has been determined to have clonally expanded T cells in a sample of peripheral blood from the individual, the method comprising administering to the individual an effective amount of a PD-L1 axis binding antagonist.

Embodiment 58. A method of inducing expansion of a population of T cells in an individual having a cancer, the method comprising:

(a) determining that the individual has clonally expanded T cells in a sample of peripheral blood from the individual; and
(b) administering an effective amount of a PD-L1 axis binding antagonist to the individual.

Embodiment 59. A method of inducing expansion of a population of T cells in an individual having a cancer and that has been determined to have clonally expanded T cells in a sample of peripheral blood from the individual, the method comprising administering to the individual an effective amount of a PD-L1 axis binding antagonist.

Embodiment 60. The method of any one of embodiments 53-59, wherein the clonally expanded T cells are CD8+ T cells.

Embodiment 61. The method of embodiment 60, wherein the CD8+ T cells are CTL.

Embodiment 62. The method of any one of embodiments 53-61, wherein the clonally expanded T cells express TCRs comprising alpha and beta chains each comprising CDRs CDR-1, CDR-2, and CDR-3, and wherein the nucleic acid sequences encoding corresponding alpha chain CDR-1, CDR-2, or CDR-3, and/or beta chain CDR-1, CDR-2, or CDR-3 are at least 85% identical to one another.

Embodiment 63. The method of embodiment 62, wherein the nucleic acid sequences encoding corresponding alpha chain CDR1 are at least 85% identical to one another.

Embodiment 64. The method of embodiment 63, wherein the nucleic acid sequences encoding corresponding alpha chain CDR1 are at least 90% identical to one another.

Embodiment 65. The method of embodiment 64, wherein the nucleic acid sequences encoding corresponding alpha chain CDR1 are at least 95% identical to one another.

Embodiment 66. The method of embodiment 65, wherein the nucleic acid sequences encoding corresponding alpha chain CDR1 are 100% identical to one another.

Embodiment 67. The method of any one of embodiments 62-66, wherein the nucleic acid sequences encoding corresponding alpha chain CDR2 are at least 85% identical to one another.

Embodiment 68. The method of embodiment 67, wherein the nucleic acid sequences encoding corresponding alpha chain CDR2 are at least 90% identical to one another.

Embodiment 69. The method of embodiment 68, wherein the nucleic acid sequences encoding corresponding alpha chain CDR2 are at least 95% identical to one another.

Embodiment 70. The method of embodiment 69, wherein the nucleic acid sequences encoding corresponding alpha chain CDR2 are 100% identical to one another.

Embodiment 71. The method of any one of embodiments 62-70, wherein the nucleic acid sequences encoding corresponding alpha chain CDR3 are at least 85% identical to one another.

Embodiment 72. The method of embodiment 71, wherein the nucleic acid sequences encoding corresponding alpha chain CDR3 are at least 90% identical to one another.

Embodiment 73. The method of embodiment 72, wherein the nucleic acid sequences encoding corresponding alpha chain CDR3 are at least 95% identical to one another.

Embodiment 74. The method of embodiment 73, wherein the nucleic acid sequences encoding corresponding alpha chain CDR3 are 100% identical to one another.

Embodiment 75. The method of any one of embodiments 62-74, wherein the nucleic acid sequences encoding corresponding beta chain CDR1 are at least 85% identical to one another.

Embodiment 76. The method of embodiment 75, wherein the nucleic acid sequences encoding corresponding beta chain CDR1 are at least 90% identical to one another.

Embodiment 77. The method of embodiment 76, wherein the nucleic acid sequences encoding corresponding beta chain CDR1 are at least 95% identical to one another.

Embodiment 78. The method of embodiment 77, wherein the nucleic acid sequences encoding corresponding beta chain CDR1 are 100% identical to one another.

Embodiment 79. The method of any one of embodiments 62-78, wherein the nucleic acid sequences encoding corresponding beta chain CDR2 are at least 85% identical to one another.

Embodiment 80. The method of embodiment 79, wherein the nucleic acid sequences encoding corresponding beta chain CDR2 are at least 90% identical to one another.

Embodiment 81. The method of embodiment 80, wherein the nucleic acid sequences encoding corresponding beta chain CDR2 are at least 95% identical to one another.

Embodiment 82. The method of embodiment 81, wherein the nucleic acid sequences encoding corresponding beta chain CDR2 are 100% identical to one another.

Embodiment 83. The method of any one of embodiments 62-82, wherein the nucleic acid sequences encoding corresponding beta chain CDR3 are at least 85% identical to one another.

Embodiment 84. The method of embodiment 83, wherein the nucleic acid sequences encoding corresponding beta chain CDR3 are at least 90% identical to one another.

Embodiment 85. The method of embodiment 84, wherein the nucleic acid sequences encoding corresponding beta chain CDR3 are at least 95% identical to one another.

Embodiment 86. The method of embodiment 85, wherein the nucleic acid sequences encoding corresponding beta chain CDR3 are 100% identical to one another.

Embodiment 87. The method of any one of embodiments 53-86, wherein the clonally expanded T cells express TCRs comprising alpha and/or beta chains having nucleic acid sequences that are at least 85% identical to one another.

Embodiment 88. The method of embodiment 87, wherein the clonally expanded T cells express TCRs comprising alpha chains having nucleic acid sequences that are at least 85% identical to one another.

Embodiment 89. The method of embodiment 88, wherein the clonally expanded T cells express TCRs comprising alpha chains having nucleic acid sequences that are at least 90% identical to one another.

Embodiment 90. The method of embodiment 89, wherein the clonally expanded T cells express TCRs comprising alpha chains having nucleic acid sequences that are at least 95% identical to one another.

Embodiment 91. The method of embodiment 90, wherein the clonally expanded T cells express TCRs comprising alpha chains having nucleic acid sequences that are 100% identical to one another.

Embodiment 92. The method of any one of embodiments 87-91, wherein the clonally expanded T cells express TCRs comprising beta chains having nucleic acid sequences that are at least 85% identical to one another.

Embodiment 93. The method of embodiment 92, wherein the clonally expanded T cells express TCRs comprising beta chains having nucleic acid sequences that are at least 90% identical to one another.

Embodiment 94. The method of embodiment 93, wherein the clonally expanded T cells express TCRs comprising beta chains having nucleic acid sequences that are at least 95% identical to one another.

Embodiment 95. The method of embodiment 94, wherein the clonally expanded T cells express TCRs comprising beta chains having nucleic acid sequences that are 100% identical to one another.

Embodiment 96. The method of any one of embodiments 53-95, wherein the clonally expanded T cells comprise at least 0.01% of all T cells in the sample.

Embodiment 97. The method of embodiment 96, wherein the clonally expanded T cells comprise at least 0.1% of all T cells in the sample.

Embodiment 98. The method of embodiment 97, wherein the clonally expanded T cells comprise at least 1% of all T cells in the sample.

Embodiment 99. The method of embodiment 98, wherein the clonally expanded T cells comprise at least 2% of all T cells in the sample.

Embodiment 100. The method of embodiment 99, wherein the clonally expanded T cells comprise at least 3% of all T cells in the sample.

Embodiment 101. The method of embodiment 100, wherein the clonally expanded T cells comprise at least 4% of all T cells in the sample.

Embodiment 102. The method of embodiment 101, wherein the clonally expanded T cells comprise at least 5% of all T cells in the sample.

Embodiment 103. The method of embodiment 102, wherein the clonally expanded T cells comprise at least 10% of all T cells in the sample.

Embodiment 104. The method of embodiment 103, wherein the clonally expanded T cells comprise at least 15% of all T cells in the sample.

Embodiment 105. The method of embodiment 104, wherein the clonally expanded T cells comprise at least 20% of all T cells in the sample.

Embodiment 106. The method of embodiment 105, wherein the clonally expanded T cells comprise at least 25% of all T cells in the sample.

Embodiment 107. A method of identifying an individual having a cancer who may benefit from a treatment comprising a PD-L1 axis binding antagonist, the method comprising determining the expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual, wherein an immune-score expression level of the one or more genes that is above a reference immune-score expression level of the one or more genes identifies the individual as one who may benefit from a treatment comprising a PD-L1 axis binding antagonist.

Embodiment 108. A method of selecting a therapy for an individual having a cancer, the method comprising determining the expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual, wherein an immune-score expression level of the one or more genes in the sample that is above a reference immune-score expression level of the one or more genes identifies the individual as one who may benefit from a treatment comprising a PD-L1 axis binding antagonist.

Embodiment 109. The method of embodiment 107 or 108, wherein the immune-score expression level of the one or more genes in the sample is above the reference immune-score expression level and the method further comprises administering to the individual an effective amount of a PD-L1 axis binding antagonist.

Embodiment 110. The method of any one of embodiments 107-109, wherein the reference immune-score expression level is an immune-score expression level of the one or more genes in a reference population.

Embodiment 111. A method of treating an individual having a cancer, the method comprising:

(a) determining the expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual, wherein an immune-score expression level of the one or more genes in the sample is determined to be above a reference immune-score expression level of the one or more genes; and
(b) administering an effective amount of a PD-L1 axis binding antagonist to the individual.

Embodiment 112. A method of treating cancer in an individual that has been determined to have an immune-score expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual that is above a reference immune-score expression level of the one or more genes, the method comprising administering to the individual an effective amount of a PD-L1 axis binding antagonist.

Embodiment 113. A method of inducing expansion of a population of T cells in an individual having a cancer, the method comprising:

(a) determining the expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual, wherein an immune-score expression level of the one or more genes in the sample is determined to be above a reference immune-score expression level of the one or more genes; and
(b) administering an effective amount of a PD-L1 axis binding antagonist to the individual.

Embodiment 114. A method of inducing expansion of a population of T cells in an individual having a cancer and that has been determined to have an immune-score expression level of one or more of genes NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1 in a sample of peripheral blood from the individual that is above a reference immune-score expression level of the one or more genes, the method comprising administering to the individual an effective amount of a PD-L1 axis binding antagonist.

Embodiment 115. The method of embodiment 113 or 114, wherein the T cells are CD8+ T cells.

Embodiment 116. The method of embodiment 115, wherein the CD8+ cells are cytotoxic T cells (CTL) or effector memory T cells (Tern).

Embodiment 117. The method of any one of embodiments 111-116, wherein the reference immune-score expression level is an immune-score expression level of the one or more genes in a reference population.

Embodiment 118. The method of embodiment 110 or 117, wherein the reference population is a population of individuals having the cancer.

Embodiment 119. The method of embodiment 118, wherein the population of individuals comprises a first subset of individuals who have been treated with a PD-L1 axis binding antagonist and a second subset of individuals who have been treated with therapy that does not comprise a PD-L1 axis binding antagonist.

Embodiment 120. The method of embodiment 119, wherein the reference immune-score expression level significantly separates each of the first and second subsets of individuals based on a significant difference between an individual's responsiveness to treatment with the PD-L1 axis binding antagonist and an individual's responsiveness to treatment with the therapy that does not comprise a PD-L1 axis binding antagonist above the reference immune-score expression level, wherein the individual's responsiveness to treatment with the PD-L1 axis binding antagonist is significantly improved relative to the individual's responsiveness to treatment with the therapy that does not comprise a PD-L1 axis binding antagonist.

Embodiment 121. The method of embodiment 119 or 120, wherein the reference immune-score expression level significantly separates each of the first and second subsets of individuals based on a significant difference between an individual's responsiveness to treatment with the PD-L1 axis binding antagonist and an individual's responsiveness to treatment with the therapy that does not comprise a PD-L1 axis binding antagonist below the reference immune-score expression level, wherein the individual's responsiveness to treatment with the therapy that does not comprise a PD-L1 axis binding antagonist is significantly improved relative to the individual's responsiveness to treatment with the PD-L1 axis binding antagonist.

Embodiment 122. The method of any one of embodiments 119-121, wherein the therapy that does not comprise a PD-L1 axis binding antagonist comprises an anti-neoplastic agent, a chemotherapeutic agent, a growth inhibitory agent, an anti-angiogenic agent, a radiation therapy, a cytotoxic agent, or a combination thereof.

Embodiment 123. The method of embodiment 122, wherein the therapy that does not comprise a PD-L1 axis binding antagonist comprises a chemotherapeutic agent.

Embodiment 124. The method of embodiment 123, wherein the chemotherapeutic agent is docetaxel.

Embodiment 125. ! he method of any one of embodiments 120-124, wherein responsiveness to treatment comprises an increase in progression-free survival (PFS).

Embodiment 126. The method of any one of embodiments 120-125, wherein responsiveness to treatment comprises an increase in overall survival (OS).

Embodiment 127. The method of any one of embodiments 110 and 117-126, wherein the reference immune-score expression level is a median of the expression level of each of the one or more genes in the reference population.

Embodiment 128. The method of embodiment 127, wherein the immune-score expression level of the one or more genes in the reference population is a median of a normalized expression level of each of the one or more genes in the reference population.

Embodiment 129. The method of any one of embodiments 110 and 117-126, wherein the reference immune-score expression level is an average of the expression level of each of the one or more genes in the reference population.

Embodiment 130. The method of embodiment 129, wherein the average of the expression level of each of the one or more genes in the reference population is an average of a normalized expression level of each of the one or more genes in the reference population.

Embodiment 131. The method of embodiment 128 or 130, wherein the normalized expression level of each of the one or more genes is the expression level of each of the one or more genes normalized to a reference gene.

Embodiment 132. The method of embodiment 131, wherein the reference gene is CD8A.

Embodiment 133. The method of any one of embodiments 107-132, wherein the reference immune-score expression level is a pre-assigned expression level of the one or more genes.

Embodiment 134. The method of any one of embodiments 53-133, wherein benefit from the treatment comprising a PD-L1 axis binding antagonist comprises an increase in OS.

Embodiment 135. The method of any one of embodiments 53-134, wherein benefit from the treatment comprising a PD-L1 axis binding antagonist comprises an increase in PFS.

Embodiment 136. The method of embodiment 134 or 135, wherein benefit from the treatment comprising a PD-L1 axis binding antagonist comprises an increase in OS and PFS.

Embodiment 137. The method of any one of embodiments 107-136, wherein the genes comprise two or more of NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1.

Embodiment 138. The method of embodiment 137, wherein the genes comprise three or more of NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1.

Embodiment 139. The method of embodiment 138, wherein the genes comprise four or more of NKG7, GNLY, PRF1, GZMH, GPR56, IFITM2, IFITM1, GZMB, TMSB10, PLAC8, LITAF, CD247, CST7, COX2, KLRD1, CLIC3, S100A4, CYBA, and COX1.

Embodiment 140. The method of any one of embodiments 107-139, wherein the expression level is a nucleic acid expression level.

Embodiment 141. The method of embodiment 140, wherein the nucleic acid expression level is an mRNA expression level.

Embodiment 142. The method of embodiment 141, wherein the mRNA expression level is determined by RNA-seq, RT-qPCR, qPCR, multiplex qPCR or RT-qPCR, microarray analysis, SAGE, MassARRAY technique, FISH, or a combination thereof.

Embodiment 143. The method of embodiment 142, wherein the mRNA expression level is detected using RNA-seq.

Embodiment 144. The method of embodiment 142, wherein the mRNA expression level is detected using RT-qPCR.

Embodiment 145. The method of any one of embodiments 53-144, wherein the cancer is a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy.

Embodiment 146. The method of embodiment 145, wherein the cancer is a lung cancer, a kidney cancer, a bladder cancer, or a breast cancer.

Embodiment 147. The method of embodiment 146, wherein the lung cancer is a non-small cell lung cancer (NSCLC).

Embodiment 148. The method of embodiment 146, wherein the kidney cancer is a renal cell carcinoma (RCC).

Embodiment 149. The method of embodiment 146, wherein the bladder cancer is an urothelial carcinoma (UC).

Embodiment 150. The method of embodiment 146, wherein the breast cancer is a triple negative breast cancer (TNBC).

Embodiment 151. The method of any one of embodiments 53-150, wherein the PD-L1 axis binding antagonist is selected from the group consisting of a PD-L1 binding antagonist, a PD-1 binding antagonist, and a PD-L2 binding antagonist.

Embodiment 152. The method of embodiment 151, wherein the PD-L1 axis binding antagonist is a PD-L1 binding antagonist.

Embodiment 153. The method of embodiment 152, wherein the PD-L1 binding antagonist inhibits the binding of PD-L1 to one or more of its ligand binding partners.

Embodiment 154. The method of embodiment 153, wherein the PD-L1 binding antagonist inhibits the binding of PD-L1 to PD-1.

Embodiment 155. The method of embodiment 153, wherein the PD-L1 binding antagonist inhibits the binding of PD-L1 to B7-1.

Embodiment 156. The method of any one of embodiments 153-155, wherein the PD-L1 binding antagonist inhibits the binding of PD-L1 to both PD-1 and B7-1.

Embodiment 157. The method of any one of embodiments 152-156, wherein the PD-L1 binding antagonist is an antibody or antigen-binding fragment thereof.

Embodiment 158. The method of embodiment 157, wherein the antibody is selected from the group consisting of atezolizumab (MPDL3280A), YW243.55.S70, MDX-1105, MEDI4736 (durvalumab), and MSB0010718C (avelumab).

Embodiment 159. The method of embodiment 157, wherein the antibody comprises a heavy chain comprising HVR-H1 sequence of SEQ ID NO: 41, HVR-H2 sequence of SEQ ID NO: 42, and HVR-H3 sequence of SEQ ID NO: 43; and a light chain comprising HVR-L1 sequence of SEQ ID NO: 44, HVR-L2 sequence of SEQ ID NO: 45, and HVR-L3 sequence of SEQ ID NO: 46.

Embodiment 160. The method of embodiment 159, wherein the antibody comprises a heavy chain variable region comprising the amino acid sequence of SEQ ID NO: 47 and a light chain variable region comprising the amino acid sequence of SEQ ID NO: 48.

Embodiment 161. The method of embodiment 151, wherein the PD-L1 axis binding antagonist is a PD-1 binding antagonist.

Embodiment 162. The method of embodiment 161, wherein the PD-1 binding antagonist inhibits the binding of PD-1 to one or more of its ligand binding partners.

Embodiment 163. The method of embodiment 162, wherein the PD-1 binding antagonist inhibits the binding of PD-1 to PD-L1.

Embodiment 164. The method of embodiment 162, wherein the PD-1 binding antagonist inhibits the binding of PD-1 to PD-L2.

Embodiment 165. The method of any one of embodiments 162-164, wherein the PD-1 binding antagonist inhibits the binding of PD-1 to both PD-L1 and PD-L2.

Embodiment 166. The method of any one of embodiments 161-165, wherein the PD-1 binding antagonist is an antibody or antigen-binding fragment thereof.

Embodiment 167. The method of embodiment 166, wherein the antibody is selected from the group consisting of: MDX-1106 (nivolumab), MK-3475 (pembrolizumab), MEDI-0680 (AMP-514), PDR001, REGN2810, and BGB-108

Embodiment 168. The method of any one of embodiments 161-165, wherein the PD-1 binding antagonist is an Fc-fusion protein.

Embodiment 169. The method of embodiment 168, wherein the Fc-fusion protein is AMP-224.

Embodiment 170. The method of any one of embodiments 53-169, wherein the individual has not been previously treated for the cancer.

Embodiment 171. The method of embodiment 170, wherein the individual has not been previously administered a PD-L1 axis binding antagonist.

Embodiment 172. The method of any one of embodiments 53-171, wherein the individual has previously been treated for the cancer by administration of a platinum-containing chemotherapeutic agent to the individual, and wherein the individual has failed to respond to the chemotherapeutic agent.

Embodiment 173. The method of any one of embodiments 53-172, wherein the treatment comprising a PD-L1 axis binding antagonist is a monotherapy.

Embodiment 174. The method of any one of embodiments 53-173, further comprising administering to the individual an effective amount of one or more additional therapeutic agents.

Embodiment 175. The method of embodiment 174, wherein the one or more additional therapeutic agents comprise an anti-neoplastic agent, a chemotherapeutic agent, a growth inhibitory agent, an anti-angiogenic agent, a radiation therapy, a cytotoxic agent, an immunomodulatory agent, or a combination thereof.

Embodiment 176. The method of embodiment 175, wherein the one or more additional therapeutic agents comprise an immunomodulatory agent.

Embodiment 177. The method of embodiment 176, wherein the immunomodulatory agent is an antibody or antigen-binding fragment thereof.

Embodiment 178. The method of any one of embodiments 53-177, wherein the individual is a human.

Embodiment 179. A method of identifying an individual having a cancer who may benefit from a treatment comprising a PD-L1 axis binding antagonist, the method comprising determining the expression level of five or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual, wherein an immune-score expression level of the five or more genes that is above a reference immune-score expression level of the five or more genes identifies the individual as one who may benefit from a treatment comprising a PD-L1 axis binding antagonist.

Embodiment 180. A method of selecting a therapy for an individual having a cancer, the method comprising determining the expression level of five or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual, wherein an immune-score expression level of the five or more genes in the sample that is above a reference immune-score expression level of the five or more genes identifies the individual as one who may benefit from a treatment comprising a PD-L1 axis binding antagonist.

Embodiment 181. The method of embodiment 179 or 180, wherein the immune-score expression level of the five or more genes in the sample is above the reference immune-score expression level and the method further comprises administering to the individual an effective amount of a PD-L1 axis binding antagonist.

Embodiment 182. The method of any one of embodiments 178-181, wherein the reference immune-score expression level is an immune-score expression level of the five or more genes in a reference population.

Embodiment 183. A method of treating an individual having a cancer, the method comprising:

(a) determining the expression level of five or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual, wherein an immune-score expression level of the five or more genes in the sample is determined to be above a reference immune-score expression level of the five or more genes; and
(b) administering an effective amount of a PD-L1 axis binding antagonist to the individual.

Embodiment 184. A method of treating cancer in an individual that has been determined to have an immune-score expression level of five or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual that is above a reference immune-score expression level of the five or more genes, the method comprising administering to the individual an effective amount of a PD-L1 axis binding antagonist.

Embodiment 185. A method of inducing expansion of a population of T cells in an individual having a cancer, the method comprising:

(a) determining the expression level of five or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual, wherein an immune-score expression level of the five or more genes in the sample is determined to be above a reference immune-score expression level of the five or more genes; and
(b) administering an effective amount of a PD-L1 axis binding antagonist to the individual.

Embodiment 186. A method of inducing expansion of a population of T cells in an individual having a cancer and that has been determined to have an immune-score expression level of five or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual that is above a reference immune-score expression level of the five or more genes, the method comprising administering to the individual an effective amount of a PD-L1 axis binding antagonist.

Embodiment 187. The method of embodiment 185 or 186, wherein the T cells are CD8+ T cells.

Embodiment 188. The method of embodiment 187, wherein the CD8+ T cells are cytotoxic T cells (CTL) or effector memory T cells (Tern).

Embodiment 189. The method of any one of embodiments 183-188, wherein the reference immune-score expression level is an immune-score expression level of the five or more genes in a reference population.

Embodiment 190. The method of embodiment 182 or 189, wherein the reference population is a population of individuals having the cancer.

Embodiment 191. The method of embodiment 190, wherein the population of individuals comprises a first subset of individuals who have been treated with a PD-L1 axis binding antagonist and a second subset of individuals who have been treated with therapy that does not comprise a PD-L1 axis binding antagonist.

Embodiment 192. The method of embodiment 191, wherein the reference immune-score expression level significantly separates each of the first and second subsets of individuals based on a significant difference between an individual's responsiveness to treatment with the PD-L1 axis binding antagonist and an individual's responsiveness to treatment with the therapy that does not comprise a PD-L1 axis binding antagonist above the reference immune-score expression level, wherein the individual's responsiveness to treatment with the PD-L1 axis binding antagonist is significantly improved relative to the individual's responsiveness to treatment with the therapy that does not comprise a PD-L1 axis binding antagonist.

Embodiment 193. The method of embodiment 191 or 192, wherein the reference immune-score expression level significantly separates each of the first and second subsets of individuals based on a significant difference between an individual's responsiveness to treatment with the PD-L1 axis binding antagonist and an individual's responsiveness to treatment with the therapy that does not comprise a PD-L1 axis binding antagonist below the reference immune-score expression level, wherein the individual's responsiveness to treatment with the therapy that does not comprise a PD-L1 axis binding antagonist is significantly improved relative to the individual's responsiveness to treatment with the PD-L1 axis binding antagonist.

Embodiment 194. The method of any one of embodiments 191-193, wherein the therapy that does not comprise a PD-L1 axis binding antagonist comprises an anti-neoplastic agent, a chemotherapeutic agent, a growth inhibitory agent, an anti-angiogenic agent, a radiation therapy, a cytotoxic agent, or a combination thereof.

Embodiment 195. The method of embodiment 194, wherein the therapy that does not comprise a PD-L1 axis binding antagonist comprises a chemotherapeutic agent.

Embodiment 196. The method of embodiment 195, wherein the chemotherapeutic agent is docetaxel.

Embodiment 197. The method of any one of embodiments 192-196, wherein responsiveness to treatment comprises an increase in progression-free survival (PFS).

Embodiment 198. The method of any one of embodiments 192-196, wherein responsiveness to treatment comprises an increase in overall survival (OS).

Embodiment 199. The method of any one of embodiments 185 and 189-198, wherein the reference immune-score expression level is a median of the expression level of each of the five or more genes in the reference population.

Embodiment 200. The method of embodiment 199, wherein the immune-score expression level of the five or more genes in the reference population is a median of a normalized expression level of each of the five or more genes in the reference population.

Embodiment 201. The method of any one of embodiments 182 and 189-198, wherein the reference immune-score expression level is an average of the expression level of each of the five or more genes in the reference population.

Embodiment 202. The method of embodiment 201, wherein the average of the expression level of each of the five or more genes in the reference population is an average of a normalized expression level of each of the five or more genes in the reference population.

Embodiment 203. The method of embodiment 200 or 202, wherein the normalized expression level of each of the five or more genes is the expression level of each of the five or more genes normalized to a reference gene.

Embodiment 204. The method of embodiment 203, wherein the reference gene is CD8A.

Embodiment 205. The method of any one of embodiments 1-204, wherein the reference immune-score expression level is a pre-assigned expression level of the five or more genes.

Embodiment 206. The method of any one of embodiments 1-205, wherein benefit from the treatment comprising a PD-L1 axis binding antagonist comprises an increase in OS.

Embodiment 207. The method of any one of embodiments 1-206, wherein benefit from the treatment comprising a PD-L1 axis binding antagonist comprises an increase in PFS.

Embodiment 208. The method of embodiment 206 or 207, wherein benefit from the treatment comprising a PD-L1 axis binding antagonist comprises an increase in OS and PFS.

Embodiment 209. The method of any one of embodiments 1-208, wherein the genes comprise five or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 210. The method of embodiment 209, wherein the genes comprise six or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 211. The method of embodiment 210, wherein the genes comprise seven or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 212. The method of embodiment 211, wherein the genes comprise eight or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 213. The method of embodiment 212, wherein the genes comprise nine or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 214. The method of embodiment 213, wherein the genes comprise ten or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 215. The method of embodiment 214, wherein the genes comprise CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 216. The method of embodiment 215, wherein the genes consist of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 217. The method of any one of embodiments 1-216, wherein the genes comprise one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 218. The method of embodiment 217, wherein the genes comprise two or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 219. The method of embodiment 218, wherein the genes comprise three or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 220. The method of embodiment 219, wherein the genes comprise four or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 221. The method of embodiment 220, wherein the genes comprise five or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 222. The method of embodiment 221, wherein the genes comprise six or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 223. The method of embodiment 222, wherein the genes comprise seven or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 224. The method of embodiment 223, wherein the genes comprise eight or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 225. The method of embodiment 224, wherein the genes comprise nine or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 226. The method of embodiment 225, wherein the genes comprise ten or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 227. The method of embodiment 226, wherein the genes comprise CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 228. The method of embodiment 227, wherein the genes consist of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 229. The method of any one of embodiments 1-214 and 217-226, wherein the genes comprise a panel set forth in any one of Tables 1-3.

Embodiment 230. The method of any one of embodiments 1-214, 217-226, and 229, wherein the genes comprise CST7, NKG7, GZMH, MT-ND4, and HLA-H.

Embodiment 231. The method of any one of embodiments 1-214, 217-227, 229, and 230, wherein the genes comprise one or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 232. The method of embodiment 231, wherein the genes comprise two or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 233. The method of embodiment 232, wherein the genes comprise three or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 234. The method of embodiment 233, wherein the genes comprise four or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 235. The method of embodiment 234, wherein the genes comprise five or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 236. The method of embodiment 235, wherein the genes comprise six or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 237. The method of embodiment 236, wherein the genes comprise seven or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 238. The method of embodiment 237, wherein the genes comprise eight or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 239. The method of embodiment 238, wherein the genes comprise nine or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 240. The method of embodiment 239, wherein the genes comprise ten or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 241. The method of embodiment 240, wherein the genes comprise GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 242. The method of embodiment 241, wherein the genes consist of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 243. The method of any one of embodiments 1-214, 217-226, and 229-240, wherein the genes comprise one or more cytotoxic genes.

Embodiment 244. The method of embodiment 243, wherein the cytotoxic genes are selected from CST7, PLEK, HCST, CTSW, KLRG1, CD8A, CCL5, CD8B, NKG7, GZMH, and HLA-C.

Embodiment 245. The method of embodiment 244, wherein the genes comprise CST7.

Embodiment 246. The method of embodiment 244 or 245, wherein the genes comprise PLEK.

Embodiment 247. The method of any one of embodiments 244-246, wherein the genes comprise HCST.

Embodiment 248. The method of any one of embodiments 244-247, wherein the genes comprise CTSW.

Embodiment 249. The method of any one of embodiments 244-248, wherein the genes comprise KLRG1.

Embodiment 250. The method of any one of embodiments 244-249, wherein the genes comprise CD8A.

Embodiment 251. The method of any one of embodiments 244-250, wherein the genes comprise CCL5.

Embodiment 252. The method of any one of embodiments 244-251, wherein the genes comprise CD8B.

Embodiment 253. The method of any one of embodiments 244-252, wherein the genes comprise NKG7.

Embodiment 254. The method of any one of embodiments 244-253, wherein the genes comprise GZMH.

Embodiment 255. The method of any one of embodiments 244-254, wherein the genes comprise HLA-C.

Embodiment 256. The method of any one of embodiments 244-255, wherein the genes comprise two or more cytotoxic genes selected from CST7, PLEK, HCST, CTSW, KLRG1, CD8A, CCL5, CD8B, NKG7, GZMH, and HLA-C.

Embodiment 257. The method of embodiment 256, wherein the genes comprise three or more cytotoxic genes selected from CST7, PLEK, HCST, CTSW, KLRG1, CD8A, CCL5, CD8B, NKG7, GZMH, and HLA-C.

Embodiment 258. The method of embodiment 257, wherein the genes comprise four or more cytotoxic genes selected from CST7, PLEK, HCST, CTSW, KLRG1, CD8A, CCL5, CD8B, NKG7, GZMH, and HLA-C.

Embodiment 259. The method of embodiment 258, wherein the genes comprise five or more cytotoxic genes selected from CST7, PLEK, HCST, CTSW, KLRG1, CD8A, CCL5, CD8B, NKG7, GZMH, and HLA-C.

Embodiment 260. The method of embodiment 259, wherein the genes comprise six or more cytotoxic genes selected from CST7, PLEK, HCST, CTSW, KLRG1, CD8A, CCL5, CD8B, NKG7, GZMH, and HLA-C.

Embodiment 261. The method of embodiment 260, wherein the genes comprise seven or more cytotoxic genes selected from CST7, PLEK, HCST, CTSW, KLRG1, CD8A, CCL5, CD8B, NKG7, GZMH, and HLA-C.

Embodiment 262. The method of embodiment 261, wherein the genes comprise eight or more cytotoxic genes selected from CST7, PLEK, HCST, CTSW, KLRG1, CD8A, CCL5, CD8B, NKG7, GZMH, and HLA-C.

Embodiment 263. The method of embodiment 262, wherein the genes comprise nine or more cytotoxic genes selected from CST7, PLEK, HCST, CTSW, KLRG1, CD8A, CCL5, CD8B, NKG7, GZMH, and HLA-C.

Embodiment 264. The method of embodiment 263, wherein the genes comprise ten or more cytotoxic genes selected from CST7, PLEK, HCST, CTSW, KLRG1, CD8A, CCL5, CD8B, NKG7, GZMH, and HLA-C.

Embodiment 265. The method of embodiment 264, wherein the genes comprise all eleven of the following cytotoxic genes CST7, PLEK, HCST, CTSW, KLRG1, CD8A, CCL5, CD8B, NKG7, GZMH, and HLA-C.

Embodiment 266. The method of any one of embodiments 1-214, 217-226, 229-241, and 243-265, wherein the genes comprise one or more mitochondrial genes.

Embodiment 267. The method of embodiment 266, wherein the mitochondrial genes are selected from MT-ND4, MT-CYB, MT-CO2, MT-ND4L, and MT-ATP6.

Embodiment 268. The method of embodiment 267, wherein the genes comprise MT-ND4.

Embodiment 269. The method of embodiment 267 or 268, wherein the genes comprise MT-CYB.

Embodiment 270. The method of any one of embodiments 267-269, wherein the genes comprise MT-CO2.

Embodiment 271. The method of any one of embodiments 267-270, wherein the genes comprise MT-ND4L.

Embodiment 272. The method of any one of embodiments 267-271, wherein the genes comprise MT-ATP6.

Embodiment 273. The method of any one of embodiments 267-272, wherein the genes comprise two or more mitochondrial genes selected from MT-ND4, MT-CYB, MT-CO2, MT-ND4L, and MT-ATP6.

Embodiment 274. The method of embodiment 273, wherein the genes comprise three or more mitochondrial genes selected from MT-ND4, MT-CYB, MT-CO2, MT-ND4L, and MT-ATP6.

Embodiment 275. The method of embodiment 274, wherein the genes comprise four or more mitochondrial genes selected from MT-ND4, MT-CYB, MT-CO2, MT-ND4L, and MT-ATP6.

Embodiment 276. The method of embodiment 275, wherein the genes comprise all five of the following mitochondrial genes MT-ND4, MT-CYB, MT-CO2, MT-ND4L, and MT-ATP6.

Embodiment 277. The method of any one of embodiments 1-214, 217-226, 229-241, and 243-276, wherein the genes comprise a panel set forth in Table 4.

Embodiment 278. The method of any one of embodiments 1-214, 217-226, 229-240, and 243-277, wherein the genes comprise one or more of NKG7, CST7, GZMH, LITAF, CTSW, CCL5, and CD8A.

Embodiment 279. The method of embodiment 278, wherein the genes comprise two or more of NKG7, CST7, GZMH, LITAF, CTSW, CCL5, and CD8A.

Embodiment 280. The method of embodiment 279, wherein the genes comprise three or more of NKG7, CST7, GZMH, LITAF, CTSW, CCL5, and CD8A.

Embodiment 281. The method of embodiment 280, wherein the genes comprise four or more of NKG7, CST7, GZMH, LITAF, CTSW, CCL5, and CD8A.

Embodiment 282. The method of embodiment 281, wherein the genes comprise five or more of NKG7, CST7, GZMH, LITAF, CTSW, CCL5, and CD8A.

Embodiment 283. The method of embodiment 282, wherein the genes comprise six or more of NKG7, CST7, GZMH, LITAF, CTSW, CCL5, and CD8A.

Embodiment 284. The method of embodiment 283, wherein the genes comprise NKG7, CST7, GZMH, LITAF, CTSW, CCL5, and CD8A.

Embodiment 285. The method of embodiment 284, wherein the genes consist of NKG7, CST7, GZMH, LITAF, CTSW, CCL5, and CD8A.

Embodiment 286. The method of any one of embodiments 179-285, wherein the expression level is a nucleic acid expression level.

Embodiment 287. The method of embodiment 286, wherein the nucleic acid expression level is an mRNA expression level.

Embodiment 288. The method of embodiment 287, wherein the mRNA expression level is determined by RNA-seq, RT-qPCR, qPCR, multiplex qPCR or RT-qPCR, microarray analysis, SAGE, MassARRAY technique, FISH, or a combination thereof.

Embodiment 289. The method of embodiment 288, wherein the mRNA expression level is detected using RNA-seq.

Embodiment 290. The method of embodiment 288, wherein the mRNA expression level is detected using RT-qPCR.

Embodiment 291. The method of any one of embodiments 179-290, wherein the expression level is detected in tumor cells, tumor-infiltrating immune cells, stromal cells, normal adjacent tissue (NAT) cells, or a combination thereof.

Embodiment 292. The method of any one of embodiments 179-291, wherein the sample is a tissue sample, a cell sample, a whole blood sample, a plasma sample, a serum sample, or a combination thereof.

Embodiment 293. The method of embodiment 292, wherein the tissue sample is a tumor tissue sample.

Embodiment 294. The method of embodiment 293, wherein the tumor tissue sample comprises tumor cells, tumor-infiltrating immune cells, stromal cells, NAT cells, or a combination thereof.

Embodiment 295. The method of embodiment 293 or 294, wherein the tumor tissue sample is a formalin-fixed and paraffin-embedded (FFPE) sample, an archival sample, a fresh sample, or a frozen sample.

Embodiment 296. The method of embodiment 295, wherein the tumor tissue sample is a FFPE sample.

Embodiment 297. The method of any one of embodiments 179-296, wherein the cancer is a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy.

Embodiment 298. The method of embodiment 297, wherein the cancer is a lung cancer, a kidney cancer, a bladder cancer, or a breast cancer.

Embodiment 299. The method of embodiment 298, wherein the lung cancer is a non-small cell lung cancer (NSCLC).

Embodiment 300. The method of embodiment 298, wherein the kidney cancer is a renal cell carcinoma (RCC).

Embodiment 301. The method of embodiment 298, wherein the bladder cancer is an urothelial carcinoma (UC).

Embodiment 302. The method of embodiment 298, wherein the breast cancer is a triple negative breast cancer (TNBC).

Embodiment 303. The method of any one of embodiments 179-302, wherein the PD-L1 axis binding antagonist is selected from the group consisting of a PD-L1 binding antagonist, a PD-1 binding antagonist, and a PD-L2 binding antagonist.

Embodiment 304. The method of embodiment 303, wherein the PD-L1 axis binding antagonist is a PD-L1 binding antagonist.

Embodiment 305. The method of embodiment 304, wherein the PD-L1 binding antagonist inhibits the binding of PD-L1 to one or more of its ligand binding partners.

Embodiment 306. The method of embodiment 305, wherein the PD-L1 binding antagonist inhibits the binding of PD-L1 to PD-1.

Embodiment 307. The method of embodiment 305, wherein the PD-L1 binding antagonist inhibits the binding of PD-L1 to B7-1.

Embodiment 308. The method of any one of embodiments 305-307, wherein the PD-L1 binding antagonist inhibits the binding of PD-L1 to both PD-1 and B7-1.

Embodiment 309. The method of any one of embodiments 304-308, wherein the PD-L1 binding antagonist is an antibody or antigen-binding fragment thereof.

Embodiment 310. The method of embodiment 309, wherein the antibody is selected from the group consisting of atezolizumab (MPDL3280A), YW243.55.S70, MDX-1105, MEDI4736 (durvalumab), and MSB0010718C (avelumab).

Embodiment 311. The method of embodiment 310, wherein the antibody comprises a heavy chain comprising HVR-H1 sequence of SEQ ID NO: 41, HVR-H2 sequence of SEQ ID NO: 42, and HVR-H3 sequence of SEQ ID NO: 43; and a light chain comprising HVR-L1 sequence of SEQ ID NO: 44, HVR-L2 sequence of SEQ ID NO: 45, and HVR-L3 sequence of SEQ ID NO: 46.

Embodiment 312. The method of embodiment 311, wherein the antibody comprises a heavy chain variable region comprising the amino acid sequence of SEQ ID NO: 47 and a light chain variable region comprising the amino acid sequence of SEQ ID NO: 48.

Embodiment 313. The method of embodiment 303, wherein the PD-L1 axis binding antagonist is a PD-1 binding antagonist.

Embodiment 314. The method of embodiment 313, wherein the PD-1 binding antagonist inhibits the binding of PD-1 to one or more of its ligand binding partners.

Embodiment 315. The method of embodiment 314, wherein the PD-1 binding antagonist inhibits the binding of PD-1 to PD-L1.

Embodiment 316. The method of embodiment 314, wherein the PD-1 binding antagonist inhibits the binding of PD-1 to PD-L2.

Embodiment 317. The method of any one of embodiments 314-316, wherein the PD-1 binding antagonist inhibits the binding of PD-1 to both PD-L1 and PD-L2.

Embodiment 318. The method of any one of embodiments 313-317, wherein the PD-1 binding antagonist is an antibody or antigen-binding fragment thereof.

Embodiment 319. The method of embodiment 318, wherein the antibody is selected from the group consisting of: MDX-1106 (nivolumab), MK-3475 (pembrolizumab), MEDI-0680 (AMP-514), PDR001, REGN2810, and BGB-108.

Embodiment 320. The method of any one of embodiments 313-317, wherein the PD-1 binding antagonist is an Fc-fusion protein.

Embodiment 321. The method of embodiment 320, wherein the Fc-fusion protein is AMP-224.

Embodiment 322. The method of any one of embodiments 179-321, wherein the individual has not been previously treated for the cancer.

Embodiment 323. The method of embodiment 322, wherein the individual has not been previously administered a PD-L1 axis binding antagonist.

Embodiment 324. The method of any one of embodiments 179-321, wherein the individual has previously been treated for the cancer by administration of a platinum-containing chemotherapeutic agent to the individual, and wherein the individual has failed to respond to the chemotherapeutic agent.

Embodiment 325. The method of any one of embodiments 179-182 and 190-324, wherein the treatment comprising a PD-L1 axis binding antagonist is a monotherapy.

Embodiment 326. The method of any one of embodiments 181 and 183-324, further comprising administering to the individual an effective amount of one or more additional therapeutic agents.

Embodiment 327. The method of embodiment 326, wherein the one or more additional therapeutic agents comprise an anti-neoplastic agent, a chemotherapeutic agent, a growth inhibitory agent, an anti-angiogenic agent, a radiation therapy, a cytotoxic agent, an immunomodulatory agent, or a combination thereof.

Embodiment 328. The method of embodiment 327, wherein the one or more additional therapeutic agents comprise an immunomodulatory agent.

Embodiment 329. The method of embodiment 328, wherein the immunomodulatory agent is an antibody or antigen-binding fragment thereof.

Embodiment 330. The method of any one of embodiments 179-329, wherein the individual is a human.

Embodiment 331. A kit for identifying an individual having a cancer who may benefit from a treatment comprising a PD-L1 axis binding antagonist, the kit comprising:

(a) one or more reagents for determining the expression level of five or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual; and, optionally,

(b) instructions for using the one or more reagents to identify an individual having a cancer who may benefit from a treatment comprising a PD-L1 axis binding antagonist according to the method of any one of embodiments 179, 181, 182, and 190-330.

Embodiment 332. An assay for identifying an individual having a cancer who is a candidate for a treatment comprising a PD-L1 axis binding antagonist, the assay comprising determining the expression level of five or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual, wherein an immune-score expression level of the five or more genes in the sample that is above a reference immune-score expression level of the five or more genes identifies the individual as one who may benefit from the treatment comprising a PD-L1 axis binding antagonist, and wherein the reference immune-score expression level is an immune-score expression level of the five or more genes in a reference population.

Embodiment 333. The assay of embodiment 332, wherein the genes comprise five or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 334. The assay of embodiment 333, wherein the genes comprise six or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 335. The assay of embodiment 334, wherein the genes comprise seven or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 336. The assay of embodiment 335, wherein the genes comprise eight or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 337. The assay of embodiment 336, wherein the genes comprise nine or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 338. The assay of embodiment 337, wherein the genes comprise ten or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 339. The assay of embodiment 338, wherein the genes comprise CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 340. The assay of embodiment 339, wherein the genes consist of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, and PATL2.

Embodiment 341. The assay of any one of embodiments 332-338, wherein the genes comprise one or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 342. The assay of embodiment 341, wherein the genes comprise two or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 343. The assay of embodiment 342, wherein the genes comprise three or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 344. The assay of embodiment 343, wherein the genes comprise four or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 345. The assay of embodiment 344, wherein the genes comprise five or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 346. The assay of embodiment 345, wherein the genes comprise six or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 347. The assay of embodiment 346, wherein the genes comprise seven or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 348. The assay of embodiment 347, wherein the genes comprise eight or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 349. The assay of embodiment 348, wherein the genes comprise nine or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 350. The assay of embodiment 349, wherein the genes comprise ten or more of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 351. The assay of embodiment 350, wherein the genes comprise CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 352. The assay of embodiment 351, wherein the genes consist of CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, and LITAF.

Embodiment 353. The assay of any one of embodiments 332-338 and 341-350, wherein the genes comprise a panel set forth in any one of Tables 1-3.

Embodiment 354. The assay of any one of embodiments 332-338, 341-350, and 353, wherein the genes comprise CST7, NKG7, GZMH, MT-ND4, and HLA-H.

Embodiment 355. The assay of any one of embodiments 332-338, 341-351, 353, and 354, wherein the genes comprise one or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 356. The assay of embodiment 355, wherein the genes comprise two or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 357. The assay of embodiment 356, wherein the genes comprise three or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 358. The assay of embodiment 357, wherein the genes comprise four or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 359. The assay of embodiment 358, wherein the genes comprise five or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 360. The assay of embodiment 359, wherein the genes comprise six or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 361. The assay of embodiment 360, wherein the genes comprise seven or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 362. The assay of embodiment 361, wherein the genes comprise eight or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 363. The assay of embodiment 362, wherein the genes comprise nine or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 364. The assay of embodiment 363, wherein the genes comprise ten or more of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 365. The assay of embodiment 364, wherein the genes comprise GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 366. The assay of embodiment 365, wherein the genes consist of GZMB, KLRD1, FGFBP2, NKG7, CST7, KLRC4-KLRK1, KLRK1, GZMH, B2M, LITAF, CTSW, GZMA, ID2, CX3CR1, PRSS23, CCL5, GNLY, PRF1, PATL2, and CD8A.

Embodiment 367. The assay of any one of embodiments 332-338, 341-350, and 353-364, wherein the genes comprise one or more cytotoxic genes.

Embodiment 368. The assay of embodiment 367, wherein the cytotoxic genes are selected from CST7, PLEK, HCST, CTSW, KLRG1, CD8A, CCL5, CD8B, NKG7, GZMH, and HLA-C.

Embodiment 369. The assay of embodiment 368, wherein the genes comprise CST7.

Embodiment 370. The assay of embodiment 368 or 369, wherein the genes comprise PLEK.

Embodiment 371. The assay of any one of embodiments 368-370, wherein the genes comprise HCST.

Embodiment 372. The assay of any one of embodiments 368-371, wherein the genes comprise CTSW.

Embodiment 373. The assay of any one of embodiments 368-372, wherein the genes comprise KLRG1.

Embodiment 374. The assay of any one of embodiments 368-373, wherein the genes comprise CD8A.

Embodiment 375. The assay of any one of embodiments 368-374, wherein the genes comprise CCL5.

Embodiment 376. The assay of any one of embodiments 368-375, wherein the genes comprise CD8B.

Embodiment 377. The assay of any one of embodiments 368-376, wherein the genes comprise NKG7.

Embodiment 378. The assay of any one of embodiments 368-377, wherein the genes comprise GZMH.

Embodiment 379. The assay of any one of embodiments 368-378, wherein the genes comprise HLA-C.

Embodiment 380. The assay of any one of embodiments 368-379, wherein the genes comprise two or more cytotoxic genes selected from CST7, PLEK, HCST, CTSW, KLRG1, CD8A, CCL5, CD8B, NKG7, GZMH, and HLA-C.

Embodiment 381. The assay of embodiment 380, wherein the genes comprise three or more cytotoxic genes selected from CST7, PLEK, HCST, CTSW, KLRG1, CD8A, CCL5, CD8B, NKG7, GZMH, and HLA-C.

Embodiment 382. The assay of embodiment 381, wherein the genes comprise four or more cytotoxic genes selected from CST7, PLEK, HCST, CTSW, KLRG1, CD8A, CCL5, CD8B, NKG7, GZMH, and HLA-C.

Embodiment 383. The assay of embodiment 382, wherein the genes comprise five or more cytotoxic genes selected from CST7, PLEK, HCST, CTSW, KLRG1, CD8A, CCL5, CD8B, NKG7, GZMH, and HLA-C.

Embodiment 384. The assay of embodiment 383, wherein the genes comprise six or more cytotoxic genes selected from CST7, PLEK, HCST, CTSW, KLRG1, CD8A, CCL5, CD8B, NKG7, GZMH, and HLA-C.

Embodiment 385. The assay of embodiment 384, wherein the genes comprise seven or more cytotoxic genes selected from CST7, PLEK, HCST, CTSW, KLRG1, CD8A, CCL5, CD8B, NKG7, GZMH, and HLA-C.

386. The assay of embodiment 385, wherein the genes comprise eight or more cytotoxic genes selected from CST7, PLEK, HCST, CTSW, KLRG1, CD8A, CCL5, CD8B, NKG7, GZMH, and HLA-C.

Embodiment 387. The assay of embodiment 386, wherein the genes comprise nine or more cytotoxic genes selected from CST7, PLEK, HCST, CTSW, KLRG1, CD8A, CCL5, CD8B, NKG7, GZMH, and HLA-C.

Embodiment 388. The assay of embodiment 387, wherein the genes comprise ten or more cytotoxic genes selected from CST7, PLEK, HCST, CTSW, KLRG1, CD8A, CCL5, CD8B, NKG7, GZMH, and HLA-C.

Embodiment 389. The assay of embodiment 388, wherein the genes comprise all eleven of the following cytotoxic genes CST7, PLEK, HCST, CTSW, KLRG1, CD8A, CCL5, CD8B, NKG7, GZMH, and HLA-C.

Embodiment 390. The assay of any one of embodiments 332-338, 341-350, 353-365, and 367-389, wherein the genes comprise one or more mitochondrial genes.

Embodiment 391. The assay of embodiment 390, wherein the mitochondrial genes are selected from MT-ND4, MT-CYB, MT-CO2, MT-ND4L, and MT-ATP6.

Embodiment 392. The assay of embodiment 391, wherein the genes comprise MT-ND4.

Embodiment 393. The assay of embodiment 391 or 392, wherein the genes comprise MT-CYB.

Embodiment 394. The assay of any one of embodiments 391-393, wherein the genes comprise MT-CO2.

Embodiment 395. The assay of any one of embodiments 391-394, wherein the genes comprise MT-ND4L.

Embodiment 396. The assay of any one of embodiments 391-395, wherein the genes comprise MT-ATP6.

Embodiment 397. The assay of any one of embodiments 391-396, wherein the genes comprise two or more mitochondrial genes selected from MT-ND4, MT-CYB, MT-CO2, MT-ND4L, and MT-ATP6.

Embodiment 398. The assay of embodiment 397, wherein the genes comprise three or more mitochondrial genes selected from MT-ND4, MT-CYB, MT-CO2, MT-ND4L, and MT-ATP6.

Embodiment 399. The assay of embodiment 398, wherein the genes comprise four or more mitochondrial genes selected from MT-ND4, MT-CYB, MT-CO2, MT-ND4L, and MT-ATP6.

Embodiment 400. The assay of embodiment 399, wherein the genes comprise all five of the following mitochondrial genes MT-ND4, MT-CYB, MT-CO2, MT-ND4L, and MT-ATP6.

Embodiment 401. The assay of any one of embodiments 332-338, 341-350, 353-365, and 367-400, wherein the genes comprise a panel set forth in Table 4.

Embodiment 402. The assay of any one of embodiments 332-338, 341-350, 353-364, and 367-401, wherein the genes comprise one or more of NKG7, CST7, GZMH, LITAF, CTSW, CCL5, and CD8A.

Embodiment 403. The assay of embodiment 402, wherein the genes comprise two or more of NKG7, CST7, GZMH, LITAF, CTSW, CCL5, and CD8A.

Embodiment 404. The assay of embodiment 403, wherein the genes comprise three or more of NKG7, CST7, GZMH, LITAF, CTSW, CCL5, and CD8A.

Embodiment 405. The assay of embodiment 404, wherein the genes comprise four or more of NKG7, CST7, GZMH, LITAF, CTSW, CCL5, and CD8A.

Embodiment 406. The assay of embodiment 405, wherein the genes comprise five or more of NKG7, CST7, GZMH, LITAF, CTSW, CCL5, and CD8A.

Embodiment 407. The assay of embodiment 406, wherein the genes comprise six or more of NKG7, CST7, GZMH, LITAF, CTSW, CCL5, and CD8A.

Embodiment 408. The assay of embodiment 407, wherein the genes comprise NKG7, CST7, GZMH, LITAF, CTSW, CCL5, and CD8A.

Embodiment 409. The assay of embodiment 408, wherein the genes consist of NKG7, CST7, GZMH, LITAF, CTSW, CCL5, and CD8A.

Embodiment 410. The assay of any one of embodiments 332-409, wherein the cancer is selected from the group consisting of a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy.

Embodiment 411. The assay of embodiment 410, wherein the cancer is a lung cancer, a kidney cancer, a bladder cancer, or a breast cancer.

Embodiment 412. The assay of embodiment 411, wherein the lung cancer is a non-small cell lung cancer (NSCLC).

Embodiment 413. The assay of embodiment 412, wherein the kidney cancer is a renal cell carcinoma (RCC).

Embodiment 414. The assay of embodiment 413, wherein the bladder cancer is an urothelial carcinoma (UC).

Embodiment 415. The assay of embodiment 414, wherein the breast cancer is a triple negative breast cancer (TNBC).

Embodiment 416. The assay of any one of embodiments 332-415, wherein the PD-L1 axis binding antagonist is a PD-L1 binding antagonist, a PD-1 binding antagonist, or a PD-L2 binding antagonist.

Embodiment 417. The assay of embodiment 416, wherein the PD-L1 axis binding antagonist is a PD-L1 binding antagonist.

Embodiment 418. The assay of embodiment 417, wherein the PD-L1 binding antagonist inhibits the binding of PD-L1 to one or more of its ligand binding partners.

Embodiment 419. The assay of embodiment 418, wherein the PD-L1 binding antagonist inhibits the binding of PD-L1 to PD-1.

Embodiment 420. The assay of embodiment 418, wherein the PD-L1 binding antagonist inhibits the binding of PD-L1 to B7-1.

Embodiment 421. The assay of any one of embodiments 418-420, wherein the PD-L1 binding antagonist inhibits the binding of PD-L1 to both PD-1 and B7-1.

Embodiment 422. The assay of any one of embodiments 417-421, wherein the PD-L1 binding antagonist is an antibody or antigen-binding fragment thereof.

Embodiment 423. The assay of embodiment 422, wherein the antibody is selected from the group consisting of atezolizumab (MPDL3280A), YW243.55.S70, MDX-1105, MEDI4736 (durvalumab), and MSB0010718C (avelumab).

Embodiment 424. The assay of embodiment 423, wherein the antibody comprises a heavy chain comprising HVR-H1 sequence of SEQ ID NO: 41, HVR-H2 sequence of SEQ ID NO: 42, and HVR-H3 sequence of SEQ ID NO: 43; and a light chain comprising HVR-L1 sequence of SEQ ID NO: 44, HVR-L2 sequence of SEQ ID NO: 45, and HVR-L3 sequence of SEQ ID NO: 46.

Embodiment 425. The assay of embodiment 424, wherein the antibody comprises a heavy chain variable region comprising the amino acid sequence of SEQ ID NO: 47 and a light chain variable region comprising the amino acid sequence of SEQ ID NO: 48.

Embodiment 426. The assay of embodiment 416, wherein the PD-L1 axis binding antagonist is a PD-1 binding antagonist.

Embodiment 427. The assay of embodiment 426, wherein the PD-1 binding antagonist inhibits the binding of PD-1 to one or more of its ligand binding partners.

Embodiment 428. The assay of embodiment 427, wherein the PD-1 binding antagonist inhibits the binding of PD-1 to PD-L1.

Embodiment 429. The assay of embodiment 427, wherein the PD-1 binding antagonist inhibits the binding of PD-1 to PD-L2.

Embodiment 430. The assay of any one of embodiments 427-429, wherein the PD-1 binding antagonist inhibits the binding of PD-1 to both PD-L1 and PD-L2.

Embodiment 431. The assay of any one of embodiments 426-430, wherein the PD-1 binding antagonist is an antibody or antigen-binding fragment thereof.

Embodiment 432. The assay of embodiment 431, wherein the antibody is selected from the group consisting of: MDX-1106 (nivolumab), MK-3475 (pembrolizumab), MEDI-0680 (AMP-514), PDR001, REGN2810, and BGB-108.

Embodiment 433. The assay of any one of embodiments 426-430, wherein the PD-1 binding antagonist is an Fc-fusion protein.

Embodiment 434. The assay of embodiment 433, wherein the Fc-fusion protein is AMP-224.

Embodiment 435. A method of identifying an individual having a cancer who may benefit from a treatment comprising a PD-L1 axis binding antagonist, the method comprising determining the expression level of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual, wherein the genes comprise a cytotoxic gene and a mitochondrial gene, and wherein an immune-score expression level of the two or more genes that is above a reference immune-score expression level of the two or more genes identifies the individual as one who may benefit from a treatment comprising a PD-L1 axis binding antagonist.

Embodiment 436. A method of selecting a therapy for an individual having a cancer, the method comprising determining the expression level of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual, wherein the genes comprise a cytotoxic gene and a mitochondrial gene, and wherein an immune-score expression level of the two or more genes in the sample that is above a reference immune-score expression level of the two or more genes identifies the individual as one who may benefit from a treatment comprising a PD-L1 axis binding antagonist.

Embodiment 437. A method of treating an individual having a cancer, the method comprising:

(a) determining the expression level of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1 orf21, C12orf75, and GIMAP4 in a sample from the individual, wherein the genes comprise a cytotoxic gene and a mitochondrial gene, and wherein an immune-score expression level of the two or more genes in the sample is determined to be above a reference immune-score expression level of the two or more genes; and
(b) administering an effective amount of a PD-L1 axis binding antagonist to the individual.

Embodiment 438. A method of treating cancer in an individual that has been determined to have an immune-score expression level of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual that is above a reference immune-score expression level of the two or more genes, wherein the genes comprise a cytotoxic gene and a mitochondrial gene, the method comprising administering to the individual an effective amount of a PD-L1 axis binding antagonist.

Embodiment 439. A method of inducing expansion of a population of T cells in an individual having a cancer, the method comprising:

(a) determining the expression level of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual, wherein the genes comprise a cytotoxic gene and a mitochondrial gene, and wherein an immune-score expression level of the two or more genes in the sample is determined to be above a reference immune-score expression level of the two or more genes; and
(b) administering an effective amount of a PD-L1 axis binding antagonist to the individual.

Embodiment 440. A method of inducing expansion of a population of T cells in an individual having a cancer and that has been determined to have an immune-score expression level of two or more of genes CST7, NKG7, GZMH, MT-ND4, HLA-H, CCL5, CD8A, CMC1, CD8B, HCST, MT-CYB, MT-ND4L, KLRG1, MT-CO2, MT-ATP6, PLEK, CTSW, HLA-C, LYAR, LITAF, GZMB, KLRD1, FGFBP2, KLRC4-KLRK1, KLRK1, B2M, GZMA, ID2, CX3CR1, PRSS23, GNLY, PRF1, PATL2, S1PR1, FCRL6, PLAC8, C1orf21, C12orf75, and GIMAP4 in a sample from the individual that is above a reference immune-score expression level of the two or more genes, wherein the genes comprise a cytotoxic gene and a mitochondrial gene, the method comprising administering to the individual an effective amount of a PD-L1 axis binding antagonist.

## Claims

1. A method of selecting a therapy for an individual having a cancer, the method comprising determining whether the individual has clonally expanded T cells in a sample of peripheral blood from the individual, wherein clonally expanded T cells in the sample identify the individual as one who may benefit from a treatment comprising a PD-L1 axis binding antagonist.

2. The method of claim 1, wherein the clonally expanded T cells are CD8+ T cells.

3. The method of claim 2, wherein the CD8+ T cells are cytotoxic T cells (CTL).

4. The method of any one of claims 1-3, wherein the clonally expanded T cells express T cell receptors (TCRs) comprising alpha and beta chains each comprising complementarity-determining regions (CDRs) CDR-1, CDR-2, and CDR-3, and wherein the nucleic acid sequences encoding corresponding alpha chain CDR-1, CDR-2, or CDR-3, and/or corresponding beta chain CDR-1, CDR-2, or CDR-3 are at least 85% identical to one another; optionally wherein:

    (a) the nucleic acid sequences encoding corresponding alpha chain CDR1 are at least 85% identical to one another;
    (b) the nucleic acid sequences encoding corresponding alpha chain CDR2 are at least 85% identical to one another;
    (c) the nucleic acid sequences encoding corresponding alpha chain CDR3 are at least 85% identical to one another;
    (d) the nucleic acid sequences encoding corresponding beta chain CDR1 are at least 85% identical to one another;
    (e) the nucleic acid sequences encoding corresponding beta chain CDR2 are at least 85% identical to one another;
    (f) the nucleic acid sequences encoding corresponding beta chain CDR3 are at least 85% identical to one another; and/or
    (g) the clonally expanded T cells express TCRs comprising alpha and/or beta chains having nucleic acid sequences that are at least 85% identical to one another.

5. The method of claim 1, wherein benefit from the treatment comprising a PD-L1 axis binding antagonist comprises an increase in overall survival (OS) and/or progression-free survival (PFS).

6. The method of any one of claims 1-5, wherein the cancer is a lung cancer, a kidney cancer, a bladder cancer, a breast cancer, a colorectal cancer, an ovarian cancer, a pancreatic cancer, a gastric carcinoma, an esophageal cancer, a mesothelioma, a melanoma, a head and neck cancer, a thyroid cancer, a sarcoma, a prostate cancer, a glioblastoma, a cervical cancer, a thymic carcinoma, a leukemia, a lymphoma, a myeloma, a mycosis fungoides, a Merkel cell cancer, or a hematologic malignancy;

    optionally wherein the cancer is a lung cancer, a kidney cancer, a bladder cancer, or a breast cancer; optionally wherein:

        (a) the lung cancer is a non-small cell lung cancer (NSCLC);
        (b) the kidney cancer is a renal cell carcinoma (RCC);
        (c) the bladder cancer is an urothelial carcinoma (UC); or
        (d) the breast cancer is a triple negative breast cancer (TNBC).

7. The method of any one of claims 1-6, wherein the PD-L1 axis binding antagonist is selected from the group consisting of a PD-L1 binding antagonist, a PD-1 binding antagonist, and a PD-L2 binding antagonist; optionally wherein:

    (a) the PD-L1 axis binding antagonist is a PD-L1 binding antagonist, optionally wherein the PD-L1 binding antagonist inhibits the binding of PD-L1 to one or more of its ligand binding partners, optionally wherein the PD-L1 binding antagonist inhibits the binding of PD-L1 to PD-1 and/or B7-1; or
    (b) the PD-L1 axis binding antagonist is a PD-1 binding antagonist, optionally wherein the PD-1 binding antagonist inhibits the binding of PD-1 to one or more of its ligand binding partners, optionally wherein the PD-1 binding antagonist inhibits the binding of PD-1 to PD-L1 and/or PD-L2.

**8.** The method of claim 7, wherein the PD-L1 binding antagonist is an antibody or antigen-binding fragment thereof; optionally wherein the antibody is selected from the group consisting of atezolizumab (MPDL3280A), YW243.55.S70, MDX-1105, MEDI4736 (durvalumab), and MSB0010718C (avelumab).

**9.** The method of claim 8, wherein the antibody comprises a heavy chain comprising HVR-H1 sequence of SEQ ID NO: 41, HVR-H2 sequence of SEQ ID NO: 42, and HVR-H3 sequence of SEQ ID NO: 43; and a light chain comprising HVR-L1 sequence of SEQ ID NO: 44, HVR-L2 sequence of SEQ ID NO: 45, and HVR-L3 sequence of SEQ ID NO: 46; optionally wherein the antibody comprises a heavy chain variable region comprising the amino acid sequence of SEQ ID NO: 47 and a light chain variable region comprising the amino acid sequence of SEQ ID NO: 48.

**10.** The method of claim 7, wherein:

(a) the PD-1 binding antagonist is an antibody or antigen-binding fragment thereof, optionally wherein the antibody is selected from the group consisting of: MDX-1106 (nivolumab), MK-3475 (pembrolizumab), MEDI-0680 (AMP-514), PDR001, REGN2810, and BGB-108; or
(b) the PD-1 binding antagonist is an Fc-fusion protein, optionally wherein the Fc-fusion protein is AMP-224.

**11.** The method of any one of claims 1-10, wherein the individual has not been previously treated for the cancer, optionally wherein the individual has not been previously administered a PD-L1 axis binding antagonist.

**12.** The method of any one of claims 1-10, wherein the individual has previously been treated for the cancer by administration of a platinum-containing chemotherapeutic agent to the individual, and wherein the individual has failed to respond to the chemotherapeutic agent.

**13.** The method of any one of claims 1-12, wherein the individual is a human.

**14.** A PD-L1 axis binding antagonist for use in a method of treating an individual having a cancer, wherein the individual has been identified as one who may benefit from a treatment comprising a PD-L1 axis binding antagonist in accordance with the method of any one of claims 1-13.

**15.** The PD-L1 axis binding antagonist for use in accordance with claim 14, wherein the method of treating the individual further comprises administering to the individual an effective amount of one or more additional therapeutic agents; optionally wherein the one or more additional therapeutic agents comprise an anti-neoplastic agent, a chemotherapeutic agent, a growth inhibitory agent, an anti-angiogenic agent, a radiation therapy, a cytotoxic agent, an immunomodulatory agent, or a combination thereof, optionally wherein the immunomodulatory agent is an antibody or antigen-binding fragment thereof.

FIG. 1A

| | Transcripts | Cells | Typed | Distinct (T/Dual/N) |
|---|---|---|---|---|
| T1 | 28,117,546 | 7,446 | 6,070 | 2880 / 284 / 1531 |
| N1 | 18,012,986 | 6,162 | 4,554 | |
| T2 | 42,735,760 | 8,770 | 7,895 | 3690 / 475 / 2258 |
| N2 | 30,307,207 | 7,388 | 6,207 | |
| T3 | 10,359,980 | 6,938 | 4,582 | 2354 / 353 / 4374 |
| N3 | 19,749,928 | 11,997 | 8,233 | |
| Total | 149,283,407 | 48,701 | 37,531 | 18,194 |

FIG. 1B

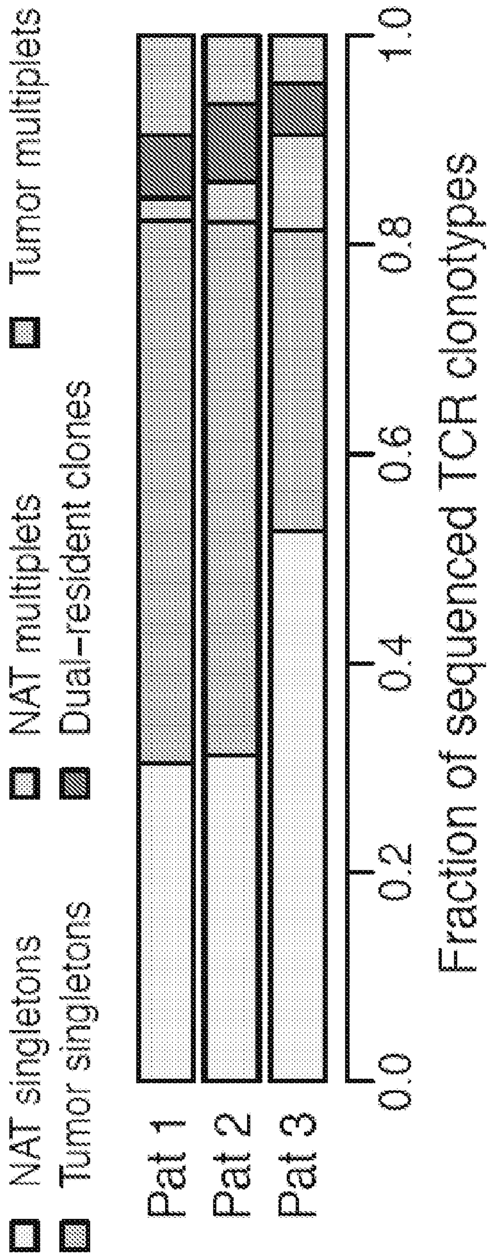
NAT singletons
Tumor singletons
NAT multipelets
Dual–resident clones
Tumor multiplets
Pat 1
Pat 2
Pat 3
0.0
0.2
0.4
0.6
0.8
1.0
Fraction of sequenced TCR clonotypes

FIG. 1C

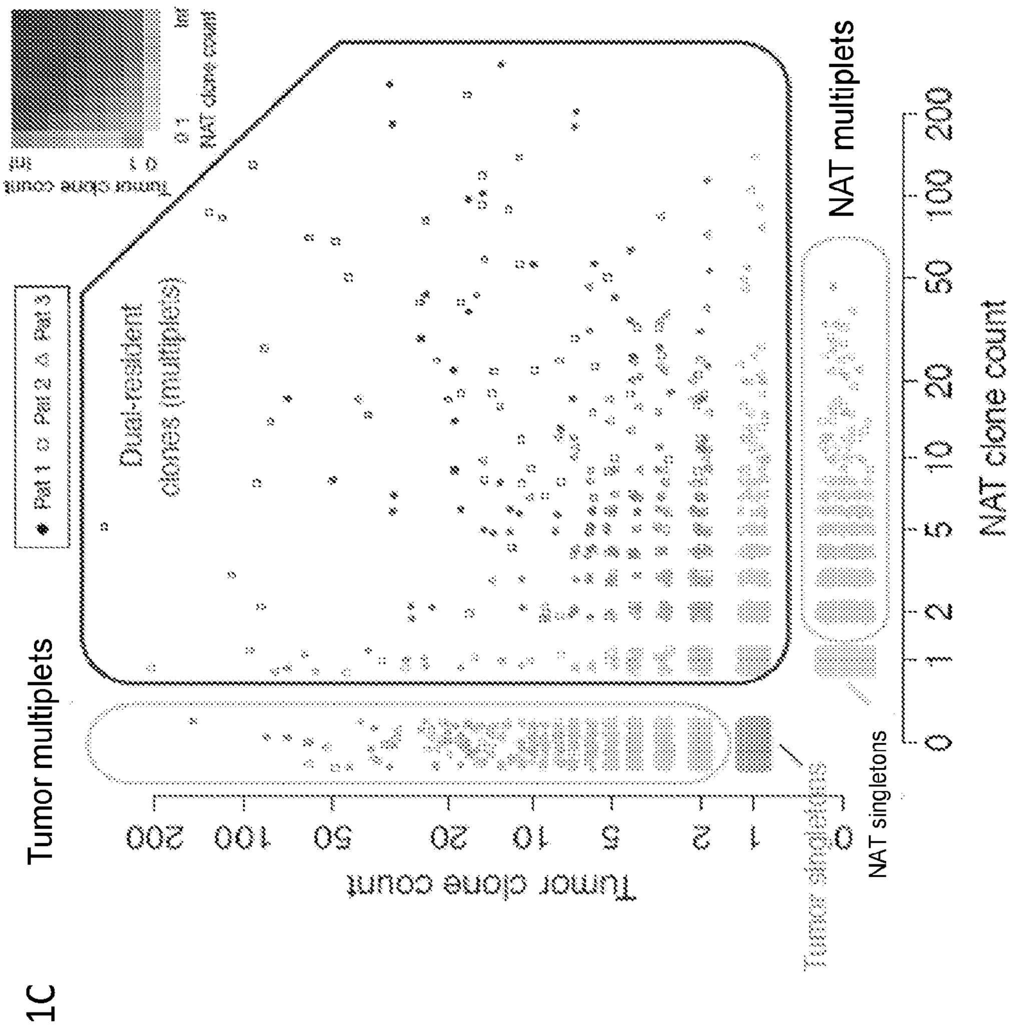
Tumor multiplets
Tumor clone count
200
100
50
20
10
5
2
1
0
Tumor singletons
NAT singletons
Pat 1
Pat 2
Pat 3
Dual-resident clones (multiplets)
Tumor clone count
NAT clone count
NAT multiplets
NAT clone count
0
1
2
5
10
20
50
100
200

FIG. 1D

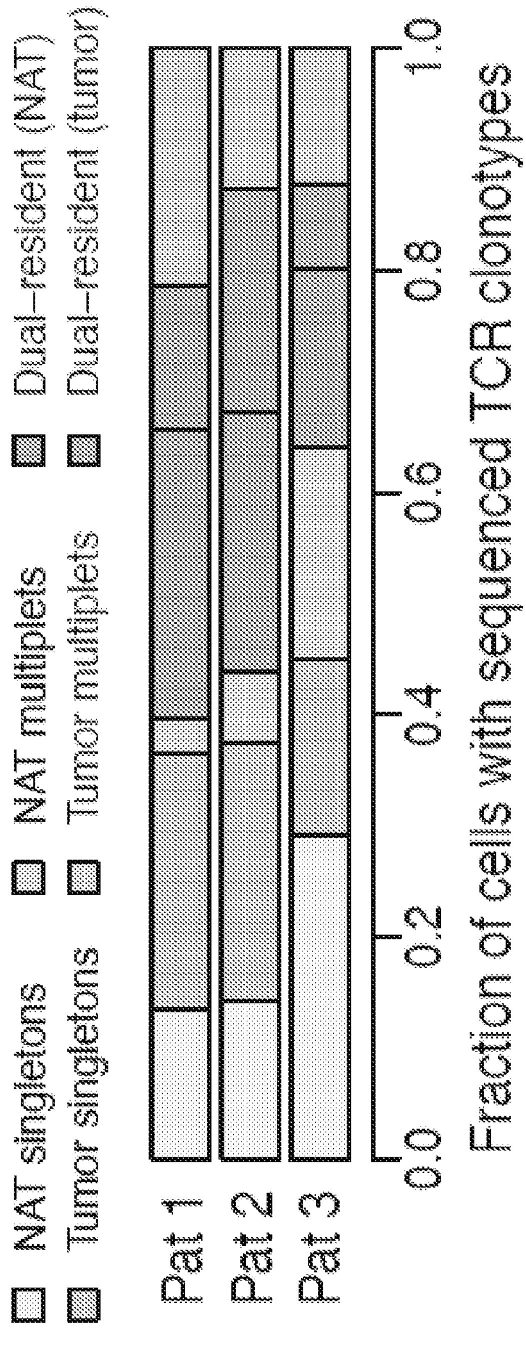
NAT singletons
Tumor singletons
NAT multiplets
Tumor multiplets
Dual–resident (NAT)
Dual–resident (tumor)
Pat 1
Pat 2
Pat 3
0.0
0.2
0.4
0.6
0.8
1.0
Fraction of cells with sequenced TCR clonotypes

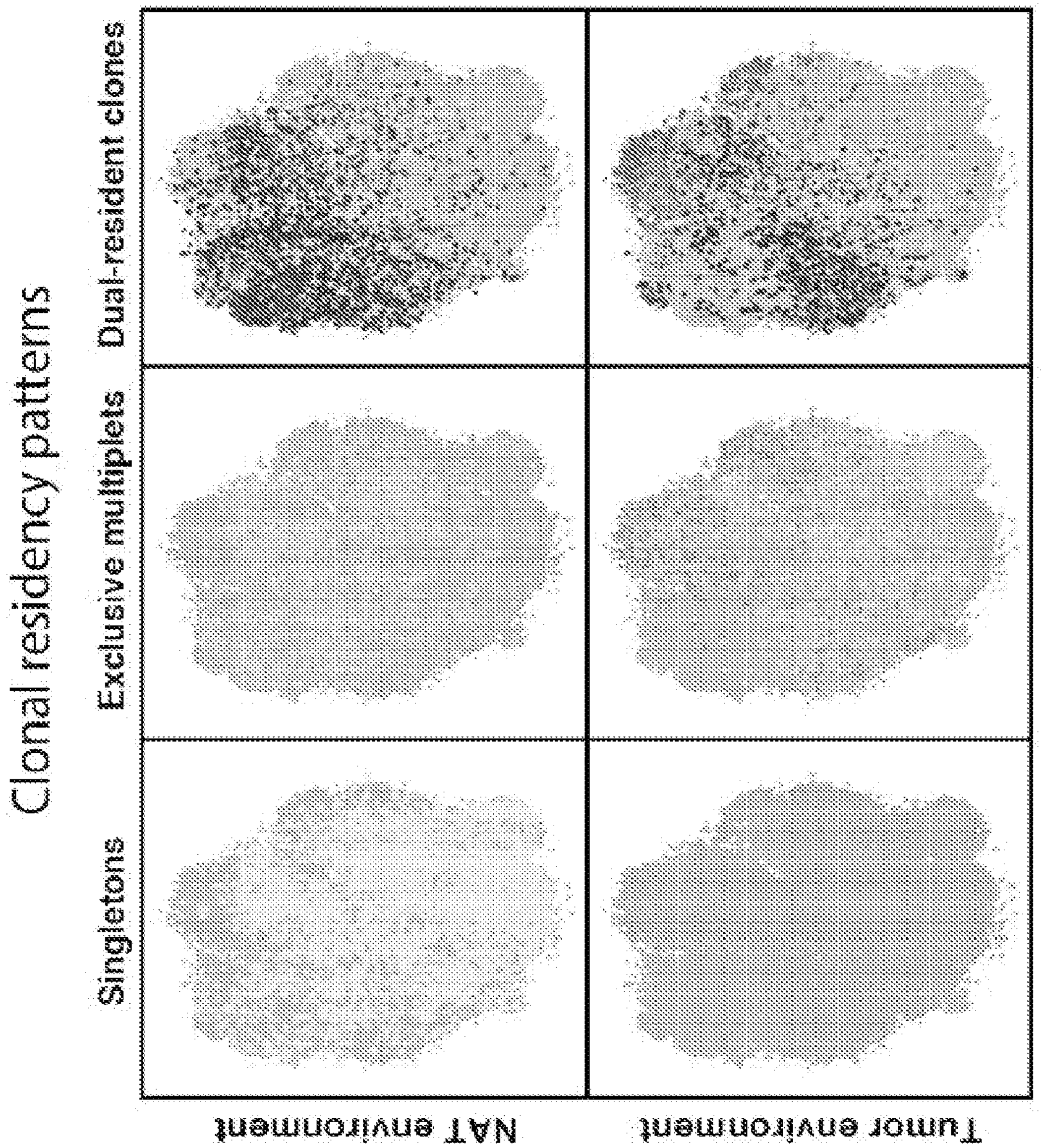
Clonal residency patterns
Singletons
Exclusive multiplets
Dual-resident clones
NAT environment
Tumor environment

FIG. 2A

FIG. 2B

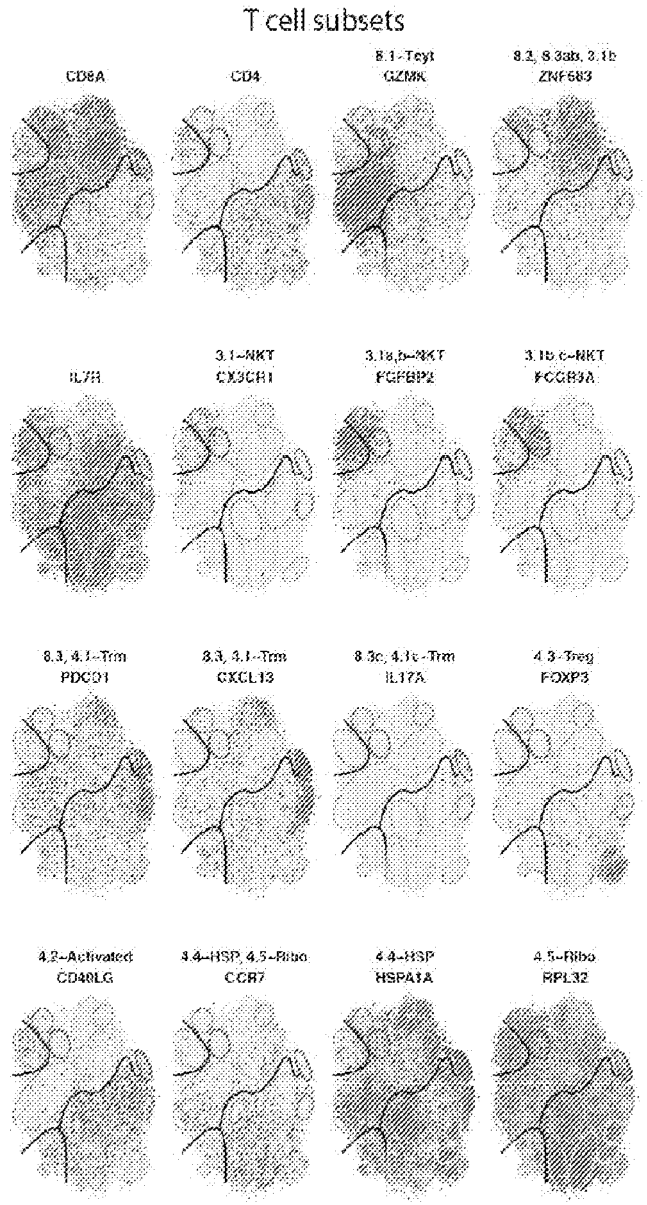
T cell subsets
CD8A
CD4
8.1~Tcyt
GZMK
8.2, 8.3ab, 3.1b
ZNF683
IL7R
3.1~NKT
CX3CR1
3.1a,b~NKT
FGFBP2
3.1b,c~NKT
FCGR3A
8.3, 4.1~Trm
PDCD1
8.3, 4.1~Trm
CXCL13
8.3c, 4.1c~Trm
IL17A
4.3~Treg
FOXP3
4.2~Activated
CD40LG
4.4~HSP, 4.5~Ribo
CCR7
4.4~HSP
HSPA1A
4.5~Ribo
RPL32

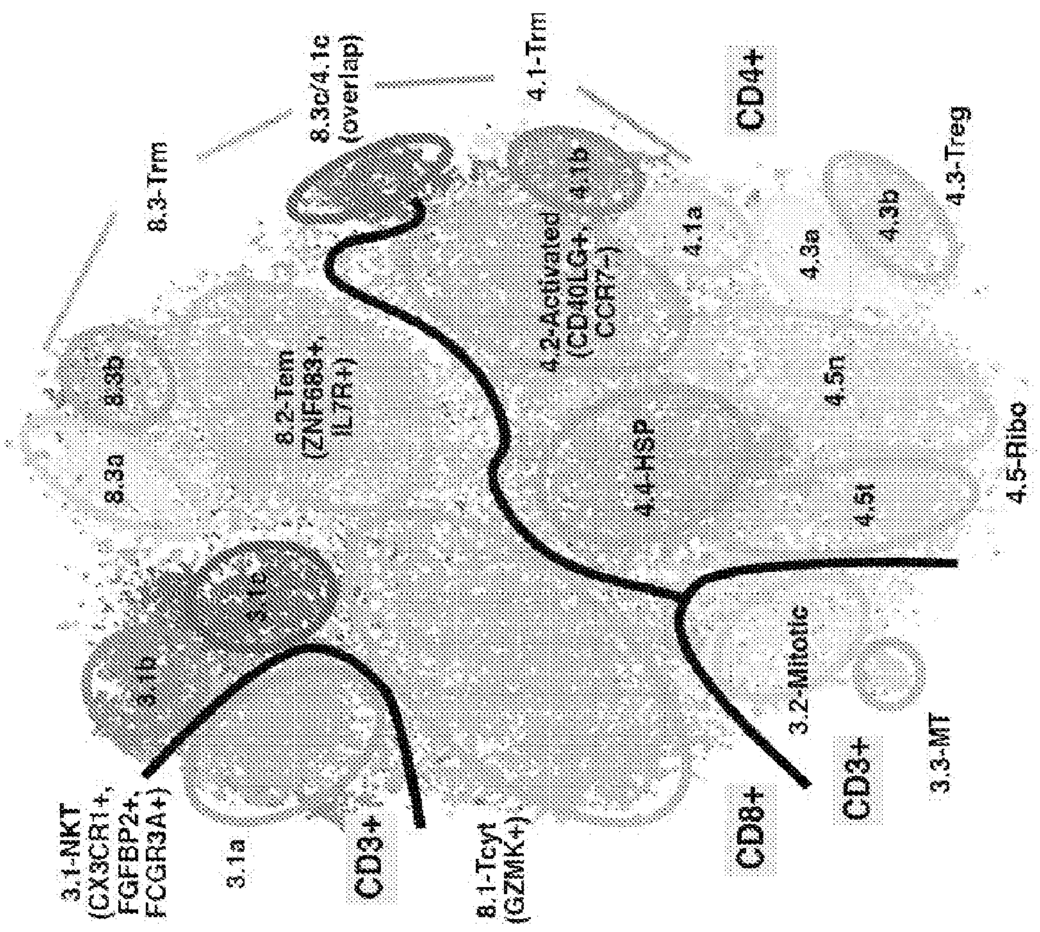
8.3c/4.1c (overlap)
4.1-Trm
8.3-Trm
CD4+
4.3-Treg
4.1b
4.1a
4.3a
4.3b
4.2-Activated (CD40LG+, CCR7−)
8.3b
8.3a
8.2-Tem (ZNF683+, IL7R+)
4.5n
4.4-HSP
4.5-Ribo
4.5t
3.1c
3.1b
3.1a
3.1-NKT (CX3CR1+, FGFBP2+, FCGR3A+)
CD3+
8.1-Tcyt (GZMK+)
CD8+
3.2-Mitotic
CD3+
3.3-MT

FIG. 2C

FIG. 2D

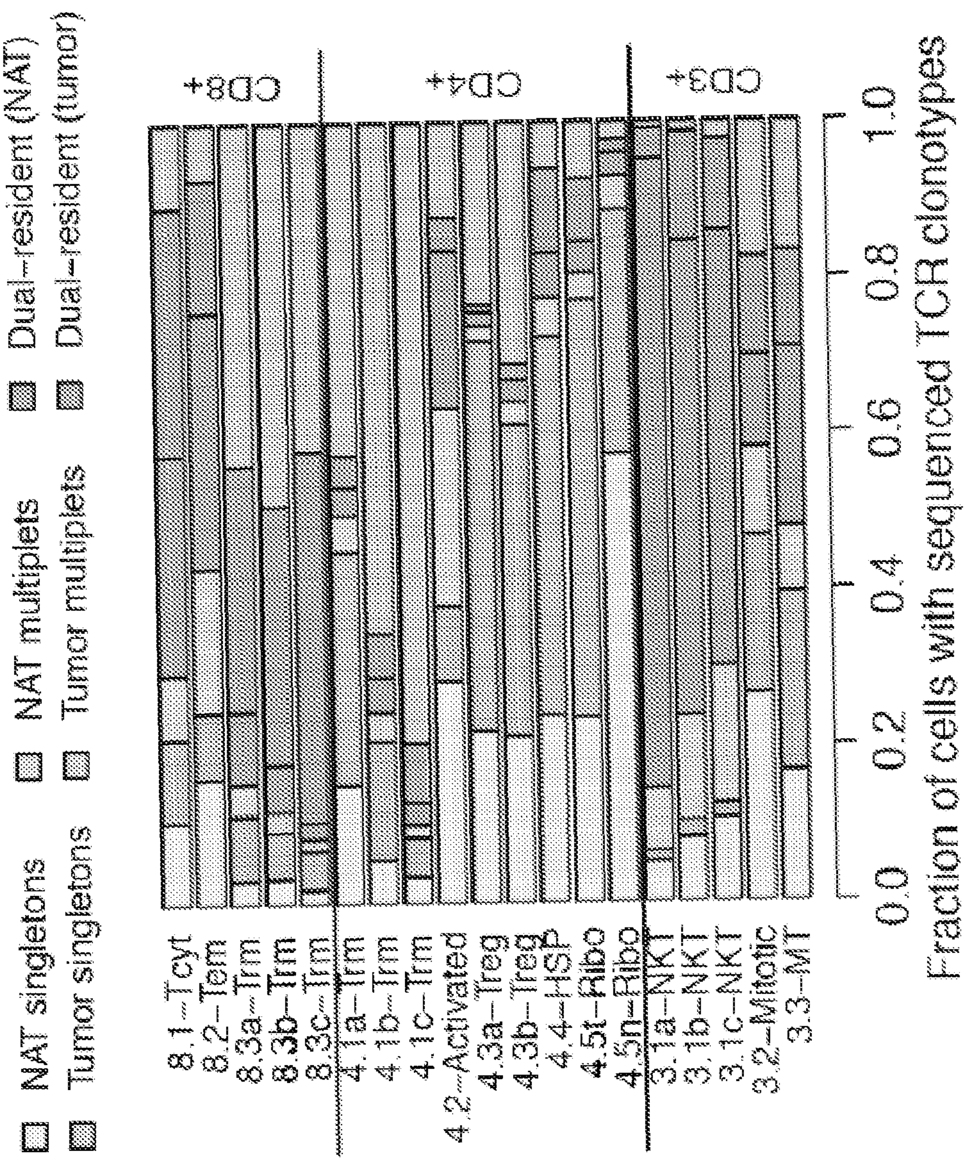
NAT singletons
NAT multiplets
Dual-resident (NAT)
Tumor singletons
Tumor multiplets
Dual-resident (tumor)
CD8+
CD4+
CD3+
8.1-Tcyt
8.2-Tem
8.3a-Trm
8.3b-Trm
8.3c-Trm
4.1a-Trm
4.1b-Trm
4.1c-Trm
4.2-Activated
4.3a-Treg
4.3b-Treg
4.4-HSP
4.5t-Ribo
4.5n-Ribo
3.1a-NKT
3.1b-NKT
3.1c-NKT
3.2-Mitotic
3.3-MT
0.0
0.2
0.4
0.6
0.8
1.0
Fraction of cells with sequenced TCR clonotypes

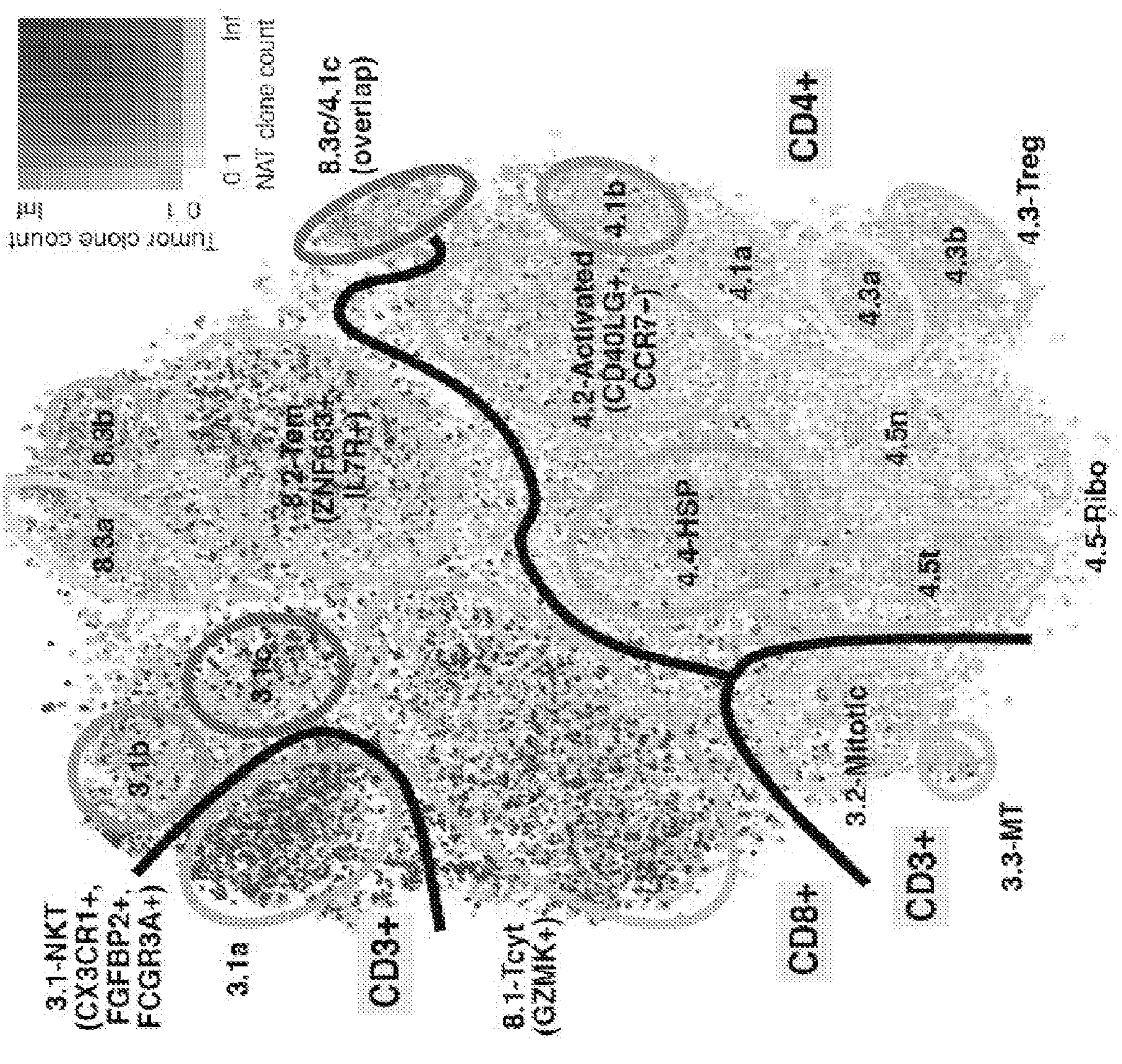
Tumor clone count
0.1
Inf
NAT clone count
8.3c/4.1c
(overlap)
CD4+
4.1b
4.1a
4.2-Activated
(CD40LG+,
CCR7−)
4.3a
4.3b
4.3-Treg
8.3a
8.3b
8.2-Tem
(ZNF683+,
IL7R+)
4.4-HSP
4.5n
4.5t
4.5-Ribo
3.1-NKT
(CX3CR1+,
FGFBP2+,
FCGR3A+)
3.1a
3.1b
3.1c
CD3+
8.1-Tcyt
(GZMK+)
CD8+
3.2-Mitotic
CD3+
3.3-MT

FIG. 2E

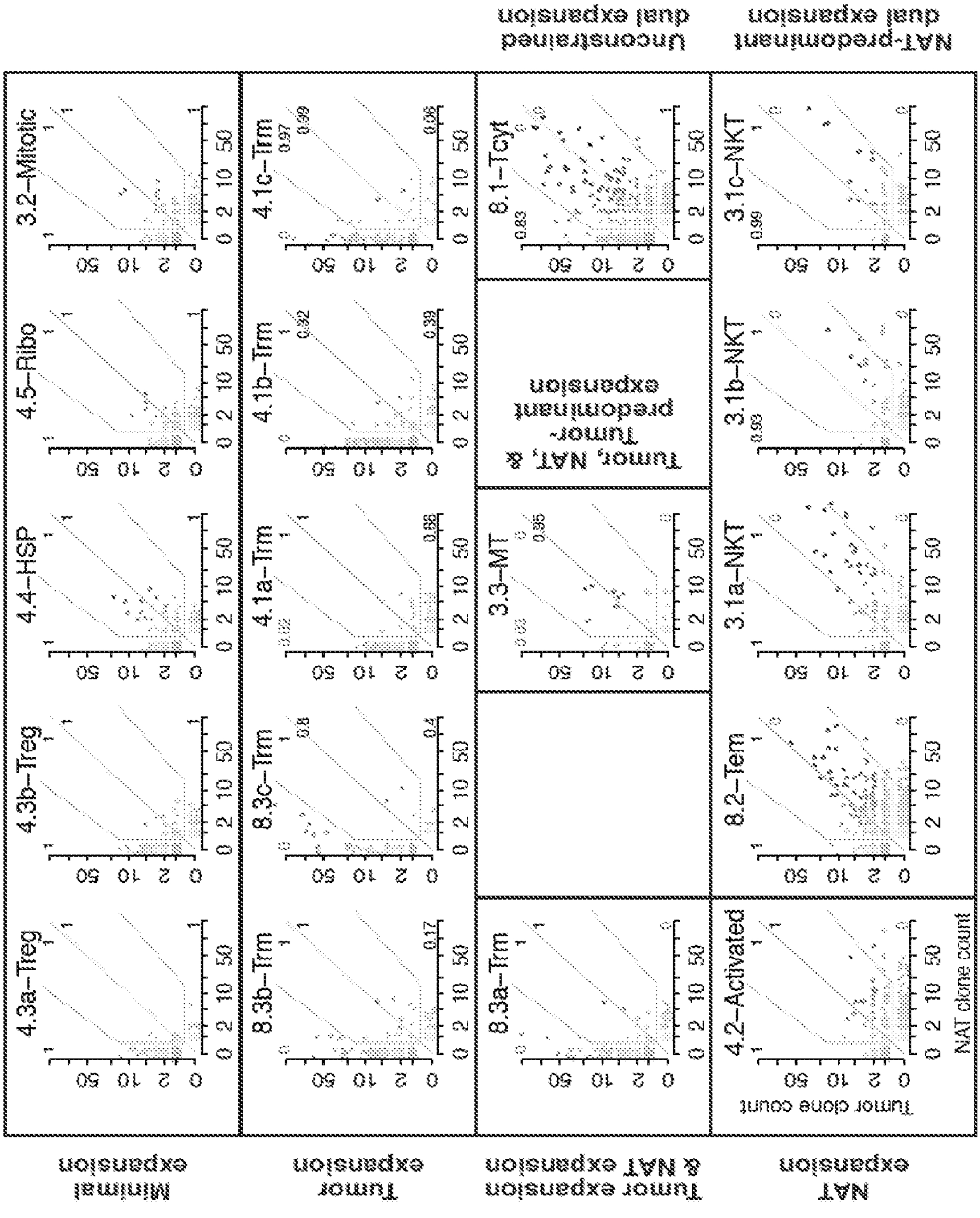
Minimal expansion
Tumor expansion
Tumor expansion & NAT expansion
NAT expansion
Unconstrained dual expansion
NAT-predominant dual expansion
Tumor, NAT, & Tumor-predominant expansion
4.3a–Treg
4.3b–Treg
4.4–HSP
4.5–Ribo
3.2–Mitotic
8.3b–Trm
8.3c–Trm
4.1a–Trm
4.1b–Trm
4.1c–Trm
8.3a–Trm
3.3–MT
8.1–Tcyt
4.2–Activated
8.2–Tem
3.1a–NKT
3.1b–NKT
3.1c–NKT
Tumor clone count
NAT clone count

FIG. 3A

FIG. 3B

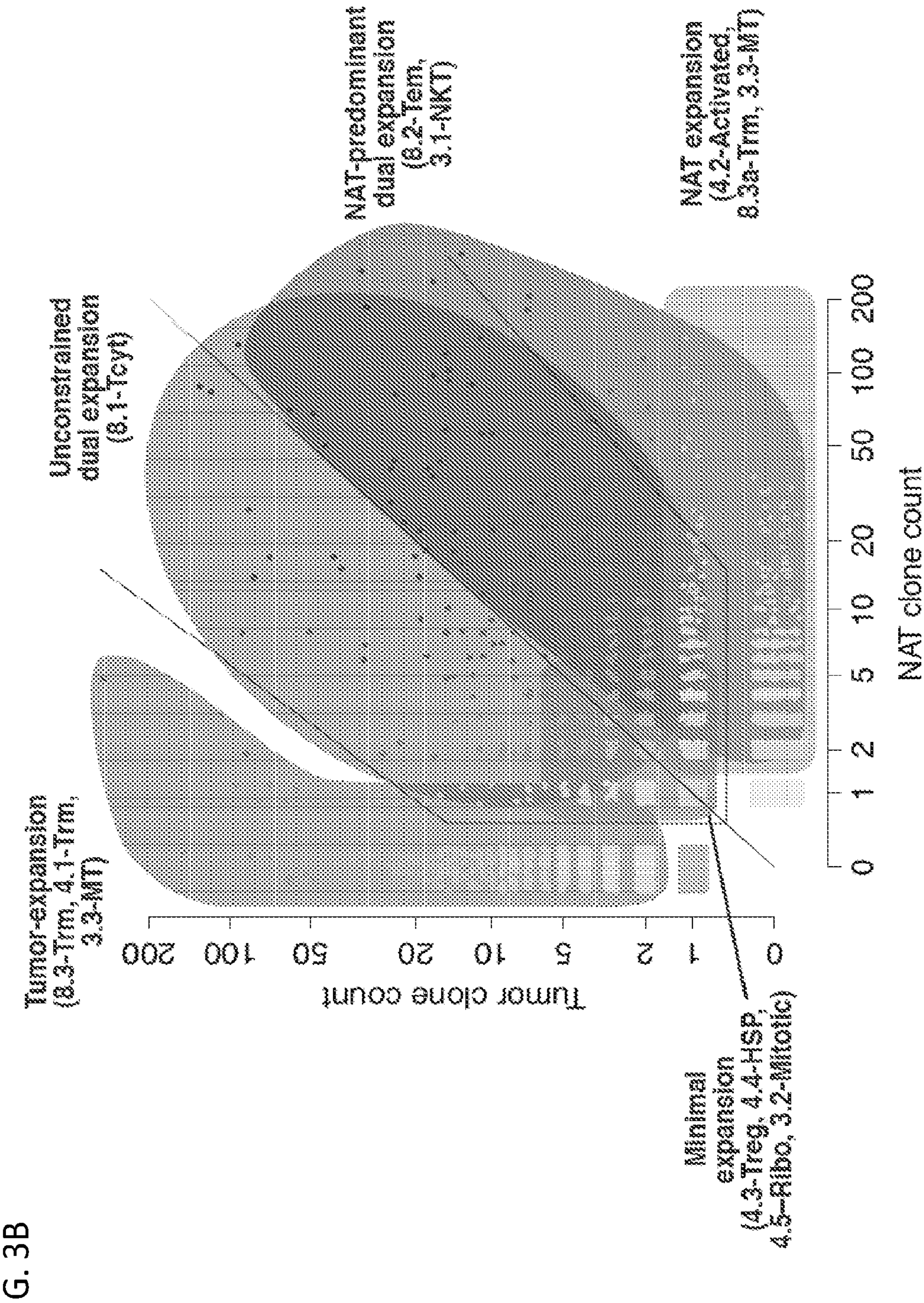
Tumor-expansion (8.3-Trm, 4.1-Trm, 3.3-MT)
Unconstrained dual expansion (8.1-Tcyt)
NAT-predominant dual expansion (8.2-Tem, 3.1-NKT)
NAT expansion (4.2-Activated, 8.3a-Trm, 3.3-MT)
Minimal expansion (4.3-Treg, 4.4-HSP, 4.5--Ribo, 3.2-Mitotic)
Tumor clone count
200
100
50
20
10
5
2
1
0
NAT clone count
0
1
2
5
10
20
50
100
200

FIG. 4B

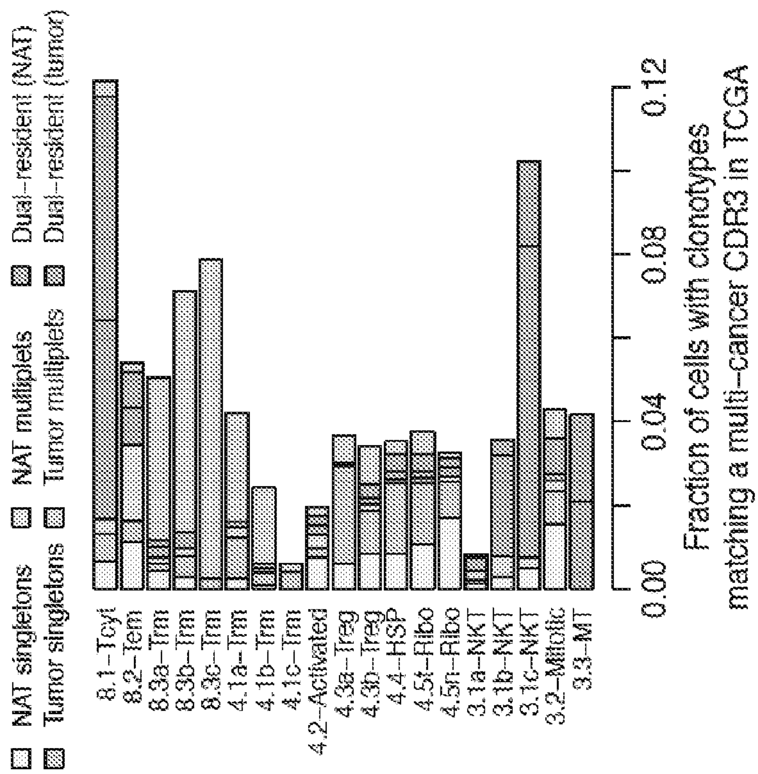
NAT singletons
NAT multiplets
Dual-resident (NAT)
Tumor singletons
Tumor multiplets
Dual-resident (tumor)
8.1–Tcyt
8.2–Tem
8.3a–Trm
8.3b–Trm
8.3c–Trm
4.1a–Trm
4.1b–Trm
4.1c–Trm
4.2–Activated
4.3a–Treg
4.3b–Treg
4.4–HSP
4.5t–Ribo
4.5n–Ribo
3.1a–NKT
3.1b–NKT
3.1c–NKT
3.2–Mitotic
3.3–MT
0.00
0.04
0.08
0.12
Fraction of cells with clonotypes
matching a multi-cancer CDR3 in TCGA

FIG. 4A

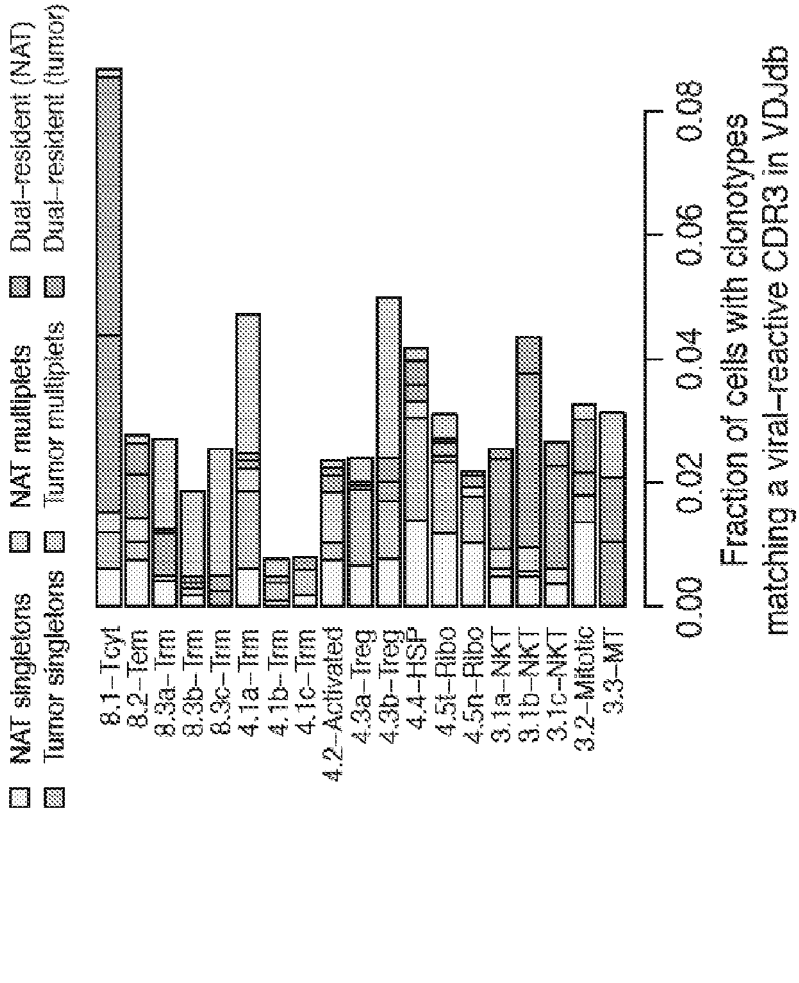
NAT singletons
NAT multiplets
Dual-resident (NAT)
Tumor singletons
Tumor multiplets
Dual-resident (tumor)
8.1–Tcyt
8.2–Tem
8.3a–Trm
8.3b–Trm
8.3c–Trm
4.1a–Trm
4.1b–Trm
4.1c–Trm
4.2–Activated
4.3a–Treg
4.3b–Treg
4.4–HSP
4.5t–Ribo
4.5n–Ribo
3.1a–NKT
3.1b–NKT
3.1c–NKT
3.2–Mitotic
3.3–MT
0.00
0.02
0.04
0.06
0.08
Fraction of cells with clonotypes
matching a viral-reactive CDR3 in VDJdb

FIG. 4C

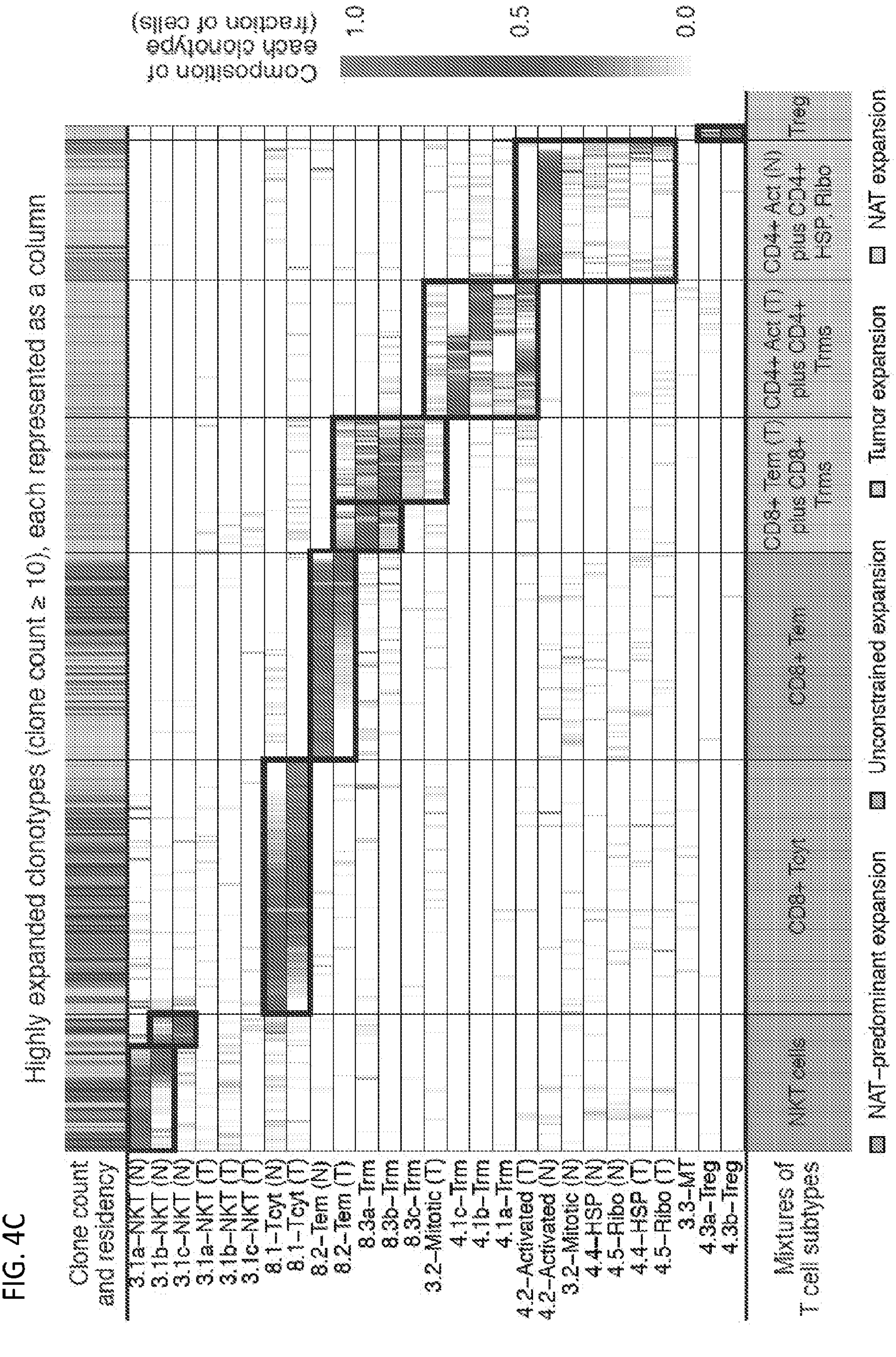
Highly expanded clonotypes (clone count ≥ 10), each represented as a column
Composition of each clonotype (fraction of cells)
1.0
0.5
0.0
Clone count and residency
3.1a–NKT (N)
3.1b–NKT (N)
3.1c–NKT (N)
3.1a–NKT (T)
3.1b–NKT (T)
3.1c–NKT (T)
8.1–Tcyt (N)
8.1–Tcyt (T)
8.2–Tem (N)
8.2–Tem (T)
8.3a–Trm
8.3b–Trm
8.3c–Trm
3.2–Mitotic (T)
4.1c–Trm
4.1b–Trm
4.1a–Trm
4.2–Activated (T)
4.2–Activated (N)
3.2–Mitotic (N)
4.4–HSP (N)
4.5–Ribo (N)
4.4–HSP (T)
4.5–Ribo (T)
3.3–MT
4.3a–Treg
4.3b–Treg
Mixtures of T cell subtypes
NKT cells
CD8+ Tcyt
CD8+ Tem
CD8+ Tem (T) plus CD8+ Trms
CD4+ Act (T) plus CD4+ Trms
CD4+ Act (N) plus CD4+ HSP, Ribo
Treg
NAT–predominant expansion
Unconstrained expansion
Tumor expansion
NAT expansion

FIG. 4D

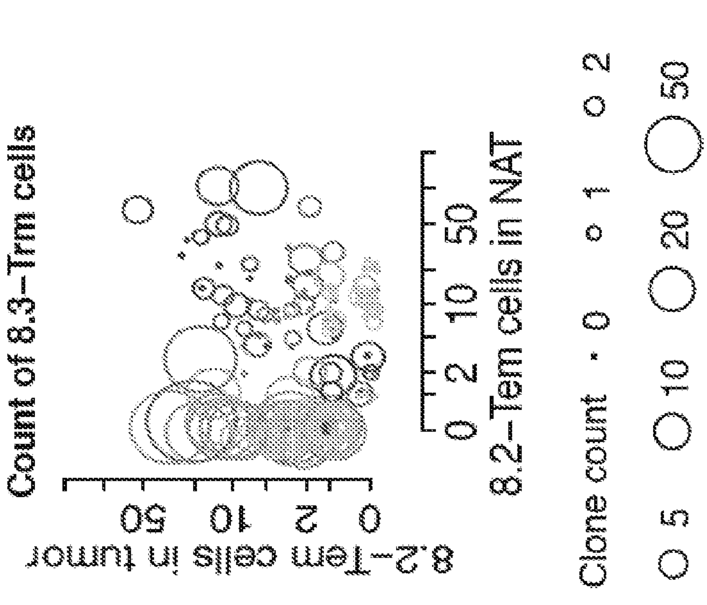


FIG. 4E

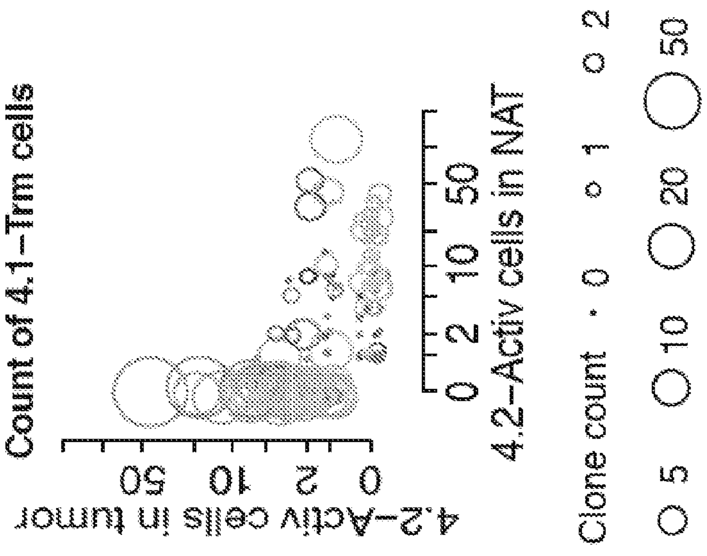


FIG. 4F

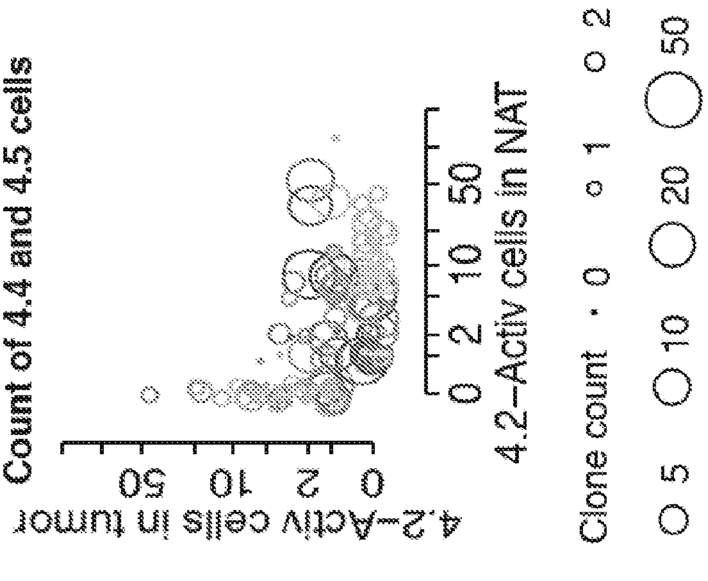

FIG. 4G

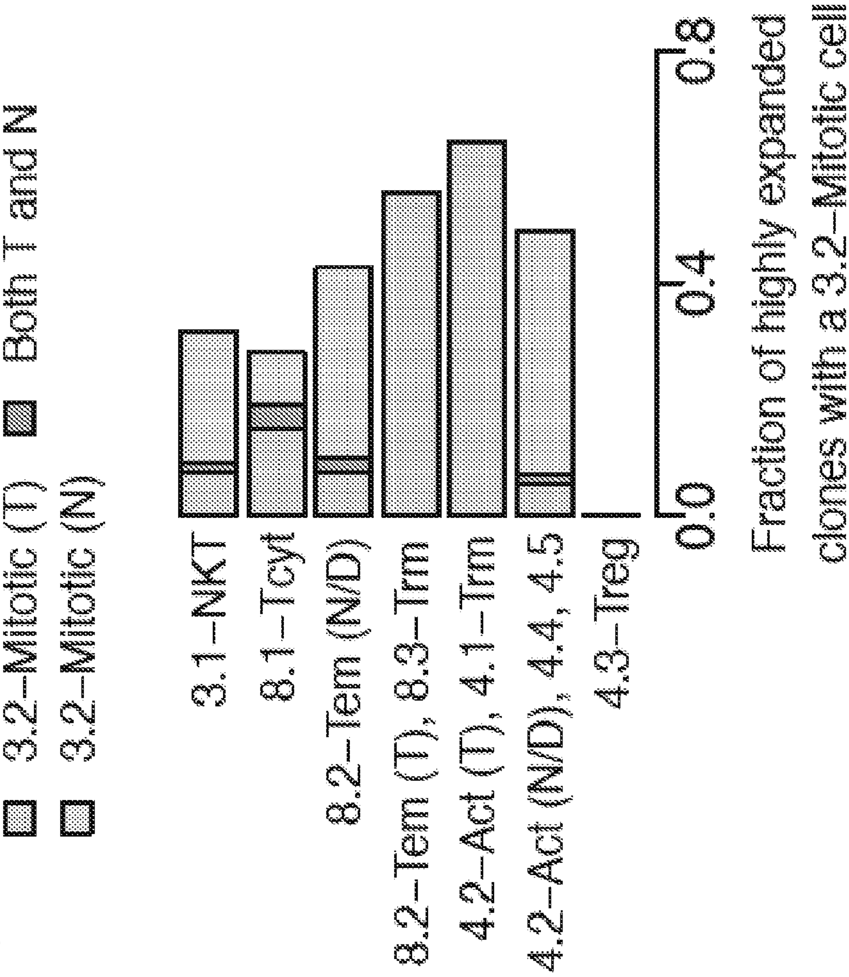
3.2–Mitotic (T)
Both T and N
3.2–Mitotic (N)
3.1–NKT
8.1–Tcyt
8.2–Tem (N/D)
8.2–Tem (T), 8.3–Trm
4.2–Act (T), 4.1–Trm
4.2–Act (N/D), 4.4, 4.5
4.3–Treg
0.0
0.4
0.8
Fraction of highly expanded
clones with a 3.2–Mitotic cell

FIG. 5A

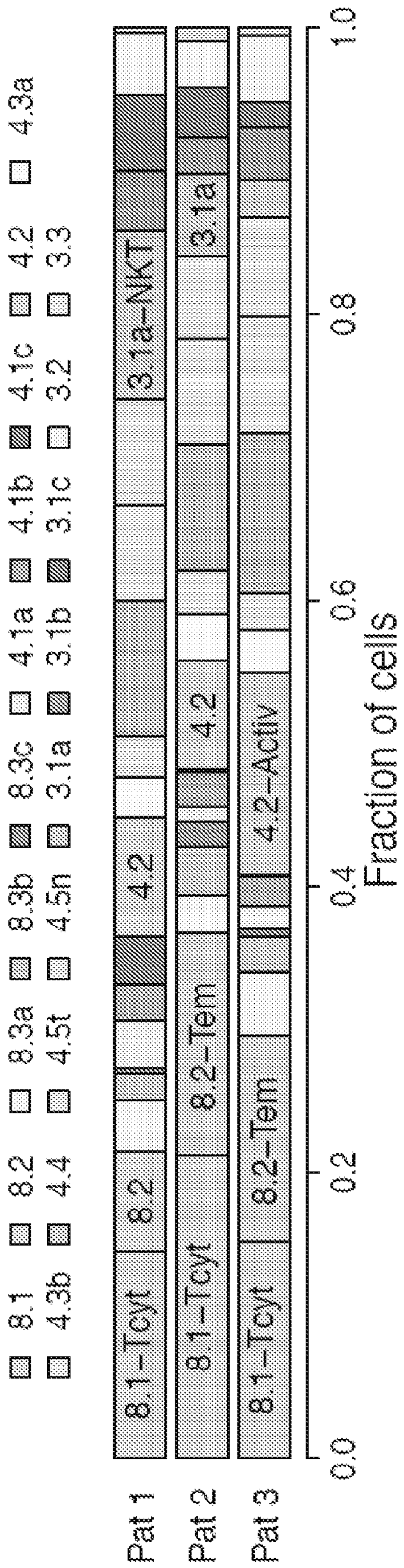
8.1
8.2
8.3a
8.3b
8.3c
4.1a
4.1b
4.1c
4.2
4.3a
4.3b
4.4
4.5t
4.5n
3.1a
3.1b
3.1c
3.2
3.3
Pat 1
Pat 2
Pat 3
8.1–Tcyt
8.2
4.2
3.1a–NKT
8.1–Tcyt
8.2–Tem
4.2
3.1a
8.1–Tcyt
8.2–Tem
4.2–Activ
0.0
0.2
0.4
0.6
0.8
1.0
Fraction of cells

FIG. 5B

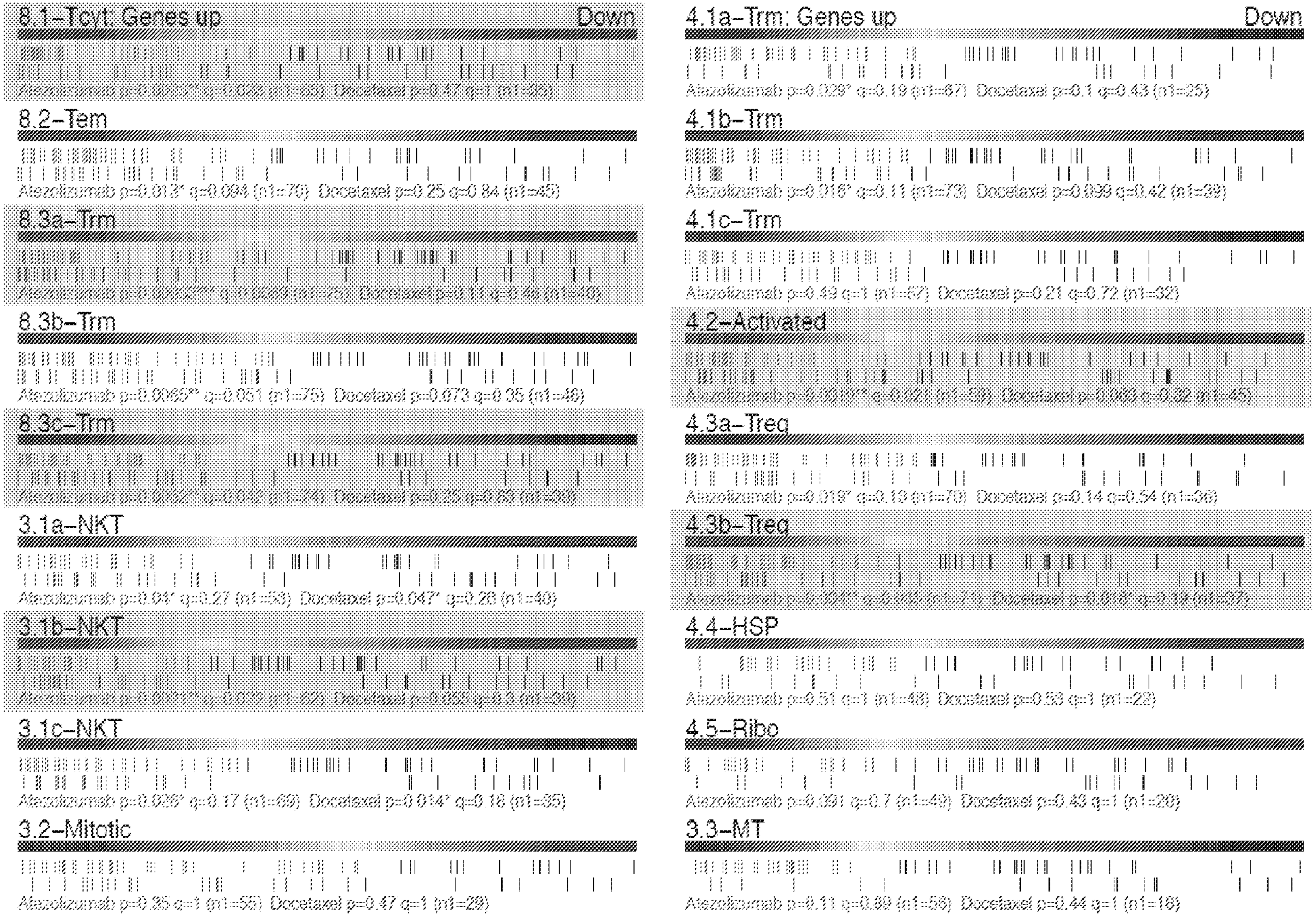
8.1–Tcyt: Genes up
Down
8.2–Tem
Atezolizumab p=0.013* q=0.094 (n1=76) Docetaxel p=0.25 q=0.84 (n1=45)
8.3a–Trm
8.3b–Trm
Atezolizumab p=0.0065** q=0.051 (n1=75) Docetaxel p=0.073 q=0.35 (n1=46)
8.3c–Trm
3.1a–NKT
Atezolizumab p=0.01* q=0.27 (n1=53) Docetaxel p=0.047* q=0.28 (n1=40)
3.1b–NKT
3.1c–NKT
Atezolizumab p=0.026* q=0.17 (n1=69) Docetaxel p=0.014* q=0.18 (n1=35)
3.2–Mitotic
Atezolizumab p=0.35 q=1 (n1=55) Docetaxel p=0.47 q=1 (n1=29)
4.1a–Trm: Genes up
Down
Atezolizumab p=0.029* q=0.19 (n1=67) Docetaxel p=0.1 q=0.43 (n1=25)
4.1b–Trm
Atezolizumab p=0.016* q=0.11 (n1=73) Docetaxel p=0.099 q=0.42 (n1=29)
4.1c–Trm
Atezolizumab p=0.49 q=1 (n1=67) Docetaxel p=0.21 q=0.72 (n1=32)
4.2–Activated
4.3a–Treg
Atezolizumab p=0.019* q=0.13 (n1=70) Docetaxel p=0.14 q=0.54 (n1=36)
4.3b–Treg
4.4–HSP
Atezolizumab p=0.51 q=1 (n1=48) Docetaxel p=0.53 q=1 (n1=22)
4.5–Ribo
Atezolizumab p=0.091 q=0.7 (n1=49) Docetaxel p=0.43 q=1 (n1=20)
3.3–MT
Atezolizumab p=0.11 q=0.89 (n1=56) Docetaxel p=0.44 q=1 (n1=18)

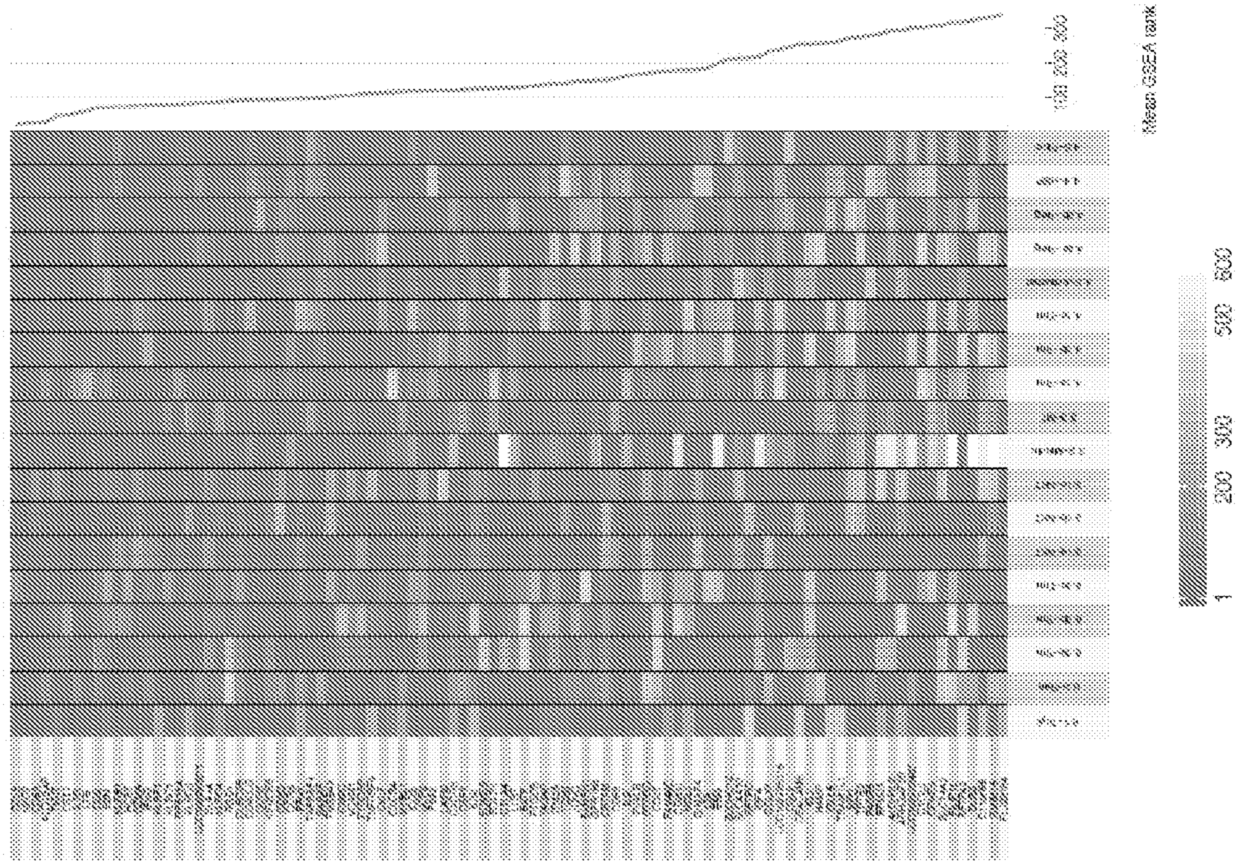

FIG. 5C

FIG. 5E

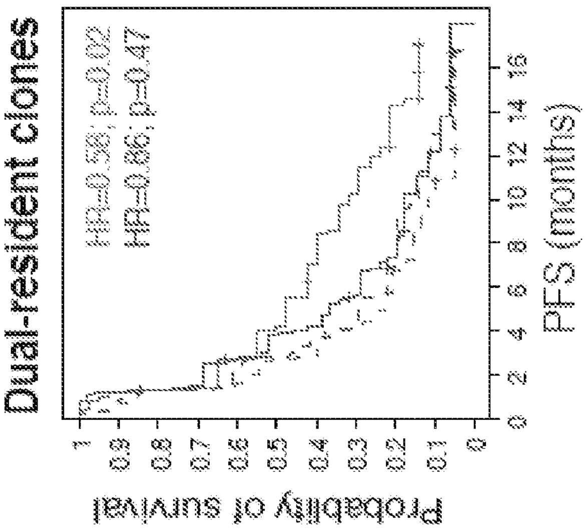
Dual-resident clones
HR=0.58; p=0.02
HR=0.86; p=0.47
Probability of survival
PFS (months)

FIG. 5D

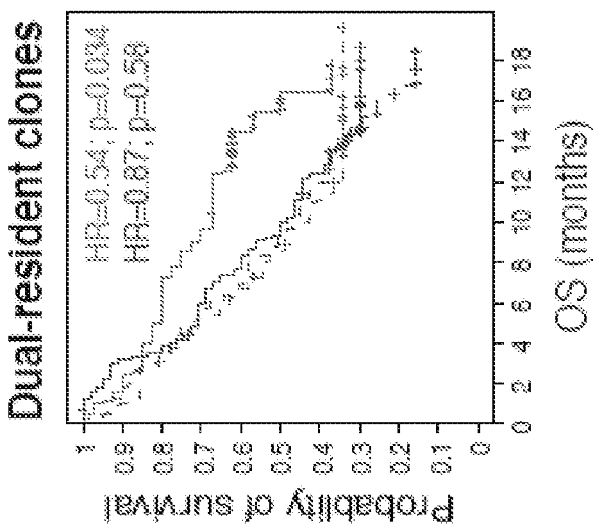
Dual-resident clones
HR=0.54; p=0.034
HR=0.87; p=0.58
Probability of survival
OS (months)

FIG. 5F

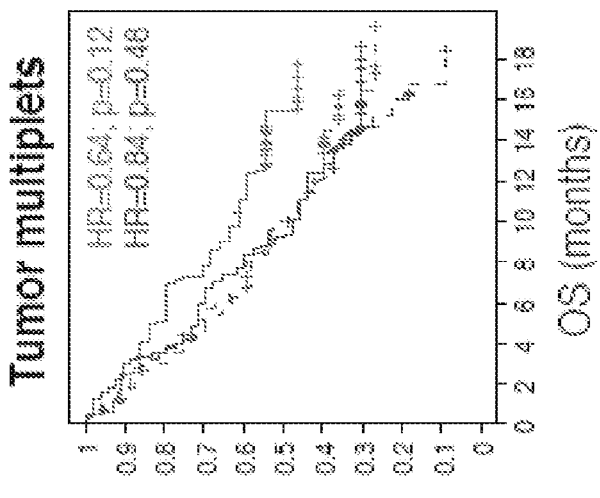
Tumor multiplets
HR=0.64; p=0.12
HR=0.84; p=0.40
1
0.9
0.8
0.7
0.6
0.5
0.4
0.3
0.2
0.1
0
0 2 4 6 8 10 12 14 16 18
OS (months)

FIG. 5G

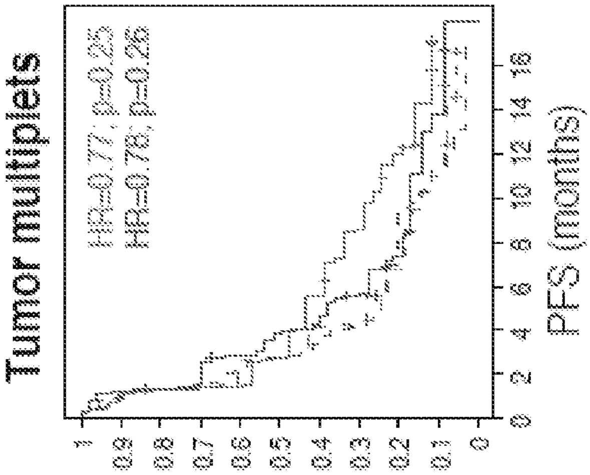
Tumor multiplets
HR=0.77; p=0.25
HR=0.78; p=0.26
1
0.9
0.8
0.7
0.6
0.5
0.4
0.3
0.2
0.1
0
0 2 4 6 8 10 12 14 16
PFS (months)

FIG. 5H

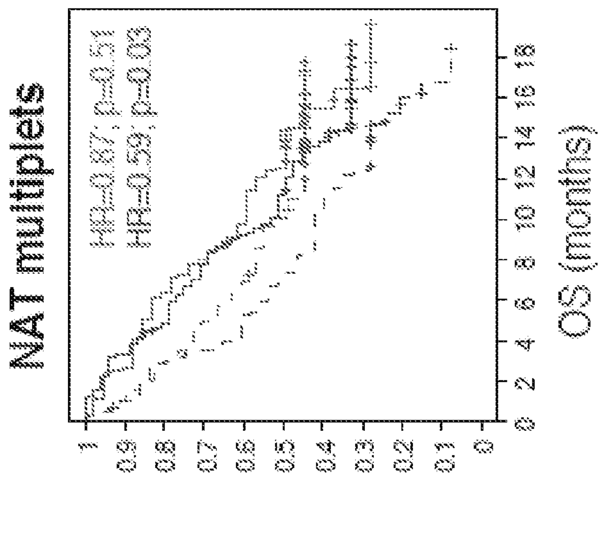
NAT multiplets
HR=0.87; p=0.51
HR=0.59; p=0.03
OS (months)

FIG. 5I

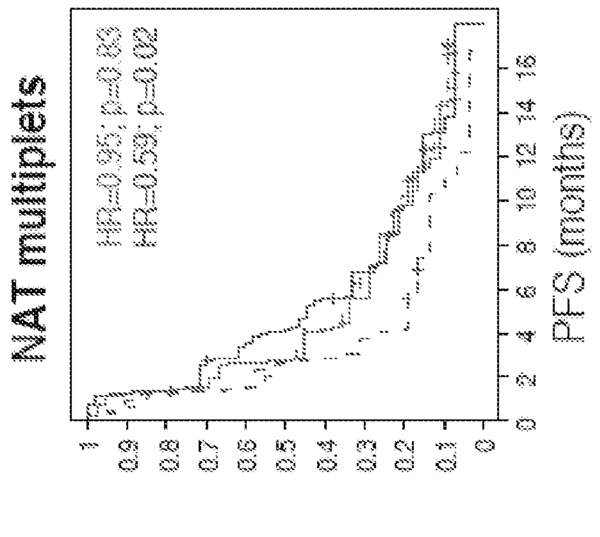
NAT multiplets
HR=0.95; p=0.83
HR=0.59; p=0.02
PFS (months)

FIG. 5J

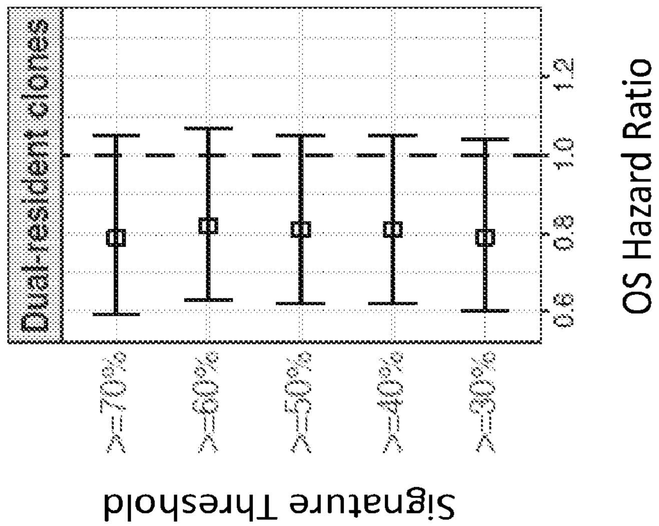
Dual-resident clones
>=70%
>=60%
>=50%
>=40%
>=30%
Signature Threshold
0.6
0.8
1.0
1.2
OS Hazard Ratio

FIG. 5K

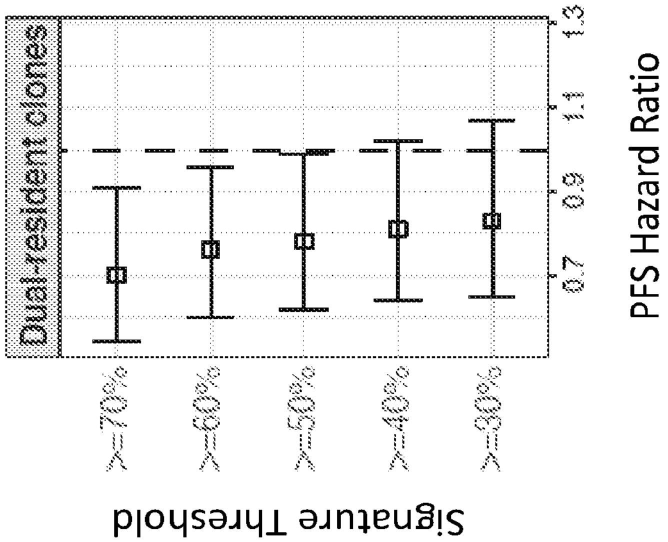
Dual-resident clones
>=70%
>=60%
>=50%
>=40%
>=30%
Signature Threshold
0.7
0.9
1.1
1.3
PFS Hazard Ratio

FIG. 5L

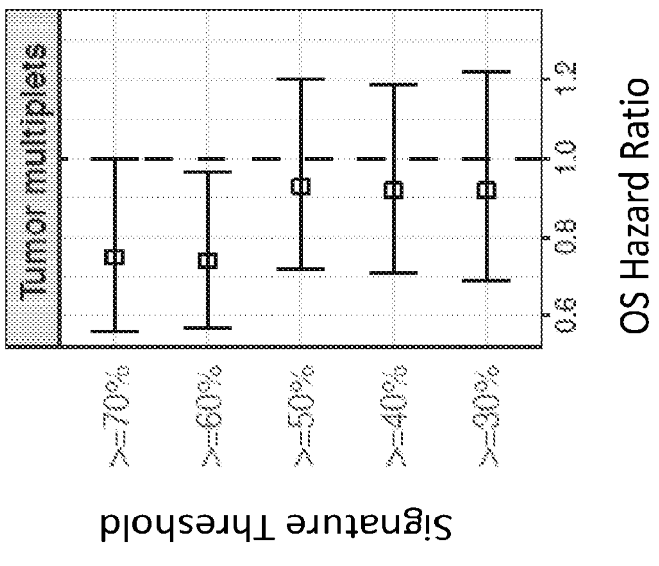
Tumor multiplets
Signature Threshold
>=70%
>=60%
>=50%
>=40%
>=30%
0.6
0.8
1.0
1.2
OS Hazard Ratio

FIG. 5M

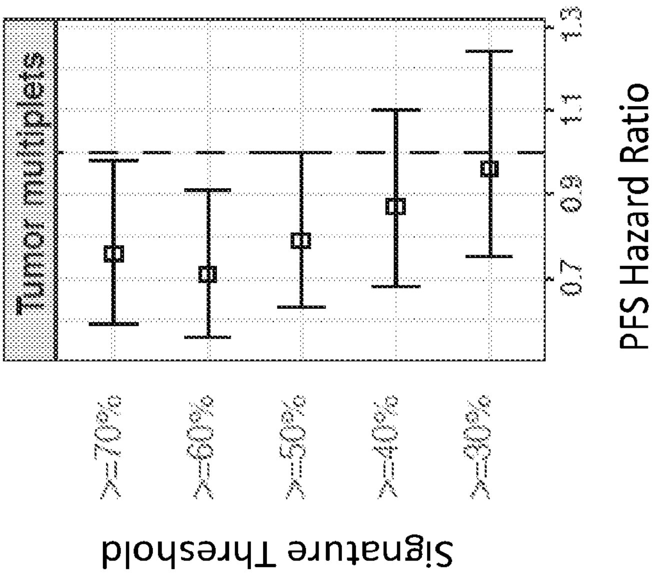
Tumor multiplets
Signature Threshold
>=70%
>=60%
>=50%
>=40%
>=30%
0.7
0.9
1.1
1.3
PFS Hazard Ratio

FIG. 5N

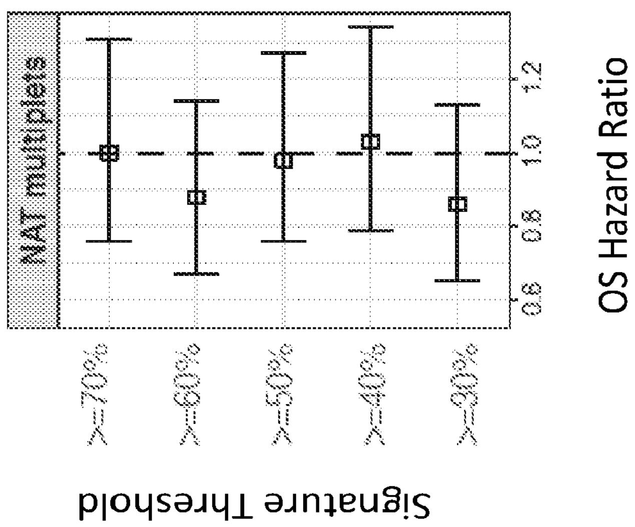
NAT multiplets
>=70%
>=60%
>=50%
>=40%
>=30%
Signature Threshold
0.6
0.8
1.0
1.2
OS Hazard Ratio

FIG. 5O

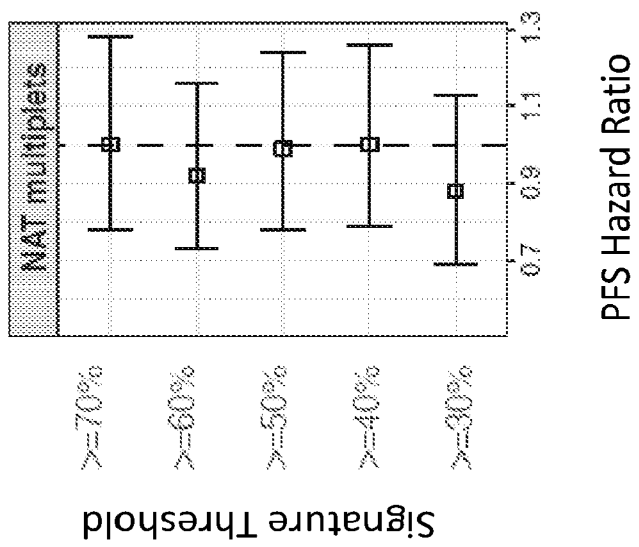
NAT multiplets
>=70%
>=60%
>=50%
>=40%
>=30%
Signature Threshold
0.7
0.9
1.1
1.3
PFS Hazard Ratio

FIG. 6

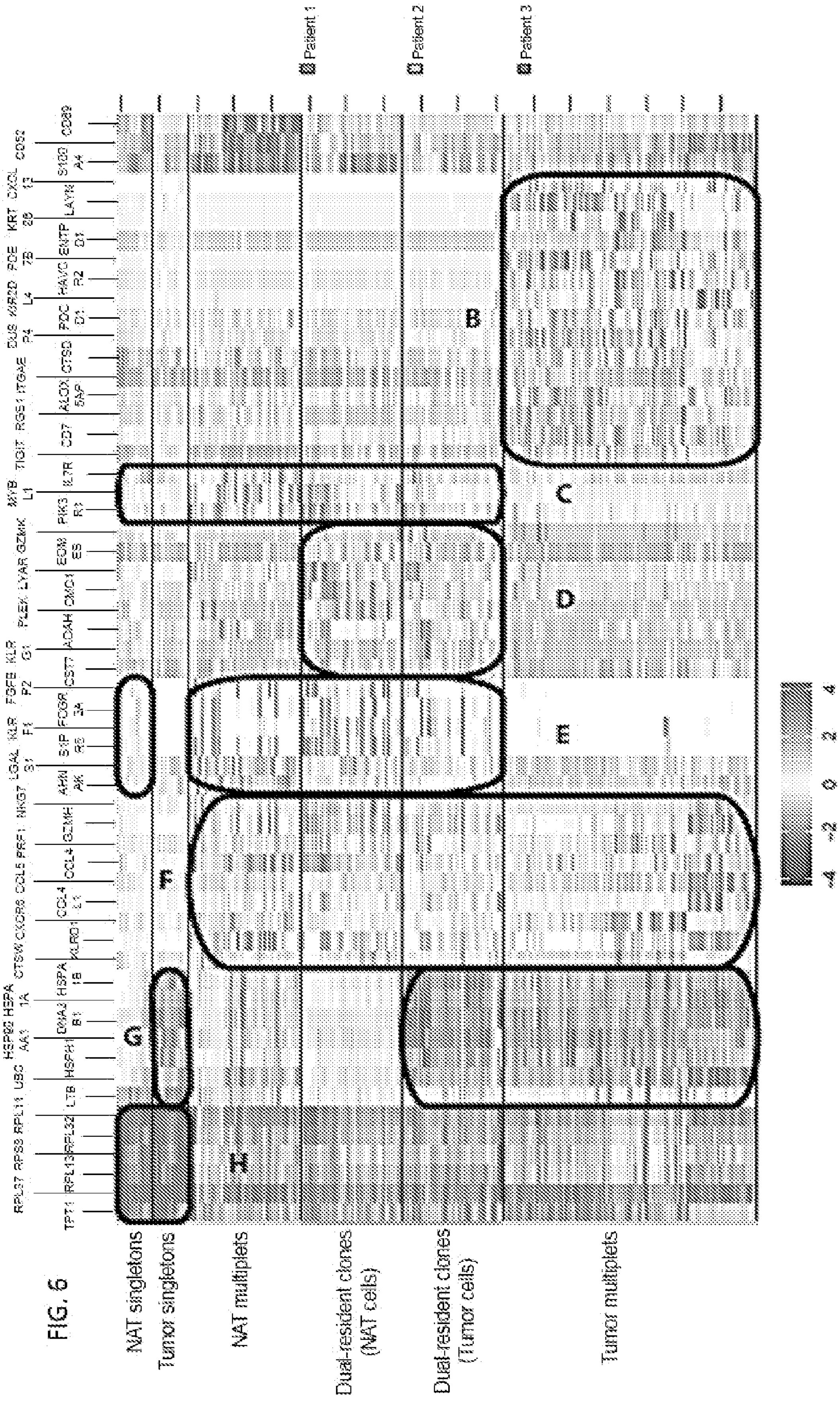

NAT singletons
Tumor singletons
NAT multiplets
Dual-resident clones (NAT cells)
Dual-resident clones (Tumor cells)
Tumor multiplets
TPT1 RPL37 RPL13 RPS8 RPL32 RPL11 LTB UBC HSPH1 HSP90 AA1 DNAJ B1 HSPA 1A HSPA 1B CTSW KLRD1 CXCR6 CCL4 L1 CCL5 CCL4 PRF1 GZMH NKG7 AHN AK LGAL S1 S1P R5 KLR F1 FCGR 3A FGFB P2 CST7 KLR G1 ADAM PLEK CMC1 LYAR EOM ES GZMK PIK3 R1 MYB L1 IL7R TIGIT CD7 RGS1 ALOX 5AP ITGAE CTSD DUS P4 PDC D1 KIR2D L4 HAVC R2 PDE 7B ENTP D1 KRT 86 LAYN CXCL 13 S100 A4 CD52 CD69
B
C
D
E
F
G
H
Patient 1
Patient 2
Patient 3
-4 -2 0 2 4

FIG. 7

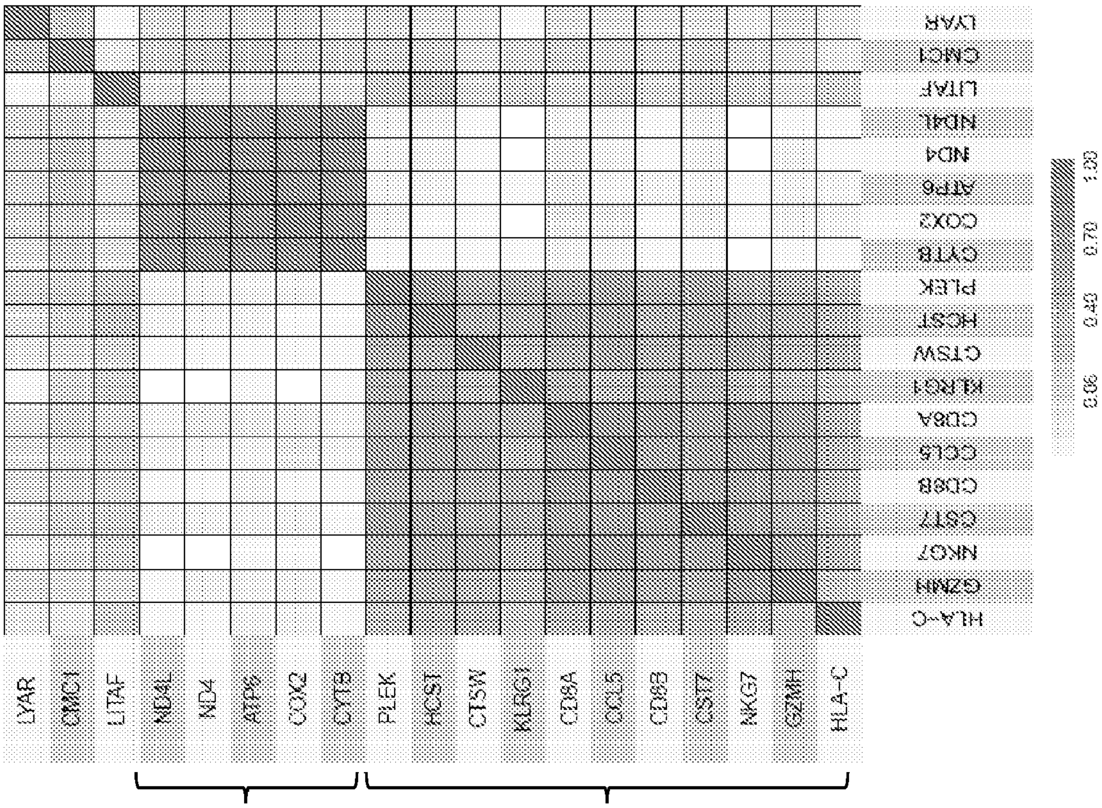
LYAR
CMC1
LITAF
ND4L
ND4
ATP6
COX2
CYTB
PLEK
HCST
CTSW
KLRG1
CD8A
CCL5
CD8B
CST7
NKG7
GZMH
HLA-C
0.06
0.40
0.70
1.00
Mitochondrial Genes
Cytotoxic Genes

FIG. 8B

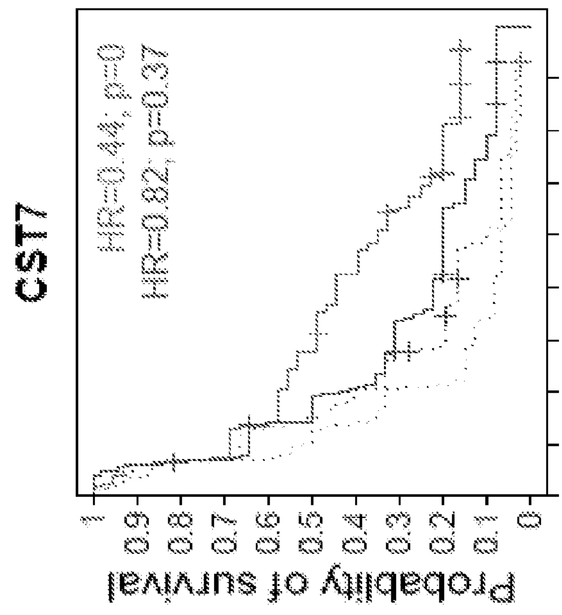
CST7
HR=0.44; p=0
HR=0.82; p=0.37
Probability of survival
0 0.1 0.2 0.3 0.4 0.5 0.6 0.7 0.8 0.9 1
0 2 4 6 8 10 14
PFS (months)

FIG. 8A

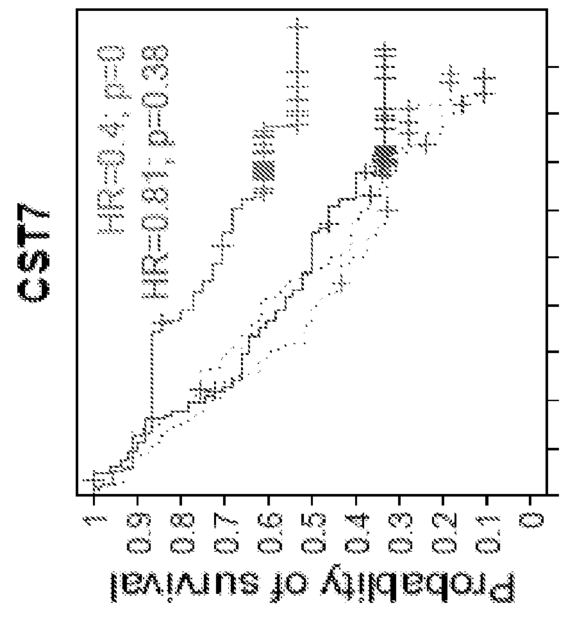
CST7
HR=0.4; p=0
HR=0.81; p=0.38
Probability of survival
0 0.1 0.2 0.3 0.4 0.5 0.6 0.7 0.8 0.9 1
0 2 4 6 8 12 16
OS (months)

FIG. 8C

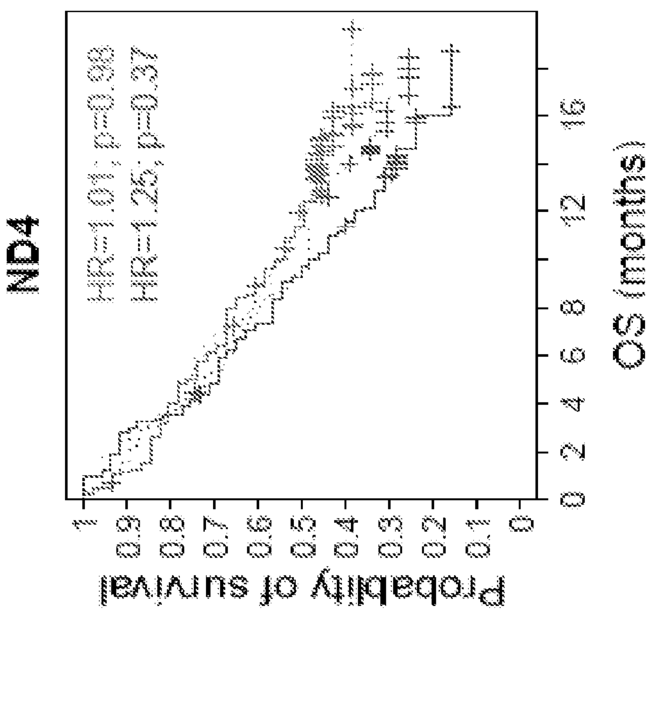
ND4
HR=1.01; p=0.98
HR=1.25; p=0.37
Probability of survival
OS (months)
0 2 4 6 8 12 16
1 0.9 0.8 0.7 0.6 0.5 0.4 0.3 0.2 0.1 0

FIG. 8D

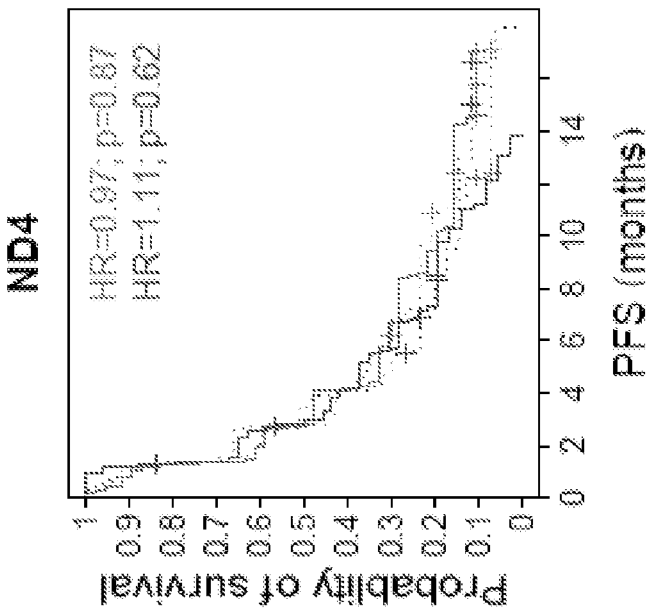
ND4
HR=0.97; p=0.87
HR=1.11; p=0.62
Probability of survival
PFS (months)
0 2 4 6 8 10 14
1 0.9 0.8 0.7 0.6 0.5 0.4 0.3 0.2 0.1 0

FIG. 8E

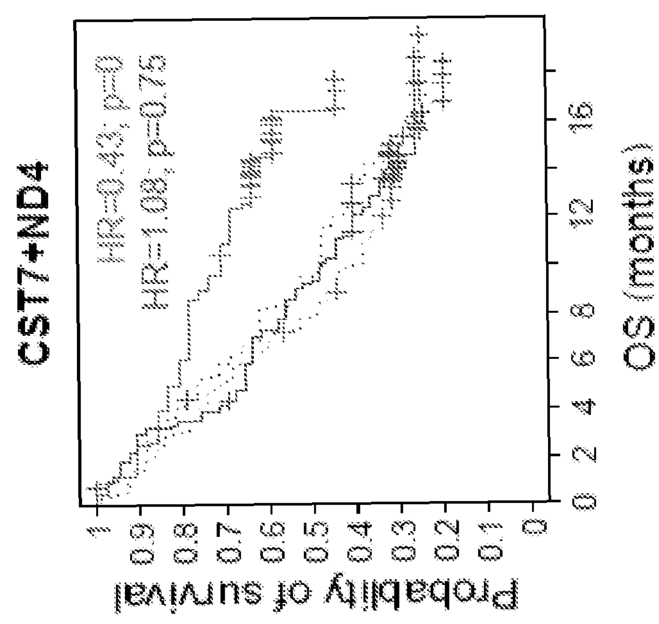
CST7+ND4
HR=0.43; p=0
HR=1.08; p=0.75
Probability of survival
1
0.9
0.8
0.7
0.6
0.5
0.4
0.3
0.2
0.1
0
0
2
4
6
8
12
16
OS (months)

FIG. 8F

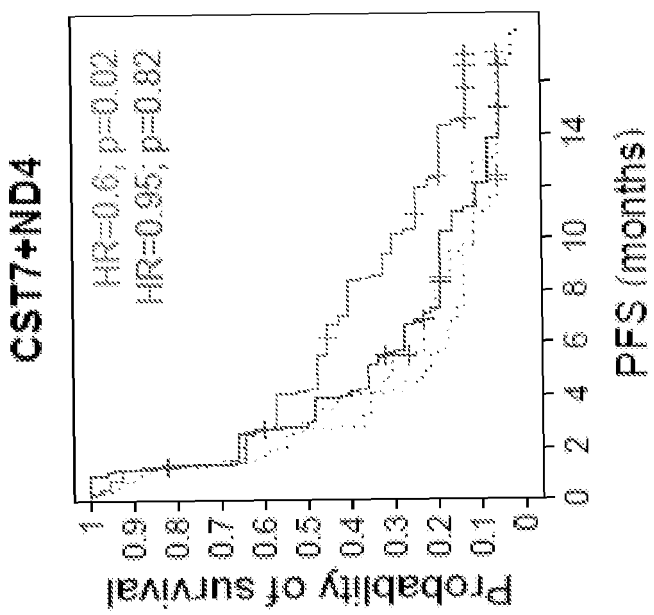
CST7+ND4
HR=0.6; p=0.02
HR=0.95; p=0.82
Probability of survival
1
0.9
0.8
0.7
0.6
0.5
0.4
0.3
0.2
0.1
0
0
2
4
6
8
10
14
PFS (months)

FIG. 8H

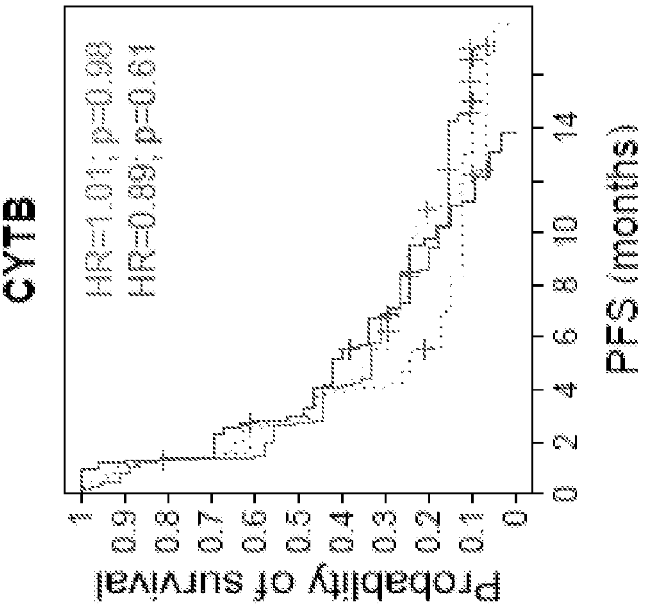
CYTB
HR=1.01; p=0.98
HR=0.89; p=0.61
Probability of survival
PFS (months)

FIG. 8G

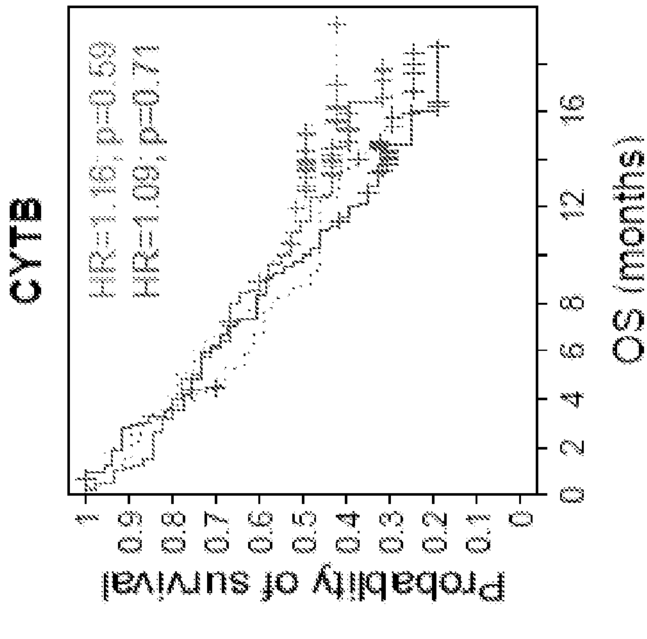
CYTB
HR=1.16; p=0.59
HR=1.09; p=0.71
Probability of survival
OS (months)

FIG. 8I

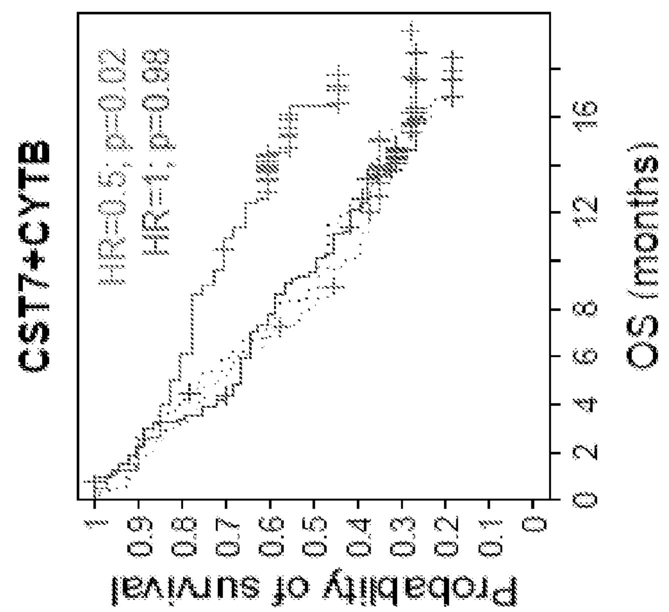
CST7+CYTB
HR=0.5; p=0.02
HR=1; p=0.98
Probability of survival
1
0.9
0.8
0.7
0.6
0.5
0.4
0.3
0.2
0.1
0
0
2
4
6
8
12
16
OS (months)

FIG. 8J

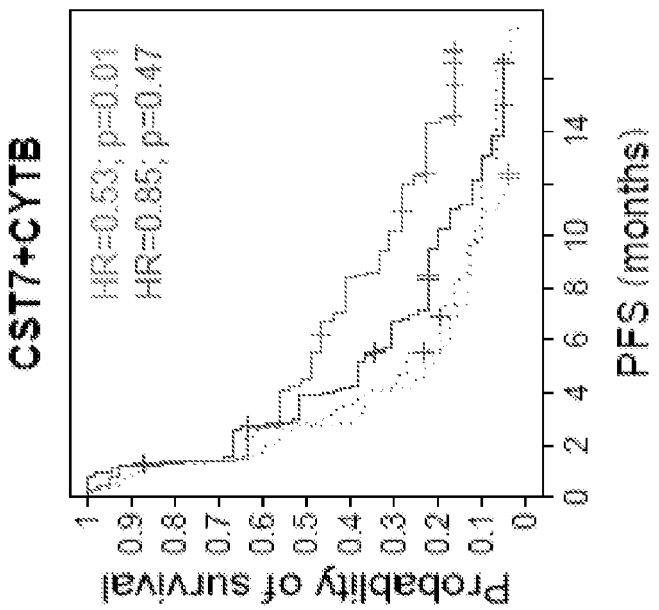
CST7+CYTB
HR=0.53; p=0.01
HR=0.85; p=0.47
Probability of survival
1
0.9
0.8
0.7
0.6
0.5
0.4
0.3
0.2
0.1
0
0
2
4
6
8
10
14
PFS (months)

FIG. 8L

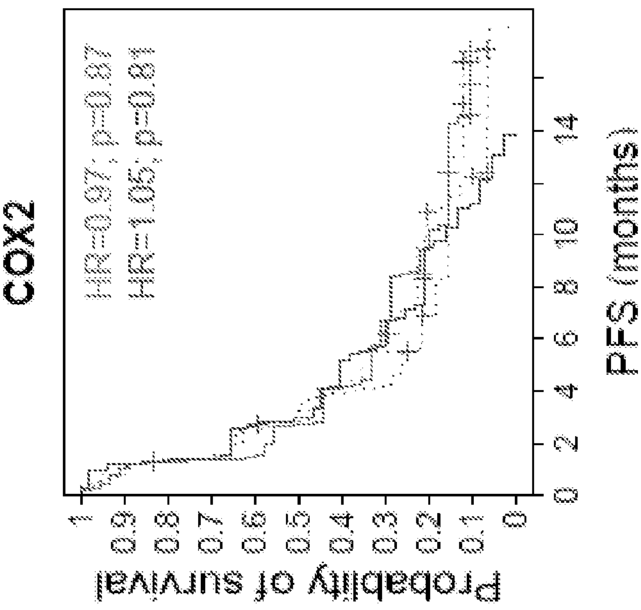
COX2
HR=0.97; p=0.87
HR=1.05; p=0.81
Probability of survival
PFS (months)

FIG. 8K

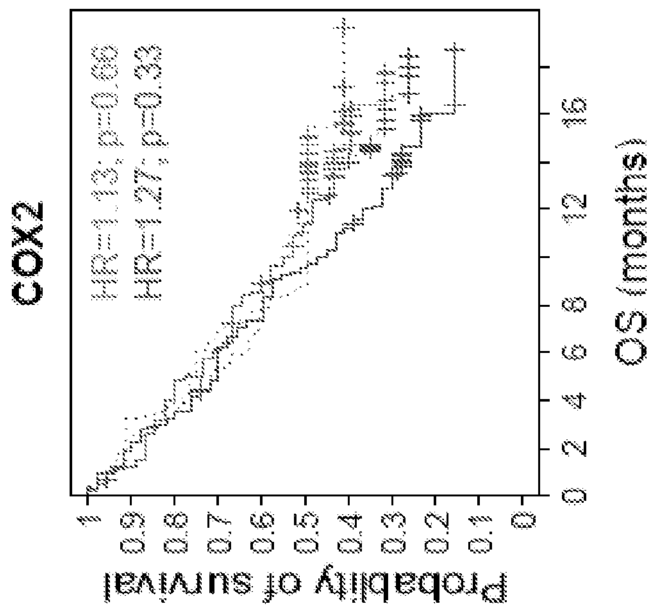
COX2
HR=1.13; p=0.66
HR=1.27; p=0.33
Probability of survival
OS (months)

FIG. 8M

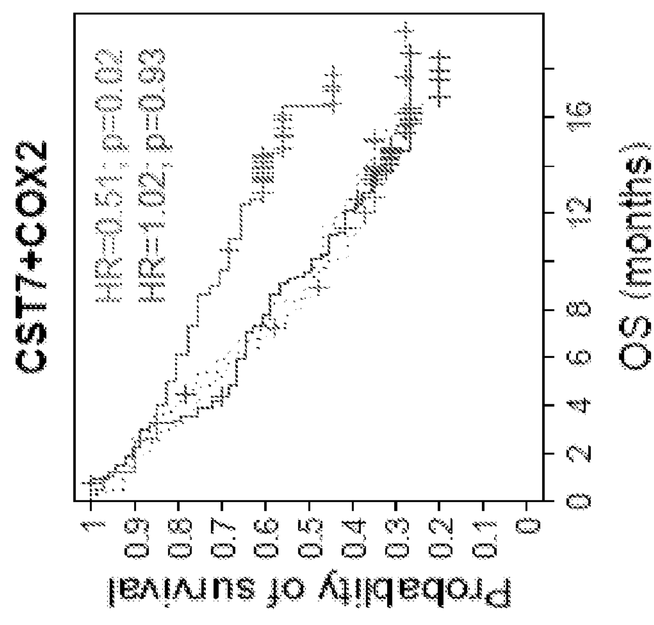
CST7+COX2
HR=0.51; p=0.02
HR=1.02; p=0.93
Probability of survival
1
0.9
0.8
0.7
0.6
0.5
0.4
0.3
0.2
0.1
0
0
2
4
6
8
12
16
OS (months)

FIG. 8N

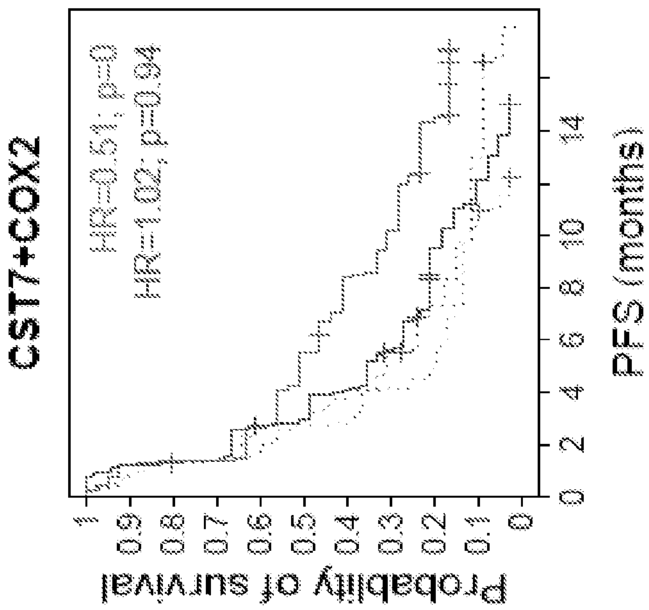
CST7+COX2
HR=0.51; p=0
HR=1.02; p=0.94
Probability of survival
1
0.9
0.8
0.7
0.6
0.5
0.4
0.3
0.2
0.1
0
0
2
4
6
8
10
14
PFS (months)

FIG. 9B

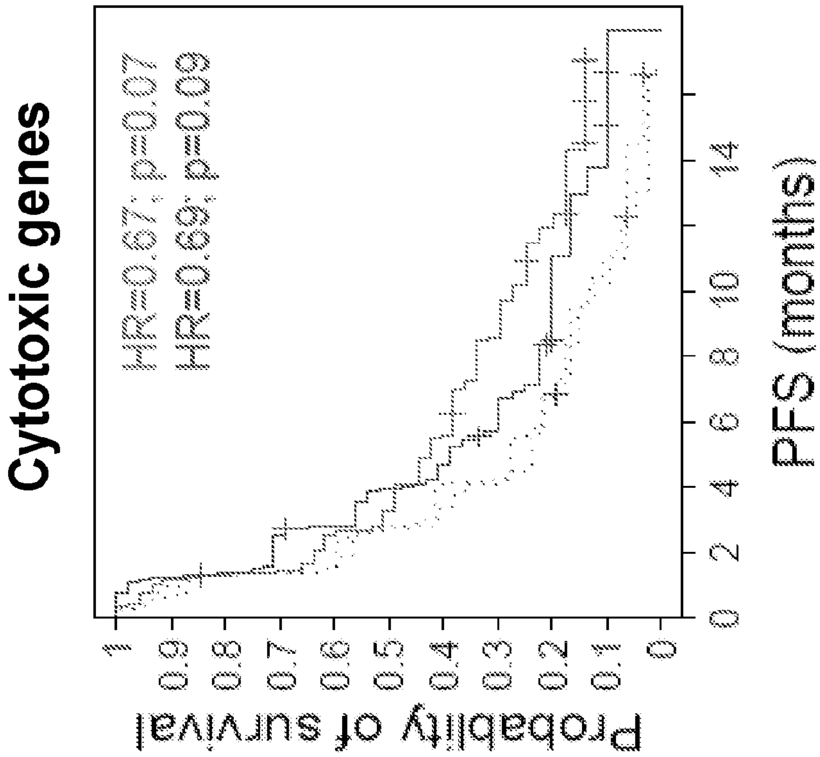
Cytotoxic genes
HR=0.67; p=0.07
HR=0.69; p=0.09
Probability of survival
1
0.9
0.8
0.7
0.6
0.5
0.4
0.3
0.2
0.1
0
0
2
4
6
8
10
14
PFS (months)

FIG. 9A

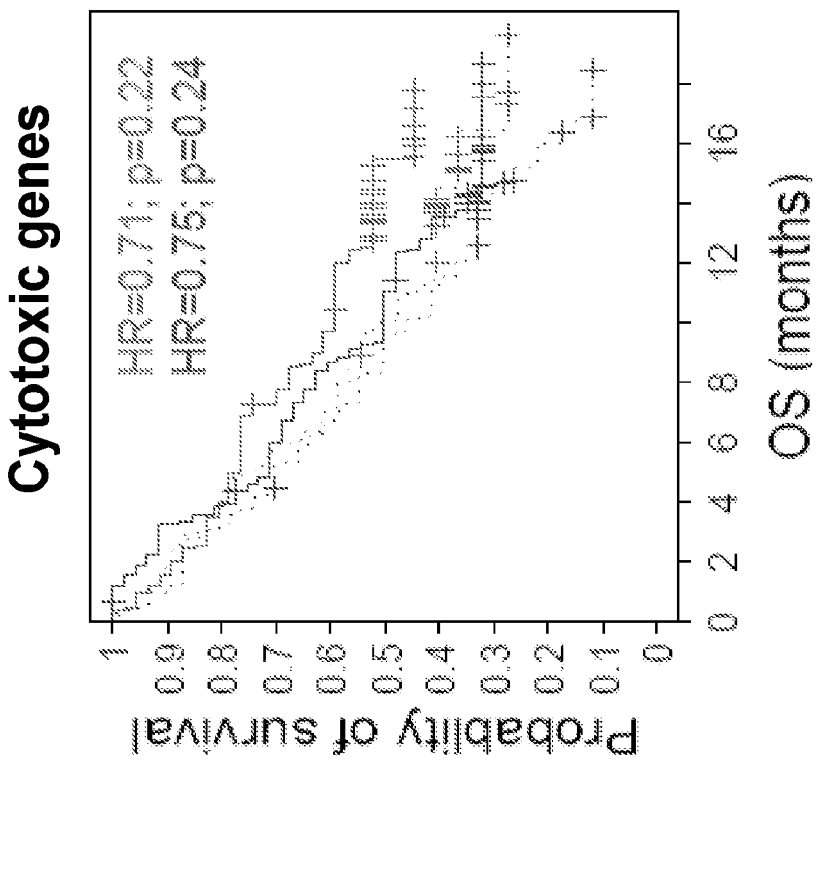
Cytotoxic genes
HR=0.71; p=0.22
HR=0.75; p=0.24
Probability of survival
1
0.9
0.8
0.7
0.6
0.5
0.4
0.3
0.2
0.1
0
0
2
4
6
8
12
16
OS (months)

FIG. 9D

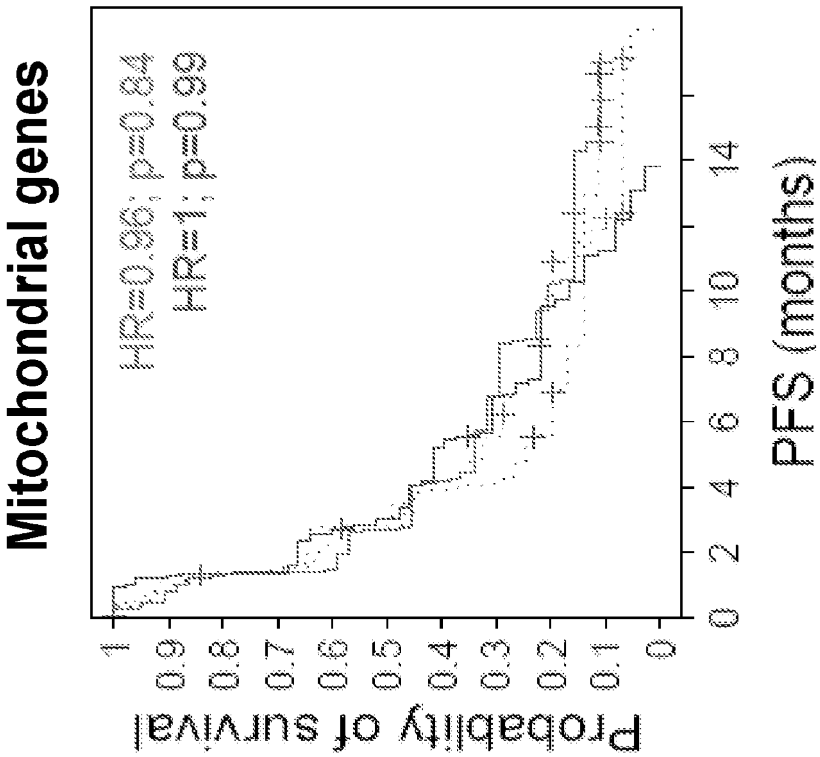
Mitochondrial genes
HR=0.96; p=0.84
HR=1; p=0.99
Probablity of survival
PFS (months)

FIG. 9C

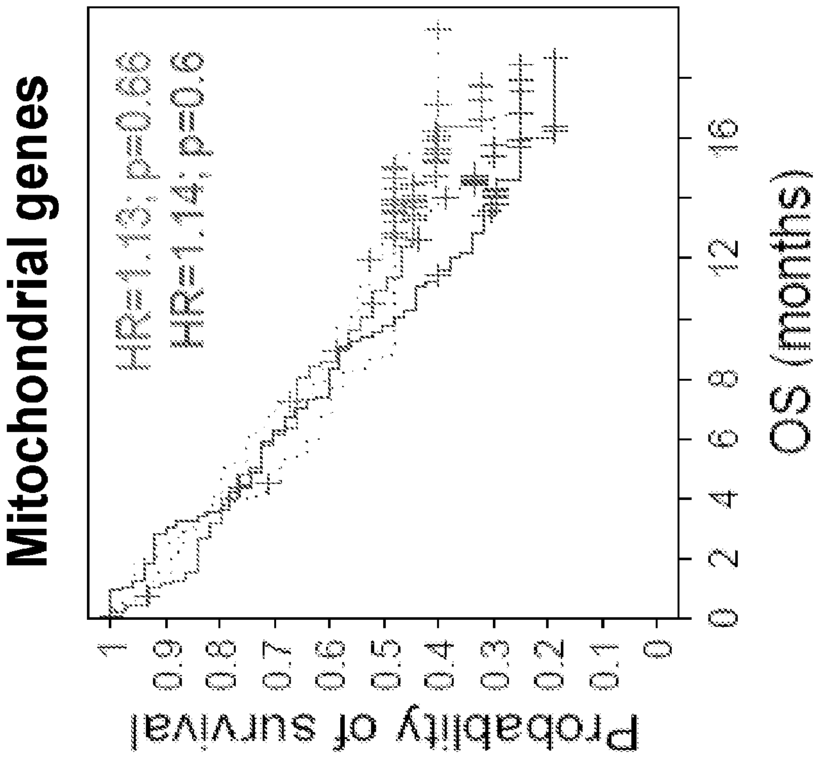
Mitochondrial genes
HR=1.13; p=0.66
HR=1.14; p=0.6
Probablity of survival
OS (months)

FIG. 9F

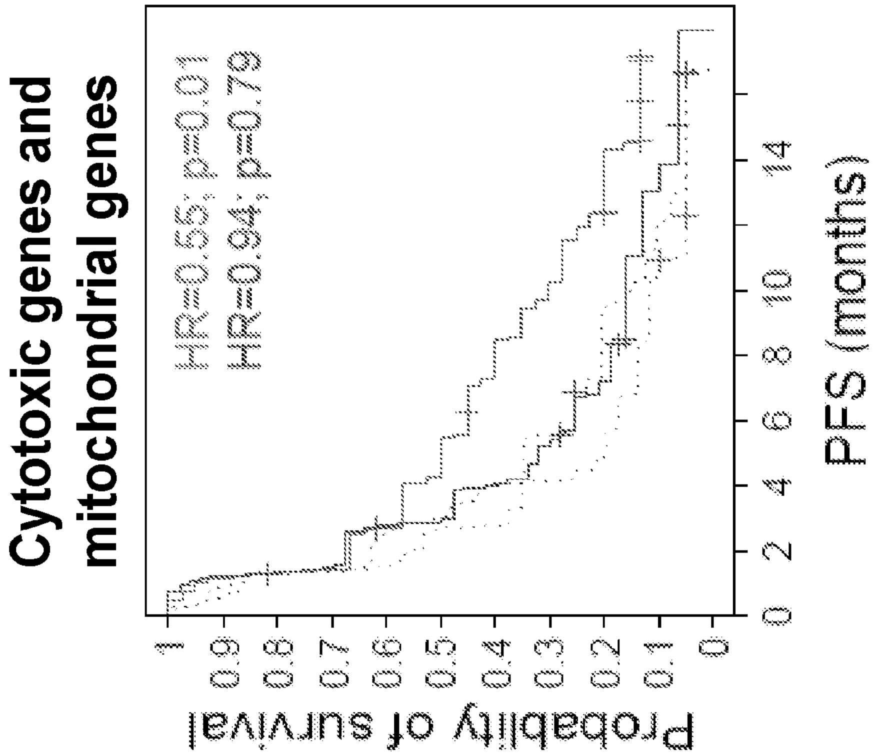
Cytotoxic genes and mitochondrial genes
HR=0.55; p=0.01
HR=0.94; p=0.79
Probability of survival
1
0.9
0.8
0.7
0.6
0.5
0.4
0.3
0.2
0.1
0
0
2
4
6
8
10
14
PFS (months)

FIG. 9E

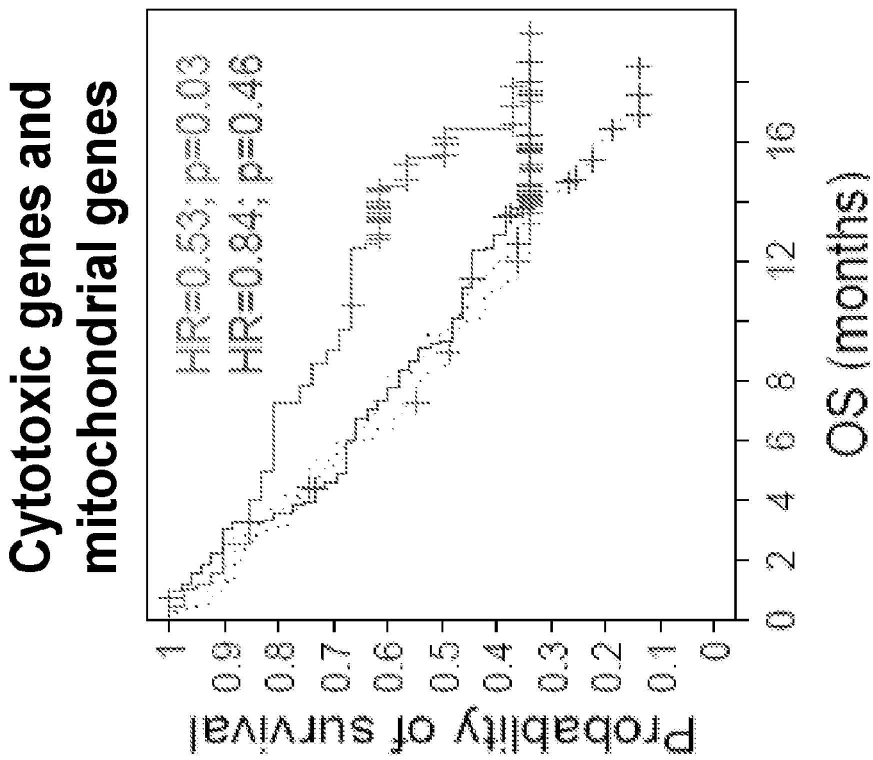
Cytotoxic genes and mitochondrial genes
HR=0.53; p=0.03
HR=0.84; p=0.46
Probability of survival
1
0.9
0.8
0.7
0.6
0.5
0.4
0.3
0.2
0.1
0
0
2
4
6
8
12
16
OS (months)

FIG. 10A

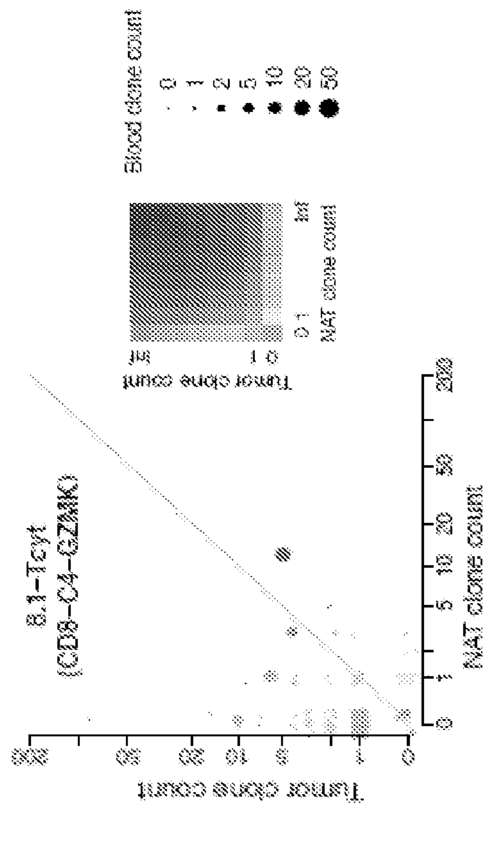


FIG. 10B

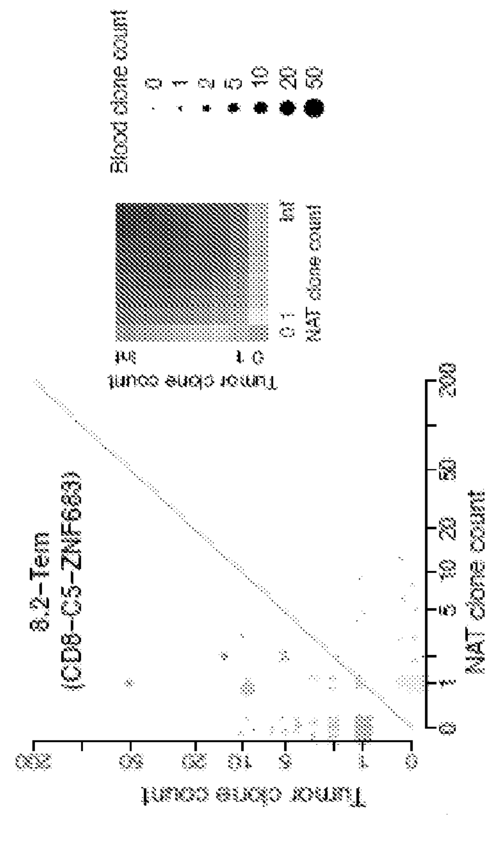

FIG. 10C

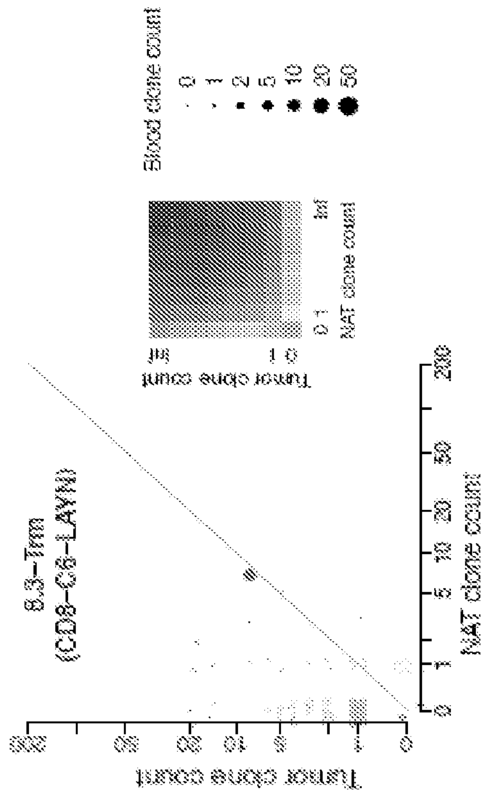


FIG. 10D

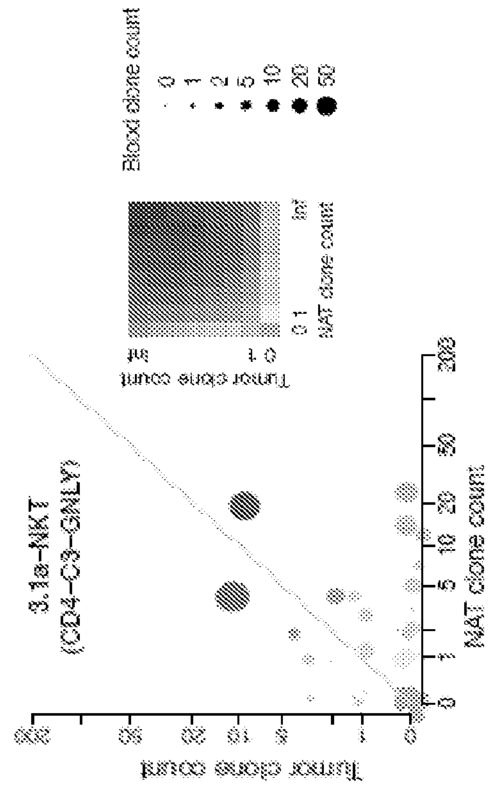

FIG. 10E

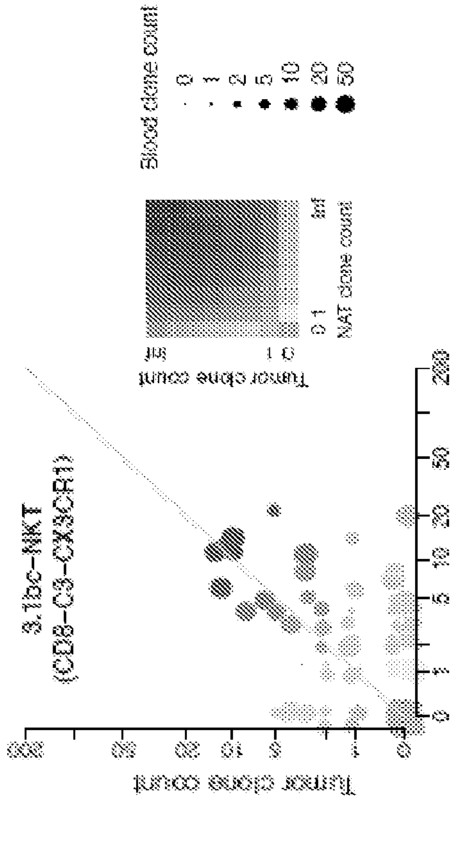


FIG. 10F

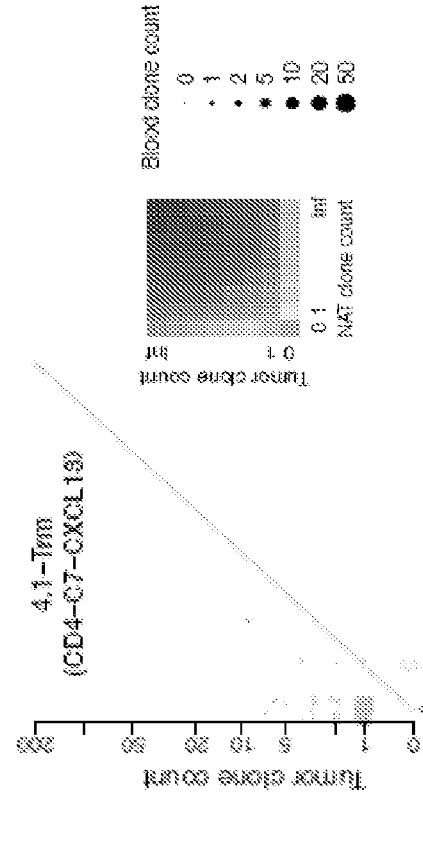

FIG. 10G

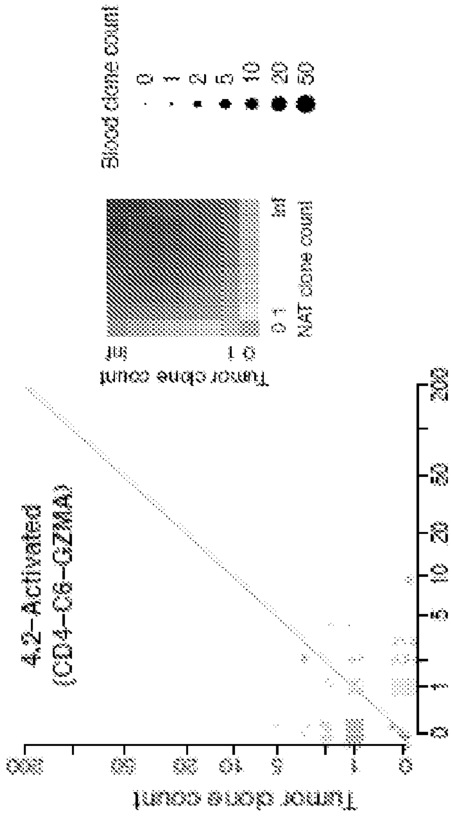


FIG. 10H

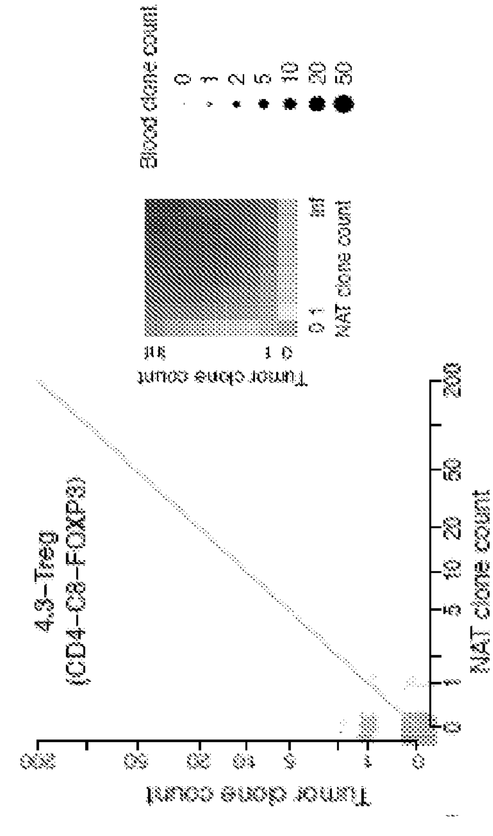

FIG. 10I

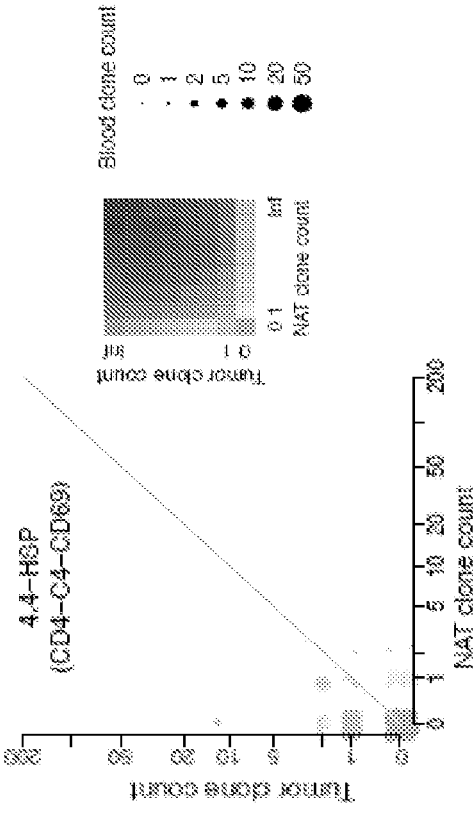


FIG. 10J

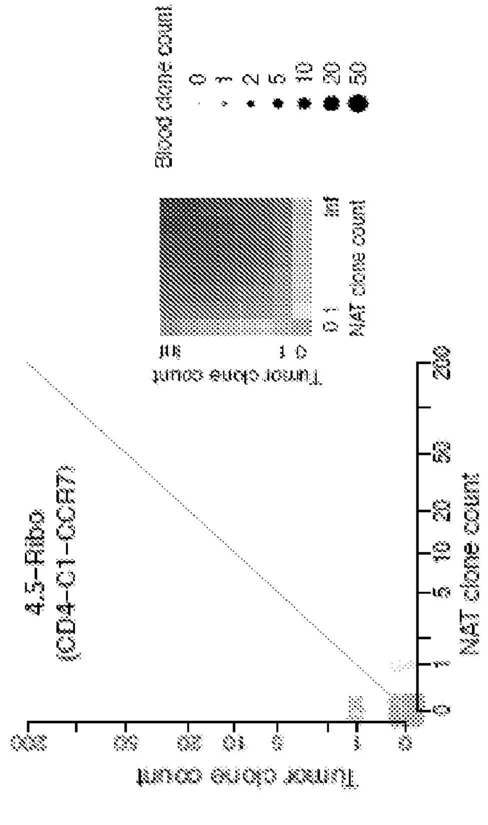

FIG. 11A

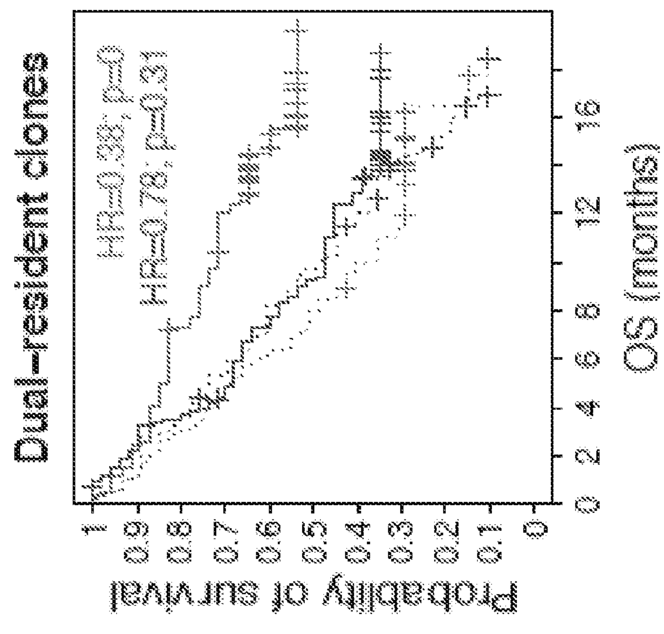


FIG. 11B

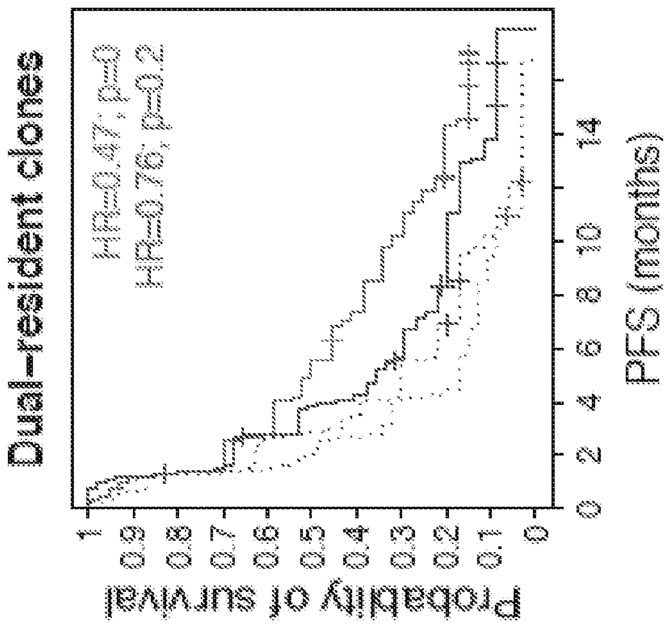

FIG. 11C

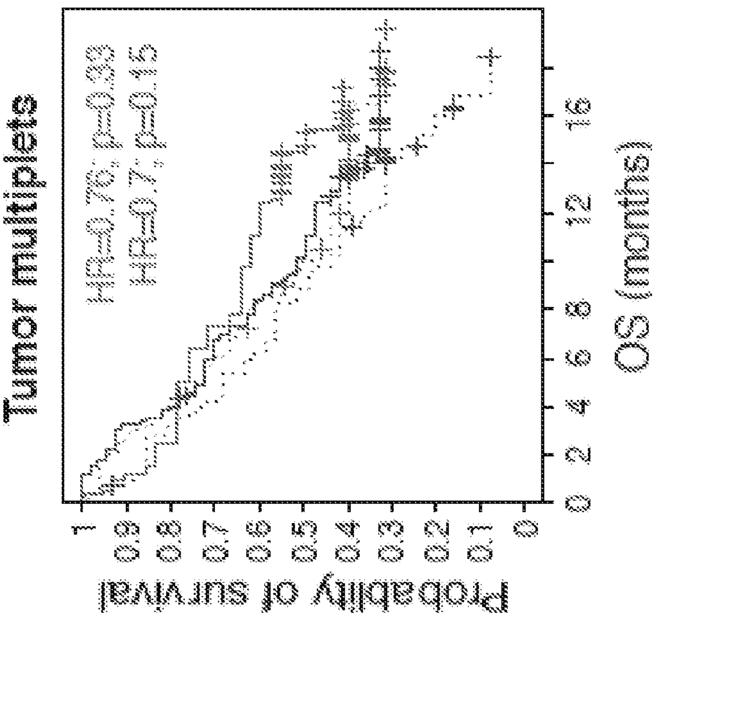


FIG. 11D

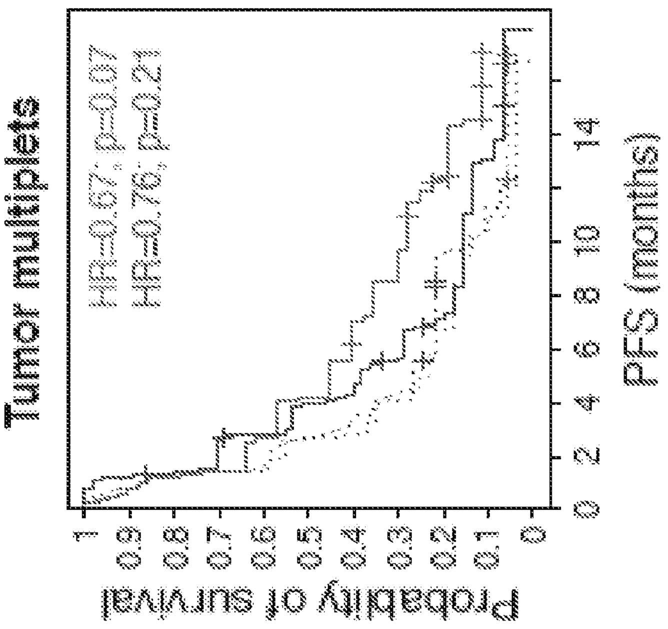

FIG. 11E

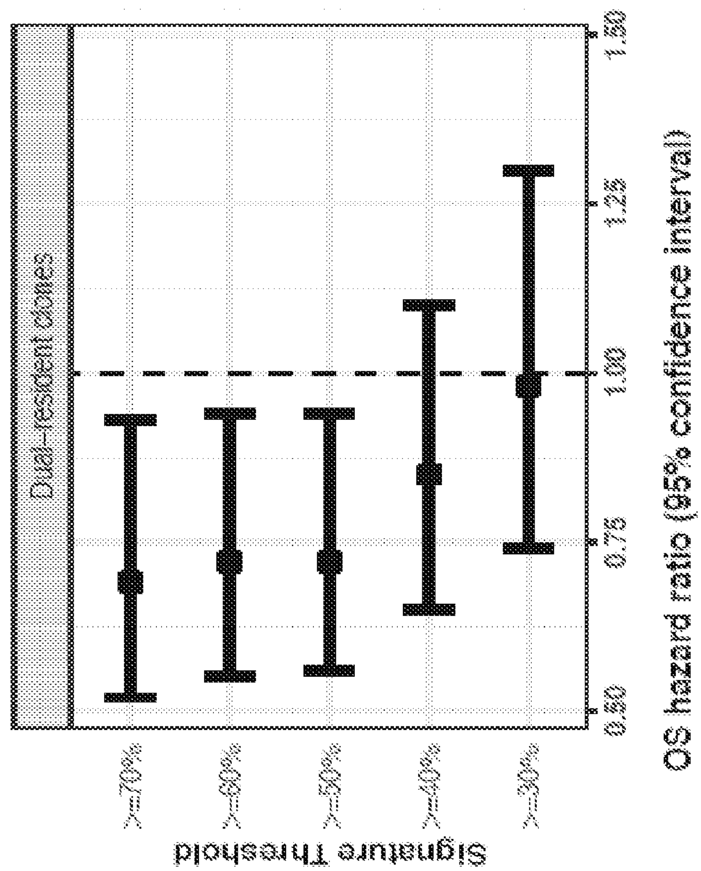


FIG. 11F

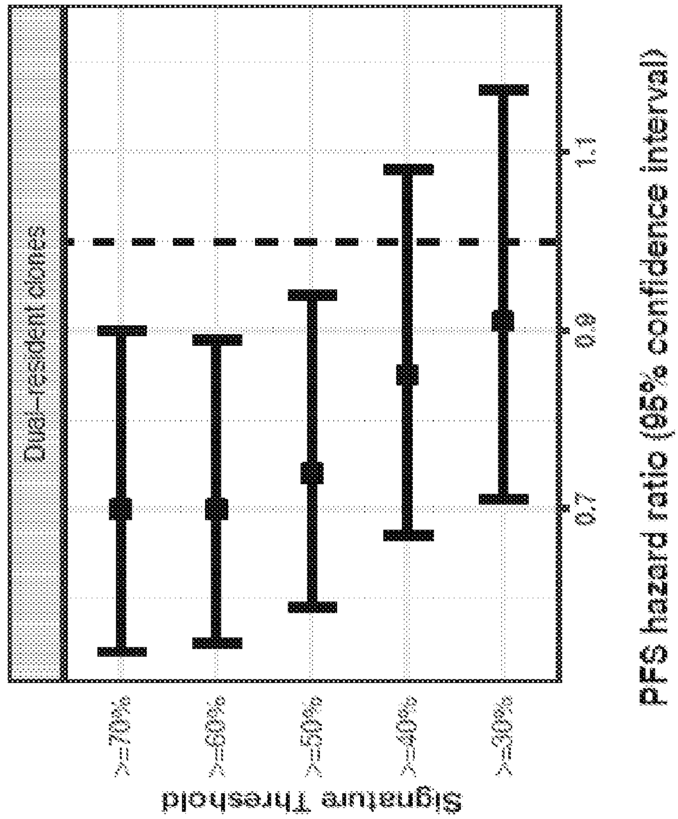

FIG. 11H

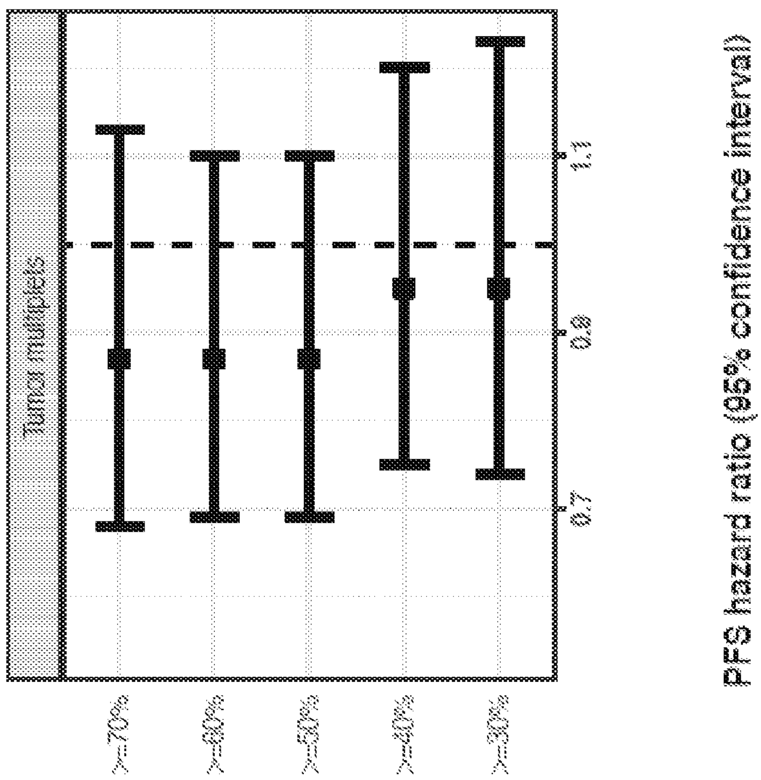


FIG. 11G

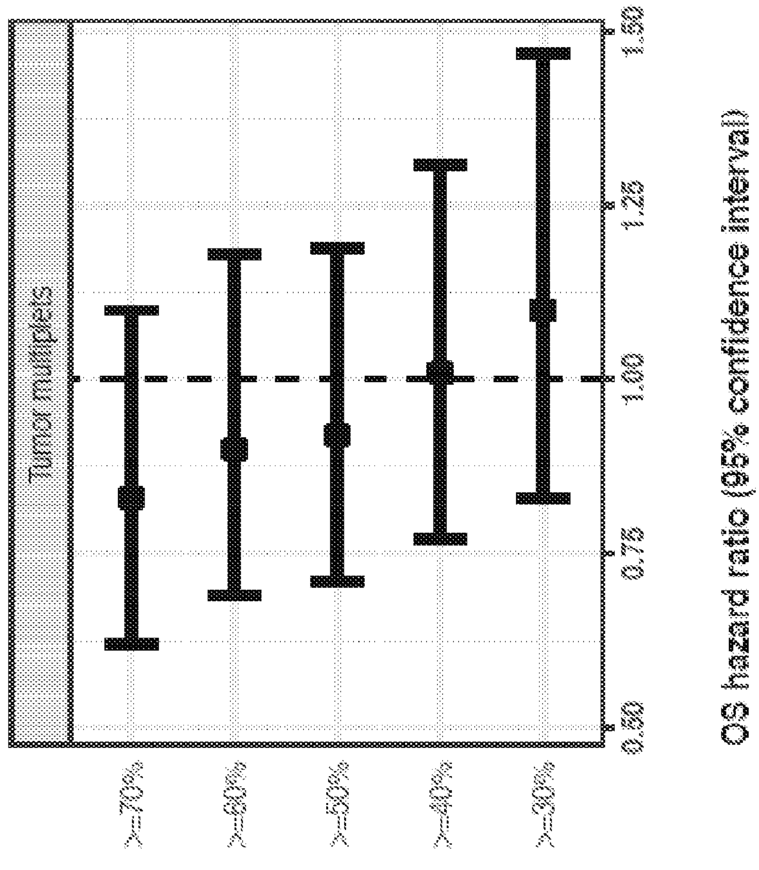

FIG. 12A

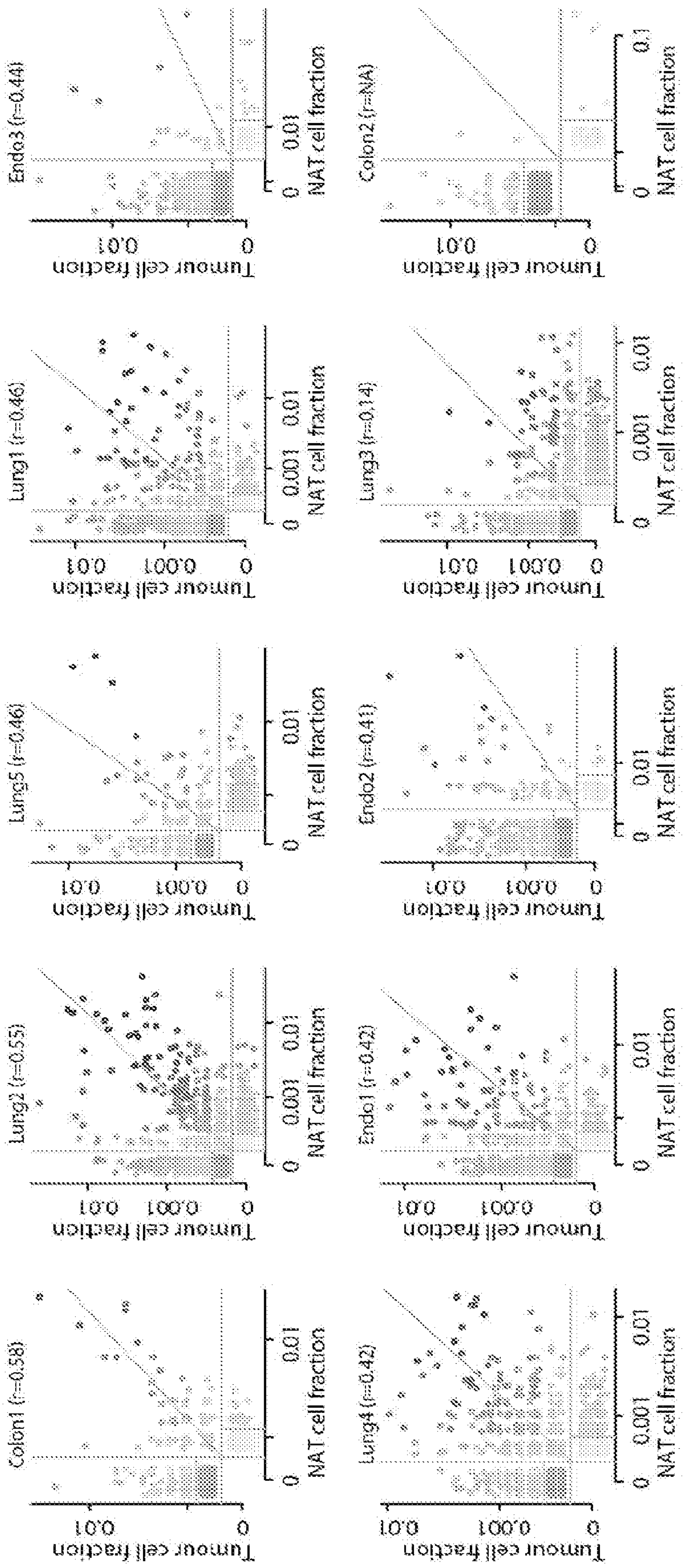
Colon1 (r=0.58)
Lung2 (r=0.55)
Lung5 (r=0.46)
Lung1 (r=0.46)
Endo3 (r=0.44)
Lung4 (r=0.42)
Endo1 (r=0.42)
Endo2 (r=0.41)
Lung3 (r=0.14)
Colon2 (r=NA)
Tumour cell fraction
NAT cell fraction
0
0.001
0.01

FIG. 12B

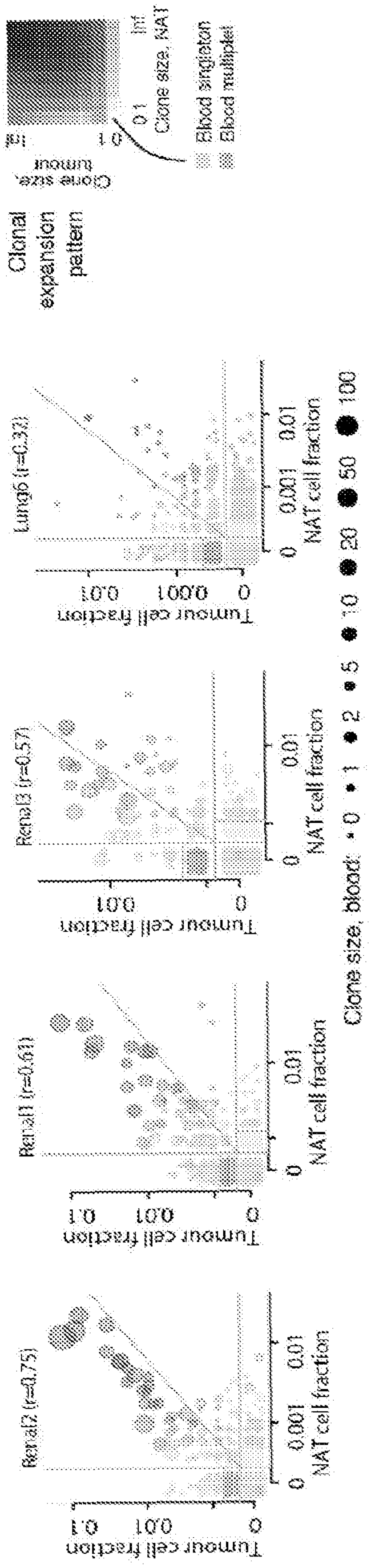
Clonal expansion pattern
Clone size, tumour
Inf
1.0
0.1
0.1
Inf
Clone size, NAT
Blood singleton
Blood multiplet
Lung6 (r=0.33)
Renal3 (r=0.57)
Renal1 (r=0.61)
Renal2 (r=0.75)
Tumour cell fraction
NAT cell fraction
0
0.001
0.01
0.1
Clone size, blood: 0 1 2 5 10 20 50 100

FIG. 12C

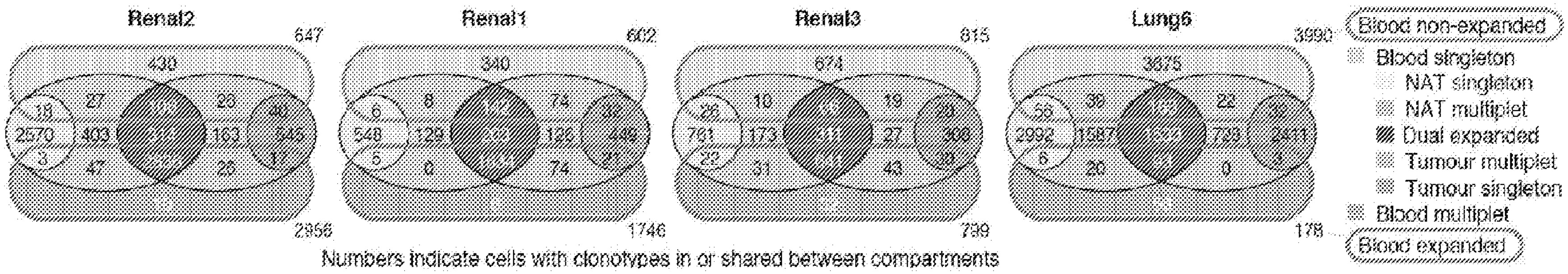
Renal2
Renal1
Renal3
Lung6
647
2956
602
1746
815
739
3990
178
Blood non-expanded
Blood singleton
NAT singleton
NAT multiplet
Dual expanded
Tumour multiplet
Tumour singleton
Blood multiplet
Blood expanded
Numbers indicate cells with clonotypes in or shared between compartments

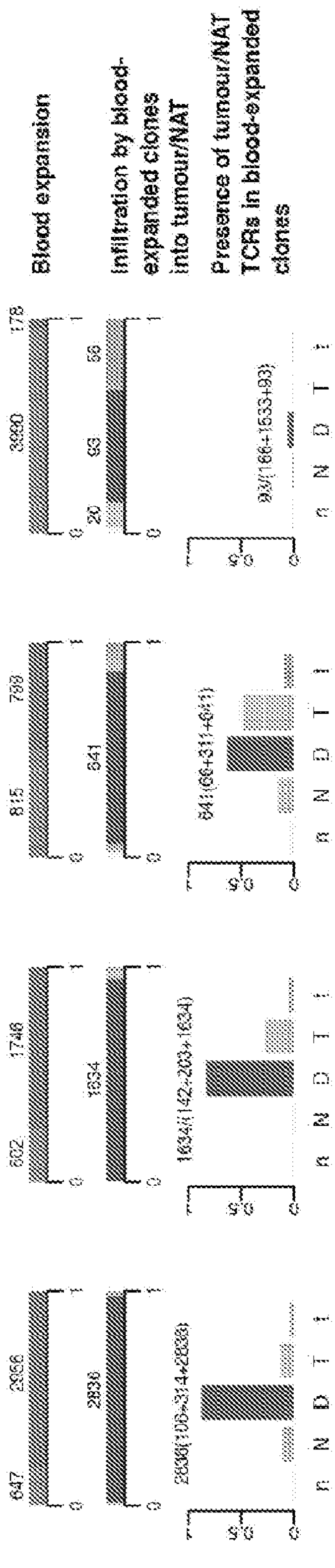


FIG. 12D

FIG. 12E

FIG. 12F

FIG. 12I

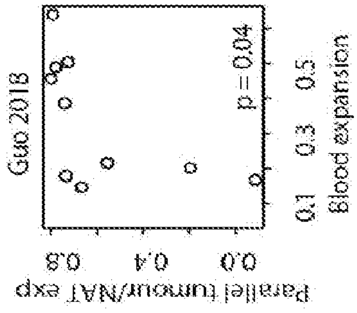


FIG. 12H

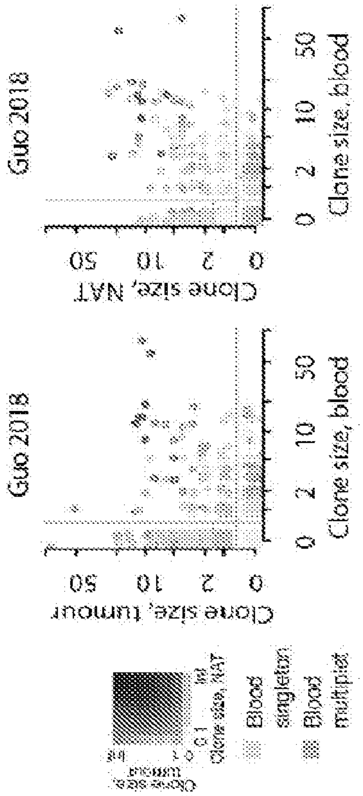


FIG. 12G

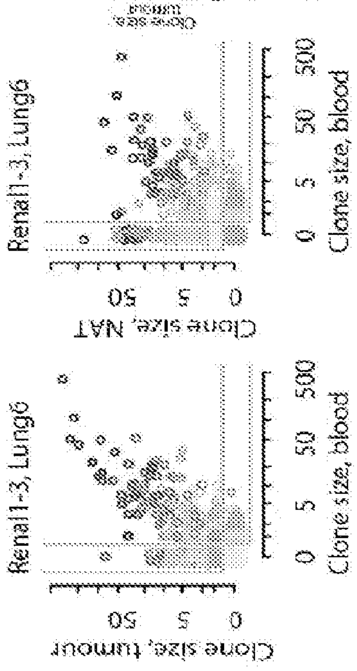

FIG. 13A

FIG. 13B

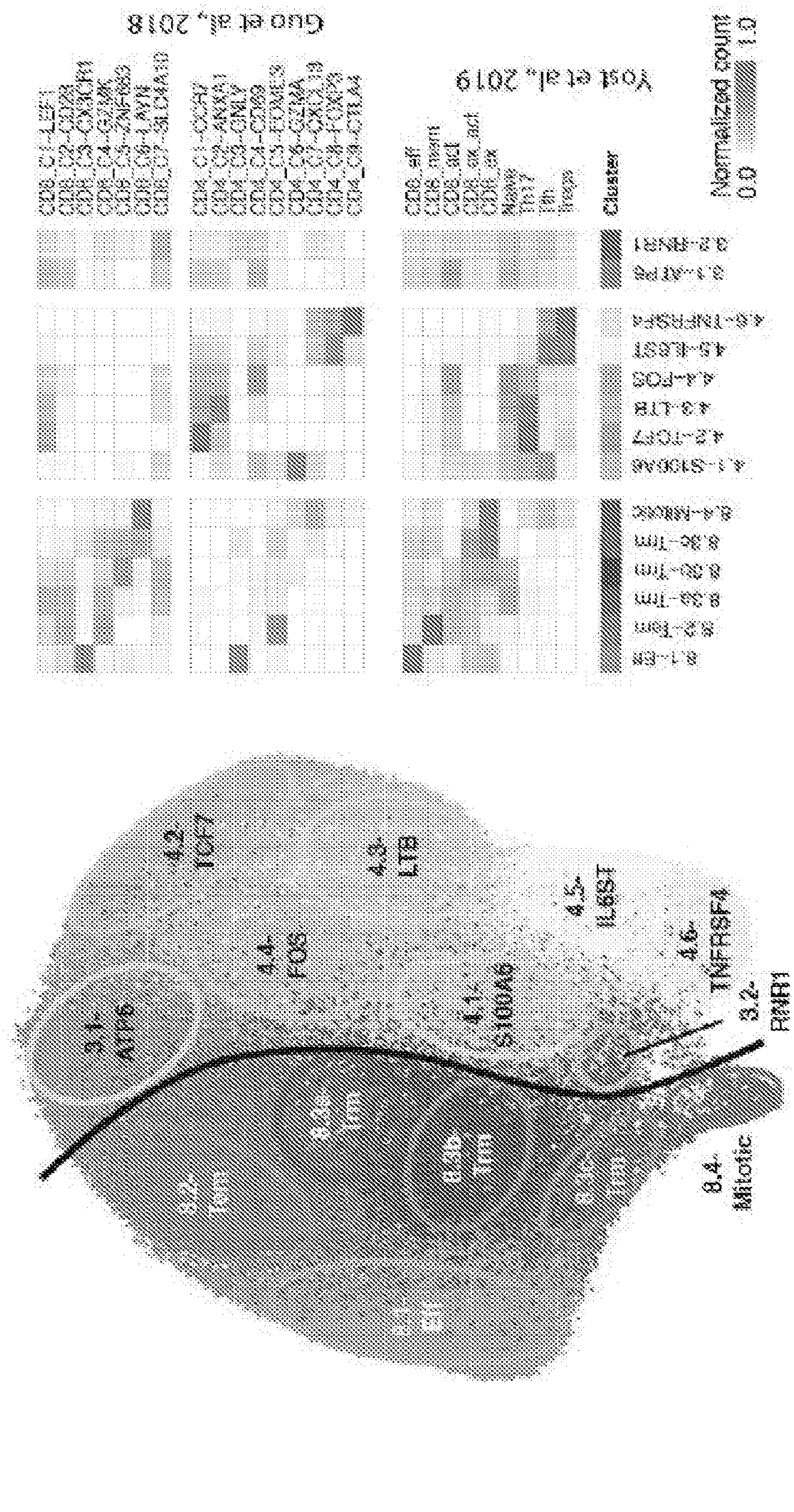

FIG. 13C

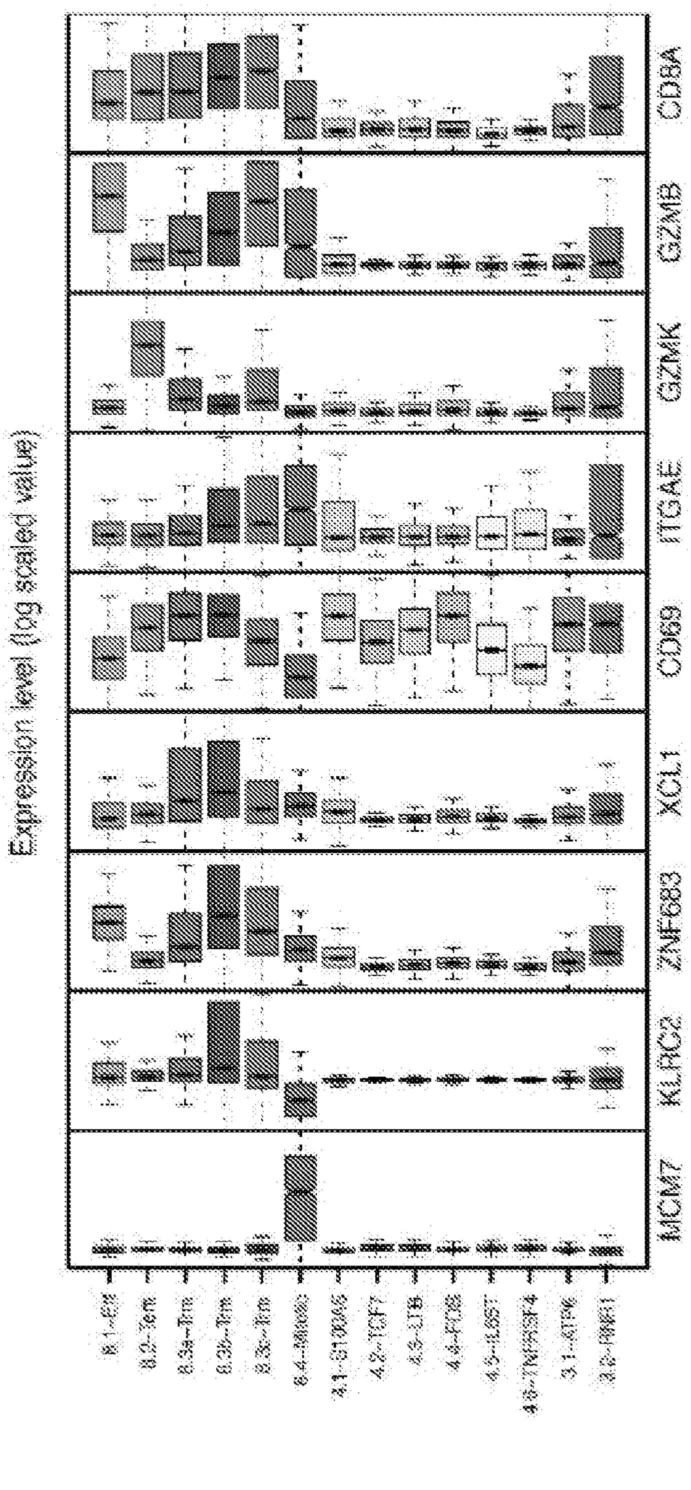
Expression level (log scaled value)
CD8A
GZMB
GZMK
ITGAE
CD69
XCL1
ZNF683
KLRC2
MCM7

FIG. 13D

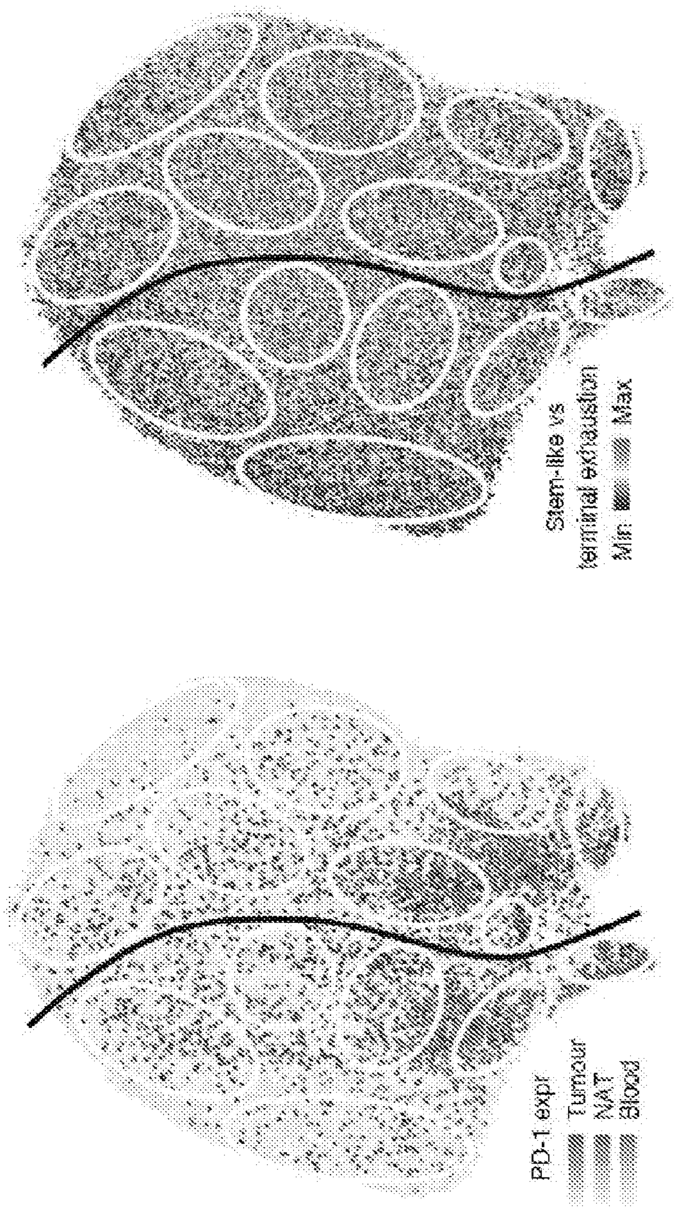
Stem-like vs
terminal exhaustion
Min
Max
PD-1 expr
Tumour
NAT
Blood

FIG. 13E

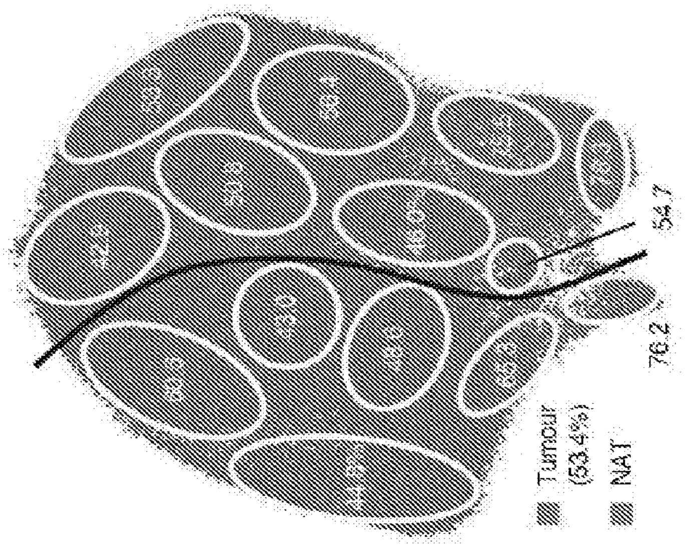


FIG. 13F

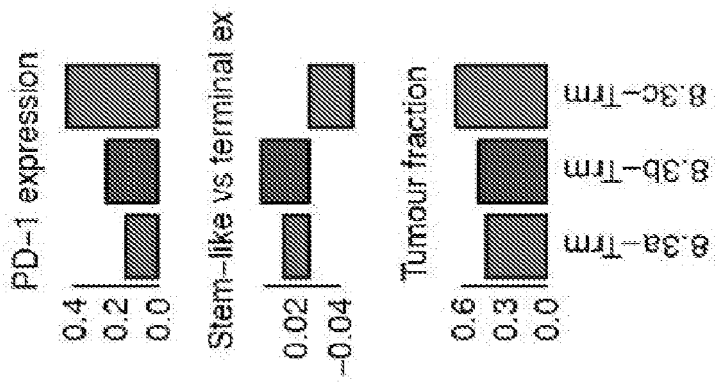

FIG. 13G

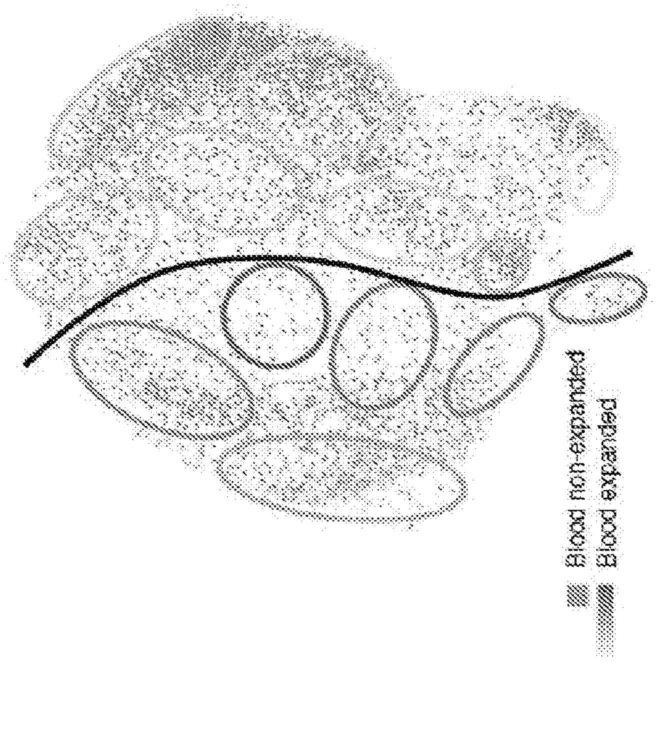


FIG. 13H

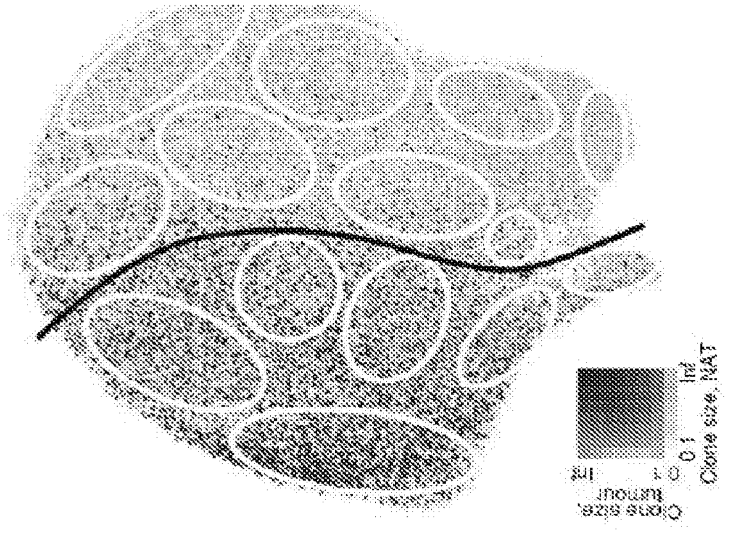

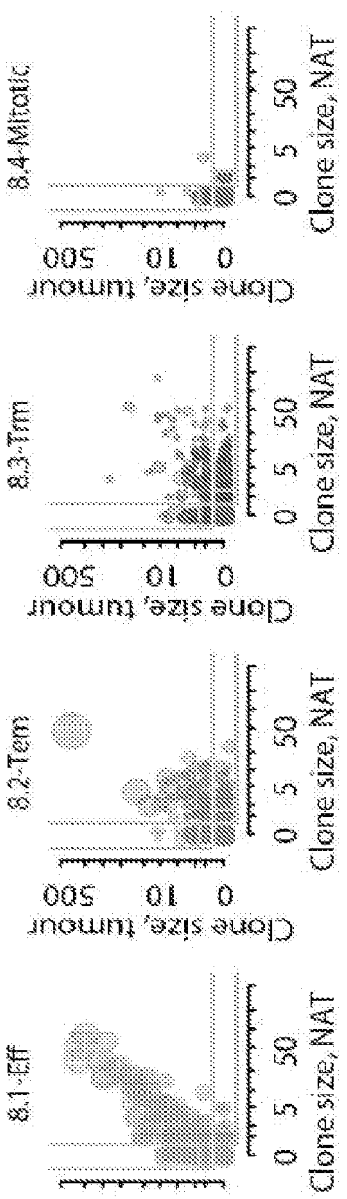


FIG. 13I

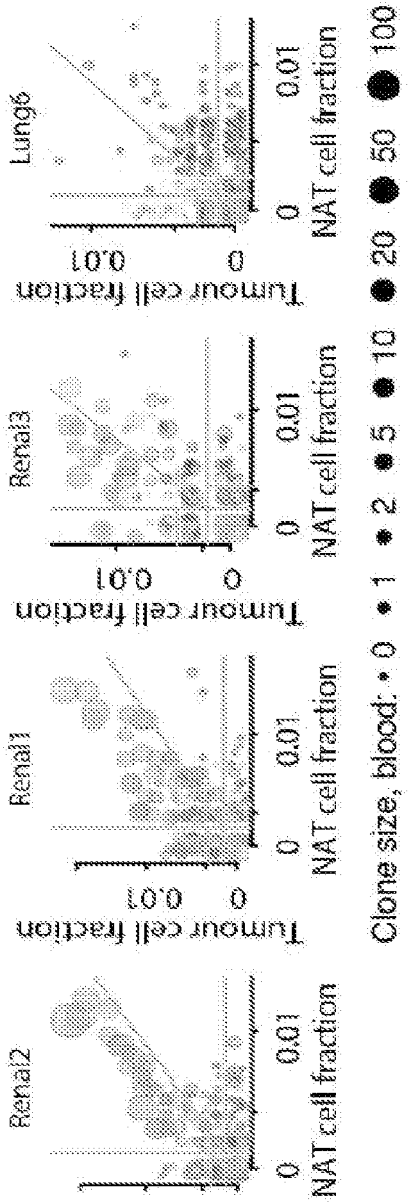


FIG. 13J

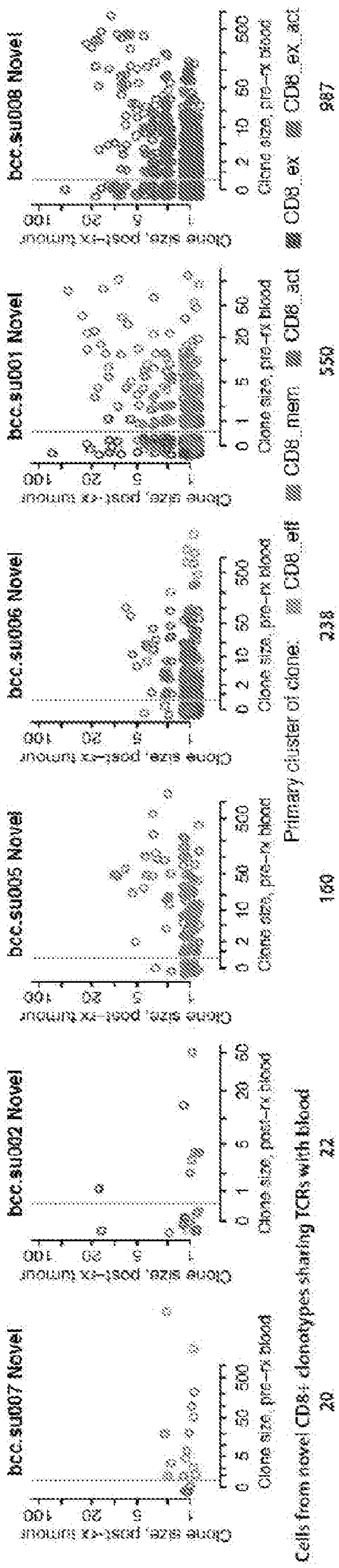
bcc.su007 Novel
bcc.su002 Novel
bcc.su005 Novel
bcc.su006 Novel
bcc.su001 Novel
bcc.su008 Novel
Clone size, post−rx tumour
Clone size, pre−rx blood
Clone size, post−rx blood
Cells from novel CD8+ clonotypes sharing TCRs with blood
20
22
160
238
550
987
Primary cluster of clone:
CD8_eff
CD8_mem
CD8_act
CD8_ex
CD8_ex_act

FIG. 14A

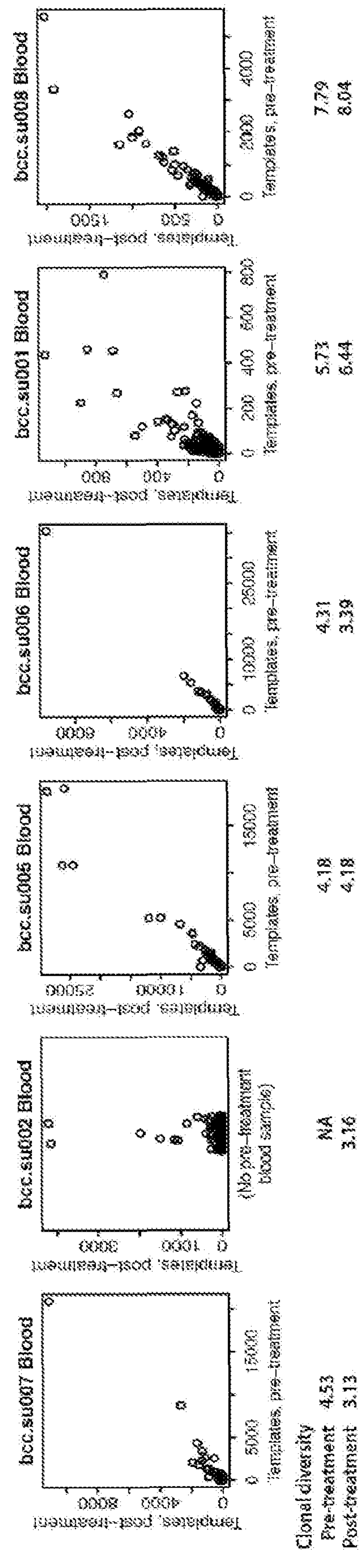
bcc.su007 Blood
bcc.su002 Blood
bcc.su005 Blood
bcc.su006 Blood
bcc.su001 Blood
bcc.su008 Blood
Templates, post-treatment
Templates, pre-treatment
(No pre-treatment blood sample)
Clonal diversity
Pre-treatment 4.53 NA 4.18 4.31 5.73 7.79
Post-treatment 3.13 3.16 4.18 3.39 6.44 8.04

FIG. 14B

FIG. 14C

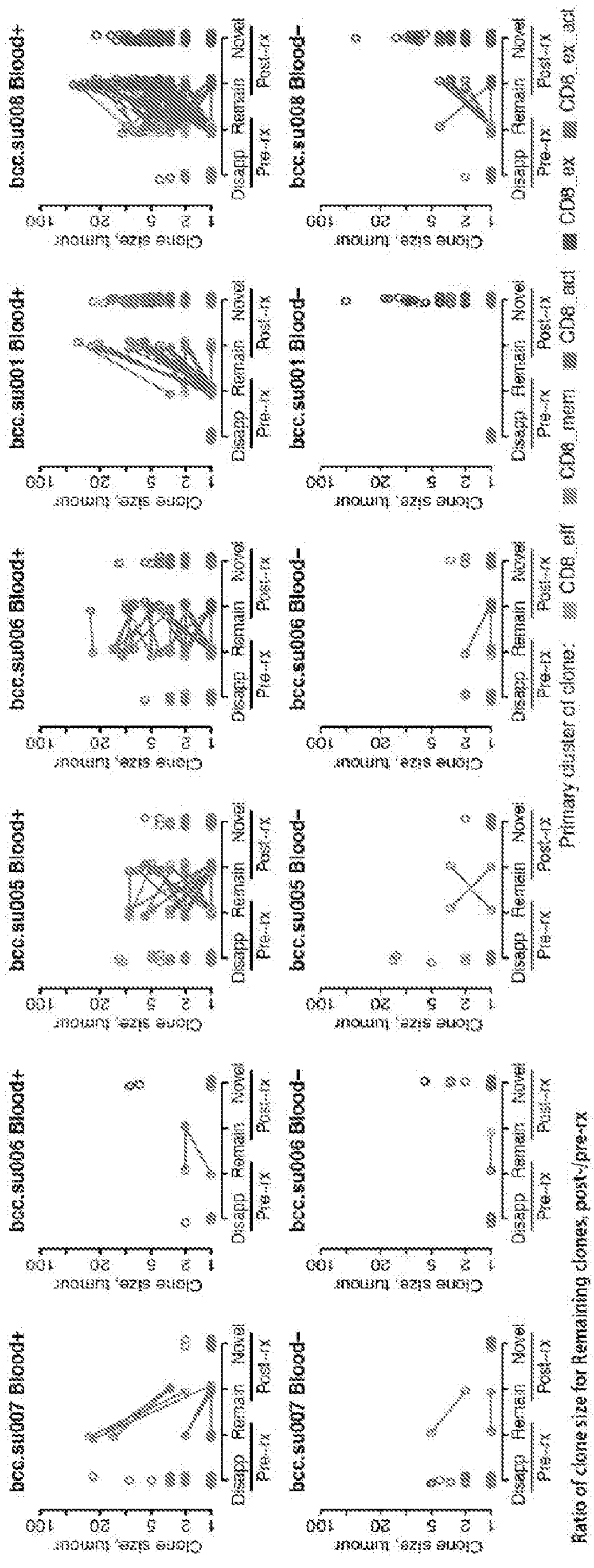

bcc.su007 Blood+
bcc.su006 Blood+
bcc.su005 Blood+
bcc.su006 Blood+
bcc.su001 Blood+
bcc.su008 Blood+
bcc.su007 Blood−
bcc.su006 Blood−
bcc.su005 Blood−
bcc.su006 Blood−
bcc.su001 Blood−
bcc.su008 Blood−
Clone size, tumour
Disapp
Remain
Novel
Pre-rx
Post-rx
Ratio of clone size for Remaining clones, post-/pre-rx
Blood+ 0.36 1.41 0.94 1.08 3.88 1.96
Blood− 0.63 1.00 1.00 0.87 NA 1.40
Primary cluster of clone:
CD8_eff
CD8_mem
CD8_act
CD8_ex
CD8_ex_act

FIG. 14D

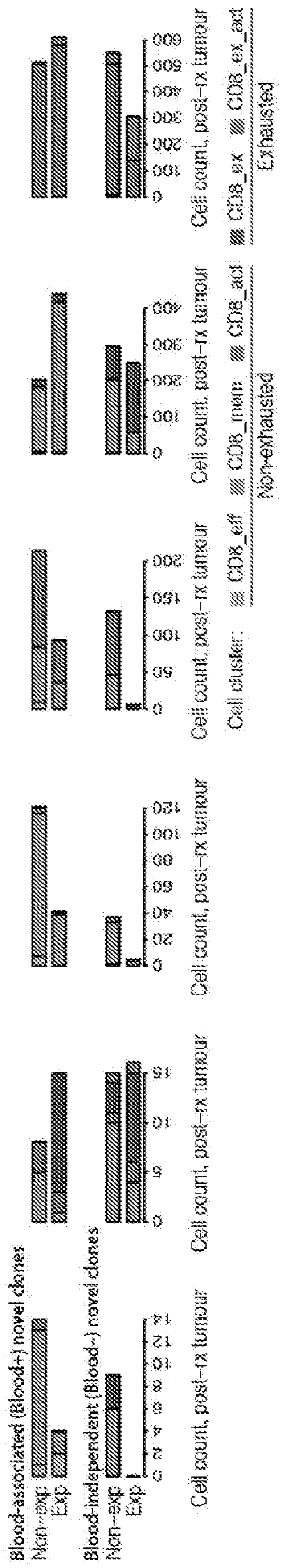

Blood-associated (Blood+) novel clones
Blood-independent (Blood-) novel clones
Non-exp
Exp
Cell count, post-rx tumour
Cell cluster:
CD8_eff
CD8_mem
CD8_act
Non-exhausted
CD8_ex
CD8_ex_act
Exhausted

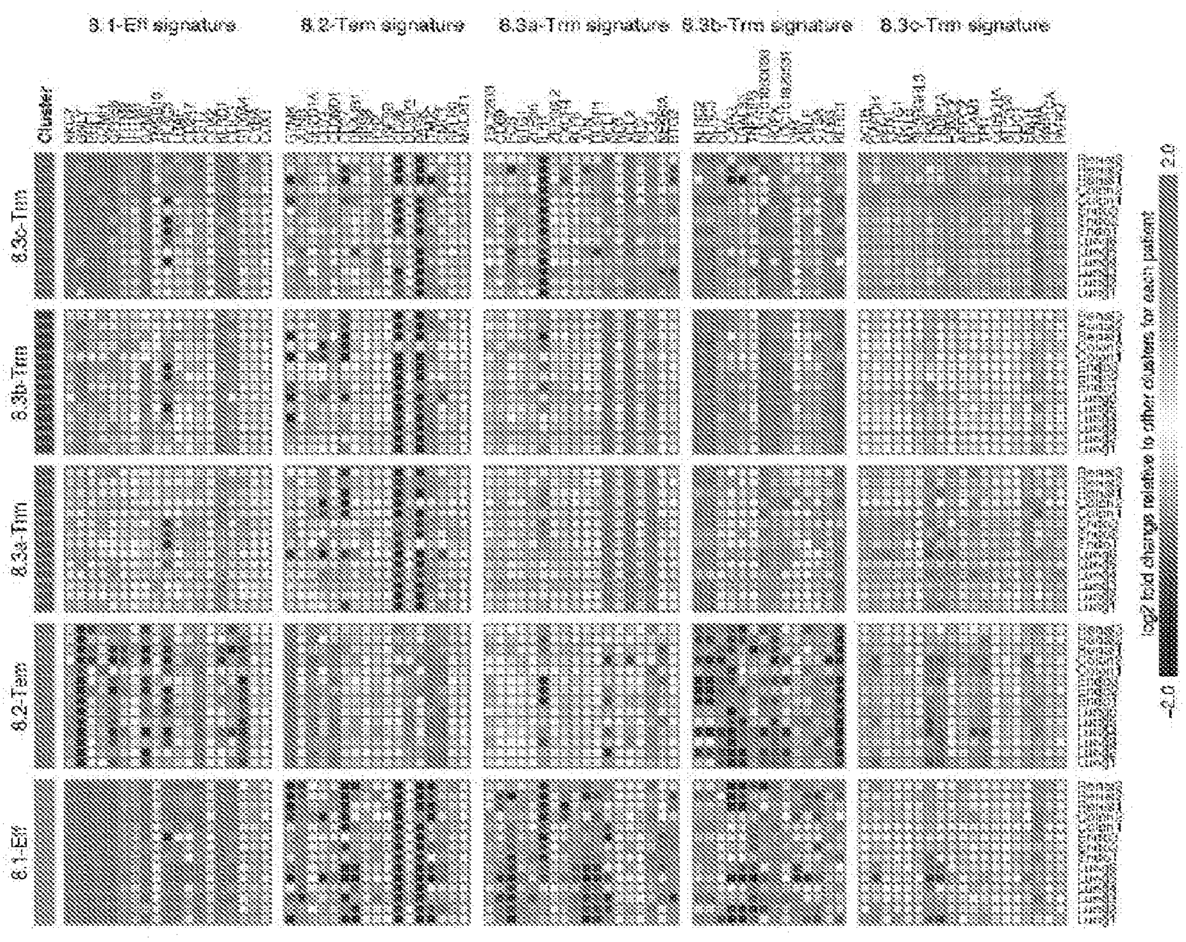
8.1-Eff signature
8.2-Tem signature
8.3a-Trm signature
8.3b-Trm signature
8.3c-Trm signature
Cluster
8.3c-Trm
8.3b-Trm
8.3a-Trm
8.2-Tem
8.1-Eff
log2 fold change relative to other clusters for each patient
−2.0
2.0

FIG. 15A

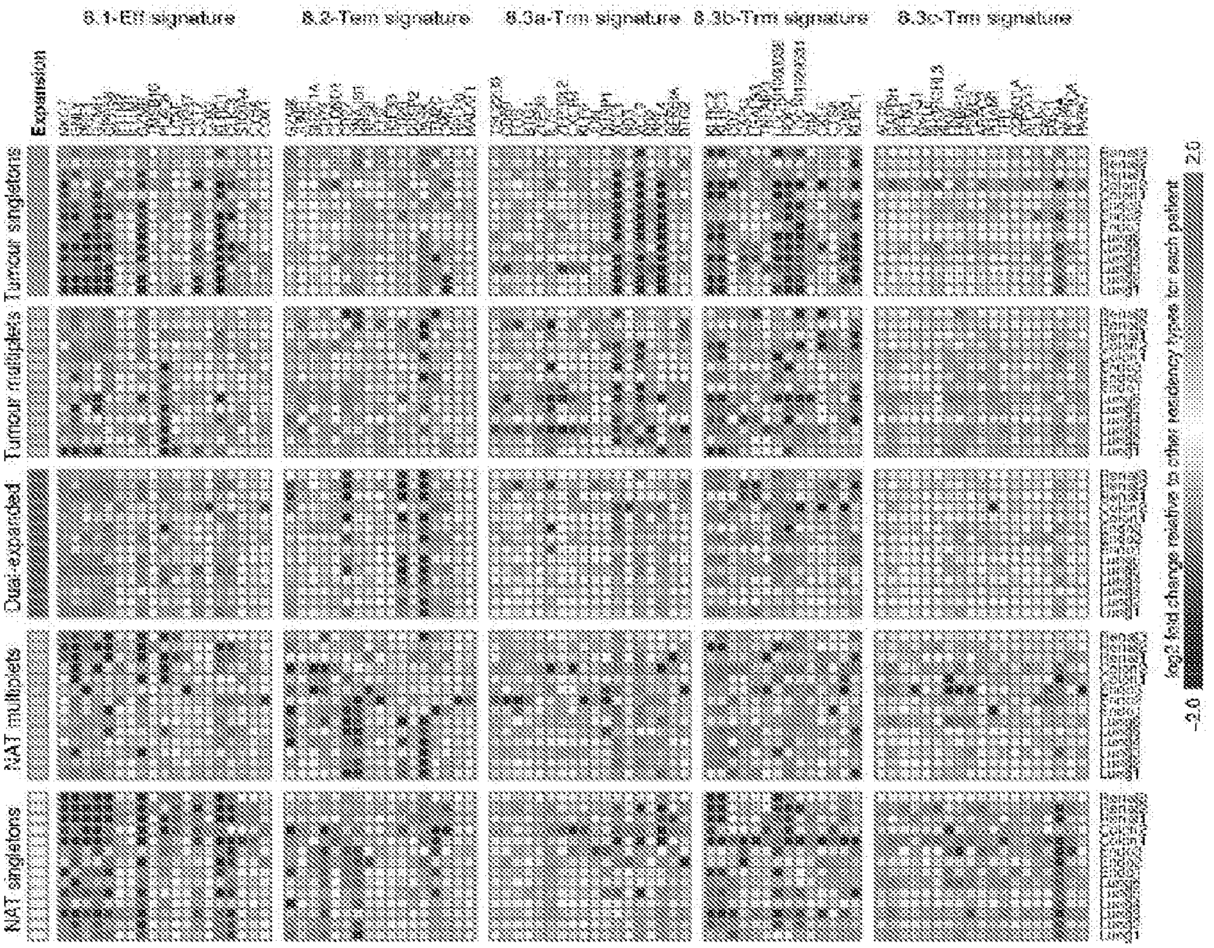
8.1-Eff signature
8.2-Tem signature
8.3a-Trm signature
8.3b-Trm signature
8.3c-Trm signature
Expression
Tumour singletons
Tumour multiplets
Dual-expanded
NAT multiplets
NAT singletons
log2 fold change relative to other residency types for each patient
-2.0
2.0

FIG. 15B

FIG. 15C

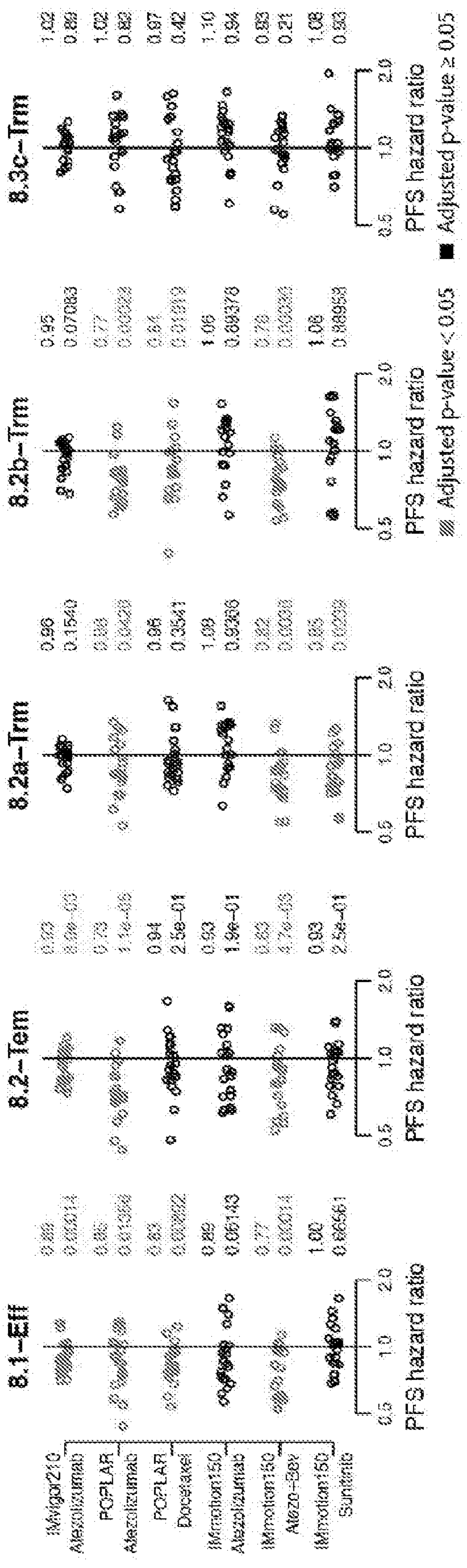
8.1–Eff
8.2–Tem
8.2a–Trm
8.2b–Trm
8.3c–Trm
IMvigor210 Atezolizumab
POPLAR Atezolizumab
POPLAR Docetaxel
IMmotion150 Atezolizumab
IMmotion150 Atezo+Bev
IMmotion150 Sunitinib
PFS hazard ratio
0.5
1.0
2.0
Adjusted p-value < 0.05
Adjusted p-value ≥ 0.05

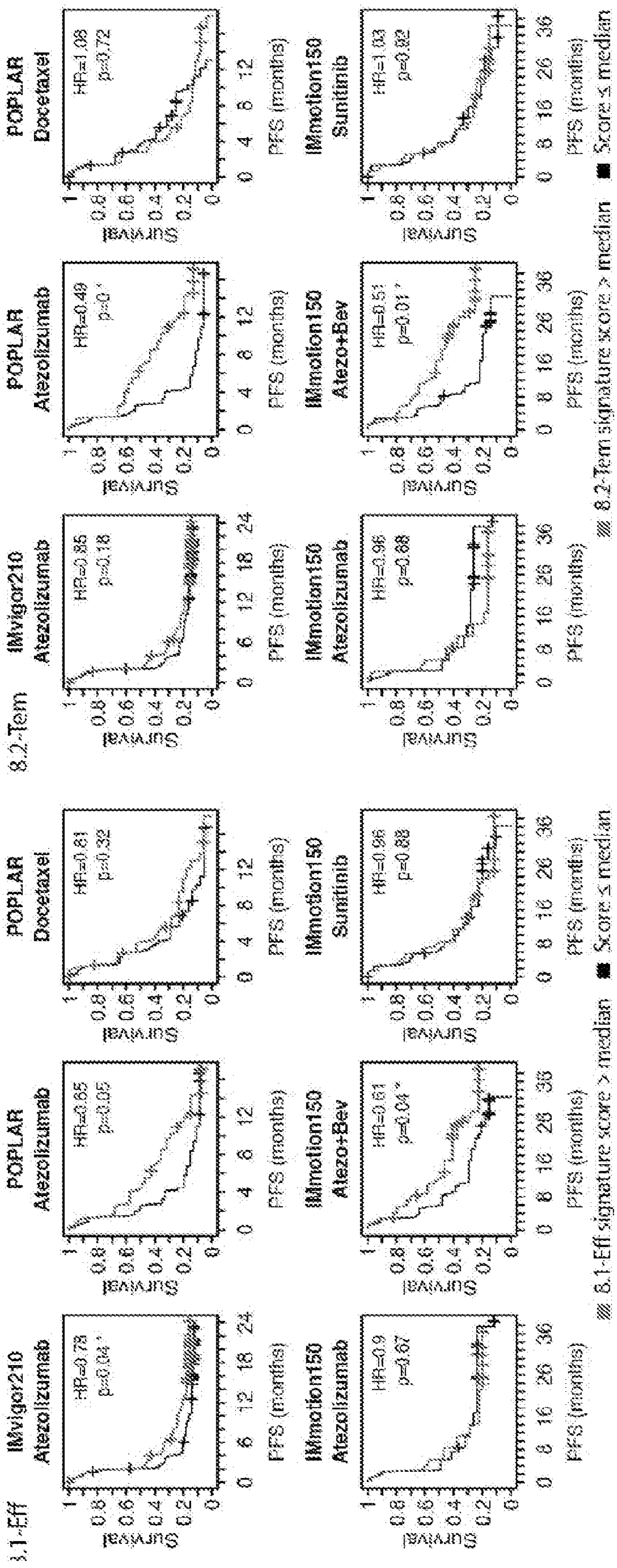
8.1-Eff
IMvigor210 Atezolizumab
HR=0.78 p=0.04 *
POPLAR Atezolizumab
HR=0.65 p=0.05
POPLAR Docetaxel
HR=0.81 p=0.32
IMmotion150 Atezolizumab
HR=0.9 p=0.67
IMmotion150 Atezo+Bev
HR=0.61 p=0.04 *
IMmotion150 Sunitinib
HR=0.96 p=0.88
8.1-Eff signature score > median
Score ≤ median
8.2-Tem
IMvigor210 Atezolizumab
HR=0.85 p=0.18
POPLAR Atezolizumab
HR=0.49 p=0 *
POPLAR Docetaxel
HR=1.08 p=0.72
IMmotion150 Atezolizumab
HR=0.96 p=0.88
IMmotion150 Atezo+Bev
HR=0.51 p=0.01 *
IMmotion150 Sunitinib
HR=1.03 p=0.82
8.2-Tem signature score > median
Score ≤ median
Survival
PFS (months)

FIG. 15D

FIG. 16B

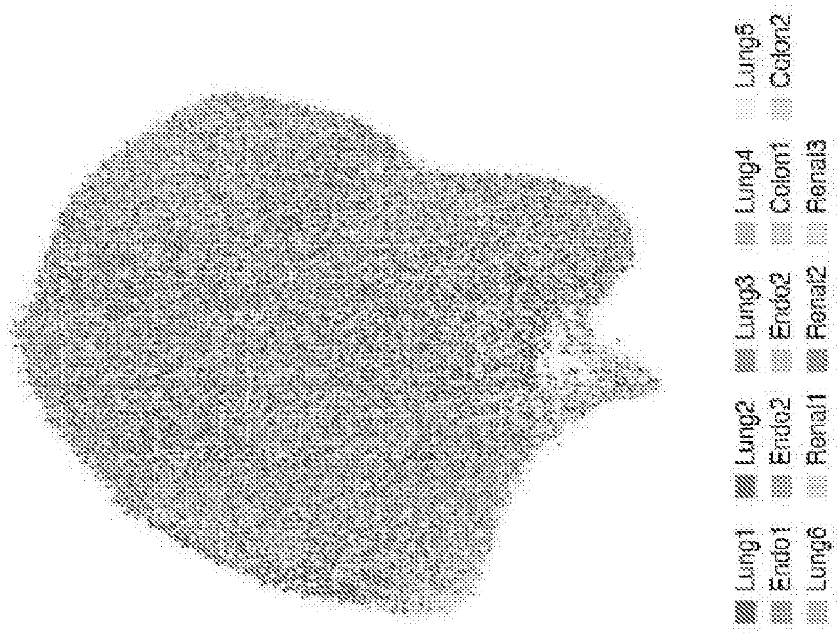


FIG. 16A

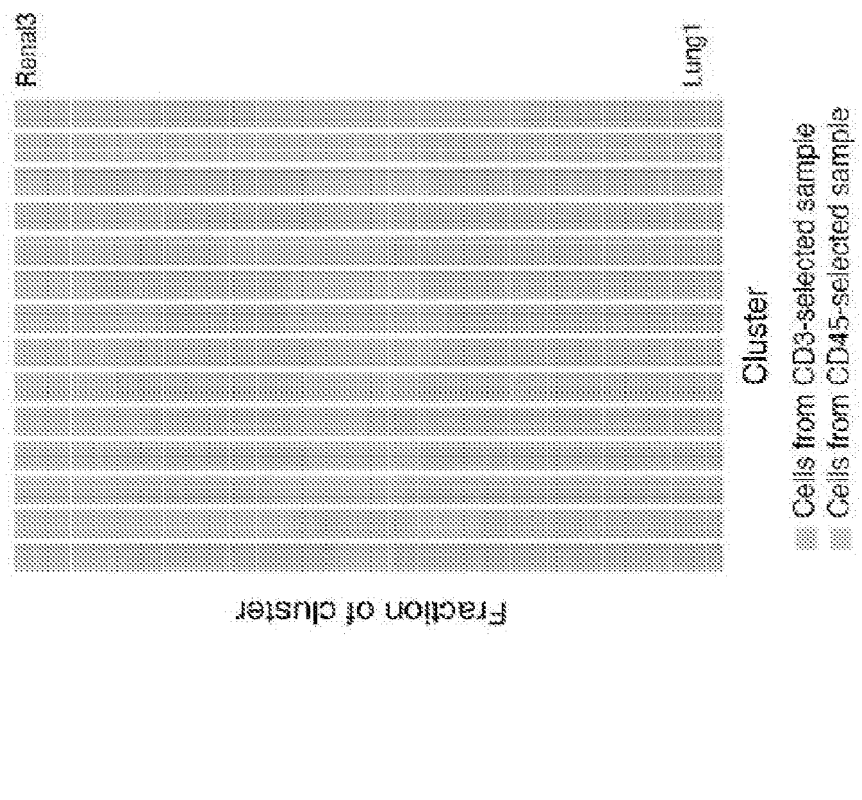

FIG. 16C

| Patient | Src | Single-cell RNA-seq: T cells Transcripts | T cells Cells | Non-T cells Transcripts | Non-T cells Cells | scTCR-seq Typed | scTCR-seq Clones |
|---|---|---|---|---|---|---|---|
| Lung1 (CD3) | T | 26,467,011 | 6,862 | 1,972,439 | 600 | 6,105 | 4,684 |
| | N | 17,694,063 | 6,048 | 325,313 | 121 | 4,376 | |
| Lung2 (CD3) | T | 41,966,695 | 8,436 | 1,030,663 | 347 | 7,905 | 6,482 |
| | N | 29,446,064 | 6,937 | 1,142,999 | 466 | 6,312 | |
| Lung3 (CD3) | T | 9,404,335 | 6,640 | 1,078,052 | 322 | 4,688 | 7,011 |
| | N | 18,054,694 | 11,493 | 1,758,249 | 513 | 7,903 | |
| Lung4 (CD3) | T | 5,291,282 | 9,046 | 68,959 | 135 | 4,857 | 4,833 |
| | N | 3,235,442 | 6,067 | 109,168 | 221 | 2,875 | |
| Lung5 (CD45) | T | 7,909,459 | 2,733 | 11,537,803 | 2,551 | 2,377 | 3,487 |
| | N | 9,035,053 | 3,957 | 8,802,842 | 1,767 | 2,938 | |
| Endo1 (CD3) | T | 10,984,770 | 10,282 | 1,768,372 | 792 | 7,050 | 5,387 |
| | N | 4,393,620 | 7,290 | 266,026 | 278 | 2,615 | |
| Endo2 (CD3) | T | 6,145,940 | 7,617 | 205,682 | 192 | 3,732 | 2,190 |
| | N | 420,345 | 1,787 | 7,717 | 39 | 202 | |
| Endo3 (CD3) | T | 9,514,096 | 5,368 | 666,326 | 155 | 3,929 | 2,485 |
| | N | 207,823 | 370 | 4,907 | 15 | 131 | |
| Colon1 (CD45) | T | 7,213,104 | 2,408 | 6,072,136 | 1,470 | 1,969 | 2,364 |
| | N | 4,632,539 | 1,745 | 4,451,001 | 1,959 | 1,150 | |
| Colon2 (CD45) | T | 1,823,577 | 632 | 9,759,630 | 2,642 | 594 | 535 |
| | N | 806,615 | 181 | 3,648,093 | 1,458 | 66 | |
| Lung6 (CD45) | T | 20,421,731 | 3,589 | 16,282,571 | 1,883 | 3,734 | 9,948 |
| | N | 23,682,347 | 7,190 | 8,308,015 | 1,317 | 5,847 | |
| | B | 18,169,347 | 4,146 | 18,743,405 | 4,089 | 3,838 | |
| Renal1 (CD45) | T | 5,496,606 | 2,951 | 11,916,793 | 4,414 | 1,878 | 1,599 |
| | N | 2,323,817 | 3,055 | 3,380,358 | 2,878 | 1,112 | |
| | B | 3,095,967 | 1,469 | 7,578,830 | 3,347 | 813 | |
| Renal2 (CD45) | T | 9,708,729 | 3,021 | 13,762,241 | 2,508 | 2,455 | 3,960 |
| | N | 9,854,803 | 4,045 | 13,633,558 | 3,931 | 3,755 | |
| | B | 4,492,259 | 1,877 | 20,145,473 | 6,694 | 1,388 | |
| Renal3 (CD45) | T | 2,736,751 | 1,397 | 19,754,822 | 5,327 | 707 | 2,009 |
| | N | 4,811,414 | 1,665 | 21,034,316 | 3,938 | 1,355 | |
| | B | 6,225,831 | 2,448 | 4,829,186 | 1,505 | 1,132 | |
| Total | | 325,716,129 | 142,752 | 216,146,965 | 57,874 | 99,738 | 56,974 |

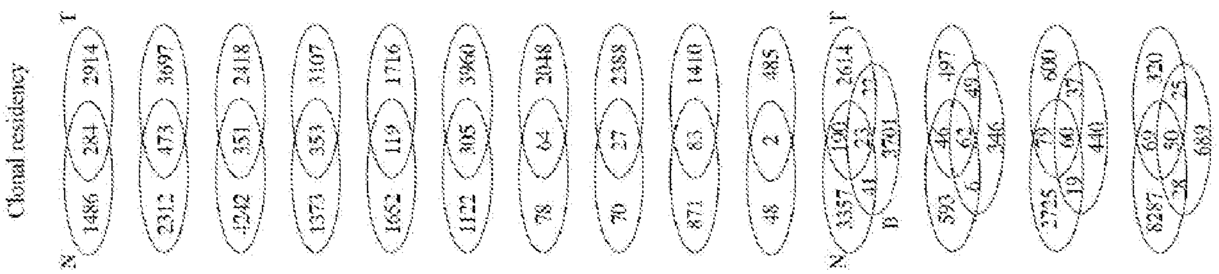


FIG. 16D

FIG. 16F

FIG. 16E

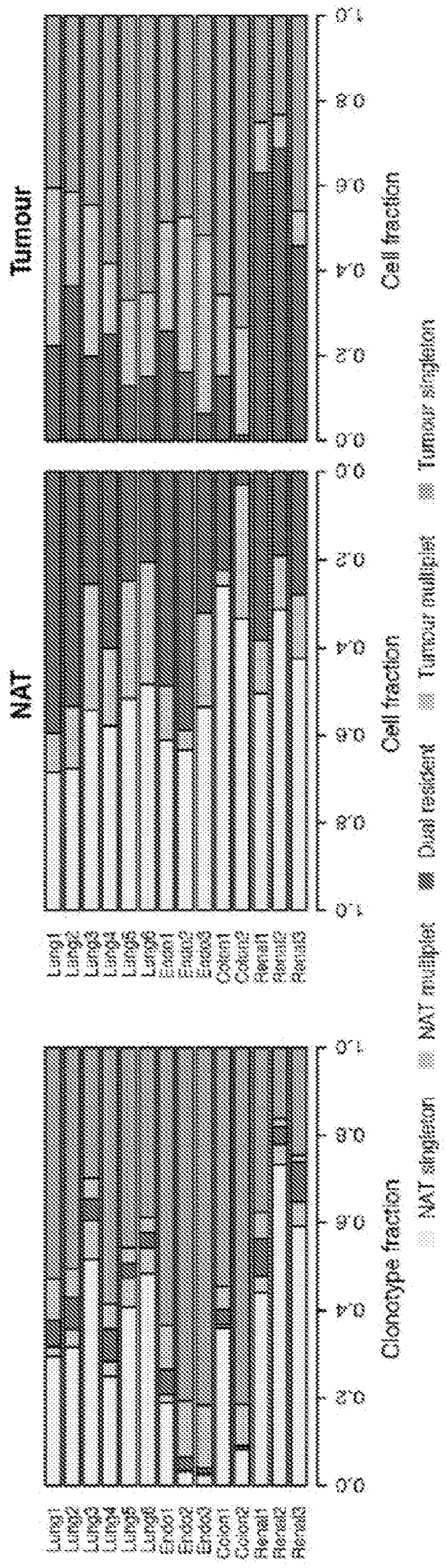

FIG. 17

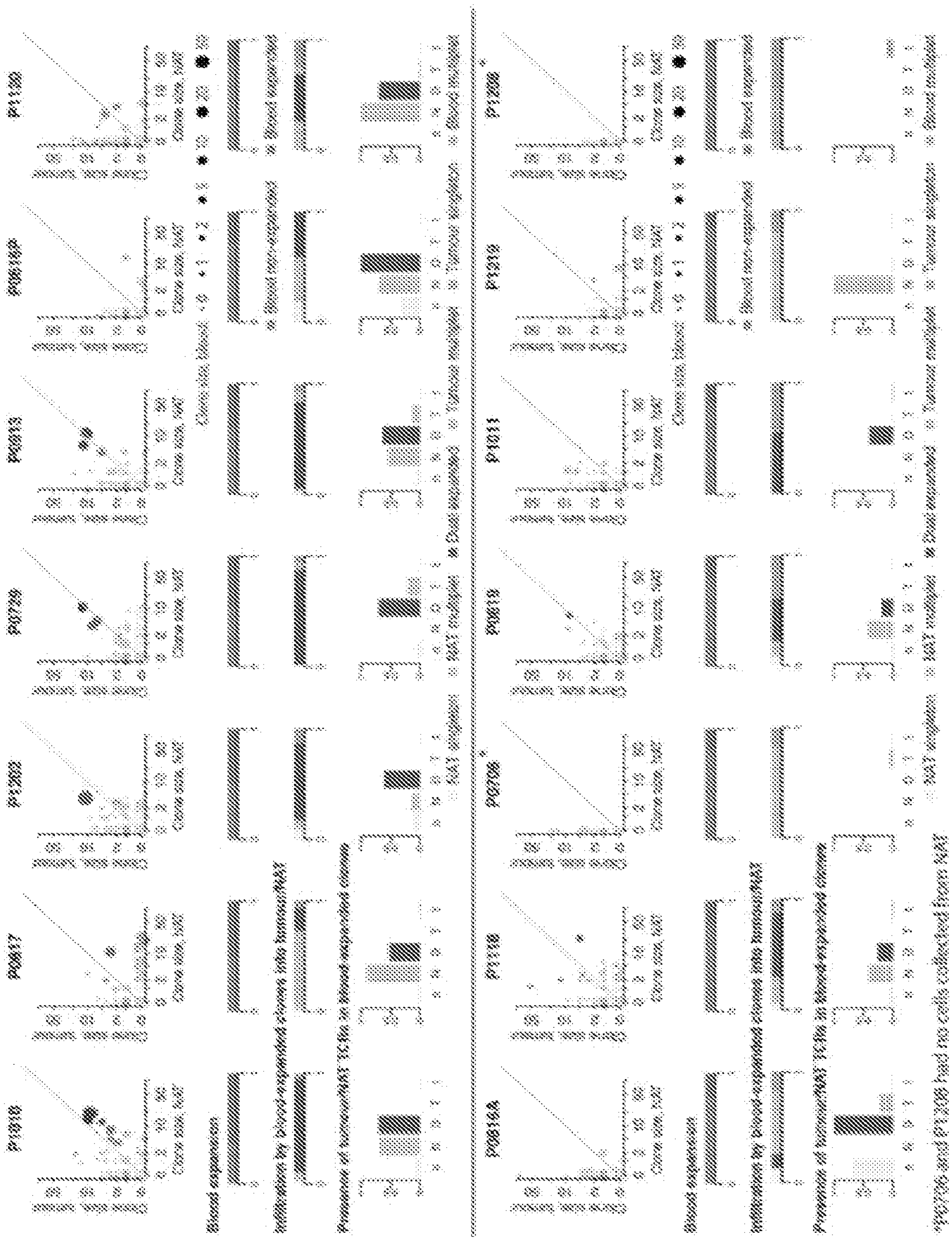

FIG. 18B

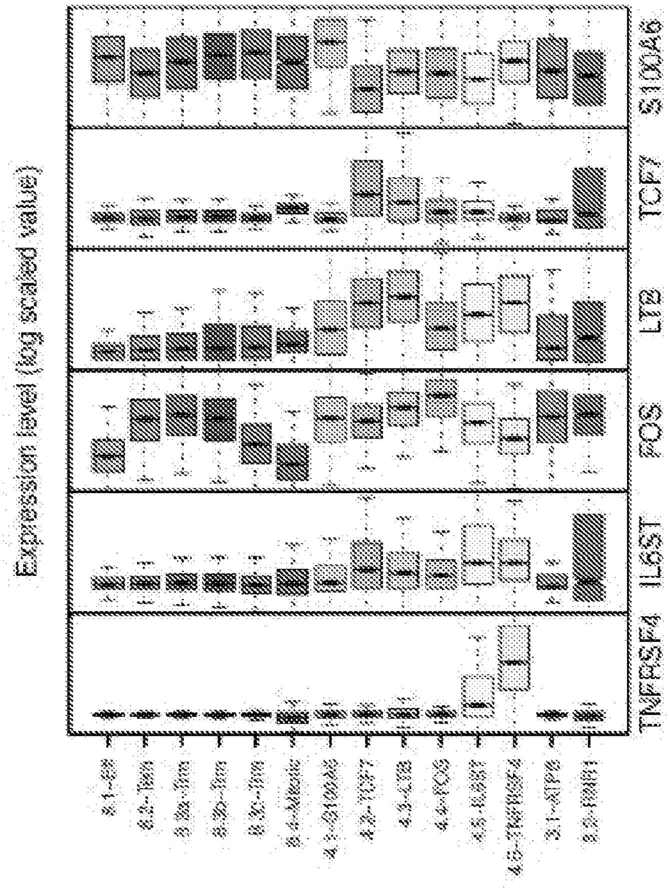


FIG. 18A

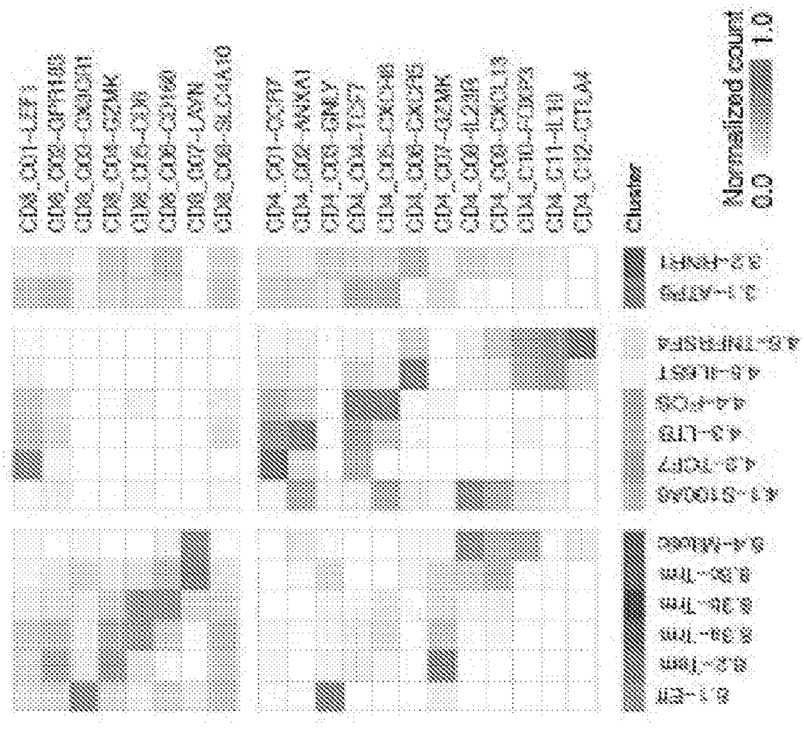

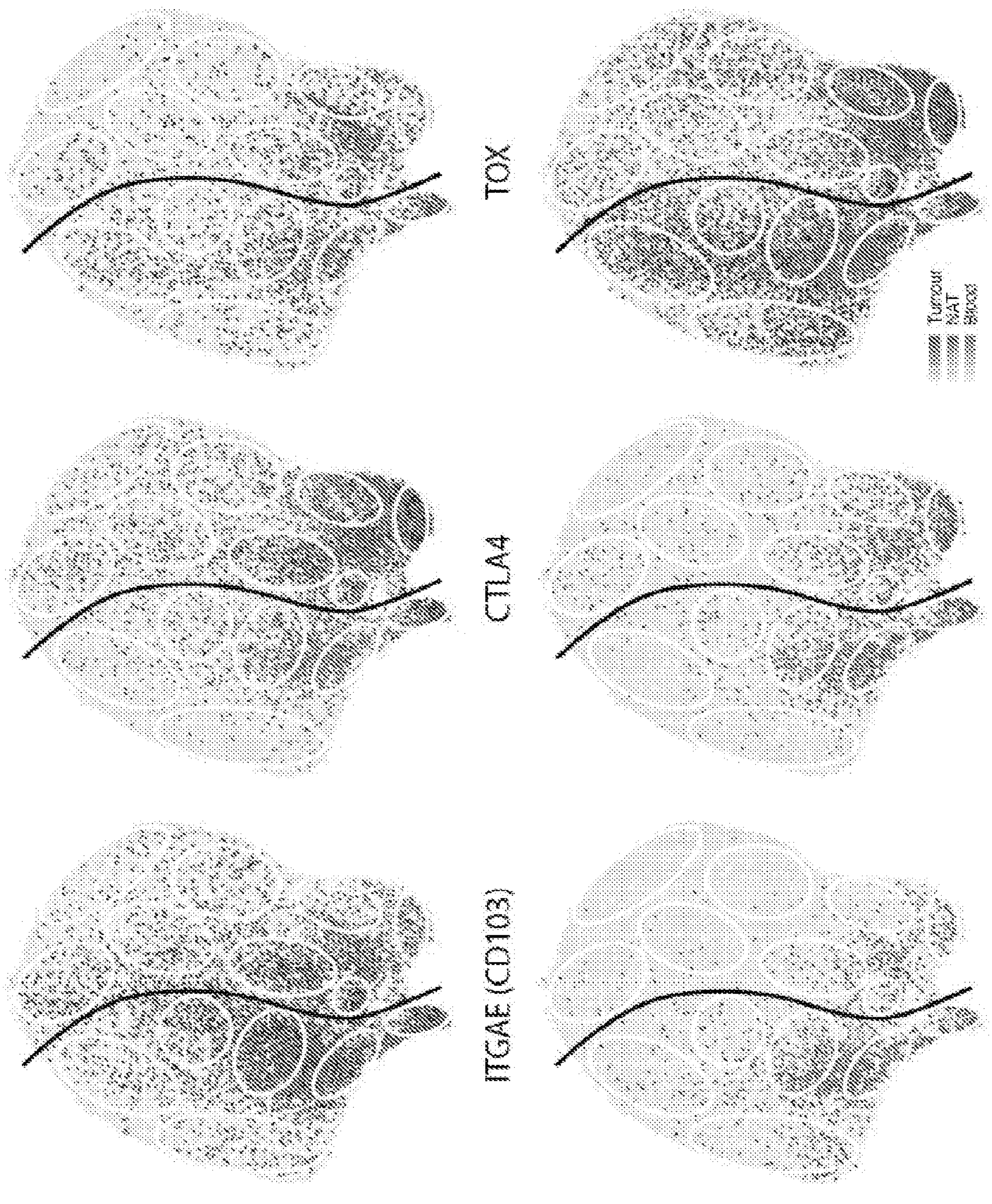
TOX
TIGIT
Tumour
NAT
Blood
CTLA4
ENTPD1 (CD39)
ITGAE (CD103)
HAVCR2 (TIM-3)

FIG. 18C

FIG. 18D

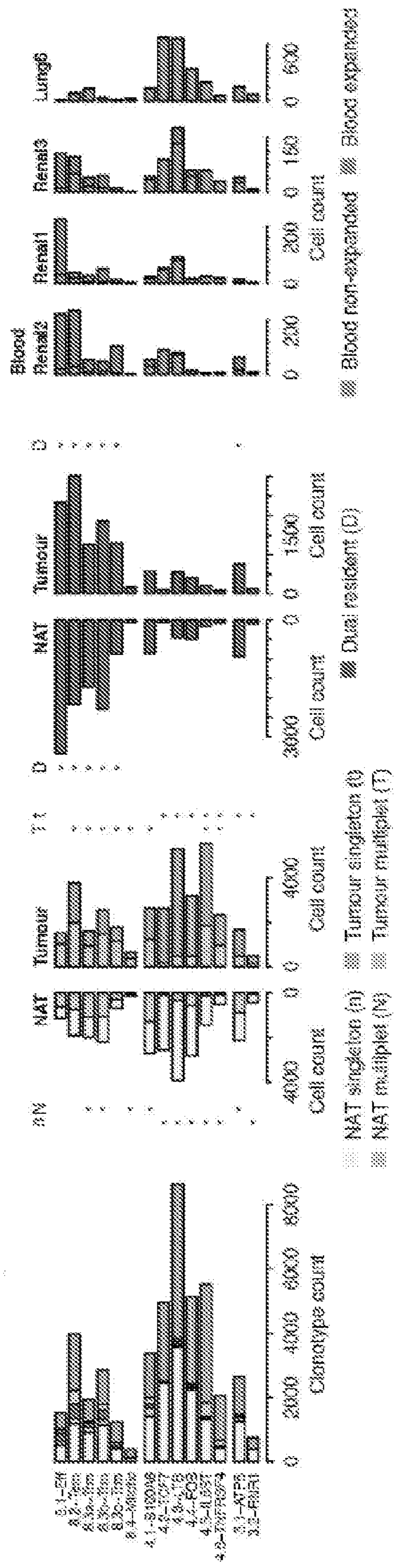
Clonotype count
Cell count
NAT
Tumour
NAT singleton (n)
NAT multiplet (N)
Tumour singleton (t)
Tumour multiplet (T)
Dual resident (D)
Blood Renal2
Renal1
Renal3
Lung6
Blood non-expanded
Blood expanded

FIG. 18E

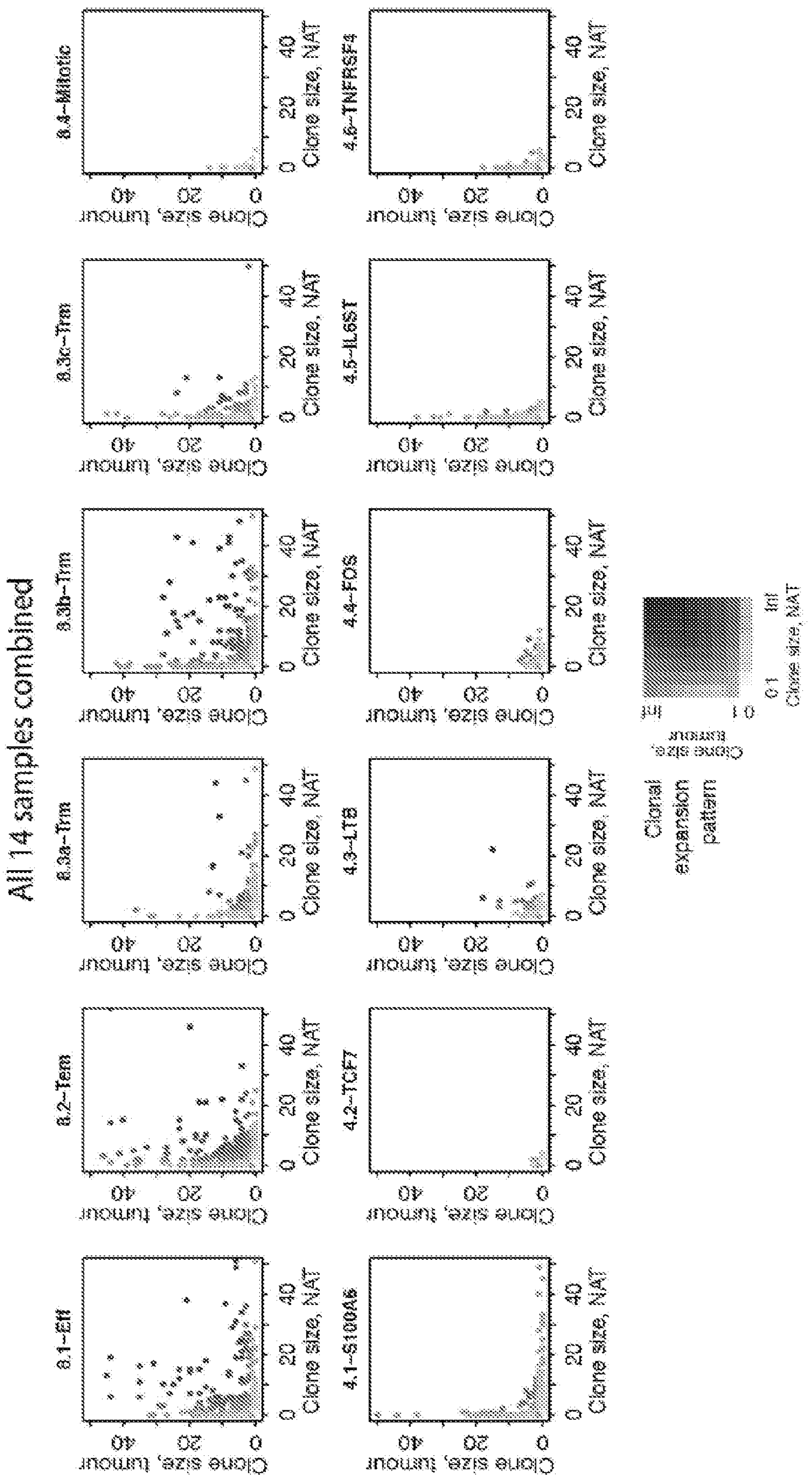
All 14 samples combined
8.1~Eff
8.2~Tem
8.3a~Trm
8.3b~Trm
8.3c~Trm
8.4~Mitotic
4.1~S100A6
4.2~TCF7
4.3~LTB
4.4~FOS
4.5~IL6ST
4.6~TNFRSF4
Clone size, tumour
Clone size, NAT
0
20
40
Clonal expansion pattern
Inf
0.1

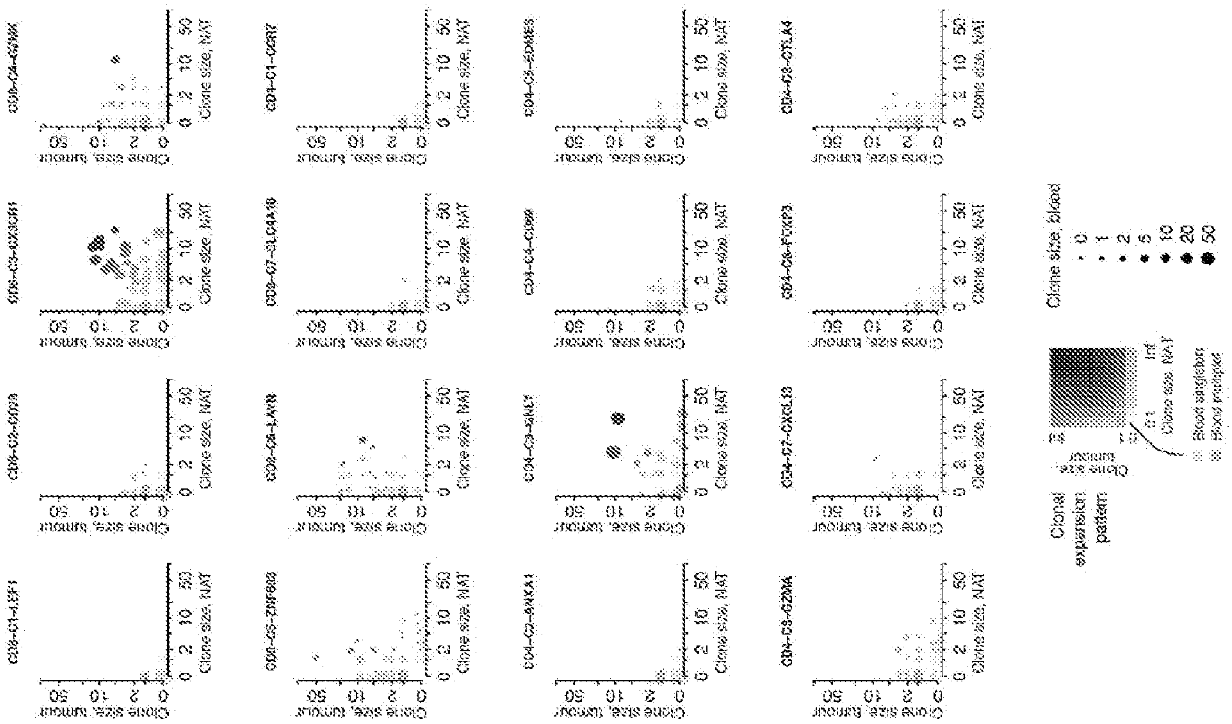

FIG. 19A

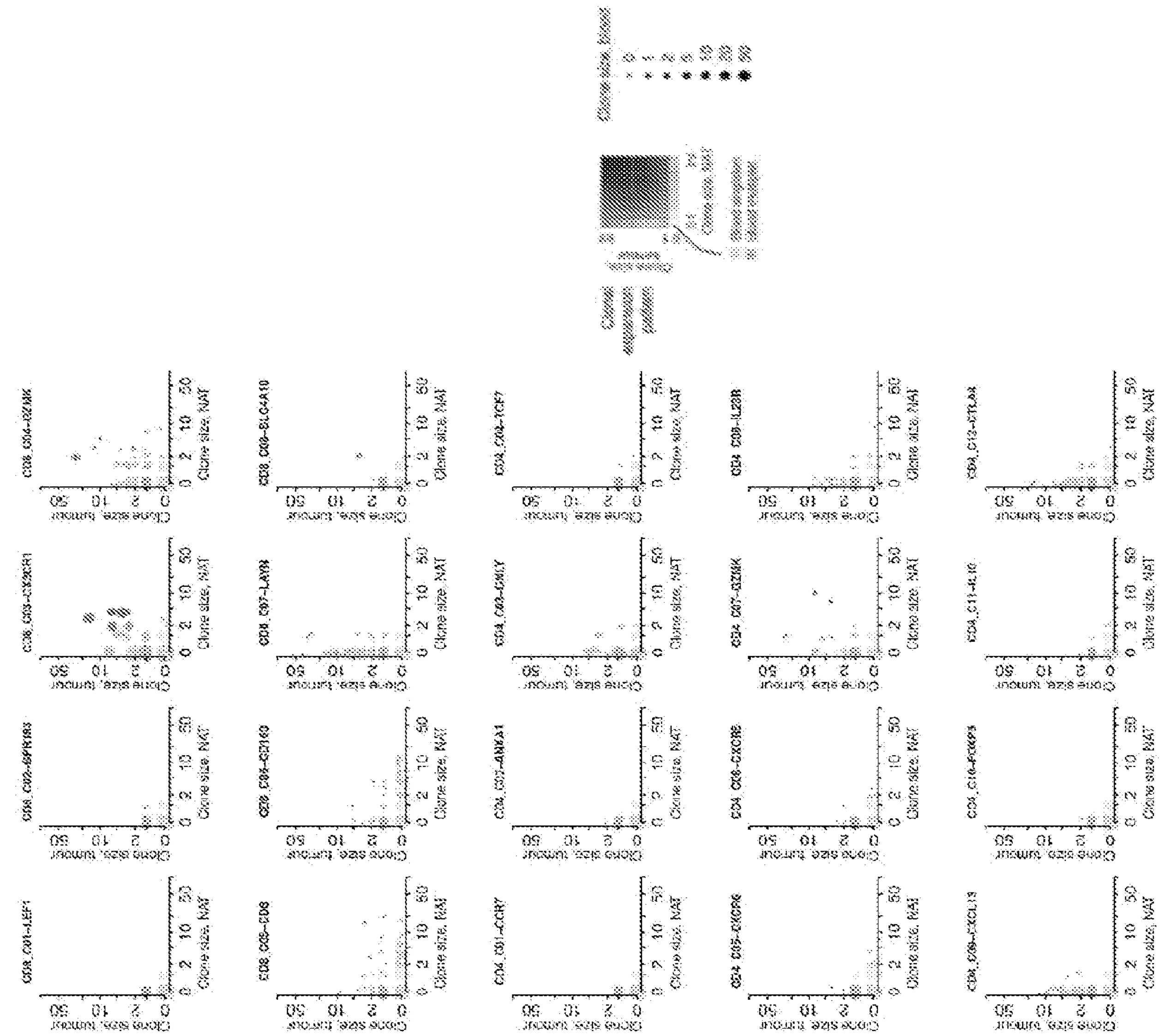

FIG. 19B

FIG. 20A

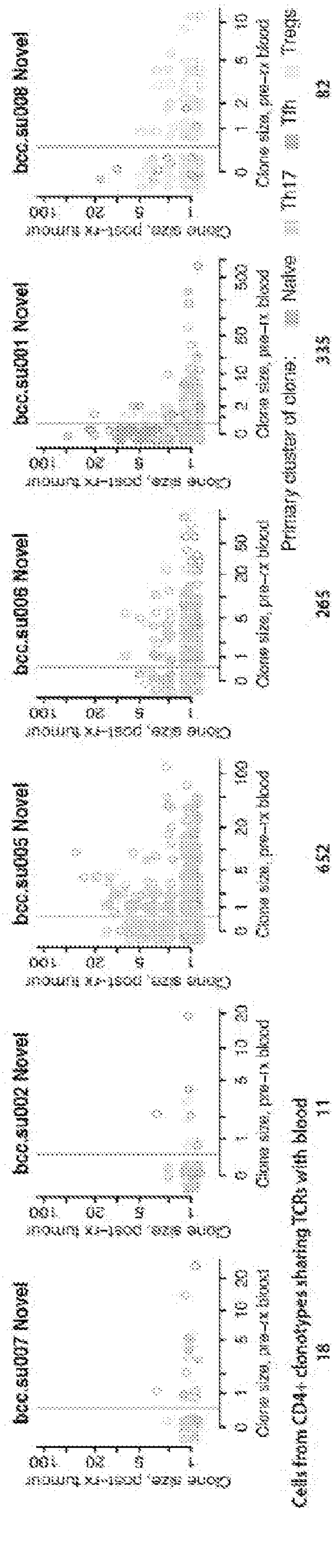
bcc.su007 Novel
bcc.su002 Novel
bcc.su005 Novel
bcc.su006 Novel
bcc.su001 Novel
bcc.su008 Novel
Clone size, post-rx tumour
Clone size, pre-rx blood
Cells from CD4+ clonotypes sharing TCRs with blood
18
11
652
265
335
82
Primary cluster of clone:
Naive
Th17
Tfh
Tregs

FIG. 20B

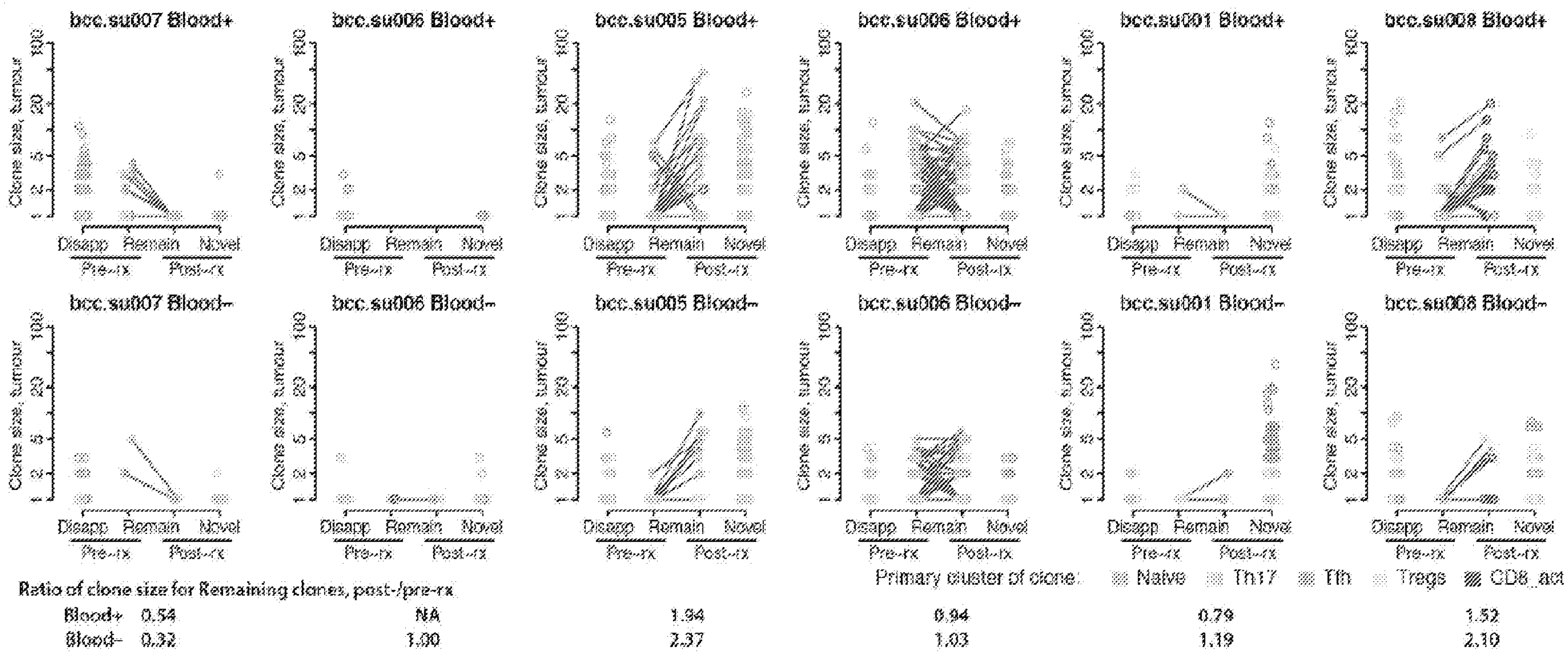
bcc.su007 Blood+
bcc.su006 Blood+
bcc.su005 Blood+
bcc.su006 Blood+
bcc.su001 Blood+
bcc.su008 Blood+
bcc.su007 Blood−
bcc.su006 Blood−
bcc.su005 Blood−
bcc.su006 Blood−
bcc.su001 Blood−
bcc.su008 Blood−
Clone size, tumour
1
2
5
20
100
Disapp
Remain
Novel
Pre−rx
Post−rx
Primary cluster of clone:
Naive
Th17
Tfh
Tregs
CD8_act
Ratio of clone size for Remaining clones, post-/pre-rx
Blood+ 0.54 NA 1.94 0.94 0.79 1.52
Blood− 0.32 1.00 2.37 1.03 1.19 2.10

FIG. 20C

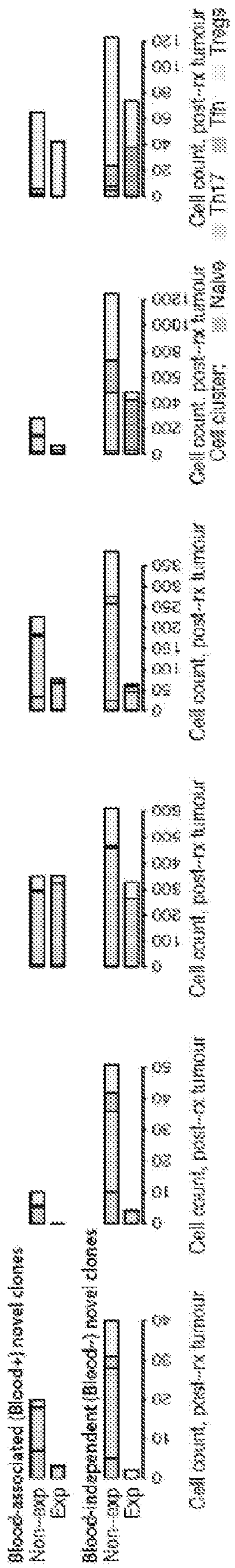
Blood-associated (Blood+) novel clones
Non-exp
Exp
Blood-independent (Blood-) novel clones
Non-exp
Exp
Cell count, post-rx tumour
Cell cluster:
Naive
Th17
Tfh
Tregs

FIG. 21A

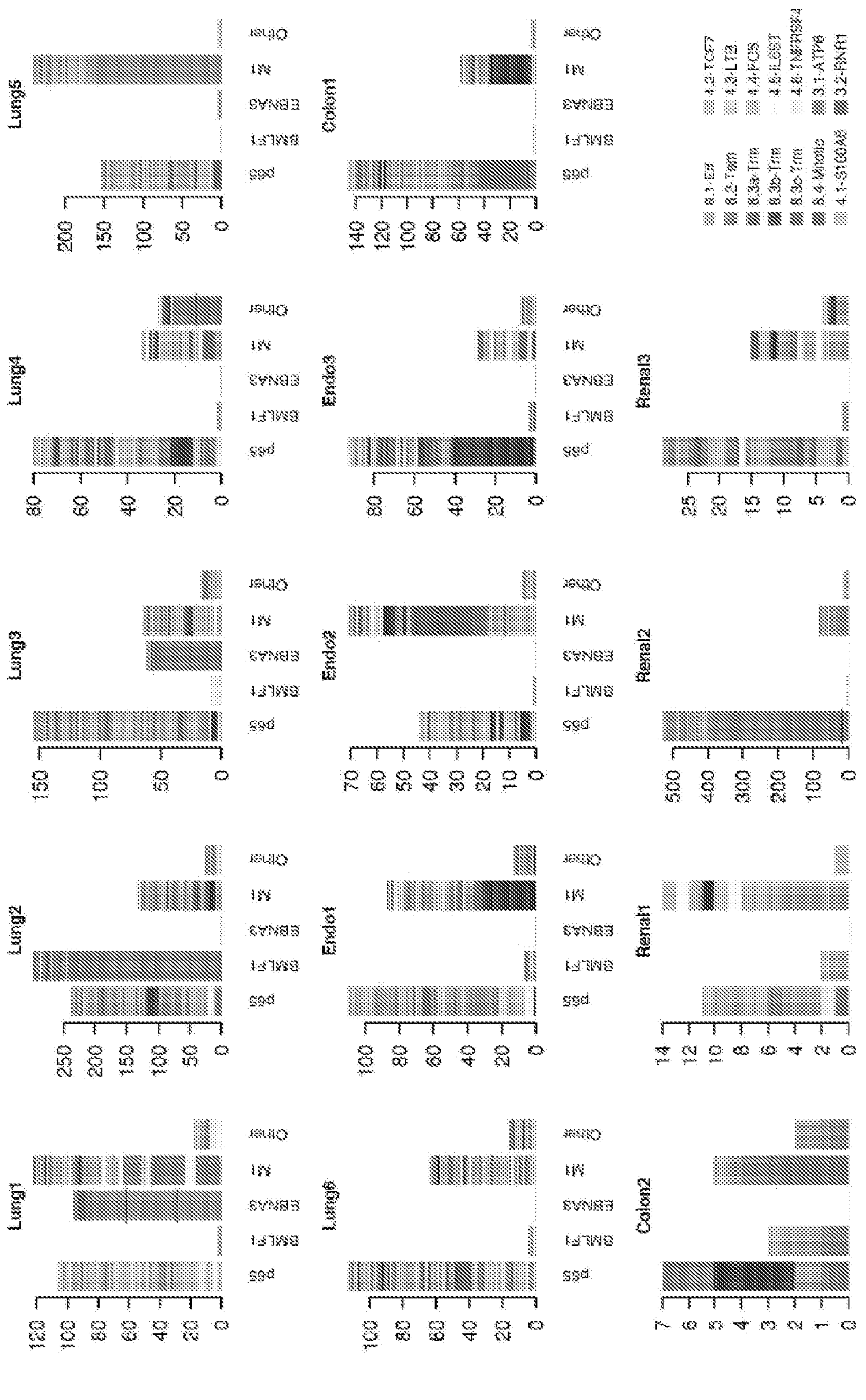
Lung1
Lung2
Lung3
Lung4
Lung5
Lung6
Endo1
Endo2
Endo3
Colon1
Colon2
Renal1
Renal2
Renal3
p65
BMLF1
EBNA3
M1
Other

FIG. 21B

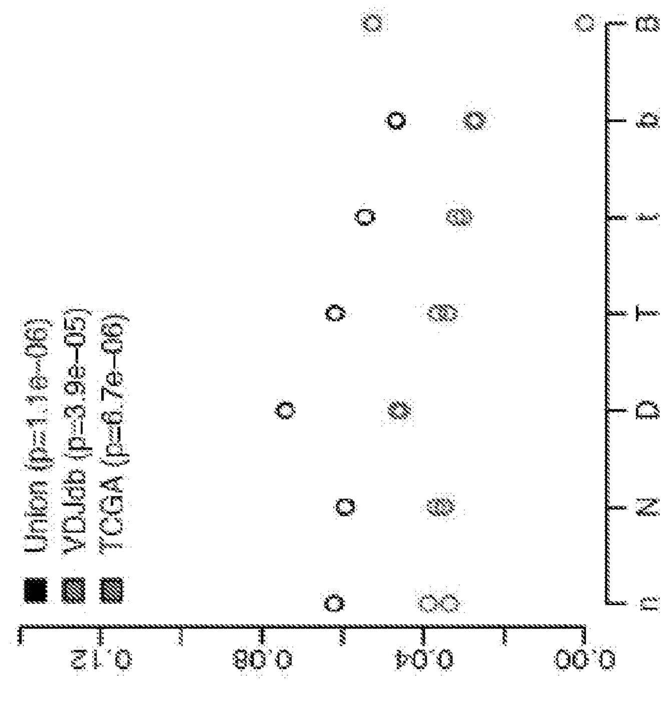


FIG. 21C

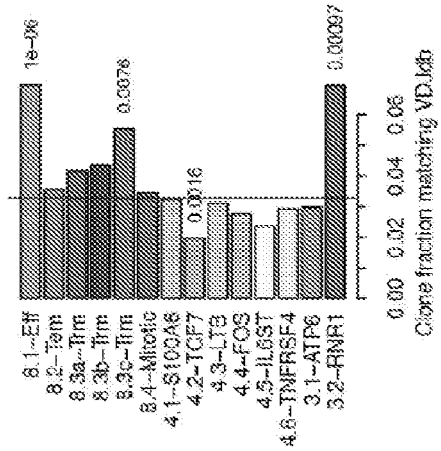


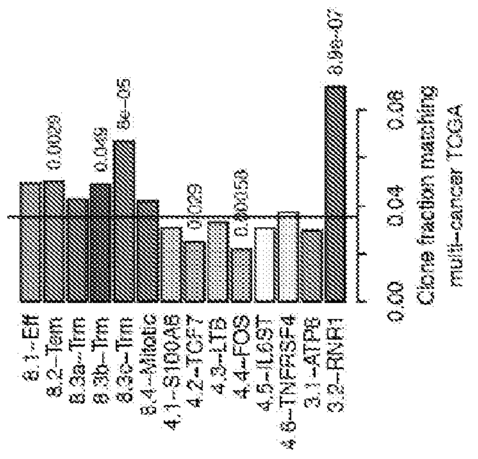

FIG. 22A

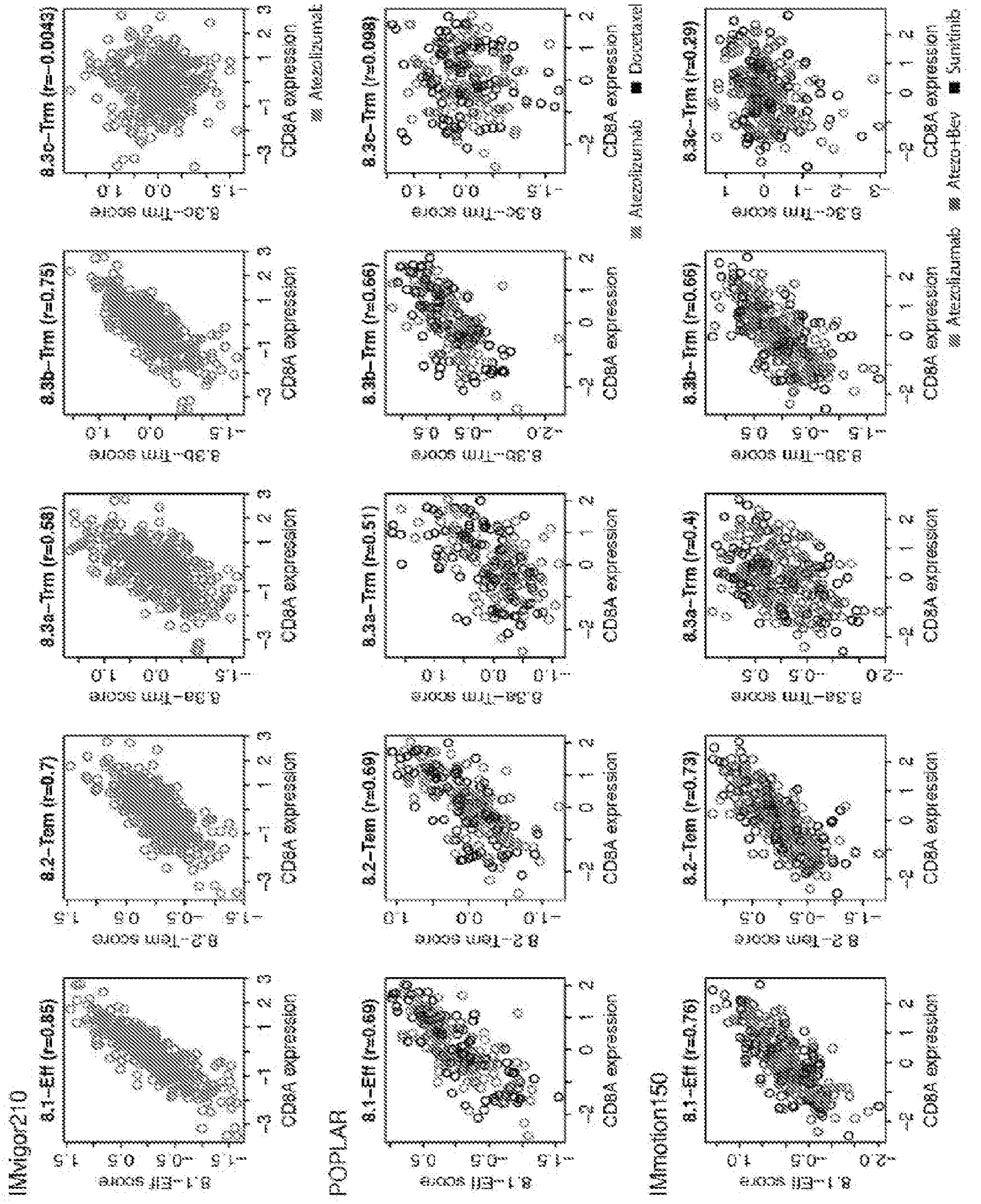
IMvigor210
8.1−Eff (r=0.85)
8.2−Tem (r=0.7)
8.3a−Trm (r=0.58)
8.3b−Trm (r=0.75)
8.3c−Trm (r=−0.0043)
Atezolizumab
POPLAR
8.1−Eff (r=0.69)
8.2−Tem (r=0.69)
8.3a−Trm (r=0.51)
8.3b−Trm (r=0.66)
8.3c−Trm (r=0.098)
Atezolizumab
Docetaxel
IMmotion150
8.1−Eff (r=0.76)
8.2−Tem (r=0.73)
8.3a−Trm (r=0.4)
8.3b−Trm (r=0.66)
8.3c−Trm (r=0.29)
Atezolizumab
Atezo+Bev
Sunitinib
CD8A expression
8.1−Eff score
8.2−Tem score
8.3a−Trm score
8.3b−Trm score
8.3c−Trm score

FIG. 22B

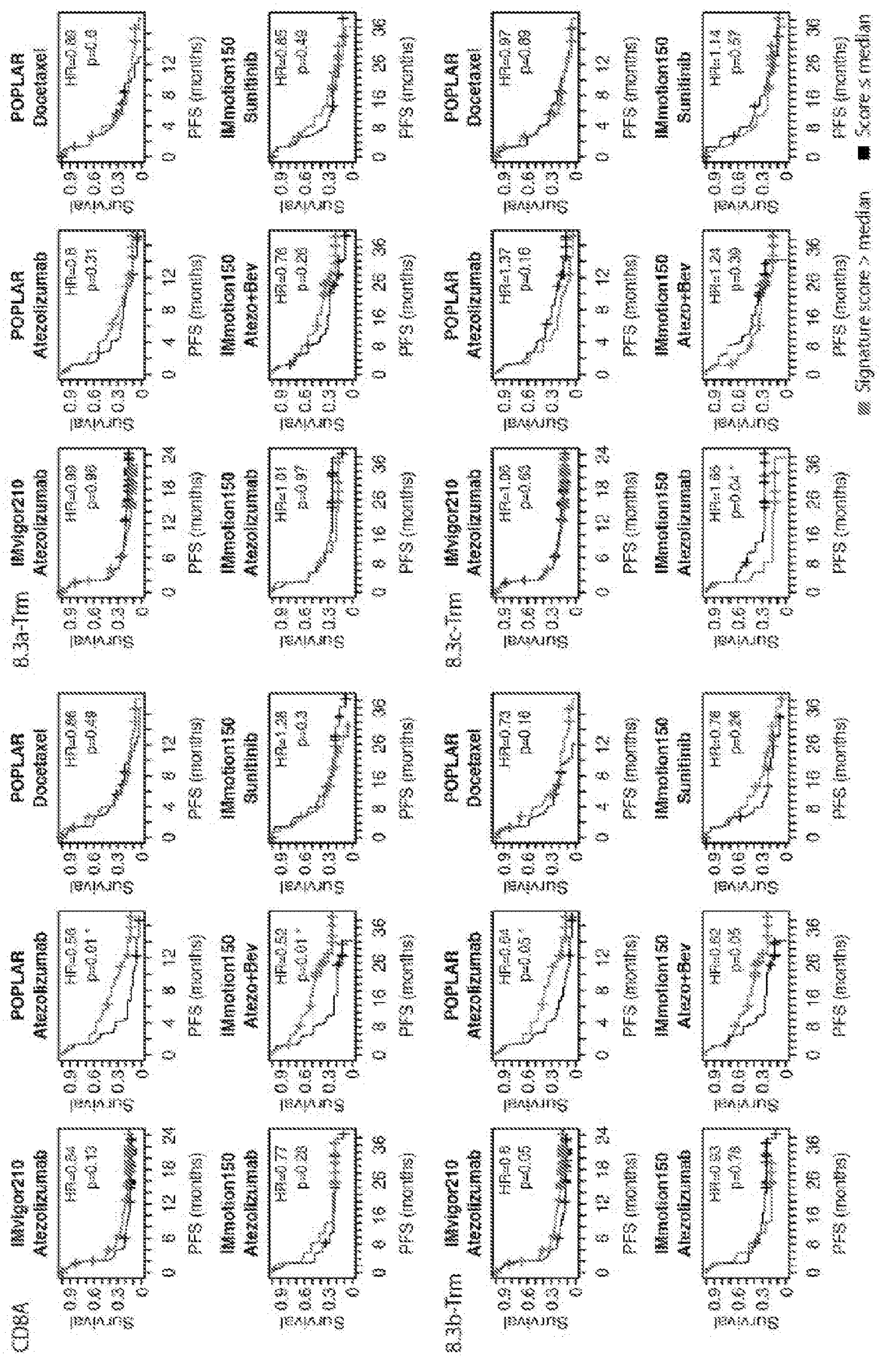
CD8A
8.3a-Tm
8.3b-Tm
8.3c-Tm
IMvigor210 Atezolizumab
POPLAR Atezolizumab
POPLAR Docetaxel
IMmotion150 Atezolizumab
IMmotion150 Atezo+Bev
IMmotion150 Sunitinib
Survival
PFS (months)
Signature score > median
Score ≤ median

Europäisches Patentamt
European Patent Office
Office européen des brevets

# EUROPEAN SEARCH REPORT

Application Number
EP 22 15 2336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/181111 A2 (GENENTECH INC [US]; HOFFMANN LA ROCHE [CH]) 19 October 2017 (2017-10-19) * abstract, paragraph bridging p. 116 to p. 117 - p. 117 first full paragraph * ----- | 1-15 | INV. C07K16/30 C12N5/0783 A61P35/00 A61K35/17 A61K39/395 A61K39/00 C07K16/22 C07K16/28 G01N33/50 |
| X | JAE-HYUN PARK ET AL: "Clonal expansion of antitumor T cells in breast cancer correlates with response to neoadjuvant chemotherapy", INTERNATIONAL JOURNAL OF ONCOLOGY, vol. 49, no. 2, 27 May 2016 (2016-05-27), pages 471-478, XP055674214, GR ISSN: 1019-6439, DOI: 10.3892/ijo.2016.3540 * abstract, the paragraph bridging p. 471 to p. 472 - p. 472 left-hand column second full paragraph, p. 472 right-hand column first - third full paragraphs, the paragraph bridging p. 476 to p. 477 - p. 477 second full paragraph * ----- | 1-15 | |
| | | | TECHNICAL FIELDS SEARCHED (IPC) |
| A | DANIEL S. CHEN ET AL: "Elements of cancer immunity and the cancer-immune set point", NATURE, vol. 541, no. 7637, 1 January 2017 (2017-01-01), pages 321-330, XP055674543, London ISSN: 0028-0836, DOI: 10.1038/nature21349 * abstract, p. 323 right-hand column first full paragraph - p. 327left-hand column first full paragraph * ----- -/-- | 1-15 | C07K C12N A61P A61K |

The present search report has been drawn up for all claims

1

| Place of search | Date of completion of the search | Examiner |
|---|---|---|
| Munich | 5 May 2023 | Hermann, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 2336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2018/209324 A2 (BROAD INST INC [US]; MASSACHUSETTS GEN HOSPITAL [US] ET AL.) 15 November 2018 (2018-11-15) * abstract, [0005], example 17 * ----- | 1-15 | |
| A | THOR STRATEN PER ET AL: "T-cell clonotypes in cancer", JOURNAL OF TRANSLATIONAL MEDICINE, BIOMED CENTRAL, vol. 2, no. 1, 8 April 2004 (2004-04-08), page 11, XP021009813, ISSN: 1479-5876, DOI: 10.1186/1479-5876-2-11 * the whole document * ----- | 1-15 | |
| A | ROY S. HERBST ET AL: "Predictive correlates of response to the anti-PD-L1 antibody MPDL3280A in cancer patients", NATURE, vol. 515, no. 7528, 26 November 2014 (2014-11-26), pages 563-567, XP055262130, London ISSN: 0028-0836, DOI: 10.1038/nature14011 * the whole document * ----- | 1-15 | TECHNICAL FIELDS SEARCHED (IPC) |
| A | WO 2016/168133 A1 (MERCK SHARP & DOHME [US]; AYERS MARK [US] ET AL.) 20 October 2016 (2016-10-20) * the whole document * ----- | 1-15 | |
| A | WO 2016/094377 A1 (MERCK SHARP & DOHME [US]; AYERS MARK D [US] ET AL.) 16 June 2016 (2016-06-16) * the whole document * ----- | 1-15 | |
| A | US 2015/071910 A1 (KOWANETZ MARCIN [US] ET AL) 12 March 2015 (2015-03-12) * the whole document * ----- | 1-15 | |

The present search report has been drawn up for all claims

1

| Place of search | Date of completion of the search | Examiner |
|---|---|---|
| Munich | 5 May 2023 | Hermann, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 15 2336

This annex lists the patent family members relating to the patent documents cited in the above-mentioned European search report. The members are as contained in the European Patent Office EDP file on
The European Patent Office is in no way liable for these particulars which are merely given for the purpose of information.

05-05-2023

| Patent document cited in search report | Publication date | Patent family member(s) | Publication date |
|---|---|---|---|
| WO 2017181111 A2 | 19-10-2017 | AU 2017250296 A1 | 01-11-2018 |
| | | CA 3019921 A1 | 19-10-2017 |
| | | CN 109154613 A | 04-01-2019 |
| | | EP 3443350 A2 | 20-02-2019 |
| | | ES 2850428 T3 | 30-08-2021 |
| | | IL 262225 A | 29-11-2018 |
| | | JP 2019521641 A | 08-08-2019 |
| | | KR 20190003958 A | 10-01-2019 |
| | | PL 3443350 T3 | 31-05-2021 |
| | | US 2019032151 A1 | 31-01-2019 |
| | | WO 2017181111 A2 | 19-10-2017 |
| WO 2018209324 A2 | 15-11-2018 | EP 3622092 A2 | 18-03-2020 |
| | | US 2020147210 A1 | 14-05-2020 |
| | | WO 2018209324 A2 | 15-11-2018 |
| WO 2016168133 A1 | 20-10-2016 | EP 3283882 A1 | 21-02-2018 |
| | | EP 3839510 A2 | 23-06-2021 |
| | | ES 2844799 T3 | 22-07-2021 |
| | | US 2018148790 A1 | 31-05-2018 |
| | | WO 2016168133 A1 | 20-10-2016 |
| WO 2016094377 A1 | 16-06-2016 | AU 2015360736 A1 | 01-06-2017 |
| | | AU 2020204557 A1 | 30-07-2020 |
| | | BR 112017012222 A2 | 30-01-2018 |
| | | CA 2968406 A1 | 16-06-2016 |
| | | CN 107109700 A | 29-08-2017 |
| | | EP 3230498 A1 | 18-10-2017 |
| | | JP 2018505658 A | 01-03-2018 |
| | | KR 20170086661 A | 26-07-2017 |
| | | RU 2017123117 A | 10-01-2019 |
| | | US 2018327848 A1 | 15-11-2018 |
| | | WO 2016094377 A1 | 16-06-2016 |
| US 2015071910 A1 | 12-03-2015 | AR 095363 A1 | 14-10-2015 |
| | | US 2015071910 A1 | 12-03-2015 |
| | | US 2017052188 A1 | 23-02-2017 |
| | | US 2018313845 A1 | 01-11-2018 |
| | | US 2019195882 A1 | 27-06-2019 |
| | | US 2020041520 A1 | 06-02-2020 |
| | | US 2020333348 A1 | 22-10-2020 |
| | | US 2021140967 A1 | 13-05-2021 |
| | | US 2022146517 A1 | 12-05-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4675187 A
- US 6075181 A
- US 6150584 A
- US 4683195 A
- US 5322770 A
- WO 2014127261 A
- WO 2014039523 A
- WO 2014099671 A
- WO 2012079000 A
- WO 2010077634 A
- WO 2007005874 A
- WO 2011066389 A
- US 2013034559 A
- US 8217149 B
- WO 2006121168 A
- WO 2009114335 A
- WO 2010027827 A
- WO 2011066342 A
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 20120251531 A
- US 5500362 A
- WO 5821337 A
- WO 2006029879 A
- WO 2005100402 A
- US 6737056 B
- US 8219149 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 20050260186 A
- US 20060104968 A
- US 6267958 B
- US 6171586 B
- WO 2006044908 A


### Non-patent literature cited in the description


- **LI, B. et al.** *Nat. Genet.,* 2016, vol. 48, 725-732
- **LAMB, J. et al.** *Science,* 2006, vol. 313, 1929-1935
- **GUO, X. et al.** *Nat. Med.,* 2018, vol. 24, 978
- **HARLOW et al.** Using Antibodies: A Laboratory Manual. Cold Spring Harbor Laboratory Press, 1999
- **HARLOW et al.** Antibodies: A Laboratory Manual. Cold Spring Harbor, 1989
- **HOUSTON et al.** *Proc. Natl. Acad. Sci. USA,* 1988, vol. 85, 5879-5883
- **BIRD et al.** *Science,* 1988, vol. 242, 423-426
- **NICOLAOU et al.** *Angew. Chem Intl. Ed. Engl.,* 1994, vol. 33, 183-186
- **KABAT et al.** Sequences of Proteins of Immunological Interest. Public Health Service, National Institutes of Health, 1991
- **HOOGENBOOM ; WINTER.** *J. Mol. Biol.,* 1991, vol. 227, 381
- **MARKS et al.** *J. Mol. Biol.,* 1991, vol. 222, 581

- **COLE et al.** Monoclonal Antibodies and Cancer Therapy. Alan R. Liss, 1985, 77
- **BOERNER et al.** *J. Immunol.,* 1991, vol. 147 (1), 86-95
- **VAN DIJK ; VAN DE WINKEL.** *Curr. Opin. Pharmacol.,* 2001, vol. 5, 368-74
- **LI et al.** *Proc. Natl. Acad. Sci. USA,* 2006, vol. 103, 3557-3562
- **CHOTHIA ; LESK.** *J. Mol. Biol.,* 1987, vol. 196, 901-917
- **MACCALLUM et al.** *J. Mol. Biol.,* 1996, vol. 262, 732-745
- **FLATMAN et al.** *J. Chromatogr. B,* 2007, vol. 848, 79-87
- **MULLIS et al.** *Cold Spring Harbor Symp. Quant. Biol.,* 1987, vol. 51, 263
- PCR Technology. Stockton Press, 1989
- **CRONIN et al.** *Am. J. Pathol.,* 2004, vol. 164 (1), 35-42
- **MA et al.** *Cancer Cell,* 2004, vol. 5, 607-616
- **WANG et al.** RNA-Seq: a revolutionary tool for transcriptomics. *Nature Reviews Genetics,* January 2009, vol. 10 (1), 57-63
- **RYAN et al.** *BioTechniques,* 2008, vol. 45 (1), 81-94
- **MAHER et al.** Transcriptome sequencing to detect gene fusions in cancer. *Nature,* January 2009, vol. 458 (7234), 97-101
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- Current Protocols In Molecular Biology. 1995
- **CRONIN et al.** *Am. J. Pathol.,* 2004, vol. 164 (l), 35-42
- **HELD et al.** *Genome Research,* 1996, vol. 6, 986-994
- **GODFREY et al.** *Malec. Diagnostics,* 2000, vol. 2, 84-91
- **SPECHT et al.** *Am. J. Pathol.,* 2001, vol. 158, 419-29
- **AUSUBEL et al.** Current Protocols of Molecular Biology. John Wiley and Sons, 1997
- **RUPP ; LOCKER.** *Lab Invest.,* 1987, vol. 56, A67
- **DE ANDRES et al.** *Bio Techniques,* 1995, vol. 18, 42044
- **SOKAL R. R. ; RHOLF, F. J.** Biometry: the principles and practice of statistics in biological research. W.H. Freeman and Co, 1995
- **CHOWDHURY.** *Methods Mol. Biol.,* 2008, vol. 207, 179-196
- **HOOGENBOOM et al.** Methods in Molecular Biology. Human Press, 2001, vol. 178, 1-37
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood.,* 2003, vol. 101, 1045-1052
- **CRAGG, M.S ; M.J. GLENNIE.** *Blood.,* 2004, vol. 103, 2738-2743
- **PETKOVA, S.B. et al.** *Int'l. Immunol.,* 2006, vol. 18 (12), 1759-1769
- **SONDERMANN et al.** *Nature,* 2000, vol. 406, 267-273
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- **YAMADA et al.** *Cancer Sci.,* 2013, vol. 104, 14-21
- Remington's Pharmaceutical Sciences. 1980
- **VAN DER MAATEN, L. ; HINTON, G.** *J Machine Learning Research,* 2008, vol. 9, 2579-2605
- **BUTLER, A. et al.** *Nature Biotechnology,* 2018, vol. 36, 411-420
- **SAVAS, P. et al.** *Nat. Med,* 2018, vol. 13, 228
- **KOLLING, M. et al.** *Scientific Reports,* 2018, vol. 8, 3438
- **ZAPPASODI, R. et al.** *Cancer Cell,* 2018, vol. 33, 1017-1032
- **GU-TRANTIEN, C. et al.** *JCI Insight,* 2017, vol. 2, e91487
- **DIEU-NOSJEAN, M-C. et al.** *Trends in Immunology,* 2014, vol. 35, 571-580
- **THOMPSON, E. D. et al.** *J Exp Med,* 2010, vol. 207, 1791-1804

- **GROGAN, J. L. ; OUYANG W.** *Eur J Immunol,* 2012, vol. 42, 2255-2262
- **THOMMEN, D. S.** *Nat. Med.,* 2018, vol. 24, 994-1004
- **GUO, X. et al.** *Nat. Med,* 2018, vol. 24, 978
- **DE SILLY, R. V. ; DIETRICH, P-Y. ; WALKER, P. R.** *Oncoimmunology,* 2016, vol. 5, e1232236
- **CHANG, C-H. ; PEARCE, E. L.** *Nat. Immunol.,* 2016, vol. 17, 364-368
- **JOYCE, J. A. ; FEARON, D. T.** *Science,* 2015, vol. 348, 74-80
- **OHRI, C. M. et al.** *BMC Cancer,* 2010, vol. 10, 172
- **PARKHURST, M. et al.** *Clin Cancer Res,* 2017, vol. 23, 2491-2505
- **SHUGAY, M. et al.** *Nucleic Acids Res,* 2018, vol. 46, D419-D427
- **WANG, G. C. et al.** *Sci Transl Med,* 2012, vol. 4, 128-42
- **KLINGER, M.** *PLoS ONE,* 2015, vol. 10, e0141561
- **DELIYANNIS, G. et al.** *J Virol,* 2002, vol. 76, 4212-4221
- **BIRNBAUM, M.E. et al.** *Cell,* 2014, vol. 157, 1073-1087
- **SIMONI, Y. et al.** *Nature,* 2018, vol. 557, 575-579
- **GROS, A. et al.** *J. Clin. Invest.,* 2014, vol. 124, 2246-2259
- **PASETTO, A. et al.** *Cancer Immunol Res,* 2016, vol. 4, 734-743
- **DIAMOND, M. S ; FARZAN, M.** *Nat Rev Immunol,* 2013, vol. 13, 46-57
- **MUELLER, S. N. et al.** *Ann Rev Immunol,* 2013, vol. 31, 137-161
- **MASOPUST, D. ; SCHENKEL, J. M.** *Nat Rev Immunol,* 2013, vol. 13, 303-320
- **SCHENKEL, J. M. ; MASOPUST, D.** *Immunity,* 2014, vol. 41, 886-897
- **SLUTTER, B. et al.** *Science Immunology,* 2017, vol. 2, eaag2013
- **CHEVRIER, S. et al.** *Cell Systems,* 2018, vol. 6, 612-620.e5
- **HAHNE, F. et al.** *BMC Bioinformatics,* 2009, vol. 10, 106
- **MORGAN, M. ; DAVIS, S. R.** *bioRxiv,* 2017, https://doi.org/10.1101/117200
- **DAVIS, S. ; MELTZER, P. S.** *Bioinformatics,* 2007, vol. 23, 1846-1847
- **QIU, X. et al.** *Nature Methods,* 2017, vol. 14, 979-982
- **STOREY, K.D.** *J. Royal Stat Soc, Series B,* vol. 64, 479-498
- **LANGFELDER, P. ; HORVATH, S.** *BMC Bioinformatics,* 2008, vol. 9, 559
- **DAVIS, S. ; MELTZER, P. S.** *Bioinformatics,* 2007, vol. 23, 1846-1847
- **DUO, A. et al.** *F1000Research,* 2018, vol. 7, 1141
- **SAVAS, P. et al.** *Nat. Med.,* 2018, vol. 13, 228
- **AZIZI, E. et al.** *Cell,* 2018, vol. 174, 1-16
- **WANG, G.C. et al.** *Sci Transl Med,* 2012, vol. 4, 128-42
- **SUBRAMANIAN, A. et al.** *Proc Natl Acad Sci USA,* 2005, vol. 102, 15445-15550
- **FEHRENBACHER, L. et al.** *Lancet,* 2016, vol. 387, 1837-1846
- **HOFFMAN-CENSITS, J. H. et al.** *J Clin Oncology,* 2016, vol. 34, 355-355
- **CHEN, D. S. ; MELLMAN, I.** *Nature,* 2017, vol. 541, 321-330
- **MELLMAN, I. ; COUKOS, G. ; DRANOFF, G.** *Nature,* 2011, vol. 480, 480-489
- **WHERRY, E. J. ; KURACHI, M.** *Nature Rev Immunol,* 2015, vol. 14, 486-499
- **TOPALIAN, S. L. ; DRAKE, C. G. ; PARDOLL, D. M.** *Cancer Cell,* 2015, vol. 27, 450-461
- **PAUKEN, K. E. et al.** *Science,* 2016, vol. 354, 1060-1065
- **PHILIP, M. et al.** *Nature,* 2017, vol. 545, 562-456
- **KHAN, O. et al.** *Nature,* 2019, vol. 571, 211-218
- **SCOTT, A. C. et al.** *Nature,* 2019, vol. 571, 270-274
- **GROS, A. et al.** *Nat Med,* 2016, vol. 22, 433-438
- **SCHUMACHER, T. N. ; SCHEPER, W.** *Nat Med,* 2016, vol. 22, 340-341
- **MANJARREZ-ORDUÑO, N. et al.** *Front Immunol,* 2018, vol. 9, 1613
- **HOGAN, S. A. et al.** *Cancer Immunol Res,* 2019, vol. 7, 77-85
- **GUO, X. et al.** *Nat Med,* 2018, vol. 24, 978-985
- **YOST, K. et al.** *Nat Med,* 2019, vol. 25, 1251-1259
- **BRATKE, K. et al.** *Eur J Immunol,* 2005, vol. 35, 2608-2616
- **ABDELSAMED, H. A. et al.** *J Exp Med,* 2017, vol. 214, 1593-1606
- **IM, S. J.** *Nature,* 2016, vol. 537, 417-421
- **MILLER, B. C. et al.** *Nature Immunol,* 2019, vol. 20, 326-336
- **MACKAY, L. K. et al.** *Science,* 2016, vol. 352, 459-463
- **THOMMEN, D. S. et al.** *Nat Med,* 2018, vol. 24, 994-1004
- **ZHANG, L. et al.** *Nature,* 2018, vol. 564, 268-272
- **SHULMAN, Z. et al.** *Nature Immunol,* 2012, vol. 13, 67-76
- **SCHEPER, W. et al.** *Nat Med,* 2019, vol. 25, 89-94
- **LI, B et al.** *Nat Genet,* 2016, vol. 48, 725-732

- **MCNALLY, J. M. et al.** *J Virol,* 2001, vol. 75, 5965-5976
- **MARIATHASAN, S.** *Nature,* 2018, vol. 22, 544-548
- **MCDERMOTT, D. F. et al.** *Nat Med,* 2018, vol. 24, 749-757
- **YAN, Y. et al.** *JCI Insight,* 2018, vol. 3, e97828
- **YAMAGUCHI, K.** *Cancer Sci,* 2018, vol. 109, 3032-3042
- **ZHENG, G. X. Y. et al.** *Nature Communications,* 2017, vol. 8, 14049
- **STUART, T. et al.** *Cell,* 2019, vol. 177, 1888-1902
- **RITCHIE, M.E. et al.** *Nucleic Acids Research,* 2015, vol. 43, e47
- **DEL CARRATORE, F. et al.** *Bioinformatics,* 2017, vol. 33, 2774-2775
- **STOREY, J. D.** *Journal of the Royal Statistical Society, Series B,* 2002, vol. 64, 479-498
- **ARAN, D. et al.** *Nat Immunol,* 2019, vol. 20, 163-172
- **THERNEAU, T. M. ; GRAMBSCH, P. M.** Modeling Survival Data: Extending the Cox Model. Springer, 2000
- **ZEILEIS, A. et al.** colorspace: a toolbox for manipulating and assessing colors and palettes. *Technical Report 1903.06490, arXiv.org,* 2019
- **MORGAN, M. T. ; DAVIS, S. R.** Genomic Data Commons: a Bioconductor interface to the NCI Genomic Data Commons, Technical Report 117200. *bioRxiv.org,* 2017
- **DAVIS, S. ; MELTZER.** *Bioinformatics,* 2007, vol. 14, 1846-1847
- **BECHT, E. et al.** *Nature Biotechnology,* 2019, vol. 37, 38-44